# EUROPEAN PATENT SPECIFICATION

(11) **EP 4 041 393 B1**
(45) Date of publication and mention of the grant of the patent: **08.04.2026**
(21) Application number: 20790232.1
(22) Date of filing: 08.10.2020
(51) Int. Cl.: A01N 43/653, A01N 43/78, A01N 43/84, A61P 33/14, C07C 317/46, C07D 401/04, C07D 401/14, C07D 403/04, C07D 403/14, C07D 405/14, C07D 413/14, C07D 417/04

(54) **NOVEL HETEROARYL-TRIAZOLE COMPOUNDS AS PESTICIDES**
NEUARTIGE HETEROARYL-TRIAZOL-VERBINDUNGEN ALS PESTIZIDE
NOUVEAUX COMPOSÉS D'HÉTÉROARYL-TRIAZOLE EN TANT QUE PESTICIDES

(30) Priority: 09.10.2019 EP 19202293; 12.12.2019 EP 19215749; 02.06.2020 EP 20177852
(43) Date of publication of application: 17.08.2022
(73) Proprietor: Bayer Aktiengesellschaft, 51373 Leverkusen (DE)
(72) Inventor: JESCHKE, Peter, 51467 Bergisch Gladbach (DE); SCHWARZ, Hans-Georg, 46282 Dorsten (DE); ARLT, Alexander, 50859 Köln (DE); FÜßLEIN, Martin, 40225 Düsseldorf (DE); CANCHO GRANDE, Yolanda, 51373 Leverkusen (DE); TELSER, Joachim, 42115 Wuppertal (DE); LÖSEL, Peter, 51371 Leverkusen (DE); LINKA, Marc, 40223 Düsseldorf (DE); EBBINGHAUS-KINTSCHER, Ulrich, 44287 Dortmund (DE); DAMIJONAITIS, Arunas Jonas, 51371 Leverkusen (DE); HELLWEGE, Elke, 40764 Langenfeld (DE); TURBERG, Andreas, 42781 Haan (DE); HEISLER, Iring, 40593 Düsseldorf (DE)
(74) Representative: Cohausz & Florack
(86) International application number: PCT/EP2020/078248
(87) International publication number: WO 2021/069567

(56) References cited:
- EP-A1- 1 544 202
- WO-A1-2012/080376
- WO-A1-2017/192385
- WO-A1-2020/079198
- WO-A1-2020/094363
- DATABASE chemcats [online] 22 September 2013 (2013-09-22), CHEMICAL LIBRARY- CHEMBRIDGE CORPORATION: "N-((3-(3-chlorophenyl)methyl-1-(1-methyl-4-piperidinyl)-1H-1,2,4-triazol-5-yl)methyl)-acetamide", XP002797849, Database accession no. 1452979-00-8
- DATABASE chemcats [online] 6 June 2014 (2014-06-06), CHEMICAL LIBRARY- CHEMBRIDGE CORPORATION: "N-((3-(3-chlorophenyl)methyl)-1-(6-methyl-2-pyridinyl)-1H-1,2,4-triazol-5-yl)methyl)acetamide", XP002797850, Database accession no. 1609831-36-8
- DATABASE chemcats [online] 16 March 2018 (2018-03-16), CHEMICAL LIBRARY- AURORA FINE CHEMICALS: "N-((1S)-1-(3-(4-methoxyphenyl)methyl)-1-(4-pyridinyl)-1H-1,2,4-triazol-5-yl)ethyl)-acetamide", XP002797851, Database accession no. 2192638-31-4
- DATABASE chemcats [online] 22 September 2013 (2013-09-22), CHEMICAL LIBRARY- CHEMBRIDGE CORPORATION: "N-((1-(1,3-benzodioxol-5-yl)3-(2-phenylethyl)-1H-1,2,4-triazol-5-yl)methyl)-acetamide", XP002797852, Database accession no. 1453044-62-6
- DATABASE chemcats [online] 21 March 2011 (2011-03-21), CHEMICAL LIBRARY- CHEMBRIDGE CORPORATION: "N-((2-(3-(2-phenylethyl)1-(2-pyridinly)-1H-1,2,4-triazol-5-yl)ethyl)-acetamide", XP002797853, Database accession no. 1269046-03-8
- DATABASE CHEMcats [online] 21 March 2011 (2011-03-21), CHEMICAL LIBRARY- CHEMBRIDGE CORPORATION: "N-((3-(2-phenylethyl)-1-(2-pyridinyl)-1H-1,2,4-triazol-5-yl)methyl)-benzamide", XP002797854, Database accession no. 1269003-64-6

## Description

The present invention relates to novel heteroaryl-triazole compounds, to formulations and compositions comprising such compounds and to their use in the control of animal pests including arthropods and insects in plant protection and to their use for the control of ectoparasites on animals.

Certain heteroaryl-triazole compounds of formula I (R^{3b} = hydrogen) are disclosed for the use in controlling ectoparasites on animals in WO 2017/192385 and for the use in controlling animal pests including arthropods and insects in the field of plant protection in WO 2019/170626 and WO 2019/215198. Further, the patent applications WO 2019/197468, WO 2019/201835, WO 2019/202077 and WO 2019/206799 disclose certain heteroaryl-triazole compounds for the use in controlling ectoparasites on animals and for the control of animal pests including arthropods and insects in the field of plant protection. WO 2020/002563, WO 2020/053364, WO 2020/053365, WO 2020/079198, WO 2020/094363, EP3696175 A1 describe azole-amide compounds all of which can be used as insecticides.

Modern plant protection products and veterinary ectoparasiticides have to meet many demands, for example in relation to efficacy, persistence, spectrum and resistance breaking properties. Questions of toxicity, the combinability with other active compounds or formulation auxiliaries play a role, as well as the question of the expense that the synthesis of an active compound requires. Furthermore, resistances may occur. For all these reasons, the search for novel crop protection compositions or veterinary ectoparasiticides cannot be considered to be complete, and there is a constant need for novel compounds having properties which, compared to the known compounds, are improved at least in respect of individual aspects.

It was an object of the present invention to provide compounds which widen the spectrum of the pesticides in various aspects.

The present invention therefore provides compounds of the general formula (I) 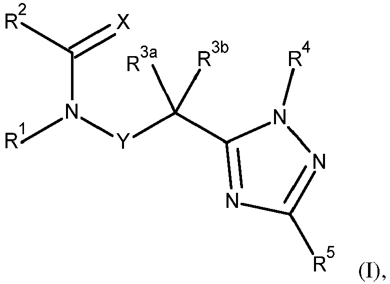 in which

### (Configuration 5-2)

- X: is O;
- Y: is a direct bond;
- R¹: is hydrogen;
- R²: is 3,5-dichlorophenyl, 3,5-dibromophenyl, 3-bromo-5-chlorophenyl, 3-cyano-5-fluorophenyl, 3-chloro-5-cyanophenyl, 3-chloro-5-(trifluoromethyl)phenyl, 3-bromo-5-(trifluoromethyl)phenyl, 3,5-bis(trifluoromethyl)phenyl, 3-chloro-5-(trifluoromethoxy)phenyl, 3-bromo-5-(trifluoromethoxy)phenyl, 3-chloro-5-(methylsulfonyl)phenyl, 3-chloro-5-(difluoromethylsulfonyl)phenyl, 3-chloro-5-(trifluoromethylsulfonyl)phenyl, 3-methylsulfonyl-5-(trifluoromethyl)phenyl, 3-ethylsulfonyl-5-(trifluoromethyl)phenyl, 3-methylsulfonyl-5-(trifluoromethoxy)phenyl, 3-ethylsulfonyl-5-(trifluoromethoxy)phenyl, 3-isopropylsulfonyl-5-(trifluoromethoxy)phenyl, 3-cyclopropylsulfonyl-5-(trifluoromethoxy)phenyl, 3-(difluoromethylsulfonyl)-5-(trifluoromethoxy)phenyl, 3-(4-chlorophenyl)sulfonyl-5-(trifluoromethoxy)phenyl, 3-cyclopropyl-5-(trifluoromethoxy)phenyl, 3-cyclopropyl-5-(trifluoromethylsulfonyl)phenyl, 3-bromo-5-[1-(trifluoromethyl)cyclopropyl]phenyl, 3-chloro-5-(1-cyano-1-methylethyl)phenyl, 2,6-dichloropyridin-4-yl, 2-chloro-6-(trifluoromethyl)-pyridin-4-yl, 5-(trifluoromethyl)-pyridin-3-yl, or 1-methyl-5-(trifluoromethyl)-pyrazol-3-yl;
- R^{3a}: is H;
- R^{3b}: is methyl;
- R⁴: is pyrimidin-2-yl, 5-chloropyrimidin-2-yl, 5-chloropyridin-2-yl, 5-cyanopyridin-2-yl, 5-cyano-1,3-thiazol-2-yl, 5-carboxypyridin-2-yl, 5-(methoxycarbonyl)pyridin-2-yl, 5-(dimethylaminocarbonyl)pyridin-2-yl, 5-[[ethyl(methyl)amino]carbonyl]pyridin-2-yl, 5-[[cyclopropyl(methyl)amino]carbonyl]pyridin-2-yl, 5-[[cyclopropylmethyl(methyl)amino]carbonyl]pyridin-2-yl, 5-[(cyclopropylcarbonyl)(methyl)amino]-pyridin-2-yl, 5-(pyrrolidin-1-ylcarbonyl)pyridin-2-yl, 5-(morpholin-4-ylcarbonyl)pyridin-2-yl, 5-(morpholin-4-carbonyl)thiazol-2-yl], 5-[[rac-(2R,6R)-2,6-dimethylmorpholin-4-yl]carbonyl]pyridin-2-yl, or 5-[[(2R,6S)-2,6-dimethylmorpholin-4-yl]carbonyl]pyridin-2-yl;
- R⁵: is -NHCO-tert-butyloxy, -NH₂, -N-methyl-CO-tert-butyloxy, -NHCO-methoxy, -NHCO-methyl, -NHCO-cyclopropyl, -NHCO-(1-chloro-cyclopropyl), -NHCO-(2-fluorocyclopropyl), -NHCO-(2,2-difluoro-cyclopropyl), -NHCO-(2,2,2-trifluoroethyl), -NHCO-(1-chloroethyl), -NHSO₂-trifluoromethyl, -N(SO₂-methyl)₂, methylamino, dimethylamino, acetyl(methyl)amino, prop-2-enoylamino, methoxy(methyl)amino, pyrrolidine-1-yl, piperidin-1-yl, or morpholin-4-yl.

In a further preferred embodiment (embodiment 1b), the invention relates to compounds of the formula (I") in which R^{3b} is Me, and R^{3a} is H 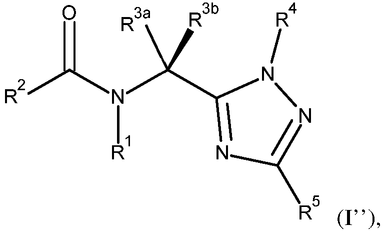 in which the structural elements Y, R¹, R², R⁴ and R⁵ have the meanings given in Configuration (5-2).

In a further preferred embodiment (embodiment 1c), the invention relates to compounds of the formula (I‴) in which R^{3b} is Me, and R^{3a} is H 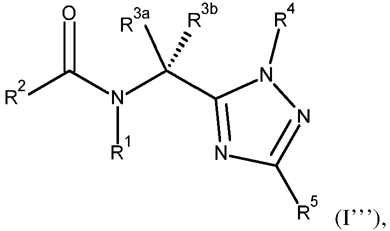 in which the structural elements Y, R¹, R², R⁴ and R⁵ have the meanings given in Configuration (5-2).

In a further preferred embodiment (embodiment 2a), the invention relates to compounds of the formula (I) 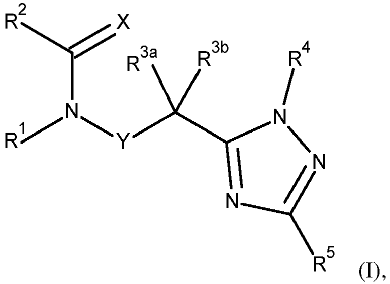 in which the structural elements X, Y, R¹, R^{3a}, R^{3b}, R⁴ and R⁵ have the meanings given in Configuration (5-2)
and
- R²: is phenyl, pyridine, pyrimidine, pyrazine or pyridazine, wherein the phenyl, pyridine, pyrimidine, pyrazine or pyridazine is substituted with a total of one to three substituents, provided the substituent(s) are not on either carbon adjacent to the carbon bonded to the C=X group and at least one and up to two substituent(s) are independently selected from group A consisting of
C₁-C₄-alkyl substituted by one to two substituents selected from the group consisting of - CN; and C₃-C₆cycloalkyl, wherein the C₃-C₆cycloalkyl is optionally substituted with halogen, -CN, C₁-C₄-alkyl or C₁-C₄-haloalkyl; C₁-C₄alkylthio, C₁-C₄alkylsulfinyl, C₁-C₄alkylsulfonyl, C₃-C₆cycloalkylsulfanyl, C₃-C₆cycloalkylsulfinyl, C₃-C₆cycloalkylsulfonyl, C₁-C₄haloalkylsulfinyl, and C₁-C₄haloalkylsulfonyl;
phenylsulfanyl wherein the phenyl is optionally substituted with one to two substituents selected from the group consisting of halogen, -CN, C₁-C₃alkyl, C₁-C₃haloalkyl and C₁-C₃haloalkoxy;
phenylsulfinyl wherein the phenyl is optionally substituted with one to two substituents selected from the group consisting of halogen, -CN, C₁-C₃alkyl, C₁-C₃haloalkyl and C₁-C₃haloalkoxy;
phenylsulfonyl wherein the phenyl is optionally substituted with one to two substituents selected from the group consisting of halogen, -CN, C₁-C₃alkyl, C₁-C₃haloalkyl and C₁-C₃haloalkoxy;
and phenyl and 5- to 6-membered heteroaryl, wherein the phenyl or 5- to 6-membered heteroaryl is optionally substituted with one to two substituents selected from the group consisting of halogen, -CN, C₁-C₃alkyl, C₁-C₃haloalkyl and C₁-C₃haloalkoxy;
   the other one to two optional substituent(s) are each independently selected from group B consisting of
halogen, -CN, -NO₂, C₁-C₆alkyl, C₃-C₆cycloalkyl, C₁-C₄haloalkyl, C₁-C₄alkoxy, C₁-C₄haloalkoxy, C₁-C₄alkylthio, C₁-C₄alkylsulfinyl, C₁-C₄alkylsulfonyl, C₁-C₄haloalkylthio, C₁-C₄haloalkylsulfinyl, and C₁-C₄haloalkylsulfonyl;
   or
- R²: is thiophene, furane, pyrazole, thiazole, isothiazole, oxazole or isoxazole each of which is optionally substituted by one to three substituents independently selected from the group consisting of halogen, hydroxy, -CN, -NO₂; C₁-C₆alkyl optionally substituted by -CN; C₃-C₆cycloalkyl, optionally substituted by halogen, -CN, C₁-C₄-alkyl or C₁-C₄-haloalkyl; C₁-C₄haloalkyl, C₁-C₄alkoxy, C₁-C₄haloalkoxy, C₁-C₄alkylthio, C₁-C₄alkylsulfinyl, C₁-C₄alkylsulfonyl, C₃-C₆cycloalkylsulfanyl, C₃-C₆cycloalkylsulfinyl, C₃-C₆cycloalkylsulfonyl, C₁-C₄haloalkylthio, C₁-C₄haloalkylsulfinyl, C₁-C₄haloalkylsulfonyl;
and phenyl and 5- to 6-membered heteroaryl, wherein the phenyl or 5- to 6-membered heteroaryl is optionally substituted with one to two substituents selected from the group consisting of halogen, -CN, C₁-C₃alkyl and C₁-C₃haloalkyl.

In a further preferred embodiment (embodiment 2b), the invention relates to compounds of the formula (I) 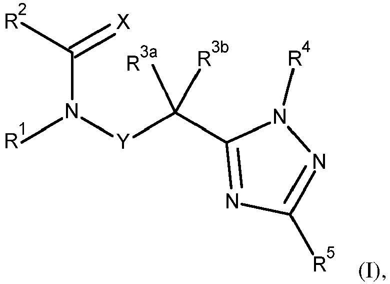 in which the structural elements X, Y, R¹, R^{3a}, R^{3b}, R⁴ and R⁵ have the meanings given in Configuration (5-2)
and
- R²: is phenyl or pyridine, wherein the phenyl or pyridine is substituted with a total of one to three substituents, provided the substituent(s) are not on either carbon adjacent to the carbon bonded to the C=X group and one substituent is independently selected from group A consisting of
1-cyano-1-methyl-ethyl, cyclopropyl, 1-cyanocyclopropyl, 1-(trifluoromethyl)cyclopropyl, methylthio, methylsulfinyl, methylsulfonyl, ethylthio, ethylsulfinyl, ethylsulfonyl, isopropylthio, isopropylsulfinyl, isopropylsulfonyl, cyclopropylthio, cyclopropylsulfinyl, cyclopropylsulfonyl, difluoromethylsulfinyl, difluoromethylsulfonyl, trifluoromethylsulfinyl, trifluoromethylsulfonyl;
phenylsulfonyl wherein the phenyl is optionally substituted with one two substituents selected from the group consisting of fluorine, chlorine, bromine, -CN, difluoromethyl, trifluoromethyl and trifluoromethoxy;
and phenyl and 5-membered heteroaryl wherein the phenyl or 5-membered heteroaryl is optionally substituted with one two substituents selected from the group consisting of fluorine, chlorine bromine, -CN, difluoromethyl, trifluoromethyl and trifluoromethoxy;
   the other one to two optional substituent(s) are each independently selected from group B consisting of
fluorine, chlorine, bromine, iodine, -CN, -NO₂, methyl, cyclopropyl, difluoromethyl, trifluoromethyl, methoxy, difluoromethoxy, trifluoromethoxy, methylthio, methylsulfinyl, methylsulfonyl, difluoromethylthio, difluoromethylsulfinyl, difluoromethylsulfonyl, trifluoromethylthio, trifluoromethylsulfinyl, trifluoromethylsulfonyl;
   or
- R²: is thiophene, furane, pyrazole, thiazole, oxazole or isoxazole each of which is optionally substituted by one to three substituents independently selected from the group consisting of of
fluorine, chlorine, bromine, iodine, -CN, -NO₂, methyl, cyclopropyl, difluoromethyl, trifluoromethyl, methoxy, difluoromethoxy, trifluoromethoxy, methylthio, methylsulfinyl, methylsulfonyl, difluoromethylthio, difluoromethylsulfinyl, difluoromethylsulfonyl, trifluoromethylthio, trifluoromethylsulfinyl, trifluoromethylsulfonyl.

In a further preferred embodiment (embodiment 2c-1), the invention relates to compounds of the formula (I) 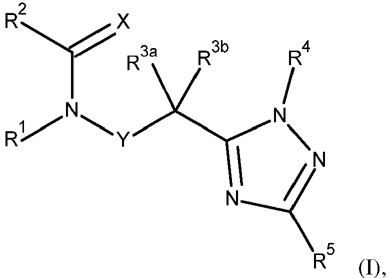 in which the structural elements X, Y, R¹, R^{3a}, R^{3b}, R⁴ and R⁵ have the meanings given Configuration (5-2)
and
- R²: is 3-chloro-5-(1-cyano-1-methyl-ethyl)phenyl, 3-methylsulfonyl-5-(trifluoromethyl)phenyl, 3-methylsulfonyl-5-(trifluoromethoxy)phenyl, 3-bromo-5-[1-(trifluoromethyl)cyclopropyl]benzamide, 3-chloro-5-(methylsulfonyl)phenyl, 3-cyclopropyl-5-(trifluoromethoxy)phenyl, or 1-methyl-5-(trifluoromethyl)-pyrazol-3-yl.

In a further preferred embodiment (embodiment 2c-2), the invention relates to compounds of the formula (I) 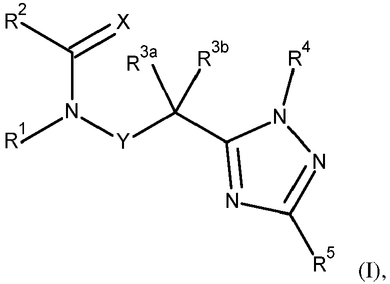 in which the structural elements X, Y, R¹, R^{3a}, R^{3b}, R⁴ and R⁵ have the meanings given in Configuration (5-2)
and
- R²: is 3-chloro-5-(1-cyano-1-methyl-ethyl)phenyl, 3-methylsulfonyl-5-(trifluoromethyl)phenyl, 3-ethylsulfonyl-5-(trifluoromethyl)phenyl, 3-methylsulfonyl-5-(trifluoromethoxy)phenyl, 3-ethylsulfonyl-5-(trifluoromethoxy)phenyl, 3-isopropylsulfonyl-5-(trifluoromethoxy)phenyl, 3-(difluoromethylsulfonyl)-5-(trifluoromethoxy)phenyl, 3-(4-chlorophenyl)sulfonyl-5-(trifluoromethoxy)phenyl, 3-bromo-5-[1-(trifluoromethyl)cyclopropyl]phenyl, 3-chloro-5-(methylsulfonyl)phenyl, 3-chloro-5-(difluoromethylsulfonyl)phenyl, 3-chloro-5-(trifluoromethylsulfonyl)phenyl, 3-cyclopropyl-5-(trifluoromethoxy)phenyl, 3-cyclopropyl-5-(trifluoromethylsulfonyl)phenyl, 3-cyclopropylsulfonyl-5-(trifluoromethoxy)phenyl or 1-methyl-5-(trifluoromethyl)-pyrazol-3-yl.

In a further preferred embodiment (embodiment 4b), the invention relates to compounds of the formula (I) 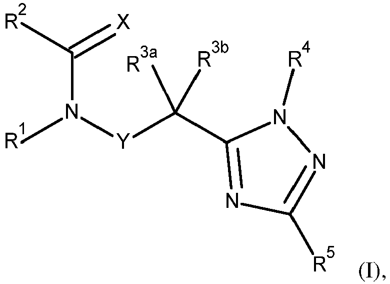 in which the structural elements X, Y, R¹, R^{3a}, R^{3b}, R⁴ and R⁵ have the meanings given in Configuration (5-2)
and in which R² is the following substructure Q1, in which the bond to the C=O-group is marked with a #: 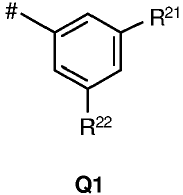 and wherein
- R²¹: is methylsulfonyl, ethylsulfonyl, cyclopropylsulfonyl, isopropylsulfonyl, trifluoromethylsulfonyl, difluoromethylsulfonyl, cyclopropyl, cyanocyclopropyl, trifluoromethyl-cyclopropyl, 1-cyano-1-methyl-ethyl, or phenylsulfonyl which may optionally carry a fluorine or chlorine substituent;
- R²²: is fluorine, chlorine, bromine, -CN, methyl, ethyl, n-propyl, isopropyl, trifluoromethyl, methoxy, ethoxy, difluoromethoxy, trifluoromethoxy, methylsulfonyl, ethylsulfonyl, cyclopropylsulfonyl, trifluoromethylsulfonyl, or cyclopropyl.

In a further preferred embodiment (embodiment 4c), the invention relates to compounds of the formula (I) 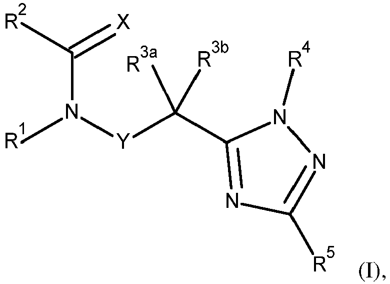 in which the structural elements X, Y, R¹, R^{3a}, R^{3b}, R⁴ and R⁵ have the meanings given in Configuration (5-2)
and in which R² is the following substructure Q1, in which the bond to the C=O-group is marked with a #: 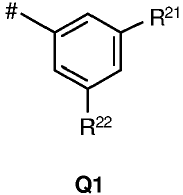 and wherein
- R²¹: is methylsulfonyl, ethylsulfonyl, cyclopropylsulfonyl, isopropylsulfonyl, trifluoromethylsulfonyl, difluoromethylsulfonyl, cyclopropyl, 1-(trifluoromethyl)-cycloprop-1-yl, 1-cyano-1-methyl-ethyl, or 4-chlorophenylsulfonyl; and
- R²²: is chlorine, bromine, cyclopropyl, trifluoromethyl, or trifluoromethoxy.

In accordance with a further aspect, the present invention covers intermediate compounds which are useful for the preparation of the compounds of general formula (I), *supra.*

Particularly, the invention covers the intermediate compounds of general formula **(a),** wherein R¹ is hydrogen, R^{3b} is methyl, R^{3a} is hydrogen and Y is a direct bond and wherein R⁴ and R⁵ have the meanings given in Configuration (5-2):

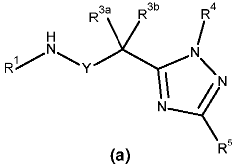

Particularly, the invention covers the intermediate compounds (INT-001 to INT-038) (see table 2):
INT-001: *tert*-butyl *N*-[5-(-1-aminoethyl)-1-pyrimidin-2-yl-1,2,4-triazol-3-yl]carbamate; R⁴ = pyrimidin-2-yl and R⁵ = NHCO(O*tert*-Bu),
INT-002: *tert*-butyl *N*-[5-(-1-aminoethyl)-1-(5-chloro-2-pyridyl)-1,2,4-triazol-3-yl]carbamate; R⁴ = 5-chloropyridin-2-yl and R⁵ = NHCO(O*tert*-Bu),
INT-003: *tert*-butyl *N*-[5-(-1-aminoethyl)-1-pyrimidin-2-yl-1,2,4-triazol-3-yl]-*N*-methyl-carbamate; R⁴ = pyrimidin-2-yl and R⁵ = NCH₃CO(O*tert*-Bu),
INT-004: *N*-[5-(1-aminoethyl)-1-pyrimidin-2-yl-1,2,4-triazol-3-yl]-1,1-difluoro-methanesulfon-amide; R⁴ = pyrimidin-2-yl and R⁵ = NHSO₂CHF₂,
INT-005: 1-[2-(5-chloro-2-pyridyl)-5-(methylsulfonylmethyl)-1,2,4-triazol-3-yl]ethanamine; R⁴ = 5-chloropyridin-2-yl and R⁵ = CH₂SO₂CH₃,
INT-006: 1-[2-(5-chloro-2-pyridyl)-5-(6-chloro-3-pyridyl)-1,2,4-triazol-3-yl]ethanamine; R⁴ = 5-chloropyridin-2-yl and R⁵ = 6-chloropyridin-3-yl,
INT-007: 1-[2-(5-chloro-2-pyridyl)-5-pyrazin-2-yl-1,2,4-triazol-3-yl]ethanamine; R⁴ = 5-chloropyridin-2-yl and R⁵ = pyrazin-2-yl,
INT-008: 1-[5-(6-chloro-3-pyridyl)-2-pyrimidin-2-yl-1,2,4-triazol-3-yl]ethanamine; R⁴ = pyrimidin-2-yl and R⁵ = 6-chloropyridin-3-yl,
INT-009: 1-(5-pyrazin-2-yl-2-pyrimidin-2-yl-1,2,4-triazol-3-yl)ethanamine; R⁴ = pyrimidin-2-yl and R⁵ = pyrazin-2-yl,
INT-010: 1-[2,5-di(pyrimidin-2-yl)-1,2,4-triazol-3-yl]ethanamine; R⁴ = pyrimidin-2-yl and R⁵ = pyrimidin-2-yl,
INT-011: 1-[5-(2-furyl)-2-pyrimidin-2-yl-1,2,4-triazol-3-yl]ethanamine; R⁴ = pyrimidin-2-yl and R⁵ = 2-furyl,
INT-012: 1-[2-(5-chloro-2-pyridyl)-5-methylsulfanyl-1,2,4-triazol-3-yl]ethanamine; R⁴ = 5-chloropyridin-2-yl and R⁵ = methylsulfanyl (or methylthio),
INT-013: 1-[2-(5-chloro-2-pyridyl)-5-(2,2,2-trifluoroethylsulfanyl)-1,2,4-triazol-3-yl]ethanamine; R⁴ = 5-chloropyridin-2-yl and R⁵ = 2,2,2-trifluoroethylsulfanyl (or 2,2,2-trifluoroethylthio),
INT-014: 1-[2-(5-chloro-2-pyridyl)-5-[1,2,2,2-tetrafluoro-1-(trifluoromethyl)ethyl]sulfanyl-1,2,4-triazol-3-yl]ethanamine; R⁴ = 5-chloropyridin-2-yl and R⁵ = 1,2,2,2-tetrafluoro-1-(trifluoromethyl)ethyl] sulfanyl (or heptafluoro-iso-propylthio),
INT-015: 1-(5-benzyl-2-pyrimidin-2-yl-1,2,4-triazol-3-yl)ethanamine; R⁴ = pyrimidin-2-yl and R⁵ = benzyl.
INT-016: 1-[5-[(2,6-difluorophenyl)methyl]-2-pyrimidin-2-yl-1,2,4-triazol-3-yl]ethanamine; R⁴ = pyrimidin-2-yl and R⁵ = 2,6-difluorobenzyl.
INT-017: 1-[5-[(3,5-difluorophenyl)methyl]-2-pyrimidin-2-yl-1,2,4-triazol-3-yl]ethanamine; R⁴ = pyrimidin-2-yl and R³ = 3,5-difluorobenzyl.
INT-018: *tert*-butyl *N*-[5-(1-aminoethyl)-1-[5-(morpholine-4-carbonyl)-2-pyridyl]-1,2,4-triazol-3-yl]carbamate; R⁴ = (3-pyridin-6-yl)-morpholino-methanoneand R⁵ = NHCO(O*tert*-Bu)*.*
INT-019: *tert*-butyl N-[5-(1-aminoethyl)-1-(5-cyano-2-pyridyl)-1,2,4-triazol-3-yl]carbamate; R⁴ = 5-cyano-2-pyridyl and R⁵ = NHCO(O*tert*-Bu)*.*
INT-020: [2-[5-[(1*S*)-1-aminoethyl]-3-(methylamino)-1,2,4-triazol-1-yl]thiazol-5-yl]-morpholino-methanone; R⁴ = (5-thiazol-2-yl)-morpholino-methanone and R⁵ = NHCH₃.
INT-021: 5-[(1*S*)-1-aminoethyl]-*N*-methyl-1-pyrimidin-2-yl-1,2,4-triazol-3-amine-hydrochlorid; R⁴ = pyrimidin-2-yl and R⁵ = NHCH₃.
INT-022: 5-[(1S)-1-aminoethyl]-N,N-dimethyl-1-pyrimidin-2-yl-1,2,4-triazol-3-amine; R⁴ = pyrimidin-2-yl and R⁵ = N(CH₃)₂.
INT-023: (1*S*)-1-(5-morpholino-2-pyrimidin-2-yl-1,2,4-triazol-3-yl)ethanamine-hydrochlorid; R⁴ = pyrimidin-2-yl and R⁵ = *N*-morpholino.
INT-024: 6-[5-[(1*S*)-1-aminoethyl]-3-(methylamino)-1,2,4-triazol-1-yl]pyridine-3-carbonitrile; R⁴ = 5-cyano-pyrimidin-2-yl and R⁵ = NHCH₃.INT-025: 6-[3-amino-5-[(1*S*)-1-aminoethyl]-1,2,4-triazol-1-yl]pyridine-3-carbonitrile-hydrochlorid; R⁴ = 5-cyano-pyrimidin-2-yl and R⁵ = NH₂.
INT-026: 5-[(1*S*)-1-aminoethyl]-N-methoxy-N-methyl-1-pyrimidin-2-yl-1,2,4-triazol-3-amine-hydrochloride; R⁴ = pyrimidin-2-yl and R⁵ = N(CH₃)-OCH₃.
INT-027: 6-[5-[(1*S*)-1-aminoethyl]-3-morpholino-1,2,4-triazol-1-yl]pyridine-3-carbonitrile-hydrochloride; R⁴ = 5-cyano-pyrimidin-2-yl and R⁵ = *N*-morpholino.
INT-028: 5-[(1*S*)-1-aminoethyl]-1-(5-chloropyrimidin-2-yl)-N,N-dimethyl-1,2,4-triazol-3-amine-hydrochloride; R⁴ = 5-chloropyrimidin-2-yl and R⁵ = N(CH₃)₂.
INT-029: 5-[(1*S*)-1-aminoethyl]-1-(5-chloropyrimidin-2-yl)-N-methyl-1,2,4-triazol-3-amine-hydrochloride; R⁴ = 5-chloropyrimidin-2-yl and R⁵ = NHCH₃.
INT-030: 5-[(1*S*)-1-aminoethyl]-1-pyrimidin-2-yl-1,2,4-triazol-3-amine-hydrochloride; R⁴ = 5-chloropyrimidin-2-yl and R⁵ = NH₂.
INT-031: (1*S*)-1-(2-pyrimidin-2-yl-5-pyrrolidin-1-yl-1,2,4-triazol-3-yl)ethanamine-hydrochloride; R⁴ = pyrimidin-2-yl and R⁵ = *N*-pyrrolidinyl.
INT-032: (1*S*)-1-[5-(1-piperidyl)-2-pyrimidin-2-yl-1,2,4-triazol-3-yl]ethanamine-hydrochloride; R⁴ = pyrimidin-2-yl and R⁵ = *N*-piperidinyl.
INT-033: 2-[5-[(1*S*)-1-aminoethyl]-3-morpholino-1,2,4-triazol-1-yl]thiazole-5-carbonitrile-hydrochloride; R⁴ = 5-cyano-thiazol-2-yl and R⁵ = N-morpholino.
INT-034: 2-[5-[(1S)-1-aminoethyl]-3-morpholino-1,2,4-triazol-1-yl]thiazole-5-carbonitrile-hydrochloride; R⁴ = 5-cyano-thiazol-2-yl and R⁵ = NHCH₃.
INT-035: 2-[5-[(1*S*)-1-aminoethyl]-3-morpholino-1,2,4-triazol-1-yl]thiazole-5-carbonitrile; R⁴ = 5-cyano-thiazol-2-yl and R⁵ = NH₂.
INT-036: methyl 6-[5-[(1S)-1-aminoethyl]-3-(dimethylamino)-1,2,4-triazolo-1-yl]pyridine-3-carboxylate-hydrochloride; R⁴ = 5-methoxycarbonyl-pyridin-2-yl and R⁵ = N(CH₃)₂.
INT-037: N-[6-[5-[(1S)-1-aminoethyl]-3-(dimethylamino)-1,2,4-triazol-1-yl]-3-pyridyl]-N-methyl-cyclopropanecarboxamide-hydrochoride; R⁴ = 5-[cyclopropanecarbonyl(methyl)amino]- pyridin-2-yl and R⁵ = N(CH₃)₂.
INT-038: 5-[(1*S*)-1-aminoethyl]-1-(5-chloro-2-pyridyl)-1,2,4-triazol-3-amine-hydrochlorid; R⁴ = 5-chloro-pyridin-2-yl and R⁵ = NH₂.

Particularly, the invention covers the intermediate compounds of general formula **(b-1)** 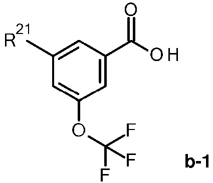 wherein
- R²¹: is difluoromethylsulfanyl, difluormethylsulfinyl, or difluoromethylsulfonyl.

Particularly, the invention covers the intermediate compound (INT-039) (see table 2):
INT-039: 3-(difluoromethylsulfonyl)-5-(trifluromethoxy)benzoic acid.

The compounds of the formula (I) may possibly also, depending on the nature of the substituents, be in the form of stereoisomers, i.e. in the form of geometric and/or optical isomers or isomer mixtures of varying composition. This invention provides both the pure stereoisomers and any desired mixtures of these isomers, even though it is generally only compounds of the formula (I) that are discussed here.

However, preference is given in accordance with the invention to using the optically active, stereoisomeric forms of the compounds of the formula (I) and salts thereof.

The invention therefore relates both to the pure enantiomers and diastereomers and to mixtures thereof for controlling animal pests, including arthropods and particularly insects.

If appropriate, the compounds of the formula (I) may be present in various polymorphic forms or as a mixture of various polymorphic forms. Both the pure polymorphs and the polymorph mixtures are provided by the invention and can be used in accordance with the invention.

### Definitions

The person skilled in the art is aware that, if not stated explicitly, the expressions "a" or "an" as used in the present application may, depending on the situation, mean "one (1)", "one (1) or more" or "at least one (1)".

For all the structures described herein, such as ring systems and groups, adjacent atoms must not be -O-O- or -O-S-.

Structures having a variable number of possible carbon atoms (C atoms) may be referred to in the present application as C_{lower limit of carbon atoms}-C_{upper limit of carbon atoms} structures (C_{LL}-C_{UL} structures), in order thus to be stipulated more specifically. Example: an alkyl group may consist of 3 to 10 carbon atoms and in that case corresponds to C₃-C₁₀alkyl. Ring structures composed of carbon atoms and heteroatoms may be referred to as "LL- to UL-membered" structures. One example of a 6-membered ring structure is toluene (a 6-membered ring structure substituted by a methyl group).

If a collective term for a substituent, for example C_{LL}-C_{UL}alkyl, is at the end of a composite substituent, for example C_{LL}-C_{UL}cycloalkyl-C_{LL}-C_{UL}alkyl, the constituent at the start of the composite substituent, for example the C_{LL}-C_{UL}cycloalkyl, may be mono- or polysubstituted identically or differently and independently by the latter substituent, for example C_{LL}-C_{UL}alkyl. All the collective terms used in this application for chemical groups, cyclic systems and cyclic groups can be stipulated more specifically through the addition "C_{LL}-C_{UL}" or "LL- to UL-membered".

In the definitions of the symbols given in the above formulae, collective terms which are generally representative of the following substituents were used:
Halogen relates to elements of the 7th main group, preferably fluorine, chlorine, bromine and iodine, more preferably fluorine, chlorine and bromine, and even more preferably fluorine and chlorine.

Examples of heteroatom are N, O, S, P, B, Si. Preferably, the term "heteroatom" relates to N, S and O.

According to the invention, "alkyl" - on its own or as part of a chemical group - represents straight-chain or branched hydrocarbons preferably having 1 to 6 carbon atoms, for example methyl, ethyl, n-propyl, isopropyl, n-butyl, isobutyl, s-butyl, t-butyl, pentyl, 1-methylbutyl, 2-methylbutyl, 3-methylbutyl, 1,2-dimethylpropyl, 1,1-dimethylpropyl, 2,2-dimethylpropyl, 1-ethylpropyl, hexyl, 1-methylpentyl, 2-methylpentyl, 3-methylpentyl, 4-methylpentyl, 1,2-dimethylpropyl, 1,3-dimethylbutyl, 1,4-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl and 2-ethylbutyl. Preference is also given to alkyls having 1 to 4 carbon atoms such as, inter alia, methyl, ethyl, ethyl, n-propyl, isopropyl, n-butyl, isobutyl, s-butyl or t-butyl. The inventive alkyls may be substituted by one or more identical or different radicals.

According to the invention, "alkenyl" - on its own or as part of a chemical group - represents straight-chain or branched hydrocarbons preferably having 2 to 6 carbon atoms and at least one double bond, for example vinyl, 2-propenyl, 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl, 3-hexenyl, 4-hexenyl, 5-hexenyl, 1-methyl-2-pentenyl, 2-methyl-2-pentenyl, 3-methyl-2-pentenyl, 4-methyl-2-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 2,2-dimethyl-3-butenyl, 2,3-dimethyl-2-butenyl, 2,3-dimethyl-3-butenyl, 1-ethyl-2-butenyl, 1-ethyl-3-butenyl, 2-ethyl-2-butenyl, 2-ethyl-3-butenyl, 1,1,2-trimethyl-2-propenyl, 1-ethyl-1-methyl-2-propenyl and 1-ethyl-2-methyl-2-propenyl. Preference is also given to alkenyls having 2 to 4 carbon atoms such as, inter alia, 2-propenyl, 2-butenyl or 1-methyl-2-propenyl. The inventive alkenyls may be substituted by one or more identical or different radicals.

According to the invention, "alkynyl" - on its own or as part of a chemical group - represents straight-chain or branched hydrocarbons preferably having 2 to 6 carbon atoms and at least one triple bond, for example 2-propynyl, 2-butynyl, 3-butynyl, 1-methyl-2-propynyl, 2-pentynyl, 3-pentynyl, 4-pentynyl, 1-methyl-3-butynyl, 2-methyl-3-butynyl, 1-methyl-2-butynyl, 1,1-dimethyl-2-propynyl, 1-ethyl-2-propynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-4-pentynyl, 4-methyl-2-pentynyl, 1,1-dimethyl-3-butynyl, 1,2-dimethyl-3-butynyl, 2,2-dimethyl-3-butynyl, 1-ethyl-3-butynyl, 2-ethyl-3-butynyl, 1-ethyl-1-methyl-2-propynyl and 2,5-hexadiynyl. Preference is also given to alkynyls having 2 to 4 carbon atoms such as, inter alia, ethynyl, 2-propynyl or 2-butynyl-2-propenyl. The inventive alkynyls may be substituted by one or more identical or different radicals.

According to the invention, "cycloalkyl" - on its own or as part of a chemical group - represents mono-, bi- or tricyclic hydrocarbons preferably having 3 to 10 carbons, for example cyclopropyl, cyclobutyl, cyclopentyl, cyclohexyl, cycloheptyl, cyclooctyl, bicyclo[2.2.1]heptyl, bicyclo[2.2.2]octyl or adamantyl. Preference is also given to cycloalkyls having 3, 4, 5, 6 or 7 carbon atoms such as, inter alia, cyclopropyl or cyclobutyl. The inventive cycloalkyls may be substituted by one or more identical or different radicals.

According to the invention, "alkylcycloalkyl" represents mono-, bi- or tricyclic alkylcycloalkyl preferably having 4 to 10 or 4 to 7 carbon atoms, for example methylcyclopropyl, ethylcyclopropyl, isopropylcyclobutyl, 3-methylcyclopentyl and 4-methylcyclohexyl. Preference is also given to alkylcycloalkyls having 4, 5 or 7 carbon atoms such as, inter alia, ethylcyclopropyl or 4-methylcyclohexyl. The inventive alkylcycloalkyls may be substituted by one or more identical or different radicals.

According to the invention, "cycloalkylalkyl" represents mono-, bi- or tricyclic cycloalkylalkyl preferably having 4 to 10 or 4 to 7 carbon atoms, for example cyclopropylmethyl, cyclobutylmethyl, cyclopentylmethyl, cyclohexylmethyl and cyclopentylethyl. Preference is also given to cycloalkylalkyls having 4, 5 or 7 carbon atoms such as, inter alia, cyclopropylmethyl or cyclobutylmethyl. The inventive cycloalkylalkyls may be substituted by one or more identical or different radicals.

According to the invention, "hydroxyalkyl" represents a straight-chain or branched alcohol preferably having 1 to 6 carbon atoms, for example methanol, ethanol, n-propanol, isopropanol, n-butanol, isobutanol, s-butanol and t-butanol. Preference is also given to hydroxyalkyl groups having 1 to 4 carbon atoms. The inventive hydroxyalkyl groups may be substituted by one or more identical or different radicals.

According to the invention, "alkoxy" represents a straight-chain or branched O-alkyl preferably having 1 to 6 carbon atoms, for example methoxy, ethoxy, n-propoxy, isopropoxy, n-butoxy, isobutoxy, s-butoxy and t-butoxy. Preference is also given to alkoxy groups having 1 to 4 carbon atoms. The inventive alkoxy groups may be substituted by one or more identical or different radicals.

According to the invention, "alkylthio", or "alkylsulfanyl" represents straight-chain or branched S-alkyl preferably having 1 to 6 carbon atoms, for example methylthio, ethylthio, n-propylthio, isopropylthio, n-butylthio, isobutylthio, s-butylthio and t-butylthio. Preference is also given to alkylthio groups having 1 to 4 carbon atoms. The inventive alkylthio groups may be substituted by one or more identical or different radicals.

According to the invention, "alkylsulfinyl" represents straight-chain or branched alkylsulfinyl preferably having 1 to 6 carbon atoms, for example methylsulfinyl, ethylsulfinyl, n-propylsulfinyl, isopropylsulfinyl, n-butylsulfinyl, isobutylsulfinyl, s-butylsulfinyl and t-butylsulfinyl. Preference is also given to alkylsulfinyl groups having 1 to 4 carbon atoms. The inventive alkylsulfinyl groups may be substituted by one or more identical or different radicals and embrace both enantiomers.

According to the invention, "alkylsulfonyl" represents straight-chain or branched alkylsulfonyl preferably having 1 to 6 carbon atoms, for example methylsulfonyl, ethylsulfonyl, n-propylsulfonyl, isopropylsulfonyl, n-butylsulfonyl, isobutylsulfonyl, s-butylsulfonyl and t-butylsulfonyl. Preference is also given to alkylsulfonyl groups having 1 to 4 carbon atoms. The inventive alkylsulfonyl groups may be substituted by one or more identical or different radicals.

According to the invention, "cycloalkylthio" or "cycloalkylsulfanyl" represents -S-cycloalkyl preferably having 3 to 6 carbon atoms, for example cyclopropylthio, cyclobutylthio, cyclopentylthio, cyclohexylthio. Preference is also given to cycloalkylthio groups having 3 to 5 carbon atoms. The inventive cycloalkylthio groups may be substituted by one or more identical or different radicals.

According to the invention, "cycloalkylsulfinyl" represents -S(O)-cycloalkyl preferably having 3 to 6 carbon atoms, for example cyclopropylsulfinyl, cyclobutylsulfinyl, cyclopentylsulfinyl, cyclohexylsulfinyl. Preference is also given to cycloalkylsulfinyl groups having 3 to 5 carbon atoms. The inventive cycloalkylsulfinyl groups may be substituted by one or more identical or different radicals and embrace both enantiomers.

According to the invention, "cycloalkylsulfonyl" represents -SO₂-cycloalkyl preferably having 3 to 6 carbon atoms, for example cyclopropylsulfonyl, cyclobutylsulfonyl, cyclopentylsulfonyl, cyclohexylsulfonyl. Preference is also given to cycloalkylsulfonyl groups having 3 to 5 carbon atoms. The inventive cycloalkylsulfonyl groups may be substituted by one or more identical or different radicals.

According to the invention, "phenylthio", or "phenylsulfanyl" represents -S-phenyl, for example phenylthio. The inventive phenylthio groups may be substituted by one or more identical or different radicals.

According to the invention, "phenylsulfinyl" represents -S(O)-phenyl, for example phenylsulfinyl. The inventive phenylsulfinyl groups may be substituted by one or more identical or different radicals and embrace both enantiomers.

According to the invention, "phenylsulfonyl" represents -SO₂-phenyl for example phenylsulfonyl. The inventive phenylsulfonyl groups may be substituted by one or more identical or different radicals.

According to the invention, "alkylcarbonyl" represents straight-chain or branched alkyl-C(=O) preferably having 2 to 7 carbon atoms such as methylcarbonyl, ethylcarbonyl, n-propylcarbonyl, isopropylcarbonyl, s-butylcarbonyl and t-butylcarbonyl. Preference is also given to alkylcarbonyls having 1 to 4 carbon atoms. The inventive alkylcarbonyls may be substituted by one or more identical or different radicals.

According to the invention, "alkoxycarbonyl" - alone or as a constituent of a chemical group - represents straight-chain or branched alkoxycarbonyl, preferably having 1 to 6 carbon atoms or having 1 to 4 carbon atoms in the alkoxy moiety, for example methoxycarbonyl, ethoxycarbonyl, n-propoxycarbonyl, isopropoxycarbonyl, s-butoxycarbonyl and t-butoxycarbonyl. The inventive alkoxycarbonyl groups may be substituted by one or more identical or different radicals.

According to the invention, "alkylaminocarbonyl" represents straight-chain or branched alkylaminocarbonyl having preferably 1 to 6 carbon atoms or 1 to 4 carbon atoms in the alkyl moiety, for example methylaminocarbonyl, ethylaminocarbonyl, n-propylaminocarbonyl, isopropylaminocarbonyl, s-butylaminocarbonyl and t-butylaminocarbonyl. The inventive alkylaminocarbonyl groups may be substituted by one or more identical or different radicals.

According to the invention, "*N,N*-dialkylaminocarbonyl" represents straight-chain or branched *N,N-*dialkylaminocarbonyl having preferably 1 to 6 carbon atoms or 1 to 4 carbon atoms in the alkyl moiety, for example *N,N*-dimethylaminocarbonyl, *N,N*-diethylaminocarbonyl, *N,N*-di(n-propylamino)carbonyl, *N,N*-di(isopropylamino)carbonyl and *N,N*-di-(s-butylamino)carbonyl. The inventive *N,N-*dialkylaminocarbonyl groups may be substituted by one or more identical or different radicals.

According to the invention, "aryl" represents a mono-, bi- or polycyclic aromatic system having preferably 6 to 14, especially 6 to 10, ring carbon atoms, for example phenyl, naphthyl, anthryl, phenanthrenyl, preferably phenyl. In addition, aryl also represents fused polycyclic systems such as tetrahydronaphthyl, indenyl, indanyl, fluorenyl, biphenyl, where the bonding site is on the aromatic system. The inventive aryl groups may be substituted by one or more identical or different radicals.

Examples of substituted aryls are the arylalkyls, which may likewise be substituted by one or more identical or different radicals in the C₁-C₄alkyl and/or C₆-C₁₄aryl moiety. Examples of such arylalkyls include benzyl and phenyl-1-ethyl.

According to the invention the term "polycyclic" ring refers to fused, bridged and spirocyclic carbocyclic and heterocyclic rings as well as ring systems linked through single or double bonds.

According to the invention, "heterocycle", "heterocyclic ring" or "heterocyclic ring system" represents a carbocyclic ring system having at least one ring in which at least one carbon atom is replaced by a heteroatom, preferably by a heteroatom from the group consisting of N, O, S, P, B, Si, Se, and which is saturated, unsaturated or heteroaromatic and may be unsubstituted or substituted, where the bonding site is on a ring atom. Unless defined differently, the heterocyclic ring contains preferably 3 to 9 ring atoms, especially 3 to 6 ring atoms, and one or more, preferably 1 to 4, especially 1, 2 or 3, heteroatoms in the heterocyclic ring, preferably from the group consisting of N, O, and S, although no two oxygen atoms should be directly adjacent. The heterocyclic rings usually contain not more than 4 nitrogen atoms and/or not more than 2 oxygen atoms and/or not more than 2 sulphur atoms. In the case of optionally substituted heterocyclyl, the invention also embraces polycyclic ring systems, for example 8-azabicyclo[3.2.1]octanyl, 1-azabicyclo[2.2.1]heptyl, 1-oxa-5-azaspiro[2.3]hexyl or 2,3-dihydro-1*H-*indole.

Inventive heterocyclyl groups are, for example, piperidinyl, piperazinyl, morpholinyl, thiomorpholinyl, dihydropyranyl, tetrahydropyranyl, dioxanyl, pyrrolinyl, pyrrolidinyl, imidazolinyl, imidazolidinyl, thiazolidinyl, oxazolidinyl, dioxolanyl, dioxolyl, pyrazolidinyl, tetrahydrofuranyl, dihydrofuranyl, oxetanyl, oxiranyl, azetidinyl, aziridinyl, oxazetidinyl, oxaziridinyl, oxazepanyl, oxazinanyl, azepanyl, oxopyrrolidinyl, dioxopyrrolidinyl, oxomorpholinyl, oxopiperazinyl and oxepanyl.

Of particular significance are heteroaryls, i.e. heteroaromatic systems. According to the invention, the term heteroaryl represents heteroaromatic compounds, i.e. completely unsaturated aromatic heterocyclic compounds which fall under the above definition of heterocycles. Preference is given to 5- to 7-membered rings having 1 to 3, preferably 1 or 2, identical or different heteroatoms from the group above. Inventive heteroaryls are, for example, furyl, thienyl, pyrazolyl, imidazolyl, 1,2,3- and 1,2,4-triazolyl, isoxazolyl, thiazolyl, isothiazolyl, 1,2,3-, 1,3,4-, 1,2,4- and 1,2,5-oxadiazolyl, azepinyl, pyrrolyl, pyridyl, pyridazinyl, pyrimidinyl, pyrazinyl, 1,3,5-, 1,2,4- and 1,2,3-triazinyl, 1,2,4-, 1,3,2-, 1,3,6- and 1,2,6-oxazinyl, oxepinyl, thiepinyl, 1,2,4-triazolonyl and 1,2,4-diazepinyl. The inventive heteroaryl groups may also be substituted by one or more identical or different radicals.

According to the invention, the substituent =O (oxo) can replace two hydrogen atoms of a methylene (CH₂) group or the lone pairs of a sulfur, nitrogen and phosphorous atom which bears only substituents other than hydrogen. For example the radical C₂-alkyl becomes for example -COCH₃ through susbstiution by =O (oxo) while the heterocycle thietan-3-yl- becomes for example 1-oxothietan-3-yl through substitution by one =O (oxo) group or 1,1-dioxothietan-3-yl through substitution by two =O (oxo) groups.

According to the invention, the substituent =S (thiono) can replace two hydrogen atoms of a methylene (CH₂) group. For example the radical C₂-alkyl becomes for examples -CSCH₃ through susbstiution by =S (thiono).

The term "in each case optionally substituted" means that a group/substituent , such as a alkyl, alkenyl, alkynyl, alkoxy, alkylthio, alkylsulfinyl, alkylsulfonyl, cycloalkyl, aryl, phenyl, benzyl, heterocyclyl and heteroaryl radical, is substituted, meaning, for example, a substituted radical derived from the unsubstituted base structure, where the substituents, for example, one (1) substituent or a plurality of substituents, preferably 1, 2, 3, 4, 5, 6 or 7, are selected from a group consisting of amino, hydroxyl, halogen, nitro, cyano, isocyano, mercapto, isothiocyanato, C₁-C₄carboxyl, carbonamide, SF₅, aminosulphonyl, C₁-C₄alkyl, C₁-C₄haloalkyl, C₃-C₄cycloalkyl, C₂-C₄alkenyl, C₅-C₆cycloalkenyl, C₂-C₄alkynyl, *N*-mono-C₁-C₄alkylamino, *N,N*-di-C₁-C₄alkylamino, *N*-C₁-C₄alkanoylamino, C₁-C₄alkoxy, C₁-C₄haloalkoxy, C₂-C₄alkenyloxy, C₂-C₄alkynyloxy, C₃-C₄cycloalkoxy, C₅-C₆cycloalkenyloxy, C₁-C₄alkoxycarbonyl, C₂-C₄alkenyloxycarbonyl, C₂-C₄alkynyloxycarbonyl, C₆-,C₁₀-,C₁₄-aryloxycarbonyl, C₁-C₄alkanoyl, C₂-C₄alkenylcarbonyl, C₂-C₄alkynylcarbonyl, C₆-,C₁₀-,C₁₄-arylcarbonyl, C₁-C₄alkylthio, C₁-C₄haloalkylthio, C₃-C₄cycloalkylthio, C₂-C₄alkenylthio, C₅-C₆cycloalkenylthio, C₂-C₄alkynylthio, C₁-C₄alkylsulfinyl, including both enantiomers of the C₁-C₄alkylsulfinyl group, C₁-C₄haloalkylsulfinyl, including both enantiomers of the C₁-C₄haloalkylsulfinyl group, C₁-C₄alkylsulfonyl, C₁-C₄haloalkylsulfonyl, *N*-mono-C₁-C₄alkylaminosulfonyl, *N,N-*di-C₁-C₄alkylaminosulfonyl, C₁-C₄alkylphosphinyl, C₁-C₄alkylphosphonyl, including both enantiomers of C₁-C₄alkylphosphinyl and C₁-C₄alkylphosphonyl, *N*-C₁-C₄alkylaminocarbonyl, *N,N*-di-C₁-C₄alkylaminocarbonyl, N-C₁-C₄alkanoylaminocarbonyl, N-C₁-C₄alkanoyl-N-C₁-C₄alkylaminocarbonyl, C₆-,C₁₀-,C₁₄-aryl, C₆-,C₁₀-,C₁₄-aryloxy, benzyl, benzyloxy, benzylthio, C₆-,C₁₀-,C₁₄-arylthio, C₆-,C₁₀-,C₁₄-arylamino, benzylamino, heterocyclyl and trialkylsilyl, substituents bonded via a double bond, such as C₁-C₄alkylidene (e.g. methylidene or ethylidene), an oxo group, an imino group and a substituted imino group. When two or more radicals form one or more rings, these may be carbocyclic, heterocyclic, saturated, partly saturated, unsaturated, for example including aromatic rings and with further substitution. The substituents mentioned by way of example ("first substituent level") may, if they contain hydrocarbonaceous components, optionally have further substitution therein ("second substituent level"), for example by one or more of the substituents each independently selected from halogen, hydroxyl, amino, nitro, cyano, isocyano, azido, acylamino, an oxo group and an imino group. The term "(optionally) substituted" group preferably embraces just one or two substituent levels.

The inventive halogen-substituted chemical groups or halogenated groups (for example alkyl or alkoxy) are mono- or polysubstituted by halogen up to the maximum possible number of substituents. Such groups are also referred to as halo groups (for example haloalkyl). In the case of polysubstitution by halogen, the halogen atoms may be the same or different, and may all be bonded to one carbon atom or may be bonded to a plurality of carbon atoms. Halogen is especially fluorine, chlorine, bromine or iodine, preferably fluorine, chlorine or bromine and more preferably fluorine. More particularly, halogen-substituted groups are monohalocycloalkyl such as 1-fluorocyclopropyl, 2-fluorocyclopropyl or 1-fluorocyclobutyl, monohaloalkyl such as 2-chloroethyl, 2-fluoroethyl, 1-chloroethyl, 1-fluoroethyl, chloromethyl, or fluoromethyl; perhaloalkyl such as trichloromethyl or trifluoromethyl or CF₂CF₃, polyhaloalkyl such as difluoromethyl, 2-fluoro-2-chloroethyl, dichloromethyl, 1,1,2,2-tetrafluoroethyl or 2,2,2-trifluoroethyl. Further examples of haloalkyls are trichloromethyl, chlorodifluoromethyl, dichlorofluoromethyl, chloromethyl, bromomethyl, 1-fluoroethyl, 2-fluoroethyl, 2,2-difluoroethyl, 2,2,2-trifluoroethyl, 2,2,2-trichloroethyl, 2-chloro-2,2-difluoroethyl, pentafluoroethyl, 3,3,3-trifluoropropyl and pentafluoro-t-butyl. Preference is given to haloalkyls having 1 to 4 carbon atoms and 1 to 9, preferably 1 to 5, identical or different halogen atoms selected from fluorine, chlorine and bromine. Particular preference is given to haloalkyls having 1 or 2 carbon atoms and 1 to 5 identical or different halogen atoms selected from fluorine and chlorine, such as, inter alia, difluoromethyl, trifluoromethyl or 2,2-difluoroethyl. Further examples of halogen-substituted compounds are haloalkoxy such as OCF₃, OCHF₂, OCH_{2F}, OCF₂CF₃, OCH₂CF₃, OCH₂CHF₂ und OCH₂CH₂Cl, haloalkylsulfanyls such as difluoromethylthio, trifluoromethylthio, trichloromethylthio, chlorodifluoromethylthio, 1-fluoroethylthio, 2-fluoroethylthio, 2,2-difluoroethylthio, 1,1,2,2-tetrafluoroethylthio, 2,2,2-trifluoroethylthio or 2-chloro-1,1,2-trifluoroethylthio, haloalkylsulfinyls such as difluoromethylsulfinyl, trifluoromethylsulfinyl, trichloromethylsulfinyl, chlorodifluoromethylsulfinyl, 1-fluoroethylsulfinyl, 2-fluoroethylsulfinyl, 2,2-difluoroethylsulfinyl, 1,1,2,2-tetrafluoroethylsulfinyl, 2,2,2-trifluoroethylsulfinyl and 2-chloro-1,1,2-trifluoroethylsulfinyl, haloalkylsulfinyls such as difluoromethylsulfinyl, trifluoromethylsulfinyl, trichloromethylsulfinyl, chlorodifluoromethylsulfinyl, 1-fluoroethylsulfinyl, 2-fluoroethylsulfinyl, 2,2-difluoroethylsulfinyl, 1,1,2,2-tetrafluoroethylsulfinyl, 2,2,2-trifluoroethylsulfinyl and 2-chloro-1,1,2-trifluoroethylsulfinyl, haloalkylsulfonyl groups such as difluoromethylsulfonyl, trifluoromethylsulfonyl, trichloromethylsulfonyl, chlorodifluoromethylsulfonyl, 1-fluoroethylsulfonyl, 2-fluoroethylsulfonyl, 2,2-difluoroethylsulfonyl, 1,1,2,2-tetrafluoroethylsulfonyl, 2,2,2-trifluoroethylsulfonyl and 2-chloro-1,1,2-trifluoroethylsulfonyl.

In the case of radicals having carbon atoms, preference is given to those having 1 to 4 carbon atoms, especially 1 or 2 carbon atoms. Preference is generally given to substituents from the group of halogen, e.g. fluorine and chlorine, (C₁-C₄)alkyl, preferably methyl or ethyl, (C₁-C₄)haloalkyl, preferably trifluoromethyl, (C₁-C₄)alkoxy, preferably methoxy or ethoxy, (C₁-C₄)haloalkoxy, nitro and cyano. Particular preference is given here to the substituents methyl, methoxy, fluorine and chlorine.

Substituted amino such as mono- or disubstituted amino means a radical from the group of the substituted amino radicals which are *N*-substituted, for example, by one or two identical or different radicals from the group of alkyl, hydroxy, amino, alkoxy, acyl and aryl; preferably *N*-mono- and *N,N*-dialkylamino, (for example methylamino, ethylamino, *N,N*-dimethylamino, *N,N*-diethylamino, *N,N*-di-n-propylamino, *N,N-*diisopropylamino or *N,N*-dibutylamino), *N*-mono- or *N,N*-dialkoxyalkylamino groups (for example *N-*methoxymethylamino, *N*-methoxyethylamino, *N,N*-di(methoxymethyl)amino or *N,N-*di(methoxyethyl)amino), *N*-mono- and *N,N*-diarylamino, such as optionally substituted anilines, acylamino, *N,N*-diacylamino, *N*-alkyl-*N*-arylamino, *N*-alkyl-*N*-acylamino and also saturated *N-*heterocycles; preference is given here to alkyl radicals having 1 to 4 carbon atoms; here, aryl is preferably phenyl or substituted phenyl; for acyl, the definition given further below applies, preferably (C₁-C₄)-alkanoyl. The same applies to substituted hydroxylamino or hydrazino.

Substituted amino also includes quaternary ammonium compounds (salts) having four organic substituents on the nitrogen atom.

Optionally substituted phenyl is preferably phenyl which is unsubstituted or mono- or polysubstituted, preferably up to trisubstituted, by identical or different radicals from the group of halogen, (C₁-C₄)alkyl, (C₁-C₄)alkoxy, (C₁-C₄)alkoxy-(C₁-C₄)alkoxy, (C₁-C₄)alkoxy-(C₁-C₄)alkyl, (C₁-C₄)haloalkyl, (C₁-C₄)haloalkoxy, (C₁-C₄)alkylthio, (C₁-C₄)haloalkylthio, (C₁-C₄)alkylsulfinyl (C₁-C₄) haloalkylsulfinyl, (C₁-C₄)alkylsulfonyl (C₁-C₄)haloalkylsulfonyl, cyano, isocyano and nitro, for example o-, m- and p-tolyl, dimethylphenyls, 2-, 3- and 4-chlorophenyl, 2-, 3- and 4-fluorophenyl, 2-, 3- and 4-trifluoromethyl- and 4-trichloromethylphenyl, 2,4-, 3,5-, 2,5- and 2,3-dichlorophenyl, o-, m- and p-methoxyphenyl, 4-heptafluorophenyl.

Optionally substituted cycloalkyl is preferably cycloalkyl which is unsubstituted or mono- or polysubstituted, preferably up to trisubstituted, by identical or different radicals from the group of halogen, cyano, (C₁-C₄)alkyl, (C₁-C₄)alkoxy, (C₁-C₄)alkoxy-(C₁-C₄)alkoxy, (C₁-C₄)alkoxy-(C₁-C₄)alkyl, (C₁-C₄)haloalkyl and (C₁-C₄)haloalkoxy, especially by one or two (C₁-C₄)alkyl radicals.

Inventive compounds may occur in preferred embodiments. Individual embodiments described herein may be combined with one another. Not included are combinations which contravene the laws of nature and which the person skilled in the art would therefore rule out on the basis of his/her expert knowledge. Ring structures having three or more adjacent oxygen atoms, for example, are excluded.

### Isomers

Depending on the nature of the substituents, the compounds of the formula (I) may be in the form of geometric and/or optically active isomers or corresponding isomer mixtures in different compositions. These stereoisomers are, for example, enantiomers, diastereomers, atropisomers or geometric isomers. Accordingly, the invention encompasses both pure stereoisomers and any mixture of these isomers.

### Methods and uses

The invention also relates to methods for controlling animal pests, in which compounds of the formula (I) are allowed to act on animal pests and/or their habitat. The control of the animal pests is preferably conducted in agriculture and forestry, and in material protection. Preferably excluded herefrom are methods for the surgical or therapeutic treatment of the human or animal body and diagnostic methods carried out on the human or animal body.

The invention furthermore relates to the use of the compounds of the formula (I) as pesticides, in particular crop protection agents.

In the context of the present application, the term "pesticide" in each case also always comprises the term "crop protection agent".

The compounds of the formula (I), having good plant tolerance, favourable homeotherm toxicity and good environmental compatibility, are suitable for protecting plants and plant organs against biotic and abiotic stressors, for increasing harvest yields, for improving the quality of the harvested material and for controlling animal pests, especially insects, arachnids, helminths, in particular nematodes, and molluscs, which are encountered in agriculture, in horticulture, in animal husbandry, in aquatic cultures, in forests, in gardens and leisure facilities, in the protection of stored products and of materials, and in the hygiene sector.

Within the context of the present patent application, the term "hygiene" is understood to mean any and all measures, procedures and practices which aim to prevent disease, in particular infectious disease, and which serve to protect the health of humans and animals and/or to protect the environment, and/or which maintain cleanliness. In accordance with the invention, this especially includes measures for cleaning, disinfection and sterilisation of, for example, textiles or hard surfaces, especially surfaces of glass, wood, concrete, porcelain, ceramics, plastic or also of metal(s), and for ensuring that these are kept free of hygiene pests and/or their excretions. Preferably excluded from the scope of the invention in this regard are surgical or therapeutic treatment procedures applicable to the human body or to the bodies of animals and diagnostic procedures which are carried out on the human body or on the bodies of animals.

The term "hygiene sector" thus covers all areas, technical fields and industrial applications in which these hygiene measures, procedures and practices are important, in relation for example to hygiene in kitchens, bakeries, airports, bathrooms, swimming pools, department stores, hotels, hospitals, stables, animal husbandries, etc.

The term "hygiene pest" is therefore understood to mean one or more animal pests whose presence in the hygiene sector is problematic, in particular for health reasons. It is therefore a primary objective to avoid or minimize the presence of hygiene pests, and/or exposure to them, in the hygiene sector. This can be achieved in particular through the application of a pesticide that can be used both to prevent infestation and to tackle an infestation which is already present. Preparations which avoid or reduce exposure to pests can also be used. Hygiene pests include, for example, the organisms mentioned below.

The term "hygiene protection" thus covers all actions to maintain and/or improve these hygiene measures, procedures and practices.

The compounds of the formula (I) can preferably be used as pesticides. They are active against normally sensitive and resistant species and against all or some stages of development. The abovementioned pests include:
pests from the phylum of the Arthropoda, in particular from the class of the Arachnida, for example Acarus spp., for example Acarus siro, Aceria kuko, Aceria sheldoni, Aculops spp., Aculus spp., for example Aculus fockeui, Aculus schlechtendali, Amblyomma spp., Amphitetranychus viennensis, Argas spp., Boophilus spp., Brevipalpus spp., for example Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., for example Eotetranychus hicoriae, Epitrimerus pyri, Eutetranychus spp., for example Eutetranychus banksi, Eriophyes spp., for example Eriophyes pyri, Glycyphagus domesticus, Halotydeus destructor, Hemitarsonemus spp., for example Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., for example Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., for example Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., for example Tarsonemus confusus, Tarsonemus pallidus, Tetranychus spp., for example Tetranychus canadensis, Tetranychus cinnabarinus, Tetranychus turkestani, Tetranychus urticae, Trombicula alfreddugesi, Vaejovis spp., Vasates lycopersici;
from the class of the Chilopoda, for example Geophilus spp., Scutigera spp.;
from the order or the class of the Collembola, for example Onychiurus armatus; Sminthurus viridis;
from the class of the Diplopoda, for example Blaniulus guttulatus;
from the class of the Insecta, for example from the order of the Blattodea, for example Blatta orientalis, Blattella asahinai, Blattella germanica, Leucophaea maderae, Loboptera decipiens, Neostylopyga rhombifolia, Panchlora spp., Parcoblatta spp., Periplaneta spp., for example Periplaneta americana, Periplaneta australasiae, Pycnoscelus surinamensis, Supella longipalpa;
from the order of the Coleoptera, for example Acalymma vittatum, Acanthoscelides obtectus, Adoretus spp., Aethina tumida, Agelastica alni, Agrilus spp., for example Agrilus planipennis, Agrilus coxalis, Agrilus bilineatus, Agrilus anxius, Agriotes spp., for example Agriotes linneatus, Agriotes mancus, Agriotes obscurus, Alphitobius diaperinus, Amphimallon solstitialis, Anobium punctatum, Anomala dubia, Anoplophora spp., for example Anoplophora glabripennis, Anthonomus spp., for example Anthonomus grandis, Anthrenus spp., Apion spp., Apogonia spp., Athous haemorrhoidales, Atomaria spp., for example Atomaria linearis, Attagenus spp., Baris caerulescens, Bruchidius obtectus, Bruchus spp., for example Bruchus pisorum, Bruchus rufimanus, Cassida spp., Cerotoma trifurcata, Ceutorrhynchus spp., for example Ceutorrhynchus assimilis, Ceutorrhynchus quadridens, Ceutorrhynchus rapae, Chaetocnema spp., for example Chaetocnema confinis, Chaetocnema denticulata, Chaetocnema ectypa, Cleonus mendicus, Conoderus spp., Cosmopolites spp., for example Cosmopolites sordidus, Costelytra zealandica, Ctenicera spp., Curculio spp., for example Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., for example Dendroctonus ponderosae, Dermestes spp., Diabrotica spp., for example Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., for example Epilachna borealis, Epilachna varivestis, Epitrix spp., for example Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hoplia argentea, Hylamorpha elegans, Hylotrupes bajulus, Hypera postica, Hypomeces squamosus, Hypothenemus spp., for example Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., for example Leucoptera coffeella, Limonius ectypus, Lissorhoptrus oryzophilus, Listronotus (= Hyperodes) spp., Lixus spp., Luperodes spp., Luperomorpha xanthodera, Lyctus spp., Megacyllene spp., for example Megacyllene robiniae, Megascelis spp., Melanotus spp., for example Melanotus longulus oregonensis, Meligethes aeneus, Melolontha spp., for example Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., for example Otiorhynchus cribricollis, Otiorhynchus ligustici, Otiorhynchus ovatus, Otiorhynchus rugosostriarus, Otiorhynchus sulcatus, Oulema spp., for example Oulema melanopus, Oulema oryzae, Oxycetonia jucunda, Phaedon cochleariae, Phyllophaga spp., Phyllophaga helleri, Phyllotreta spp., for example Phyllotreta armoraciae, Phyllotreta pusilla, Phyllotreta ramosa, Phyllotreta striolata, Popillia japonica, Premnotrypes spp., Prostephanus truncatus, Psylliodes spp., for example Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., for example Scolytus multistriatus, Sinoxylon perforans, Sitophilus spp., for example Sitophilus granarius, Sitophilus linearis, Sitophilus oryzae, Sitophilus zeamais, Sphenophorus spp., Stegobium paniceum, Sternechus spp., for example Sternechus paludatus, Symphyletes spp., Tanymecus spp., for example Tanymecus dilaticollis, Tanymecus indicus, Tanymecus palliatus, Tenebrio molitor, Tenebrioides mauretanicus, Tribolium spp., for example Tribolium audax, Tribolium castaneum, Tribolium confusum, Trogoderma spp., Tychius spp., Xylotrechus spp., Zabrus spp., for example Zabrus tenebrioides;
from the order of the Dermaptera, for example Anisolabis maritime, Forficula auricularia, Labidura riparia;
from the order of the Diptera, for example Aedes spp., for example Aedes aegypti, Aedes albopictus, Aedes sticticus, Aedes vexans, Agromyza spp., for example Agromyza frontella, Agromyza parvicornis, Anastrepha spp., Anopheles spp., for example Anopheles quadrimaculatus, Anopheles gambiae, Asphondylia spp., Bactrocera spp., for example Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., for example Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., for example Culex pipiens, Culex quinquefasciatus, Culicoides spp., Culiseta spp., Cuterebra spp., Dacus oleae, Dasineura spp., for example Dasineura brassicae, Delia spp., for example Delia antiqua, Delia coarctata, Delia florilega, Delia platura, Delia radicum, Dermatobia hominis, Drosophila spp., for example Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., for example Liriomyza brassicae, Liriomyza huidobrensis, Liriomyza sativae, Lucilia spp., for example Lucilia cuprina, Lutzomyia spp., Mansonia spp., Musca spp., for example Musca domestica, Musca domestica vicina, Oestrus spp., Oscinella frit, Paratanytarsus spp., Paralauterborniella subcincta, Pegomya or Pegomyia spp., for example Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., for example Rhagoletis cingulata, Rhagoletis completa, Rhagoletis fausta, Rhagoletis indifferens, Rhagoletis mendax, Rhagoletis pomonella, Sarcophaga spp., Simulium spp., for example Simulium meridionale, Stomoxys spp., Tabanus spp., Tetanops spp., Tipula spp., for example Tipula paludosa, Tipula simplex, Toxotrypana curvicauda;
from the order of the Hemiptera, for example Acizzia acaciaebaileyanae, Acizzia dodonaeae, Acizzia uncatoides, Acrida turrita, Acyrthosipon spp., for example Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., for example Amrasca bigutulla, Amrasca devastans, Anuraphis cardui, Aonidiella spp., for example Aonidiella aurantii, Aonidiella citrina, Aonidiella inornata, Aphanostigma piri, Aphis spp., for example Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., for example Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., for example Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., for example Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., for example Dysaphis apiifolia, Dysaphis plantaginea, Dysaphis tulipae, Dysmicoccus spp., Empoasca spp., for example Empoasca abrupta, Empoasca fabae, Empoasca maligna, Empoasca solana, Empoasca stevensi, Eriosoma spp., for example Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., for example Icerya purchasi, Idiocerus spp., Idioscopus spp., Laodelphax striatellus, Lecanium spp., for example Lecanium corni (=Parthenolecanium corni), Lepidosaphes spp., for example Lepidosaphes ulmi, Lipaphis erysimi, Lopholeucaspis japonica, Lycorma delicatula, Macrosiphum spp., for example Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., for example Myzus ascalonicus, Myzus cerasi, Myzus ligustri, Myzus ornatus, Myzus persicae,. Myzus nicotianae, Nasonovia ribisnigri, Neomaskellia spp., Nephotettix spp., for example Nephotettix cincticeps,, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., for example Paratrioza cockerelli, Parlatoria spp., Pemphigus spp., for example Pemphigus bursarius, Pemphigus populivenae, Peregrinus maidis, Perkinsiella spp., Phenacoccus spp., for example Phenacoccus madeirensis, Phloeomyzus passerinii, Phorodon humuli, Phylloxera spp., for example Phylloxera devastatrix, Phylloxera notabilis, Pinnaspis aspidistrae, Planococcus spp., for example Planococcus citri, Prosopidopsylla flava, Protopulvinaria pyriformis, Pseudaulacaspis pentagona, Pseudococcus spp., for example Pseudococcus calceolariae, Pseudococcus comstocki, Pseudococcus longispinus, Pseudococcus maritimus, Pseudococcus viburni, Psyllopsis spp., Psylla spp., for example Psylla buxi, Psylla mali, Psylla pyri, Pteromalus spp., Pulvinaria spp., Pyrilla spp., Quadraspidiotus spp., for example Quadraspidiotus juglansregiae, Quadraspidiotus ostreaeformis, Quadraspidiotus perniciosus, Quesada gigas, Rastrococcus spp., Rhopalosiphum spp., for example Rhopalosiphum maidis, Rhopalosiphum oxyacanthae, Rhopalosiphum padi, Rhopalosiphum rufiabdominale, Saissetia spp., for example Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., for example Toxoptera aurantii, Toxoptera citricidus, Trialeurodes vaporariorum, Trioza spp., for example Trioza diospyri, Typhlocyba spp., Unaspis spp., Viteus vitifolii, Zygina spp.;
from the suborder of the Heteroptera, for example Aelia spp., Anasa tristis, Antestiopsis spp., Boisea spp., Blissus spp., Calocoris spp., Campylomma livida, Cavelerius spp., Cimex spp., for example Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., for example Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., for example Lygocoris pabulinus, Lygus spp., for example Lygus elisus, Lygus hesperus, Lygus lineolaris, Macropes excavatus, Megacopta cribraria, Miridae, Monalonion atratum, Nezara spp., for example Nezara viridula, Nysius spp., Oebalus spp., Pentomidae, Piesma quadrata, Piezodorus spp., for example Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
from the order of the Hymenoptera, for example Acromyrmex spp., Athalia spp., for example Athalia rosae, Atta spp., Camponotus spp., Dolichovespula spp., Diprion spp., for example Diprion similis, Hoplocampa spp., for example Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., for example Sirex noctilio, Solenopsis invicta, Tapinoma spp., Technomyrmex albipes, Urocerus spp., Vespa spp., for example Vespa crabro, Wasmannia auropunctata, Xeris spp.;
from the order of the Isopoda, for example Armadillidium vulgare, Oniscus asellus, Porcellio scaber;
from the order of the Isoptera, for example Coptotermes spp., for example Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., for example Reticulitermes flavipes, Reticulitermes hesperus;
from the order of the Lepidoptera, for example Achroia grisella, Acronicta major, Adoxophyes spp., for example Adoxophyes orana, Aedia leucomelas, Agrotis spp., for example Agrotis segetum, Agrotis ipsilon, Alabama spp., for example Alabama argillacea, Amyelois transitella, Anarsia spp., Anticarsia spp., for example Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., for example Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., for example Cydia nigricana, Cydia pomonella, Dalaca noctuides, Diaphania spp., Diparopsis spp., Diatraea saccharalis, Dioryctria spp., for example Dioryctria zimmermani, Earias spp., Ecdytolopha aurantium, Elasmopalpus lignosellus, Eldana saccharina, Ephestia spp., for example Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., for example Euproctis chrysorrhoea, Euxoa spp., Feltia spp., Galleria mellonella, Gracillaria spp., Grapholitha spp., for example Grapholita molesta, Grapholita prunivora, Hedylepta spp., Helicoverpa spp., for example Helicoverpa armigera, Helicoverpa zea, Heliothis spp., for example Heliothis virescens, Hepialus spp., for example Hepialus humuli, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., for example Leucoptera coffeella, Lithocolletis spp., for example Lithocolletis blancardella, Lithophane antennata, Lobesia spp., for example Lobesia botrana, Loxagrotis albicosta, Lymantria spp., for example Lymantria dispar, Lyonetia spp., for example Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., for example Ostrinia nubilalis, Panolis flammea, Parnara spp., Pectinophora spp., for example Pectinophora gossypiella, Perileucoptera spp., Phthorimaea spp., for example Phthorimaea operculella, Phyllocnistis citrella, Phyllonorycter spp., for example Phyllonorycter blancardella, Phyllonorycter crataegella, Pieris spp., for example Pieris rapae, Platynota stultana, Plodia interpunctella, Plusia spp., Plutella xylostella (=Plutella maculipennis), Podesia spp., for example Podesia syringae, Prays spp., Prodenia spp., Protoparce spp., Pseudaletia spp., for example Pseudaletia unipuncta, Pseudoplusia includens, Pyrausta nubilalis, Rachiplusia nu, Schoenobius spp., for example Schoenobius bipunctifer, Scirpophaga spp., for example Scirpophaga innotata, Scotia segetum, Sesamia spp., for example Sesamia inferens, Sparganothis spp., Spodoptera spp., for example Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., for example Trichoplusia ni, Tryporyza incertulas, Tuta absoluta, Virachola spp.;
from the order of the Orthoptera or Saltatoria, for example Acheta domesticus, Dichroplus spp., Gryllotalpa spp., for example Gryllotalpa gryllotalpa, Hieroglyphus spp., Locusta spp., for example Locusta migratoria, Melanoplus spp., for example Melanoplus devastator, Paratlanticus ussuriensis, Schistocerca gregaria;
from the order of the Phthiraptera, for example Damalinia spp., Haematopinus spp., Linognathus spp., Pediculus spp., Phylloxera vastatrix, Phthirus pubis, Trichodectes spp.;
from the order of the Psocoptera, for example Lepinotus spp., Liposcelis spp.;
from the order of the Siphonaptera, for example, Ceratophyllus spp., Ctenocephalides spp., for example Ctenocephalides canis, Ctenocephalides felis, Pulex irritans, Tunga penetrans, Xenopsylla cheopis;
from the order of the Thysanoptera, for example Anaphothrips obscurus, Baliothrips biformis, Chaetanaphothrips leeuweni, Drepanothrips reuteri, Enneothrips flavens, Frankliniella spp., for example Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., for example Thrips palmi, Thrips tabaci;
from the order of the Zygentoma (= Thysanura), for example Ctenolepisma spp., Lepisma saccharina, Lepismodes inquilinus, Thermobia domestica;
from the class of the Symphyla, for example Scutigerella spp., for example Scutigerella immaculata;
pests from the phylum of the Mollusca, for example from the class of the Bivalvia, for example Dreissena spp.,
and also from the class of the Gastropoda, for example Arion spp., for example Arion ater rufus, Biomphalaria spp., Bulinus spp., Deroceras spp., for example Deroceras laeve, Galba spp., Lymnaea spp., Oncomelania spp., Pomacea spp., Succinea spp.;
plant pests from the phylum of the Nematoda, i.e. phytoparasitic nematodes, in particular Aglenchus spp., for example Aglenchus agricola, Anguina spp., for example Anguina tritici, Aphelenchoides spp., for example Aphelenchoides arachidis, Aphelenchoides fragariae, Belonolaimus spp., for example Belonolaimus gracilis, Belonolaimus longicaudatus, Belonolaimus nortoni, Bursaphelenchus spp., for example Bursaphelenchus cocophilus, Bursaphelenchus eremus, Bursaphelenchus xylophilus, Cacopaurus spp., for example Cacopaurus pestis, Criconemella spp., for example Criconemella curvata, Criconemella onoensis, Criconemella ornata, Criconemella rusium, Criconemella xenoplax (= Mesocriconema xenoplax), Criconemoides spp., for example Criconemoides ferniae, Criconemoides onoense, Criconemoides ornatum, Ditylenchus spp., for example Ditylenchus dipsaci, Dolichodorus spp., Globodera spp., for example Globodera pallida, Globodera rostochiensis, Helicotylenchus spp., for example Helicotylenchus dihystera, Hemicriconemoides spp., Hemicycliophora spp., Heterodera spp., for example Heterodera avenae, Heterodera glycines, Heterodera schachtii, Hirschmaniella spp., Hoplolaimus spp., Longidorus spp., for example Longidorus africanus, Meloidogyne spp., for example Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., for example Paratrichodorus minor, Paratylenchus spp., Pratylenchus spp., for example Pratylenchus penetrans, Pseudohalenchus spp., Psilenchus spp., Punctodera spp., Quinisulcius spp., Radopholus spp., for example Radopholus citrophilus, Radopholus similis, Rotylenchulus spp., Rotylenchus spp., Scutellonema spp., Subanguina spp., Trichodorus spp., for example Trichodorus obtusus, Trichodorus primitivus, Tylenchorhynchus spp., for example Tylenchorhynchus annulatus, Tylenchulus spp., for example Tylenchulus semipenetrans, Xiphinema spp., for example Xiphinema index.

The compounds of the formula (I) can optionally, at certain concentrations or application rates, also be used as herbicides, safeners, growth regulators or agents to improve plant properties, as microbicides or gametocides, for example as fungicides, antimycotics, bactericides, viricides (including agents against viroids) or as agents against MLO (mycoplasma-like organisms) and RLO (rickettsia-like organisms). If appropriate, they can also be used as intermediates or precursors for the synthesis of other active compounds.

### Formulations/Use forms

The present invention further relates to formulations, in particular formulations for controlling unwanted controlling animal pests. The formulation may be applied to the animal pest and/or in their habitat.

The formulation of the invention may be provided to the end user as "ready-for-use" use form, i.e. the formulations may be directly applied to the plants or seeds by a suitable device, such as a spraying or dusting device. Alternatively, the formulations may be provided to the end user in the form of concentrates which have to be diluted, preferably with water, prior to use. Unless otherwise indicated, the wording "formulation" therefore means such concentrate, whereas the wording "use form" means the end user as "ready-for-use" solution, i.e. usually such diluted formulation.

The formulation of the invention can be prepared in conventional manners, for example by mixing the compound of the invention with one or more suitable auxiliaries, such as disclosed herein.

The formulation comprises at least one compound of the invention and at least one agriculturally suitable auxiliary, e.g. carrier(s) and/or surfactant(s).

A carrier is a solid or liquid, natural or synthetic, organic or inorganic substance that is generally inert. The carrier generally improves the application of the compounds, for instance, to plants, plants parts or seeds. Examples of suitable solid carriers include, but are not limited to, ammonium salts, in particular ammonium sulfates, ammonium phosphates and ammonium nitrates, natural rock flours, such as kaolins, clays, talc, chalk, quartz, attapulgite, montmorillonite and diatomaceous earth, silica gel and synthetic rock flours, such as finely divided silica, alumina and silicates. Examples of typically useful solid carriers for preparing granules include, but are not limited to crushed and fractionated natural rocks such as calcite, marble, pumice, sepiolite and dolomite, synthetic granules of inorganic and organic flours and granules of organic material such as paper, sawdust, coconut shells, maize cobs and tobacco stalks. Examples of suitable liquid carriers include, but are not limited to, water, organic solvents and combinations thereof. Examples of suitable solvents include polar and nonpolar organic chemical liquids, for example from the classes of aromatic and nonaromatic hydrocarbons (such as cyclohexane, paraffins, alkylbenzenes, xylene, toluene, tetrahydronaphthalene, alkylnaphthalenes, chlorinated aromatics or chlorinated aliphatic hydrocarbons such as chlorobenzenes, chloroethylenes or methylene chloride), alcohols and polyols (which may optionally also be substituted, etherified and/or esterified, such as ethanol, propanol, butanol, benzylalcohol, cyclohexanol or glycol), ketones (such as acetone, methyl ethyl ketone, methyl isobutyl ketone, acetophenone, or cyclohexanone), esters (including fats and oils) and (poly)ethers, unsubstituted and substituted amines, amides (such as dimethylformamide or fatty acid amides) and esters thereof, lactams (such as N-alkylpyrrolidones, in particular N-methylpyrrolidone) and lactones, sulfones and sulfoxides (such as dimethyl sulfoxide), oils of vegetable or animal origin, nitriles (alkyl nitriles such as acetonitrile, propionotrilie, butyronitrile, or aromatic nitriles, such as benzonitrile), carbonic acid esters (cyclic carbonic acid esters, such as ethylene carbonate, propylene carbonate, butylene carbonate, or dialkyl carbonic acid esters, such as dimethyl carbonate, diethyl carbonate, dipropyl carbonate, dibutyl carbonate, dioctyl carbonate). The carrier may also be a liquefied gaseous extender, i.e. liquid which is gaseous at standard temperature and under standard pressure, for example aerosol propellants such as halohydrocarbons, butane, propane, nitrogen and carbon dioxide.

Preferred solid carriers are selected from clays, talc and silica.

Preferred liquid carriers are selected from water, fatty acid amides and esters thereof, aromatic and nonaromatic hydrocarbons, lactams, lactones, carbonic acid esters, ketones, (poly)ethers.

The amount of carrier typically ranges from 1 to 99.99%, preferably from 5 to 99.9%, more preferably from 10 to 99.5%, and most preferably from 20 to 99% by weight of the formulation.

Liquid carriers are typically present in a range of from 20 to 90%, for example 30 to 80% by weight of the formulation.

Solid carriers are typically present in a range of from 0 to 50%, preferably 5 to 45%, for example 10 to 30% by weight of the formulation.

If the formulation comprises two or more carriers, the outlined ranges refer to the total amount of carriers.

The surfactant can be an ionic (cationic or anionic), amphoteric or non-ionic surfactant, such as ionic or non-ionic emulsifier(s), foam former(s), dispersant(s), wetting agent(s), penetration enhancer(s) and any mixtures thereof. Examples of suitable surfactants include, but are not limited to, salts of polyacrylic acid, ethoxylated polya(alpha-substituted)acrylate derivatives, salts of lignosulfonic acid (such as sodium lignosulfonate), salts of phenolsulfonic acid or naphthalenesulfonic acid, polycondensates of ethylene oxide and/or propylene oxide with or without alcohols, fatty acids or fatty amines (for example, polyoxyethylene fatty acid esters such as castor oil ethoxylate, polyoxyethylene fatty alcohol ethers, for example alkylaryl polyglycol ethers), substituted phenols (preferably alkylphenols or arylphenols), salts of sulfosuccinic esters, taurine derivatives (preferably alkyl taurates), phosphoric esters of polyethoxylated alcohols or phenols, fatty esters of polyols (such a fatty acid esters of glycerol, sorbitol or sucrose), sulfates (such as alkyl sulfates and alkyl ether sulfates), sulfonates (for example, alkylsulfonates, arylsulfonates and alkylbenzene sulfonates), sulfonated polymers of naphthalene/formaldehyde, phosphate esters, protein hydrolysates, lignosulfite waste liquors and methylcellulose. Any reference to salts in this paragraph refers preferably to the respective alkali, alkaline earth and ammonium salts.

Preferred surfactants are selected from ethoxylated polya(alpha-substituted)acrylate derivatives, polycondensates of ethylene oxide and/or propylene oxide with alcohols, polyoxyethylene fatty acid esters, alkylbenzene sulfonates, sulfonated polymers of naphthalene/formaldehyde, polyoxyethylene fatty acid esters such as castor oil ethoxylate, sodium lignosulfonate and arylphenol ethoxylate.

The amount of surfactants typically ranges from 5 to 40%, for example 10 to 20%, by weight of the formulation.

Further examples of suitable auxiliaries include water repellents, siccatives, binders (adhesive, tackifier, fixing agent, such as carboxymethylcellulose, natural and synthetic polymers in the form of powders, granules or latices, such as gum arabic, polyvinyl alcohol and polyvinyl acetate, natural phospholipids such as cephalins and lecithins and synthetic phospholipids, polyvinylpyrrolidone and tylose), thickeners and secondary thickeners (such as cellulose ethers, acrylic acid derivatives, xanthan gum, modified clays, e.g. the products available under the name Bentone, and finely divided silica), stabilizers (e.g. cold stabilizers, preservatives (e.g. dichlorophene, benzyl alcohol hemiformal, 1,2-Benzisothiazolin-3-on, 2-methyl-4-isothiazolin-3-one), antioxidants, light stabilizers, in particular UV stabilizers, or other agents which improve chemical and/or physical stability), dyes or pigments (such as inorganic pigments, e.g. iron oxide, titanium oxide and Prussian Blue; organic dyes, e.g. alizarin, azo and metal phthalocyanine dyes), antifoams (e.g. silicone antifoams and magnesium stearate), antifreezes, stickers, gibberellins and processing auxiliaries, mineral and vegetable oils, perfumes, waxes, nutrients (including trace nutrients, such as salts of iron, manganese, boron, copper, cobalt, molybdenum and zinc), protective colloids, thixotropic substances, penetrants, sequestering agents and complex formers.

The choice of the auxiliaries depends on the intended mode of application of the compound of the invention and/or on the physical properties of the compound(s). Furthermore, the auxiliaries may be chosen to impart particular properties (technical, physical and/or biological properties) to the formulations or use forms prepared therefrom. The choice of auxiliaries may allow customizing the formulations to specific needs.

The formulation comprises an insecticidal/acaricidal/nematicidal effective amount of the compound(s) of the invention. The term "effective amount" denotes an amount, which is sufficient for controlling harmful insects/mites/nematodes on cultivated plants or in the protection of materials and which does not result in a substantial damage to the treated plants. Such an amount can vary in a broad range and is dependent on various factors, such as the insect/mite/nematode species to be controlled, the treated cultivated plant or material, the climatic conditions and the specific compound of the invention used. Usually, the formulation according to the invention contains from 0.01 to 99% by weight, preferably from 0.05 to 98% by weight, more preferred from 0.1 to 95% by weight, even more preferably from 0.5 to 90% by weight, most preferably from 1 to 80% by weight of the compound of the invention. It is possible that a formulation comprises two or more compounds of the invention. In such case the outlined ranges refer to the total amount of compounds of the present invention.

The formulation of the invention may be in any customary formulation type, such as solutions (e.g aqueous solutions), emulsions, water- and oil-based suspensions, powders (e.g. wettable powders, soluble powders), dusts, pastes, granules (e.g. soluble granules, granules for broadcasting), suspoemulsion concentrates, natural or synthetic products impregnated with the compound of the invention, fertilizers and also microencapsulations in polymeric substances. The compound of the invention may be present in a suspended, emulsified or dissolved form. Examples of particular suitable formulation types are solutions, watersoluble concentrates (e.g. SL, LS), dispersible concentrates (DC), suspensions and suspension concentrates (e.g. SC, OD, OF, FS), emulsifiable concentrates (e.g. EC), emulsions (e.g. EW, EO, ES, ME, SE), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GW, GF). These and further formulations types are defined by the Food and Agriculture Organization of the United Nations (FAO). An overview is given in the "Catalogue of pesticide formulation types and international coding system", Technical Monograph No. 2, 6th Ed. May 2008, Croplife International.

Preferably, the formulation of the invention is in form of one of the following types: EC, SC, FS, SE, OD, WG, WP, CS, more preferred EC, SC, OD , WG, CS.

Further details about examples of formulation types and their preparation are given below. If two or more compounds of the invention are present, the outlined amount of compound of the invention refers to the total amount of compounds of the present invention. This applies mutatis mutandis for any further component of the formulation, if two or more representatives of such component, e.g. wetting agent, binder, are present.

### i) Water-soluble concentrates (SL, LS)

10-60 % by weight of at least one compound of the invention and 5-15 % by weight surfactant (e.g. polycondensates of ethylene oxide and/or propylene oxide with alcohols) are dissolved in such amount of water and/or water-soluble solvent (e.g. alcohols such as propylene glycol or carbonates such as propylene carbonate) to result in a total amount of 100 % by weight. Before application the concentrate is diluted with water.

### ii) Dispersible concentrates (DC)

5-25 % by weight of at least one compound of the invention and 1-10 % by weight surfactant and/or binder (e.g. polyvinylpyrrolidone) are dissolved in such amount of organic solvent (e.g. cyclohexanone) to result in a total amount of 100 % by weight. Dilution with water gives a dispersion.

### iii) Emulsifiable concentrates (EC)

15-70 % by weight of at least one compound of the invention and 5-10 % by weight surfactant (e.g. a mixture of calcium dodecylbenzenesulfonate and castor oil ethoxylate) are dissolved in such amount of water-insoluble organic solvent (e.g. aromatic hydrocarbon or fatty acid amide) and if needed additional water-soluble solvent to result in a total amount of 100 % by weight. Dilution with water gives an emulsion.

### iv) Emulsions (EW, EO, ES)

5-40 % by weight of at least one compound of the invention and 1-10 % by weight surfactant (e.g. a mixture of calcium dodecylbenzenesulfonate and castor oil ethoxylate, or polycondensates of ethylene oxide and/or propylene oxide with or without alcohols) are dissolved in 20-40 % by weight water-insoluble organic solvent (e.g. aromatic hydrocarbon). This mixture is added to such amount of water by means of an emulsifying machine to result in a total amount of 100 % by weight. The resulting formulation is a homogeneous emulsion. Before application the emulsion may be further diluted with water.

### v) Suspensions and suspension concentrates

### v-1) Water-based (SC, FS)

In a suitable grinding equipment, e.g. an agitated ball mill, 20-60 % by weight of at least one compound of the invention are comminuted with addition of 2-10 % by weight surfactant (e.g. sodium lignosulfonate and polyoxyethylene fatty alcohol ether), 0.1-2 % by weight thickener (e.g. xanthan gum) and water to give a fine active substance suspension. The water is added in such amount to result in a total amount of 100 % by weight. Dilution with water gives a stable suspension of the active substance. For FS type formulations up to 40 % by weight binder (e.g. polyvinylalcohol) is added.

### v-2) Oil-based(OD, OF)

In a suitable grinding equipment, e.g. an agitated ball mill, 20-60 % by weight of at least one compound of the invention are comminuted with addition of 2-10 % by weight surfactant (e.g. sodium lignosulfonate and polyoxyethylene fatty alcohol ether), 0.1-2 % by weight thickener (e.g. modified clay, in particular Bentone, or silica) and an organic carrier to give a fine active substance oil suspension. The organic carrier is added in such amount to result in a total amount of 100 % by weight. Dilution with water gives a stable dispersion of the active substance.

### vi) Water-dispersible granules and water-soluble granules (WG, SG)

1-90 % by weight, preferably 20-80%, most preferably 50-80 % by weight of at least one compound of the invention are ground finely with addition of surfactant (e.g. sodium lignosulfonate and sodium alkylnaphthylsulfonates) and potentially carrier material and converted to water-dispersible or water-soluble granules by means of typical technical appliances like e. g. extrusion, spray drying, fluidized bed granulation. The surfactant and carrier material is used in such amount to result in a total amount of 100 % by weight. Dilution with water gives a stable dispersion or solution of the active substance.

### vii) Water-dispersible powders and water-soluble powders (WP, SP, WS)

50-80 % by weight of at least one compound of the invention are ground in a rotor-stator mill with addition of 1-20 % by weight surfactant (e.g. sodium lignosulfonate, sodium alkylnaphthylsulfonates) and such amount of solid carrier, e.g. silica gel, to result in a total amount of 100 % by weight. Dilution with water gives a stable dispersion or solution of the active substance.

### viii) Gel (GW, GF)

In an agitated ball mill, 5-25 % by weight of at least one compound of the invention are comminuted with addition of 3-10 % by weight surfactant (e.g. sodium lignosulfonate), 1-5 % by weight binder (e.g. carboxymethylcellulose) and such amount of water to result in a total amount of 100 % by weight. This results in a fine suspension of the active substance. Dilution with water gives a stable suspension of the active substance.

### ix) Microemulsion (ME)

5-20 % by weight of at least one compound of the invention are added to 5-30 % by weight organic solvent blend (e.g. fatty acid dimethylamide and cyclohexanone), 10-25 % by weight surfactant blend (e.g. polyoxyethylene fatty alcohol ether and arylphenol ethoxylate), and such amount of water to result in a total amount of 100 % by weight. This mixture is stirred for 1 h to produce spontaneously a thermodynamically stable microemulsion.

### x) Microcapsules (CS)

An oil phase comprising 5-50 % by weight of at least one compound of the invention, 0-40 % by weight water-insoluble organic solvent (e.g. aromatic hydrocarbon), 2-15 % by weight acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 % by weight of at least one compound of the invention, 0-40 % by weight water-insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol), this resulting in the formation of polyurea microcapsules. Optionally, the addition of a polyamine (e.g. hexamethylenediamine) is also used to result in the formation of polyurea microcapsules. The monomers amount to 1-10 % by weight of the total CS formulation.

### xi) Dustable powders (DP, DS)

1-10 % by weight of at least one compound of the invention are ground finely and mixed intimately with such amount of solid carrier, e.g. finely divided kaolin, to result in a total amount of 100 % by weight.

### xii) Granules (GR, FG)

0.5-30 % by weight of at least one compound of the invention are ground finely and associated with such amount of solid carrier (e.g. silicate) to result in a total amount of 100 % by weight.

### xiii) Ultra-low volume liquids (UL)

*1-50* % by weight of at least one compound of the invention are dissolved in such amount of organic solvent, e.g. aromatic hydrocarbon, to result in a total amount of 100 % by weight.

The formulations types i) to xiii) may optionally comprise further auxiliaries, such as 0.1-1 % by weight preservatives, 0.1-1 % by weight antifoams, 0.1-1 % by weight dyes and/or pigments, and 5-10% by weight antifreezes.

### Mixtures

The compounds of the formula (I) may also be employed as a mixture with one or more suitable fungicides, bactericides, acaricides, molluscicides, nematicides, insecticides, microbiologicals, beneficial species, herbicides, fertilizers, bird repellents, phytotonics, sterilants, safeners, semiochemicals and/or plant growth regulators, in order thus, for example, to broaden the spectrum of action, to prolong the duration of action, to increase the rate of action, to prevent repulsion or prevent evolution of resistance. In addition, such active compound combinations may improve plant growth and/or tolerance to abiotic factors, for example high or low temperatures, to drought or to elevated water content or soil salinity. It is also possible to improve flowering and fruiting performance, optimize germination capacity and root development, facilitate harvesting and improve yields, influence maturation, improve the quality and/or the nutritional value of the harvested products, prolong storage life and/or improve the processability of the harvested products.

Furthermore, the compounds of the formula (I) can be present in a mixture with other active compounds or semiochemicals such as attractants and/or bird repellants and/or plant activators and/or growth regulators and/or fertilizers. Likewise, the compounds of the formula (I) can be used to improve plant properties such as, for example, growth, yield and quality of the harvested material.

In a particular embodiment according to the invention, the compounds of the formula (I) are present in formulations or the use forms prepared from these formulations in a mixture with further compounds, preferably those as described below.

If one of the compounds mentioned below can occur in different tautomeric forms, these forms are also included even if not explicitly mentioned in each case. Further, all named mixing partners can, if their functional groups enable this, optionally form salts with suitable bases or acids.

### Insecticides/acaricides/nematicides

The active compounds identified here by their common names are known and are described, for example, in the pesticide handbook ("The Pesticide Manual" 16th Ed., British Crop Protection Council 2012) or can be found on the Internet (e.g. http://www.alanwood.net/pesticides). The classification is based on the current IRAC Mode of Action Classification Scheme at the time of filing of this patent application.
(1) Acetylcholinesterase (AChE) inhibitors, preferably carbamates selected from alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb, or organophosphates selected from acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.
(2) GABA-gated chloride channel blockers, preferably cyclodiene-organochlorines selected from chlordane and endosulfan, or phenylpyrazoles (fiproles) selected from ethiprole and fipronil.
(3) Sodium channel modulators, preferably pyrethroids selected from acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)- isomer)], tralomethrin and transfluthrin, or DDT or methoxychlor.
(4) Nicotinic acetylcholine receptor (nAChR) competitive modulators, preferably neonicotinoids selected from acetamiprid, clothianidin, dinotefuran, imidacloprid, nitenpyram, thiacloprid and thiamethoxam, or nicotine, or sulfoximines selected from sulfoxaflor, or butenolids selected from flupyradifurone, or mesoionics selected from triflumezopyrim.
(5) Nicotinic acetylcholine receptor (nAChR) allosteric modulators (Site I), preferably spinosyns selected from spinetoram and spinosad.
(6) Glutamate-gated chloride channel (GluCl) allosteric modulators, preferably avermectins/milbemycins selected from abamectin, emamectin benzoate, lepimectin and milbemectin.
(7) Juvenile hormone mimics, preferably juvenile hormone analogues selected from hydroprene, kinoprene and methoprene, or fenoxycarb or pyriproxyfen.
(8) Miscellaneous non-specific (multi-site) inhibitors, preferably alkyl halides selected from methyl bromide and other alkyl halides, or chloropicrine or sulphuryl fluoride or borax or tartar emetic or methyl isocyanate generators selected from diazomet and metam.
(9) Chordotonal organ TRPV channel modulators, preferably pyridine azomethanes selected from pymetrozine and pyrifluquinazone, or pyropenes selected from afidopyropen.
(10) Mite growth inhibitors affecting CHS1 selected from clofentezine, hexythiazox, diflovidazin and etoxazole.
(11) Microbial disruptors of the insect gut membranes selected from *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and *B.t.* plant proteins selected from Cry1Ab, Cry1Ac, Cry1Fa, Cry1A.105, Cry2Ab, Vip3A, mCry3A, Cry3Ab, Cry3Bb and Cry34Ab1/35Ab1.
(12) Inhibitors of mitochondrial ATP synthase, preferably ATP disruptors selected from diafenthiuron, or organotin compounds selected from azocyclotin, cyhexatin and fenbutatin oxide, or propargite or tetradifon.
(13) Uncouplers of oxidative phosphorylation via disruption of the proton gradient selected from chlorfenapyr, DNOC and sulfluramid.
(14) Nicotinic acetylcholine receptor channel blockers selected from bensultap, cartap hydrochloride, thiocylam and thiosultap-sodium.
(15) Inhibitors of chitin biosynthesis affecting CHS1, preferably benzoylureas selected from bistrifluron, chlorfluazuron, diflubenzuron, flucycloxuron, flufenoxuron, hexaflumuron, lufenuron, novaluron, noviflumuron, teflubenzuron and triflumuron.
(16) Inhibitors of chitin biosynthesis, type 1 selected from buprofezin.
(17) Moulting disruptor (in particular for Diptera, i.e. dipterans) selected from cyromazine.
(18) Ecdysone receptor agonists, preferably diacylhydrazines selected from chromafenozide, halofenozide, methoxyfenozide and tebufenozide.
(19) Octopamine receptor agonists selected from amitraz.
(20) Mitochondrial complex III electron transport inhibitors selected from hydramethylnone, acequinocyl, fluacrypyrim and bifenazate.
(21) Mitochondrial complex I electron transport inhibitors, preferably METI acaricides and insecticides selected from fenazaquin, fenpyroximate, pyrimidifen, pyridaben, tebufenpyrad and tolfenpyrad, or rotenone (Derris).
(22) Voltage-dependent sodium channel blockers, preferably oxadiazines selected from indoxacarb, or semicarbazones selected from metaflumizone.
(23) Inhibitors of acetyl CoA carboxylase, preferably tetronic and tetramic acid derivatives selected from spirodiclofen, spiromesifen, spiropidion and spirotetramat.
(24) Mitochondrial complex IV electron transport inhibitors, preferably phosphides selected from aluminium phosphide, calcium phosphide, phosphine and zinc phosphide, or cyanides selected from calcium cyanide, potassium cyanide and sodium cyanide.
(25) Mitochondrial complex II electron transport inhibitors, preferably *beta-*ketonitrile derivatives selected from cyenopyrafen and cyflumetofen, or carboxanilides selected from pyflubumide.
(28) Ryanodine receptor modulators, preferably diamides selected from chlorantraniliprole, cyantraniliprole, cyclaniliprole, flubendiamide and tetraniliprole.
(29) Chordotonal organ Modulators (with undefined target site) selected from flonicamid.
(30) GABA-gated chlorid channel allosteric modulators, preferably meta-diamides selected from broflanilide, or isoxazoles selected from fluxametamide.
(31) Baculovisuses, preferably Granuloviruses (GVs) selected from *Cydia pomonella* GV and *Thaumatotibia leucotreta* (GV), or Nucleopolyhedroviruses (NPVs) selected from *Anticarsia gemmatalis* MNPV and *Helicoverpa armigera* NPV.
(32) Nicotinic acetylcholine receptor allosteric modulators (Site II) selected from GS-omega/kappa HXTX-Hv1a peptide.
(33) further active compounds selected from Acynonapyr, Afoxolaner, Azadirachtin, Benclothiaz, Benzoximate, Benzpyrimoxan, Bromopropylate, Chinomethionat, Chloroprallethrin, Cryolite, Cyclobutrifluram, Cycloxaprid, Cyetpyrafen, Cyhalodiamide, Cyproflanilide (CAS 2375110-88-4), Dicloromezotiaz, Dicofol, Dimpropyridaz, epsilon-Metofluthrin, epsilon-Momfluthrin, Flometoquin, Fluazaindolizine, Flucypyriprole (CAS 1771741-86-6), Fluensulfone, Flufenerim, Flufenoxystrobin, Flufiprole, Fluhexafon, Fluopyram, Flupyrimin, Fluralaner, Fufenozide, Flupentiofenox, Guadipyr, Heptafluthrin, Imidaclothiz, Iprodione, Isocycloseram, kappa-Bifenthrin, kappa-Tefluthrin, Lotilaner, Meperfluthrin, Nicofluprole (CAS 1771741-86-6), Oxazosulfyl, Paichongding, Pyridalyl, Pyrifluquinazon, Pyriminostrobin, Sarolaner, Spidoxamat, Spirobudiclofen, Tetramethylfluthrin, Tetrachlorantraniliprole, Tigolaner, Tioxazafen, Thiofluoximate, Tyclopyrazoflor, Iodomethane; furthermore preparations based on *Bacillus firmus (I-1582,* Votivo) and azadirachtin (BioNeem), and also the following compounds: 1-{2-fluoro-4-methyl-5-[(2,2,2-trifluoroethyl)sulphinyl]phenyl}-3-(trifluoromethyl)-1H-1,2,4-triazole-5-amine (known from WO2006/043635) (CAS 885026-50-6), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6), PF1364 (known from JP2010/018586) (CAS 1204776-60-2), (3E)-3-[1-[(6-chloro-3-pyridyl)methyl]-2-pyridylidene]-1,1,1-trifluoro-propan-2-one (known from WO2013/144213) (CAS 1461743-15-6), N-[3-(benzylcarbamoyl)-4-chlorophenyl]-1-methyl-3-(pentafluoroethyl)-4-(trifluoromethyl)-1H-pyrazole-5-carboxamide (known from WO2010/051926) (CAS 1226889-14-0), 5-bromo-4-chloro-N-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-N-(cis-1-oxido-3-thietanyl)-benzamide, 4-[5-(3,5-dichlorophenyl)-4,5-dihydro-5-(trifluoromethyl)-3-isoxazolyl]-2-methyl-*N*-(trans-1-oxido-3-thietanyl)-benzamide and 4-[(5S)-5-(3,5-dichlorophenyl)-4,5-dihydro-5-(trifluoromethyl)-3-isoxazolyl]-2-methyl-*N*-(cis-1-oxido-3-thietanyl) benzamide (known from WO 2013/050317 A1) (CAS 1332628-83-7), *N*-[3-chloro-1-(3-pyridinyl)-1*H-*pyrazol-4-yl]-*N*-ethyl-3-[(3,3,3-trifluoropropyl)sulfinyl]-propanamide, (+)-*N*-[3-chloro-1-(3-pyridinyl)-1*H*-pyrazol-4-yl]-*N*-ethyl-3-[(3,3,3-trifluoropropyl)sulfinyl]-propanamide and (-)-*N*-[3-chloro-1-(3-pyridinyl)-1*H*-pyrazol-4-yl]-*N*-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2*E*)-3-chloro-2-propen-1-yl]amino]-1-[2,6-dichloro-4-(trifluoromethyl)phenyl]-4-[(trifluoromethyl)sulfinyl]-1*H*-pyrazole-3-carbonitrile (known from CN 101337937 A) (CAS 1105672-77-2), 3-bromo-*N*-[4-chloro-2-methyl-6-[(methylamino)thioxomethyl]phenyl]-1-(3-chloro-2-pyridinyl)-1*H*-pyrazole-5-carboxamide, (Liudaibenjiaxuanan, known from CN 103109816 A) (CAS 1232543-85-9); *N*-[4-chloro-2-[[(1,1-dimethylethyl)amino]carbonyl]-6-methylphenyl]-1-(3-chloro-2-pyridinyl)-3-(fluoromethoxy)-1*H-*pyrazole-5-carboxamide (known from WO 2012/034403 A1) (CAS 1268277-22-0), *N*-[2-(5-amino-1,3, 4-thiadiazol-2-yl)-4-chloro-6-methylphenyl]-3-bromo-1-(3-chloro-2-pyridinyl)-1*H*-pyrazole-5-carboxamide (known from WO 2011/085575 A1) (CAS 1233882-22-8), 4-[3-[2,6-dichloro-4-[(3,3-dichloro-2-propen-1-yl)oxy]phenoxy]propoxy]-2-methoxy-6-(trifluoromethyl)-pyrimidine (known from CN 101337940 A) (CAS 1108184-52-6); (2E)- and 2(*Z*)-2-[2-(4-cyanophenyl)-1-[3-(trifluoromethyl) phenyl]ethylidene]-*N*-[4-(difluoromethoxy)phenyl]-hydrazinecarboxamide (known from CN 101715774 A) (CAS 1232543-85-9); 3-(2,2-dichloroethenyl)-2,2-dimethyl-4-(1*H*-benzimidazol-2-yl)phenyl-cyclopropanecarboxylic acid ester (known from CN 103524422 A) (CAS 1542271-46-4); (4aS) -7-chloro-2,5-dihydro-2-[[(methoxycarbonyl)[4-[(trifluoromethyl)thio]phenyl]amino]carbonyl]-indeno[1,2-*e*][1,3,4]oxadiazine-4a(3*H*)-carboxylic acid methyl ester (known from CN 102391261 A) (CAS 1370358-69-2); 6-deoxy-3-*O*-ethyl-2,4-di-*O*-methyl-, 1-[*N*-[4-[1-[4-(1,1,2,2,2-pentafluoroethoxy) phenyl]-1*H*-1,2,4-triazol-3-yl]phenyl]carbamate]-α-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1 ]octane (CAS 1253850-56-4), (8-*anti*)-8-(2-cyclopropylmethoxy-4-trifluoromethyl-phenoxy)-3-(6-trifluoromethyl-pyridazin-3-yl)-3-aza-bicyclo[3.2.1 ]octane (CAS 933798-27-7), (8-*syn*)-8-(2-cyclopropylmethoxy-4-trifluoromethyl-phenoxy) -3-(6-trifluoromethyl-pyridazin-3-yl)-3-aza-bicyclo[3.2.1]octane (known from WO 2007040280 A1, WO 2007040282 A1) (CAS 934001-66-8), N-[4-(aminothioxomethyl)-2-methyl-6-[(methylamino)carbonyl]phenyl]-3-bromo-1-(3-chloro-2-pyridinyl)-1*H*-pyrazole-5-carboxamide (known from CN 103265527 A) (CAS 1452877-50-7), 3-(4-chloro-2,6-dimethylphenyl)-8-methoxy-1-methyl-1,8-diazaspiro[4.5]decane-2,4-dione (known from WO 2014/187846 A1) (CAS 1638765-58-8), 3-(4-chloro-2,6-dimethylphenyl)-8-methoxy-1-methyl-2-oxo-1,8-diazaspiro[4.5]dec-3-en-4-yl-carbonic acid ethyl ester (known from WO 2010/066780 A1, WO 2011151146 A1) (CAS 1229023-00-0), N-[1-(2,6-difluorophenyl)-1H-pyrazol-3-yl]-2-(trifluoromethyl)benzamide (known from WO 2014/053450 A1) (CAS 1594624-87-9), *N*-[2-(2,6-difluorophenyl)-2*H*-1,2,3-triazol-4-yl]-2-(trifluoromethyl)benzamide (known from WO 2014/053450 A1) (CAS 1594637-65-6), N-[1-(3,5-difluoro-2-pyridinyl)-1H-pyrazol-3-yl]-2-(trifluoromethyl)benzamide (known from WO 2014/053450 A1) (CAS 1594626-19-3), (3R)-3-(2-chloro-5-thiazolyl)-2,3-dihydro-8-methyl-5,7-dioxo-6-phenyl-5H-thiazolo[3,2-a]pyrimidinium inner salt (known from WO 2018/177970 A1) (CAS 2246757-58-2); 3-(2-chloro-5-thiazolyl)-2,3-dihydro-8-methyl-5,7-dioxo-6-phenyl-5*H*-thiazolo[3,2-a]pyrimidinium inner salt (known from WO 2018/177970 A1) (CAS 2246757-56-0); *N*-[3-chloro-1-(3-pyridinyl)-1H-pyrazol-4-yl]-2-(methylsulfonyl)-propanamide (known from WO 2019/236274 A1) (CAS 2396747-83-2), N-[2-bromo-4-[1,2,2,2-tetrafluoro-1-(trifluoromethyl)ethyl]-6-(trifluoromethyl)phenyl]-2-fluoro-3-[(4-fluorobenzoyl)amino]-benzamide (known from WO 2019059412 A1) (CAS 1207977-87-4).

### Fungicides

The active ingredients specified herein by their Common Name are known and described, for example, in The Pesticide Manual (16th Ed.British Crop Protection Council) or can be searched in the internet (e.g. www.alanwood.net/pesticides).

All named fungicidal mixing partners of the classes (1) to (15) can, if their functional groups enable this, optionally form salts with suitable bases or acids. All named mixing partners of the classes (1) to (15) can include tautomeric forms, where applicable.
1) Inhibitors of the ergosterol biosynthesis, for example (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1-({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{ [3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) Mefentrifluconazole, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione,(1.058)2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenylj-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{ [3-(2,2,3,3-tetrafluoropropoxy)phenyl] sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy }phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxy]-2-methylpyridin-3-yl}-N-ethyl-N-methylimidoformamide, (1.078) N'-{5-bromo-6-[(cis-4-isopropylcyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) ipfentrifluconazole, (1.082) 2-[4-(4-chlorophenoxy)-2-(trifluoromethyl)phenyl]-1-(1H-1,2,4-triazol-1-yl)propan-2-ol, (1.083) 2-[6-(4-bromophenoxy)-2-(trifluoromethyl)-3-pyridyl]-1-(1,2,4-triazol-1-yl)propan-2-ol, (1.084) 2-[6-(4-chlorophenoxy)-2-(trifluoromethyl)-3-pyridyl]-1-(1,2,4-triazol-1-yl)propan-2-ol, (1.085) 3-[2-(1-chlorocyclopropyl)-3-(3-chloro-2-fluoro-phenyl)-2-hydroxypropyl]imidazole-4-carbonitrile and (1.086) 4-[[6-[rac-(2R)-2-(2,4-difluorophenyl)-1,1-difluoro-2-hydroxy-3-(5-thioxo-4H-1,2,4-triazol-1-yl)propyl]-3-pyridyl]oxy]benzonitrile.
2) Inhibitors of the respiratory chain at complex I or II, for example (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028)inpyrfluxam, (2.029) 3-(difluoromethyl)-1-methyl-N-[(3S)-1,1,3-trimethyl-2,3-dihydro-1H-inden-4-yl]-1H-pyrazole-4-carboxamide, (2.030) fluindapyr, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{[4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-3-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide, (2.057) pyrapropoyne.
3) Inhibitors of the respiratory chain at complex III, for example (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025)fenpicoxamid, (3.026) mandestrobin, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate, (3.030) metyltetraprole, (3.031) florylpicoxamid.
4) Inhibitors of the mitosis and cell division, for example (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine.
5) Compounds capable to have a multisite action, for example (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile.
6) Compounds capable to induce a host defence, for example (6.001) acibenzolar-S-methyl, (6.002) isotianil, (6.003) probenazole, (6.004) tiadinil.
7) Inhibitors of the amino acid and/or protein biosynthesis, for example (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinoline.
8) Inhibitors of the ATP production, for example (8.001) silthiofam.
9) Inhibitors of the cell wall synthesis, for example (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one.
10) Inhibitors of the lipid and membrane synthesis, for example (10.001) propamocarb, (10.002) propamocarb hydrochloride, (10.003) tolclofos-methyl.
11) Inhibitors of the melanin biosynthesis, for example (11.001) tricyclazole, (11.002) 2,2,2-trifluoroethyl {3-methyl-1-[(4-methylbenzoyl)amino]butan-2-yl}carbamate.
12) Inhibitors of the nucleic acid synthesis, for example (12.001) benalaxyl, (12.002) benalaxyl-M (kiralaxyl), (12.003) metalaxyl, (12.004) metalaxyl-M (mefenoxam).
13) Inhibitors of the signal transduction, for example (13.001) fludioxonil, (13.002) iprodione, (13.003) procymidone, (13.004) proquinazid, (13.005) quinoxyfen, (13.006) vinclozolin.
14) Compounds capable to act as an uncoupler, for example (14.001) fluazinam, (14.002) meptyldinocap.
15) Further fungicides selected from the group consisting of (15.001) abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) Oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) dipymetitrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{ 5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{ [3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) Ipflufenoquin, (15.042) 2-{2-fluoro-6-[(8-fluoro-2-methylquinolin-3-yl)oxy]phenyl}propan-2-ol, (15.043)fluoxapiprolin, (15.044) 2-{3-[2-(1-{ [3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) quinofumelin, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methyleneamino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one, (15.063) aminopyrifen, (15.064) (N'-[2-chloro-4-(2-fluorophenoxy)-5-methylphenyl]-N-ethyl-N-methylimidoformamide), (15.065) (N'-(2-chloro-5-methyl-4-phenoxyphenyl)-N-ethyl-N-methylimido¬formamide), (15.066) (2-{2-[(7,8-difluoro-2-methylquinolin-3-yl)oxy]-6-fluorophenyl}propan-2-ol), (15.067) (5-bromo-1-(5,6-dimethylpyridin-3-yl)-3,3-dimethyl-3,4-dihydroisoquinoline), (15.068) (3-(4,4-difluoro-5,5-dimethyl-4,5-dihydrothieno[2,3-c]pyridin-7-yl)quinoline), (15.069) (1-(4,5-dimethyl-1H-benzimidazol-1-yl)-4,4-difluoro-3,3-dimethyl-3,4-dihydroisoquinoline), (15.070) 8-fluoro-3-(5-fluoro-3,3-dimethyl-3,4-dihydroisoquinolin-1-yl)quinolone, (15.071) 8-fluoro-3-(5-fluoro-3,3,4,4-tetramethyl-3,4-dihydroisoquinolin-1-yl)quinolone, (15.072) 3-(4,4-difluoro-3,3-dimethyl-3,4-dihydroisoquinolin-1-yl)-8-fluoroquinoline, (15.073) (N-methyl-N-phenyl-4-[5-(trifluoromethyl)-1,2,4-oxadiazol-3-yl]benzamide), (15.074) (methyl{4-[5-(trifluoromethyl)-1,2,4-oxadiazol-3-yl]phenyl}carbamate), (15.075) (N-{4-[5-(trifluoromethyl)-1,2,4-oxadiazol-3-yl]benzyl}-cyclopropane-carboxamide), (15.076) N-methyl-4-(5-(trifluoromethyl)-1,2,4-oxadiazol-3-yl]¬-benzamide, (15.077) N-[(E)-methoxyiminomethyl]-4-[5-(trifluoromethyl)-1,2,4-oxadiazol-3-yl]benzamide, (15.078) N-[(Z)-methoxyiminomethyl]-4-[5-(trifluoromethyl)-1,2,4-oxadiazol-3-yl]benzamide, (15.079) N-[4-[5-(trifluoromethyl)-1,2,4-oxadiazol-3-yl]phenyl]-cyclopropane-carboxamide, (15.080) N-(2-fluorophenyl)-4-[5-(trifluoromethyl)-1,2,4-oxadiazol-3-yl]benzamide, (15.081) 2,2-difluoro-N-methyl-2-[4-[5-(trifluoromethyl)-1,2,4-oxadiazol-3-yl]phenyl]-acetamide, (15.082) N-allyl-N-[[4-[5-(trifluoromethyl)-1,2,4-oxadiazol-3-yl)phenyl]methyl]acetamide, (15.083) N-[(E)-N-methoxy-C-methyl-carbonimidoyl]-4-(5-(trifluoromethyl)-1,2,4-oxadiazol-3-yl]-benzamide, (15.084) N-[(Z)-N-methoxy-C-methyl-carbonimidoyl]-4-[5-(trifluoromethyl)-1,2,4-oxadiazol-3-yl]benzamide, (15.085) N-allyl-N-[[4-[5-(trifluoromethyl)-1,2,4-oxadiazol-3-yl]phenyl]-methyl]-propanamide, (15.086) 4,4-dimethyl-1-[[4-[5-(trifluoromethyl)-1,2,4-oxadiazol-3-yl]phenyl]methyl]-pyrrolidin-2-one, (15.087) N-methyl-4-[5-(trifluoromethyl)-1,2,4-oxadiazol-3-yl]-benzenecarbothioamide, (15.088) 5-methyl-1-[[4-[5-(trifluoromethyl)-1,2,4-oxadiazol-3-yl]phenyl]methyl]pyrrolidin-2-one, (15.089) N-((2,3-difluoro-4-[5-(trifluoromethyl)-1,2,4-oxadiazol-3-yl]phenyl]methyl]-3,3,3-trifluoro-propanamide, (15.090) 1-methoxy-1-methyl-3-[[4-[5-(trifluoromethyl}-1,2,4-oxadiazol-3-yl]phenyl]¬methyl]urea, (15.091) 1,1-diethyl-3-[[4-[5-(trifluoromethyl}-1,2,4-oxadiazol-3-yl]phenyl]methyl]urea, (15.092) N-[[4-[5-(trifluoromethyl)-1,2,4-oxadiazol-3-yl]phen¬yl]methyl]propanamide, (15.093) N-methoxy-N-[[4-[5-(trifluoromethyl)-1,2,4-oxadiazol-3-yl]phenyl]methyl]cyclopropanecarboxamide, (15.094) 1-methoxy-3-methyl-1-[[4-[5-(trifluoromethyl)-1,2,4-oxadiazol-3-yl]phenyl]methyl]urea, (15.095) N-methoxy-N-[[4-[5-(trifluoromethyl)-1,2,4-oxadiazol-3-yl]phenyl]¬methyl)¬cyclopropane¬carboxamide, (15.096) N,2-dimethoxy-N-[[4-[5-(trifluoromethyl }-1,2,4-oxadiazol-3-yl]phenyl]¬methyl]¬propanamide, (15.097) N-ethyl-2-methyl-N-[[4-[5-(trifluoromethyl)-1,2,4-oxadiazol-3-yl)phenyl]methyl]¬propanamide, (15.098) 1-methoxy-3-methyl-1-[[4-[5-(trifluoromethyl)-1,2,4-oxadiazol-3-yl]phenyl]¬methyl]¬urea, (15.099) 1,3-dimethoxy-1-[[4-[5-(trifluoromethyl)-1,2,4-oxadiazol-3-yl]phenyl]methyl]urea, (15.100) 3-ethyl-1-methoxy-1-[[4-[5-(trifluoromethyl)-1,2,4-oxadiazol-3-yl]phenyl]methyl]urea, (15.101) 1-[[4-[5-(trifluoromethyl)-1,2,4-oxadiazol-3-yl]phenyl]methyl]piperidin-2-one, (15.102) 4,4-dimethyl-2-[[4-[5-(trifluoromethyl)-1,2,4-oxadiazol-3-yl]phenyl]methyl]isooxazolidin-3-one, (15.103) 5,5-dimethyl-2-[[4-[5-(trifluoromethyl)-1,2,4-oxadiazol-3-yl]phenyl]methyl]isoxazolidin-3-one, (15.104) 3,3-dimethyl-1-[[4-[5-(trifluoromethyl)-1,2,4-oxadiazol-3-yl]¬phenyl]¬methyl]¬piperidin-2-one, (15.105) 1-[[3-fluoro-4-(5-(trifluoromethyl)-1,2,4-oxadiazol-3-yl]¬phenyl]¬methyl]¬azepan-2-one, (15.106) 4,4-dimethyl-2-[[4-(5-(trifluoromethyl)-1,2,4-oxadiazol-3-yl]¬phenyl]¬methyl]isoxazolidin-3-one (15.107) 5,5-dimethyl-2-[[4-[5-(trifluoromethyl)-1,2,4-oxadiazol-3-yl]¬phenyl]methyl]isoxazolidin-3-one, (15.108) ethyl (1-{4-[5-(trifluoromethyl)-1,2,4-oxadiazol-3-yl]benzyl}-1H-pyrazol-4-yl)acetate, (15.109) N,N-dimethyl-1-{4-[5-(trifluoromethyl)-1,2,4-oxadiazol-3-yl]benzyl}-1H-1,2,4-triazol-3-amine and (15.110) N-{2,3-difluoro-4-[5-(trifluoromethyl)-1,2,4-oxadiazol-3-yl]benzyl}butanamide.

### Biological pesticides as mixing components

The compounds of the formula (I) can be combined with biological pesticides.

Biological pesticides comprise in particular bacteria, fungi, yeasts, plant extracts and products formed by microorganisms, including proteins and secondary metabolites.

Biological pesticides comprise bacteria such as spore-forming bacteria, root-colonising bacteria and bacteria which act as biological insecticides, fungicides or nematicides.

Examples of such bacteria which are employed or can be used as biological pesticides are:
*Bacillus amyloliquefaciens,* strain FZB42 (DSM 231179), or *Bacillus cereus,* in particular *B. cereus* strain CNCM I-1562 or *Bacillus firmus,* strain I-1582 (Accession number CNCM I-1582) or *Bacillus pumilus,* in particular strain GB34 (Accession No. ATCC 700814) and strain QST2808 (Accession No. NRRL B-30087), or *Bacillus subtilis,* in particular strain GB03 (Accession No. ATCC SD-1397), or *Bacillus subtilis* strain QST713 (Accession No. NRRL B-21661) or *Bacillus subtilis strain* OST 30002 (Accession No. NRRL B-50421) *Bacillus thuringiensis,* in particular *B. thuringiensis* subspecies *israelensis* (serotype H-14), strain AM65-52 (Accession No. ATCC 1276), or *B. thuringiensis subsp. aizawai,* in particular strain ABTS-1857 (SD-1372), or *B. thuringiensis subsp. kurstaki* strain HD-1, or *B. thuringiensis subsp. tenebrionis* strain NB 176 (SD-5428), *Pasteuria penetrans, Pasteuria spp.* (Rotylenchulus reniformis nematode)-PR3 (Accession Number ATCC SD-5834), *Streptomyces microflavus* strain AQ6121 (= QRD 31.013, NRRL B-50550), *Streptomyces galbus* strain AQ 6047 (Acession Number NRRL 30232).

Examples of fungi and yeasts which are employed or can be used as biological pesticides are: *Beauveria bassiana,* in particular strain ATCC 74040, *Coniothyrium minitans,* in particular strain CON/M/91-8 (Accession No. DSM-9660), *Lecanicillium spp.,* in particular strain HRO LEC 12, *Lecanicillium lecanii,* (formerly known as *Verticillium lecanii),* in particular strain KV01, *Metarhizium anisopliae,* in particular strain F52 (DSM3884/ ATCC 90448), *Metschnikowia fructicola,* in particular strain NRRL Y-30752, *Paecilomyces fumosoroseus (*now: *Isaria fumosorosea)*, in particular strain IFPC 200613, or strain Apopka 97 (Accesion No. ATCC 20874), *Paecilomyces lilacinus,* in particular P. *lilacinus* strain 251 (AGAL 89/030550), *Talaromyces flavus,* in particular strain V117b, *Trichoderma atroviride,* in particular strain SC1 (Accession Number CBS 122089), *Trichoderma harzianum,* in particular *T. harzianum rifai T39.* (Accession Number CNCM I-952).

Examples of viruses which are employed or can be used as biological pesticides are:
*Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus (NPV), *Spodoptera exigua* (beet armyworm) mNPV, *Spodoptera frugiperda* (fall armyworm) mNPV, *Spodoptera littoralis* (African cotton leafworm) NPV.

Also included are bacteria and fungi which are added as 'inoculant' to plants or plant parts or plant organs and which, by virtue of their particular properties, promote plant growth and plant health. Examples which may be mentioned are:
*Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*), *Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp., Streptomyces spp.*

Examples of plant extracts and products formed by microorganisms including proteins and secondary metabolites which are employed or can be used as biological pesticides are:
Allium sativum, Artemisia absinthium, azadirachtin, Biokeeper WP, Cassia nigricans, Celastrus angulatus, Chenopodium anthelminticum, chitin, Armour-Zen, Dryopteris filix-mas, Equisetum arvense, Fortune Aza, Fungastop, Heads Up (Chenopodium quinoa saponin extract), Pyrethrum/Pyrethrins, Quassia amara, Quercus, Quillaja, Regalia, "Requiem ^{™} Insecticide", rotenone, ryania/ryanodine, Symphytum officinale, Tanacetum vulgare, thymol, Triact 70, TriCon, Tropaeulum majus, Urtica dioica, Veratrin, Viscum album, Brassicaceae extract, in particular oilseed rape powder or mustard powder, as well as bioinsecticidal / acaricidal active substances obtained from olive oil, in particular unsaturated fatty/carboxylic acids having carbon chain lengths C₁₆-C₂₀ as active ingredients, such as, for example, contained in the product with the trade name FLiPPER^{®}.

### Safener as mixing components

The compounds of the formula (I) can be combined with safeners such as, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr (-diethyl), naphthalic anhydride, oxabetrinil, 2-methoxy-N-({4-[(methylcarbamoyl)amino]phenyl}sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

### Plants and plant parts

All plants and plant parts can be treated in accordance with the invention. Here, plants are to be understood to mean all plants and plant parts such as wanted and unwanted wild plants or crop plants (including naturally occurring crop plants), for example cereals (wheat, rice, triticale, barley, rye, oats), maize, soya bean, potato, sugar beet, sugar cane, tomatoes, pepper, cucumber, melon, carrot, watermelon, onion, lettuce, spinach, leek, beans, *Brassica oleracea* (e.g. cabbage) and other vegetable species, cotton, tobacco, oilseed rape, and also fruit plants (with the fruits apples, pears, citrus fruits and grapevines). Crop plants can be plants which can be obtained by conventional breeding and optimization methods or by biotechnological and genetic engineering methods or combinations of these methods, including the transgenic plants and including the plant varieties which can or cannot be protected by varietal property rights. Plants should be understood to mean all developmental stages, such as seeds, seedlings, young (immature) plants up to mature plants. Plant parts should be understood to mean all parts and organs of the plants above and below ground, such as shoot, leaf, flower and root, examples given being leaves, needles, stalks, stems, flowers, fruit bodies, fruits and seeds, and also tubers, roots and rhizomes. Parts of plants also include harvested plants or harvested plant parts and vegetative and generative propagation material, for example seedlings, tubers, rhizomes, cuttings and seeds.

Treatment according to the invention of the plants and plant parts with the compounds of the formula (I) is carried out directly or by allowing the compounds to act on the surroundings, environment or storage space by the customary treatment methods, for example by immersion, spraying, evaporation, fogging, scattering, painting on, injection and, in the case of propagation material, in particular in the case of seeds, also by applying one or more coats.

As already mentioned above, it is possible to treat all plants and their parts according to the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (genetically modified organisms), and parts thereof are treated. The term "parts" or "parts of plants" or "plant parts" has been explained above. The invention is used with particular preference to treat plants of the respective commercially customary cultivars or those that are in use. Plant cultivars are to be understood as meaning plants having new properties ("traits") and which have been obtained by conventional breeding, by mutagenesis or by recombinant DNA techniques. They can be cultivars, varieties, bio- or genotypes.

### Transgenic plant, seed treatment and integration events

According to the invention, the compounds of formula (I) can be advantageously used to treat transgenic plants, plant cultivars or plant parts that received genetic material which imparts advantageous and/or useful properties (traits) to these plants, plant cultivars or plant parts. Therefore, it is contemplated that the present invention may be combined with one or more recombinant traits or transgenic event(s) or a combination thereof. For the purposes of this application, a transgenic event is created by the insertion of a specific recombinant DNA molecule into a specific position (locus) within the chromosome of the plant genome. The insertion creates a novel DNA sequence referred to as an "event" and is characterized by the inserted recombinant DNA molecule and some amount of genomic DNA immediately adjacent to/flanking both ends of the inserted DNA. Such trait(s) or transgenic event(s) include, but are not limited to, pest resistance, water use efficiency, yield performance, drought tolerance, seed quality, improved nutritional quality, hybrid seed production, and herbicide tolerance, in which the trait is measured with respect to a plant lacking such trait or transgenic event. Concrete examples of such advantageous and/or useful properties (traits) are better plant growth, vigor, stress tolerance, standability, lodging resistance, nutrient uptake, plant nutrition, and/or yield, in particular improved growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processability of the harvested products, and increased resistance or tolerance against animal and microbial pests, such as against insects, arachnids, nematodes, mites, slugs and snails.

Among DNA sequences encoding proteins which confer properties of resistance or tolerance to such animal and microbial pests, in particular insects, mention will particularly be made of the genetic material from Bacillus thuringiensis encoding the Bt proteins widely described in the literature and well known to those skilled in the art. Mention will also be made of proteins extracted from bacteria such as *Photorhabdus* (WO97/17432 and WO98/08932). In particular, mention will be made of the Bt Cry or VIP proteins which include the CrylA, CryIAb, CryIAc, CryIIA, CryIIIA, CryIIIB2, Cry9c Cry2Ab, Cry3Bb and CryIF proteins or toxic fragments thereof and also hybrids or combinations thereof, especially the CrylF protein or hybrids derived from a CrylF protein (e.g. hybrid CrylA-CrylF proteins or toxic fragments thereof), the CrylA-type proteins or toxic fragments thereof, preferably the CrylAc protein or hybrids derived from the CrylAc protein (e.g. hybrid CrylAb-CrylAc proteins) or the CrylAb or Bt2 protein or toxic fragments thereof, the Cry2Ae, Cry2Af or Cry2Ag proteins or toxic fragments thereof, the CrylA.105 protein or a toxic fragment thereof, the VIP3Aa19 protein, the VIP3Aa20 protein, the VIP3A proteins produced in the COT202 or COT203 cotton events, the VIP3Aa protein or a toxic fragment thereof as described in Estruch et al. (1996), Proc Natl Acad Sci US A. 28;93(11):5389-94, the Cry proteins as described in WO2001/47952, the insecticidal proteins from *Xenorhabdus* (as described in WO98/50427), *Serratia* (particularly from S. *entomophila*) or *Photorhabdus* species strains, such as Tc-proteins from Photorhabdus as described in WO98/08932. Also any variants or mutants of any one of these proteins differing in some amino acids (1-10, preferably 1-5) from any of the above named sequences, particularly the sequence of their toxic fragment, or which are fused to a transit peptide, such as a plastid transit peptide, or another protein or peptide, is included herein.

Another and particularly emphasized example of such properties is conferred tolerance to one or more herbicides, for example imidazolinones, sulphonylureas, glyphosate or phosphinothricin. Among DNA sequences encoding proteins which confer properties of tolerance to certain herbicides on the transformed plant cells and plants, mention will be particularly be made to the bar or PAT gene or the Streptomyces coelicolor gene described in WO2009/152359 which confers tolerance to glufosinate herbicides, a gene encoding a suitable EPSPS (5-Enolpyruvylshikimat-3-phosphat-synthase) which confers tolerance to herbicides having EPSPS as a target, especially herbicides such as glyphosate and its salts, a gene encoding glyphosate-n-acetyltransferase, or a gene encoding glyphosate oxidoreductase. Further suitable herbicide tolerance traits include at least one ALS (acetolactate synthase) inhibitor (e.g. WO2007/024782), a mutated Arabidopsis ALS/AHAS gene (e.g. U.S. Patent 6,855,533), genes encoding 2,4-D-monooxygenases conferring tolerance to 2,4-D (2,4- dichlorophenoxyacetic acid) and genes encoding Dicamba monooxygenases conferring tolerance to dicamba (3,6-dichloro-2- methoxybenzoic acid).

Further and particularly emphasized examples of such properties are increased resistance against phytopathogenic fungi, bacteria and/or viruses owing, for example, to systemic acquired resistance (SAR), systemin, phytoalexins, elicitors and also resistance genes and correspondingly expressed proteins and toxins.

Particularly useful transgenic events in transgenic plants or plant cultivars which can be treated with preference in accordance with the invention include Event 531/ PV-GHBK04 (cotton, insect control, described in WO2002/040677), Event 1143-14A (cotton, insect control, not deposited, described in WO2006/128569); Event 1143-51B (cotton, insect control, not deposited, described in WO2006/128570); Event 1445 (cotton, herbicide tolerance, not deposited, described in US-A 2002-120964 or WO2002/034946); Event 17053 (rice, herbicide tolerance, deposited as PTA-9843, described in WO2010/117737); Event 17314 (rice, herbicide tolerance, deposited as PTA-9844, described in WO2010/117735); Event 281-24-236 (cotton, insect control - herbicide tolerance, deposited as PTA-6233, described in WO2005/103266 or US-A 2005-216969); Event 3006-210-23 (cotton, insect control - herbicide tolerance, deposited as PTA-6233, described in US-A 2007-143876 orWO2005/103266); Event 3272 (corn, quality trait, deposited as PTA-9972, described in WO2006/098952 or US-A 2006-230473); Event 33391 (wheat, herbicide tolerance, deposited as PTA-2347, described in WO2002/027004), Event 40416 (corn, insect control - herbicide tolerance, deposited as ATCC PTA-11508, described in WO 11/075593); Event 43A47 (corn, insect control - herbicide tolerance, deposited as ATCC PTA-11509, described in WO2011/075595); Event 5307 (corn, insect control, deposited as ATCC PTA-9561, described in WO2010/077816); Event ASR-368 (bent grass, herbicide tolerance, deposited as ATCC PTA-4816, described in US-A 2006-162007 or WO2004/053062); Event B16 (corn, herbicide tolerance, not deposited, described in US-A 2003-126634); Event BPS-CV127- 9 (soybean, herbicide tolerance, deposited as NCIMB No. 41603, described in WO2010/080829); Event BLRl (oilseed rape, restoration of male sterility, deposited as NCIMB 41193, described in WO2005/074671), Event CE43-67B (cotton, insect control, deposited as DSM ACC2724, described in US-A 2009-217423 or WO2006/128573); Event CE44-69D (cotton, insect control, not deposited, described in US-A 2010- 0024077); Event CE44-69D (cotton, insect control, not deposited, described in WO2006/128571); Event CE46-02A (cotton, insect control, not deposited, described in WO2006/128572); Event COT102 (cotton, insect control, not deposited, described in US-A 2006-130175 or WO2004/039986); Event COT202 (cotton, insect control, not deposited, described in US-A 2007-067868 or WO2005/054479); Event COT203 (cotton, insect control, not deposited, described in WO2005/054480); ); Event DAS21606-3 / 1606 (soybean, herbicide tolerance, deposited as PTA-11028, described in WO2012/033794), Event DAS40278 (corn, herbicide tolerance, deposited as ATCC PTA-10244, described in WO2011/022469); Event DAS-44406-6 / pDAB8264.44.06.l (soybean, herbicide tolerance, deposited as PTA-11336, described in WO2012/075426), Event DAS-14536-7 /pDAB8291.45.36.2 (soybean, herbicide tolerance, deposited as PTA-11335, described in WO2012/075429), Event DAS-59122-7 (corn, insect control - herbicide tolerance, deposited as ATCC PTA 11384, described in US-A 2006-070139); Event DAS-59132 (corn, insect control - herbicide tolerance, not deposited, described in WO2009/100188); Event DAS68416 (soybean, herbicide tolerance, deposited as ATCC PTA-10442, described in WO2011/066384 or WO2011/066360); Event DP-098140-6 (corn, herbicide tolerance, deposited as ATCC PTA-8296, described in US-A 2009- 137395 or WO 08/112019); Event DP-305423-1 (soybean, quality trait, not deposited, described in US-A 2008-312082 or WO2008/054747); Event DP-32138-1 (corn, hybridization system, deposited as ATCC PTA-9158, described in US-A 2009-0210970 or WO2009/103049); Event DP-356043-5 (soybean, herbicide tolerance, deposited as ATCC PTA-8287, described in US-A 2010-0184079 or WO2008/002872); Event EE-I (brinjal, insect control, not deposited, described in WO 07/091277); Event Fil 17 (corn, herbicide tolerance, deposited as ATCC 209031, described in US-A 2006-059581 or WO 98/044140); Event FG72 (soybean, herbicide tolerance, deposited as PTA-11041, described in WO2011/063413), Event GA21 (corn, herbicide tolerance, deposited as ATCC 209033, described in US-A 2005-086719 or WO 98/044140); Event GG25 (corn, herbicide tolerance, deposited as ATCC 209032, described in US-A 2005-188434 or WO98/044140); Event GHB119 (cotton, insect control - herbicide tolerance, deposited as ATCC PTA-8398, described in WO2008/151780); Event GHB614 (cotton, herbicide tolerance, deposited as ATCC PTA-6878, described in US-A 2010-050282 or WO2007/017186); Event GJ11 (corn, herbicide tolerance, deposited as ATCC 209030, described in US-A 2005-188434 or WO98/044140); Event GM RZ13 (sugar beet, virus resistance, deposited as NCIMB-41601, described in WO2010/076212); Event H7-l (sugar beet, herbicide tolerance, deposited as NCIMB 41158 or NCIMB 41159, described in US-A 2004-172669 or WO 2004/074492); Event JOPLINl (wheat, disease tolerance, not deposited, described in US-A 2008-064032); Event LL27 (soybean, herbicide tolerance, deposited as NCIMB41658, described in WO2006/108674 or US-A 2008-320616); Event LL55 (soybean, herbicide tolerance, deposited as NCIMB 41660, described in WO 2006/108675 or US-A 2008-196127); Event LLcotton25 (cotton, herbicide tolerance, deposited as ATCC PTA-3343, described in WO2003/013224 or US- A 2003-097687); Event LLRICE06 (rice, herbicide tolerance, deposited as ATCC 203353, described in US 6,468,747 or WO2000/026345); Event LLRice62 ( rice, herbicide tolerance, deposited as ATCC 203352, described in WO2000/026345), Event LLRICE601 (rice, herbicide tolerance, deposited as ATCC PTA-2600, described in US-A 2008-2289060 or WO2000/026356); Event LY038 (corn, quality trait, deposited as ATCC PTA-5623, described in US- A 2007-028322 or WO2005/061720); Event MIR162 (corn, insect control, deposited as PTA-8166, described in US-A 2009-300784 or WO2007/142840); Event MIR604 (corn, insect control, not deposited, described in US-A 2008-167456 or WO2005/103301); Event MON15985 (cotton, insect control, deposited as ATCC PTA-2516, described in US-A 2004-250317 or WO2002/100163); Event MON810 (corn, insect control, not deposited, described in US-A 2002-102582); Event MON863 (corn, insect control, deposited as ATCC PTA-2605, described in WO2004/011601 or US-A 2006-095986); Event MON87427 (corn, pollination control, deposited as ATCC PTA-7899, described in WO2011/062904); Event MON87460 (corn, stress tolerance, deposited as ATCC PTA-8910, described in WO2009/111263 or US-A 2011-0138504); Event MON87701 (soybean, insect control, deposited as ATCC PTA- 8194, described in US-A 2009-130071 or WO2009/064652); Event MON87705 (soybean, quality trait - herbicide tolerance, deposited as ATCC PTA-9241, described in US-A 2010-0080887 or WO2010/037016); Event MON87708 (soybean, herbicide tolerance, deposited as ATCC PTA-9670, described in WO2011/034704); Event MON87712 (soybean, yield, deposited as PTA-10296, described in WO2012/051199), Event MON87754 (soybean, quality trait, deposited as ATCC PTA-9385, described in WO2010/024976); Event MON87769 (soybean, quality trait, deposited as ATCC PTA- 8911, described in US-A 2011-0067141 or WO2009/102873); Event MON88017 (corn, insect control - herbicide tolerance, deposited as ATCC PTA-5582, described in US-A 2008-028482 or WO2005/059103); Event MON88913 (cotton, herbicide tolerance, deposited as ATCC PTA-4854, described in WO2004/072235 or US-A 2006-059590); Event MON88302 (oilseed rape, herbicide tolerance, deposited as PTA-10955, described in WO2011/153186), Event MON88701 (cotton, herbicide tolerance, deposited as PTA-11754, described in WO2012/134808), Event MON89034 (corn, insect control, deposited as ATCC PTA-7455, described in WO 07/140256 or US-A 2008-260932); Event MON89788 (soybean, herbicide tolerance, deposited as ATCC PTA-6708, described in US-A 2006-282915 or WO2006/130436); Event MSl 1 (oilseed rape, pollination control - herbicide tolerance, deposited as ATCC PTA-850 or PTA-2485, described in WO2001/031042); Event MS8 (oilseed rape, pollination control - herbicide tolerance, deposited as ATCC PTA-730, described in WO2001/041558 or US-A 2003-188347); Event NK603 (corn, herbicide tolerance, deposited as ATCC PTA-2478, described in US-A 2007-292854); Event PE-7 (rice, insect control, not deposited, described in WO2008/114282); Event RF3 (oilseed rape, pollination control - herbicide tolerance, deposited as ATCC PTA-730, described in WO2001/041558 or US-A 2003-188347); Event RT73 (oilseed rape, herbicide tolerance, not deposited, described in WO2002/036831 or US-A 2008-070260); Event SYHT0H2 / SYN-000H2-5 (soybean, herbicide tolerance, deposited as PTA-11226, described in WO2012/082548), Event T227-1 (sugar beet, herbicide tolerance, not deposited, described in WO2002/44407 or US-A 2009-265817); Event T25 (corn, herbicide tolerance, not deposited, described in US-A 2001-029014 or WO2001/051654); Event T304-40 (cotton, insect control - herbicide tolerance, deposited as ATCC PTA-8171, described in US-A 2010-077501 or WO2008/122406); Event T342-142 (cotton, insect control, not deposited, described in WO2006/128568); Event TC1507 (corn, insect control - herbicide tolerance, not deposited, described in US-A 2005-039226 or WO2004/099447); Event VIP1034 (corn, insect control - herbicide tolerance, deposited as ATCC PTA-3925, described in WO2003/052073), Event 32316 (corn, insect control-herbicide tolerance, deposited as PTA-11507, described in WO2011/084632), Event 4114 (corn, insect control-herbicide tolerance, deposited as PTA-11506, described in WO2011/084621), event EE-GM3 / FG72 (soybean, herbicide tolerance, ATCC Accession N° PTA-11041) optionally stacked with event EE-GM1/LL27 or event EE-GM2/LL55 (WO2011/063413A2), event DAS-68416-4 (soybean, herbicide tolerance, ATCC Accession N° PTA-10442, WO2011/066360Al), event DAS-68416-4 (soybean, herbicide tolerance, ATCC Accession N° PTA-10442, WO2011/066384Al), event DP-040416-8 (corn, insect control, ATCC Accession N° PTA-11508, WO2011/075593Al), event DP-043A47-3 (corn, insect control, ATCC Accession N° PTA-11509, WO2011/075595Al), event DP- 004114-3 (corn, insect control, ATCC Accession N° PTA-11506, WO2011/084621Al), event DP-032316-8 (corn, insect control, ATCC Accession N° PTA-11507, WO2011/084632Al), event MON-88302-9 (oilseed rape, herbicide tolerance, ATCC Accession N° PTA-10955, WO2011/153186Al), event DAS-21606-3 (soybean, herbicide tolerance, ATCC Accession No. PTA-11028, WO2012/033794A2), event MON-87712-4 (soybean, quality trait, ATCC Accession N°. PTA-10296, WO2012/051199A2), event DAS-44406-6 (soybean, stacked herbicide tolerance, ATCC Accession N°. PTA-11336, WO2012/075426Al), event DAS-14536-7 (soybean, stacked herbicide tolerance, ATCC Accession N°. PTA-11335, WO2012/075429Al), event SYN-000H2-5 (soybean, herbicide tolerance, ATCC Accession N°. PTA-11226, WO2012/082548A2), event DP-061061-7 (oilseed rape, herbicide tolerance, no deposit N° available, WO2012071039Al), event DP-073496-4 (oilseed rape, herbicide tolerance, no deposit N° available, US2012131692), event 8264.44.06.1 (soybean, stacked herbicide tolerance, Accession N° PTA-11336, WO2012075426A2), event 8291.45.36.2 (soybean, stacked herbicide tolerance, Accession N°. PTA-11335, WO2012075429A2), event SYHT0H2 (soybean, ATCC Accession N°. PTA-11226, WO2012/082548A2), event MON88701 (cotton, ATCC Accession N° PTA-11754, WO2012/134808Al), event KK179-2 (alfalfa, ATCC Accession N° PTA-11833, WO2013/003558Al), event pDAB8264.42.32.1 (soybean, stacked herbicide tolerance, ATCC Accession N° PTA-11993, WO2013/010094Al), event MZDT09Y (corn, ATCC Accession N° PTA-13025, WO2013/012775Al).

Further, a list of such transgenic event(s) is provided by the United States Department of Agriculture's (USDA) Animal and Plant Health Inspection Service (APHIS) and can be found on their website on the world wide web at aphis.usda.gov. For this application, the status of such list as it is/was on the filing date of this application, is relevant.

The genes/events which impart the desired traits in question may also be present in combinations with one another in the transgenic plants. Examples of transgenic plants which may be mentioned are the important crop plants, such as cereals (wheat, rice, triticale, barley, rye, oats), maize, soya beans, potatoes, sugar beet, sugar cane, tomatoes, peas and other types of vegetable, cotton, tobacco, oilseed rape and also fruit plants (with the fruits apples, pears, citrus fruits and grapes), with particular emphasis being given to maize, soya beans, wheat, rice, potatoes, cotton, sugar cane, tobacco and oilseed rape. Traits which are particularly emphasized are the increased resistance of the plants to insects, arachnids, nematodes and slugs and snails, as well as the increased resistance of the plants to one or more herbicides.

Commercially available examples of such plants, plant parts or plant seeds that may be treated with preference in accordance with the invention include commercial products, such as plant seeds, sold or distributed under the GENUITY^{®}, DROUGHTGARD^{®}, SMARTSTAX^{®}, RIB COMPLETE^{®}, ROUNDUP READY^{®}, VT DOUBLE PRO^{®}, VT TRIPLE PRO^{®}, BOLLGARD II^{®}, ROUNDUP READY 2 YIELD^{®}, YIELDGARD^{®}, ROUNDUP READY^{®} 2 XTEN^{DTM}, INTACTA RR2 PRO^{®}, VISTIVE GOLD^{®}, and/or XTENDFLEX^{™} trade names.

### Crop protection - types of treatment

The treatment of the plants and plant parts with the compounds of the formula (I) is carried out directly or by action on their surroundings, habitat or storage space using customary treatment methods, for example by dipping, spraying, atomizing, irrigating, evaporating, dusting, fogging, broadcasting, foaming, painting, spreading-on, injecting, watering (drenching), drip irrigating and, in the case of propagation material, in particular in the case of seed, furthermore as a powder for dry seed treatment, a solution for liquid seed treatment, a water-soluble powder for slurry treatment, by incrusting, by coating with one or more coats, etc. It is furthermore possible to apply the compounds of the formula (I) by the ultra-low volume method or to inject the application form or the compound of the formula (I) itself into the soil.

A preferred direct treatment of the plants is foliar application, i.e. the compounds of the formula (I) are applied to the foliage, where treatment frequency and the application rate should be adjusted according to the level of infestation with the pest in question.

In the case of systemically active compounds, the compounds of the formula (I) also access the plants via the root system. The plants are then treated by the action of the compounds of the formula (I) on the habitat of the plant. This may be done, for example, by drenching, or by mixing into the soil or the nutrient solution, i.e. the locus of the plant (e.g. soil or hydroponic systems) is impregnated with a liquid form of the compounds of the formula (I), or by soil application, i.e. the compounds of the formula (I) according to the invention are introduced in solid form (e.g. in the form of granules) into the locus of the plants, or by drip application (often also referred to as "chemigation"), i.e. the liquid application of the compounds of the formula (I) according to the invention from surface or sub-surface driplines over a certain period of time together with varying amounts of water at defined locations in the vicinity of the plants. In the case of paddy rice crops, this can also be done by metering the compound of the formula (I) in a solid application form (for example as granules) into a flooded paddy field.

### Digital Technologies

The compounds of the invention can be used in combination with models e.g. embedded in computer programs for site specific crop management, satellite farming, precision farming or precision agriculture. Such models support the site specific management of agricultural sites with data from various sources such as soils, weather, crops (e.g. type, growth stage, plant health), weeds (e.g. type, growth stage), diseases, pests, nutrients, water, moisture, biomass, satellite data, yield etc. with the purpose to optimize profitability, sustainability and protection of the environment. In particular, such models can help to optimize agronomical decisions, control the precision of pesticide applications and record the work performed.

As an example, the compounds of the invention can be applied to a crop plant according to an appropriate dose regime if a model models the development of a pest and calculates that a threshold has been reached for which it is recommendable to apply the compound of the invention to the crop plant.

Commercially available systems which include agronomic models are e.g. FieldScriptsTM from The Climate Corporation, XarvioTM from BASF, AGLogicTM from John Deere, etc.

The compounds of the invention can also be used in combination with smart spraying equipment such as e.g. spot spraying or precision spraying equipment attached to or housed within a farm vehicle such as a tractor, robot, helicopter, airplane, unmanned aerial vehicle (UAV) such as a drone, etc. Such an equipment usually includes input sensors (such as e.g. a camera) and a processing unit configured to analyze the input data and configured to provide a decision based on the analysis of the input data to apply the compound of the invention to the crop plants (respectively the weeds) in a specific and precise manner. The use of such smart spraying equipment usually also requires positions systems (e.g. GPS receivers) to localize recorded data and to guide or to control farm vehicles; geographic information systems (GIS) to represent the information on intelligible maps, and appropriate farm vehicles to perform the required farm action such as the spraying.

In an example, pests can be detected from imagery acquired by a camera. In an example the pests can be identified and/or classified based on that imagery. Such identification and/ classification can make use of image processing algorithms. Such image processing algorithms can utilize machine learning algorithms, such as trained neutral networks, decision trees and utilize artificial intelligence algorithms. In this manner, the compounds described herein can be applied only where needed.

### Treatment of seed

The control of animal pests by treating the seed of plants has been known for a long time and is the subject of continuous improvements. However, the treatment of seed entails a series of problems which cannot always be solved in a satisfactory manner. Thus, it is desirable to develop methods for protecting the seed and the germinating plant which dispense with, or at least reduce considerably, the additional application of pesticides during storage, after sowing or after emergence of the plants. It is furthermore desirable to optimize the amount of active compound employed in such a way as to provide optimum protection for the seed and the germinating plant from attack by animal pests, but without damaging the plant itself by the active compound employed. In particular, methods for the treatment of seed should also take into consideration the intrinsic insecticidal or nematicidal properties of pest-resistant or -tolerant transgenic plants in order to achieve optimum protection of the seed and also the germinating plant with a minimum of pesticides being employed.

The present invention therefore in particular also relates to a method for the protection of seed and germinating plants, from attack by pests, by treating the seed with one of the compounds of the formula (I). The method according to the invention for protecting seed and germinating plants against attack by pests furthermore comprises a method where the seed is treated simultaneously in one operation or sequentially with a compound of the formula (I) and a mixing component. It also comprises a method where the seed is treated at different times with a compound of the formula (I) and a mixing component.

The invention likewise relates to the use of the compounds of the formula (I) for the treatment of seed for protecting the seed and the resulting plant from animal pests.

Furthermore, the invention relates to seed which has been treated with a compound of the formula (I) according to the invention so as to afford protection from animal pests. The invention also relates to seed which has been treated simultaneously with a compound of the formula (I) and a mixing component. The invention furthermore relates to seed which has been treated at different times with a compound of the formula (I) and a mixing component. In the case of seed which has been treated at different points in time with a compound of the formula (I) and a mixing component, the individual substances may be present on the seed in different layers. Here, the layers comprising a compound of the formula (I) and mixing components may optionally be separated by an intermediate layer. The invention also relates to seed where a compound of the formula (I) and a mixing component have been applied as component of a coating or as a further layer or further layers in addition to a coating.

Furthermore, the invention relates to seed which, after the treatment with a compound of the formula (I), is subjected to a film-coating process to prevent dust abrasion on the seed.

One of the advantages encountered with a systemically acting compound of the formula (I) is the fact that, by treating the seed, not only the seed itself but also the plants resulting therefrom are, after emergence, protected against animal pests. In this manner, the immediate treatment of the crop at the time of sowing or shortly thereafter can be dispensed with.

It has to be considered a further advantage that by treatment of the seed with a compound of the formula (I), germination and emergence of the treated seed may be enhanced.

It is likewise to be considered advantageous that compounds of the formula (I) can be used in particular also for transgenic seed.

Furthermore, compounds of the formula (I) can be employed in combination with compositions or compounds of signalling technology, leading to better colonization by symbionts such as, for example, rhizobia, mycorrhizae and/or endophytic bacteria or fungi, and/or to optimized nitrogen fixation.

The compounds of the formula (I) are suitable for protection of seed of any plant variety which is used in agriculture, in the greenhouse, in forests or in horticulture. In particular, this takes the form of seed of cereals (for example wheat, barley, rye, millet and oats), corn, cotton, soya beans, rice, potatoes, sunflowers, coffee, tobacco, canola, oilseed rape, beets (for example sugarbeets and fodder beets), peanuts, vegetables (for example tomatoes, cucumbers, bean, cruciferous vegetables, onions and lettuce), fruit plants, lawns and ornamental plants. The treatment of the seed of cereals (such as wheat, barley, rye and oats), maize, soya beans, cotton, canola, oilseed rape, vegetables and rice is of particular importance.

As already mentioned above, the treatment of transgenic seed with a compound of the formula (I) is also of particular importance. This takes the form of seed of plants which, as a rule, comprise at least one heterologous gene which governs the expression of a polypeptide with in particular insecticidal and/or nematicidal properties. The heterologous genes in transgenic seed can originate from microorganisms such as Bacillus, Rhizobium, Pseudomonas, Serratia, Trichoderma, Clavibacter, Glomus or Gliocladium. The present invention is particularly suitable for the treatment of transgenic seed which comprises at least one heterologous gene originating from Bacillus sp. It is particularly preferably a heterologous gene derived from Bacillus thuringiensis.

In the context of the present invention, the compound of the formula (I) is applied to the seed. Preferably, the seed is treated in a state in which it is stable enough to avoid damage during treatment. In general, the seed may be treated at any point in time between harvest and sowing. The seed usually used has been separated from the plant and freed from cobs, shells, stalks, coats, hairs or the flesh of the fruits. For example, it is possible to use seed which has been harvested, cleaned and dried down to a moisture content which allows storage. Alternatively, it is also possible to use seed which, after drying, has been treated with, for example, water and then dried again, for example priming. In the case of rice seed, it is also possible to use seed which has been soaked, for example in water to a certain stage of the rice embryo ('pigeon breast stage'), stimulating the germination and a more uniform emergence.

When treating the seed, care must generally be taken that the amount of the compound of the formula (I) applied to the seed and/or the amount of further additives is chosen in such a way that the germination of the seed is not adversely affected, or that the resulting plant is not damaged. This must be ensured particularly in the case of active compounds which can exhibit phytotoxic effects at certain application rates.

In general, the compounds of the formula (I) are applied to the seed in a suitable formulation. Suitable formulations and processes for seed treatment are known to the person skilled in the art.

The compounds of the formula (I) can be converted to the customary seed dressing formulations, such as solutions, emulsions, suspensions, powders, foams, slurries or other coating compositions for seed, and also ULV formulations.

These formulations are prepared in a known manner, by mixing the compounds of the formula (I) with customary additives such as, for example, customary extenders and also solvents or diluents, colorants, wetting agents, dispersants, emulsifiers, antifoams, preservatives, secondary thickeners, adhesives, gibberellins and also water.

Colorants which may be present in the seed-dressing formulations which can be used in accordance with the invention are all colorants which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of agrochemically active compounds. Preference is given to using alkylnaphthalenesulphonates, such as diisopropyl- or diisobutylnaphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Preference is given to using nonionic or anionic dispersants or mixtures of nonionic or anionic dispersants. Suitable nonionic dispersants include in particular ethylene oxide/propylene oxide block polymers, alkylphenol polyglycol ethers and tristryrylphenol polyglycol ethers, and the phosphated or sulphated derivatives thereof. Suitable anionic dispersants are in particular lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Preference is given to using silicone antifoams and magnesium stearate.

Preservatives which may be present in the seed dressing formulations usable in accordance with the invention are all substances usable for such purposes in agrochemical compositions. Examples include dichlorophene and benzyl alcohol hemiformal.

Secondary thickeners which may be present in the seed dressing formulations usable in accordance with the invention are all substances which can be used for such purposes in agrochemical compositions. Cellulose derivatives, acrylic acid derivatives, xanthan, modified clays and finely divided silica are preferred.

Adhesives which may be present in the seed dressing formulations usable in accordance with the invention are all customary binders usable in seed dressing products. Polyvinylpyrrolidone, polyvinyl acetate, polyvinyl alcohol and tylose may be mentioned as being preferred.

Gibberellins which can be present in the seed-dressing formulations which can be used in accordance with the invention are preferably the gibberellins A1, A3 (= gibberellic acid), A4 and A7; gibberellic acid is especially preferably used. The gibberellins are known (cf. R. Wegler "Chemie der Pflanzenschutz- and Schädlingsbekämpfungsmittel", vol. 2, Springer Verlag, 1970, pp. 401-412).

The seed dressing formulations usable in accordance with the invention can be used to treat a wide variety of different kinds of seed either directly or after prior dilution with water. For instance, the concentrates or the preparations obtainable therefrom by dilution with water can be used to dress the seed of cereals, such as wheat, barley, rye, oats, and triticale, and also the seed of maize, rice, oilseed rape, peas, beans, cotton, sunflowers, soya beans and beets, or else a wide variety of different vegetable seed. The seed dressing formulations usable in accordance with the invention, or the dilute use forms thereof, can also be used to dress seed of transgenic plants.

For treatment of seed with the seed dressing formulations usable in accordance with the invention, or the use forms prepared therefrom by adding water, all mixing units usable customarily for the seed dressing are useful. Specifically, the procedure in the seed dressing is to place the seed into a mixer, operated batchwise or continously, to add the particular desired amount of seed dressing formulations, either as such or after prior dilution with water, and to mix everything until the formulation is distributed homogeneously on the seed. If appropriate, this is followed by a drying operation.

The application rate of the seed dressing formulations usable in accordance with the invention can be varied within a relatively wide range. It is guided by the particular content of the compounds of the formula (I) in the formulations and by the seed. The application rates of the compound of the formula (I) are generally between 0.001 and 50 g per kilogram of seed, preferably between 0.01 and 15 g per kilogram of seed.

### Animal health

In the animal health field, i.e. in the field of veterinary medicine, the compounds of the formula (I) are active against animal parasites, in particular ectoparasites or endoparasites. The term endoparasite includes in particular helminths and protozoae, such as coccidia. Ectoparasites are typically and preferably arthropods, in particular insects or acarids.

In the field of veterinary medicine the compounds of the formula (I) are suitable, with favourable toxicity in warm blooded animals, for controlling parasites which occur in animal breeding and animal husbandry in livestock, breeding, zoo, laboratory, experimental and domestic animals. They are active against all or specific stages of development of the parasites.

Agricultural livestock include, for example, mammals, such as, sheep, goats, horses, donkeys, camels, buffaloes, rabbits, reindeers, fallow deers, and in particular cattle and pigs; or poultry, such as turkeys, ducks, geese, and in particular chickens; or fish or crustaceans, e.g. in aquaculture; or, as the case may be, insects such as bees.

Domestic animals include, for example, mammals, such as hamsters, guinea pigs, rats, mice, chinchillas, ferrets or in particular dogs, cats; cage birds; reptiles; amphibians or aquarium fish.

According to a particular embodiment, the compounds of the formula (I) are administered to mammals.

According to another particular embodiment, the compounds of the formula (I) are administered to birds, namely cage birds or in particular poultry.

By using the compounds of the formula (I) to control animal parasites, it is intended to reduce or prevent illness, cases of deaths and performance reductions (in the case of meat, milk, wool, hides, eggs, honey and the like), so that more economical and simpler animal keeping is made possible and better animal well-being is achievable.

The term "control" or "controlling", as used herein with regard to the animal health field, means that the compounds of the formula (I) are effective in reducing the incidence of the respective parasite in an animal infected with such parasites to innocuous levels. More specifically, "controlling", as used herein, means that the compounds of the formula (I) are effective in killing the respective parasite, inhibiting its growth, or inhibiting its proliferation.

Exemplary arthropods include, without any limitation
from the order of the Anoplurida, for example, Haematopinus spp., Linognathus spp., Pediculus spp., Phtirus spp., Solenopotes spp.;
from the order of the Mallophagida and the suborders Amblycerina and Ischnocerina, for example Bovicola spp., Damalina spp., Felicola spp., Lepikentron spp., Menopon spp., Trichodectes spp., Trimenopon spp., Trinoton spp., Werneckiella spp.;
from the order of the Diptera and the suborders Nematocerina and Brachycerina, for example Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.
from the order of the Siphonapterida, for example Ceratophyllus spp.; Ctenocephalides spp., Pulex spp., Tunga spp., Xenopsylla spp.;
from the order of the Heteropterida, for example Cimex spp., Panstrongylus spp., Rhodnius spp., Triatoma spp.; as well as nuisance and hygiene pests from the order of the Blattarida.

Further, among the arthropods, the following acari may be mentioned by way of example, without any limitation:
from the subclass of the Acari (Acarina) and the order of the Metastigmata, for example, from the family of argasidae like Argas spp., Ornithodorus spp., Otobius spp., from the family of Ixodidae like Amblyomma spp., Dermacentor spp., Haemaphysalis spp., Hyalomma spp., Ixodes spp., Rhipicephalus (Boophilus) spp , Rhipicephalus spp. (the original genus of multi host ticks); from the order of mesostigmata like Dermanyssus spp., Ornithonyssus spp., Pneumonyssus spp., Raillietia spp., Sternostoma spp., Tropilaelaps spp., Varroa spp.; from the order of the Actinedida (Prostigmata), for example Acarapis spp., Cheyletiella spp., Demodex spp., Listrophorus spp., Myobia spp., Neotrombicula spp., Ornithocheyletia spp., Psorergates spp., Trombicula spp.; and from the order of the Acaridida (Astigmata), for example Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Exemplary parasitic protozoa include, without any limitation:
Mastigophora (Flagellata) such as:
Metamonada: from the order Diplomonadida, for example, Giardia spp., Spironucleus spp.

Parabasala: from the order Trichomonadida, for example, Histomonas spp., Pentatrichomonas spp.,Tetratrichomonas spp., Trichomonas spp., Tritrichomonas spp.

Euglenozoa: from the order Trypanosomatida, for example, Leishmania spp., Trypanosoma spp
Sarcomastigophora (Rhizopoda), such as Entamoebidae, for example, Entamoeba spp., Centramoebidae, for example, Acanthamoeba sp., Euamoebidae, e.g. Hartmanella sp.

Alveolata such as Apicomplexa (Sporozoa): e.g. Cryptosporidium spp.; from the order Eimeriida, for example, Besnoitia spp., Cystoisospora spp., Eimeria spp., Hammondia spp., Isospora spp., Neospora spp., Sarcocystis spp., Toxoplasma spp.; from the order Adeleida e.g. Hepatozoon spp., Klossiella spp.; from the order Haemosporida e.g. Leucocytozoon spp., Plasmodium spp.; from the order Piroplasmida e.g. Babesia spp., Ciliophora spp., Echinozoon spp., Theileria spp.; from the order Vesibuliferida e.g. Balantidium spp., Buxtonella spp.

Microspora such as Encephalitozoon spp., Enterocytozoon spp., Globidium spp., Nosema spp., and furthermore, e.g. Myxozoa spp.

Helminths pathogenic for humans or animals include, for example, acanthocephala, nematodes, pentastoma and platyhelmintha (e.g. monogenea, cestodes and trematodes).

Exemplary helminths include, without any limitation:
Monogenea: e.g.: Dactylogyrus spp., Gyrodactylus spp., Microbothrium spp., Polystoma spp., Troglocephalus spp.

Cestodes: from the order of the Pseudophyllidea, for example: Bothridium spp., Diphyllobothrium spp., Diplogonoporus spp., Ichthyobothrium spp., Ligula spp., Schistocephalus spp., Spirometra spp.
from the order of the Cyclophyllida, for example: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: from the class of the Digenea, for example: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematodes: from the order of the Trichinellida, for example: Capillaria spp., Eucoleus spp., Paracapillaria spp., Trichinella spp., Trichomosoides spp., Trichuris spp.
from the order of the Tylenchida, for example: Micronema spp., Parastrongyloides spp., Strongyloides spp.
from the order of the Rhabditina, for example: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.
from the order of the Spirurida, for example: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acantocephala: from the order of the Oligacanthorhynchida, for example: Macracanthorhynchus spp., Prosthenorchis spp.; from the order of the Moniliformida, for example: Moniliformis spp.
from the order of the Polymorphida, for example: Filicollis spp.; from the order of the Echinorhynchida, for example: Acanthocephalus spp., Echinorhynchus spp., Leptorhynchoides spp.

Pentastoma: from the order of the Porocephalida, for example: Linguatula spp.

In the veterinary field and in animal keeping, the administration of the compounds of the formula (I) is carried out by methods generally known in the art, such as enterally, parenterally, dermally or nasally, in the form of suitable preparations. Administration can be carried out prophylactically, methaphylactically or therapeutically.

Thus, one embodiment of the present invention refers to the compounds of the formula (I) for use as a medicament.

Another aspect refers to the compounds of the formula (I) for use as an antiendoparasitical agent.

Another particular aspect refers to the compounds of the formula (I) for use as a anthelmintic agent, more particular for use as a nematicidal agent, a platyhelminthicidal agent, an acanthocephalicidal agent, or a pentastomicidal agent.

Another particular aspect refers to the compounds of the formula (I) for use as an antiprotozoal agent.

Another aspect refers to the compounds of the formula (I) for use as an antiectoparasitical agent, in particular an arthropodicidal agent, more particular an insecticidal agent or acaricidal agent.

Further aspects of the invention are veterinary formulations, comprising an effective amount of at least one compound of the formula (I) and at least one of the following: pharmaceutically acceptable excipient (e.g. solid or liquid diluents), pharmaceutically acceptable auxiliary (e.g. surfactants), in particular a pharmaceutically acceptable excipient and/or pharmaceutically acceptable auxiliary which is normally used in veterinary formulations.

A related aspect of the invention is a method for preparing a veterinary formulation as described herein, comprising the step of mixing at least one compound of the formula (I) with pharmaceutically acceptable excipients and/or auxiliaries , in particular with pharmaceutically acceptable excipients and/or auxiliaries which are normally used in veterinary formulations.

Another particular aspect of the invention are veterinary formulations, selected from the group of ectoparasiticidal and endoparasiticidal formulations, more particular selected from the group of anthelmintic, antiprotozoal, and arthropodicidal formulations, even more particular selected from the group of nematicidal, platyhelminthicidal, acanthocephalicidal, pentastomicidal, insecticidal, and acaricidal formulations, in accordance with the mentioned aspects, as well as their methods for preparation.

Another aspect refers to a method for treatment of a parasitic infection, in particular an infection by a parasite selected from the group of ectoparasites and endoparasites mentioned herein, by applying an effective amount of a compound of the formula (I) to an animal, in particular a non-human animal, in need thereof.

Another aspect refers to a method for treatment of a parasitic infection, in particular an infection by a parasite selected from the group of ectoparasites and endoparasites mentioned herein, by applying a veterinary formulation as defined herein to an animal, in particular a non-human animal, in need thereof.

Another aspect refers to the use of the compounds of the formula (I) in the treatment of a parasitic infection, in particular an infection by a parasite selected from the group of ectoparasites and endoparasites mentioned herein, in an animal, in particular a non-human animal.

In the present context of the animal health or veterinary field, the term "treatment" includes prophylactic, metaphylactic or therapeutical treatment.

In a particular embodiment, mixtures of at least one compound of the formula (I) with other active ingredients, particularly with endo- and ectoparasiticides, for the veterinary field are provided herewith.

In the field of animal health "mixture" not only means that two (or more) different active ingredients are formulated in a joint formulation and are accordingly applied together but also refers to products which comprise separate formulations for each active compound. Accordingly, if more than two active compounds are to be applied, all active compounds may be formulated in a joint formulation or all active compounds may be formulated in separate formulations; also feasible are mixed forms where some of the active compounds are formulated jointly and some of the active compounds are formulated separately. Separate formulations allow the separate or successive application of the active compounds in question.

The active compounds specified herein by their common names are known and described, for example, in the Pesticide Manual (see above) or can be searched in the internet (e.g. http://www.alanwood.net/pesticides).

Exemplary active ingredients from the group of ectoparasiticides, as mixing partners, include, without limitation insecticides and acaricides listed in detail above. Further active ingredients which may be used are listed below following the aforementioned classification which is based on the current IRAC Mode of Action Classification Scheme: (1) Acetylcholinesterase (AChE) inhibitors; (2) GABA-gated chloride channel blockers; (3) Sodium channel modulators; (4) Nicotinic acetylcholine receptor (nAChR) competitive modulators; (5) Nicotinic acetylcholine receptor (nAChR) allosteric modulators; (6) Glutamate-gated chloride channel (GluCl) allosteric modulators; (7) Juvenile hormone mimics; (8) Miscellaneous non-specific (multi-site) inhibitors; (9) Modulators of Chordotonal Organs; (10) Mite growth inhibitors; (12) Inhibitors of mitochondrial ATP synthase, such as, ATP disruptors; (13) Uncouplers of oxidative phosphorylation via disruption of the proton gradient; (14) Nicotinic acetylcholine receptor channel blockers; (15) Inhibitors of chitin biosynthesis, type 0; (16) Inhibitors of chitin biosynthesis, type 1; (17) Moulting disruptor (in particular for Diptera, i.e. dipterans); (18) Ecdysone receptor agonists; (19) Octopamine receptor agonists; (21) Mitochondrial complex I electron transport inhibitors; (25) Mitochondrial complex II electron transport inhibitors; (20) Mitochondrial complex III electron transport inhibitors; (22) Voltage-dependent sodium channel blockers; (23) Inhibitors of acetyl CoA carboxylase; (28) Ryanodine receptor modulators; (30) GABA-gated chloride channel allosteric modulators.

Active compounds with unknown or non-specific mode of action, e.g., fentrifanil, fenoxacrim, cycloprene, chlorobenzilate, chlordimeform, flubenzimine, dicyclanil, amidoflumet, quinomethionate, triarathene, clothiazoben, tetrasul, potassium oleate, petroleum, metoxadiazone, gossyplure, flutenzin, bromopropylate, cryolite;

Compounds from other classes, e.g. butacarb, dimetilan, cloethocarb, phosphocarb, pirimiphos (-ethyl), parathion (-ethyl), methacrifos, isopropyl o-salicylate, trichlorfon, tigolaner, sulprofos, propaphos, sebufos, pyridathion, prothoate, dichlofenthion, demeton-S-methylsulphone, isazofos, cyanofenphos, dialifos, carbophenothion, autathiofos, aromfenvinfos (-methyl), azinphos (-ethyl), chlorpyrifos (-ethyl), fosmethilan, iodofenphos, dioxabenzofos, formothion, fonofos, flupyrazofos, fensulfothion, etrimfos;
organochlorines, e.g. camphechlor, lindane, heptachlor; or phenylpyrazoles, e.g. acetoprole, pyrafluprole, pyriprole, vaniliprole, sisapronil; or isoxazolines, e.g. sarolaner, afoxolaner, lotilaner, fluralaner;
pyrethroids, e.g. (cis-, trans-), metofluthrin, profluthrin, flufenprox, flubrocythrinate, fubfenprox, fenfluthrin, protrifenbute, pyresmethrin, RU15525, terallethrin, cis-resmethrin, heptafluthrin, , bioethanomethrin, biopermethrin, fenpyrithrin, cis-cypermethrin, cis-permethrin, clocythrin, cyhalothrin (lambda-), chlovaporthrin, or halogenated carbonhydrogen compounds (HCHs),
neonicotinoids, e.g. nithiazine
dicloromezotiaz, triflumezopyrim
macrocyclic lactones, e.g. nemadectin, ivermectin, latidectin, moxidectin, selamectin, eprinomectin, doramectin, emamectin benzoate; milbemycin oxime
triprene, epofenonane, diofenolan;

Biologicals, hormones or pheromones, for example natural products, e.g. thuringiensin, codlemone or neem components
dinitrophenols, e.g. dinocap, dinobuton, binapacryl;
benzoylureas, e.g. fluazuron, penfluron,
amidine derivatives, e.g. chlormebuform, cymiazole, demiditraz
Bee hive varroa acaricides, for example organic acids, e.g. formic acid, oxalic acid.

Exemplary active ingredients from the group of endoparasiticides, as mixing partners, include, without limitation, anthelmintically active compounds and antiprotozoal active compounds.

Anthelmintically active compounds, including, without limitation, the following nematicidally, trematicidally and/or cestocidally active compounds:
from the class of macrocyclic lactones, for example: eprinomectin, abamectin, nemadectin, moxidectin, doramectin, selamectin, lepimectin, latidectin, milbemectin, ivermectin, emamectin, milbemycin;
from the class of benzimidazoles and probenzimidazoles, for example: oxibendazole, mebendazole, triclabendazole, thiophanate, parbendazole, oxfendazole, netobimin, fenbendazole, febantel, thiabendazole, cyclobendazole, cambendazole, albendazole-sulphoxide, albendazole, flubendazole;
from the class of depsipeptides, preferably cyclic depsipetides, in particular 24-membered cyclic depsipeptides, for example: emodepside, PF1022A;
from the class of tetrahydropyrimidines, for example: morantel, pyrantel, oxantel;
from the class of imidazothiazoles, for example: butamisole, levamisole, tetramisole;
from the class of aminophenylamidines, for example: amidantel, deacylated amidantel (dAMD), tribendimidine;
from the class of aminoacetonitriles, for example: monepantel;
from the class of paraherquamides, for example: paraherquamide, derquantel;
from the class of salicylanilides, for example: tribromsalan, bromoxanide, brotianide, clioxanide, closantel, niclosamide, oxyclozanide, rafoxanide;
from the class of substituted phenols, for example: nitroxynil, bithionol, disophenol, hexachlorophene, niclofolan, meniclopholan;
from the class of organophosphates, for example: trichlorfon, naphthalofos, dichlorvos/DDVP, crufomate, coumaphos, haloxon;
from the class of piperazinones / quinolines, for example: praziquantel, epsiprantel;
from the class of piperazines, for example: piperazine, hydroxyzine;
from the class of tetracyclines, for example: tetracyclin, chlorotetracycline, doxycyclin, oxytetracyclin, rolitetracyclin;
from diverse other classes, for example: bunamidine, niridazole, resorantel, omphalotin, oltipraz, nitroscanate, nitroxynile, oxamniquine, mirasan, miracil, lucanthone, hycanthone, hetolin, emetine, diethylcarbamazine, dichlorophen, diamfenetide, clonazepam, bephenium, amoscanate, clorsulon.

Antiprotozoal active compounds, including, without limitation, the following active compounds:
from the class of triazines, for example: diclazuril, ponazuril, letrazuril, toltrazuril;
from the class of polylether ionophore, for example: monensin, salinomycin, maduramicin, narasin;
from the class of macrocyclic lactones, for example: milbemycin, erythromycin;
from the class of quinolones, for example: enrofloxacin, pradofloxacin;
from the class of quinines, for example: chloroquine;
from the class of pyrimidines, for example: pyrimethamine;
from the class of sulfonamides, for example: sulfaquinoxaline, trimethoprim, sulfaclozin;
from the class of thiamines, for example: amprolium;
from the class of lincosamides, for example: clindamycin;
from the class of carbanilides, for example: imidocarb;
from the class of nitrofuranes, for example: nifurtimox;
from the class of quinazolinone alkaloids, for example: halofuginon;
from diverse other classes, for example: oxamniquin, paromomycin;
from the class of vaccines or antigenes from microorganisms, for example: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

All named mixing partners can, if their functional groups enable this, optionally form salts with suitable bases or acids.

### Vector control

The compounds of the formula (I) can also be used in vector control. For the purpose of the present invention, a vector is an arthropod, in particular an insect or arachnid, capable of transmitting pathogens such as, for example, viruses, worms, single-cell organisms and bacteria from a reservoir (plant, animal, human, etc.) to a host. The pathogens can be transmitted either mechanically (for example trachoma by non-stinging flies) to a host, or by injection (for example malaria parasites by mosquitoes) into a host.

Examples of vectors and the diseases or pathogens they transmit are:
1) Mosquitoes
   - Anopheles: malaria, filariasis;
   - Culex: Japanese encephalitis, other viral diseases, filariasis, transmission of other worms;
   - Aedes: yellow fever, dengue fever, other viral diseases, filariasis;
   - Simuliidae: transmission of worms, in particular Onchocerca volvulus;
   - Psychodidae: transmission of leishmaniasis
2) Lice: skin infections, epidemic typhus;
3) Fleas: plague, endemic typhus, cestodes;
4) Flies: sleeping sickness (trypanosomiasis); cholera, other bacterial diseases;
5) Mites: acariosis, epidemic typhus, rickettsialpox, tularaemia, Saint Louis encephalitis, tick-borne encephalitis (TBE), Crimean-Congo haemorrhagic fever, borreliosis;
6) Ticks: borellioses such as Borrelia burgdorferi sensu lato., Borrelia duttoni, tick-borne encephalitis, Q fever (Coxiella burnetii), babesioses (Babesia canis canis), ehrlichiosis.

Examples of vectors in the sense of the present invention are insects, for example aphids, flies, leafhoppers or thrips, which are capable of transmitting plant viruses to plants. Other vectors capable of transmitting plant viruses are spider mites, lice, beetles and nematodes.

Further examples of vectors in the sense of the present invention are insects and arachnids such as mosquitoes, in particular of the genera Aedes, Anopheles, for example A. gambiae, A. arabiensis, A. funestus, A. dirus (malaria) and Culex, psychodids such as Phlebotomus, Lutzomyia, lice, fleas, flies, mites and ticks capable of transmitting pathogens to animals and/or humans.

Vector control is also possible if the compounds of the formula (I) are resistance-breaking.

Compounds of the formula (I) are suitable for use in the prevention of diseases and/or pathogens transmitted by vectors. Thus, a further aspect of the present invention is the use of compounds of the formula (I) for vector control, for example in agriculture, in horticulture, in gardens and in leisure facilities, and also in the protection of materials and stored products.

### Protection of industrial materials

The compounds of the formula (I) are suitable for protecting industrial materials against attack or destruction by insects, for example from the orders Coleoptera, Hymenoptera, Isoptera, Lepidoptera, Psocoptera and Zygentoma.

Industrial materials in the present context are understood to mean inanimate materials, such as preferably plastics, adhesives, sizes, papers and cards, leather, wood, processed wood products and coating compositions. The use of the invention for protecting wood is particularly preferred.

In a further embodiment, the compounds of the formula (I) are used together with at least one further insecticide and/or at least one fungicide.

In a further embodiment, the compounds of the formula (I) are present as a ready-to-use pesticide, i.e. they can be applied to the material in question without further modifications. Suitable further insecticides or fungicides are in particular those mentioned above.

Surprisingly, it has also been found that the compounds of the formula (I) can be employed for protecting objects which come into contact with saltwater or brackish water, in particular hulls, screens, nets, buildings, moorings and signalling systems, against fouling. Likewise, the compounds of the formula (I), alone or in combinations with other active compounds, can be used as antifouling agents.

### Control of animal pests in the hygiene sector

The compounds of the formula (I) are suitable for controlling animal pests in the hygiene sector. In particular, the invention can be applied in the domestic sector, in the hygiene sector and in the protection of stored products, especially for controlling insects, arachnids, ticks and mites encountered in enclosed spaces such as dwellings, factory halls, offices, vehicle cabins, animal husbandries. For controlling animal pests, the compounds of the formula (I) are used alone or in combination with other active compounds and/or auxiliaries. They are preferably used in domestic insecticide products. The compounds of the formula (I) are effective against sensitive and resistant species, and against all developmental stages.

These pests include, for example, pests from the class Arachnida, from the orders Scorpiones, Araneae and Opiliones, from the classes Chilopoda and Diplopoda, from the class Insecta the order Blattodea, from the orders Coleoptera, Dermaptera, Diptera, Heteroptera, Hymenoptera, Isoptera, Lepidoptera, Phthiraptera, Psocoptera, Saltatoria or Orthoptera, Siphonaptera and Zygentoma and from the class Malacostraca the order Isopoda.

They are used, for example, in aerosols, pressure-free spray products, for example pump and atomizer sprays, automatic fogging systems, foggers, foams, gels, evaporator products with evaporator tablets made of cellulose or plastic, liquid evaporators, gel and membrane evaporators, propeller-driven evaporators, energy-free, or passive, evaporation systems, moth papers, moth bags and moth gels, as granules or dusts, in baits for spreading or in bait stations.

### Abbreviations and Symbols

- AcOH:: acetic acid
- aq.:: aqueous
- br.:: broad
- d:: doublet
- DCC:: N,N'-dicyclohexylcarbodiimide
- DIPEA:: N,N-diisopropylethylamine
- DMF:: N,N-dimethylformamide
- DMSO:: dimethylsulfoxide
- ee:: enantiomeric excess
- eq.:: equivalent
- ES:: electrospray ionization
- Et₃N: triethylamine
- EtOAc:: ethyl acetate
- hr(s): hour(s)
- HATU:: 1-[bis(dimethylamino)methylene]-1H-1,2,3-triazolo[4,5-b]pyridinium-3-oxid hexafluorophosphate
- HOBt:: 1-hydroxybenzotriazole hydrate
- HPLC:: high performance liquid chromatography
- *i*PrOH:: isopropanol
- *J*:: coupling constant
- LCMS:: liquid chromatography-mass spectrometry
- *m*/*z*:: mass-to-charge ratio
- M:: molarity
- m:: multiplet
- mCPBA: meta-chloroperoxybenzoic acid
- MeCN: acetonitrile
- MeOH:: methanol
- NaH₂PO₄: monosodium phosphate
- NaOH: sodium hydroxide
- Na₂SO₄: sodium sulfate
- NH₄Cl: ammonium chloride
- NMR:: nuclear magnetic resonance
- q:: quartet
- r. t.:: room temperature
- Rt:: retention time
- s:: singlet
- sat.:: saturated
- T:: temperature
- t:: triplet
- T3P^{®}:: propylphosphonic anhydride
- THF:: tetrahydrofuran
- TMSOK: potassium trimethylsilanolate
- wt.:: weight
- δ:: chemical shift
- λ:: wavelength

### Description of the processes and intermediates

Compounds of formula I' may be prepared as illustrated in the following scheme 1 where R¹, R², R^{3a}, R^{3b}, R⁴, R⁵ and Y are as previously defined and X stands for OH or Cl.

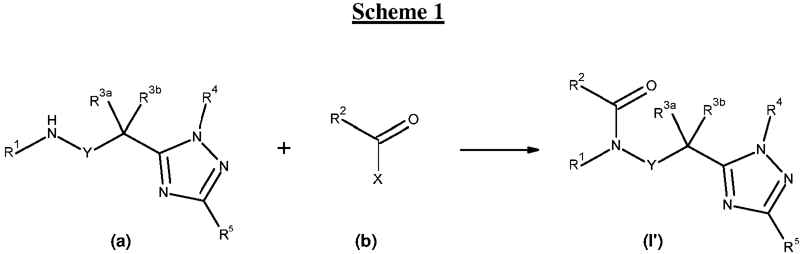

X = OH: An azole compound of formula (a) is reacted with a carboxylic acid of formula (b) (X = OH) to form compounds of formula I'. For example, a mixture of an azole of formula (a), a carboxylic acid of formula (b) (X = OH), a suitable coupling reagent, such as T3P^{®}, HATU, DCC or HOBt, a suitable base such as triethylamine or DIPEA, in a suitable solvent, such as ethyl acetate or DMF are mixed at temperatures ranging from around 0 to 100 °C to provide compounds of formula I' which may then be isolated and, if necessary and desired, purified using techniques well known in the art, such as chromatography.

X = Cl: An azole compound of formula (a) is reacted with a carboxylic acid chloride of formula (b) (X = Cl) to form compounds of formula I'. For example, a mixture of an azole of formula (a), a carboxylic acid chloride of formula (b) (X = Cl), a suitable base such as triethylamine or DIPEA, in a suitable solvent, such as dichloromethane or THF are mixed at temperatures ranging from around 0 to 100 °C to provide compounds of formula I' which may then be isolated and, if necessary and desired, purified using techniques well known in the art, such as chromatography.

Carboxylic acids of formula (b) (X = OH) and carboxylic acid chlorides of formula (b) (X = Cl) are commercially available or may be synthesized by methods known to a person skilled in the state of the art.

For example, the carboxylic acids can be synthesized in analogy to WO 2016198507, WO 2015084936, WO 2015148354 and WO 2015148373.

Compounds of formula I"d may be prepared as illustrated in the following scheme 2, where R¹, R², R^{3a}, R^{3b}, R⁵, R⁴¹ and Y are as previously defined. T is R⁴ as previously described and at least substituted with one NO₂, -NH₂, -NHA¹ or -NA¹A² group respectively. LG is suitable leaving group and A¹ and A² represent R⁴¹Z-, R⁴¹Y¹-, R⁴¹OCO-, R⁴⁴OCO-, R⁴² and R⁴³Y¹-. Z and Y¹ are CO or SO₂. R⁴², R⁴³ and R⁴⁴ are in each case optionally substituted C₁-C₆alkyl, C₁-C₆haloalkyl, C₂-C₆alkenyl, C₂-C₆haloalkenyl, C₂-C₆alkynyl, C₂-C₆haloalkynyl, C₃-C₆cycloalkyl, C₃-C₆cycloalkyl-C₁-C₆alkyl, phenyl-C₁-C₆alkyl, naphthyl-C₁- C₆alkyl;

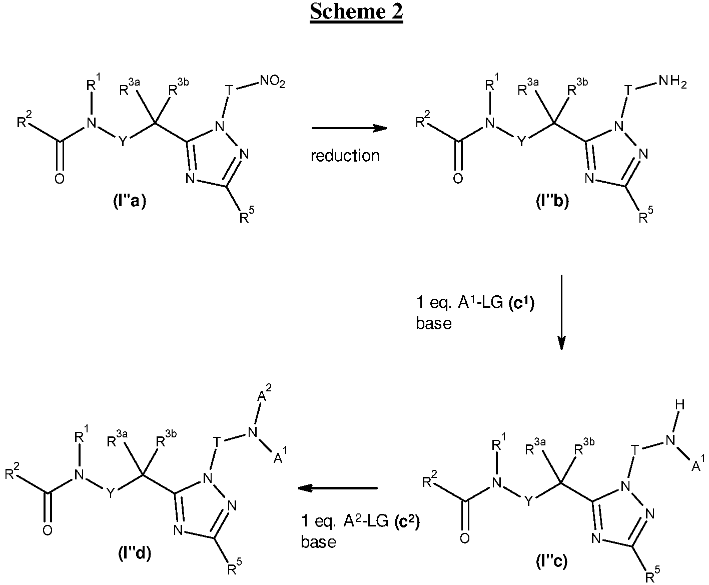

A nitro compound of formula (I"a) is converted into the respective amino compound of formula (I"b) under reducing conditions, likewise with hydrogen and palladium on charcoal in a suitable solvent like THF or ethanol (European Journal of Medicinal Chemistry, 158, 322-333; 2018), with tin(II) chloride and HCl in a suitable solvent like ethanol (WO 2018085247), with iron powder and HCl in a suitable solvent like ethanol (WO 2017216293) or with iron powder in a mixture of acetic acid and ethanol. The resulting amino compound (I"b) reacts, in the presence of a suitbale base such as DIPEA or potassium carbonate, with chloroformates or acylation, benzoylation, sulfonlyation or alkylation reagents of formula A¹-LG (c¹) or A²-LG (c²). If one equivalent of A¹-LG (c¹) is used compounds of formula (I"c) are obtained. Further treatment with one equivalent of A²-LG (c²) yields compounds of formula (I"d). The obtained compounds of formula (I"c) and (I"d) are then if necessary and desired, purified using techniques well known in the art, such as chromatography.

Compounds of formula I"e may be prepared as illustrated in the following scheme 3, where R¹, R², R^{3a}, R^{3b}, R⁵, R⁴¹, R⁴², R⁴⁵ and Y are as previously defined, wherein T is R⁴ as previously described and at least substituted with one -CO₂alkyl-group, -COOH or -CONE¹E² group respectively. -NE¹E² represents - NR⁴¹R⁴², -NR⁴¹R⁴¹, -NR⁴⁵NR⁴¹R⁴⁵, -NR⁴¹NR⁴⁵R⁴⁵, -NR⁴⁵NR⁴⁵R⁴⁵, -NR⁴¹NR⁴⁵R⁴¹, -NR⁴¹OR⁴⁵, -NR⁴⁵OR⁴⁵ and -NR⁴⁵OR⁴¹. R⁴¹ and R⁴² together with the nitrogen atom to which they are attached may also represent a heterocyclyl as previously defined.

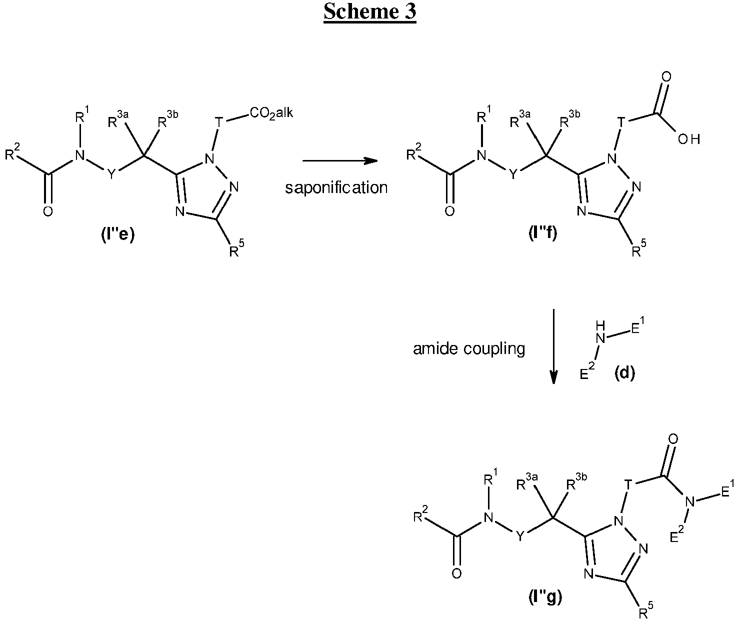

An ester compound of formula (I"e) is saponified to obtain the respective carboxylic acid compound of formula (I"f) followed by an amide coupling step with amines, hydrazines or substituted hydroxylamines of formula (d) to obtain amides of formula (I"g) by methods known to a person skilled in the state of the art.

For example, a mixture of an amine, hydrazine or substituted hydroxylamine of formula (d), a carboxylic acid (I"f), a suitable coupling reagent, such as T3P^{®}, HATU, DCC or HOBt, a suitable base such as triethylamine or DIPEA, in a suitable solvent such as ethyl acetate or DMF are mixed at temperatures ranging from around 0 to 100 °C to provide compounds of formula (I"g) which may then be isolated and, if necessary and desired, purified using techniques well known in the art, such as chromatography.

Scheme 4 illustrates the preparation 3-N-Boc-amino or 3-amino-1,2,4-triazoles (I''h) and (I"i) starting with 3-N-Boc-amino-1,2,4-triazole containing amines (j) (see examples I-001 to I-008). R¹ is hydrogen, R^{3a} is methyl, R^{3b} is hydrogen, X is oxygen, Y is a direct bond, R⁵ is N-Boc-amino or amino and R² as well as R⁴ have the meanings given in of formula I.

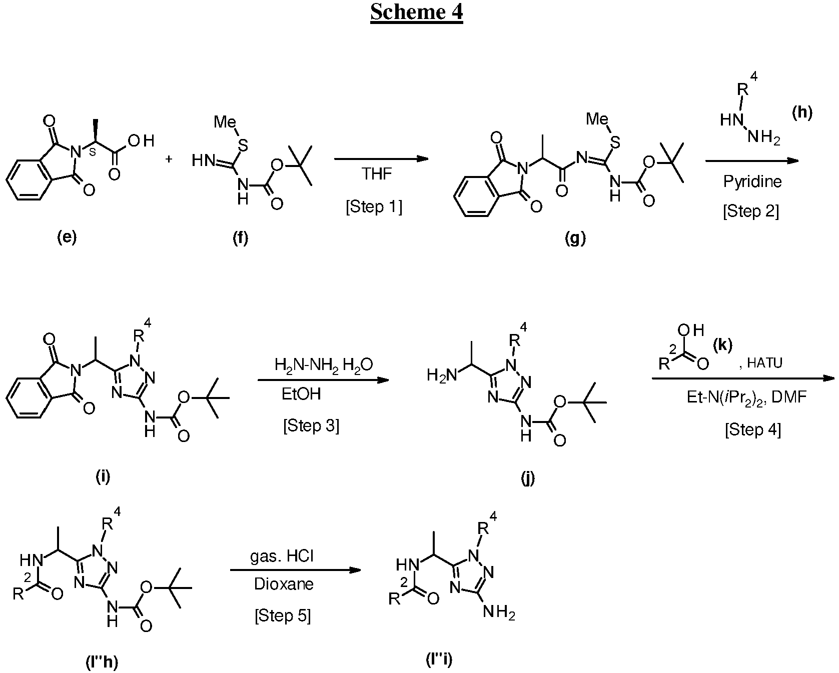

In a first step, (α*S*)-1,3-dihydro-α-methyl-1,3-dioxo-2*H*-isoindole-2-acetic acid (e) (Pht-Ala-OH purchased from ABCR) reacts with 1-N-Boc-2-methyl-isothiourea (f) (purchased from ABCR) in the presence of a base and the coupling reagent HATU to form the *N*-acylated 1-N-Boc-2-methyl-isothiourea (g). Thereby a partially or fully racemization is possible. In a second step the cyclization occurs with R⁴-substituted hydrazines (h) and in the presence of a base, like pyridine, as described in WO 2014009425 A1 to form the 3-N-Boc-amino-substituted 1,2,4-triazoles of formula (i). In the third step the phthalimide protecting group is cleaved with hydrazine hydrate in a suitable solvent, like ethanol, as described in WO 2018/086605. Then, in a fourth step, the obtained N-Boc-protected amines (j) react with different carboxylic acids (k) to form the compounds (I"h). After *N*-Boc-deprotection in the fifth step under acidic conditions (HCl in dioxane) 3-amino- substituted 1,2,4-triazoles (I"i) are obtained as described in scheme 4.

Scheme 5 illustrates the preparation of amines (ja) containing 3-alkyl- or 3-haloalkyl Boc protected amino rests which are intermediates in the synthesis of, e.g., example I-009.

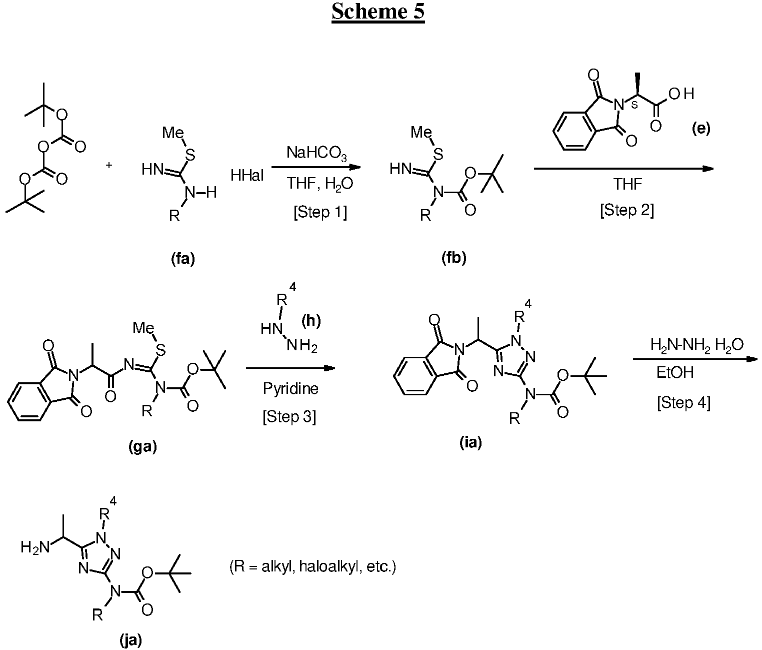

In a first step, *tert*-butoxycarbonyl *tert-butyl* carbonate reacts with a salt of *N*-alkyl or *N*-haloalkyl methyl-isothiourea hydrohalogenide (fa) e.g. hydroiodide, in presence of NaHCO₃ to form the *N*-alkyl or *N-*haloalkyl *tert-*butyl *N*-(methylsulfanylcarbonimidoyl)carbamates (fb) which reacts with (αS)-1,3-dihydro-α-methyl-1,3-dioxo-2*H*-isoindole-2-acetic acid (e) in a second step in the presence of a base and the coupling reagent HATU to form the *N*-acylated *N*-alkyl or *N*-haloalkyl *tert*-butyl *N-*(methylsulfanylcarbonimidoyl) carbamates (ga). Thereby a partially or fully racemization is possible. In a third step the cyclization of (ga) occurs with R⁴-substituted hydrazines (h) in the presence of a base, like pyridine, as described in WO 2014009425 to form the 1,2,4-triazoles of formula (ia). In the last step the phthalimide protecting group is cleaved with hydrazine hydrate in a suitable solvent, like ethanol, as described in WO 2018/086605 to form the corresponding amines (ja) wearing 3-alkyl- or 3-haloalkyl-3-*N*-Boc-amino groups as a substituents at the triazole core.

The *N*-methyl-carbamimidothioic acid methyl ester are either commercially available or may be synthesized by methods known to the person skilled in the art.

For example:
For *N*-Methyl-carbamimidothioic acid methyl ester, hydriodide (1:1), *see* CAS-no: 41306-45-0 (ABCR, WO 2006138350); R = methyl.

For *N*-Ethyl-carbamimidothioic acid methyl ester, hydriodide (1:1), *see* CAS-no: 7204-32-2 (Milestone Pharmtech Product List, WO 2009115515); R = ethyl.

For N-Propyl-carbamimidothioic acid methyl ester, *see* CAS-no: 925-59-7 (Chemieliva Pharmaceutical Product List; S. Pockes, et al., ACS Omega, 3(3), 2865-2882; 2018); R = propyl.

For 1-Methylethyl-carbamimidothioic acid methyl ester, monohydrochloride, *see* CAS-no: 5734-00-9, Shanghai Chemhere Product List; S. Pockes, et al., ACS Omega, 3(3), 2865-2882; 2018); R = *iso-*propyl*.*

For 2-Propenyl-carbamimidothioic acid methyl ester, *see* CAS-no: 50420-33-2, FCH Group Reagents for Synthesis); R = allyl.

Scheme 6 illustrates the synthesis of amines (jb) wearing 3-alkylsulfonylamino, 3-cycloalkylsulfonylamino or 3-halogenalkylsulfonylamino groups as substituents at the triazole ring, which are used as intermediates.

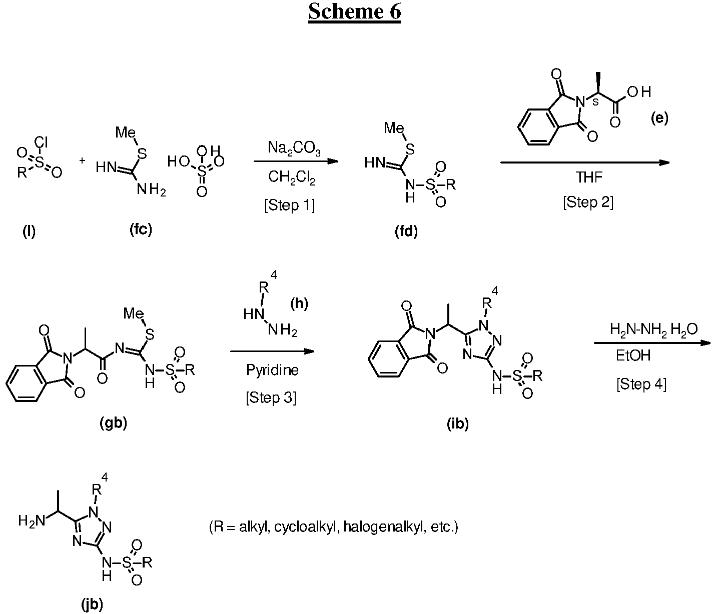

In a first step, alkyl, cycloalkyl or halogenalkylsulfonyl chlorides (1) react with methyl carbamimidothioate sulfate (fc) in presence of Na₂CO₃ to form the 1-*N-*alkyl, cycloalkyl or halogenalkylsulfonyl-2-methyl-isothioureas (fd) which reacts with (αS)-1,3-dihydro-α-methyl-1,3-dioxo-2*H*-isoindole-2-acetic acid (e) in a second step in the presence of a base and the coupling reagent HATU to form the *N*-acylated 1-*N*-alkyl, cycloalkyl or halogenalkylsulfonyl-2-methyl-isothioureas (gb). Thereby a partially or fully racemization is possible. In a third step the cyclization occurs with R⁴-substituted hydrazines (h) and in the presence of a base, like pyridine, as described in WO 2014009425 to form the 1,2,4-triazoles of formula (ib). In the last step the phthalimide protecting group is cleaved with hydrazine hydrate in a suitable solvent, like ethanol, as described in WO 2018/086605 to form the 3-alkyl, 3-cycloalkyl or 3-halogenalkylsulfonylamino-1,2,4-triazole amines (jb), which can be uses as intermediates (e.g. INT-004 with R⁵ = NH-SO₂CHF₂).

Scheme 7 illustrates the preparation of 3-carbamoylamino, 3-acylamino or 3-sulfonylamino 1,2,4-triazoles (I"k) to (I''m) starting from the examples I-006 to I-008.

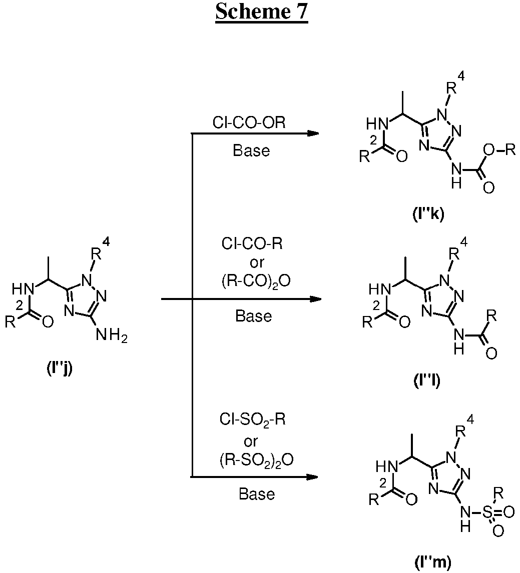

The 3-amino- substituted 1,2,4-triazoles (l"j) react e.g. with aryl or alkyl chloroformates, acyl chlorides or corresponding anhydrides and aryl or alkylsulfonyl chlorides in presence of base and a solvent, to form 3-carbamoylamino, 3-acylamino or 3-sulfonylamino 1,2,4-triazoles (I"k) to (I''m) as exemplified with I-010 (3-*N*-carbamoylamino-1,2,4-triazoles), I-012 to I-024 (3-*N*-acylamino-1,2,4-triazoles) and I-025 (3-*N*-sulfonylamino-1,2,4-triazole).

The aryl or alkyl chloroformates, acyl chlorides or corresponding anhydrides and aryl or alkylsulfonyl chlorides are either commercially available or may be synthesized by methods known to the skilled artisan.

Scheme 8 illustrates the preparation of 3-methylsulfonylmethylen or 3-hetaryl 1,2,4-triazole amines (jc) used for the synthesis of e.g. examples I-027 to I-058.

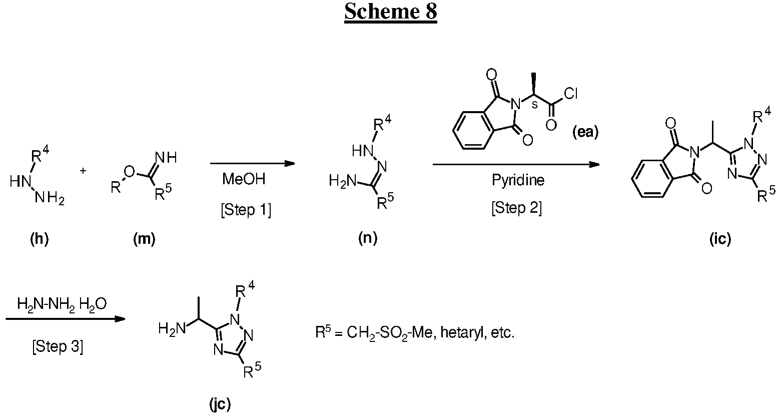

In a first step, a hydrazone amide (n) is formed from R⁴-substituted hydrazines (h) and (m) as described in EP 1099695. In a second step hydrazine amine (n) reacts with (α*S*)-1,3-dihydro-α-methyl-1,3-dioxo-2*H*-isoindole-2-acetyl chloride (ea), prepared from (α*S*)-1,3-dihydro-α-methyl-1,3-dioxo-2*H*-isoindole-2-acetic acid (e) ) and oxalyl chloride according to Tetrahedron: Asymmetry, 21(8), 936-942, 2010, in the presence of a base, like pyridine, as described in EP 1099695 to form triazoles (ic). Thereby a partially or fully racemization is possible. In a third step, the phthalimide protecting group is cleaved with hydrazine hydrate in a suitable solvent, like ethanol, as described in WO 2018086605. In a final step, the obtained 3-methylsulfonylmethylen or 3-hetaryl-1,2,4-triazole amines (jc) react as intermediates INT-005 to INT-011 with different carboxylic acids to form the example compounds, e.g. examples I-027 to I-058 as described in scheme 1.

The imidic acid alkyl ester (m) are either commercially available or may be synthesized by methods known to the skilled artisan.

For example:
For 2-(methylsulfonyl)-ethanimidic acid ethyl ester hydrochloride (1:1), *see* W. Tang, et al., J. Amer. Chem. Soc. 137(18), 5980-5989, 2015 (used for R⁵ = CH₂-SO₂-Me; intermediate INT 005, examples I-027 to I-030)
For 6-chloro-3-pyridinecarboximidic acid methyl ester, *see* WO 2009105500 (used for R⁵ = 6-chloro-3-pyridyl; intermediates INT 006 and INT 008, examples I-031 to I-033)
For 2-pyrazinecarboximidic acid methyl ester, *see* EP 388528 A2 (used for R⁵ = 2-pyrazinyl; intermediates INT 007 and INT 009, examples I-034 to I-040)
For 2-pyrimidinecarboximidic acid methyl ester, *see* WO 2012048129 A2 (used for R⁵ = 2-pyrimidinyl; intermediate INT 010, examples I-051 to I-056)
For 2-furancarboximidic acid methyl ester hydrochloride (1:1), *see* WO 2013066684 (used for R⁵ = 2-furanyl; intermediate INT 011, examples I-057 and I-058)

Scheme 9 illustrates the preparation of amines (jd) substituted with different sulfides at the triazol ring which are used as intermediates in the synthesis of e.g. examples I-059, I-062, I-065, I-067 and I-069.

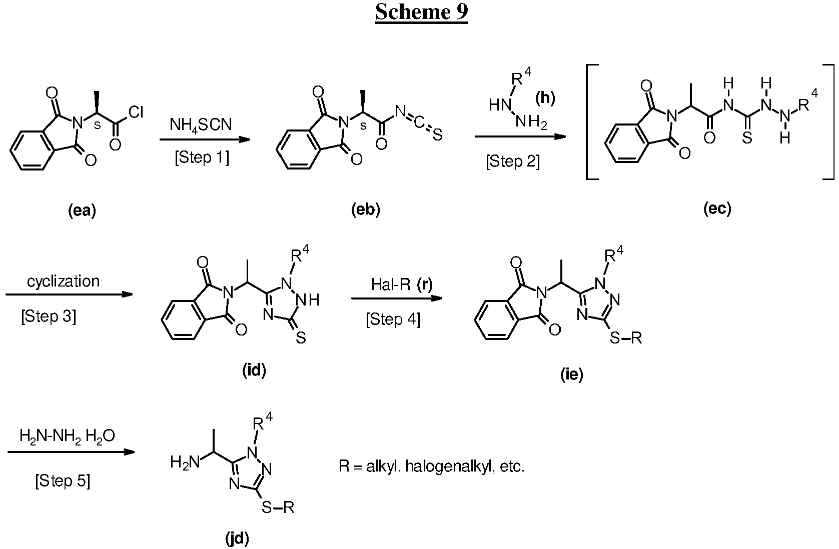

In a first step (αS)-1,3-dihydro-α-methyl-1,3-dioxo-2H-isoindole-2-acetyl chloride (ea), prepared from (αS)-1,3-dihydro-α-methyl-1,3-dioxo-2H-isoindole-2-acetic acid (e) and oxalyl chloride, reacts with the ammonium thiocyanate in acetonitrile at room temperature as described by M. M. Hemdan, et al. Phosphorus, Sulfur and Silicon and the Related Elements 187(2), 181-189, 2012 to form the phthalimidoacyl isothiocyanate (eb), which was used without further purification. In a second step isothiocyanate (eb) reacts with R⁴-substituted hydrazines (h) to form intermediates (ec) which cyclize in situ to intermediates (id) by heating the reaction mixture (*see* M. M. Hemdan, et al. Phosphorus, Sulfur and Silicon and the Related Elements 187(2), 181-189, 2012). Thereby a partially or fully racemization is possible. In a fourth step, the 5-thioxo-1*H*-1,2,4-triazoles of formula (id) react with alkyl or halogen alkyl halides (r) in the presence of a base, like sodium hydride, to form 5-methylsulfanyl-1,2,4-triazoles of formula (ie). In a fifth step, the phthalimide protecting group is cleaved with hydrazine hydrate in a suitable solvent, like ethanol, as described in WO 2018086605. In a final step, the amine intermediates (jd) INT-012 to INT-014 react with different carboxylic acid to form the example compounds, e.g. I-059, I-062, I-065, I-067 and I-069 as described in scheme 1.

Scheme 10 illustrates the preparation of 3-alkylsulfinyl or 3-halogenalkylsulfinyl-1,2,4-triazoles (I"o) and 3-alkylsulfonyl or 3-halogenalkylsulfonyl-1,2,4-triazoles (I''p) starting from 3-alkylthio or 3-halogenalkylthio-1,2,4-triazoles (I"n).

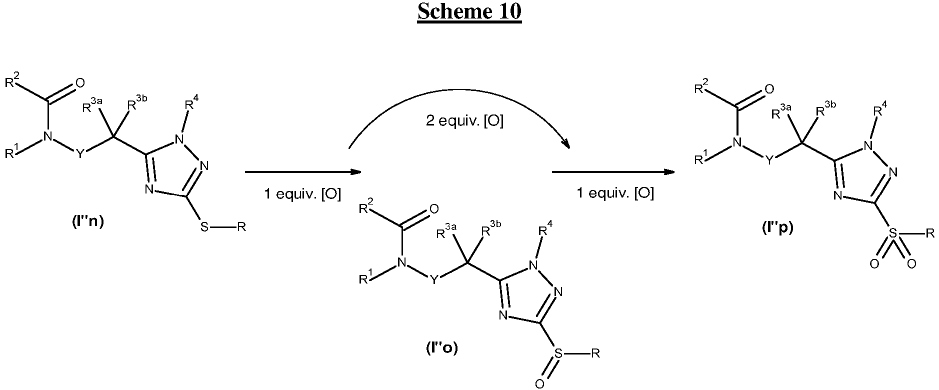

A 3-sulfanyl group (or 3-alkylthio group) containing 1,2,4-triazoles of formula (I''n) reacts with an oxidizing reagent such as 3-chloroperoxybenzoic acid or ruthenium(III) chloride in combination with sodium periodate to form 1,2,4-triazoles substituted with sulfoxides (I"o) or sulfones (I''p), at triazole ring, depending on the equivalents of the oxidizing reagent used, as exemplied by I-060, I-061, I-063, I-064, I-066 and I-068.

Scheme 11 illustrates the preparation of N-substituted 1,2,4-triazole-3-carboxamides (I"o) starting from methyl-1,2,4-triazole-3-carboxylates (q).

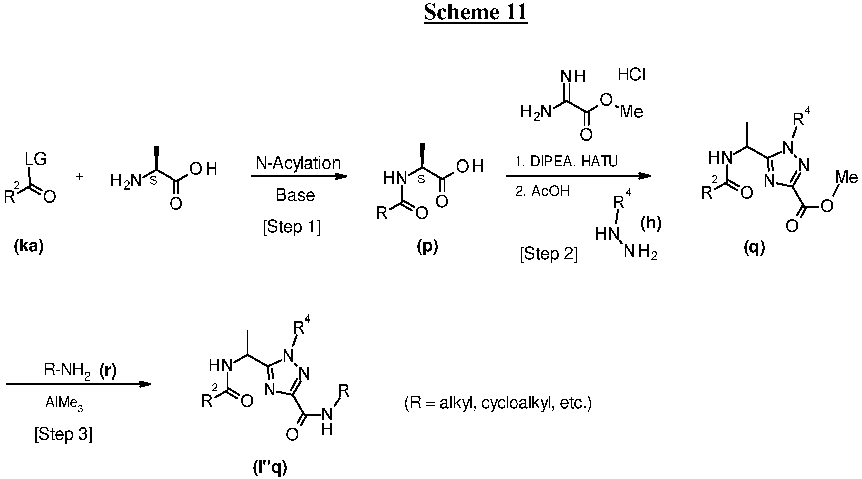

In a first step, amino acids like *L*-alanine react with different leaving group (LG)-activated carboxylic acids (ka) (e.g. LG = halogen) in presence of a base, to form the *N*-acylated *L*-alanines (p) which react in a second step, with methyl 2-amino-2-imino-acetate hydrochloride *(see* CAS-no: 60189-97-1, ABCR) in presence of DIPEA and HATU, followed by cyclization with R⁴-substituted hydrazines (h) in the presence of acetic acid to afford the methyl-1,2,4-triazole-3-carboxylates of formula (q) (see G. M. Castanedo et al. J. Org. Chem., 76(4), 1177-1179, 2011). Thereby a partially or fully racemization is possible. In the third step the *N*-substituted 1,2,4-triazole-3-carboxamides (I''q) were formed by reaction of intermediates q with different amines and aluminium trimethyl (AlMe₃) in a suitable solvent, like dichloromethane or toluene (J. Li, et al., Org. Lett., 14(1), 214-217, 2012) as demonstrated by the examples I-076 to I-079.

The (2*S*)-2-[[substituted-benzoyl]amino]propanoic acids (p) (*N*-acylated *L*-alanines) are either commercially available or may be synthesized by methods known to the skilled artisan (e.g. WO 2017148967).

For example:
For (2*S*)-2-{[3-bromo-5-chloro-phenyl]formamido}propanoic acid, *see* CAS-no: 1689965-18-1 (Aurora Building Blocks 3)
For (2*S*)-2-{[3,5-dichloro-phenyl]formamido}propanoic acid, *see* CAS-no: 1101188-46-8 (Aurora Building Blocks 3).
For (2*S*)-2-{[3-chloro-5-(trifluoromethyl)phenyl]formamido}propanoic acid, *see* CAS-no: 1938896-54-8 (FCH Group Premium Screening Compounds).

Scheme 12 illustrates the preparation of 1,2,4-triazole-3-carboxylic acids (I"r), 1,2,4-triazole-3-carboxamides (I''qa) and 3-cyano-1,2,4-triazoles (I"s) starting from methyl-1,2,4-triazole-3-carboxylates (q).

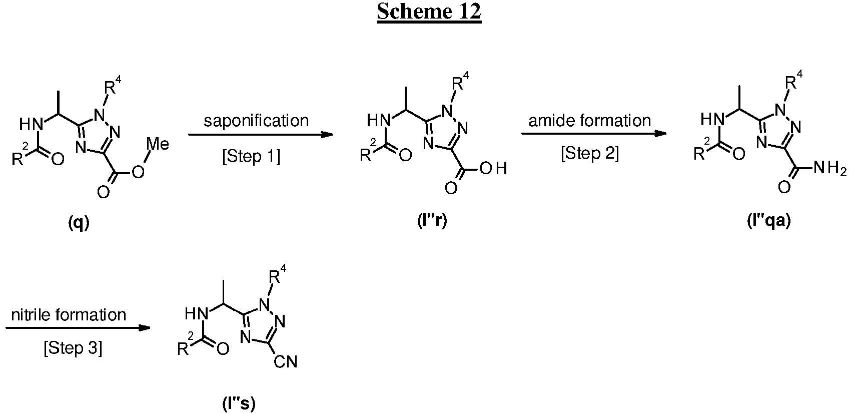

In a first step methyl-1,2,4-triazole-3-carboxylates of formula (q) are saponified with NaOH in a mixture of MeOH and water to form the corresponding 1,2,4-triazole-3-carboxylic acids (I''r). In a second step, the 1,2,4-triazole-3-carboxylic acids (I''r) react with HATU in DMF and ammonium chloride in presence of a base (DIEA) at room temperature to give1,2,4-triazole-3-carboxamides of formula (I''qa) which can be transformed then to the corresponding 3-cyano-1,2,4-triazoles of formula (I''s) by using dehydrating agents such as trifluoroacetic acid anhydride (TFAA) and a base (triethylamine or pyridine; see WO 2009137742) as demonstrated by the examples I-080 to 1-081.

Scheme 13 illustrates the alternative preparation of 3-cyano-1,2,4-triazoles (I"s) starting from 3-N-Boc-amino or 3-amino-1,2,4-triazoles (I"h) and (I"i).

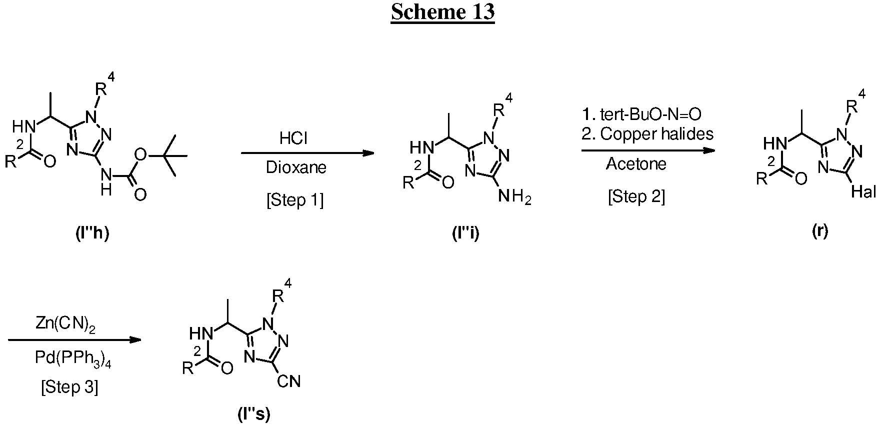

After *N*-Boc-deprotection of the 1,2,4-triazoles of formula (I"h) under acidic conditions (HCl in dioxane) the corresponding 3-amino-1,2,4-triazoles (I"i) can be treated with *tert-butyl* nitrite and afterwards with a copper halides as salts, like CuCl₂ (Hal = Cl) described by N. Desroy et al., J. Med. Chem. 2013, 56, 1418-1430, CuBr₂ (Hal = Br) described in JP-Pat. 2010070503 A, CuI/I₂ mixture (Hal = I) as described by K. Pchalek and M. P. Hay J. Org. Chem., 2006, 71, 6530-6535, or with diiodomethane (Hal = I) as described by N. R. Norcross et al. J. Med. Chem., 2016, 59(13), 6101-6120 to form the 3-halogen-substituted 1,2,4-triazoles (r). In a third step, the halogen can be replaced by the -CN group in the presence of zinc cyanide and tetrakis(triphenylphosphin)palladium(0) as described in WO 2018011628, forming the 3-cyano-1,2,4-triazoles (I''s) as demonstrated by the example I-071.

Scheme 14 illustrates the preparation of 3-aryl or 3-arylalkyl containing amines (je) (see INT-015 to INT-017) as used as intermediates for the synthesis of e.g. examples I-082 to I-085.

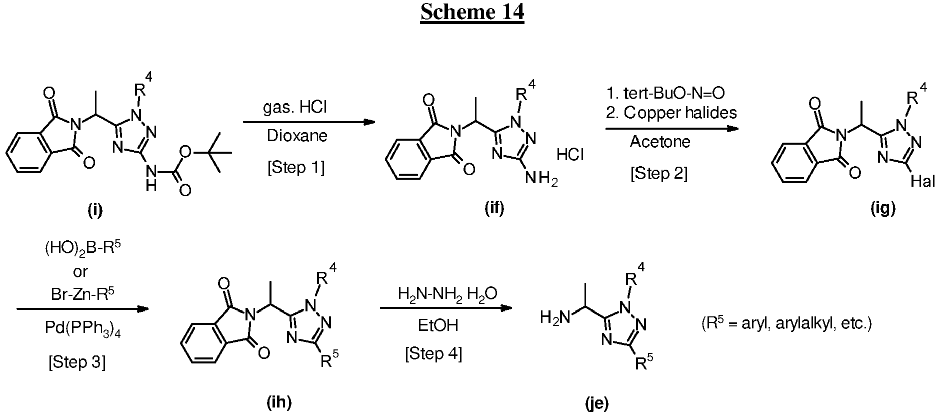

In the same way as described in Scheme 13 the phthalimide protected 3-*N*-Boc-amino-1,2,4-triazoles of formula (i) can be transformed in a two-step reaction to form the 3-halogen-1,2,4-triazoles of formula (ig). In the third step, cross-coupling reactions (L.-Ch. Campeau and N. Hazari, Organometallics 38(1), 3-35, 2019; J. Carreras, et al., Chemistry - An Asian Journal 14(3), 329-343, 2019) in 3-position of the 1,2,4-triazoles (ig) are possible with aryl boronic acids as described in WO 2016081807 or arylalkyl zinc bromides as described in WO 2015164573 in the presence of tetrakis(triphenylphosphine) palladium(0), leading to R⁵-substituted 1,2,4-triazoles (ih). In a fourth step, the phthalimide protecting group is cleaved with hydrazine hydrate in a suitable solvent, like ethanol, as described in WO 2018086605. In a final step, the obtained 3-aryl or 3-arylalkyl containing 1,2,4-triazole amine intermediates (je) (INT-015 to INT-017) react with different carboxylic acid to form the example compounds, e.g. I-082 as described in scheme 1.

The boronic acids or arylalkyl zinc bromides are either commercially available or may be synthesized by methods known to the skilled artisan.

For bromo(phenylmethyl)-zinc, 0.5M in THF, *see* CAS-no: 62673-31-8 (ABCR).

For bromo(1-phenylethyl)-zinc, 0.5M in THF, see CAS-no: 85459-20-7 (ABCR).

For bromo[(2,6-difluorophenyl)methyl]-zinc, 0.5M in THF, see CAS-no: 307496-33-9 (ABCR).

For bromo[(3,5-difluorophenyl)methyl]-zinc, 0.5M in THF, see CAS-no: 308796-30-7 (ABCR).

For bromo[2-(1,1-dimethylethoxy)-2-oxoethyl]-zinc, 0.5M in diethyl ether, see CAS-no: 51656-70-3 (ABCR).

The preparation and use examples which follow illustrate the invention without limiting it.

Scheme 15 illustrates the preparation 3-haloalkylamino or 3-alkylamino-1,2,4-triazoles (I"t) starting with 3-alkylamino-1,2,4-triazole or 3-haloalkylamino-1,2,4-triazole containing Boc-protected amines (ib) as intermediates (*see* INT-020 and INT-021).

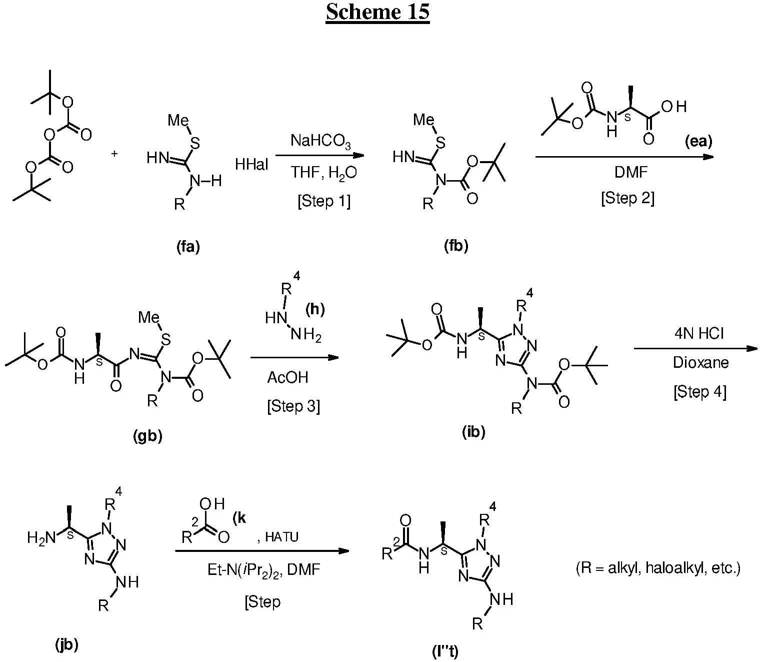

In a first step, di-*tert*-butyl dicarbonate reacts with a salt of *N*-alkyl or *N*-haloalkyl methyl-isothiourea hydrohalogenides (fa) e.g. available as hydroiodide salt, in presence of a base like NaHCO₃, to form *N-*alkyl or *N*-haloalkyl *tert-butyl N*-(methylsulfanylcarbonimidoyl)carbamates (fb) which react with*N*-Boc-alanine (ea) in a second step in the presence of a base and the coupling reagent HATU to form the *N-*acylated *N*-alkyl or *N*-haloalkyl *tert-*butyl *N*-(methylsulfanylcarbonimidoyl) carbamates (gb), which can be used without purification. In a third step the cyclization of (gb) occurs with R⁴-substituted hydrazines (h) and in the presence of an acid, like acetic acid to form the 1,2,4-triazoles of formula (ib), wherein R⁵ is NR-Boc. In a fourth step, both *N*-Boc protection groups are removed from the 1,2,4-triazoles of formula (ib), wherein R⁵ is NR-Boc by reaction with 4N HCl in dioxane to form the 3-alkyl- or 3-haloalkyl-amino-1,2,4-triazole containing amines or the related hydrochloride salt (jb) (*see* INT-020, INT-021, INT-024, INT-029 and INT-034) as demonstrated by the example compounds I-126 to I-132, I-136,I-137, I-142 to I-143, I-180 to I-186, I-202, I-206 to I-210, I-212, I-217 to I-219, I-223, I-226 to I-228 and I-231.

The N-methyl-carbamimidothioic acid methyl ester are either commercially available or may be synthesized by methods known to the skilled artisan (*see* Scheme 5).

Scheme 16 illustrates the preparation of 3-(*N,N*-dialkyl)-amino-, 3-(*N*-alkyl-*N*-cycloalkyl)-amino- or 3-heterocyclyl substituted 1,2,4-triazoles (I"u) starting with 3-(*N,N-*dialkyl)-amino, 3-(*N*-alkyl-*N-*cycloalkyl)-amino- or 3-heterocyclyl-1,2,4-triazole containing amines (jc) as intermediates *(see* INT-022 to INT-024). R¹ is hydrogen, R^{3a} is methyl, R^{3b} is hydrogen, X is oxygen, Y is a direct bond, R⁵ is T¹ = NR⁵¹R⁵² and R² as well as R⁴ have the meanings given in of formula I.

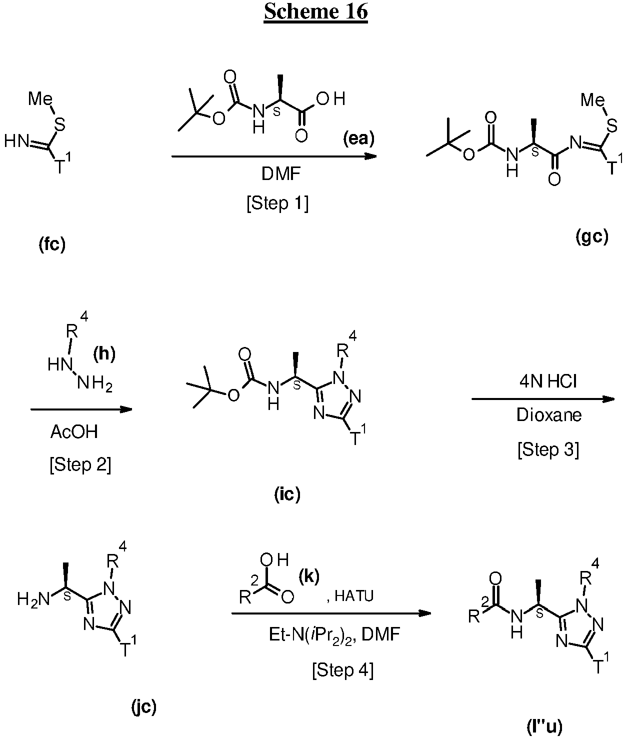

In a first step, *N*-Boc-alanine (ea) reacts for example with *N,N*-dialkyl-carbamimidothioic acid methyl ester (fc; T¹ = -N(alkyl)₂) or 4-morpholinecarboximidothioic acid methyl ester (fc; T¹ = morpholinyl) in the presence of a base and a coupling reagent like HATU to form the *N*-acylated *tert-*butyl *N-*[(1*S*)-2-[[*N,N*-dialkylamino(methylsulfanyl)methylene]amino]-1-methyl-2-oxo-ethyl] carbamates (gc; T¹ = - N(alkyl)₂) or *N*-[(1*S*)-2-[[morpholino(methylsulfanyl)methylene]amino]-1-methyl-2-oxo-ethyl] carbamate acetic acid (fc; T¹ = morpholinyl) which can be used without further purification. In a second step the cyclization of (gc) occurs with R⁴-substituted hydrazines (h) and in the presence of an acid, like acetic acid to form the 1,2,4-triazoles of formula (ic). In the third step the *N*-Boc protection group is removed from the 1,2,4-triazoles of formula (ic), by reaction with 4N HCl in dioxane to form the i3-(*N,N-*dialkyl)- or morpholino-1,2,4-triazole containing amines or the related hydrochloride salt (jc) (*see* INT-022 to INT-023, INT-027, INT-028, INT-031 to INT-033, INT-036 and INT-037), as demonstrated by the example compounds I-138 to I-141, I-156, I-157, I-162, I-164 to I-166, I-170, I-171, I-176 to I-179, I-191 to I-194, I-229, I-232 to I-240 and I-252 to I-272 (T¹ = -N(CH₃)₂), I-145 to I-147, I-158 to I-161, I-174, I-175, I-195, I-197, I-199, I-201, I-204 (T¹ = morpholinyl), I-196, I-198, I-200 and I-220 to I-222 (T¹ = piperidinyl) and I-190, I-203, I-224 to I-225 and I-230 (T¹ = pyrrolidinyl).

The *N,N*-dialkyl-carbamimidothioic acid methyl ester are either commercially available or may be synthesized by methods known to the skilled artisan.

For *N,N-*dimethyl-carbamimidothioic acid methyl ester, *see* CAS-no: 57618-94-7 (Enamine building blocks).

For *N*-ethyl-*N*-methyl-carbamimidothioic acid methyl ester, *see* CAS-no: 787521-54-4 (Chemieliva Pharmaceutical Product List).

For *N,N*-diethyl-carbamimidothioic acid methyl ester, *see* CAS-no: 89269-29-4 (Chemieliva Pharmaceutical Product List).

For *N*-methyl-*N*-(1-methylethyl)-carbamimidothioic acid methyl ester, *see* CAS-no: 1365957-47-6 (FCH Group Reagents for Synthesis).

For *N*-methyl-*N*-propyl-carbamimidothioic acid methyl ester, *see* CAS-no: 764620-88-4 (Chemieliva Pharmaceutical Product List).

The *N*-cycloalkylaminecarboximidothioic acid methyl ester are either commercially available or may be synthesized by methods known to the skilled artisan (*e.g.* WO 2018/089904 A1).

For 4-morpholinecarboximidothioic acid methyl ester, *see* CAS-no: 89269-30-7 (Chemieliva Pharmaceutical Product List).

For 1-pyrrolidinecarboximidothioic acid methyl ester, *see* CAS-no: 758658-51-4 (Chemieliva Pharmaceutical Product List).

For 1-piperidinecarboximidothioic acid methyl ester, the 1-piperidinecarbothioamide (CAS-no: 14294-09-8 (ABCR GmbH Product List) can be used as starting compound for S-methylation by methods known to the skilled artisan.

Scheme 17 illustrates the preparation 3-(*N*-alkyl, *N*-alkoxy)-amino substituted 1,2,4-triazoles (I"v) starting with 3-(*N*-alkyl, *N*-alkoxy)-amino-1,2,4-triazole containing amines (jd) as intermediates (see INT-026). R¹ is hydrogen, R^{3a} is methyl, R^{3b} is hydrogen, X is oxygen, Y is a direct bond, R⁵ is a N(R')OR group and R² as well as R⁴ have the meanings given in of formula I.

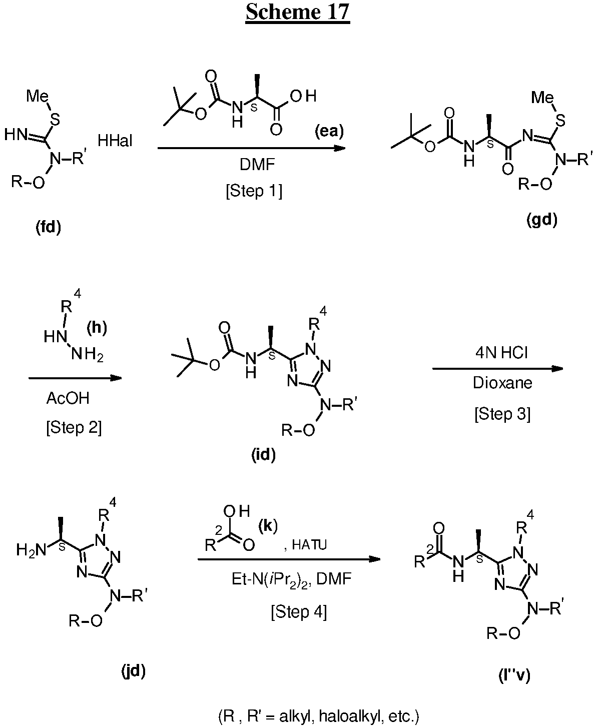

In a first step, *N*-Boc-alanine (ea) reacts for example with *N*-alkoxy-*N*-alkyl-carb-amimidothioic acid methyl ester monohydroiodides (Hal = I) (fd) in the presence of a base and the coupling reagent HATU to form the *N*-acylated *tert-*butyl *N*-[(1*S*)-2-[[*N*-alkoxy-*N*-alkylamino (methylsulfanyl)methylene]amino]-1-methyl-2-oxo-ethyl] carbamates (gd), which can be used without purification. In a second step the cyclization of (gd) occurs with R⁴-substituted hydrazines (h) and in the presence of an acid, like acetic acid to form the 1,2,4-triazoles of formula (id), wherein R⁵ is a N(R')OR group. In the third step the *N-*Boc protection group is removed from the 1,2,4-triazoles of formula (id), wherein R⁵ is a N(R')OR group (see scheme 17), by reaction with 4N HCl in dioxane to form the 3-(*N*-alkoxy-*N*-alkyl) containing amines or the related hydrochloride salt (jd) (*see* INT-026) as demonstrated by the example compounds I-149 to I-153.

The *N*-alkoxy-*N*-alkyl-,carbamimidothioic acid methyl ester may be synthesized by methods known to the skilled artisan.

For *N*-methoxy-*N*-methyl-carbamimidothioic acid methyl ester-monohydroiodide, *see* CAS-no: 749181-24-6 (WO 9300336 A1).

For *N*-ethoxy-*N*-methyl-carbamimidothioic acid methyl ester-monohydroiodide, *see* CAS-no: 153068-71-4 (WO 9300336 A1).

For *N*-methyl-*N*-propoxy-carbamimidothioic acid methyl ester-monohydroiodide, *see* CAS-no: 153068-72-5 (WO 9300336 A1).

Scheme 18 illustrates the preparation 3-amino-1,2,4-triazoles (I"w) starting with 3-amino-1,2,4-triazole containing amines (jd) (*see* INT-025

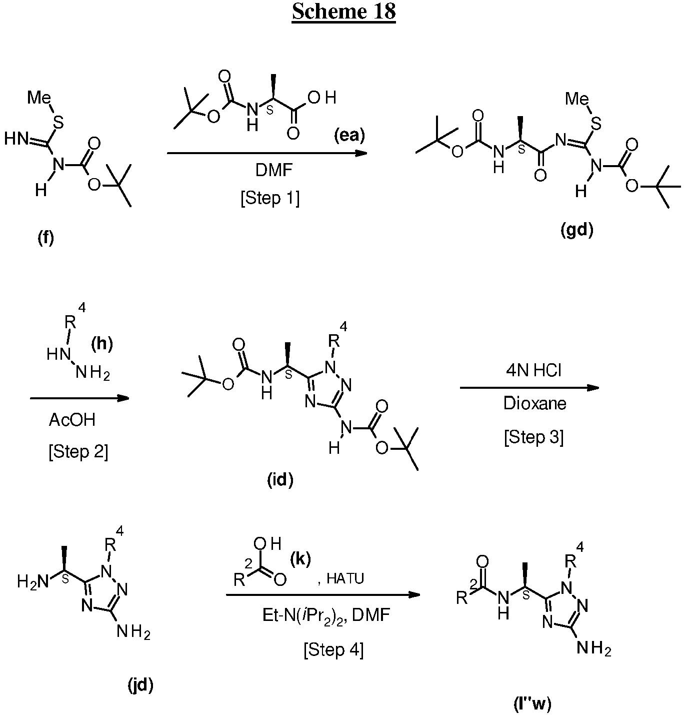

In a first step, N-Boc-alanine (ea) reacts with 1-N-Boc-2-methyl-isothiourea (f) (purchased from ABCR) (see scheme 4) in the presence of a base and the coupling reagent HATU to form the *tert-*butyl *N*-[(1*S*)-2-[[(tert-butoxycarbonylamino)-methylsulfanyl-methylene]amino]-1-methyl-2-oxo-ethyl]carbamate (gd), which can be used without purification. In a second step the cyclization of (gd) occurs with R⁴-substituted hydrazines (h) and in the presence of an acid, like acetic acid to form the 1,2,4-triazoles of formula (id), wherein R⁵ is NH-Boc. In the third step both N-Boc protection groups are removed from the 1,2,4-triazoles of formula (id), wherin R⁵ is NH-Boc, by reaction with 4N HCl in dioxane to form the 3-amino-1,2,4-triazole containing amines or the related hydrochloride salt (jd) (*see* INT-025, INT-030, INT-035 and INT-038) as demonstrated by the example compounds I-148, I-154, I-155, I-169, I-172, I-173, I-187 to I-189, I-205, I-211, I-213 to 216, I-241 to I-251 and I-285 to I-286.

The 1-N-Boc-2-methyl-isothiourea is either commercially available or may be synthesized by methods known to the skilled artisan (*see* scheme 4).

Scheme 19 illustrates the preparation of enantiomeric enriched 3-tert-butoxycarbonyl-amino-, 3-*tert-*butoxycarbonyl-alkylamino- (I"x), (I"xa) as well as 3-alkylamino- and 3-amino-1,2,4-triazoles (I"w), (I"t) by chromatographic separation of the racemic 3-tert-butoxycarbonyl-amino- and 3-*tert-*butoxycarbonyl-alkylamino-1,2,4-triazoles (I"h) and (I"ha) on a chiral stationary phase (*see* example I-133 and I-135). R¹ is hydrogen, R^{3a} is methyl, R^{3b} is hydrogen, X is oxygen, Y is a direct bond, R⁵ is *N-*Boc-amino, N-Boc-alkylamino or amino, N-alkylamino and R² as well as R⁴ have the meanings given in of formula I.

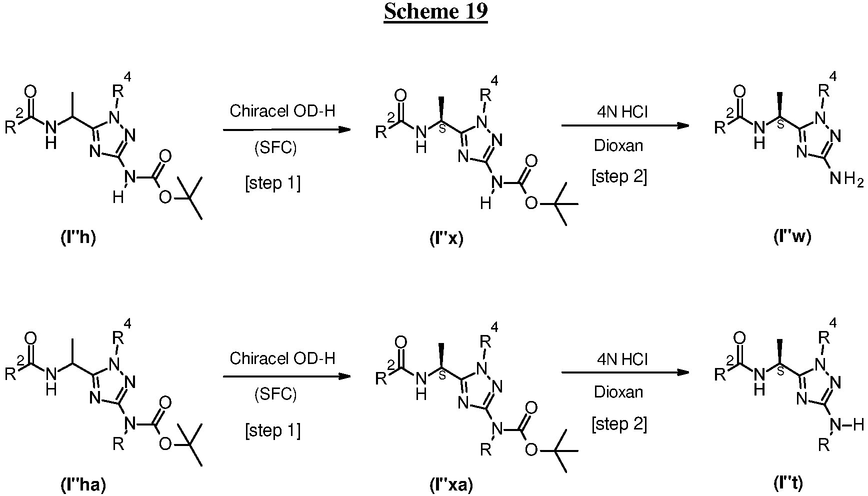

In a first step, the racemic 3-tert-butoxycarbonyl-amino- and 3-*tert*-butoxycarbonyl-alkylamino-1,2,4-triazoles (I"h) and (I''ha), prepared as crude materials according to scheme 4 and scheme 5 (e.g. by using the 3-alkylamino-1,2,4-triazole containing amines (ja)), were separated by supercritical fluid chromatography (SFC) on a chiral stationary phase (Chiracel OD-H) into their *(R)* and *(S)-*isomers*.* The substances were isolated from the fractions by evaporation at 40 °C under vacuum and dried at 10 mbar for 10 minutes to afford the enantiomeric enriched *(S)*-isomers of 3-*tert*-butoxycarbonyl-amino- and 3-*tert*-butoxycarbonyl-alkylamino-1,2,4-triazoles (I"x) and (I"xa). In a second step the *N*-Boc protectiongroups are removed from the 1,2,4-triazoles of formula (I"x) and (I"xa), wherin R⁵ is NH-Boc and NR-Boc by reaction with 4N HCl in dioxane to form the 3-amino-1,2,4-triazoles or the related hydrochloride salt (I"w) and 3-alkylamino-1,2,4-triazoles or the related hydrochloride salt (I''t) as demonstrated by the example compounds (I-127 and I-134).

Alternatively, the enantiomeric enriched 3-alkylamino-1,2,4-triazole (I''t) and 3-amino-1,2,4-triazoles (I"w) can be preared by total syntheses according to the schemes 15 and 18.

### Preparation of examples

### Synthesis of tert-butyl N-[5-[1-[[3-chloro-5-(trifluoromethyl)benzoyl]amino]ethyl]-1-pyrimidin-2-yl-1,2,4-triazol-3-yl]carbamate (example 1-001, reference compound)

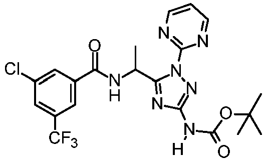

### Step 1

### tert-Butyl N-[N-[2-(1,3-dioxoisoindolin-2-yl)propanoyl]-C-methylsulfanyl-carbonimidoyl]carbamate

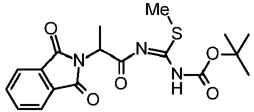

To 1.09 g (5.0 mmol) (α*S*)-1,3-dihydro-α-methyl-1,3-dioxo-2*H*-isoindole-2-acetic acid (Pht-Ala-OH purchased from ABCR) und 0.95 g (5.0 mmol) 1-*N*-Boc-2-methyl-isothiourea (purchased from ABCR) in THF (30 ml), triethylamine (2.1 mL) and 2.85 g (7.5 mmol) HATU was added, and the reaction mixture was stirred at 80 °C temperature and stirred further 2 h at the same temperature. Then water was added and the mixture was extracted with NaHCO₃ solution and dichloromethane. After drying the solvent was evaporated. The remaining solid residue was chromatographed with a cyclohexane/acetone gradient on silica gel to afford 1.40 g (purity: 97.0 %; yield: 70 %) of the racemic title compound.

| | |
|---|---|
| Formula: C₁₈H₂₁N₃O₅S | Molecular weight: 391.44 g/mol |

UPLC-MS (acid) [m/z]: 392.2 [M+H]⁺
¹H-NMR peaklist (400 MHz, DMSO-d₆, ppm): δ = 11.8958 (0.5); 11.4353 (0.9); 7.9299 (0.5); 7.9221 (1.0); 7.9152 (1.2); 7.9102 (1.0); 7.9063 (1.2); 7.9000 (2.1); 7.8930 (1.1); 7.8847 (2.6); 7.8785 (1.3); 7.8744 (1.2); 7.8627 (0.8); 4.9976 (0.8); 4.9794 (0.8); 3.3230 (9.5); 2.5251 (0.4); 2.5204 (0.6); 2.5117 (8.2); 2.5072 (16.6); 2.5027 (21.9); 2.4981 (15.8); 2.4936 (7.6); 2.2949 (2.4); 1.9720 (6.0); 1.6029 (2.9); 1.5848 (3.0); 1.5719 (1.3); 1.5540 (1.1); 1.4430 (16.0); 1.3971 (11.0); 1.2665 (6.6); -0.0002 (0.5).

### Step 2

### tert-Butyl N-[5-[1-(1,3-dioxoisoindolin-2-yl)ethyl]-1-pyrimidin-2-yl-1,2,4-triazol-3-yl]carbamate

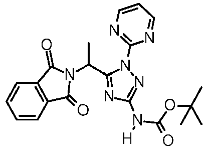

To a solution of 1.0 g (2.55 mmol) *tert-*butyl *N*-[(*E*)-*N-*[2-(1,3-dioxoisoindolin-2-yl)propanoyl]-C-methylsulfanyl-carbonimidoyl]carbamate in pyridine (50 ml), 337.5 mg (3.06 mmol) 2-hydrazino-pyrimidine was added and the reaction mixture was stirred at room temperature for 2 h at 80 °C temperature. Afterwards the solvent was evaporated *in vacuo* the crude product was chromatographed with a cyclohexane/acetone gradient on silica gel to afford 780 mg (purity: 95.9 %; yield: 67 %) of the racemic title compound.

| | |
|---|---|
| Formula: C₂₁H₂₁N₇O₄ | Molecular weight: 435.43 g/mol |

UPLC-MS (acid) [m/z]: 436.3 [M+H]⁺
¹H-NMR peaklist (600 MHz, DMSO-d₆, ppm): δ = 9.9739 (1.2); 8.7635 (2.7); 8.7554 (2.7); 7.8169 (0.6); 7.8126 (0.8); 7.8097 (2.9); 7.8052 (3.0); 7.8020 (0.9); 7.7979 (0.6); 7.4395 (0.7); 7.4315 (1.4); 7.4234 (0.7); 6.0850 (0.8); 6.0732 (0.8); 5.7533 (0.3); 3.3088 (9.2); 2.5080 (3.8); 2.5050 (8.0); 2.5020 (11.1); 2.4989 (8.1); 2.4960 (3.8); 1.9448 (0.4); 1.9123 (0.4); 1.8008 (2.4); 1.7890 (2.4); 1.4365 (16.0); 1.3974 (6.1); -0.0001 (1.9).

### Step 3

### tert-Butyl N-[5-(1-aminoethyl)-1-pyrimidin-2-yl-1,2,4-triazol-3-yl]carbamate (INT-001)

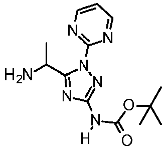

To 900.0 mg (2.06 mmol) *tert-*butyl *N*-[5-[1-(1,3-dioxoisoindolin-2-yl)ethyl]-1-pyrimidin-2-yl-1,2,4-triazol-3-yl]carbamate in ethanol (50 mL), 470.3 mg (5.16 mmol) hydrazine-hydrate were added, and the reaction mixture was heated under reflux. After 30 minutes a colorless precipitate was formed. The reaction mixture was stirred and heated under reflux two additional hour, acetone (30 mL) was added and the heating was continued for further 30 minutes. The reaction mixture was concentrated and the solid residue was treated with ethanol. After filtration, the filtrate was evaporated under reduced pressure to afford the racemic intermediate (INT-001), which was used in step 4 without purification.

| | |
|---|---|
| Formula: C₁₃H₁₉N₇O₂ | Molecular weight: 305.34 g/mol |

HPLC-MS (ESI-positive) [m/z]: 306.2 [M+H]⁺

The intermediate of the formulae **(INT-002)** listed in Table 2 below can be prepared analogously.

### Step 4

### tert-Butyl N-[5-[1-[[3-chloro-5-(trifluoromethyl)benzoyl]amino]ethyl]-1-pyrimidin-2-yl-1,2,4-triazol-3-yl]carbamate

To 973.8 mg (2.60 mmol) *tert-*butyl N-[5-(1-aminoethyl)-1-pyrimidin-2-yl-1,2,4-triazol-3-yl]carbamate, 601.9 mg (2.60 mmol) 3-chloro-5-(trifluoromethyl)-benzoic acid, 436.8 mg (3.38 mmol) DIPEA in DMF (10 mL), 1.18 g (3.12 mmol) HATU were added, and the reaction mixture was stirred at room temperature over night. The reaction mixture was concentrated under reduced pressure and the solid residue was treated with dichloromethane and then extracted with a saturated aqueous NaHCO₃ solution and water. The organic phase was separated, dried over Na₂SO₄ and the solvent was evaporated under reduced pressure. The remaining crude product was chromatographed with a cyclohexane/acetone gradient on silica gel to afford 284 mg (purity: 90.0 %; yield: 19 %) of the racemic title compound.

| | |
|---|---|
| Formula: C₂₁H₂₁ClFN₇O₃ | Molecular weight: 511.89 g/mol |

UPLC-MS neutral: 512.2 [M+H]⁺

The compounds of the formulae (**I-002**) - (**I-003**) listed in Table 1 below can be prepared analogously.

### Synthesis of N-[1-(5-amino-2-pyrimidin-2-yl-1,2,4-triazol-3-yl)ethyl]-3-chloro-5-(trifluoromethyl)benzamide (example 1-004, reference compound)

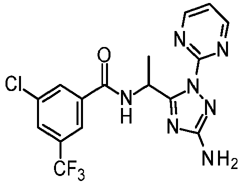

2.8 g (2.64 mmol) tert-butyl *N*-[5-[1-[[3-chloro-5-(trifluoromethyl)benzoyl]amino]ethyl]-1-pyrimidin-2-yl-1,2,4-triazol-3-yl]carbamate were dissolved in 4N HCl-dioxane solution (50 mL) and the mixture was stirred 18 h at room temperature. Then, the solvent was evaporated under reduced pressure to afford 2.3 g (purity: 94%) of the racemic title compound, which was used for further reaction steps.

| | |
|---|---|
| Formula: C₁₆H₁₄Cl₂F₃N₇O | Molecular weight: 248.23 g/mol |

HPLC-MS neutral (ESI-positive) [m/z]: 412.1 [M-HCl]⁺

The compounds of the formulae (**I-005**) - (**I**-**008**) listed in Table 1 below can be prepared analogously.

### Synthesis of tert-butyl N-[5-[1-[[3,5-bis(trifluoromethyl)benzoyl]amino]ethyl]-1-pyrimidin-2-yl-1,2,4-triazol-3-yl]-N-methyl-carbamate (example 1-009, reference compound)

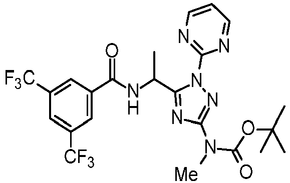

### Step 1

### tert-butyl N-methyl-N-(methylsulfanylcarbonimidoyl)carbamate

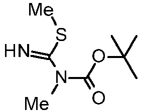

To a mixture of 10.0 g (43.0 mmol) 1,2-dimethylisothiourea hydroiodide, tetrahydrofurane (150 mL), water (150 mL) and NaHCO₃ solution, 10.3 g (47.3 mmol) di-*tert*-butyl dicarbonate was added, and the reaction mixture was stirred at room temperature overnight. Then water was added and the mixture was extracted with EtOAc. After drying the solvent was evaporated to afford 7.10 g (purity: 71.0 %; yield: 81 %) of the title compound as yellow oil.

| | |
|---|---|
| Formula: C₈H₁₆N₂O₂S | Molecular weight: 204.29 g/mol |

UPLC-MS (neutral) [m/z]: 148.9 [M-H₂C=CMe₂]⁺
¹H-NMR peaklist (600 MHz, CD₃CN, ppm): δ = 3.2027 (1.0); 3.0846 (0.6); 2.3498 (0.5); 2.2547 (1.4); 2.1637 (8.9); 1.9485 (0.8); 1.9444 (1.4); 1.9403 (2.1); 1.9362 (1.4); 1.9320 (0.7); 1.4932 (2.4); 1.4819 (16.0); 1.4324 (2.0).

### Step 2

### tert-Butyl N-[N-[2-(1,3-dioxoisoindolin-2-yl)propanoyl]-C-methylsulfanyl-carbonimidoyl]-N-methylcarbamate

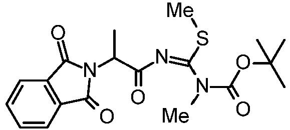

To 7.60 g (34.6 mmol) (α*S*)-1,3-dihydro-α-methyl-1,3-dioxo-2*H*-isoindole-2-acetic acid (Pht-Ala-OH purchased from ABCR) und 7.08 g (34.6 mmol) *tert-*butyl N-methyl-*N*-(methylsulfanyl-carbonimidoyl)carbamate in THF (321.8 mL), triethylamin (14.5 ml) and HATU was added, and the reaction mixture was stirred at 80 °C temperature and stirred further 2 h at the same temperature. Then tetrahydrofuran was evaporated and the mixture was extracted with NaHCO₃ solution and dichloromethane. The organic phase was dried and evaporated to afford the racemic title compound as colorless oil.

| | |
|---|---|
| Formula: C₁₉H₂₃N₃O₅S | Molecular weight: 405.47 g/mol |

HPLC-MS neutral (ESI-positive) [m/z]: 406.2 [M+H]⁺

### Step 3

### tert-Butyl N-[5-[1-(1,3-dioxoisoindolin-2-yl)ethyl]-1-pyrimidin-2-yl-1,2,4-triazol-3-yl]-N-methylcarbamate

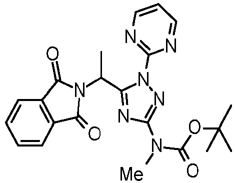

To a solution of 7.5 g (18.4 mmol) *tert-*butyl *N*-[(*E*)-N-[2-(1,3-dioxoisoindolin-2-yl)propanoyl]-C-methylsulfanyl-carbonimidoyl]-*N*-methyl-carbamate in pyridine (50 ml), 2.5 g (22.7 mmol) 2-hydrazinopyrimidine was added and the reaction mixture was stirred at room temperature for 2 h at 80 °C temperature. Afterwards the solvent was evaporated *in vacuo* and the crude product was chromatographed with a cyclohexane/acetone gradient on silica gel to afford 6.0 g (purity: 97.5 %; yield: 70 %) of the racemic title compound.

| | |
|---|---|
| Formula: C₂₂H₂₃N₇O₄ | Molecular weight: 449.47 g/mol |

HPLC-MS acid (ESI-positive) [m/z]: 450.2 [M+H]⁺

### Step 4

### tert-Butyl N-[5-(1-aminoethyl)-1-pyrimidin-2-yl-1,2,4-triazol-3-yl]-N-methyl-carbamate (INT-003)

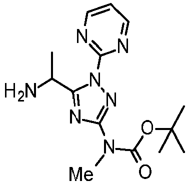

To 6.0 g (13.3 mmol) *tert-*butyl *N*-[5-[1-(1,3-dioxoisoindolin-2-yl)ethyl]-1-pyrimidin-2-yl-1,2,4-triazol-3-yl]-*N*-methyl-carbamate in ethanol (150 mL), 3.06 g (33.7 mmol) hydrazine-hydrate were added, and the reaction mixture was heated under reflux. After 30 minutes a colorless precipitate was formed. The reaction mixture was stirred and heated under reflux two additional hours, acetone (10 mL) was added and the heating was continued for further 30 minutes. The reaction mixture was concentrated and the solid residue was treated with ethanol. After filtration, the filtrate was evaporated under reduced pressure to afford the racemic intermediate (INT-003), which was used in step 4 without purification.

| | |
|---|---|
| Formula: C₁₄H₂₁N₇O₂ | Molecular weight: 319.36 g/mol |

HPLC-MS acid (ESI-positive) [m/z]: 320.2 [M+H]⁺

### Step 5

### tert-Butyl N-[5-[1-[[3,5-bis(trifluoromethyl)benzoyl]amino]ethyl]-1-pyrimidin-2-yl-1,2,4-triazol-3-yl]-N-methyl-carbamate

To 671.55 mg (2.60 mmol) *tert*-butyl *N*-[5-(1-aminoethyl)-1-pyrimidin-2-yl-1,2,4-triazol-3-yl]-*N*-methylcarbamate, 250.0 mg (0.93 mmol) 3,5-bis-(trifluoromethyl)-benzoic acid, 157.8 mg (1.22 mmol) DIPEA in 15.78 g (384.5 mmol) MeCN, 228.67 mg (1.12 mmol) HATU were added, and the reaction mixture was stirred at room temperature overnight. The reaction mixture was concentrated under reduced pressure and the solid residue was treated with dichloromethane and then extracted with a saturated aqueous NaHCO₃ solution and water. The organic phase was separated, dried over Na₂SO₄ and the solvent was evaporated under reduced pressure. The remaining residue was purified by HPLC with a water/MeCN neutral gradient to obtain 422 mg (purity: 99.0 %; yield: 79 %) of the racemic title compound.

| | |
|---|---|
| Formula: C₂₃H₂₃F₆N₇O₃ | Molecular weight: 559.47 g/mol |

HPLC-MS acid (ESI-positive): 512.2 [M+H]⁺

### Synthesis of methyl N-[1-(5-chloro-2-pyridyl)-5-[1-[[3-chloro-5-(trifluoromethyl)benzoyl]amino] ethyl]-1,2,4-triazol-3-yl]carbamate (example 1-010)

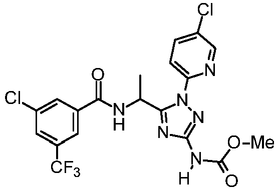

To 200.0 mg (0.44 mmol) *N*-[1-[5-amino-2-(5-chloro-2-pyridyl)-1,2,4-triazol-3-yl]ethyl]-3-chloro-5-(trifluoromethyl)benzamide in THF (15 mL), 77.2 mg (0.76 mmol) triethylamine and 488.1 mg (5.16 mmol) methyl chloroformate was added, and then the reaction mixture was stirred 18 h at room temperature. Because a low conversion was observed, 10 equivalents of methyl chloroformate were added and the reaction mixture was stirred further 18 h at room temperature. After filtration, the filtrate was evaporated under reduced pressure. The remaining residue was purified by HPLC with a water/acetonitrile neutral gradient to obtain 110.5 mg (purity: 100 %; yield: 49 %) of the racemic title compound.

| | |
|---|---|
| Formula: C₁₉H₁₅Cl₂F₃N₆O₃ | Molecular weight: 503.27 g/mol |

HPLC-MS (ESI-positive): 503.1 [M+H]⁺

### Synthesis of N-[1-[5-acetamido-2-(5-chloro-2-pyridyl)-1,2,4-triazol-3-yl]ethyl]-3-chloro-5-(trifluoromethyl)benzamide (example 1-011, reference compound)

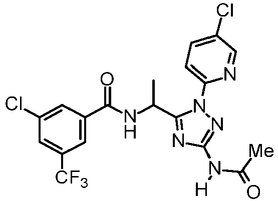

To 200.0 mg (0.44 mmol) *N*-[1-[5-amino-2-(5-chloro-2-pyridyl)-1,2,4-triazol-3-yl]ethyl]-3-chloro-5-(trifluoromethyl)benzamide in THF (15 mL), 77.2 mg (0.76 mmol) triethylamine and 52.8 mg (0.67 mmol) acetyl chloride was added, and then the reaction mixture was stirred 18 h at room temperature. After filtration, the filtrate was evaporated under reduced pressure to obtain 166.0 mg (purity: 96.9 %; yield: 74 %) of the racemic title compound.

| | |
|---|---|
| Formula: C₁₉H₁₅Cl₂F₃N₆O₂ | Molecular weight: 487.27 g/mol |

HPLC-MS (ESI-positive) [m/z]: 487.1 [M+H]⁺

The compounds of the formulae (**I-012**) - (**I-024**) listed in Table 1 below can be prepared analogously.

### Synthesis of N-[5-(1-aminoethyl)-1-pyrimidin-2-yl-1,2,4-triazol-3-yl]-1,1-difluoro-methanesulfonamide (intermediate INT-004)

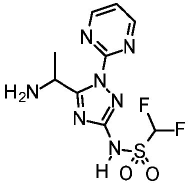

### Step 1

### 1-(difluoromethylsulfonyl)-2-methyl-isothiourea

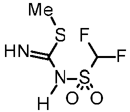

To 250.0 mg (1.32 mmol) 2-methylisothiourea sulfuric acid salt in dichloromethane (10 mL) and 250.0 mg (2.35 mmol) Na₂CO₃, 239.9 mg (1.59 mmol) difluoromethane sulfonylchloride was added, and the reaction mixture was stirred at room temperature overnight. Then water was added and the mixture was extracted with dichloromethane. After separation of the layers, the organic layer was dried and the solvent evaporated to afford 158 mg (purity: 78.0 %; yield: 58 %) of the title compound as yellow oil, which was used for the second step.

| | |
|---|---|
| Formula: C₃H₆F₂N₂O₂S₂ | Molecular weight: 204.21 g/mol |

HPLC-MS acid (ESI-positive) [m/z]: 205.1 [M+H]⁺

### Step 2

### N-[(Difluoromethylsulfonylamino)-methylsulfanyl-methylene]-2-(1,3-dioxoisoindolin-2-yl)propenamide

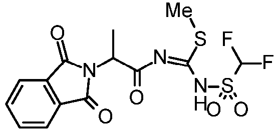

To 0.18 g (0.82 mmol) (αS)-1,3-dihydro-α-methyl-1,3-dioxo-2H-isoindole-2-acetic acid (Pht-Ala-OH purchased from ABCR) and 0.15 g (0.77 mmol 1-(difluoromethylsulfonyl)-2-methyl-isothiourea in tetrahydrofuran (3.56 mL), triethylamin (0.34 mL) and 0.46 g (1.23 mmol) HATU was added, and the reaction mixture was stirred at 80 °C temperature and stirred further 2 h at the same temperature. Then tetrahydrofuran was evaporated and the mixture was extracted with sodium hydrogencarbonate solution and dichloromethane. The organic phase was dried and evaporated to afford the racemic title compound, which was used for step 3 without purification.

| | |
|---|---|
| Formula: C₁₄H₁₃F₂N₃O₅S₂ | Molecular weight: 405.40 g/mol |

HPLC-MS neutral (ESI-positive) [m/z]: 406.0 [M+H]⁺

### Step 3

### N-[5-[1-(1,3-dioxoisoindolin-2-yl)ethyl]-1-pyrimidin-2-yl-1,2,4-triazol-3-yl]-1,1-difluoro-methanesulfonamide

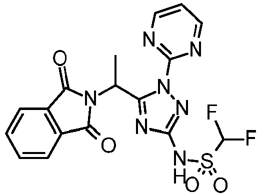

To a solution of 700 mg (1.72 mmol) N-[(difluoromethylsulfonylamino)-methylsulfanyl-methylene]-2-(1,3-dioxoisoindolin-2-yl)propanamide in pyridine (20 ml), 233.9 mg (2.12 mmol) 2-hydrazinopyrimidine was added and the reaction mixture was stirred at room temperature for 2 h at 80 °C temperature. Afterwards the solvent was evaporated *in vacuo* and the crude product was chromatographed with a cyclohexane/acetone gradient on silica gel to afford 380.0 mg (purity: 98.0 %; yield: 48 %) of the racemic title compound (55 mol-%) as pyridine (45 mol%) adduct (according to the ¹³C with ¹H decoupling data, CPD).

| | |
|---|---|
| Formula: C₁₇H₁₃F₂N₇O₄S | Molecular weight: 449.39 g/mol |

HPLC-MS acid (ESI-positive) [m/z]: 450.1 [M+H]⁺
¹H-NMR peaklist (600 MHz, DMSO-d₆, ppm): δ = 8.7717 (15.4); 8.7636 (15.7); 8.6322 (3.5); 8.6293 (4.8); 8.6268 (2.5); 8.6249 (2.5); 8.6222 (4.8); 8.6195 (3.4); 7.9149 (0.8); 7.9120 (1.6); 7.9090 (0.8); 7.9022 (1.6); 7.8992 (3.2); 7.8963 (1.6); 7.8894 (0.9); 7.8865 (1.7); 7.8835 (0.9); 7.8303 (0.5); 7.8280 (1.0); 7.8273 (1.0); 7.8247 (0.7); 7.8193 (2.2); 7.8127 (15.6); 7.8100 (16.0); 7.8072 (5.5); 7.8035 (2.2); 7.7981 (0.7); 7.7954 (1.0); 7.4922 (4.5); 7.4898 (2.5); 7.4848 (2.6); 7.4824 (4.5); 7.4794 (4.4); 7.4770 (2.3); 7.4721 (2.3); 7.4697 (4.1); 7.4586 (4.2); 7.4505 (8.0); 7.4425 (4.0); 7.1798 (1.7); 7.0913 (3.9); 7.0029 (2.0); 6.0923 (1.1); 6.0806 (4.4); 6.0688 (4.4); 6.0570 (1.1); 3.4408 (0.4); 3.4338 (0.4); 3.4204 (0.4); 3.4101 (0.4); 3.3821 (0.4); 3.3697 (0.3); 3.3237 (0.3); 2.6182 (0.4); 2.6152 (0.5); 2.6121 (0.4); 2.5920 (0.5); 2.5242 (1.2); 2.5211 (1.5); 2.5180 (1.4); 2.5092 (26.6); 2.5061 (56.6); 2.5031 (79.1); 2.5000 (58.5); 2.4971 (27.2); 2.3901 (0.4); 2.3870 (0.5); 2.3840 (0.4); 2.0866 (7.8); 1.9675 (0.5); 1.9345 (0.5); 1.7998 (13.1); 1.7881 (13.1); -0.0001 (4.4).

### Step 4

### N-[5-(1-Aminoethyl)-1-pyrimidin-2-yl-1,2,4-triazol-3-yl]-1,1-difluoro-methanesulfonamide (INT-004)

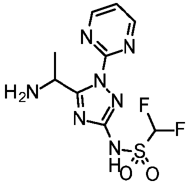

To 500 mg (1.11 mmol) N-[5-[1-(1,3-dioxoisoindolin-2-yl)ethyl]-1-pyrimidin-2-yl-1,2,4-triazol-3-yl]-1,1-difluoro-methanesulfonamide in ethanol (30 mL), 270 mg (2.96 mmol) hydrazine-hydrate were added, and the reaction mixture was heated under reflux. After 30 minutes a colorless precipitate was formed. The reaction mixture was stirred and heated under reflux two additional hour, acetone (10 mL) was added and the heating was continued for further 30 minutes. The reaction mixture was concentrated and the solid residue was treated with ethanol. After filtration, the filtrate was evaporated under reduced pressure to afford the racemic intermediate (INT-004), which can be used without purification.

| | |
|---|---|
| Formula: C₉H₁₁F₂N₇O₂S | Molecular weight: 319.29 g/mol |

HPLC-MS acid (ESI-positive) [m/z]: 320.0 [M+H]⁺

### Synthesis of 3-chloro-N-[1-[2-(5-chloro-2-pyridyl)-5-(methanesulfonamido)-1,2,4-triazol-3-yl]ethyl]-5-methylsulfonyl-benzamide (example I-025)

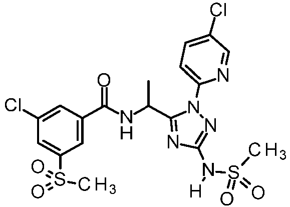

To 200.0 mg (0.43 mmol) *N*-[1-[5-amino-2-(5-chloro-2-pyridyl)-1,2,4-triazol-3-yl]ethyl]-3-chloro-5-methylsulfonyl-benzamidein THF (15 mL), 53.3 mg (0.52 mmol) triethylamine and 55.3 mg (0.48 mmol) methylsulfonyl chloride was added, and then the reaction mixture was stirred 18 h at room temperature. Afterwards the solvent was evaporated *in vacuo* and the crude product was chromatographed with a cyclohexane/acetone gradient on silica gel to obtain 50.0 mg (purity: 95.9 %; yield: 20.4 %) of the racemic title compound and 50.9 mg (purity: 98.6 %; yield: 19 %) of N-[1-[5-[bis(methylsulfonyl)amino]-2-(5-chloro-2-pyridyl)-1,2,4-triazol-3-yl]ethyl]-3-chloro-5-methylsulfonyl-benzamide as racemic side product.

| | |
|---|---|
| Formula: C₁₈H₁₈Cl₂N₆O₅S₂ | Molecular weight: 533.41 g/mol |

HPLC-MS (ESI-positive) [m/z]: 533.1 [M+H]⁺

### Synthesis of N-[1-[5-[bis(methylsulfonyl)amino]-2-(5-chloro-2-pyridyl)-1,2,4-triazol-3-yl]ethyl]-3-chloro-5-methylsulfonyl-benzamide (example I-026)

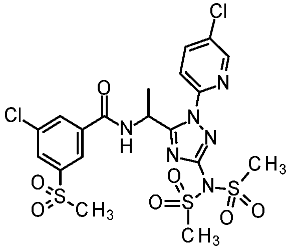

| | |
|---|---|
| Formula: C₁₉H₂₀Cl₂N₆O₇S₃ | Molecular weight: 611.49 g/mol |

HPLC-MS (ESI-positive) [m/z]: 611.1 [M+H]⁺

### Synthesis of 3-chloro-N-[1-[2-(5-chloro-2-pyridyl)-5-(methylsulfonylmethyl)-1,2,4-triazol-3-yl]ethyl]-5-methylsulfonyl-benzamide (example I-027)

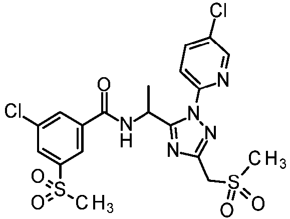

### Step 1

### N'-[(5-chloro-2-pyridyl)amino]-2-methylsulfonyl-acetamidine

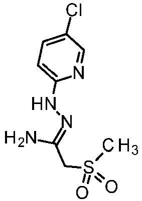

To 4.0 g (27.8 mmol) 5-chloro-2-hydrazino-pyridine in 37.2 g (471.0 mmol) pyridine, 2-(methyl-sulfonyl) -ethanimidic acid ethyl ester hydrochloride (1:1) (W. Tang, et al., J. Amer. Chem. Soc. 137(18), 5980-5989, 2015) was added, and the reaction mixture was stirred at room temperature overnight. The solvent was evaporated and the residue was then stirred with MeOH. The precipitate was separated and dried to obtain 7.3 g (purity: 100 %; yield: 99 %) *N'*-[(5-Chloro-2-pyridyl)amino]-2-methylsulfonyl-acetamidine.

| | |
|---|---|
| Formula: C₈H₁₁ClN₄O₂S | Molecular weight: 262,71 g/mol |

HPLC-MS (ESI-positive) [m/z]: 263.0 [M+H]⁺

### Step 2

### 2-[1-[2-(5-chloro-2-pyridyl)-5-(methylsulfonylmethyl)-1,2,4-triazol-3-yl]ethyl]isoindoline-1,3-dione

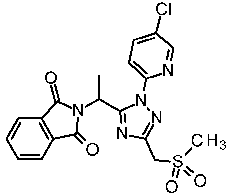

To 7.30 g (27.78 mmol) *N'*-[(5-chloro-2-pyridyl)amino]-2-methylsulfonyl-acetamidine in pyridine (300 mL), 5.42 g (22.80 mmol) (α*S*)-1,3-dihydro-α-methyl-1,3-dioxo-2*H*-isoindole-2-acetyl chloride (see preparation from (α*S*)-1,3-dihydro-α-methyl-1,3-dioxo-2*H*-isoindole-2-acetic acid (Pht-Ala-OH purchased from ABCR) and oxalyl chloride: D. A. Gruzdev et al., Tetrahedron: Asymmetry, 21(8), 936-942, 2010) was added, and the reaction mixture was stirred at room temperature overnight. Then water and dichloromethane was added and the mixture was treated with NaHCO₃ solution. The organic phase was separated, dried and the solvent was evaporated. The remaining residue was treated with DMF filtered and purified by HPLC with a acetonitrile/water gradient (acid) to afford 1.01 g (purity: 94 %; yield: 9 %) of the racemic title compound and 2.20 g (purity: 83.0 %; yield: 12 %) *N*-[6-chloro-2-[2-(1,3-dioxoisoindolin-2-yl)propanoyl]-3-(methylsulfonylmethyl)-[1,2,4]triazolo [4,3-a]pyridin-3-yl]-2-(1,3-dioxoisoindolin-2-yl)propenamide as side product.

| | |
|---|---|
| Formula: C₁₉H₁₆ClN₅O₄S | Molecular weight: 445.88 g/mol |

HPLC-MS neutral (ESI positive) [m/z]: 446.1 [M+H]⁺
¹H-NMR (600.1 MHz, CD₃CN, ppm): δ = 8.2135 (1.8); 8.2093 (1.9); 7.8432 (1.4); 7.8389 (1.4); 7.8287 (1.8); 7.8244 (1.8); 7.7437 (16.0); 7.7061 (2.4); 7.6916 (2.0); 6.0901 (0.5); 6.0783 (1.8); 6.0666 (1.9); 6.0548 (0.6); 4.4882 (5.9); 4.0787 (0.4); 3.0993 (11.5); 3.0514 (0.3); 3.0427 (0.7); 2.1342 (13.9); 2.0862 (1.8); 1.9640 (1.7); 1.9559 (0.9); 1.9518 (1.0); 1.9479 (6.5); 1.9439 (11.4); 1.9397 (16.7); 1.9356 (11.4); 1.9315 (5.8); 1.8544 (7.4); 1.8426 (7.4); 1.6343 (0.5); 1.6222 (0.5); 1.4364 (8.2); -0.0001 (0.5).

### Step 3

### 1-[2-(5-Chloro-2-pyridyl)-5-(methylsulfonylmethyl)-1,2,4-triazol-3-yl]ethanamine (INT-005)

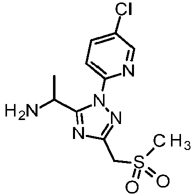

To 1.0 g (2.24 mmol) 2-[1-[2-(5-chloro-2-pyridyl)-5-(methylsulfonylmethyl)-1,2,4-triazol-3-yl]ethyl]isoindoline-1,3-dione in ethanol (25 mL), 510.3 mg (5.60 mmol) hydrazine-hydrate was added, and the reaction mixture was heated under reflux. The reaction mixture was stirred and heated under reflux for 2 h, acetone (25 mL) was added and the heating was continued for further 1 h. The reaction mixture was concentrated and the solid residue was treated with ethanol (100 mL). Then, the solvent was evaporated and the crystals were separated to afford 1.0 g of the racemic intermediate (INT-005), which was used for the N-acylation reaction (step 4) without purification.

| | |
|---|---|
| Formula: C₁₁H₁₄ClN₅O₂S | Molecular weight: 315,78 g/mol |

### Step 4

### 3-Chloro-N-[1-[2-(5-chloro-2-pyridyl)-5-(methylsulfonylmethyl)-1,2,4-triazol-3-yl]ethyl]-5-methylsulfonyl-benzamide

To 254.6 mg (0.80 mmol) 1-[2-(5-chloro-2-pyridyl)-5-(methylsulfonylmethyl)-1,2,4-triazol-3-yl]ethanamine (INT-005), 270.2 mg (1.15 mmol) 3-chloro-5-(methylsulfonyl)-benzoic acid, 744.3 mg (5.75 mmol) DIPEA in DMF (6.9 mL), 525.5 mg (1.38 mmol) HATU was added, and the reaction mixture was stirred at room temperature overnight. The reaction mixture filtered, concentrated and the residue was purified by HPLC with a acetonitrile/water gradient (acid) to afford 251.5 mg (purity: 95.2 %; yield: 39 %) of the title compound.

| | |
|---|---|
| Formula: C₁₉H₁₉Cl₂N₅O₅S₂ | Molecular weight: 532.42 g/mol |

HPLC-MS acid (ESI positive): 532.0 [M+H]⁺

The compounds of the formulae (**I-028**) - (**I-030**) listed in Table 1 below can be prepared analogously.

### Synthesis of 3-chloro-N-[1-[2-(5-chloro-2-pyridyl)-5-(6-chloro-3-pyridyl)-1,2,4-triazol-3-yl]ethyl]-5-(trifluoromethoxy)benzamide (example I-031, reference compound)

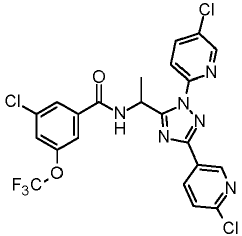

### Step 1

### 6-chloro-N'-[(5-chloro-2-pyridyl)amino]pyridine-3-carboxamidine

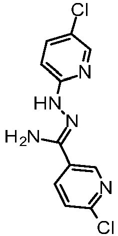

To 1.00 g (6.96 mmol) 5-chloro-2-hydrazino-pyridine in methanol (6.9 mL) 2.17 (9.75 mmol) 3-6-chloro-pyridinecarboximidic acid methyl ester (CAS-no. 154753-15-8; e.g. commercial available: Chemieliva Pharmaceutical Product List) was added, and the reaction mixture was stirred at room temperature overnight. The formed precipitate was separated and the residue was then stirred with methanol. The precipitate was separated and dried to obtain 1.64 g (purity: 100 %; yield: 83 %) 6-chloro-N'-[(5-chloro-2-pyridyl)amino]pyridine-3-carboxamidine, which was used for step 2.

| | |
|---|---|
| Formula: C₁₁H₉Cl₂N₅ | Molecular weight: 282.13 g/mol |

HPLC-MS acid (ESI positive): 282.3 [M+H]

### Step 2

### 2-[1-[2-(5-Chloro-2-pyridyl)-5-(6-chloro-3-pyridyl)-1,2,4-triazol-3-yl]ethyl]isoindoline-1,3-dione

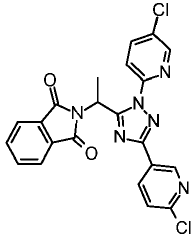

To 1.84 g (5.82 mmol) 6-chloro-N'-[(5-chloro-2-pyridyl)amino]pyridine-3-carboxamidine in pyridine (22 mL), 1.38 g (5.82 mmol) (α*S*)-1,3-dihydro-α-methyl-1,3-dioxo-2*H*-isoindole-2-acetyl chloride (see preparation from (α*S*)-1,3-dihydro-α-methyl-1,3-dioxo-2*H*-isoindole-2-acetic acid (Pht-Ala-OH purchased from ABCR) and oxalyl chloride: D. A. Gruzdev et al., Tetrahedron: Asymmetry, 21(8), 936-942, 2010) was added, and the reaction mixture was stirred at room temperature overnight. Then water and dichloromethane was added and the mixture was treated with NaHCO₃ solution. The organic phase was separated, dried and the solvent was evaporated. The remaining residue was treated with DMF filtered and purified by HPLC with a MeCN/water gradient (acid) to afford 350.0 mg (purity: 100 %; yield: 13 %) of the racemic title compound and 791.0 g (purity: 99.0 %; yield: 2 %) N-[6-chloro-3-(6-chloro-3-pyridyl)-2-[2-(1,3-dioxoisoindolin-2-yl)propanoyl]-[1,2,4]triazolo[4,3-a]pyridin-3-yl]-2-(1,3-dioxoisoindolin-2-yl)propenamide as side product.

| | |
|---|---|
| Formula: C₂₂H₁₄Cl₂N₆O₂ | Molecular weight: 465.30 g/mol |

HPLC-MS (ESI positive) [m/z]: 465.1 [M+H]⁺
¹H-NMR peaklist (600 MHz, d₆-DMSO, ppm): δ = 9.0704 (6.3); 9.0666 (6.1); 8.4602 (4.0); 8.4562 (3.9); 8.4464 (4.1); 8.4423 (4.0); 8.3054 (6.4); 8.3012 (6.4); 8.1116 (3.8); 8.1073 (3.6); 8.0971 (4.4); 8.0929 (4.2); 7.8968 (7.0); 7.8824 (6.2); 7.8371 (3.4); 7.8310 (4.4); 7.8292 (4.3); 7.8265 (5.1); 7.8224 (11.6); 7.8169 (5.9); 7.8120 (11.8); 7.8080 (5.2); 7.8056 (4.3); 7.8038 (4.3); 7.7976 (3.4); 7.7111 (6.4); 7.6972 (6.2); 6.1120 (1.3); 6.1004 (4.8); 6.0887 (4.9); 6.0769 (1.3); 3.3157 (95.8); 2.6199 (0.7); 2.5288 (1.6); 2.5259 (1.9); 2.5227 (2.1); 2.5107 (90.8); 2.5079 (119.6); 2.5051 (91.5); 2.3919 (0.7); 1.8947 (15.9); 1.8830 (16.0); 0.0054 (4.3).

### Step 3

### 1-[2-(5-Chloro-2-pyridyl)-5-(6-chloro-3-pyridyl)-1,2,4-triazol-3-yl]ethanamine (INT-006)

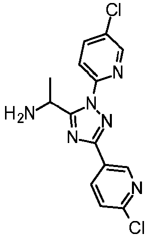

To 1.67 g (3.58 mmol) 2-[1-[2-(5-chloro-2-pyridyl)-5-(6-chloro-3-pyridyl)-1,2,4-triazol-3-yl]ethyl]isoindoline-1,3-dione in ethanol (150 mL), 816.7 mg (8.97 mmol) hydrazine-hydrate was added, and the reaction mixture was heated under reflux. The reaction mixture was stirred and heated under reflux for 2 h, acetone (25 mL) was added and the heating was continued for further 1 h. The reaction mixture was concentrated and the solid residue was treated with ethanol (100 mL). Then, the solvent was evaporated and the crystals were separated to afford 710.3 g (yield: 59 %) racemic 1-[2-(5-chloro-2-pyridyl)-5-(6-chloro-3-pyridyl)-1,2,4-triazol-3-yl]ethanamine , which was used for the N-acylation reaction (step 4) without purification.

| | |
|---|---|
| Formula: C₁₄H₁₂Cl₂N₆ | Molecular weight: 335.19 g/mol |

HPLC-MS acid (ESI positive): 335.1 [M+H]⁺

The intermediates of the formulae (**INT-007**) - (**INT-011**) listed in Table 2 below can be prepared analogously.

### Step 4

### 3-Chloro-N-[1-[2-(5-chloro-2-pyridyl)-5-(6-chloro-3-pyridyl)-1,2,4-triazol-3-yl]ethyl]-5-(trifluoromethoxy)benzamide

To 222.9 mg (0.66 mmol) 1-[2-(5-chloro-2-pyridyl)-5-(6-chloro-3-pyridyl)-1,2,4-triazol-3-yl]ethanamine (INT-006), 235.6 mg (0.95 mmol) 3-chloro-5-(trifluoromethoxy)-benzoic acid, 614.0 mg (4.75 mmol) DIPEA in DMF (6.5 mL), 433.5 mg (1.14 mmol) HATU was added, and the reaction mixture was stirred at room temperature overnight. The reaction mixture was filtered, concentrated and the residue was purified by HPLC with a MeCN/water gradient (acid) to afford 103.6 mg (purity: 100 %; yield: 16 %) of the title compound. The enantiomeric excess of the (S)-enantiomer has been determined via screen OD_RH (acid): ee-value = 90%; Rₜ = 16.54 min.

| | |
|---|---|
| Formula: C₂₂H₁₄Cl₃F₃N₆O₂ | Molecular weight: 557.75 g/mol |

HPLC-MS acid (ESI positive): 557.1 [M+H]⁺

The compounds of formulae (**I-032**) - (**I-058**) listed in Table 1 below can be prepared analogously.

### Synthesis of 3-chloro-N-[1-[2-(5-chloro-2-pyridyl)-5-methylsulfanyl-1,2,4-triazol-3-yl]ethyl]-5-methylsulfonyl-benzamide (example I-059)

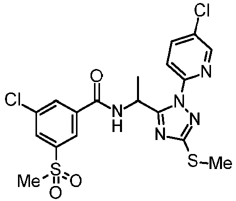

### Step 1

### (2S)-2-(1,3-dioxoisoindolin-2-yl)propanoyl isothiocyanate

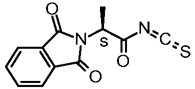

To 5.42 g (22.80 mmol) (α*S*)-1,3-dihydro-α-methyl-1,3-dioxo-2*H*-isoindole-2-acetyl chloride (see preparation from (α*S*)-1,3-dihydro-α-methyl-1,3-dioxo-2*H*-isoindole-2-acetic acid (Pht-Ala-OH purchased from ABCR) and oxalyl chloride: D. A. Gruzdev et al., Tetrahedron: Asymmetry, 21(8), 936-942, 2010) in acetonitrile (210 mL) 1.73 g (22.80 mmol) ammonium thiocyanate was added, and the reaction mixture was stirred at room temperature overnight. The reaction mixture was filtered and used for step 2 without isolation.

### Step 2

### 2-[1-[2-(5-Chloro-2-pyridyl)-5-thioxo-1H-1,2,4-triazol-3-yl]ethyl]isoindoline-1,3-dione

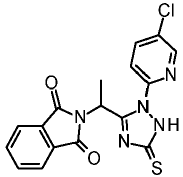

To 2.96 g (11.40 mmol) (2S)-2-(1,3-dioxoisoindolin-2-yl)propanoyl isothiocyanate in acetonitrile (100 mL) 1.63 g (11.40 mmol) 5-chloro-2-hydrazino-pyridine was added, and the reaction mixture was stirred 3 h under reflux temperature. Then, the reaction mixture was concentrated and used for the S-methylation step without purification.

### Step 3

### 2-[1-[2-(5-chloro-2-pyridyl)-5-methylsulfanyl-1,2,4-triazol-3-yl]ethyl]isoindoline-1,3-dione

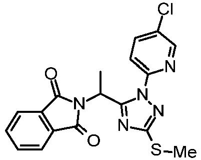

To 1.40 g (3.62 mmol) 2-[1-[2-(5-chloro-2-pyridyl)-5-thioxo-1H-1,2,4-triazol-3-yl]ethyl]isoindoline-1,3-dione in DMF (30 mL) 152.38 g (3.81 mmol) sodium hydride (purity: 60%) was added, and the reaction mixture was stirred 10 minutes at room temperature. Afterwards 772.52 g (5.44 mmol) iodomethane was added, and the reaction mixture was stirred 3 h at room temperature. Then, the reaction mixture was treated with water (0.5 mL) and stirred further 30 minutes at room temperature. The reaction mixture was evaporated and the remaining residue was chromatographed with a cyclohexane/acetone gradient on silica gel later on and purified by HPLC with a acetonitrile/water gradient (acid) to afford 150.0 mg (purity: 100 %; yield: 10 %) of the racemic title compound.

| | |
|---|---|
| Formula: C₁₈H₁₄ClN₅O₂S | Molecular weight: 399.86 g/mol |

HPLC-MS acid (ESI positive) [m/z]: 400.1 [M+H]⁺
¹H-NMR peaklist (600 MHz, DMSO-d₆, ppm): δ = 8.2732 (1.8); 8.2722 (1.8); 8.2689 (1.9); 8.2679 (1.8); 8.0535 (1.6); 8.0491 (1.5); 8.0389 (1.8); 8.0346 (1.7); 7.8289 (1.0); 7.8245 (0.8); 7.8223 (1.2); 7.8212 (1.1); 7.8180 (1.5); 7.8139 (3.8); 7.8069 (4.0); 7.8027 (1.8); 7.7996 (1.1); 7.7986 (1.1); 7.7963 (0.7); 7.7919 (1.1); 7.7566 (2.1); 7.7556 (2.0); 7.7421 (2.0); 7.7411 (1.9); 6.0350 (0.4); 6.0233 (1.7); 6.0115 (1.8); 5.9997 (0.4); 3.3068 (47.6); 2.6130 (0.3); 2.5954 (16.0); 2.5221 (0.6); 2.5190 (0.8); 2.5158 (0.9); 2.5072 (17.2); 2.5041 (36.0); 2.5010 (49.3); 2.4979 (35.4); 2.4949 (16.1); 2.0727 (0.4); 1.7901 (5.1); 1.7784 (5.2); -0.0001 (1.0).

### Step 4

### 1-[2-(5-Chloro-2-pyridyl)-5-methylsulfanyl-1,2,4-triazol-3-yl]ethanamine (INT-012)

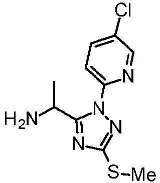

To 880.0 mg (2.22 mmol) 2-[1-[2-(5-chloro-2-pyridyl)-5-methylsulfanyl-1,2,4-triazol-3-yl]ethyl]isoindoline-1,3-dione in ethanol (20.4 mL), 505.3 mg (5.55 mmol) hydrazine-hydrate was added, and the reaction mixture was heated under reflux. The reaction mixture was stirred and heated under reflux for 2 h. Then acetone (25 mL) was added and the heating was continued for further 1 h. The reaction mixture was concentrated and the solid residue was separated to afford 599 mg of the racemic intermediate (INT-012), which was used for the N-acylation reaction (step 5) without purification.

| | |
|---|---|
| Formula: C₁₀H₁₂ClN₅S | Molecular weight: 269.75 g/mol |

HPLC-MS acid (ESI positive): 270.1 [M+H]⁺

The intermediates of the formulae (**INT-013**) **-** (**INT-014**) listed in Table 2 below can be prepared analogously.

### Step 5

### 3-Chloro-N-[(1S)-1-[2-(5-chloro-2-pyridyl)-5-methylsulfanyl-1,2,4-triazol-3-yl]ethyl]-5-methylsulfonyl-benzamide

To 603.5 mg (2.23 mmol) 1-[2-(5-chloro-2-pyridyl)-5-methylsulfanyl-1,2,4-triazol-3-yl]ethanamine (INT-012), 750.0 mg (3.19 mmol) 3-chloro-5-(methylsulfonyl)-benzoic acid, 2.06 g (15.98 mmol) DIPEA in DMF (13.3 mL), 1.45 g (3.83 mmol) HATU was added, and the reaction mixture was stirred at room temperature overnight. The reaction mixture was filtered, concentrated and the residue was purified by HPLC with a MeCN/water gradient (acid) to afford 830.0 mg (purity: 100 %; yield: 53 %) of the racemic title compound.

| | |
|---|---|
| Formula: C₁₈H₁₇Cl₂N₅O₃S₂ | Molecular weight: 486.40 g/mol |

HPLC-MS acid (ESI positive): 486.1 [M+H]⁺

### Synthesis of 3-chloro-N-[1-[2-(5-chloro-2-pyridyl)-5-methylsulfonyl-1,2,4-triazol-3-yl]ethyl]-5-methylsulfonyl-benzamide (example I-060)

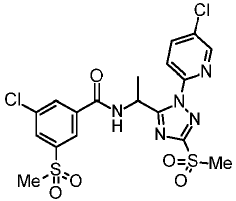

To 200.0 mg (0.41 mmol) 3-chloro-N-[(1S)-1-[2-(5-chloro-2-pyridyl)-5-methylsulfanyl-1,2,4-triazol-3-yl]ethyl]-5-methylsulfonyl-benzamide in dichloromethane (25 mL) 202.7 mg (0.82 mmol) *meta-*chlorobenzoic acid (mCPBA, 70%) was added, and the reaction mixture was stirred overnight. Then, the reaction mixture was quenched with Na₂CO₃ solution and concentrated. The residue was purified by HPLC with an MeCN/water gradient (acid) to afford 161.3 mg (purity: 100 %; yield: 76 %) of the racemic title compound and 10.7 mg (purity: 81 %, yield: 4 %) racemic 3-chloro-*N*-[1-[2-(5-chloro-2-pyridyl)-5-methylsulfinyl-1,2,4-triazol-3-yl]ethyl]-5-methylsulfonyl-benzamide (example 1-061) as second compound.

| | |
|---|---|
| Formula: C₁₈H₁₇Cl₂N₅O₄S₂ | Molecular weight: 518.40 g/mol |

HPLC-MS acid (ESI positive): 518.1 [M+H]⁺

### Synthesis of 3-chloro-N-[1-[2-(5-chloro-2-pyridyl)-5-methylsulfinyl-1,2,4-triazol-3-yl]ethyl]-5-methylsulfonyl-benzamide (example I-061)

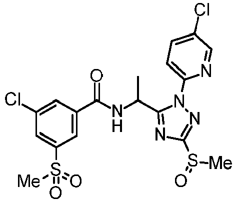

| | |
|---|---|
| Formula: C₁₈H₁₇Cl₂N₅O₃S₂ | Molecular weight: 502.39 g/mol |

HPLC-MS acid (ESI positive): 502.0 [M+H]⁺

The compounds of the formulae (**I-062**) - (**I-070**) listed in Table 1 below can be prepared analogously.

### Synthesis of 3-chloro-N-[1-[2-(5-chloro-2-pyridyl)-5-cyano-1,2,4-triazol-3-yl]ethyl]-5-(trifluoromethyl)benzamide (example I-071, reference compound)

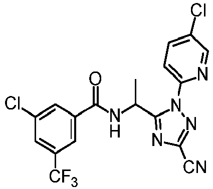

### Step 1

### N-[1-[5-Bromo-2-(5-chloro-2-pyridyl)-1,2,4-triazol-3-ylethyl]-3-chloro-5-(trifluoromethyl)benzamide

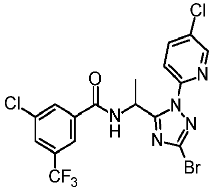

To 200.0 mg (0.44 mmol) racemic *N*-[1-[5-amino-2-(5-chloro-2-pyridyl)-1,2,4-triazol-3-yl]ethyl]-3-chloro-5-(trifluoromethyl)benzamide (I-006) (synthesized according to I-004) in MeCN (10 mL), 170.5 mg (0.76 mmol) Cu(II)-bromide was added, and then the reaction mixture was treated drop by drop at room temperature with 64.8 mg (0.62 mmol) *tert-*butyl nitrite. Then the reaction mixture was stirred 1 h at 70 °C temperature. The reaction mixture was treated with acetic acid ethyl ester and then extracted with a saturated NaCl solution. Afterwards the solvent was evaporated *in vacuo* and the crude product was chromatographed with a cyclohexane/acetone gradient on silica gel to afford 93.8 mg (purity: 99.3 %; yield: 41 %) of the racemic title compound.

| | |
|---|---|
| Formula: C₁₇H₁₁BrCl₂F₃N₅O | Molecular weight: 509,12 g/mol |

HPLC-MS acid (ESI positive) [m/z]: 510.0 [M+H]⁺
¹H-NMR peaklist (600.1 MHz, DMSO-d₆): δ = 9.4115 (2.6); 9.4001 (2.7); 8.6396 (0.4); 8.6349 (4.7); 8.6311 (4.5); 8.6307 (4.4); 8.2163 (3.4); 8.2120 (3.3); 8.2089 (0.4); 8.2018 (3.6); 8.1975 (3.6); 8.1214 (4.6); 8.0745 (4.3); 8.0615 (4.7); 7.8651 (5.0); 7.8506 (4.6); 5.9417 (0.4); 5.9302 (2.0); 5.9253 (0.4); 5.9186 (3.2); 5.9139 (0.5); 5.9071 (2.1); 5.8955 (0.4); 3.3216 (92.2); 2.6184 (0.4); 2.6154 (0.5); 2.6124 (0.4); 2.5244 (1.1); 2.5214 (1.4); 2.5182 (1.5); 2.5094 (26.3); 2.5064 (55.2); 2.5033 (76.9); 2.5003 (57.3); 2.4973 (27.2); 2.3904 (0.4); 2.3873 (0.5); 2.3843 (0.3); 1.6353 (11.1); 1.6285 (2.0); 1.6237 (11.1); 1.3974 (16.0); -0.0001 (2.7).

### Step 2

### Synthesis of 3-chloro-N-[1-[2-(5-chloro-2-pyridyl)-5-cyano-1,2,4-triazol-3-yl]ethyl]-5-(trifluoromethyl)benzamide

To 500.0 mg (0,98 mmol) racemic *N*-[1-[5-bromo-2-(5-chloro-2-pyridyl)-1,2,4-triazol-3-yl]ethyl]-3-chloro-5-(trifluoromethyl)benzamide in DMF (20 mL), 567.45 mg (0.49 mmol) tetrakis(triphenylphosphine)palladium(0) and 1.15 g (9.82 mmol) zinc cyanide were added, and the reaction mixture was stirred 8 h at 95 °C under argon atmosphere. The reaction mixture was filtered, concentrated and the residue was purified by HPLC with a MeCN/water gradient (acid) to afford 216.8 mg (purity: 100 %; yield: 48 %) of the racemic title compound.

| | |
|---|---|
| Formula: C₁₈H₁₁Cl₂F₃N₆O | Molecular weight: 455.22 g/mol |

HPLC-MS acid (ESI positive) [m/z]: 455.1 [M+H]⁺

### Synthesis of 5-[1-[[3-chloro-5-(trifluoromethyl)benzoyl]amino]ethyl]-1-pyrimidin-2-yl-1,2,4-triazole-3-carboxylic acid (example I-072, reference compound)

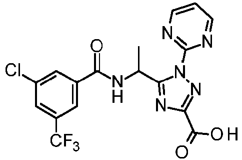

### Step 1

### (2S)-2-[[3-chloro-5-(trifluoromethyl)benzoyl]amino]propanoic acid

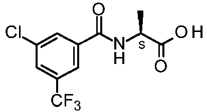

A mixture of 22.0 g (246.9 mmol) L-alanine and 39.5 g (987.6 mmol) NaOH in a mixture of water (270 mL) and MeCN (90 mL) was stirred for 20 min at 0 °C. To the above mixture was added (60.0 g, 246.9 mmol) 3-chloro-5-(trifluoromethyl)benzoyl chloride which was dissolved in MeCN (30 mL) dropwise over 30 min at 0°C. The resulting mixture was stirred for additional 1 h at room temperature. Then, the mixture was concentrated under reduced pressure. The mixture was acidified to pH 6 with HCl (aq.). The aqueous layer was extracted with EtOAc (3 x 200 mL). The combined organic layers were washed by brine (100 mL), dried by Na₂SO₄ and concentrated under reduced pressure. The residue was purified by reverse flash chromatography (C₁₈ silica gel column; mobile phase, methanol in water, 10% to 50% gradient in 10 min, detector, UV 254 nm) to afford 35.0 g (yield: 48 %) of the title compound.

### Step 2

### methyl 5-[1-[[3-chloro-5-(trifluoromethyl)benzoyl]amino]ethyl]-1-pyrimidin-2-yl-1,2,4-triazole-3-carboxylate

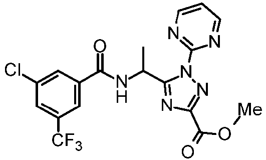

To a stirred mixture of 10.0 g (33.8 mmol) (2S)-2-[[3-chloro-5-(trifluoromethyl)benzoyl] amino]propanoic acid and 19.3 g (50.7 mmol) HATU in DMF (300 mL) were added 4.7 g (33.8 mmol) methyl 2-amino-2-imino-acetate hydrochloride and 13.1 g (101.5 mmol) DIPEA in portions at room temperature. The resulting mixture was stirred for 3 h at room temperature. To the above mixture was added (698.1 mmol) AcOH (40 mL) and 4.5 g (40.6 mmol) 2-hydrazinylpyrimidine in portions within 1 minute at room temperature. The resulting mixture was stirred for additional 1 h at 80°C. The aqueous layer was extracted with ethyl acetate (500 mL). The organic layer was washed by brine (100 mL), dried by Na₂SO₄ and concentrated under reduced pressure. The residue was purified by reverse flash chromatography (C₁₈ silica gel column; mobile phase, methanol in water, 10% to 50% gradient in 10 min; detector, UV 254 nm) to afford 4.9 g (32 %) of the racemic title compound (partial racemization) as a white solid; 900 mg of which were purified by chiral HPLC chromatography to afford 127.6 mg (yield: 14 %) methyl 5-[1-[[3-chloro-5-(trifluoromethyl)benzoyl]amino]ethyl]-1-pyrimidin-2-yl-1,2,4-triazole-3-carboxylate as a white solid (major enantiomer) and 104.7 mg (yield: 12 %) (minor enantiomer) as a white solid.

¹H-NMR peaklist (300 MHz, DMSO-d₆, ppm) - major enantiomer: δ = 9.5192 (1.3); 9.4959 (1.3); 9.0451 (8.1); 9.0288 (8.3); 8.1212 (2.4); 8.1165 (1.8); 8.0722 (4.7); 8.0691 (4.5); 7.7265 (2.2); 7.7102 (4.0); 7.6940 (2.1); 6.0320 (1.0); 6.0087 (1.5); 5.9855 (1.0); 3.8958 (16.0); 3.3280 (17.2); 2.5158 (5.6); 2.5098 (10.8); 2.5038 (14.1); 2.4978 (9.6); 2.4919 (4.3); 1.6917 (5.2); 1.6685 (5.1); -0.0004 (7.6).

### Step 3

### 5-[1-[[3-chloro-5-(trifluoromethyl)benzoyl]amino]ethyl]-1-pyrimidin-2-yl-1,2,4-triazole-3-carboxylic acid

To a stirred mixture of 2.5 g (5.5 mmol) racemic methyl 5-[1-[[3-chloro-5-(trifluoromethyl) phenyl]formamido]ethyl]-1-(pyrimidin-2-yl)-1,2,4-triazole-3-carboxylate (from step 2) in a mixture of MeOH (60 mL) and water (20 mL), 0.66 g (16.5 mmol) NaOH was added in portions at room temperature. The resulting mixture was stirred for 30 min at room temperature. Then, the mixture was acidified to pH 5 with HCl (aq.). The resulting mixture was concentrated under reduced pressure. The aqueous layer was extracted with ethyl acetate (3 x 40 mL). The combined organic layers were washed by brine (100 mL), dried by Na₂SO₄ and concentrated under reduced pressure to get 1.6 g (66 %) of the racemic title compound as a white solid.

¹H-NMR peaklist (300 MHz, DMSO-d₆, ppm): δ= 9.4845 (2.0); 9.4608 (2.0); 9.0248 (15.6); 9.0085 (16.0); 8.1180 (5.2); 8.0638 (11.0); 7.6967 (3.8); 7.6804 (7.1); 7.6642 (3.6); 6.0407 (0.4); 6.0177 (1.7); 5.9942 (2.8); 5.9807 (1.0); 5.9707 (1.8); 5.9483 (0.3); 3.3143 (95.7); 2.7332 (1.0); 2.7270 (1.3); 2.7206 (0.8); 2.5130 (83.9); 2.5070 (162.9); 2.5009 (215.8); 2.4949 (146.0); 2.4890 (66.0); 2.2770 (0.9); 2.2708 (1.2); 2.2646 (0.9); 1.9088 (0.4); 1.6790 (10.6); 1.6558 (10.4); 1.2349 (1.9); 1.1662 (0.4); 1.1466 (0.4); 0.4842 (0.7); 0.0106 (1.0); -0.0003 (28.4); -0.0114 (0.9).

### Synthesis of 5-[1-[[3-chloro-5-(trifluoromethyl)benzoyl]amino]ethyl]-1-(5-cyano-2-pyridyl)-1,2,4-triazole-3-carboxylic acid (example I-073, reference compound)

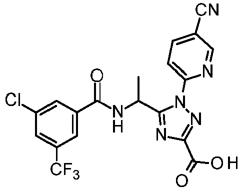

### Step 1

### methyl 5-[1-[[3-chloro-5-(trifluoromethyl)benzoyl]amino]ethyl]-1-(5-cyano-2-pyridyl)-1,2,4-triazole-3-carboxylate

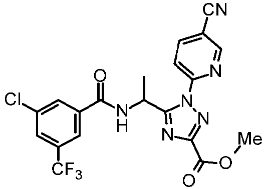

The preparation was carried out analogously to the reaction instructions of example 1-071 (step 2), using:
16.0 g (54.2 mmol) (2S)-2-[[3-chloro-5-(trifluoromethyl)benzoyl] amino]propanoic acid
30.9 g (81.4 mmol) HATU
DMF (320 mL)
7.5 g (54.2 mmol) methyl carbamimidoylformate hydrochloride
16.4 g (162.7 mmol) DIPEA
acetic acid (60 mL)
8.7 g (65.1 mmol) 5-cyano-2-hydrazinylpyrimidine

The residue was purified chiral HPLC chromatography to afford 128.6 mg (yield: 2 %) methyl 5-[1-[[3-chloro-5-(trifluoromethyl)benzoyl]amino]ethyl]-1-(5-cyano-2-pyridyl)-1,2,4-triazole-3-carboxylate as major enantiomer and 102.2 mg (yield: 2 %) as minor enantiomer.

¹H-NMR peaklist (300 MHz, DMSO-d₆, ppm) - major enantiomer: δ = 9.5483 (1.6); 9.5255 (1.6); 9.1190 (2.7); 9.1169 (2.9); 9.1119 (3.0); 9.1098 (2.7); 8.6426 (2.0); 8.6353 (1.9); 8.6141 (2.2); 8.6067 (2.2); 8.1648 (2.9); 8.1377 (2.9); 8.1358 (2.9); 8.1094 (5.2); 8.0749 (2.7); 6.1075 (1.1); 6.0845 (1.7); 6.0614 (1.1); 3.9100 (16.0); 3.3373 (4.2); 2.5265 (2.2); 2.5207 (4.1); 2.5148 (5.4); 2.5090 (3.7); 2.0872 (3.5); 1.6946 (5.8); 1.6714 (5.7); 1.3273 (0.3); -0.0004 (0.7).

### Step 2

### 5-[1-[[3-chloro-5-(trifluoromethyl)benzoyl]amino]ethyl]-1-(5-cyano-2-pyridyl)-1,2,4-triazole-3-carboxylic acid

To a stirred mixture of 2.0 g (4.2 mmol) racemic methyl 5-[1-[[3-chloro-5-(trifluoromethyl)benzoyl]-amino]ethyl]-1-(5-cyano-2-pyridyl)-1,2,4-triazole-3-carboxylate in toluene (60 mL) was added 1.5 g (8.4 mmol) Sn(CH₃)₃OH in portions at 60 °C. The resulting mixture was stirred for 5 h at 60 °C. The precipitated solids were collected by filtration and washed with toluene (2 x 10 mL). The organic layer was concentrated under reduced pressure to afford 1.7 g (yield: 88 %) of the racemic title compounds as a white solid.

¹H-NMR peaklist (300 MHz, DMSO-d₆, ppm): δ= 9.5082 (4.2); 9.4854 (4.2); 9.0893 (8.4); 9.0842 (8.6); 8.6227 (6.5); 8.6154 (6.1); 8.5942 (7.1); 8.5868 (7.0); 8.1587 (7.8); 8.1021 (15.7); 8.0737 (15.5); 6.1150 (0.7); 6.0911 (2.6); 6.0681 (4.0); 6.0452 (2.6); 6.0221 (0.7); 3.8979 (0.4); 3.3145 (128.3); 2.7330 (1.4); 2.7272 (1.9); 2.7208 (1.4); 2.6550 (0.4); 2.5131 (120.7); 2.5071 (236.4); 2.5011 (314.8); 2.4951 (213.4); 2.4891 (96.6); 2.2770 (1.3); 2.2711 (1.9); 2.2648 (1.3); 1.9091 (0.5); 1.6742 (16.0); 1.6512 (15.8); 1.2348 (1.4); 1.1578 (0.6); 1.1471 (0.5); 1.1334 (0.4); 0.8550 (0.3); 0.8300 (0.4); 0.0107 (1.6); -0.0003 (43.6); - 0.0113 (1.2)

### Synthesis of 5-[1-[[3-chloro-5-(trifluoromethyl)benzoyl]amino]ethyl]-1-pyrimidin-2-yl-1,2,4-triazole-3-carboxamide (example I-074, reference compound)

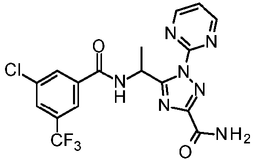

To a stirred mixture of 1.5 g (3.4 mmol) racemic 5-[1-[[3-chloro-5-(trifluoromethyl)benzoyl]amino] ethyl]-1-pyrimidin-2-yl-1,2,4-triazole-3-carboxylic acid and 1.9 g (5.1 mmol) HATU in DMF (40 mL) were added 0.55 g (10.2 mmol) ammonium chloride and 1.3 g (10.2 mmol) DIEA in portions at room temperature. The resulting mixture was stirred for 1.5 h at room temperature. The residue was dissolved in water (200 mL). The resulting mixture was extracted with ethylacetate (2 x 60 mL). The combined organic layers were washed with water (3 x 20 mL), dried over anhydrous Na₂SO₄. After filtration, the filtrate was concentrated under reduced pressure. The residue was purified by reverse flash chromatography (C₁₈ silica gel column; mobile phase, MeOH in water, 10% to 50% gradient in 10 min; detector, UV 254 nm) to afford 1.0 g (66.8 %) racemic 5-[1-[[3-chloro-5-(trifluoromethyl)benzoyl] amino]ethyl]-1-pyrimidin-2-yl-1,2,4-triazole-3-carboxamide as a white solid; 400 mg of which was purified by chiral HPLC column to afford 116.6 mg (29 %) 5-[1-[[3-chloro-5-(trifluoromethyl) benzoyl]amino]ethyl]-1-pyrimidin-2-yl-1,2,4-triazole-3-carboxamide as a white solid (major enantiomer) and 66.4 mg (17 %) as a white solid (minor enantiomer).

¹H-NMR (major enantiomer) see Table 1.

The compound of the formula (**I-075**) listed in Table 1 below can be prepared analogously.

### Synthesis of 5-[1-[[3-chloro-5-(trifluoromethyl)benzoyl]amino]ethyl]-N-cyclopropyl-1-pyrimidin-2-yl-1,2,4-triazole-3-carboxamide (example I-076, reference compound)

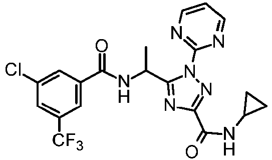

To a stirred mixture of 141.2 mg (24.7 mmol) cyclopropane amine and 356.6 mg (4.9 mmol) trimethyl aluminium in dichloromethane (40 mL) was added 750.0 mg (1.6 mmol) methyl 5-[1-[[3-chloro-5-(trifluoromethyl)benzoyl] amino]ethyl]-1-pyrimidin-2-yl-1,2,4-triazole-3-carboxylate (example 1-072, step 2) dropwise at room temperature under a nitrogen atmosphere. The resulting mixture was stirred for 3 h at room temperature under a nitrogen atmosphere. The resulting mixture was washed with water (3 x 30 mL). The organic layer was dried by Na₂SO₄ and concentrated under reduced pressure. The crude product was purified by preparative HPLC (XBridge Shield RP18 OBD column, 19 x 250 mm,10 um; mobile phase A: water (10 mmol/L NH₄HCO₃), mobile p hase B: acetonitrile; Flow rate: 25 mL/min; Gradient: 47 B to 47 B in 7 min; 254/220 nm) to afford 480.0 mg (yield: 60.6 %) of the racemic title compound as a white solid. The resulting product was further purified by chiral HPLC column to afford 112.7 mg (yield: 24 %) of 5-[1-[[3-chloro-5-(trifluoromethyl)benzoyl]amino]ethyl]-N-cyclopropyl-1-pyrimidin-2-yl-1,2,4-triazole-3-carboxamide (major enantiomer) as a white solid and 87.1 mg (yield: 18 %) of the minor enantiomer as a white solid.

¹H-NMR (major enentiomer) see Table 1.

The compound of the formulae (**I-077**) - (**I**-**079**) listed in Table 1 below can be prepared analogously.

### Synthesis of 3-chloro-N-[1-(5-cyano-2-pyrimidin-2-yl-1,2,4-triazol-3-yl)ethyl]-5-(trifluoromethyl)benzamide (example I-080, reference compound)

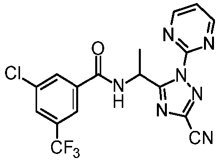

To a stirred mixture of 560.0 mg (1.3 mmol) 5-[1-[[3-chloro-5-(trifluoromethyl)benzoyl]amino]ethyl]-1-pyrimidin-2-yl-1,2,4-triazole-3-carboxamide (example 1-074) and 644.3 mg (6.4 mmol) triethylamine in THF (20 mL) 1069.79 mg (5.093 mmol) trifluoroacetic acid anhydride (TFAA) was added dropwise at 0 °C. The resulting mixture was stirred for overnight at room temperature. The resulting mixture was concentrated under reduced pressure. The residue was purified by preparative TLC (petrolether/EtOAc 3:1) to afford 430.0 mg (yield: 80.1%) of the racemic 3-chloro-*N*-[1-(5-cyano-2-pyrimidin-2-yl-1,2,4-triazol-3-yl)ethyl]-5-(trifluoromethyl)benzamide as a white solid. The resulting product was purified by chiral HPLC column to afford 122.5 mg (yield: 28 %) 3-chloro-*N*-[1-(5-cyano-2-pyrimidin-2-yl-1,2,4-triazol-3-yl)ethyl]-5-(trifluoromethyl)benzamide (major enantiomer) as a white solid and 109.5 mg (yield: 26 %) enantiomer compound as a white solid.

¹H-NMR (major enantiomer) see Table 1.

The compound of the formula (**I-081**) listed in Table 1 below can be prepared analogously.

### Synthesis of N-[1-(5-benzyl-2-pyrimidin-2-yl-1,2,4-triazol-3-yl)ethyl]-3,5-bis(trifluoromethyl)benzamide (example I-82, reference compound)

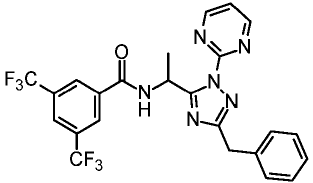

### Step 1

### 2-[(1S)-1-[3-(amino)-1-pyrimidinyl)-1H-1,2,4-triazol-5-yl)ethyl]-1H-isoindole-1,3(2H)-dione-hydrochloride

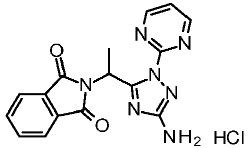

8.8 g (17.1 mmol) 2-[(1*S*)-1-[3-(*N*-Boc-amino)-1-pyrimidinyl)-1*H*-1,2,4-triazol-5-yl)ethyl]-1*H-*isoindole-1,3(2*H*)-dione (synthesisized according to example 1-001, steps 1-2), was treated with 4N HCl in dioxane solution (150 ml) and the reaction mixture was stirred 18 h at room temperature. Then the reaction mixture was concentrated and the solid residue was used for the halogen introduction (step 4) without purification.

| | |
|---|---|
| Formula: C₁₆H₁₄ClN₇O₂ | Molecular weight: 371.78 g/mol |

HPLC-MS (ESI positive) [m/z]: 336.2 [M-HCl]⁺

### Step 2

### 2-[1-(5-Iodo-2-pyrimidin-2-yl-1,2,4-triazol-3-yl)ethyl]isoindoline-1,3-dione

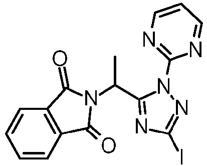

To 11.22 g (30.17 mmol) 2-[(1*S*)-1-[3-(amino)-1-pyrimidinyl)-1*H*-1,2,4-triazol-5-yl)ethyl]-1*H*-isoindole-1,3(2*H*)-dione-hydrochloride in acetonitrile (448 ml), 180.95 g (675.64 mmol) diiodomethane was added (argon atmosphere), and then the reaction mixture was treated drop by drop at room temperature with 14.31 g (138.82 mmol) *tert-*butyl nitrite. Then the reaction mixture was stirred 3 h at 80 °C temperature. The reaction mixture was treated with acetic acid ethyl ester and then extracted with a saturated NaCl solution. Afterwards the solvent was evaporated *in vacuo* the crude product was chromatographed with a cyclohexane/acetone gradient on silica gel to afford 8.1 g (purity: 99 %; yield: 60 %) of the racemic title compound.

| | |
|---|---|
| Formula: C₁₆H₁₁IN₆O₂ | Molecular weight: 446,20 g/mol |

HPLC-MS acid (ESI positive): 447.0 [M+H]⁺
¹H-NMR peaklist (600 MHz, DMSO-d₆, ppm): δ = 8.7920 (10.1); 8.7839 (10.2); 8.7780 (7.2); 8.7699 (7.2); 7.9209 (0.5); 7.9156 (0.6); 7.9124 (0.5); 7.9111 (0.6); 7.9065 (1.1); 7.8865 (1.1); 7.8821 (0.6); 7.8775 (0.6); 7.8723 (0.6); 7.8272 (1.6); 7.8220 (2.5); 7.8200 (3.0); 7.8159 (4.7); 7.8122 (12.5); 7.8073 (16.0); 7.8036 (5.1); 7.8010 (3.2); 7.7995 (4.0); 7.7971 (5.8); 7.7928 (3.1); 7.7889 (1.8); 7.7827 (1.6); 7.5145 (2.8); 7.5064 (5.5); 7.4983 (2.8); 7.4911 (2.0); 7.4830 (3.8); 7.4750 (1.9); 6.1032 (0.8); 6.0915 (3.1); 6.0798 (3.5); 6.0672 (2.7); 6.0553 (2.2); 6.0436 (0.6); 4.8841 (0.6); 4.8719 (0.6); 3.3419 (6.3); 2.5116 (11.4); 2.5087 (15.6); 2.5057 (11.5); 2.5028 (5.4); 2.0892 (0.4); 1.7986 (10.2); 1.7870 (12.4); 1.7772 (7.0); 1.7441 (3.2); 1.5644 (2.1); 1.5522 (2.1); 1.3888 (0.7); 1.2301 (8.8)

### Step 3

### 2-[1-(5-Benzyl-2-pyrimidin-2-yl-1,2,4-triazol-3-yl)ethyl]isoindoline-1,3-dione

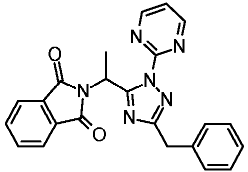

To 250.0 mg (0.56 mmol) 2-[1-(5-iodo-2-pyrimidin-2-yl-1,2,4-triazol-3-yl)ethyl]isoindoline-1,3-dione in THF (20 mL), 397.39 mg (1.68 mmol) benzyl zinc bromide (0.5M in THF, ABCR) and 25.0 mg (0.02 mmol) tetrakis(triphenylphosphine)palladium(0) were added, and the reaction mixture was stirred 2 h at 50 °C under argon atmosphere. The reaction mixture was filtered, concentrated and the residue was purified by HPLC with a MeCN/water gradient (acid) to afford 176.0 mg (purity: 100 %; yield: 76 %) of the racemic title compound.

| | |
|---|---|
| Formula: C₂₃H₁₈N₆O₂ | Molecular weight: 410.43 g/mol |

HPLC-MS acid (ESI positive) [m/z]: 411.2 [M+H]⁺
¹H-NMR peaklist (600 MHz, d6-DMSO-d₆, ppm): δ = 8.7418 (15.2); 8.7337 (15.4); 7.8139 (2.4); 7.8096 (1.8); 7.8070 (3.4); 7.8029 (4.0); 7.7989 (12.4); 7.7928 (12.7); 7.7887 (4.4); 7.7850 (3.4); 7.7820 (1.7); 7.7777 (2.4); 7.4318 (4.2); 7.4237 (8.1); 7.4157 (4.1); 7.3451 (1.8); 7.3422 (2.7); 7.3311 (8.7); 7.3291 (7.2); 7.3252 (8.8); 7.3212 (1.3); 7.3131 (7.4); 7.3105 (2.4); 7.2999 (2.7); 7.2441 (1.2); 7.2415 (1.9); 7.2384 (1.0); 7.2299 (2.7); 7.2210 (0.7); 7.2183 (1.2); 7.2155 (0.6); 6.0709 (1.1); 6.0592 (4.4); 6.0474 (4.5); 6.0357 (1.1); 4.0926 (16.0); 3.3235 (100.8); 2.6176 (0.3); 2.6145 (0.4); 2.5235 (1.0); 2.5205 (1.3); 2.5174 (1.3); 2.5085 (24.5); 2.5055 (51.6); 2.5025 (71.7); 2.4994 (53.3); 2.4965 (25.2); 2.3894 (0.3); 2.3864 (0.4); 2.3834 (0.3); 2.0748 (12.6); 1.7921 (14.2); 1.7804 (14.2); -0.0001 (1.2)

### Step 4

### 1-(5-Benzyl-2-pyrimidin-2-yl-1,2,4-triazol-3-yl)ethanamine (INT-015)

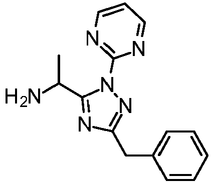

To 913.0 mg (2.22 mmol) 2-[1-(5-benzyl-2-pyrimidin-2-yl-1,2,4-triazol-3-yl)ethyl]isoindoline-1,3-dione in ethanol (30.4 mL), 506.2 mg (5.56 mmol) hydrazine-hydrate was added, and the reaction mixture was heated under reflux. The reaction mixture was stirred and heated under reflux 2 h, acetone (2 mL) was added and the stirring was continued for further 30 minutes. The reaction mixture was concentrated and the solid residue was separated to afford 623.0 mg (yield: 99 %) of the racemic intermediate (INT-015), which was used for the N-acylation reaction (step 5) without purification.

| | |
|---|---|
| Formula: C₁₅H₁₆N₆ | Molecular weight: 280.33 g/mol |

HPLC-MS acid (ESI positive): 281.1 [M+H]⁺

The intermediates of the formulae (**INT-016**) - (**INT-017**) listed in Table 2 below can be prepared analogously.

### Step 5

### N-[1-(5-benzyl-2-pyrimidin-2-yl-1,2,4-triazol-3-yl)ethyl]-3,5-bis(trifluoromethyl)benzamide (example I-82)

To 112.4 mg (0.40 mmol) (1S)-1-[2-(5-chloro-2-pyridyl)-5-methylsulfanyl-1,2,4-triazol-3-yl]ethanamine (INT-015), 117.0 mg (0.44 mmol) bis-3,5-(trifluoromethyl)-benzoic acid, 79.7 mg (0.61 mmol) DIPEA in DMF (3.5 mL), 206.0 mg (0.54 mmol) HATU was added, and the reaction mixture was stirred at room temperature overnight. The reaction mixture was filtered, concentrated and the residue was purified by HPLC with a MeCN/water gradient (acid) to afford 43.4 mg (purity: 100 %; yield: 19 %) of the racemic title compound.

| | |
|---|---|
| Formula: C₂₄H₁₈Cl₃F₆N₆O | Molecular weight: 520.44 g/mol |

HPLC-MS acid (ESI positive): 521.2 [M+H]⁺

The compounds of the formula (**I-83**)-(**I-87**) and (**I-109**)-(**I-111**) listed in Table 1 below can be prepared analogously.

### Synthesis of tert-butyl N-[5-[1-[(3,5-dibromobenzoyl)amino]ethyl]-1-[5-(morpholine-4-carbonyl)-2-pyridyl]-1,2,4-triazol-3-yl]carbamate (example I-106, reference compound)

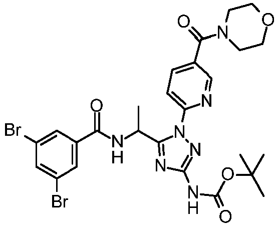

### Step 1: 6-[3-(tert-butoxycarbonylamino)-5-[1-(1,3-dioxoisoindolin-2-yl)ethyl]-1,2,4-triazol-1-yl]pyridine-3-carboxylic acid

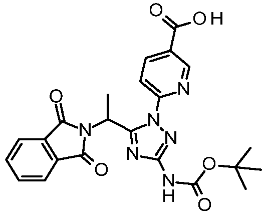

To a solution of 1.2 g (3.06 mmol) *tert-butyl N*-[(*E*)-*N*-[(2*S*)-2-(1,3-dioxoisoindolin-2-yl)propanoyl]-*C-*methylsulfanyl-carbonimidoyl]carbamate in pyridine (10 ml), 0.7 g (3.69 mmol) 6-hydrazinopyridine-3-carboxylic acid hydrochloride was added and the reaction mixture was stirred for 3.5 h at 80 °C temperature. Afterwards the solvent was evaporated *in vacuo.* The racemic crude product (2.7 g, purity: 56%) was used in step 2 without purification.

| | |
|---|---|
| Formula: C₂₃H₂₂N₆O₆ | Molecular weight: 478.46 g/mol |

HPLC-MS (ESI positive) [m/z]: 479.2 [M+H]⁺

### Step 2: tert-Butyl N-[5-[1-(1,3-dioxoisoindolin-2-yl)ethyl]-1-[5-(morpholine-4-carbonyl)-2-pyridyl]-1,2,4-triazol-3-yl]carbamate

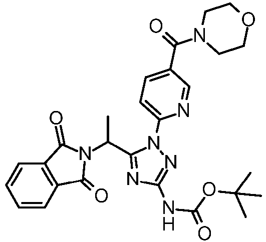

To a solution of 2.7 g (3.16 mmol) 6-[3-(tert-butoxycarbonylamino)-5-[1-(1,3-dioxoisoindolin-2-yl)ethyl]-1,2,4-triazol-1-yl]pyridine-3-carboxylic acid (step 1) in acetonitril (50 ml), 1.4 g (3.79 mmol) HATU , 531 mg (4.10 mmol) DIPEA and 330 mg (3.76 mmol) morpholine were added and the reaction mixture was stirred at room temperature overnight. Afterwards the solvent was evaporated *in vacuo.* The crude product was chromatographed with a cyclohexane/acetone gradient on silica gel to afford 1.3 g (purity: 80 %; yield: 60 %) of the racemic title compound.

| | |
|---|---|
| Formula: C₂₇H₂₉N₇O₆ | Molecular weight: 547.57 g/mol |

HPLC-MS (ESI positive) [m/z]: 548.2 [M+H]⁺

### Step 3: tert-butyl N-[5-(1-aminoethyl)-1-[5-(morpholine-4-carbonyl)-2-pyridyl]-1,2,4-triazol-3-yl]carbamate (INT-018)

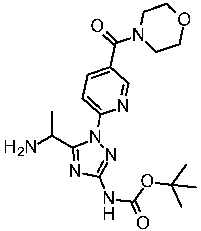

To 880 mg (1.60 mmol) *tert-*butyl *N*-[5-[1-(1,3-dioxoisoindolin-2-yl)ethyl]-1-[5-(morpholine-4-carbonyl)-2-pyridyl]-1,2,4-triazol-3-yl]carbamate (step 2) in ethanol (20 mL), 370 mg (4.06 mmol) hydrazine-hydrate were added, and the reaction mixture was heated under reflux. After 30 minutes a colorless precipitate was formed. The reaction mixture was stirred and heated under reflux two additional hours, acetone (2 mL) was added and the heating was continued for further 30 minutes. The reaction mixture was concentrated and the solid residue was treated with ethanol. After filtration, the filtrate was evaporated under reduced pressure to afford 900 mg of the racemic intermediate, which was used in step 4 without purification.

| | |
|---|---|
| Formula: C₁₉H₂₇N₇O₄ | Molecular weight: 417.21 g/mol |

HPLC-MS (ESI positive) [m/z]: 418.2 [M+H]⁺

The intermediate of the formula (**INT-019**) listed in Table 2 below can be prepared analogously.

### Step 4: tert-butyl N-[5-[1-[(3,5-dibromobenzoyl)amino]ethyl]-1-[5-(morpholine-4-carbonyl)-2-pyridyl]-1,2,4-triazol-3-yl]carbamate

To 900 mg (2.15 mmol) *tert-*butyl *N*-[5-(1-aminoethyl)-1-[5-(morpholine-4-carbonyl)-2-pyridyl]-1,2,4-triazol-3-yl]carbamate (INT-018), 297 mg (1.03 mmol) 3,5-dibromobenzoic acid, 170 mg (1.31 mmol) DIPEA in acetonitrile (15 mL), 460 mg (1.21 mmol) HATU were added, and the reaction mixture was stirred at room temperature overnight. The reaction mixture was concentrated under reduced pressure and the solid residue was treated with dichloromethane and then extracted with a saturated aqueous NaHCO₃ solution and water. The organic phase was separated, dried over Na₂SO₄ and the solvent was evaporated under reduced pressure. The remaining crude product was chromatographed with a cyclohexane/acetone gradient on silica gel to afford 439 mg (purity: 98%; yield: 62%) of the racemic title compound.

| | |
|---|---|
| Formula: C₂₆H₂₉Br₂N₇O₅ | Molecular weight: 679.38 g/mol |

HPLC-MS (ESI positive) [m/z]: 680.1 [M+H]⁺

### Synthesis of N-[1-[5-amino-2-[5-(morpholine-4-carbonyl)-2-pyridyl]-1,2,4-triazol-3-yl]ethyl]-3,5-dibromo-benzamide (example I-107, reference compound)

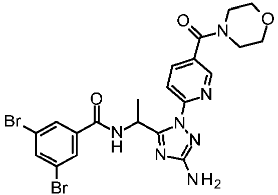

380 mg (0.55 mmol) *tert*-butyl *N*-[5-[1-[(3,5-dibromobenzoyl)amino]ethyl]-1-[5-(morpholine-4-carbonyl)-2-pyridyl]-1,2,4-triazol-3-yl]carbamate were dissolved in 4N HCl-dioxane solution (5 mL) and the mixture was stirred 18 h at room temperature. Then the solvent was evaporated under reduced pressure to afford 103 mg (purity: 96%) of the racemic title compound.

| | |
|---|---|
| Formula: C₂₁H₂₁Br₂N₇O₃ | Molecular weight: 579.26 g/mol |

HPLC-MS neutral (ESI-positive) [m/z]: 580.0 [M+H]⁺

### Synthesis of N-[(1S)-1-[5-(methylamino)-2-[5-(morpholine-4-carbonyl)thiazol-2-yl]-1,2,4-triazol-3-yl]ethyl]-3,5-bis(trifluoromethyl)benzamide (example I-126, reference compound)

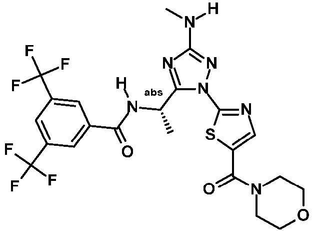

### Step 1: (2-hydrazinothiazol-5-yl)-morpholino-methanone

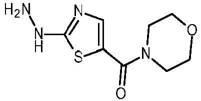

A mixture of 1.3 g (5.58 mmol) (2-chlorothiazol-5-yl)-morpholino-methanone (preparation, see WO 2014118186 A1) in THF (56.5 mL) and 11.2 ml (11.1 mmol) of a 1M solution of hydrazine in THF was refluxed for 2.5 h. After cooling to room temperature, the mixture was evaporated. The remaining 1.3 g crude product (yield: 99.8%) of the title compound was used for step 2.

**Step 2:** *tert*-butyl *N*-[5-[(1*S*)-1-(*tert*-butoxycarbonylamino)ethyl]-1-[5-(morpholine-4-carbonyl)thiazol-2-yl]-1,2,4-triazol-3-yl]-*N*-methyl-carbamate

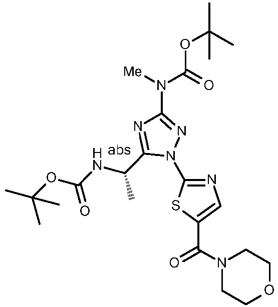

To 300 mg (1.58 mmol) *N*-Boc-alanin (Boc-Ala-OH purchased from Sigma-Aldrich) und 323.9 mg (1.58 mmol) *tert*-butyl *N*-methyl-*N*-(methylsulfanyl-carbonimidoyl)carbamate (preparation, see example 1-009, step 1) in *N,N*-dimethylformamide (20 mL), 481.3 mg (4.75 mmol) DIPEA and 904.3 mg (2.37 mmol) HATU were added, and the reaction mixture was stirred 3 hours at room temperature. Then, to the *in-situ* formed *tert*-butyl *N*-[*N*-[(2*S*)-2-(tert-butoxycarbonylamino)propanoyl]-C-methylsulfanyl-carbonimidoyl]-N-methyl-carbamate acetic acid (0.109 mL), 434.4 mg (1.90 mmol) (2-hydrazinothiazol-5-yl)-morpholino-methanone were added and the reaction mixture was stirred for 1 h at 50 °C temperature. Afterwards, the reaction mixture was treated with ethyl acetate and then extracted with a saturated aqueous NaCl solution and water. The organic phase was separated, dried over Na₂SO₄ and the solvent was evaporated under reduced pressure. The remaining crude product was chromatographed by HPLC with a water/acetonitrile gradient (neutral) to afford 273.6 mg (purity: 100%; yield: 22%) of the title compound. The enantiomeric excess of the chiral title compounds has been determined *via* screen OD_RH (acid): ee-value = 100%; Rₜ = 13.62 min.

| | |
|---|---|
| Formula: C₂₃H₃₅F₆N₇O₆S | Molecular weight: 537.64 g/mol |

UPLC-MS neutral [m/z]: 538.2 [M+H]⁺

### Step 3: [2-[5-[(1S)-1-aminoethyl]-3-(methylamino)-1,2,4-triazol-1-yl]thiazol-5-yl]-morpholino-methanone (INT-020)

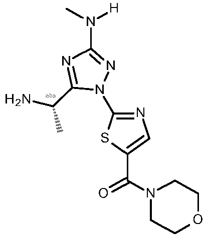

273.6 mg (0.55 mmol) *tert-*butyl *N*-[5-[(1*S*)-1-(*tert*-butoxycarbonylamino)ethyl]-1-[5-(morpholine-4-carbonyl)thiazol-2-yl]-1,2,4-triazol-3-yl]-N-methyl-carbamate were dissolved in 4N HCl-dioxane solution (50 mL) and the mixture was stirred 18 h at room temperature. Then the solvent was evaporated under reduced pressure to afford 169 mg (purity: 98%) of the title compound, which can be used for the coupling reaction in step 4.

| | |
|---|---|
| Formula: C₁₃H₁₉N₇O₂S | Molecular weight: 337.40 g/mol |

### Step 4: N-[(1S)-1-[5-(methylamino)-2-[5-(morpholine-4-carbonyl)thiazol-2-yl]-1,2,4-triazol-3-yl]ethyl]-3,5-bis(trifluoromethyl)benzamide (example I-126)

To 164.8 mg (0.48 mmol) [2-[5-[(1S)-1-aminoethyl]-3-(methylamino)-1,2,4-triazol-1-yl]thiazol-5-yl]-morpholino-methanone (INT-020), 130.0 mg (0.48 mmol) bis-3,5-(trifluoromethyl)-benzoic acid, 82.1 mg (0.63 mmol) DIPEA in N,N-dimethylformamide (4.5 mL), 22.9 mg (0.58 mmol) HATU were added, and the reaction mixture was stirred at room temperature overnight. The reaction mixture was concentrated under reduced pressure and the remaining crude product was chromatographed by preparative HPLC with a water/acetonitrile gradient (neutral) to afford 142.1 mg (purity: 96%; yield: 48%) of the title compound.

| | |
|---|---|
| Formula: C₂₂H₂₁F₆N₇O₃S | Molecular weight: 577.51 g/mol |

HPLC-MS neutral (ESI-positive) [m/z]: 578.1 [M+H]⁺
¹³C-NMR peaklist (150.9 MHz, d₆-DMSO): δ = 164.1849 (4.3); 163.2775 (3.2); 160.2210 (3.6); 159.8269 (3.5); 158.4937 (4.1); 141.9436 (3.6); 135.9002 (3.7); 131.0035 (0.8); 130.7826 (2.6); 130.5621 (2.6); 130.3417 (0.8); 129.1643 (4.1); 128.5089 (2.7); 125.9904 (0.8); 125.3732 (1.0); 124.1812 (2.2); 122.3721 (2.2); 120.5633 (0.5); 66.1823 (6.1); 43.8331 (4.4); 40.2357 (9.7); 40.1163 (52.1); 39.9775 (155.2); 39.8386 (304.3); 39.6995 (356.3); 39.5604 (301.7); 39.4213 (151.0); 39.2822 (49.0); 29.3257 (6.4); 18.4308 (7.0); 1.3248 (0.6); 0.2743 (2.3) ppm.

### Synthesis of N-[(1S)-1-[5-(methylamino)-2-pyrimidin-2-yl-1,2,4-triazol-3-yl]ethyl]-3,5-bis(trifluoromethyl)benzamide (example I-127, reference compound)

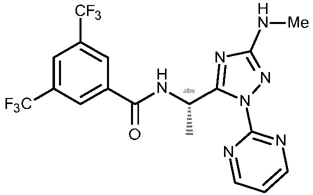

### Step 1: tert-butyl N-[5-[(1S)-1-(tert-butoxycarbonylamino)ethyl]-1-pyrimidin-2-yl-1,2,4-triazol-3-yl]-N-methyl-carbamate

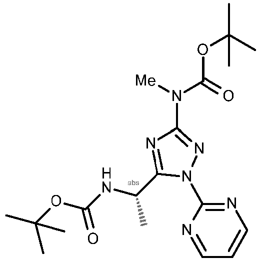

To 1.0 g (5.28 mmol) *N*-Boc-alanin (Boc-Ala-OH purchased from Sigma-Aldrich) und 1.1 g (5.28 mmol) *tert-*butyl N-methyl-N-(methylsulfanyl-carbonimidoyl)carbamate (preparation, see example 1-009, step 1) in *N,N*-dimethylformamide (66.6 mL), 1.6 g (15.8 mmol) DIPEA and 3.0 g (7.92 mmol) HATU were added, and the reaction mixture was stirred 3 hours at room temperature. Then, to the in-situ formed *tert-*butyl *N*-[*N*-[(2*S*)-2-(tert-butoxycarbonylamino)propanoyl]-C-methylsulfanyl-carbonimidoyl]-*N*-methylcarbamate acetic acid (0.327 mL), 628.7 mg (5.71 mmol) pyrimidin-2-ylhydrazine were added and the reaction mixture was stirred for 1 h at 50 °C temperature. Afterwards, the reaction mixture was treated with ethyl acetate and then extracted with a saturated aqueous NaCl solution and water. The organic phase was separated, dried over Na₂SO₄ and the solvent was evaporated under reduced pressure. The remaining crude product was chromatographed by HPLC with a water/acetonitrile gradient (neutral) to afford 970 mg (purity: 100%; yield: 49%) of the title compound. The enantiomeric excess of the chiral title compounds has been determined via screen OD_RH (acid): ee-value = 100%; Rₜ = 10.67 min..

| | |
|---|---|
| Formula: C₁₉H₂₉N₇O₄ | Molecular weight: 419.48 g/mol |

HPLC-MS neutral [m/z]: 420.2 [M+H]⁺
¹H-NMR(400.2 MHz, d₆-DMSO): δ = 8.9825 (1.4); 8.9704 (1.4); 7.6350 (0.7); 7.6228 (1.3); 7.6107 (0.7); 3.3275 (13.3); 3.2074 (6.7); 2.5122 (4.7); 2.5079 (9.0); 2.5034 (11.5); 2.4989 (8.2); 2.4945 (3.9); 2.0751 (0.5); 1.4508 (1.4); 1.4305 (16.0); 1.2951 (5.2); 1.0440 (0.4); 0.0079 (0.6); -0.0002 (14.4); -0.0085 (0.6) ppm.

### Step 2: 5-[(1S)-1-aminoethyl]-N-methyl-1-pyrimidin-2-yl-1,2,4-triazol-3-amine hydrochlorid (INT-021)

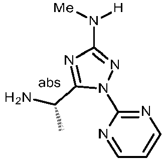

1.75 g (4.18 mmol) *tert*-butyl *N*-[5-[(1*S*)-1-(tert-butoxycarbonylamino)ethyl]-1-pyrimidin-2-yl-1,2,4-triazol-3-yl]-*N*-methyl-carbamate were dissolved in 4N HCl-dioxane solution (35 mL) and the mixture was stirred 18 h at room temperature. Then the solvent was evaporated under reduced pressure to afford 1.0 g (yield: 99%) of the title compound, which can be used for the coupling reaction in step 3.

| | |
|---|---|
| Formula: C₉H₁₃N₇ HCl | Molecular weight: 255.71 g/mol |

HPLC-MS acid (ESI-positive) [m/z]: 220.1 [M-HCl]⁺

### Step 3: N-[(1S)-1-[5-(methylamino)-2-pyrimidin-2-yl-1,2,4-triazol-3-yl]ethyl]-3,5-bis(trifluoromethyl) benzamide (example I-127)

To 200.0 mg (0.75 mmol) bis-3,5-(trifluoromethyl)-benzoic acid, 126.2 mg (0.97 mmol) DIPEA in *N,N-*dimethylformamide (6.9 mL) and 342.9 mg (0.9 mmol) HATU were added, and the reaction mixture was stirred for 10 minutes. Then 164.8 mg (0.48 mmol) 5-[(1S)-1-aminoethyl]-N-methyl-1-pyrimidin-2-yl-1,2,4-triazol-3-amine (INT-021) were added and the reaction mixture was stirred at room temperature overnight. The reaction mixture was concentrated under reduced pressure and the remaining crude product was chromatographed by preparative HPLC with a water/acetonitrile gradient (neutral) to afford 181.3 mg (purity: 100%; yield: 52%) of the title compound. The enantiomeric excess of the chiral title compounds has been determined via screen OD_RH (acid): *ee*-value = 100%; Rₜ = 8.88 min.

| | |
|---|---|
| Formula: C₁₈H₁₅F₆N₇O | Molecular weight: 459.35 g/mol |

HPLC-MS neutral (ESI-positive) [m/z]: 460.1 [M+H]⁺
¹³C-NMR peaklist (150.9 MHz, d₆-DMSO): δ = 163.8284 (3.1); 163.0033 (2.4); 159.3393 (3.0); 159.2264 (7.0); 155.3047 (2.4); 136.1059 (2.8); 130.9315 (0.5); 130.7114 (1.8); 130.4909 (1.9); 130.2706 (0.6); 128.4171 (2.0); 128.3979 (2.0); 125.9791 (0.5); 125.2183 (0.8); 124.1697 (1.5); 122.3607 (1.4); 120.5516 (0.4); 119.2484 (2.9); 44.2100 (3.8); 40.3488 (0.3); 40.2360 (5.8); 40.1163 (31.6); 39.9776 (94.0); 39.8386 (184.3); 39.6996 (215.8); 39.5605 (182.8); 39.4213 (91.5); 39.2822 (29.8); 29.5907 (5.2); 18.9774 (5.1) ppm.

The compounds of the formula (**I-128**)-(**I-132**), (**I-136**), (**I-137**), (**I-142**)-(**I-143**), (**I-180**)-(**I-186**), (**I-202**), (**I-206**)-(**I-210**), (**I-212**), (**I-217**)-(**I-219**), (**I-223**), (**I-226**)-(**I-228**) and (**I-231**) listed in Table 1 below can be prepared analogously.

Alternatively, the compound of the formula (**I-127**) can be prepared by chromatographic separation of the racemic compound of the formula (**I-009**) forming the compound of the formula (**I-133**) and following *N-*Boc group cleavage.

### Step 1: tert-butyl N-[5-[(1S)-1-[[3,5-bis(trifluoromethyl)benzoyl]amino]ethyl]-1-pyrimidin-2-yl-1,2,4-triazol-3-yl]-N-methyl-carbamate (example I-133, reference compound)

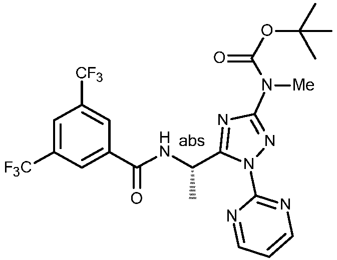

2.3 g (0.41 mmol) example 1-009 (crude material) was separated by SFC-chromatography on a chiral stationary phase (Chiracel OD-H). The substances were isolated from the fractions by evaporation at 40 °C under vacuum. The last used solvent was methanol and the substance was dried at 10 mbar for 10 min to afford two charges of the title compound, 721 mg (ee-value = 99%, Rₜ = 2.7 min) and 98 mg (ee-value = 92.3%Rt = 2.7 min).

As side product 860 mg (*ee*-value = 98.9%; Rₜ = 2.4 min) of the *tert*-butyl *N*-[5-[(1*R*)-1-[[3,5-bis(trifluoromethyl) benzoyl]amino]ethyl]-1-pyrimidin-2-yl-1,2,4-triazol-3-yl]-*N*-methyl-carbamate was isolated.

### Step 2: N-[(1S)-1-[5-(methylamino)-2-pyrimidin-2-yl-1,2,4-triazol-3-yl]ethyl]-3,5-bis(trifluoromethyl) benzamide (example I-127)

721 mg (1.28 mmol) *tert*-butyl N-[5-[(1*S*)-1-[[3,5-bis(trifluoromethyl)benzoyl]amino]ethyl]-1-pyrimidin-2-yl-1,2,4-triazol-3-yl]-*N*-methyl-carbamate (example I-133) were dissolved in 4N HCl-dioxane solution (10 mL) and the mixture was stirred 18 h at room temperature. Then the solvent was evaporated under reduced pressure to afford 101 mg (purity: 99.1; yield: 17%) of the title compound together with 297 mg unsoluble solid. Specific rotation [α]₅₈₉ = +107.5° (5.0 ml, DMSO at 20 °C).

| | |
|---|---|
| Formula: C₁₈H₁₅F₆N₇O | Molecular weight: 459.35 g/mol |

HPLC-MS neutral (ESI-positive) [m/z]: 460.1 [M+H]⁺
¹H-NMR peaklist (600.1 MHz, d₆-DMSO): δ = 9.4999 (3.1); 9.4880 (3.1); 8.8706 (15.4); 8.8625 (15.5); 8.4678 (10.6); 8.3124 (4.6); 7.4473 (4.1); 7.4393 (7.7); 7.4312 (3.9); 6.2352 (0.9); 6.2269 (3.0); 6.2184 (2.9); 6.2100 (0.8); 6.0711 (0.5); 6.0596 (2.3); 6.0479 (3.6); 6.0362 (2.3); 6.0246 (0.5); 3.3436 (0.5); 3.3167 (538.4); 2.7470 (15.9); 2.7386 (16.0); 2.6188 (1.7); 2.6160 (3.8); 2.6130 (5.2); 2.6100 (3.8); 2.6070 (1.8); 2.5371 (0.6); 2.5220 (10.4); 2.5189 (13.2); 2.5158 (13.4); 2.5069 (290.8); 2.5040 (615.6); 2.5009 (852.6); 2.4979 (639.4); 2.4950 (308.9); 2.4552 (0.5); 2.4476 (0.4); 2.3907 (1.8); 2.3879 (3.8); 2.3849 (5.3); 2.3818 (3.9); 2.3789 (1.9); 2.0738 (1.2); 1.6148 (12.5); 1.6032 (12.6); -0.0001 (5.3) ppm.

### Synthesis of N-[(1S)-1-(5-amino-2-pyrimidin-2-yl-1,2,4-triazol-3-yl)ethyl]-3,5-bis(trifluoromethyl) benzamide (example I-134, reference compound)

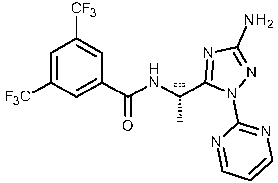

### Step 1: tert-butyl N-[5-[(1S)-1-[[3,5-bis(trifluoromethyl)benzoyl]amino]ethyl]-1-pyrimidin-2-yl-1,2,4-triazol-3-yl]carbamate (example I-135, reference compound)

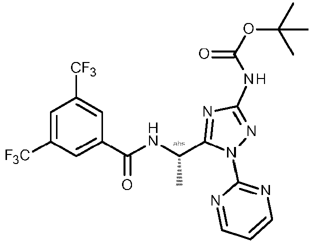

To 2.3 g (8.91 mmol) bis-3,5-(trifluoromethyl)-benzoic acid in acetonitrile (162 mL), 1.6 g (12.3 mmol) DIPEA and 3.8 g (10.0 mmol) HATU were added, and the reaction mixture was stirred for 10 minutes. Then 2,7 g (8.91 mmol) racemic *tert-*butyl *N*-[5-(1-aminoethyl)-1-pyrimidin-2-yl-1,2,4-triazol-3-yl]carbamate (INT-001) were added and the reaction mixture was stirred at room temperature overnight. Afterwards, the reaction mixture was treated with water and then extracted with dichloromethane. The organic phase was separated, dried over Mg₂SO₄ and the solvent was evaporated under reduced pressure. The remaining crude product was chromatographed by preparative HPLC with a water/acetonitrile gradient (neutral) to afford 862 mg (purity: 98%) of a racemic mixture, which was separated by SFC-chromatography on a chiral stationary phase (Chiracel OD-H). The substances were isolated from the fractions by evaporation at 40 °C under vacuum. The substance was dried at 10 mbar for 10 min to afford two charges, 270 mg (yield: 5.5%) of the title compound and 280 mg (yield: 5.8%) of the *tert*-butyl *N*-[5-[(1*R*)-1-[[3,5-bis(trifluoromethyl)benzoyl]amino]ethyl]-1-pyrimidin-2-yl-1,2,4-triazol-3-yl] carbamate.

| | |
|---|---|
| Formula: C₂₂H₂₁F₆N₇O₃ | Molecular weight: 545.44 g/mol |

UPLC-MS neutral [m/z]: 446.2 [M+H]⁺

### Step 2: N-[(1S)-1-(5-amino-2-pyrimidin-2-yl-1,2,4-triazol-3-yl)ethyl]-3,5-bis(trifluoromethyl) benzamide (example I-134)

259 mg (0.47 mmol) *tert*-butyl *N*-[5-[(1*S*)-1-[[3,5-bis(trifluoromethyl)benzoyl]amino]ethyl]-1-pyrimidin-2-yl-1,2,4-triazol-3-yl]carbamate (example 1-135) were dissolved in 4N HCl-dioxane solution (5 mL) and the mixture was stirred 18 h at room temperature. Then the solvent was evaporated under reduced pressure to afford 199 mg (purity: 93; yield: 87.5%) of the title compound. The enantiomeric excess of the chiral title compounds has been determined via screen OD_RH (acid): *ee*-value = 98.6%; Rₜ = 10.34 min.

| | |
|---|---|
| Formula: C₁₇H₁₃F₆N₇O | Molecular weight: 445.33 g/mol |

HPLC-MS neutral (ESI-positive) [m/z]: 446.1 [M+H]⁺
¹³C-NMR peaklist (150.9 MHz, d₆-DMSO): δ = 162.9407 (1.0); 162.7383 (0.4); 159.2277 (1.0); 158.9032 (0.4); 155.2440 (0.4); 136.0900 (1.2); 130.9293 (0.2); 130.7092 (0.8); 130.4889 (0.9); 130.2682 (0.3); 128.3989 (1.1); 125.9811 (0.2); 125.2194 (0.4); 124.1722 (0.7); 122.3632 (0.7); 119.3218 (0.5); 91.3806 (0.4); 72.3366 (0.3); 70.6930 (0.3); 66.5235 (7.0); 60.3557 (0.3); 59.3842 (0.5); 44.1276 (0.7); 43.7807 (0.2); 40.2357 (6.3); 40.1162 (48.3); 39.9775 (143.6); 39.8385 (281.5); 39.6995 (329.3); 39.5604 (279.1); 39.4213 (139.9); 39.2822 (45.6); 19.0353 (1.3); 18.9788 (0.2); 0.2795 (1.5) ppm.

### Synthesis of N-[(1S)-1-[5-(dimethylamino)-2-pyrimidin-2-yl-1,2,4-triazol-3-yl]ethyl]-3,5-bis(trifluoromethyl)benzamide (example I-141, reference compound)

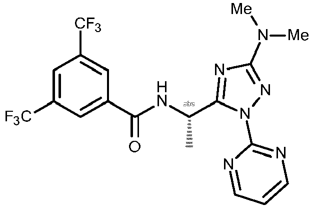

### Step 1: tert-Butyl N-[(1S)-1-[5-(dimethylamino)-2-pyrimidin-2-yl-1,2,4-triazol-3-yl]ethyl]carbamate

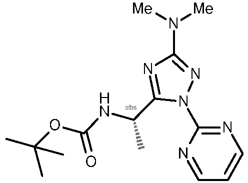

To 8.2 g (43.60 mmol) N-Boc-alanin (Boc-Ala-OH purchased from Sigma-Aldrich) in tetrahydrofurane (125 mL), 13.2 g (130.80 mmol) triethylamine (18.2 mL) and 24.8 g (65.40 mmol) HATU were added, and the reaction mixture was stirred for 10 minutes. Then 4.5 g (38.20 mmol) N,N-dimethyl(methylsulfa nyl)methanimidamide (can be perchased from Enamine building blocks, 57618-94-7) were added, and the reaction mixture was stirred 1 hour at 80°C. Then, to the in-situ formed *tert*-butyl *N*-[(1*S*)-2-[(*E*)-[dimethylamino(methylsulfanyl)methylene]amino]-1-methyl-2-oxo-ethyl]carbamate acetic acid (0.93 mL), 628.7 mg (5.71 mmol) pyrimidin-2-ylhydrazine in N,N-dimethylformamide (20 mL) were added and the reaction mixture was stirred for 1 h at 50 °C temperature. Afterwards, tetrahydrofurane was removed under reduced pressure and the reaction mixture was treated with water and then extracted with ethyl acetate. The organic phase was separated, dried over Na₂SO₄ and the solvent was evaporated under reduced pressure. The remaining crude product was chromatographed by HPLC with a water/acetonitrile gradient (neutral) to afford 4.1 mg (purity: 100%; yield: 90.5%) of the title compound. The enantiomeric excess of the chiral title compounds has been determined via screen OD_RH (acid): ee-value = 99.2%; Rₜ = 9.07 min.

| | |
|---|---|
| Formula: C₁₅H₂₃N₇O₂ | Molecular weight: 333.39 g/mol |

HPLC-MS neutral [m/z]: 334.2 [M+H]⁺

### Step 2: 5-[(1S)-1-aminoethyl]-N,N-dimethyl-1-pyrimidin-2-yl-1,2,4-triazol-3-amine (INT-022)

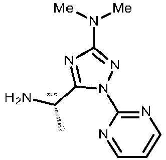

2.5 g (7.49 mmol) *tert-*butyl *N*-[(1*S*)-1-[5-(dimethylamino)-2-pyrimidin-2-yl-1,2,4-triazol-3-yl]ethyl]carbamate were dissolved in 4N HCl-dioxane solution (50 mL) and the mixture was stirred 18 h at room temperature. Then the solvent was evaporated under reduced pressure to afford 1.73 g (yield: 99%) of the title compound, which can be used for the coupling reaction in step 3.

| | |
|---|---|
| Formula: C₁₀H₁₅N₇ | Molecular weight: 233.27 g/mol |

HPLC-MS neutral (ESI-positive) [m/z]: 234.2 [M+H]⁺

### Step 3: N-[(1S)-1-[5-(Dimethylamino)-2-pyrimidin-2-yl-1,2,4-triazol-3-yl]ethyl]-3,5-bis (trifluoromethyl)benzamide (example I-141)

To 200.0 mg (0.75 mmol) bis-3,5-(trifluoromethyl)-benzoic acid, 126.2 mg (0.97 mmol) DIPEA in *N,N-*dimethylformamide (6.9 mL) and 342.9 mg (0.9 mmol) HATU were added, and the reaction mixture was stirred for 10 minutes. Then 175.3 mg (0.75 mmol) 5-[(1S)-1-aminoethyl]-N,N-dimethyl-1-pyrimidin-2-yl-1,2,4-triazol-3-amine (INT-022) were added an the reaction mixture was stirred at room temperature overnight. The reaction mixture was concentrated under reduced pressure and the remaining crude product was chromatographed by preparative HPLC with a water/acetonitrile gradient (neutral) to afford 43.8 mg (purity: 97.5%; yield: 12%) of the title compound.

| | |
|---|---|
| Formula: C₁₉H₁₇F₆N₇O | Molecular weight: 473.38 g/mol |

HPLC-MS neutral (ESI-positive) [m/z]: 474.1 [M+H]⁺
¹³C-NMR peaklist (150.9 MHz, CD₃CN): δ = 165.9962 (1.1); 164.7345 (1.1); 160.2068 (1.6); 159.8730 (6.4); 156.5348 (0.8); 137.6856 (1.4); 132.5785 (0.3); 132.3555 (1.0); 132.1325 (1.1); 131.9095 (0.3); 129.0356 (1.4); 129.0168 (1.4); 126.9883 (0.2); 126.0362 (0.3); 126.0109 (0.6); 125.9860 (0.8); 125.9611 (0.6); 125.9361 (0.3); 125.1852 (0.6); 123.3824 (0.6); 119.9591 (3.3); 118.2929 (140.1); 45.8585 (3.4); 38.4922 (7.0); 19.6631 (3.5); 1.7124 (21.6); 1.5748 (72.5); 1.4371 (150.3); 1.2995 (178.3); 1.1618 (155.5); 1.0943 (6.8); 1.0241 (80.8); 0.9561 (6.2); 0.8865 (29.4); 0.8183 (4.5); 0.6805 (2.8); -0.0852 (1.2); -4.8936 (0.4) ppm.

The compounds of the formula (**I-138**)-(**I-141**), (**I-156**), (**I-157**), (**I-162**), (**I-164**)-(**I-166**), (**I-170**), (**I-171**), (**I-176**)-(**I-179**), (**I-191**)-(**I-194**), (**I-229**), (**I-232**)-(**I-240**) and (**I-252**)-(**I-270**) listed in Table 1 below can be prepared analogously.

The racemic compounds of the formula (**I-156**), (**I-157**), (**I-162**), (**I-164**)-(**I-166**), (**I-171**) and (**I-191**)-(**I-194**) listed in Table 1 below can be prepared analogously by using racemic N-Boc-alanin as starting component.

### Synthesis of N-[(1S)-1-(5-morpholino-2-pyrimidin-2-yl-1,2,4-triazol-3-yl)ethyl]-3,5-bis(trifluoromethyl)benzamide (example I-145, reference compound)

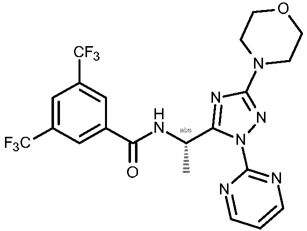

### Step 1: tert-Butyl N-[(1S)-1-(5-morpholino-2-pyrimidin-2-yl-1,2,4-triazol-3-yl)ethyl]carbamate

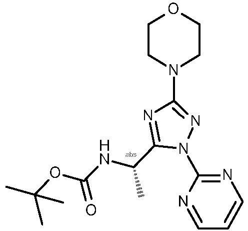

To 2.6 g (13.67 mmol) N-Boc-alanin (Boc-Ala-OH purchased from Sigma-Aldrich) and 8.8 g (20.51 mmol) HATU in *N,N*-dimethylformamide (133 mL), 4.1 g (41.0 mmol) DIPEA (5.7 mL) and were added, and the reaction mixture was stirred for 10 minutes. Then 4.5 g (38.20 mmol) morpholine-4-carboximidothioic acid methyl ester (can be perchased from Chemieliva Pharmaceutical Product List, 89269-30-7) were added, and the reaction mixture was stirred 3 hour at room temperature. Then, to the in-situ formed methyl (4*E*)-*N*-[(2*S*)-2-(*tert*-butoxycarbonylamino)propanoyl]morpholine-4-carboximido-thioate acetic acid (0.93 mL), 1.8 g (16.41 mmol) pyrimidin-2-ylhydrazine in *N,N*-dimethylformamide (100 mL) were added and the reaction mixture was stirred for 1 h at 50 °C and 18 hours at room temperature. Afterwards, N,N-dimethylformamide was removed under reduced pressure and the reaction mixture treated with water and then extracted with ethyl acetate. The organic phase was separated, dried over Na₂SO₄ and the solvent was evaporated under reduced pressure. The remaining crude product was chromatographed by HPLC with a water/acetonitrile gradient (neutral) to afford 1.2 g (purity: 99%; yield: 23%) of the title compound.

| | |
|---|---|
| Formula: C₁₇H₂₅N₇O₃ | Molecular weight: 375.43 g/mol |

HPLC-MS acid [m/z]: 376.2 [M+H]⁺

### Step 2: (1S)-1-(5-morpholino-2-pyrimidin-2-yl-1,2,4-triazol-3-yl)ethanamine-hydrochlorid (INT-023) (JES 13786-1-1)

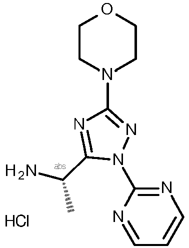

1.19 g (3.17 mmol) *tert*-butyl *N*-[(1*S*)-1-(5-morpholino-2-pyrimidin-2-yl-1,2,4-triazol-3-yl)ethyl]carbamate were dissolved in 4N HCl-dioxane solution (30 mL) and the mixture was stirred 18 h at room temperature. Then the solvent was evaporated under reduced pressure to afford 1.1 g crude product (yield: 109%) of the title compound, which can be used for the coupling reaction in step 3.

| | |
|---|---|
| Formula: C₁₂H₁₇N₇O HCl | Molecular weight: 311.77 g/mol |

HPLC-MS neutral (ESI-positive) [m/z]: 276.2 [M-HCl]⁺

### Step 3: N-[(1S)-1-(5-Morpholino-2-pyrimidin-2-yl-1,2,4-triazol-3-yl)ethyl]-3,5-bis(trifluoromethyl)benzamide (example I-145)

To 200.0 mg (0.75 mmol) bis-3,5-(trifluoromethyl)-benzoic acid, 126.2 mg (0.97 mmol) DIPEA in *N,N-*dimethylformamide (6.9 mL) and 342.9 mg (0.9 mmol) HATU were added, and the reaction mixture was stirred for 10 minutes. Then 175.3 mg (0.75 mmol) (1S)-1-(5-morpholino-2-pyrimidin-2-yl-1,2,4-triazol-3-yl)ethanamine-hydrochlorid (INT-023) were added an the reaction mixture was stirred at room temperature overnight. The reaction mixture was concentrated under reduced pressure and the remaining crude product was chromatographed by preparative HPLC with a water/acetonitrile gradient (neutral) to afford 144.6 mg (purity: 100%; yield: 37%) of the title compound. The enantiomeric excess of the chiral title compounds has been determined via screen OD_RH (acid): ee-value = 100%; Rₜ = 9.86 min.

| | |
|---|---|
| Formula: C₂₁H₁₉F₆N₇O₂ | Molecular weight: 515.42 g/mol |

UPLC-MS neutral (ESI-positive) [m/z]: 516.2 [M+H]⁺
¹³C-NMR peaklist (150.9 MHz, d6-DMSO): δ = 164.1457 (2.8); 163.1322 (2.2); 159.3835 (3.9); 159.3678 (7.0); 155.2292 (2.2); 136.0798 (2.6); 130.9320 (0.5); 130.7115 (1.7); 130.4912 (1.8); 130.2705 (0.6); 128.4139 (1.9); 125.9634 (0.5); 125.2398 (0.8); 124.1541 (1.4); 122.3451 (1.3); 120.5362 (0.4); 119.8711 (2.7); 65.7703 (6.3); 46.4104 (6.2); 44.0056 (3.5); 40.2359 (4.5); 40.1162 (23.9); 39.9775 (71.2); 39.8386 (139.6); 39.6995 (163.5); 39.5604 (138.5); 39.4213 (69.4); 39.2822 (22.5); 18.9118 (4.8); 0.2708 (1.4)

The compounds of the formula (**I-146**),(**I-147**), (**I-158**)-(**I-161**), (**I-174**), (**I-175**), (**I-195**), (**I-197**), (**I-199**), (**I-201**) and (**I-204**) listed in Table 1 (wherin R⁵ is morpholinyl) below can be prepared analogously. The racemic compounds of the formula (**I-158**)-(**I-161**) and (**I-201**) listed in Table 1 (wherin R⁵ is morpholinyl) below can be prepared analogously by using racemic N-Boc-alanin as starting component.

The compounds of the formula (**I-196**), (**I-198**), (**I-200**) and (**I-220**)-(**I-222**) listed in Table 1 (wherin R⁵ is piperidinyl) below can be prepared analogously.

The racemic compounds of the formula (**I-196**), (**I-198**) and (**I-200**) listed in Table 1 (wherin R⁵ is piperidinyl) below can be prepared analogously by using racemic N-Boc-alanin as starting component.

The compounds of the formula (**I-190**), (**I-203**), (**I-224**)-(**I-225**) and (**I-230**) listed in Table 1 (wherin R⁵ is pyrrolidinyl) below can be prepared analogously.

### Synthesis of N-[(1S)-1-[5-amino-2-(5-cyano-2-pyridyl)-1,2,4-triazol-3-yl]ethyl]-3-chloro-5-methylsulfonyl-benzamide (example I-148)

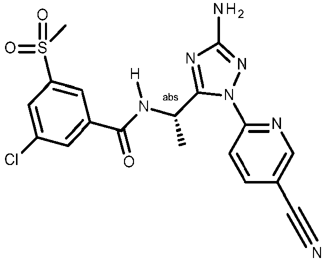

### Step 1: tert-butyl N-[(1S)-1-[5-(tert-butoxycarbonylamino)-2-(5-cyano-2-pyridyl)-1,2,4-triazol-3-yl]ethyl]carbamate

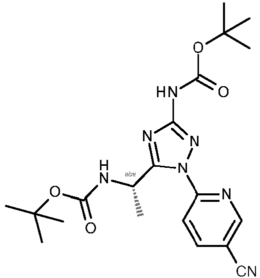

To 4.7 g (24.8 mmol) N-Boc-alanin (Boc-Ala-OH purchased from Sigma-Aldrich) and 14.1 g (37.0 mmol) HATU in *N,N*-dimethylformamide (50 mL), 7.5 g (74,10 mmol) DIPEA (18.2 mL) and 4,64 g (24.40 mmol) 1-*N*-Boc-2-methyl-isothiourea (purchased from ABCR) were added, and the reaction mixture was stirred for 3 hours at room temperature. Then, to the *in-situ* formed *tert*-butyl *N*-[(*Z*)-*N*-[(2*S*)-2-(*tert-*butoxycarbonylamino)propanoyl]-C-methylsulfanyl-carbonimidoyl]-*N*-carbamate acetic acid (3.9 mL), 8.3 g (61.8 mmol) 6-hydrazinopyridine-3-carbonitrile were added and the reaction mixture was stirred for 2 hours at 50 °C temperature. Afterwards, the solvent was removed under reduced pressure and the remaining crude product was chromatographed with a acetone/cyclohexane gradient (neutral) to afford 7.0 g (purity: 88%; yield: 27%) of the title compound. The enantiomeric excess of the chiral title compounds has been determined via screen OD_RH (acid): ee-value = 100%; Rₜ = 11.28 min.

| | |
|---|---|
| Formula: C₂₀H₂₇N₇O₄ | Molecular weight: 429.48 g/mol |

HPLC-MS neutral [m/z]: 230.1 [M+H, cleavage of 2 x Boc]⁺

### Step 2: 6-[3-amino-5-[(1S)-1-aminoethyl]-1,2,4-triazol-1-yl]pyridine-3-carbonitrile-hydrochloride (INT-025)

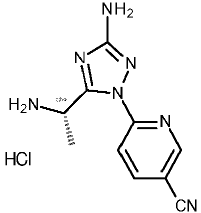

7.0 g (16.2 mmol) *tert-*butyl *N*-[(1*S*)-1-[5-(*tert*-butoxycarbonylamino)-2-(5-cyano-2-pyridyl)-1,2,4-triazol-3-yl]ethyl]carbamate were dissolved in 4N HCl-dioxane solution (50 mL) and the mixture was stirred 18 h at room temperature. Then the solvent was evaporated under reduced pressure to afford 4.8 g crude product (yield: 111%) of the title compound, which can be used for the coupling reaction in step 3. The enantiomeric excess of the chiral title compounds has been determined via screen OD_RH (acid): ee-value = 70.7%; Rₜ = 1.64 min.

| | |
|---|---|
| Formula: C₁₀H₁₁N₇ HCl | Molecular weight: 265.70 g/mol |

### Step 3: N-[(1S)-1-[5-amino-2-(5-cyano-2-pyridyl)-1,2,4-triazol-3-yl]ethyl]-3-chloro-5-methylsulfonylbenzamide (example 1-148)

To 230.0 mg (0.95 mmol) 3-chloro-5-methylsulfonyl-benzoic acid, 370.0 mg (2.86 mmol) DIPEA in acetonitrile (10 mL) and 434.3 mg (1.14 mmol) HATU were added, and the reaction mixture was stirred for 10 minutes. Then 330.0 mg (1.24 mmol) 6-[3-amino-5-[(1S)-1-aminoethyl]-1,2,4-triazol-1-yl]pyridine-3-carbonitrile-hydrochloride (INT-025) were added an the reaction mixture was stirred at room temperature overnight. The reaction mixture was concentrated under reduced pressure and the remaining crude product was chromatographed by preparative HPLC with a water/acetonitrile gradient (neutral) to afford 76.0 mg (purity: 100%; yield: 18%) of the title compound.

| | |
|---|---|
| Formula: C₁₈H₁₆ClN₇O₃S | Molecular weight: 445.89 g/mol |

UPLC-MS neutral (ESI-positive) [m/z]: 446.1 [M+H]⁺

The compounds of the formula (**I-154**), (**I-155**), (**I-169**), (**I-172**), (**I-173**), (**I-187**)-(**I-189**), (**I-205**), (I-**211**), (**I-213**)-(**I-216**) and (**I-241**)-(**I-251**) listed in Table 1 below can be prepared analogously.

### Synthesis of N-[(1S)-1-[5-[methoxy(methyl)amino]-2-pyrimidin-2-yl-1,2,4-triazol-3-yl]ethyl]-3,5-bis(trifluoromethyl)benzamide (example I-149, reference compound)

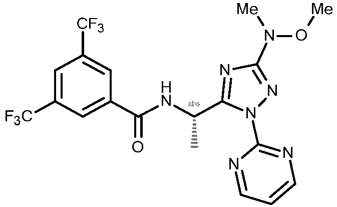

### Step 1: tert-butyl N-[(1S)-1-[5-[methoxy(methyl)amino]-2-pyrimidin-2-yl-1,2,4-triazol-3-yl]ethyl] carbamate

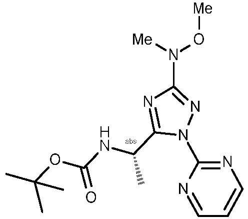

To 3.0 g (15.85 mmol) N-Boc-alanin (Boc-Ala-OH purchased from Sigma-Aldrich) and 9.0 g (23.78 mmol) HATU in *N,N*-dimethylformamide (200 mL), 2.1 g (20.61 mmol) DIPEA (2.9 mL) and 2.1 g (15.85 mmol) N-methoxy-N-methyl-carbamimidothioic acid methyl ester (preparation according to WO 9300336 A1) were added, and the reaction mixture was stirred for 3 hours at room temperature. Then, to the *in-situ* formed methyl *N*-[(2*S*)-2-(*tert*-butoxycarbonylamino)propanoyl]-2-methoxy-propanimido-thioate acetic acid (1.1 mL), 2.09 g (19.0 mmol) pyrimidin-2-yl-hydrazine were added and the reaction mixture was stirred for 1 h at 50 °C temperature. Afterwards, the reaction mixture treated with water and then extracted with ethyl acetate. The organic phase was separated, dried over Na₂SO₄ and the solvent was evaporated under reduced pressure. The remaining crude product was chromatographed by HPLC with a water/acetonitrile gradient (neutral) to afford 891.3 mg (purity: 97%; yield: 16%) of the title compound. The enantiomeric excess of the chiral title compounds has been determined via screen OD_RH (acid): ee-value = 100%; Rₜ = 8.70 min.

| | |
|---|---|
| Formula: C₁₅H₂₃N₇O₃ | Molecular weight: 349.39 g/mol |

HPLC-MS neutral [m/z]: 350.2 [M+H]⁺
¹H-NMR peaklist (400.2 MHz, d₆-DMSO): δ = 8.9480 (4.1); 8.9359 (4.1); 7.5811 (1.9); 7.5689 (3.5); 7.5568 (1.8); 7.5004 (0.6); 7.4805 (0.6); 5.5129 (0.4); 5.4957 (0.6); 5.4789 (0.4); 3.7441 (16.0); 3.3259 (29.4); 3.0877 (14.5); 2.6718 (0.4); 2.5252 (1.6); 2.5204 (2.4); 2.5118 (25.1); 2.5073 (51.0); 2.5028 (66.3); 2.4982 (46.9); 2.4937 (22.1); 2.3296 (0.4); 2.0504 (0.4); 1.5274 (1.1); 1.4358 (2.8); 1.4184 (2.6); 1.3057 (12.5); 1.0489 (0.8); 0.1460 (0.5); 0.0079 (4.6); -0.0002 (122.5); -0.0086 (4.7); -0.1496 (0.5) ppm

### Step 2: 5-[(1S)-1-aminoethyl]-N-methoxy-N-methyl-1-pyrimidin-2-yl-1,2,4-triazol-3-amine-hydrochloride (INT-026)

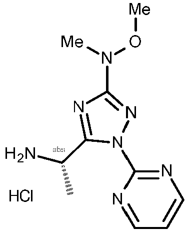

870.0 mg (2.49 mmol) *tert-*butyl *N*-[(1*S*)-1-[5-[methoxy(methyl)amino]-2-pyrimidin-2-yl-1,2,4-triazol-3-yl]ethyl] carbamate were dissolved in 4N HCl-dioxane solution (18.6 mL) and the mixture was stirred 18 h at room temperature. Then the solvent was evaporated under reduced pressure to afford the title compound, which can be used for the coupling reaction in step 3.

| | |
|---|---|
| Formula: C₁₀H₁₅N₇O HCl | Molecular weight: 285.73 g/mol |

HPLC-MS neutral (ESI-positive) [m/z]: 250.1 [M-HCl]⁺

### Step 3: N-[(1S)-1-[5-[methoxy(methyl)amino]-2-pyrimidin-2-yl-1,2,4-triazol-3-yl]ethyl]-3,5-bis(trifluoromethyl)benzamide (example I-149)

To 130.0 mg (0.48 mmol) bis-3,5-(trifluoromethyl)-benzoic acid, 82.0 mg (0.63 mmol) DIPEA in *N,N-*dimethylformamide (3.25 mL) and 222.9 mg (0.58 mmol) HATU were added, and the reaction mixture was stirred for 10 minutes. Then 139.5 mg (0.48 mmol) 5-[(1*S*)-1-aminoethyl]-N-methoxy-N-methyl-1-pyrimidin-2-yl-1,2,4-triazol-3-amine-hydrochloride (INT-026) were added an the reaction mixture was stirred at room temperature overnight. The reaction mixture was concentrated under reduced pressure and the remaining crude product was chromatographed by preparative HPLC with a water/acetonitrile gradient (neutral) to afford 60.3 mg (purity: 100%; yield: 25%) of the title compound.

| | |
|---|---|
| Formula: C₁₉H₁₇F₆N₇O₂ | Molecular weight: 489.38 g/mol |

HPLC-MS neutral (ESI-positive) [m/z]: 490.1 [M+H]⁺

The compounds of the formula (**I-150**)-(**I-153**) listed in Table 1 below can be prepared analogously.

### Synthesis of N-{1-[1-(5-cyano-1,3-thiazol-2-yl)-3-(methylsulfanyl)-1H-1,2,4-triazol-5-yl]ethyl}-3-(methylsulfonyl)-5-(trifluoromethoxy)benzamide (example I-167)

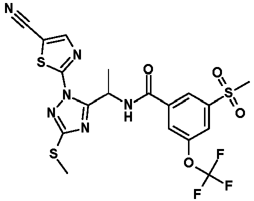

### Step 1: 2-hydrazino-1,3-thiazole-5-carbonitrile

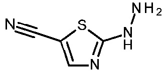

A mixture of 9.00 g (62.2 mmol) 2-chloro-1,3-thiazole-5-carbonitrile and 124.5 ml (124.5 mmol) of a 1 M solution of hydrazine in THF was refluxed for 2 h. After cooling to room temperature, the mixture was evaporated and then the residue was suspended in 50 ml of hot water. The resulting precipitate was filtered, washed with water and dried under vacuo to yield the title compound (9.00 g). Further drying by coevaporation with absolute toluene resulted in a decrease in mass and this material was used in the next step.
¹H NMR peak list (DMSO-d₆, 400 MHz): δ= 9.7694 (1.7); 7.8727 (13.8); 5.3331 (16.0); 3.3330 (6.9); 2.5083 (12.7); 2.5040 (16.5); 2.4997 (12.5)

### Step 2: O-methyl [(2S)-2-(1,3-dioxo-1,3-dihydro-2H-isoindol-2-yl)propanoyl]carbamothioate

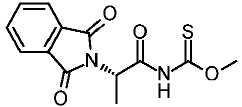

To a solution of 4.73 g (20.5 mmol) (2S)-2-(1,3-dioxo-1,3-dihydro-2H-isoindol-2-yl) propanoic acid in dichloromethane (30 ml) was added at 0 °C 3.58 ml (41.0 mmol) oxalyl chloride and 0.08 mL N,N-dimethylformamide. The reaction mixture was stirred for 1 h at 0 °C and overnight at room temperature. After this time the solvent and excess oxalyl chloride were evaporated under reduced pressure. The crude residue was dissolved in acetone (40 ml) and then 2.99 g (30.7 mmol) KSCN were added dropwise as a solution in acetone (40 ml). The addition funnel was rinsed with 10 ml acetone and the mixture was stirred at 60 °C for 2 h. After cooling to room temperature 2.1 ml (51 mmol) of methanol were added and the mixture was stirred at 55 °C overnight, cooled to room temperature and evaporated under reduced pressure. The resulting residue was suspended in a mixture of water and EtOAc. After separation of the layers the aqueous phase was extracted with EtOAc. The combined organic layers were washed with water and brine respectively and finally the organic layer was dried over anhydrous Na₂SO₄ and then concentrated under reduced pressure. The crude product was purified by silica gel chromatography to yield 3.69 g of the title compound.
ESI mass [m/z]: 293.1 [M+H]⁺

### Step 3: 2-{5-[1-(1,3-dioxo-1,3-dihydro-2H-isoindol-2-yl)ethyl]-3-(methylsulfanyl)-1H-1,2,4-triazol-1-yl}-1,3-thiazole-5-carbonitrile

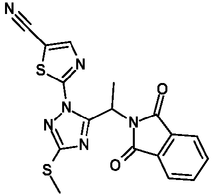

To a solution of 700 mg (2.39 mmol) O-methyl [(2S)-2-(1,3-dioxo-1,3-dihydro-2H-isoindol-2-yl)propanoyl]carbamothioate in ethanol (15 ml) were added 0.36 g (2.4 mmol) 2-hydrazino-1,3-thiazole-5-carbonitrile and the reaction mixture was stirred overnight at 75 °C, then for 6 h at 85 °C and again overnight at 75 °C. The mixture was cooled to room temperature, evaporated under reduced pressure and the resulting residue was purified by HPLC (H₂O / acetonitrile). This resulted in the isolation of 206 mg 2-{5-[(1S)-1-(1,3-dioxo-1,3-dihydro-2H-isoindol-2-yl)ethyl]-3-methoxy-1H-1,2,4-triazol-1-yl}-1,3-thiazole-5-carbonitrile and 77 mg 2-{5-[1-(1,3-dioxo-1,3-dihydro-2H-isoindol-2-yl)ethyl]-3-(methylsulfanyl)-1H-1,2,4-triazol-1-yl}-1,3-thiazole-5-carbonitrile. The enantiomeric excess of the obtained title compound was not determined.
ESI mass [m/z]: 397.1 [M+H]⁺
¹H-NMR (400 MHz, DMSO-d6): δ = 8.37 (s, 1H), 7.85 (s, 4H), 5.95 (q, *J =* 7.2 Hz, 1H), 2.62 (s, 3H), 1.80 (d, *J =* 7.2 Hz, 3H).

### Step 4: 2-[5-(1-aminoethyl)-3-(methylsulfanyl)-1H-1,2,4-triazol-1-yl]-1,3-thiazole-5-carbonitrile

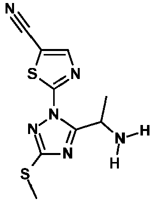

A solution of 75 mg (0.18 mmol) 2-{5-[1-(1,3-dioxo-1,3-dihydro-2H-isoindol-2-yl)ethyl]-3-(methylsulfanyl)-1H-1,2,4-triazol-1-yl}-1,3-thiazole-5-carbonitrile and 0.03 mL hydrazine hydrate in 2 mL ethanol was stirred for 90 min at room temperature. At this point all of the starting material had converted to a mixture of N-{1-[1-(5-cyano-1,3-thiazol-2-yl)-3-(methylsulfanyl)-1H-1,2,4-triazol-5-yl]ethyl}-2-(hydrazinocarbonyl)benzamide and the desired product as determined by HPLC/MS. 2 ml of 1 M hydrochloric acid were added and the resulting mixture was further stirred for 2 h at room temperature. The pH of the reaction mixture was then adjusted to pH 8 by the addition of a sat. aqueous solution of NaHCO₃. Water was added, and the mixture repeatedly extracted with ethyl acetate. The combined organic layers were washed with brine, dried using Na₂SO₄, filtered and the solvent was evaporated under reduced pressure to yield 37 mg of a residue containing 2-[5-(1-aminoethyl)-3-(methylsulfanyl)-1H-1,2,4-triazol-1-yl]-1,3-thiazole-5-carbonitrile which was used fort he next step without further purification.
ESI mass [m/z]: 267.1 [M+H]⁺

### Step 5: N-{ 1-[1-(5-cyano-1,3-thiazol-2-yl)-3-(methylsulfanyl)-1H-1,2,4-triazol-5-yl]ethyl}-3-(methylsulfonyl)-5-(trifluoromethoxy)benzamide

A solution of 41 mg (0.15 mmol) 3-(methylsulfonyl)-5-(trifluoromethoxy)benzoic acid, 100 mg (0.263 mmol) HATU, 0.08 mL (0.5 mmol) N-ethyldiisopropylamine in 2 mL DMF was stirred for 60 min at room temperature. 35 mg of the crude product obtained in step 4 were then added and the mixture stirred over night. The reaction mixture was then diluted with 1 mL acetonitrile and purified directly by HPLC (H₂O / acetonitrile) to provide 23 mg N-{1-[1-(5-cyano-1,3-thiazol-2-yl)-3-(methylsulfanyl)-1H-1,2,4-triazol-5-yl]ethyl}-3-(methylsulfonyl)-5-(trifluoromethoxy)benzamide.
ESI mass [m/z]: 533.1 [M+H]⁺

### Synthesis of 3-methylsulfonyl-5-(trifluromethoxy)benzoic acid

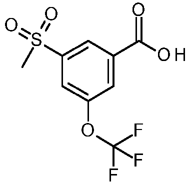

A mixture of 2.95 g (17.5 mmol) trans-N,N-dimethylcyclohexane-1-2-diamine and 11.4 g (35 mmol) cesium carbonate in 60 mL DMF was degassed for 30 min by purging with argon. 5 g (17.5 mmol) 3-bromo-5-(trifluoromethoxy)benzoic acid, 3.58 g (35 mmol) sodium methanesulfinate and 3.34 g (17.5 mmol) copper(I) iodide were added and the mixture further purged with argon for 5 min. The mixture was stirred at 120 °C over night, cooled to room temperature and then three times extracted with dichloromethane. The aqueous layer was acidified to pH 2 using concentrated hydrochloric acid and again extracted with dichloromethane. The dichlormethane phase was washed with brine several times. The layers were separated, and the combined organic layers were dried over anhydrous Na₂SO₄ and filtered. The solvent was removed under reduced pressure and the residue triturated with n-pentane, filtered-off and dried to provide 3.2 g of 3-methylsulfonyl-5-(trifluromethoxy)benzoic acid.
¹H NMR (DMSO-d₆, 400 MHz): δ= 14.00 (br s, 1H, COOH), 8.42 (s, 1H), 8.20 (s, 1H), 8.14 (s, 1H), 3.39 (s, 3H).
ESI mass [m/z]: 285.0 [M+H]⁺

### Synthesis of 3-(difluoromethylsulfonyl)-5-(trifluromethoxy)benzoic acid (INT-039)

### Step 1: Methyl 3-(trifluoromethoxy)-5-triisopropylsilylsulfanyl-benzoate

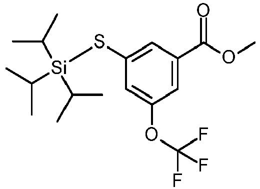

To a stirred solution of triisopropylsilanethiol (21.45 g, 112 mmol) in toluene (500 mL), under a stream of argon, NaH (5.03 g, 122 mmol, 60% disperse in oil) was added in portions. The mixture was stirred until no more gas evolved. Then methyl 3-bromo-5-(trifluoromethoxy)benzoate (CAS: 1306763-53-0) (30 g, 100 mmol), XantPhos (6.13 g, 11.2 mmol) and Pd₂(dba)₃ (4.85 g, 5.3 mmol) were added to the reaction mixture sequentially. The mixture was stirred at 100 °C overnight, cooled to r.t., diluted with EtOAc (500 mL) and filtered through a thin pad of silica gel. After evaporation, crude methyl 3-(trifluoromethoxy)-5-triisopropylsilylsulfanyl-benzoate (50 g, 50% purity by LC/MS, 64 mmol, 57% yield) was obtained and used in the next step without further purification.

### Step 2: Methyl 3-(difluoromethylsulfanyl)-5-(trifluoromethoxy)benzoate

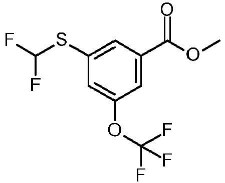

To a stirred solution of crude methyl 3-(trifluoromethoxy)-5-triisopropylsilylsulfanyl-benzoate (50 g, 50% purity by LC/MS, 64 mmol) in DMF (1000 mL) sodium 2-chloro-2,2-difluoroacetate (29.27 g, 192 mmol) and cesium carbonate (62.55 g, 192 mmol) were added under a stream of argon. The mixture was stirred at 100 °C overnight, cooled to r.t., and evaporated under reduced pressure. The residue was dissolved in water (1000 mL) and extracted with EtOAc (5 × 250 mL). Methyl 3-(difluoromethylsulfanyl)-5-(trifluoromethoxy)benzoate (10.5 g, 34.7 mmol, 54.3% yield) was obtained after column chromatography.

### Step 3: Methyl 3-(difluoromethylsulfonyl)-5-(trifluoromethoxy)benzoate

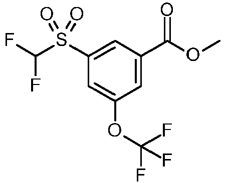

To a solution of methyl 3-(difluoromethylsulfanyl)-5-(trifluoromethoxy)benzoate (10.5 g, 34.7 mmol) in dichloromethane (200 mL) mCPBA (16.35 g, 93.9 mmol, 75% purity) was added in portions at 0 °C under a stream of argon. The mixture was stirred overnight at room temperature and evaporated under reduced pressure. Methyl 3-(difluoromethylsulfonyl)-5-(trifluoromethoxy)benzoate (6.8 g, 20.34 mmol, 58.63%) was obtained after column chromatography on silica gel.

### Step 4: 3-(Difluoromethylsulfonyl)-5-(trifluromethoxy)benzoic acid (INT-039)

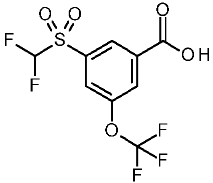

To a stirred solution of methyl 3-(difluoromethylsulfonyl)-5-(trifluoromethoxy)benzoate (6.8 g, 20.34 mmol) in THF (80 mL) / water (20 mL) mixture at 0 °C LiOH monohydrate (1.146 g, 27.459 mmol) was added and the mixture was stirred overnight at r.t. THF was evaporated under reduced pressure, the water phase was acidified to pH=3 and extracted with MTBE (5 × 10 mL). Pure 3-(difluoromethylsulfonyl)-5-(trifluromethoxy)benzoic acid (3 g, 9.37 mmol, 34.12% yield) was obtained after recrystallization from 30% aqueous EtOH as white solid.
¹H NMR (DMSO-d₆, 400 MHz): δ= 7.47 (t, 1H), 8.21 (s, 1H), 8.32 (s, 1H), 8.40 (s, 1H), 13.79 (s, 1H).
ESI mass [m/z]: 319.0 [M+H]⁺

### Synthesis of 6-[5-{(1S)-1-[3,5-bis(trifluoromethyl)benzamido]ethyl}-3-(dimethylamino)-1H-1,2,4-triazol-1-yl]-N-ethyl-N-methylnicotinamide (example I-232, reference compound)

### Step 1: Methyl 6-[5-{(1S)-1-[(tert-butoxycarbonyl)amino]ethyl}-3-(dimethylamino)-1H-1,2,4-triazol-1-yl]nicotinate

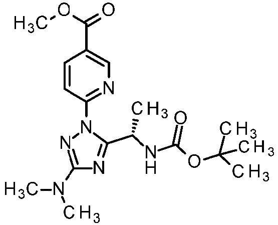

A solution of 2 g (10.57 mmol) N-(tert-butoxycarbonyl)-L-alanine in 25 ml dioxane under argon was treated with 4.42 g (11.63 mmol) bis(dimethylamino)(3-oxido-1H-[1,2,3]triazolo[4,5-b]pyridin-1-yl)methylium hexafluorophosphate (HATU) and 8.28 ml (47.50 mmol) N,N-diisopropylethylamine. The reaction mixture was stirred 10 minutes at room temperature and then 3.90 g (15.86 mmol) methyl N,N-dimethylcarbamimidothioate hydroiodide (1:1) was added and the mixture was stirred further 1 h at room temperature. Finally 2.65 g (15.86 mmol) methyl 6-hydrazinonicotinate, 25 ml dioxane and 6 ml acetic acid were added into the mixture and the stirring was kept overnight at room temperature. The mixture was diluted with ethyl acetate and washed with a saturated NaHCO₃ solution and brine, dried over sodium sulfate and then the solvent was reduced under pressure. The residue was purified by reversed phase chromatography (H₂O/acetonitrile) to provide 3.51 g (purity: 99%; yield: 84 %) of the title compound. ESI mass [m/z]: 391.2 [M+H]⁺
¹H-NMR(400.2 MHz, d6-DMSO): δ= 8.9645 (1.0); 8.9602 (0.9); 8.4646 (1.0); 8.4588 (0.9); 8.4429 (1.0); 8.4371 (1.0); 7.8941 (1.3); 7.8928 (1.4); 7.8725 (1.2); 7.8711 (1.3); 7.4274 (0.4); 7.4074 (0.4); 5.6538 (0.4); 5.6360 (0.6); 5.6179 (0.4); 3.9006 (8.0); 3.3283 (35.3); 2.9711 (16.0); 2.5253 (0.6); 2.5118 (12.2); 2.5075 (23.9); 2.5030 (31.2); 2.4984 (23.3); 2.4941 (11.6); 1.4158 (2.0); 1.3985 (1.9); 1.3342 (7.2); 1.0612 (0.4); 0.0080 (0.8); -0.0002 (25.1); -0.0084 (1.0)

### Step 2: Methyl 6-{5-[(1S)-1-aminoethyl]-3-(dimethylamino)-1H-1,2,4-triazol-1-yl }nicotinate hydrochloride (1:1) (INT-037)

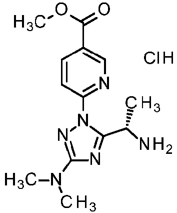

A solution of 2.52 g (6.45 mmol) methyl 6-[5-{(1S)-1-[(tert-butoxycarbonyl)amino]ethyl}-3-(dimethylamino)-1H-1,2,4-triazol-1-yl]nicotinate in 26 ml dioxane was treated with 4N HCl-dioxane solution (30.17 ml) and the mixture was stirred 18 h at room temperature. Then, the solvent was evaporated under reduced pressure to afford 8.58 g (purity: 100%) of the title compound which was used in the next step without further purification.
ESI mass [m/z]: 291.5 [M+H - HCl]⁺
¹H-NMR(400.2 MHz, d₆-DMSO): δ= 8.9997 (1.4); 8.9987 (1.4); 8.9942 (1.4); 8.6467 (1.4); 8.5193 (1.0); 8.5136 (1.0); 8.4977 (1.1); 8.4919 (1.0); 7.9443 (1.4); 7.9227 (1.3); 5.3583 (0.9); 5.3413 (0.9); 3.9130 (8.3); 3.5687 (3.0); 3.3328 (17.5); 3.0343 (16.0); 2.6727 (0.4); 2.5262 (0.6); 2.5127 (10.1); 2.5084 (19.5); 2.5038 (25.0); 2.4993 (18.3); 2.4950 (9.0); 1.6214 (3.3); 1.6045 (3.2); 0.0078 (0.6); -0.0002 (16.9); - 0.0086 (0.6)

### Step 3: Methyl 6-[5-{(1S)-1-[3,5-bis(trifluoromethyl)benzamido]ethyl}-3-(dimethylamino)-1H-1,2,4-triazol-1-yl]nicotinate

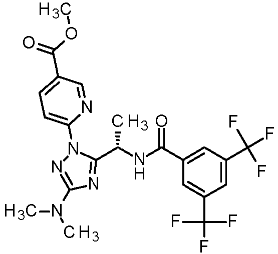

To a solution of 2.13 g (6.50 mmol) methyl 6-{5-[(1S)-1-aminoethyl]-3-(dimethylamino)-1H-1,2,4-triazol-1-yl}nicotinate hydrochloride (1:1) in 90 ml dichloromethane were added 3.0 ml (17.36 mmol) N,N.diisopropylethylamine and then a solution of 1.2 g (4.33 mmol) 3,5-bis(trifluoromethyl)benzoyl chloride in 90 ml dichloromethane and then the reaction mixture was stirred at room temperature overnight. The mixture was diluted with dichloromethane and washed with a 5% NaH₂PO₄ aqueous solution, a bicarbonate solution and brine. The organic phase was dried over sodium sulfate, and the solvent was reduced under pressure. The residue was crystallized in acetonitrile and the filtrate was reduced under pressure and purified by reversed phase chromatography (H₂O/acetonitrile) to provide 641 mg (purity: 98%; yield: 27 %) of the title compound.
ESI mass [m/z]: 531.4 [M+H]⁺
¹H-NMR(400.2 MHz, d6-DMSO): δ= 9.5405 (0.7); 9.5224 (0.8); 8.9423 (1.3); 8.9380 (1.3); 8.4684 (1.1); 8.4625 (1.4); 8.4548 (2.7); 8.4470 (1.3); 8.4411 (1.1); 8.3155 (0.9); 8.3093 (1.2); 7.9082 (1.3); 7.8879 (1.2); 7.8866 (1.2); 6.1599 (0.6); 6.1423 (0.9); 6.1247 (0.6); 3.8906 (8.3); 3.3249 (112.0); 2.9656 (16.0); 2.6800 (0.4); 2.6757 (0.9); 2.6712 (1.2); 2.6667 (0.9); 2.5247 (4.5); 2.5199 (7.0); 2.5112 (73.5); 2.5068 (144.4); 2.5023 (187.9); 2.4977 (137.7); 2.4933 (67.9); 2.3337 (0.9); 2.3291 (1.2); 2.3245 (0.8); 1.6462 (3.0); 1.6288 (3.0); 0.1460 (0.6); 0.0080 (5.0); -0.0002 (133.0); -0.0085 (4.8); -0.1496 (0.6)

### Step 4: 6-[5-{(1S)-1-[3,5-Bis(trifluoromethyl)benzamido]ethyl}-3-(dimethylamino)-1H-1,2,4-triazol-1-yl]nicotinic acid

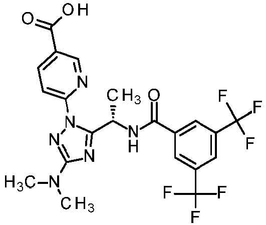

A solution of 508 mg ( 0.95 mmol) methyl 6-[5-{(1S)-1-[3,5-bis(trifluoromethyl)benzamido]ethyl}-3-(dimethylamino)-1H-1,2,4-triazol-1-yl]nicotinate in a mixture of 10.5 ml THF and 1 ml water was treated with 80 mg (1.91 mmol) lithium hydroxide monohydrate. The reaction mixture was stirred at room temperature overnight. The mixture was acidified with a 10% HCl aq. solution and was extracted with ethyl acetate. The combined organic layer were washed with brine, dried over sodium sulfate and the solvent was removed under reduced pressure to provide 457 mg (purity: 100%; yield: 93.0 %) of the title compound.
ESI mass [m/z]: 517.0 [M+H]⁺
¹H-NMR(400.2 MHz, d6-DMSO): δ= 9.5431 (0.7); 9.5253 (0.7); 8.9210 (1.2); 8.9194 (1.4); 8.9154 (1.3); 8.9136 (1.3); 8.4560 (2.5); 8.4439 (1.1); 8.4382 (1.0); 8.4224 (1.1); 8.4167 (1.1); 8.3071 (1.1); 7.8906 (1.3); 7.8891 (1.3); 7.8692 (1.2); 7.8676 (1.3); 6.1645 (0.5); 6.1470 (0.9); 6.1294 (0.5); 4.0384 (0.6); 4.0206 (0.6); 3.3305 (36.8); 2.9634 (16.0); 2.6723 (0.4); 2.6679 (0.3); 2.5259 (1.5); 2.5212 (2.3); 2.5125 (25.4); 2.5080 (50.6); 2.5034 (66.0); 2.4988 (47.4); 2.4942 (22.5); 2.3303 (0.4); 1.9895 (2.5); 1.9097 (0.8); 1.6479 (2.9); 1.6305 (2.9); 1.1935 (0.7); 1.1758 (1.4); 1.1580 (0.7); 0.0080 (1.9); 0.0059 (1.0); -0.0002 (51.5); -0.0085 (1.4)

### Step 5: 6-[5-{(1S)-1-[3,5-Bis(trifluoromethyl)benzamido]ethyl}-3-(dimethylamino)-1H-1,2,4-triazol-1-yl]-N-ethyl-N-methylnicotinamide

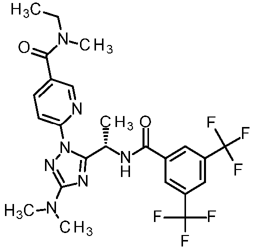

A solution of 72 mg (0.13 mmol) 6-[5-{(1S)-1-[3,5-bis(trifluoromethyl)benzamido]ethyl}-3-(dimethylamino)-1H-1,2,4-triazol-1-yl]nicotinic acid in 2 ml dichloromethane was treated with 64 mg (0.17 mmol) bis(dimethylamino)(3-oxido-1H-[1,2,3]triazolo[4,5-b]pyridin-1-yl)methylium hexafluorophosphate (HATU) and 0.034 ml (0.19 mmol) N,N-diisopropylethylamine and then the mixture was stirred 10 minutes at room temperature. Finally, 0.018 ml (0.21 mmol) N-methylethylamine was added to the reaction and the mixture was stirred overnight at room temperature. The mixture was treated with a 5% aqueous NaH₂PO₄ solution and extracted several times with dichloromethane. The combined organic layers were washed with sodium bicarbonate and brine, dried and finally the solvent was reduced under pressure. The residue was purified by reversed phase chromatography (H₂O/acetonitrile) to provide 56 mg (purity: 99%; yield: 72 %) of the title compound.

### Synthesis of N-{(1S)-1-[1-{5-[(cyclopropylcarbonyl)(methyl)amino]pyridin-2-yl}-3-(dimethylamino)-1H-1,2,4-triazol-5-yl]ethyl}-3,5-bis(trifluoromethyl)benzamide

### Example I-269, reference compound)

### Step 1: N-(6-Chloropyridin-3-yl)-N-methylcyclopropanecarboxamide

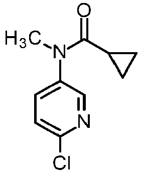

To a solution of 3 g (21.0 mmol) 6-chloro-N-methylpyridin-3-amine in 55 ml dichloromethane and 11.0 ml (63.2 mmol) N,N.diisopropylethylamine was added a solution of 2.10 ml (23.1 mmol) cyclopropanecarbonyl chloride in 5 ml dichloromethane and the reaction mixture was stirred at room temperature for 1 h. The mixture was washed with an 5% NaH₂PO₄ aqueous solution, brine and then the organic phase was dried and evaporated under pressure. The residue was purified by flash chromatography using a mixture of cyclohexan/ethyl acetate as a eluent to provide 3.94 g (purity: 97%; yield: 86 %) of the title compound.
ESI mass [m/z]: 211.1 [M+H]⁺

### Step 2: tert-Butyl 2-{5-[(cyclopropylcarbonyl)(methyl)amino]pyridin-2-yl}hydrazinecarboxy-late

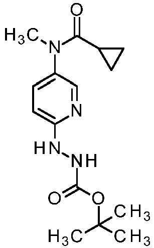

To a solution of 3.94 g (18.7 mmol) N-(6-chloropyridin-3-yl)-N-methylcyclopropanecarboxamide in 101 ml toluene under argon were added 4.95 g (37.4 mmol) tert-butyl hydrazinecarboxylate, 685 mg (0.75 mmol) Pd₂(dba)₃ , 1.24 g (2.24 mmol) dppf and 12.2 mg (37.4 mmol) cesium carbonate and the reaction mixture was stirred at 100 °C overnight. The mixture was diluted with dichloromethane, filtered and evaporated under pressure. The residue was purified by reversed phase chromatography (H₂O/acetonitrile) to provide 2.96 g (purity: 96%, yield: 50 %) of the title compound.
ESI mass [m/z]: 307.1 [M+H]⁺

### Step 3: N-(6-Hydrazinopyridin-3-yl)-N-methylcyclopropanecarboxamide

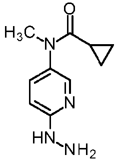

A solution of 3.1 g (10.1 mmol) tert-butyl 2-{5-[(cyclopropylcarbonyl)(methyl)amino]pyridin-2-yl}hydrazinecarboxylate in 71 ml dioxane was treated with 4N HCl-dioxane solution (25.2 ml) and the mixture was stirred 18 h at room temperature. Then, the solvent was evaporated under reduced pressure to afford 3.31 g (purity: 53%, yield:85 %) of the title compound which was used in the next step without further purification.
ESI mass [m/z]: 207.1 [M+H - HCl]⁺

### Step 4: tert-Butyl {(1S)-1-[1-{5-[(cyclopropylcarbonyl)(methyl)amino]pyridin-2-yl}-3-(dimethylamino)-1H-1,2,4-triazol-5-yl]ethyl}carbamate

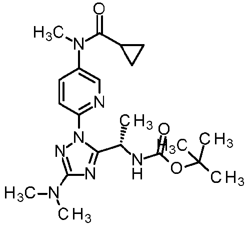

A solution of 1.5 g (7.93mmol) N-(tert-butoxycarbonyl)-L-alanine in 15 ml dioxane was treated with 3.32 g (8.72 mmol) bis(dimethylamino)(3-oxido-1H-[1,2,3]triazolo[4,5-b]pyridin-1-yl)methylium hexafluorophosphate (HATU) and 6.21 ml (35.66 mmol) N,N-diisopropylethylamine . The reaction mixture was stirred 10 minutes at room temperature and then 2.93 g (11.89 mmol) methyl N,N-dimethylcarbamimidothioate hydroiodide (1:1) was added and the mixture was stirred 1 h at room temperature. Finally 2.45 g (11.89 mmol) N-(6-hydrazinopyridin-3-yl)-N-methylcyclopropanecarboxamide, 15 ml dioxane and 4.6 ml acetic acid were added into the mixture and the stirring was kept further at room temperature overnight. The mixture was diluted with ethyl acetate and washed with a saturated NaHCO₃ solution, brine, dried over sodium sulfate and then the solvent was reduced under pressure. The residue was purified by reversed phase chromatography (H₂O/acetonitrile) to provide 641 mg (purity: 96%, yield: 18 %) of the title compound.
ESI mass [m/z]: 391.2 [M+H]⁺
¹H-NMR(400.2 MHz, d₆-DMSO): δ= 8.5201 (0.8); 8.0420 (0.5); 8.0359 (0.5); 8.0202 (0.6); 8.0146 (0.5); 7.8328 (1.0); 7.8110 (0.8); 7.3962 (0.4); 7.3774 (0.4); 5.5726 (0.4); 5.5546 (0.5); 4.0561 (0.4); 4.0383 (1.1); 4.0205 (1.1); 4.0028 (0.4); 3.3296 (20.7); 3.2607 (1.1); 2.9526 (16.0); 2.5887 (0.4); 2.5116 (12.2); 2.5076 (21.8); 2.5032 (26.9); 2.4987 (19.6); 1.9894 (4.8); 1.4265 (2.4); 1.4093 (2.3); 1.3257 (8.5); 1.1933 (1.3); 1.1756 (2.6); 1.1578 (1.3); 1.0554 (0.5); 0.8429 (1.0); 0.8342 (1.6); 0.8233 (1.1); 0.6919 (0.7); 0.0077 (0.5); -0.0002 (8.3)

### Step 5: N-(6-{5-[(1S)-1-Aminoethyl]-3-(dimethylamino)-1H-1,2,4-triazol-1-yl}pyridin-3-yl)-N-methylcyclopropanecarboxamide hydrochloride (1:1)

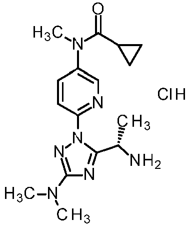

A solution of 611 mg (1.42 mmol) tert-butyl {(1S)-1-[1-{5-[(cyclopropylcarbonyl) (methyl)amino]pyridin-2-yl}-3-(dimethylamino)-1H-1,2,4-triazol-5-yl]ethyl}carbamate in 5.6 ml dioxane was treated with 4N HCl-dioxane solution (6.65 ml) and the mixture was stirred 18 h at room temperature. Then, the solvent was evaporated under reduced pressure to afford 573 mg (purity: 96%, yield: 100%) of the title compound which was used in the next step without further purification.
ESI mass [m/z]: 330.4[M+H - HCl]⁺
¹H-NMR(400.2 MHz, d6-DMSO): δ= 8.5478 (1.7); 8.5421 (1.7); 8.1004 (0.4); 8.0941 (0.3); 8.0789 (0.4); 8.0728 (0.4); 7.8919 (0.9); 7.8700 (0.7); 5.3057 (0.3); 3.5681 (10.6); 3.3463 (62.6); 3.3089 (0.4); 3.2803 (0.8); 3.0193 (16.0); 2.9701 (0.8); 2.6761 (0.4); 2.6715 (0.6); 2.6669 (0.4); 2.6598 (0.5); 2.5250 (2.1); 2.5202 (3.2); 2.5116 (38.2); 2.5071 (75.5); 2.5025 (98.0); 2.4979 (70.6); 2.4934 (33.8); 2.3341 (0.4); 2.3294 (0.6); 2.3249 (0.4); 1.6129 (3.1); 1.5961 (2.9); 0.8650 (0.4); 0.8549 (0.9); 0.8474 (1.2); 0.8363 (1.0); 0.8288 (0.4); 0.7020 (0.5); 0.0079 (1.0); -0.0002 (31.1); -0.0086 (1.0)

### Step 6:

### N-{(1S)-1-[1-{5-[(Cyclopropylcarbonyl)(methyl)amino]pyridin-2-yl}-3-(dimethylamino)-1H-1,2,4-triazol-5-yl]ethyl}-3,5-bis(trifluoromethyl)benzamide

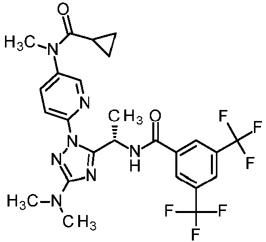

To a solution of 137 mg (0.37 mmol) N-(6-{5-[(1S)-1-aminoethyl]-3-(dimethylamino)-1H-1,2,4-triazol-1-yl}pyridin-3-yl)-N-methylcyclopropanecarboxamide hydrochloride (1:1) in 2 ml dichloromethane were added 0.17 ml (0.99 mmol) N,N.diisopropylethylamine and then a solution of 69 mg (0.25 mmol) 3,5-bis(trifluoromethyl)benzoyl chloride in 2 ml dichloromethane and the reaction mixture was stirred at room temperature overnight. The mixture was diluted with dichloromethane and was washed with a 5% NaH₂PO₄ aqueous solution, bicarbonate solution and then brine. The organic phase was dried over sodium sulfate and the solvent was reduced under pressure. The residue was purified by reversed phase chromatography (H₂O/acetonitrile) to provide 116 mg (purity: 97%; yield: 79 %) of the title compound.

### Intermediates: BOC-protected amines

Table A shows enantiomeric enriched intermediate compounds (Boc-INT) of general formula (a) according to the invention described in table A, wherin R¹ is *tert*-butyloxycarbonyl (Boc), R^{3b} is methyl, R^{3a} is hydrogen and Y is a direct bond and wherein R⁴ and R⁵ have the meanings given in Configuration (4-1) or the meanings given in Configuration (5-1), which are obtained according to or analogously to the preparation examples described above.

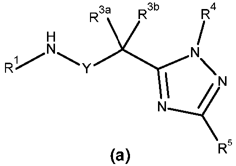

**Table A**

| **Example** | **R4** | **R⁵** | ***ee*-Value** | **Ret. Time** | **ESI Mass (m/z)¹⁾** |
|---|---|---|---|---|---|
| Boc-INT-024 | 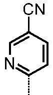 | 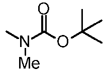 | 100% | 11.28 min | 444.2 [M+H]⁺ |
| Boc-INT-027 | 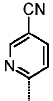 | 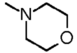 | 96.9% | 11.20 min | 400.1 [M+H]⁺ |
| Boc-INT-028 | 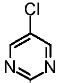 | 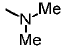 | 100% | 10.74 min | 368.1 [M+H]⁺ |
| Boc-INT-029 | 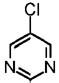 | 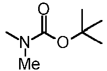 | 100% | 10.51 min | 454.1 [M+H]⁺ |
| Boc-INT-030 | 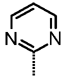 | 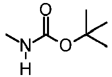 | 100% | 9.26 min | 406.2 [M+H]⁺ |
| Boc-INT-031 | 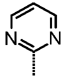 | 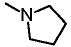 | 75.5% | 11.17 min | 360.2 [M+H]⁺ |
| Boc-INT-032 | 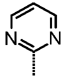 | 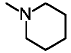 | 100% | 10.30 min | 374.1 [M+H]⁺ |
| Boc-INT-033 | 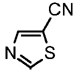 | 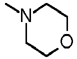 | 100% | 12.09 min | 406.1 [M+H]⁺ |
| Boc-INT-034 | 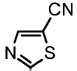 | 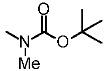 | 100% | 15.35 min | 450.2 [M+H]⁺ |
| Boc-INT-035 | 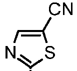 | 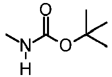 | 100% | 15.24 min | 436.4 [M+H]⁺ |
| Boc-INT-036 | 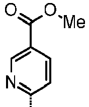 | 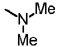 | 100% | 11.35 min | 391.2 [M+H]⁺ |
| Boc-INT-038 | 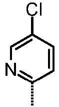 | 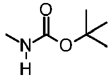 | 100% | 12.25 min | 439.2 [M+H]⁺ |

| | | | | | |
|---|---|---|---|---|---|
| ¹) The stated mass corresponds to the peak from the isotope pattern of the [M+H]⁺ ion with the highest intensity. | | | | | |

### Analytical data of the compounds

The determination of [M+H]⁺ or [M-H]⁻ by LC-MS under acidic chromatographic conditions was done with 1 ml formic acid per liter acetonitrile and 0.9 ml formic acid per liter Millipore water as eluents. The column Zorbax Eclipse Plus C18 50 mm * 2.1 mm was used. The temperature of the column oven was 55 °C.

The enantomeric excesses of the intermediate compounds *tert-*butyl *N*-[5-[(1*S*)-1-(tert-butoxycarbonylamino)ethyl]-1-[5-(morpholine-4-carbonyl)thiazol-2-yl]-1,2,4-triazol-3-yl]-*N*-methylcarbamate, *tert-*butyl *N*-[5-[(15)-1-(tert-butoxycarbonylamino)ethyl]-1-pyrimidin-2-yl-1,2,4-triazol-3-yl]-N-methyl-carbamate, *tert-*Butyl *N*-[(1*S*)-1-[5-(dimethylamino)-2-pyrimidin-2-yl-1,2,4-triazol-3-yl]ethyl]carbamate, *tert*-butyl *N*-[(1*S*)-1-[5-(tert-butoxycarbonylamino)-2-(5-cyano-2-pyridyl)-1,2,4-triazol-3-yl]ethyl]carbamate, *tert*-butyl *N*-[(1*S*)-1-[5-[methoxy(methyl) amino]-2-pyrimidin-2-yl-1,2,4-triazol-3-yl]ethyl] carbamate, the intermediate compounds in table A and examples I-031, I-127, I-134 were determined using chiral HPLC: Chiralcel OD-RH column (4.6 mm x 150 mm x 5 µm), room temperature, eluting with 0.1% phosphoric acid (A) and acetonitrile (B), gradient A:B 95/5 to 10/90, detecting at 210 nm. Analogously, the enantiomeric excess were determined for the examples given in Table 1: I-132 (ee-value: 92%; Rₜ = 10.17 min); I-137 (ee-value: 100%; Rₜ = 7.70 min); I-145 (ee-value: 100%; Rₜ = 9.86 min); I-169 (ee-value: 96%; Rₜ = 8.90 min); I-175 (ee-value: 81%; Rₜ = 14.30 min); I-176 (ee-value: 78%; Rₜ = 12.87 min); I-202 (ee-value: 100%; Rₜ = 8.35 min); I-206 (ee-value: 97%; Rₜ = 12.90 min); I-222 (ee-value: 100%; Rt = 12.01 min).

The determination of the ¹H NMR data was effected with a Bruker Avance III 400 MHz equipped with a 1.7 mm TCI cryo probe, a Bruker Avance III 600 MHz equipped with a 5 mm multi-nuclear cryo probe or a Bruker Avance NEO 600 MHz equipped with a 5 mm TCI cryo probe with tetramethylsilane as reference (0.0) and the solvents CD₃CN, CDCl₃ or D₆-DMSO.

The NMR data of selected examples are listed either in conventional form (δ values, multiplet splitting, number of hydrogen atoms) or as NMR peak lists.

### NMR peak list method

The ¹H NMR data of selected examples are stated in the form of ¹H NMR peak lists. For each signal peak, first the δ value in ppm and then the signal intensity in round brackets are listed. The pairs of δ value-signal intensity numbers for different signal peaks are listed with separation from one another by semicolons.

The peak list for one example therefore takes the form of:
δ₁ (intensity₁); δ₂ (intensity₂);........; δᵢ (intensityᵢ);......; δₙ (intensityₙ)

The intensity of sharp signals correlates with the height of the signals in a printed example of an NMR spectrum in cm and shows the true ratios of the signal intensities. In the case of broad signals, several peaks or the middle of the signal and the relative intensity thereof may be shown in comparison to the most intense signal in the spectrum.

For calibration of the chemical shift of ¹H NMR spectra, we use tetramethylsilane and/or the chemical shift of the solvent, particularly in the case of spectra which are measured in DMSO. Therefore, the tetramethylsilane peak may but need not occur in NMR peak lists.

The lists of the ¹H NMR peaks are similar to the conventional ¹H NMR printouts and thus usually contain all peaks listed in a conventional NMR interpretation.

In addition, like conventional ¹H NMR printouts, they may show solvent signals, signals of stereoisomers of the target compounds which are likewise provided by the invention, and/or peaks of impurities.

In the reporting of compound signals within the delta range of solvents and/or water, our lists of ¹H NMR peaks show the standard solvent peaks, for example peaks of DMSO in DMSO-D₆ and the peak of water, which usually have a high intensity on average.

The peaks of stereoisomers of the target compounds and/or peaks of impurities usually have a lower intensity on average than the peaks of the target compounds (for example with a purity of > 90%).

Such stereoisomers and/or impurities may be typical of the particular preparation process. Their peaks can thus help in identifying reproduction of our preparation process with reference to "by-product fingerprints".

A person skilled in the art calculating the peaks of the target compounds by known methods (MestreC, ACD simulation, but also with empirically evaluated expected values) can, if required, isolate the peaks of the target compounds, optionally using additional intensity filters. This isolation would be similar to the peak picking in question in conventional ¹H NMR interpretation.

Further details of ¹H NMR peak lists can be found in the Research Disclosure Database Number 564025.

The compounds according to the invention described in table 1 below are likewise preferred compounds of the formula (I), wherin R¹ is hydrogen, R^{3b} is methyl, R^{3a} is hydrogen, X is oxygen and Y is a direct bond which are obtained according to or analogously to the preparation examples described above.

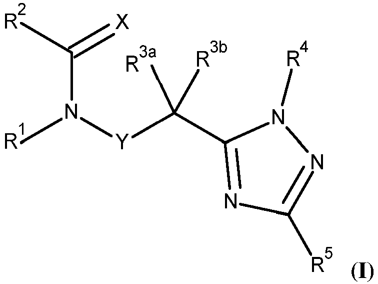

**Table 1**

| **Example** | **Structure³⁾** | **NMR Peaklist¹⁾** | **ESI mass [m/z]²⁾** |
|---|---|---|---|
| I-001 | 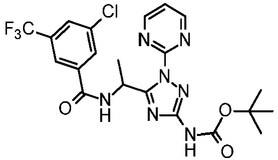 (reference compound) | ¹H-NMR (400.2 MHz, d₆-DMSO, ppm): δ = 9.9040 (1.2); 9.4253 (0.5); 9.4078 (0.5); 8.9559 (2.6); 8.9437 (2.7); 8.1409 (0.9); 8.0909 (0.9); 8.0608 (0.8); 7.5881 (0.7); 7.5760 (1.3); 7.5639 (0.7); 5.9945 (0.4); 5.9772 (0.6); 5.9598 (0.4); 3.3216 (13.8); 2.5246 (0.8); 2.5111 (14.8); 2.5068 (30.1); 2.5023 (39.8); 2.4977 (28.9); 2.4933 (14.0); 1.6393 (2.0); 1.6219 (2.1); 1.4648 (0.9); 1.4280 (16.0); 1.3978 (0.6); 0.0080 (1.0); -0.0002 (30.1); -0.0085 (1.1). | 512.2 [M+H]⁺ |
| I-002 | 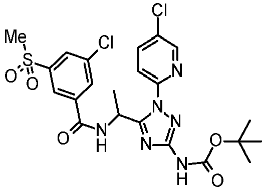 | ¹H-NMR(400.2 MHz, d₆-DMSO): | 499.1 [M-*iso*Buten]⁺ |
| | | δ= 9.9597 (1.3); 9.4565 (0.6); 9.4394 (0.6); 8.6066 (1.0); 8.6053 (1.1); 8.6003 (1.1); 8.5989 (1.0); 8.2971 (0.8); 8.2933 (1.4); 8.2895 (0.9); 8.1982 (0.9); 8.1944 (1.0); 8.1912 (1.5); 8.1859 (0.9); 8.1764 (0.8); 8.1699 (0.8); 8.1471 (1.0); 8.1428 (1.2); 8.1383 (0.7); 7.8087 (1.1); 7.8074 (1.0); 7.7867 (1.0); 7.7854 (1.0); 5.9913 (0.4); 5.9742 (0.6); 5.9569 (0.4); 5.7562 (5.8); 3.3328 (75.4); 3.3203 (7.0); 3.1753 (1.6); 3.1621 (1.6); 2.6717 (0.4); 2.5251 (1.2); 2.5116 (23.5); 2.5072 (45.3); 2.5027 (57.1); 2.4982 (40.7); 2.4937 (19.6); 2.3295 (0.3); 1.6306 (2.0); 1.6131 (2.0); 1.4326 (16.0); -0.0002 (0.6) | |
| I-003 | 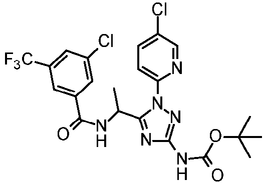 (reference compound) | ¹H-NMR(400.2 MHz, d₆-DMSO): | 545.3 [M+H]⁺ |
| | | δ= 9.9516 (1.2); 9.4034 (0.5); 9.3864 (0.6); 8.5937 (1.0); 8.5883 (1.0); 8.5873 (1.0); 8.1906 (0.7); 8.1842 (0.7); 8.1687 (0.8); 8.1622 (0.8); 8.1531 (1.0); 8.0996 (0.9); 8.0699 (0.9); 7.8018 (1.0); 7.8008 (1.0); 7.7800 (0.9); 7.7788 (1.0); 5.9753 (0.4); 5.9581 (0.6); 5.9408 (0.4); 5.7569 (1.4); 4.1056 (0.4); 4.0925 (0.4); 3.3307 (36.2); 3.1758 (2.5); 3.1627 (2.4); 2.5255 (0.8); 2.5207 (1.3); 2.5121 (15.2); 2.5076 (30.4); 2.5031 (39.2); 2.4985 (28.0); 2.4940 (13.4); 1.6262 (2.0); 1.6088 (2.0); 1.4311 (16.0); -0.0002 (0.4) | |
| I-004 | 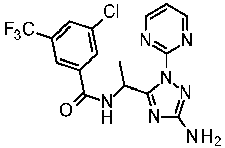 (reference compound) | ¹H-NMR(400 MHz, d₆-DMSO, ppm): δ = 9.3548 (1.1); 9.3368 (1.0); 8.8619 (4.0); 8.8498 (4.1); 8.3485 (0.4); 8.3365 (0.4); 8.1819 (1.9); 8.1365 (2.0); 8.0652 (1.8); 7.4487 (1.1); 7.4366 (2.0); 7.4245 (1.0); 6.0247 (0.7); 6.0071 (1.2); 5.9896 (0.8); 4.6986 (0.3); 4.6863 (0.3); 4.6771 (0.3); 4.6316 (0.4); 4.5829 (0.4); 4.5729 (0.4); 4.5594 (0.4); 4.5410 (0.4); 4.5155 (0.4); 4.4724 (0.4); 4.4552 (0.4); 4.4369 (0.4); 4.4244 (0.4); 4.4060 (0.4); 4.3866 (0.3); 3.6474 (0.5); 3.5686 (16.0); 2.8920 (1.8); 2.7326 (1.5); 2.7316 (1.4); 2.6774 (0.4); 2.6728 (0.6); 2.6683 (0.4); 2.5431 (0.4); 2.5263 (1.9); 2.5128 (36.1); 2.5084 (70.8); 2.5039 (91.3); 2.4993 (67.1); 2.4950 (33.4); 2.3352 (0.4); 2.3307 (0.6); 2.3262 (0.4); 1.5960 (4.2); 1.5787 (4.2); 1.5093 (0.4); 1.4919 (0.4); 0.0080 (2.0); -0.0002 (56.4); -0.0085 (2.3) | 412.1 [M-HCl]⁺ |
| I-005 | 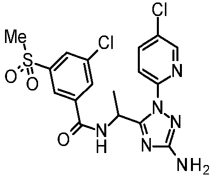 | ¹H-NMR(600.4 MHz, d₆-DMSO): | 455.1 [M+H]⁺ |
| | | δ= 9.3491 (4.0); 9.3373 (4.2); 8.5220 (6.8); 8.5211 (6.7); 8.5176 (7.0); 8.5167 (6.4); 8.3231 (5.7); 8.3206 (10.2); 8.3180 (5.7); 8.2195 (5.6); 8.2166 (8.0); 8.2137 (5.5); 8.1387 (5.8); 8.1358 (8.6); 8.1328 (5.0); 8.1024 (5.5); 8.0980 (5.2); 8.0878 (5.8); 8.0834 (5.7); 7.7024 (7.4); 7.7015 (7.0); 7.6878 (7.1); 7.6868 (6.7); 6.0264 (0.6); 6.0149 (3.0); 6.0032 (4.8); 5.9915 (3.1); 5.9799 (0.6); 5.7800 (12.2); 5.7521 (1.7); 3.3232 (46.4); 3.3058 (281.5); 3.2824 (0.4); 3.1733 (0.3); 2.6184 (0.9); 2.6154 (1.8); 2.6124 (2.5); 2.6093 (1.8); 2.6063 (0.8); 2.5214 (6.7); 2.5183 (8.7); 2.5151 (10.2); 2.5065 (145.2); 2.5034 (291.9); 2.5003 (393.4); 2.4973 (283.2); 2.4943 (131.6); 2.3904 (0.8); 2.3874 (1.8); 2.3843 (2.4); 2.3812 (1.7); 2.3782 (0.8); 1.5848 (16.0); 1.5733 (16.0); 0.0053 (1.7); -0.0001 (44.0); -0.0057 (1.3) | |
| I-006 | 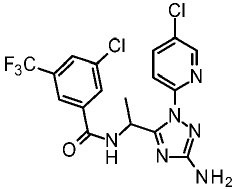 (reference compound) | ¹H-NMR(400.2 MHz, d₆-DMSO): | 445.1 [M+H]⁺ |
| | | δ= 9.3376 (2.9); 9.3201 (3.0); 8.5158 (5.5); 8.5097 (5.6); 8.1910 (5.4); 8.1414 (5.4); 8.1077 (3.3); 8.1013 (3.2); 8.0857 (3.7); 8.0792 (3.9); 8.0681 (5.2); 7.7040 (5.6); 7.6820 (5.1); 6.0224 (0.4); 6.0051 (2.1); 5.9877 (3.4); 5.9703 (2.2); 5.9528 (0.5); 5.7573 (16.0); 4.0982 (0.4); 3.7817 (5.0); 3.5612 (0.8); 3.5314 (0.6); 3.5100 (0.6); 3.5038 (0.6); 3.4939 (0.6); 3.4745 (0.5); 3.4621 (0.5); 3.4504 (0.4); 3.1690 (3.8); 2.6775 (0.6); 2.6731 (0.9); 2.6687 (0.6); 2.5264 (1.8); 2.5126 (74.2); 2.5086 (150.3); 2.5042 (197.0); 2.4997 (147.2); 2.4955 (76.8); 2.3913 (0.5); 2.3353 (1.1); 2.3309 (1.4); 2.3266 (1.1); 1.5867 (11.8); 1.5694 (12.0); 1.2340 (0.4); -0.0003 (1.6) | |
| I-007 | 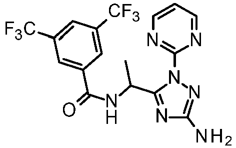 (reference compound) | ¹H-NMR(600.4 MHz, d₆-DMSO): | 446.2 [M+H]⁺ |
| | | δ= 9.4837 (3.5); 9.4721 (3.4); 8.8573 (5.6); 8.8508 (5.5); 8.4734 (16.0); 8.3054 (7.9); 7.4313 (2.7); 7.2051 (0.6); 7.1201 (0.7); 7.0350 (0.6); 6.0520 (1.6); 6.0406 (2.2); 6.0292 (1.6); 5.7257 (0.5); 5.6841 (0.4); 3.6473 (0.3); 3.5034 (0.5); 3.4955 (0.5); 3.4891 (0.4); 3.4793 (0.5); 3.4725 (0.6); 3.4635 (0.6); 3.4557 (0.6); 3.4536 (0.6); 3.3904 (5.2); 3.3534 (17.4); 3.1693 (0.7); 2.6193 (1.2); 2.6164 (2.3); 2.6134 (3.1); 2.6104 (2.2); 2.6074 (1.0); 2.5224 (8.9); 2.5193 (11.7); 2.5161 (14.4); 2.5073 (217.0); 2.5044 (420.5); 2.5014 (557.9); 2.4984 (411.1); 2.4954 (200.7); 2.3913 (1.2); 2.3884 (2.3); 2.3854 (3.1); 2.3823 (2.2); 2.3794 (1.1); 1.9086 (0.6); 1.6141 (10.7); 1.6028 (10.2); 1.2353 (0.6); 0.0053 (1.8); -0.0001 (47.8); -0.0057 (1.5) | |
| I-008 | 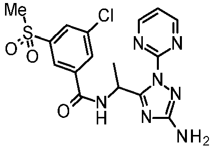 | ¹H-NMR(400 MHz, d₆-DMSO, ppm): δ = 3.5682 (16.0); 3.3248 (1.4); 2.5117 (6.8); 2.5074 (13.7); 2.5028 (18.0); 2.4983 (13.4); 2.4939 (6.8); 1.5995 (0.4); 1.5824 (0.4); 1.2892 (0.6); 1.2725 (0.7); 1.2663 (0.8); 1.2591 (0.4); 1.2499 (0.7); -0.0002 (9.9); - 0.0085 (0.4). | 422.1 [M+H]⁺ |
| I-009 | 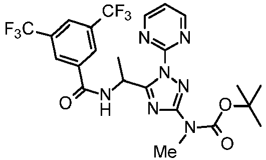 (reference compound) | ¹H-NMR(400.2 MHz, d₆-DMSO): | 561.1 [M+H]⁺ |
| | | δ= 9.6183 (0.5); 9.6006 (0.5); 8.9926 (2.6); 8.9804 (2.7); 8.4547 (1.8); 8.3216 (0.8); 7.6303 (0.7); 7.6182 (1.3); 7.6060 (0.7); 6.0646 (0.4); 6.0469 (0.6); 6.0293 (0.4); 5.7568 (2.2); 3.3346 (54.4); 3.2143 (7.0); 2.5263 (0.7); 2.5127 (14.4); 2.5084 (28.8); 2.5038 (37.0); 2.4993 (26.5); 2.4948 (12.8); 1.6756 (2.0); 1.6582 (2.0); 1.5031 (0.4); 1.3216 (16.0); -0.0002 (0.4) | |
| I-010 | 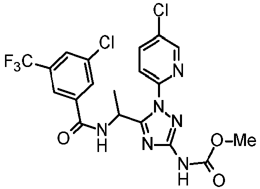 (reference compound) | ¹H-NMR(400.2 MHz, d₆-DMSO): | 503.1 [M+H]⁺ |
| | | δ= 10.3323 (3.0); 9.3962 (1.4); 9.3792 (1.4); 8.5911 (2.5); 8.5852 (2.6); 8.1943 (1.6); 8.1878 (1.5); 8.1725 (1.7); 8.1659 (1.7); 8.1421 (2.6); 8.0864 (2.6); 8.0684 (2.4); 7.7959 (2.6); 7.7738 (2.3); 5.9673 (1.0); 5.9501 (1.5); 5.9329 (1.0); 5.7568 (4.4); 4.1058 (0.5); 4.0926 (0.5); 3.6448 (16.0); 3.3308 (75.9); 3.1759 (2.6); 3.1628 (2.6); 2.6765 (0.4); 2.6724 (0.6); 2.6678 (0.4); 2.5076 (74.1); 2.5032 (94.4); 2.4987 (69.4); 2.3344 (0.4); 2.3301 (0.6); 2.3255 (0.4); 1.6324 (5.2); 1.6150 (5.2); -0.0002 (5.6) | |
| 1-011 | 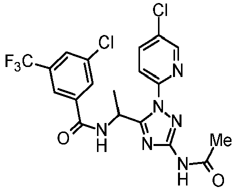 (reference compound) | ¹H-NMR(400.2 MHz, d₆-DMSO): | 487.1 [M+H]⁺ |
| | | δ= 10.6310 (1.2); 9.3851 (3.1); 9.3685 (3.2); 8.5918 (5.9); 8.5856 (5.9); 8.1953 (3.5); 8.1889 (3.3); 8.1734 (3.9); 8.1670 (3.8); 8.1478 (5.8); 8.0923 (5.8); 8.0705 (5.4); 7.8092 (5.6); 7.7873 (5.0); 5.9875 (0.5); 5.9703 (2.2); 5.9532 (3.5); 5.9361 (2.2); 5.9189 (0.5); 5.7569 (12.2); 4.1184 (0.8); 4.1055 (2.5); 4.0924 (2.5); 4.0791 (0.9); 3.3307 (118.0); 3.1759 (16.0); 3.1628 (15.6); 2.6766 (0.8); 2.6722 (1.1); 2.6679 (0.8); 2.5255 (3.8); 2.5077 (156.8); 2.5033 (198.8); 2.4988 (144.2); 2.3344 (0.9); 2.3301 (1.2); 2.3255 (0.9); 2.0560 (4.1); 1.6366 (12.2); 1.6193 (12.2); 1.4313 (0.4); 1.2341 (0.5); -0.0002 (2.1) | |
| I-012 | 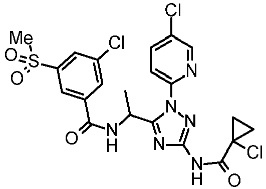 | ¹H-NMR(400.2 MHz, d₆-DMSO): | 557.1 [M+H]⁺ |
| | | δ= 10.5593 (6.0); 9.4902 (2.4); 9.4734 (2.5); 8.6470 (4.4); 8.6459 (4.4); 8.6406 (4.5); 8.6395 (4.3); 8.3155 (0.3); 8.3004 (3.4); 8.2967 (6.0); 8.2929 (3.5); 8.2263 (3.1); 8.2199 (2.8); 8.2045 (3.4); 8.1979 (3.7); 8.1935 (3.4); 8.1890 (5.1); 8.1850 (3.5); 8.1539 (3.9); 8.1497 (5.5); 8.1451 (2.9); 7.8360 (4.6); 7.8142 (4.2); 6.0317 (0.3); 6.0143 (1.6); 5.9970 (2.6); 5.9797 (1.7); 5.9622 (0.3); 5.7561 (16.0); 4.1196 (0.5); 4.1064 (1.4); 4.0933 (1.4); 4.0802 (0.5); 3.3326 (186.2); 3.3234 (32.5); 3.1753 (6.9); 3.1622 (6.7); 2.6763 (0.8); 2.6717 (1.0); 2.6672 (0.8); 2.5250 (3.8); 2.5116 (72.4); 2.5073 (140.4); 2.5028 (178.2); 2.4983 (127.1); 2.4939 (61.2); 2.3341 (0.8); 2.3295 (1.0); 2.3252 (0.8); 1.6534 (8.6); 1.6360 (8.6); 1.5904 (2.2); 1.5774 (5.1); 1.5694 (5.7); 1.5573 (2.9); 1.3803 (3.0); 1.3681 (5.6); 1.3606 (5.7); 1.3470 (2.2); -0.0002 (1.7) | |
| I-013 | 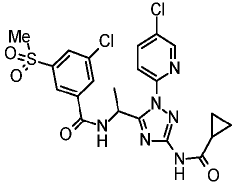 | ¹H-NMR(600.4 MHz, d₆-DMSO): | 523.1 [M+H]⁺ |
| | | δ= 8.5997 (0.5); 8.5955 (0.5); 8.2970 (0.4); 8.2945 (0.7); 8.2920 (0.4); 8.1940 (0.4); 8.1896 (0.4); 8.1870 (0.4); 8.1840 (0.6); 8.1811 (0.4); 8.1795 (0.4); 8.1751 (0.4); 8.1427 (0.4); 8.1398 (0.6); 8.1368 (0.3); 7.8004 (0.5); 7.7859 (0.5); 5.9716 (0.3); 3.3223 (3.4); 3.3079 (36.9); 3.0733 (2.9); 3.0619 (2.8); 2.6156 (0.3); 2.6126 (0.4); 2.5216 (1.0); 2.5185 (1.3); 2.5154 (1.3); 2.5066 (25.0); 2.5036 (52.2); 2.5006 (71.5); 2.4975 (52.4); 2.4945 (24.9); 2.3845 (0.4); 1.6414 (1.1); 1.6298 (1.1); 1.2004 (8.0); 1.1883 (16.0); 1.1762 (7.8); 0.7934 (1.1); 0.7829 (1.5); -0.0001 (7.7) | |
| I-014 | 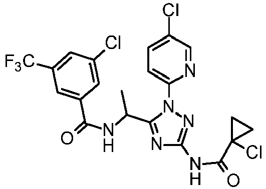 (reference compound) | ¹H-NMR(400.2 MHz, d₆-DMSO): | 549.1 [M+H]⁺ |
| | | δ= 10.5523 (4.6); 9.4356 (2.2); 9.4186 (2.2); 8.6328 (4.1); 8.6273 (4.2); 8.2176 (2.8); 8.2111 (2.6); 8.1957 (3.1); 8.1892 (3.1); 8.1531 (4.0); 8.1001 (4.0); 8.0754 (3.7); 7.8278 (4.4); 7.8060 (4.0); 6.0139 (0.3); 5.9966 (1.6); 5.9794 (2.5); 5.9621 (1.6); 5.7568 (6.1); 4.1193 (0.8); 4.1062 (2.4); 4.0930 (2.5); 4.0800 (0.8); 3.3315 (129.4); 3.1757 (16.0); 3.1626 (15.7); 2.6766 (0.7); 2.6720 (0.9); 2.6675 (0.7); 2.5254 (3.2); 2.5120 (62.6); 2.5076 (122.7); 2.5031 (156.1); 2.4985 (111.1); 2.4941 (53.0); 2.3344 (0.7); 2.3299 (0.9); 2.3253 (0.6); 1.6501 (8.4); 1.6327 (8.4); 1.5907 (2.2); 1.5777 (5.0); 1.5698 (5.5); 1.5576 (2.8); 1.3804 (2.9); 1.3681 (5.3); 1.3605 (5.4); 1.3469 (2.1); -0.0002 (1.7) | |
| I-015 | 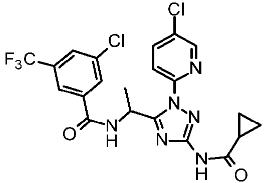 (reference compound) | ¹H-NMR(400.2 MHz, d₆-DMSO): | 513.1 [M+H]⁺ |
| | | δ= 10.9181 (1.3); 9.3849 (2.2); 9.3681 (2.2); 8.5886 (4.0); 8.5832 (4.0); 8.5822 (3.9); 8.1935 (2.7); 8.1870 (2.5); 8.1716 (3.0); 8.1651 (3.0); 8.1512 (4.0); 8.0959 (3.9); 8.0715 (3.7); 7.7979 (4.0); 7.7759 (3.6); 5.9931 (0.3); 5.9758 (1.5); 5.9587 (2.3); 5.9416 (1.5); 5.9242 (0.3); 5.7568 (8.7); 4.1190 (0.9); 4.1059 (2.8); 4.0927 (2.8); 4.0797 (1.0); 3.3315 (138.5); 3.1758 (16.0); 3.1627 (15.6); 2.6767 (0.7); 2.6722 (0.9); 2.6676 (0.7); 2.5255 (3.4); 2.5119 (62.6); 2.5077 (121.5); 2.5032 (154.8); 2.4986 (111.1); 2.4943 (54.0); 2.3344 (0.6); 2.3300 (0.9); 2.3255 (0.6); 1.8973 (0.5); 1.6410 (8.1); 1.6236 (8.1); 0.7974 (9.6); 0.7819 (10.5); - 0.0002 (1.8) | |
| I-016 | 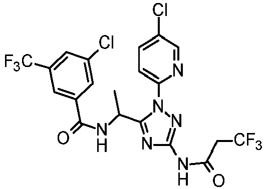 (reference compound) | ¹H-NMR(400.2 MHz, d₆-DMSO): | 555.1 [M+H]⁺ |
| | | δ= 11.1330 (1.6); 9.3925 (2.5); 9.3758 (2.5); 8.6051 (4.5); 8.5993 (4.6); 8.3162 (0.3); 8.2109 (2.8); 8.2045 (2.7); 8.1890 (3.2); 8.1825 (3.2); 8.1449 (4.9); 8.0885 (4.9); 8.0718 (4.6); 7.8249 (3.9); 7.8030 (3.6); 5.9848 (0.4); 5.9674 (1.9); 5.9503 (2.9); 5.9332 (1.9); 5.9160 (0.4); 5.7567 (16.0); 4.1204 (0.5); 4.1073 (1.6); 4.0942 (1.6); 4.0812 (0.6); 3.5521 (1.1); 3.5278 (1.1); 3.3339 (289.5); 3.1759 (9.2); 3.1627 (8.8); 2.6768 (0.9); 2.6723 (1.3); 2.6677 (0.9); 2.5257 (4.2); 2.5122 (82.7); 2.5078 (164.8); 2.5033 (212.8); 2.4987 (153.3); 2.4943 (74.3); 2.3347 (0.9); 2.3300 (1.2); 2.3256 (0.9); 1.6442 (10.2); 1.6268 (10.2); -0.0002 (2.1) | |
| I-017 | 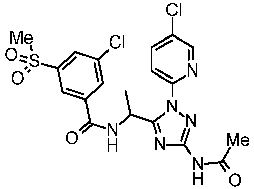 | ¹H-NMR(400.2 MHz, d₆-DMSO): | 497.1 [M+H]⁺ |
| | | δ= 9.4423 (0.4); 9.4255 (0.5); 8.6056 (1.3); 8.6001 (1.3); 8.3160 (16.0); 8.2904 (1.8); 8.2868 (1.1); 8.2041 (0.8); 8.1976 (0.8); 8.1822 (2.4); 8.1782 (1.5); 8.1760 (1.4); 8.1496 (1.1); 8.1454 (1.6); 8.1411 (0.9); 7.8178 (1.2); 7.7955 (1.1); 5.9849 (0.4); 5.9685 (0.5); 5.9517 (0.4); 5.7562 (6.3); 3.3361 (48.9); 3.3234 (11.8); 3.3129 (4.8); 3.1677 (1.3); 2.6717 (0.4); 2.6675 (0.3); 2.5073 (56.6); 2.5029 (71.4); 2.4985 (53.0); 2.3295 (0.4); 2.3254 (0.3); 2.0546 (1.0); 1.6400 (2.7); 1.6226 (2.7); -0.0002 (0.5) | |
| I-018 | 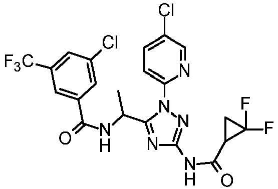 (reference compound) | ¹H-NMR(400.2 MHz, d₆-DMSO): | 549.1 [M+H]⁺ |
| | | δ= 11.2326 (0.6); 9.3981 (1.4); 9.3814 (1.6); 9.3708 (0.7); 8.6019 (2.6); 8.5969 (3.3); 8.2088 (1.2); 8.2027 (1.4); 8.1870 (1.4); 8.1809 (1.6); 8.1510 (2.7); 8.0951 (2.7); 8.0720 (3.2); 7.8144 (2.8); 7.7925 (2.5); 5.9796 (0.9); 5.9624 (1.3); 5.9562 (0.8); 5.9453 (0.9); 5.7569 (16.0); 4.1054 (0.8); 4.0923 (0.9); 3.3303 (119.9); 3.1757 (5.6); 3.1626 (5.4); 2.8335 (0.3); 2.6765 (0.7); 2.6720 (1.0); 2.6675 (0.7); 2.6630 (0.4); 2.5254 (3.4); 2.5206 (5.5); 2.5119 (61.8); 2.5076 (122.0); 2.5031 (156.6); 2.4985 (112.3); 2.4941 (54.1); 2.3344 (0.6); 2.3298 (0.9); 2.3254 (0.6); 2.0257 (0.6); 2.0056 (1.2); 1.9888 (1.1); 1.9792 (1.2); 1.9587 (0.6); 1.6455 (6.5); 1.6281 (6.4); 1.2346 (0.4); -0.0002 (2.0) | |
| I-019 | 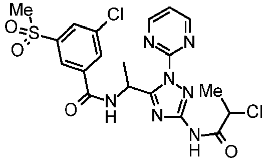 | ¹H-NMR(400.2 MHz, d₆-DMSO): | 512.1 [M+H]⁺ |
| | | δ= 11.1079 (1.4); 9.4730 (1.1); 9.4649 (1.3); 9.4563 (1.3); 9.4481 (1.2); 8.9810 (5.1); 8.9781 (5.2); 8.9689 (5.3); 8.9660 (5.2); 8.2691 (3.1); 8.2648 (3.2); 8.2609 (1.6); 8.1721 (2.8); 8.1678 (2.8); 8.1629 (1.6); 8.1415 (3.4); 7.6171 (1.4); 7.6147 (1.4); 7.6050 (2.6); 7.6026 (2.6); 7.5929 (1.4); 7.5905 (1.3); 6.0132 (1.1); 5.9958 (1.8); 5.9789 (1.2); 5.7565 (16.0); 4.6982 (0.5); 3.3342 (104.1); 3.3193 (20.8); 3.1755 (1.2); 3.1624 (1.2); 2.6767 (0.6); 2.6722 (0.7); 2.5076 (101.7); 2.5033 (127.8); 2.4988 (94.1); 2.3343 (0.6); 2.3301 (0.7); 2.3255 (0.5); 1.6658 (6.6); 1.6484 (6.7); 1.5961 (8.7); 1.5794 (8.6); -0.0002 (1.2) | |
| I-020 | 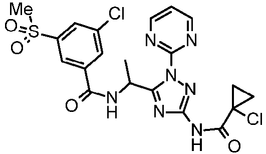 | ¹H-NMR(400.2 MHz, d₆-DMSO): | 524.1 [M+H]⁺ |
| | | δ= 10.5453 (4.0); 9.5069 (1.8); 9.4895 (1.8); 8.9958 (7.1); 8.9837 (7.3); 8.2743 (2.4); 8.2708 (4.3); 8.2671 (2.8); 8.1790 (2.2); 8.1745 (3.8); 8.1707 (2.7); 8.1462 (2.8); 8.1419 (3.9); 8.1376 (2.1); 7.6394 (1.9); 7.6273 (3.5); 7.6151 (1.8); 6.0368 (1.1); 6.0194 (1.8); 6.0020 (1.1); 5.7563 (16.0); 4.1053 (0.3); 4.0921 (0.4); 3.3749 (0.4); 3.3620 (0.7); 3.3316 (67.2); 3.3192 (20.5); 3.1754 (1.9); 3.1623 (1.8); 2.6764 (0.5); 2.6720 (0.7); 2.6674 (0.5); 2.5251 (2.8); 2.5073 (95.6); 2.5029 (121.4); 2.4985 (90.0); 2.3341 (0.5); 2.3298 (0.7); 2.3253 (0.5); 1.6669 (6.4); 1.6495 (6.4); 1.5895 (1.4); 1.5779 (3.1); 1.5694 (3.9); 1.5578 (1.9); 1.3844 (2.0); 1.3727 (3.9); 1.3650 (3.9); 1.3523 (1.5); -0.0002 (1.2) | |
| I-021 | 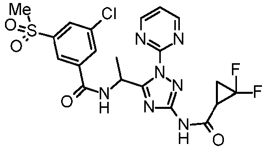 | ¹H-NMR(400.2 MHz, d₆-DMSO): | 526.1 [M+H]⁺ |
| | | δ= 11.2094 (0.6); 9.4583 (1.2); 9.4418 (1.2); 8.9706 (4.8); 8.9691 (4.7); 8.9586 (5.0); 8.2613 (3.1); 8.1655 (2.8); 8.1621 (2.9); 8.1422 (2.7); 8.1383 (3.8); 7.6054 (1.5); 7.5934 (2.8); 7.5813 (1.4); 6.0092 (0.9); 5.9935 (1.4); 5.9765 (0.9); 5.7564 (16.0); 4.1063 (0.3); 4.0933 (0.3); 3.3330 (89.1); 3.3180 (20.2); 3.1754 (2.0); 3.1623 (1.9); 2.6764 (0.6); 2.6720 (0.8); 2.6676 (0.6); 2.5075 (112.0); 2.5031 (142.1); 2.4987 (104.6); 2.3343 (0.6); 2.3299 (0.8); 2.3253 (0.6); 2.0281 (0.6); 2.0033 (1.1); 1.9927 (1.0); 1.9807 (1.3); 1.6630 (5.9); 1.6457 (5.9); -0.0002 (1.5) | |
| I-022 | 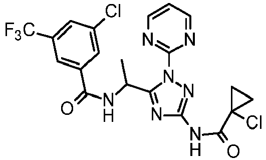 (reference compound) | ¹H-NMR(400.2 MHz, d₆-DMSO, ppm): δ = 11.7003 (0.4); 10.5420 (3.1); 9.4658 (1.3); 9.4485 (1.3); 8.9917 (6.4); 8.9795 (6.6); 8.1447 (2.3); 8.0962 (2.4); 8.0704 (2.2); 7.6351 (1.7); 7.6230 (3.2); 7.6108 (1.6); 6.0247 (1.0); 6.0073 (1.5); 5.9899 (1.0); 5.7569 (0.5); 4.2608 (0.4); 3.7780 (0.3); 3.6476 (0.3); 3.3359 (23.8); 2.8917 (0.6); 2.7325 (0.5); 2.6767 (0.4); 2.6723 (0.6); 2.6679 (0.5); 2.5257 (1.8); 2.5209 (2.7); 2.5122 (35.5); 2.5078 (72.8); 2.5033 (96.9); 2.4988 (72.6); 2.4944 (37.0); 2.4250 (0.3); 2.3347 (0.4); 2.3300 (0.6); 2.3257 (0.4); 1.9894 (0.4); 1.9094 (16.0); 1.6628 (5.2); 1.6453 (5.2); 1.5892 (1.2); 1.5777 (2.5); 1.5670 (4.3); 1.5535 (6.2); 1.5454 (6.3); 1.5335 (3.4); 1.4918 (0.4); 1.4184 (0.4); 1.3837 (1.7); 1.3763 (4.0); 1.3720 (3.3); 1.3646 (8.9); 1.3568 (6.2); 1.3520 (1.9); 1.3432 (2.3); 1.2342 (0.5); 1.1755 (0.4); 0.1459 (0.5); 0.0080 (3.4); -0.0002 (103.6); -0.0084 (4.5); -0.1495 (0.5). | 514.1 [M+H]⁺ |
| I-023 | 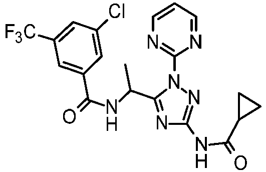 (reference compound) | ¹H-NMR(400.2 MHz, d₆-DMSO): | 480.2 [M+H]⁺ |
| | | δ= 13.1206 (0.4); 11.7976 (0.4); 10.8800 (1.6); 9.4104 (2.6); 9.3935 (2.6); 9.0016 (0.6); 8.9894 (0.6); 8.9539 (10.6); 8.9418 (10.7); 8.8594 (0.5); 8.8475 (0.6); 8.2583 (0.7); 8.2203 (0.8); 8.1783 (0.4); 8.1370 (5.0); 8.0855 (5.1); 8.0655 (4.8); 7.5851 (2.8); 7.5730 (5.2); 7.5608 (2.6); 7.2144 (0.3); 6.0147 (0.4); 6.0068 (0.4); 5.9979 (1.8); 5.9806 (2.9); 5.9634 (1.8); 5.9461 (0.4); 5.7566 (16.0); 5.7443 (0.5); 4.2174 (0.5); 4.0108 (0.9); 3.3315 (152.7); 3.1758 (1.3); 3.1627 (1.3); 2.6765 (0.9); 2.6721 (1.2); 2.6679 (0.9); 2.5252 (4.0); 2.5075 (176.0); 2.5032 (223.6); 2.4989 (164.9); 2.3343 (1.0); 2.3301 (1.4); 2.3257 (1.0); 1.9097 (0.6); 1.8472 (0.4); 1.6521 (10.0); 1.6348 (10.0); 1.6046 (0.7); 1.5941 (0.9); 1.5872 (0.7); 1.5777 (0.7); 1.5297 (1.2); 1.5126 (1.1); 1.3513 (0.4); 1.2982 (0.3); 1.2590 (0.5); 1.2345 (0.9); 0.8918 (0.5); 0.8847 (0.7); 0.8650 (1.1); 0.8539 (1.0); 0.8346 (0.6); 0.7964 (10.6); 0.7810 (13.6); -0.0002 (2.3) | |
| I-024 | 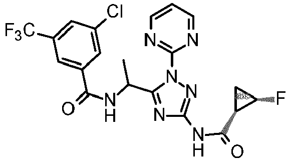 (reference compound) | ¹H-NMR(400.2 MHz, d₆-DMSO): | 498.2 [M+H]⁺ |
| | | δ= 11.0950 (0.7); 9.4161 (2.8); 9.3991 (2.8); 8.9577 (14.6); 8.9456 (15.0); 8.1352 (5.0); 8.0822 (5.0); 8.0664 (4.6); 7.5924 (3.9); 7.5803 (7.2); 7.5681 (3.8); 6.0119 (0.4); 5.9948 (2.0); 5.9775 (3.2); 5.9603 (2.0); 5.9433 (0.4); 5.7565 (16.0); 4.9328 (0.9); 4.7707 (0.9); 3.3372 (324.4); 3.1761 (1.2); 3.1629 (1.2); 2.6903 (0.5); 2.6815 (0.4); 2.6772 (0.8); 2.6727 (1.1); 2.6680 (0.8); 2.6636 (0.4); 2.5261 (3.5); 2.5213 (5.7); 2.5127 (67.2); 2.5082 (135.1); 2.5037 (175.5); 2.4991 (125.7); 2.4946 (60.4); 2.3395 (0.5); 2.3350 (0.9); 2.3305 (1.1); 2.3259 (0.9); 1.6532 (10.2); 1.6358 (10.2); 1.5505 (0.5); 1.5412 (0.7); 1.5250 (0.5); 1.5108 (0.6); 1.4850 (0.7); 1.4709 (0.5); 1.4430 (0.6); 1.2539 (0.5); 1.2383 (1.3); 1.2221 (1.6); 1.2056 (1.6); 1.1894 (1.3); 1.1729 (0.7); -0.0002 (1.9) | |
| I-025 | 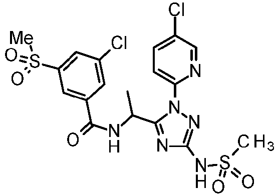 | | 533.1 [M+H]⁺ |
| I-026 | 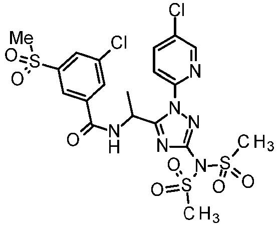 | ¹H-NMR(400.2 MHz, d₆-DMSO): | 611.1 [M+H]⁺ |
| | | δ= 9.5449 (0.8); 9.5271 (0.8); 8.6885 (1.5); 8.6832 (1.6); 8.2629 (1.0); 8.2564 (1.0); 8.2412 (2.2); 8.2381 (2.3); 8.2346 (2.4); 8.1597 (1.1); 8.1552 (1.9); 8.1509 (1.2); 8.1168 (1.3); 8.1127 (1.7); 8.1084 (1.0); 7.9674 (1.5); 7.9454 (1.4); 6.0185 (0.6); 6.0008 (0.9); 5.9831 (0.6); 5.7563 (11.5); 3.6401 (16.0); 3.3300 (76.3); 3.3208 (11.0); 3.1750 (0.7); 3.1619 (0.7); 2.6758 (0.4); 2.6713 (0.5); 2.6666 (0.4); 2.5248 (1.7); 2.5112 (33.7); 2.5069 (66.7); 2.5024 (85.6); 2.4978 (61.5); 2.4934 (29.8); 2.3337 (0.4); 2.3292 (0.5); 2.3248 (0.4); 1.6563 (3.0); 1.6389 (3.0); -0.0002 (1.0) | |
| I-027 | 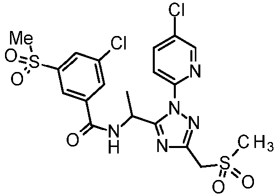 | ¹H-NMR(600.4 MHz, d₆-DMSO): | 532.0 [M+H]⁺ |
| | | δ= 9.4695 (2.0); 9.4579 (2.1); 8.6538 (3.4); 8.6530 (3.2); 8.6496 (3.4); 8.6487 (3.0); 8.2478 (2.8); 8.2453 (5.0); 8.2427 (2.8); 8.2274 (2.6); 8.2231 (2.5); 8.2128 (2.8); 8.2085 (2.8); 8.1407 (1.6); 8.1374 (5.1); 8.1347 (7.9); 8.1322 (4.5); 8.1289 (1.3); 7.8809 (3.7); 7.8802 (3.4); 7.8664 (3.5); 7.8656 (3.1); 5.9792 (1.5); 5.9675 (2.3); 5.9559 (1.5); 5.7519 (13.9); 4.6872 (7.4); 3.3144 (23.8); 3.3057 (131.6); 3.1369 (16.0); 2.6184 (0.5); 2.6153 (1.0); 2.6123 (1.3); 2.6092 (1.0); 2.6063 (0.4); 2.5213 (3.7); 2.5182 (4.8); 2.5150 (5.8); 2.5064 (78.9); 2.5033 (158.2); 2.5003 (212.9); 2.4972 (152.9); 2.4942 (70.8); 2.3902 (0.4); 2.3873 (1.0); 2.3842 (1.3); 2.3812 (0.9); 2.3782 (0.4); 1.6531 (7.7); 1.6415 (7.7); 0.0053 (0.6); -0.0001 (15.3); -0.0057 (0.5) | |
| I-028 | 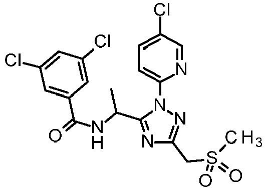 (reference compound) | ¹H-NMR(600.4 MHz, d₆-DMSO): | 489.9 [M+H]⁺ |
| | | δ= 12.6639 (2.2); 9.2674 (2.0); 9.2558 (2.0); 8.6416 (3.3); 8.6408 (3.2); 8.6373 (3.4); 8.6364 (3.1); 8.3199 (0.9); 8.3149 (0.7); 8.3126 (1.1); 8.3078 (0.8); 8.3046 (1.0); 8.2223 (2.6); 8.2180 (2.5); 8.2078 (2.8); 8.2034 (2.8); 8.1926 (5.7); 8.1894 (6.4); 8.1412 (1.7); 8.1380 (2.7); 8.1348 (1.3); 7.9841 (0.3); 7.9758 (1.7); 7.9721 (1.3); 7.9685 (1.4); 7.9672 (1.3); 7.9636 (1.5); 7.9606 (1.9); 7.9596 (1.8); 7.9516 (0.4); 7.9242 (1.3); 7.9205 (0.9); 7.9156 (1.1); 7.9130 (0.6); 7.9089 (0.7); 7.8685 (3.7); 7.8677 (3.5); 7.8540 (3.4); 7.8532 (3.2); 7.8021 (2.2); 7.7990 (4.6); 7.7958 (2.8); 7.7641 (11.5); 7.7609 (9.7); 5.9302 (1.5); 5.9186 (2.4); 5.9070 (1.5); 5.8954 (0.3); 4.6822 (7.4); 3.3057 (210.1); 3.2911 (0.5); 3.2821 (0.3); 3.1338 (16.0); 2.6185 (0.7); 2.6154 (1.5); 2.6124 (2.1); 2.6093 (1.5); 2.6062 (0.7); 2.5214 (5.4); 2.5183 (6.9); 2.5152 (8.0); 2.5065 (122.6); 2.5034 (246.0); 2.5004 (330.6); 2.4973 (237.8); 2.4943 (110.4); 2.3905 (0.7); 2.3874 (1.5); 2.3843 (2.0); 2.3813 (1.4); 2.3783 (0.7); 2.0719 (0.6); 1.6287 (7.9); 1.6170 (7.9); 0.0053 (1.0); -0.0001 (26.2); -0.0057 (0.8) | |
| I-029 | 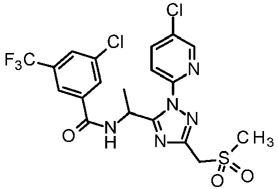 (reference compound) | ¹H-NMR (600 MHz, d₆-DMSO, ppm): δ = 9.4181 (2.0); 9.4065 (2.1); 8.6401 (3.3); 8.6393 (3.2); 8.6358 (3.4); 8.6350 (3.1); 8.2177 (2.6); 8.2133 (2.5); 8.2031 (2.8); 8.1988 (2.8); 8.1000 (3.2); 8.0613 (2.9); 8.0412 (3.2); 7.8725 (3.7); 7.8717 (3.4); 7.8580 (3.4); 7.8572 (3.2); 5.9626 (1.5); 5.9510 (2.3); 5.9394 (1.5); 4.6884 (7.6); 3.3096 (30.6); 3.1350 (16.0); 2.5229 (0.6); 2.5198 (0.7); 2.5167 (0.7); 2.5080 (16.6); 2.5049 (35.6); 2.5018 (49.2); 2.4988 (35.8); 2.4958 (16.6); 2.0735 (1.4); 1.6520 (7.8); 1.6404 (7.8); -0.0001 (1.9) | 522.1 [M+H]⁺ |
| I-030 | 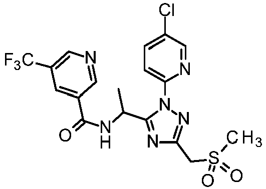 (reference compound) | ¹H-NMR (600 MHz, d₆-DMSO, ppm): δ = 9.5022 (2.0); 9.4906 (2.0); 9.1661 (2.9); 9.1630 (2.9); 9.1229 (2.5); 9.1208 (2.5); 8.6507 (3.2); 8.6499 (3.2); 8.6464 (3.4); 8.6455 (3.1); 8.4753 (2.8); 8.4721 (1.6); 8.2236 (2.6); 8.2193 (2.5); 8.2091 (2.8); 8.2048 (2.8); 7.8840 (3.6); 7.8832 (3.5); 7.8695 (3.4); 7.8687 (3.2); 5.9964 (1.5); 5.9848 (2.4); 5.9732 (1.5); 4.6911 (7.8); 3.3092 (56.3); 3.1360 (16.0); 2.6132 (0.4); 2.5223 (0.9); 2.5192 (1.1); 2.5161 (1.1); 2.5073 (24.1); 2.5043 (51.5); 2.5012 (71.3); 2.4982 (51.9); 2.4952 (24.3); 2.3852 (0.4); 2.0729 (0.9); 1.6638 (7.9); 1.6521 (7.9); -0.0001 (4.5). | 489.2 [M+H]⁺ |
| I-031 | major enantiomer (absolute configuration: S) 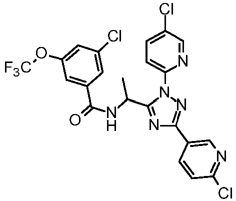 (reference compound) | ¹H-NMR(600 MHz, d₆-DMSO, ppm): δ = 9.3866 (4.4); 9.3752 (4.6); 9.0449 (6.6); 9.0410 (6.7); 8.6467 (7.0); 8.6426 (7.1); 8.4321 (4.8); 8.4280 (4.6); 8.4182 (5.0); 8.4141 (4.9); 8.2523 (4.6); 8.2480 (4.4); 8.2378 (5.1); 8.2335 (5.1); 8.0062 (7.7); 7.9918 (6.9); 7.9289 (5.3); 7.9263 (8.4); 7.9237 (5.4); 7.7735 (5.7); 7.7067 (5.8); 7.6888 (7.0); 7.6750 (6.8); 6.0195 (0.7); 6.0079 (3.0); 5.9964 (4.8); 5.9849 (3.1); 5.9732 (0.6); 3.9195 (1.0); 3.3268 (30.6); 2.8954 (1.0); 2.7367 (0.8); 2.6198 (0.4); 2.5288 (0.8); 2.5258 (1.0); 2.5227 (1.1); 2.5138 (20.0); 2.5108 (41.8); 2.5078 (57.4); 2.5048 (42.4); 2.5019 (20.2); 2.3917 (0.4); 1.7082 (15.9); 1.6966 (16.0); -0.0001 (3.4). | 557.1 [M+H]⁺ |
| I-032 | 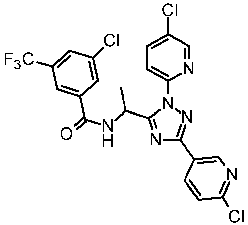 (reference compound) | ¹H-NMR(600.4 MHz, d₆-DMSO): | 543.1 [M+H]⁺ |
| | | δ= 9.4517 (4.0); 9.4403 (4.1); 9.0463 (6.7); 9.0452 (6.7); 9.0423 (6.8); 9.0411 (6.4); 8.6496 (6.9); 8.6487 (6.8); 8.6453 (7.1); 8.6444 (6.5); 8.4340 (5.7); 8.4300 (5.4); 8.4201 (6.0); 8.4161 (6.0); 8.2509 (5.5); 8.2465 (5.2); 8.2364 (6.1); 8.2320 (6.1); 8.1301 (6.7); 8.1275 (4.2); 8.0653 (12.7); 8.0057 (7.7); 8.0048 (7.2); 7.9912 (6.9); 7.9903 (6.6); 7.6904 (7.1); 7.6892 (6.8); 7.6765 (7.0); 7.6753 (6.6); 6.0292 (0.6); 6.0176 (3.0); 6.0060 (4.8); 5.9944 (3.1); 5.9829 (0.6); 3.9176 (0.6); 3.3059 (719.0); 3.2893 (0.8); 3.2820 (0.8); 2.6186 (1.9); 2.6155 (4.0); 2.6125 (5.5); 2.6094 (3.9); 2.6064 (1.8); 2.5366 (0.8); 2.5215 (15.2); 2.5184 (19.7); 2.5153 (22.7); 2.5066 (314.7); 2.5036 (636.7); 2.5005 (860.8); 2.4974 (617.7); 2.4944 (284.0); 2.3905 (1.8); 2.3875 (3.8); 2.3844 (5.3); 2.3814 (3.7); 2.3783 (1.6); 1.7141 (16.0); 1.7025 (16.0); 1.6939 (0.6); 1.2345 (0.5); 0.9486 (0.5); 0.9377 (0.6); 0.0968 (0.3); 0.0053 (3.8); -0.0001 (96.2); -0.0057 (2.8); -0.1002 (0.3) | |
| I-033 | 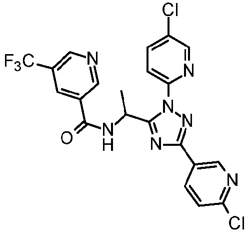 (reference compound) | ¹H-NMR(600.4 MHz, d₆-DMSO): | 508.0 [M+H]⁺ |
| | | δ= 9.5369 (3.9); 9.5255 (4.0); 9.1934 (6.1); 9.1902 (5.9); 9.1240 (5.2); 9.1219 (5.1); 9.0515 (6.4); 9.0505 (6.4); 9.0475 (6.5); 9.0465 (6.0); 8.6616 (6.6); 8.6609 (6.5); 8.6574 (6.8); 8.6566 (6.3); 8.5035 (5.7); 8.4391 (5.3); 8.4350 (5.0); 8.4252 (5.5); 8.4211 (5.4); 8.2577 (5.0); 8.2534 (4.8); 8.2432 (5.6); 8.2389 (5.5); 8.0192 (7.4); 8.0047 (6.6); 8.0039 (6.3); 7.6914 (6.8); 7.6905 (6.4); 7.6775 (6.6); 7.6765 (6.3); 6.0657 (0.6); 6.0542 (3.0); 6.0426 (4.8); 6.0311 (3.0); 6.0195 (0.6); 3.9177 (0.5); 3.3066 (245.3); 3.2829 (0.3); 2.6191 (0.8); 2.6161 (1.7); 2.6131 (2.3); 2.6100 (1.6); 2.6070 (0.7); 2.5221 (6.3); 2.5190 (8.1); 2.5158 (9.5); 2.5071 (138.4); 2.5041 (277.3); 2.5011 (373.0); 2.4980 (269.0); 2.4950 (125.4); 2.3911 (0.9); 2.3880 (1.7); 2.3850 (2.3); 2.3819 (1.6); 2.3789 (0.7); 1.8883 (0.3); 1.8766 (0.3); 1.7260 (16.0); 1.7144 (16.0); 0.0053 (1.0); -0.0001 (26.3); -0.0057 (0.8) | |
| I-034 | 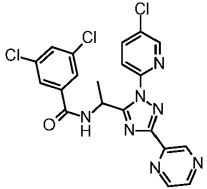 (reference compound) | ¹H-NMR(600.4 MHz, d₆-DMSO): | 475.1 [M+H]⁺ |
| | | δ= 9.3501 (1.3); 9.3387 (1.4); 9.3190 (3.1); 9.3165 (3.0); 8.7830 (1.8); 8.7804 (1.9); 8.7788 (2.4); 8.7763 (2.2); 8.7582 (3.7); 8.7541 (2.6); 8.6721 (2.3); 8.6679 (2.3); 8.5585 (1.9); 8.2690 (1.8); 8.2647 (1.6); 8.2545 (2.0); 8.2502 (1.9); 8.0212 (2.5); 8.0067 (2.3); 7.8068 (14.0); 7.6530 (0.4); 7.3637 (1.0); 7.3605 (0.9); 6.0174 (1.0); 6.0059 (1.6); 5.9942 (1.0); 3.3061 (2015.4); 3.2825 (2.7); 2.6181 (5.7); 2.6151 (11.8); 2.6121 (16.0); 2.6090 (11.2); 2.6060 (5.0); 2.5361 (2.4); 2.5211 (44.4); 2.5180 (57.6); 2.5148 (66.8); 2.5062 (955.7); 2.5031 (1909.6); 2.5001 (2556.6); 2.4970 (1828.8); 2.4940 (841.4); 2.4049 (0.4); 2.4017 (0.4); 2.3901 (5.8); 2.3871 (11.8); 2.3840 (15.8); 2.3810 (11.0); 2.3779 (4.9); 2.0849 (0.6); 2.0716 (1.2); 1.7052 (5.3); 1.6935 (5.1); 1.6853 (0.4); 1.5086 (0.5); 1.2353 (1.0); 0.0967 (0.5); 0.0053 (5.7); - 0.0001 (146.2); -0.0057 (4.3); -0.1000 (0.6) | |
| I-035 | 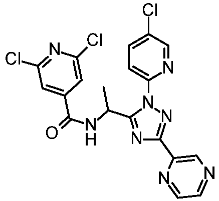 (reference compound) | ¹H-NMR(600.4 MHz, d₆-DMSO): | 475.0 [M+H]⁺ |
| | | δ= 9.5918 (1.1); 9.5810 (1.1); 9.3291 (3.5); 9.3267 (3.4); 8.7899 (2.0); 8.7873 (2.2); 8.7857 (2.8); 8.7832 (2.5); 8.7652 (4.1); 8.7611 (2.9); 8.6791 (2.6); 8.6782 (2.5); 8.6748 (2.6); 8.6739 (2.4); 8.2779 (2.0); 8.2736 (1.9); 8.2634 (2.2); 8.2591 (2.2); 8.0302 (2.9); 8.0294 (2.7); 8.0157 (2.6); 8.0148 (2.4); 7.9189 (1.3); 7.8468 (16.0); 6.0431 (0.7); 6.0318 (1.1); 6.0204 (0.7); 4.2860 (0.3); 3.8267 (0.4); 3.8156 (0.6); 3.8045 (0.4); 3.3070 (126.9); 2.6190 (0.4); 2.6160 (0.8); 2.6130 (1.1); 2.6099 (0.8); 2.6070 (0.3); 2.5220 (3.0); 2.5189 (4.0); 2.5157 (4.8); 2.5070 (65.1); 2.5040 (129.3); 2.5010 (173.2); 2.4979 (124.6); 2.4949 (57.8); 2.3910 (0.4); 2.3880 (0.8); 2.3849 (1.0); 2.3819 (0.7); 2.3788 (0.3); 1.7108 (6.2); 1.6992 (6.2); 1.2376 (5.3); 1.2264 (5.5); 1.2201 (7.1); 1.2090 (6.7); 0.0053 (0.5); - 0.0001 (11.4); -0.0057 (0.4) | |
| I-036 | 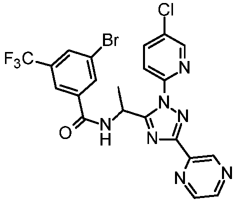 (reference compound) | ¹H-NMR(600.4 MHz, d₆-DMSO): | 554.0 [M+H]⁺ |
| | | δ= 9.4929 (3.9); 9.4814 (4.0); 9.3207 (9.5); 9.3182 (9.4); 8.7827 (4.9); 8.7801 (5.6); 8.7786 (7.1); 8.7761 (6.7); 8.7582 (11.1); 8.7540 (8.0); 8.6688 (6.7); 8.6681 (6.4); 8.6645 (7.0); 8.2787 (6.8); 8.2637 (5.1); 8.2594 (4.9); 8.2492 (5.7); 8.2449 (5.7); 8.1676 (6.0); 8.1146 (6.5); 8.0226 (7.7); 8.0082 (6.9); 6.0588 (0.6); 6.0473 (2.9); 6.0357 (4.6); 6.0241 (2.9); 6.0125 (0.6); 3.3070 (261.3); 3.2831 (0.4); 2.6191 (0.8); 2.6162 (1.8); 2.6131 (2.5); 2.6101 (1.8); 2.6070 (0.9); 2.5222 (5.8); 2.5191 (6.9); 2.5160 (6.6); 2.5072 (133.8); 2.5042 (283.1); 2.5011 (390.3); 2.4981 (284.0); 2.4950 (133.3); 2.3911 (0.8); 2.3881 (1.7); 2.3851 (2.4); 2.3820 (1.7); 2.3791 (0.8); 1.7257 (15.9); 1.7141 (16.0); 0.0053 (0.8); -0.0001 (27.8); -0.0056 (0.9) | |
| I-037 | 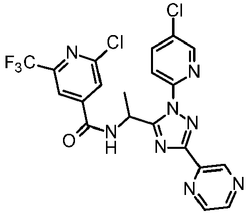 (reference compound) | ¹H-NMR(600.4 MHz, d₆-DMSO): | 509.1 [M+H]⁺ |
| | | δ= 9.7335 (2.4); 9.7252 (2.5); 9.3307 (9.4); 9.3282 (9.3); 8.7888 (5.0); 8.7863 (5.7); 8.7847 (7.2); 8.7822 (6.8); 8.7649 (11.1); 8.7608 (7.9); 8.6805 (6.6); 8.6797 (6.5); 8.6762 (6.9); 8.6754 (6.4); 8.2747 (5.0); 8.2704 (4.8); 8.2602 (5.8); 8.2559 (5.8); 8.2151 (0.5); 8.2051 (9.0); 8.2034 (9.3); 8.1632 (8.7); 8.0331 (7.4); 8.0186 (6.6); 8.0178 (6.3); 6.0802 (1.3); 6.0693 (2.0); 6.0586 (1.5); 3.8313 (0.4); 3.3070 (270.7); 3.2807 (0.3); 2.6191 (0.8); 2.6162 (1.7); 2.6131 (2.4); 2.6100 (1.7); 2.6070 (0.8); 2.5222 (5.8); 2.5191 (7.0); 2.5160 (6.8); 2.5072 (134.8); 2.5042 (281.6); 2.5011 (385.6); 2.4981 (281.7); 2.4951 (133.6); 2.3912 (0.8); 2.3881 (1.8); 2.3851 (2.4); 2.3820 (1.7); 2.3790 (0.8); 1.7340 (15.9); 1.7224 (16.0); 1.2475 (3.1); 1.2364 (3.4); 1.2310 (4.8); 1.2198 (4.4); 0.0053 (0.8); -0.0001 (23.0); -0.0056 (0.8) | |
| I-038 | 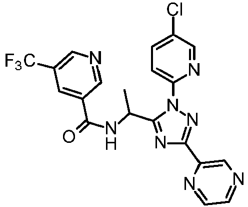 (reference compound) | ¹H-NMR(600.4 MHz, d₆-DMSO): | 475.1 [M+H]⁺ |
| | | δ= 9.5805 (3.8); 9.5688 (3.8); 9.3607 (0.8); 9.3269 (9.5); 9.3244 (9.2); 9.2038 (5.9); 9.2006 (5.7); 9.1257 (5.0); 9.1236 (4.9); 9.0620 (0.8); 9.0597 (0.8); 8.7837 (5.3); 8.7812 (5.8); 8.7796 (7.4); 8.7770 (6.9); 8.7673 (0.3); 8.7595 (11.1); 8.7554 (7.9); 8.6809 (6.8); 8.6799 (6.4); 8.6766 (6.9); 8.6757 (6.1); 8.5407 (0.8); 8.5241 (5.6); 8.5208 (3.2); 8.2701 (5.4); 8.2658 (5.1); 8.2556 (6.0); 8.2513 (5.9); 8.0358 (7.7); 8.0349 (7.0); 8.0213 (6.9); 8.0204 (6.3); 7.8994 (0.3); 6.2438 (0.4); 6.2232 (0.4); 6.0976 (0.6); 6.0861 (3.0); 6.0745 (4.8); 6.0629 (3.1); 6.0513 (0.6); 4.2769 (0.4); 3.8046 (0.4); 3.3068 (314.0); 3.2925 (0.9); 2.6190 (1.1); 2.6160 (2.2); 2.6129 (3.1); 2.6098 (2.2); 2.6068 (1.0); 2.5220 (7.9); 2.5188 (10.1); 2.5157 (11.5); 2.5070 (179.6); 2.5040 (364.0); 2.5009 (491.9); 2.4978 (353.2); 2.4948 (162.5); 2.3910 (1.1); 2.3879 (2.3); 2.3849 (3.1); 2.3818 (2.1); 2.3788 (1.0); 1.7398 (16.0); 1.7281 (16.0); 1.2443 (4.3); 1.2331 (5.1); 1.2288 (5.4); 1.2176 (4.6); 0.0053 (1.6); -0.0001 (41.4); - 0.0057 (1.2) | |
| I-039 | 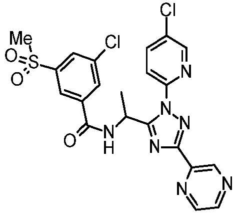 (reference compound) | ¹H-NMR(600.4 MHz, d₆-DMSO): | |
| | | δ= 9.5538 (4.2); 9.5422 (4.3); 9.3241 (9.6); 9.3217 (9.6); 8.7826 (5.1); 8.7801 (5.7); 8.7785 (7.4); 8.7760 (7.0); 8.7580 (11.4); 8.7539 (8.1); 8.6838 (6.8); 8.6830 (6.7); 8.6795 (7.1); 8.6787 (6.6); 8.2887 (5.6); 8.2861 (10.4); 8.2836 (5.9); 8.2743 (5.4); 8.2700 (5.2); 8.2598 (6.0); 8.2554 (6.0); 8.1854 (5.3); 8.1824 (8.3); 8.1796 (5.8); 8.1439 (6.0); 8.1411 (8.7); 8.1380 (4.8); 8.0340 (7.8); 8.0332 (7.5); 8.0196 (7.0); 8.0187 (6.6); 6.0803 (0.6); 6.0687 (3.1); 6.0571 (4.9); 6.0455 (3.1); 6.0339 (0.6); 5.7525 (0.5); 3.3135 (49.6); 3.3065 (301.9); 3.2833 (0.5); 2.6188 (0.8); 2.6158 (1.9); 2.6127 (2.7); 2.6097 (2.0); 2.6066 (0.9); 2.5368 (0.3); 2.5218 (6.1); 2.5187 (7.5); 2.5156 (7.3); 2.5068 (142.8); 2.5038 (303.6); 2.5007 (419.9); 2.4977 (305.6); 2.4947 (142.9); 2.3907 (0.8); 2.3877 (1.9); 2.3847 (2.6); 2.3817 (1.8); 2.3786 (0.8); 2.0724 (2.9); 1.9069 (0.5); 1.7297 (15.9); 1.7180 (16.0); 0.0053 (0.7); -0.0001 (22.8); -0.0057 (0.7) | 518.0 [M+H]⁺ |
| I-040 | 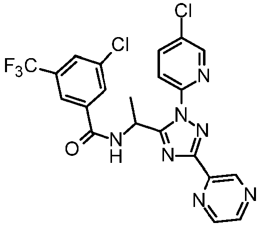 (reference compound) | ¹H-NMR(600 MHz, d₆-DMSO, ppm): δ = 9.4996 (3.9); 9.4881 (4.0); 9.3231 (9.2); 9.3207 (9.3); 8.7846 (4.8); 8.7820 (5.5); 8.7805 (7.1); 8.7780 (6.6); 8.7597 (11.0); 8.7556 (7.9); 8.6700 (6.6); 8.6692 (6.5); 8.6657 (6.9); 8.6650 (6.4); 8.2641 (5.1); 8.2598 (4.9); 8.2496 (5.7); 8.2453 (5.7); 8.1487 (6.6); 8.0893 (6.5); 8.0629 (5.9); 8.0255 (7.6); 8.0248 (7.3); 8.0110 (6.8); 8.0103 (6.5); 6.0673 (0.6); 6.0557 (2.9); 6.0441 (4.5); 6.0325 (2.9); 6.0209 (0.6); 4.0942 (1.0); 4.0854 (3.0); 4.0767 (3.1); 4.0679 (1.0); 3.3133 (44.2); 3.1785 (12.5); 3.1698 (12.3); 2.6174 (0.4); 2.5264 (0.8); 2.5234 (1.0); 2.5203 (1.0); 2.5115 (22.9); 2.5085 (49.5); 2.5054 (69.1); 2.5024 (50.6); 2.4993 (23.8); 2.3894 (0.4); 1.7313 (15.9); 1.7197 (16.0); -0.0001 (5.9). | 508.0 [M+H]⁺ |
| I-041 | 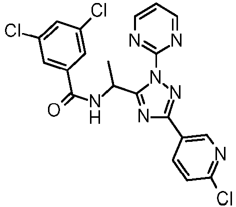 (reference compound) | ¹H-NMR(600.4 MHz, d₆-DMSO): | |
| | | δ= 10.8694 (1.2); 9.3294 (3.1); 9.3179 (3.2); 9.0421 (4.8); 9.0383 (4.8); 9.0232 (10.7); 9.0151 (10.7); 8.4415 (3.0); 8.4375 (2.9); 8.4276 (3.1); 8.4236 (3.0); 7.9216 (9.7); 7.9021 (3.6); 7.7922 (7.7); 7.7882 (16.0); 7.6897 (4.8); 7.6759 (7.6); 7.6678 (5.7); 7.6597 (2.9); 6.0483 (0.5); 6.0370 (2.1); 6.0254 (3.2); 6.0139 (2.1); 6.0022 (0.5); 3.3072 (209.0); 2.6129 (2.3); 2.5214 (8.2); 2.5185 (10.6); 2.5035 (296.2); 2.5008 (368.9); 2.4983 (283.9); 2.3849 (2.4); 1.7112 (11.3); 1.6996 (11.3); -0.0001 (19.7) | 474.0 [M+H]⁺ |
| I-042 | 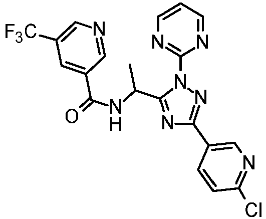 (reference compound) | ¹H-NMR(600 MHz, d₆-DMSO, ppm): δ = 9.5688 (3.8); 9.5572 (3.9); 9.1772 (5.7); 9.1745 (5.7); 9.1204 (5.1); 9.1186 (5.0); 9.0615 (0.7); 9.0533 (1.0); 9.0492 (5.9); 9.0453 (6.0); 9.0335 (15.8); 9.0254 (16.0); 9.0145 (1.2); 9.0063 (0.4); 8.8889 (0.8); 8.8853 (0.7); 8.8632 (0.8); 8.5754 (1.1); 8.5674 (1.1); 8.5018 (5.6); 8.4485 (4.1); 8.4444 (4.0); 8.4346 (4.2); 8.4305 (4.2); 8.2872 (0.4); 8.2836 (0.4); 8.2733 (0.4); 8.2697 (0.4); 8.1318 (0.7); 7.7118 (0.4); 7.7040 (0.4); 7.6927 (6.5); 7.6790 (10.2); 7.6711 (8.4); 7.6631 (4.3); 7.6502 (0.4); 7.2432 (0.3); 6.1141 (0.6); 6.1026 (2.7); 6.0910 (4.2); 6.0793 (2.7); 6.0678 (0.6); 3.3190 (89.5); 3.1739 (0.3); 3.1652 (0.3); 2.6175 (1.0); 2.6146 (1.3); 2.6116 (1.0); 2.5235 (3.1); 2.5205 (3.9); 2.5173 (4.2); 2.5055 (151.3); 2.5025 (204.0); 2.4996 (151.3); 2.3893 (0.9); 2.3865 (1.3); 2.3836 (0.9); 1.8739 (0.9); 1.8623 (0.9); 1.7460 (14.7); 1.7344 (14.6); 1.7203 (0.5); 1.7088 (0.4); 1.6791 (0.6); 1.6675 (0.5); 1.2327 (0.4); 1.1043 (0.4); 1.0924 (0.8); 1.0806 (0.4); 0.0053 (0.5); -0.0001 (13.6); -0.0056 (0.5). | 475.1 [M+H]⁺ |
| I-043 | 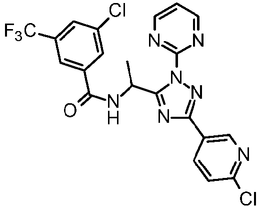 (reference compound) | ¹H-NMR(600 MHz, d₆-DMSO, ppm): δ = 9.4797 (3.2); 9.4681 (3.3); 9.0449 (4.9); 9.0440 (4.9); 9.0410 (5.1); 9.0400 (4.7); 9.0254 (15.7); 9.0173 (16.0); 8.4435 (4.0); 8.4395 (3.8); 8.4297 (4.2); 8.4256 (4.2); 8.1258 (5.2); 8.0636 (5.6); 8.0604 (5.1); 8.0568 (4.8); 7.9550 (1.3); 7.6896 (5.4); 7.6888 (5.2); 7.6756 (5.9); 7.6744 (7.4); 7.6658 (8.1); 7.6578 (4.1); 6.0832 (0.5); 6.0717 (2.4); 6.0600 (3.8); 6.0484 (2.4); 6.0368 (0.5); 3.3125 (70.5); 2.8926 (10.8); 2.7338 (8.8); 2.6915 (0.4); 2.6185 (0.4); 2.6156 (0.6); 2.6124 (0.4); 2.5246 (1.1); 2.5215 (1.3); 2.5184 (1.3); 2.5096 (30.6); 2.5066 (64.4); 2.5035 (88.2); 2.5004 (64.0); 2.4975 (29.9); 2.3905 (0.4); 2.3875 (0.5); 2.3844 (0.4); 1.7349 (12.6); 1.7233 (12.6); 1.2726 (0.5); 1.2591 (0.7); 1.2478 (0.6); -0.0001 (6.0). | 508.1 [M+H]⁺ |
| I-044 | 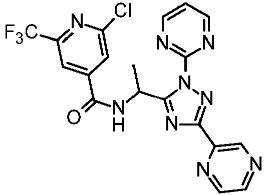 (reference compound) | ¹H-NMR(600.4 MHz, d₆-DMSO): | 476.2 [M+H]⁺ |
| | | δ= 9.7602 (2.7); 9.7485 (2.8); 9.3139 (6.7); 9.3115 (6.4); 9.0498 (15.8); 9.0417 (16.0); 8.7944 (3.9); 8.7919 (4.3); 8.7903 (5.2); 8.7878 (4.8); 8.7655 (7.8); 8.7614 (5.8); 8.2052 (6.7); 8.2034 (6.7); 8.1567 (6.4); 7.7012 (4.3); 7.6931 (8.1); 7.6851 (4.1); 6.1317 (0.5); 6.1201 (2.2); 6.1085 (3.5); 6.0969 (2.2); 6.0853 (0.5); 3.3071 (175.5); 3.2940 (0.6); 2.6193 (0.6); 2.6161 (1.2); 2.6131 (1.6); 2.6101 (1.1); 2.6070 (0.5); 2.5222 (4.4); 2.5191 (5.8); 2.5159 (6.8); 2.5072 (96.9); 2.5042 (195.0); 2.5011 (262.8); 2.4980 (189.0); 2.4950 (87.6); 2.3911 (0.6); 2.3881 (1.2); 2.3851 (1.6); 2.3820 (1.1); 2.3790 (0.5); 1.7520 (11.7); 1.7404 (11.7); 0.0053 (0.7); -0.0001 (19.6); -0.0057 (0.6) | |
| I-045 | 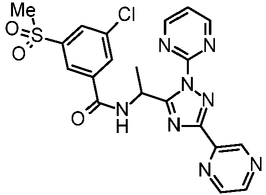 (reference compound) | ¹H-NMR(600.4 MHz, d₆-DMSO): | 485.1 [M+H]⁺ |
| | | δ= 9.5659 (2.9); 9.5541 (3.0); 9.3095 (6.7); 9.3070 (6.6); 9.0468 (15.8); 9.0387 (16.0); 8.7894 (3.9); 8.7869 (4.4); 8.7853 (5.2); 8.7828 (4.8); 8.7597 (7.8); 8.7556 (5.9); 8.2604 (4.1); 8.2579 (7.4); 8.2553 (4.1); 8.1705 (3.9); 8.1675 (5.8); 8.1648 (4.1); 8.1355 (4.4); 8.1326 (6.1); 8.1295 (3.3); 7.6951 (4.2); 7.6870 (8.1); 7.6789 (4.1); 6.1142 (0.5); 6.1026 (2.2); 6.0910 (3.4); 6.0793 (2.2); 6.0676 (0.4); 3.3061 (207.5); 2.6188 (0.7); 2.6157 (1.4); 2.6126 (2.0); 2.6096 (1.4); 2.6065 (0.7); 2.5217 (5.4); 2.5186 (7.0); 2.5154 (8.1); 2.5068 (117.5); 2.5037 (235.4); 2.5006 (316.2); 2.4976 (226.8); 2.4946 (104.6); 2.3907 (0.7); 2.3876 (1.5); 2.3846 (2.0); 2.3816 (1.4); 2.3785 (0.6); 2.0853 (0.5); 2.0723 (0.6); 1.7481 (11.5); 1.7365 (11.5); 0.0053 (0.9); -0.0001 (23.0); -0.0057 (0.7) | |
| I-046 | 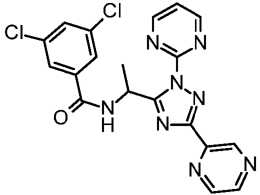 (reference compound) | ¹H-NMR(600.4 MHz, d₆-DMSO): | 441.1 [M+H]⁺ |
| | | δ= 9.3708 (1.4); 9.3591 (1.5); 9.3048 (3.4); 9.3023 (3.3); 9.0413 (7.7); 9.0332 (7.8); 8.7901 (1.9); 8.7875 (2.2); 8.7860 (2.6); 8.7834 (2.4); 8.7599 (3.9); 8.7557 (3.0); 7.7997 (16.0); 7.6934 (2.1); 7.6853 (4.0); 7.6772 (2.0); 6.0565 (1.1); 6.0448 (1.8); 6.0332 (1.1); 3.3067 (94.6); 2.6190 (0.3); 2.6159 (0.7); 2.6129 (0.9); 2.6098 (0.6); 2.5219 (2.6); 2.5188 (3.3); 2.5157 (3.8); 2.5069 (57.1); 2.5039 (114.4); 2.5009 (153.9); 2.4978 (110.8); 2.4948 (51.6); 2.3910 (0.4); 2.3879 (0.7); 2.3848 (1.0); 2.3817 (0.7); 1.7225 (6.0); 1.7109 (6.0); 0.0053 (0.4); -0.0001 (9.3) | |
| I-047 | 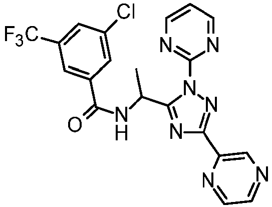 (reference compound) | ¹H-NMR(600 MHz, d₆-DMSO, ppm): δ = 9.5221 (3.0); 9.5104 (3.1); 9.3068 (6.8); 9.3044 (6.9); 9.0429 (15.7); 9.0348 (16.0); 8.7893 (3.7); 8.7867 (4.3); 8.7852 (5.3); 8.7826 (4.9); 8.7598 (8.1); 8.7557 (6.1); 8.1378 (5.1); 8.0811 (5.1); 8.0592 (4.5); 7.6911 (4.2); 7.6831 (8.1); 7.6750 (4.1); 6.1025 (0.5); 6.0909 (2.3); 6.0793 (3.7); 6.0676 (2.4); 6.0560 (0.5); 3.3078 (107.1); 2.6168 (0.6); 2.6138 (0.9); 2.6108 (0.6); 2.5229 (1.8); 2.5198 (2.2); 2.5167 (2.0); 2.5079 (49.1); 2.5049 (106.0); 2.5018 (148.2); 2.4987 (108.5); 2.4957 (51.0); 2.3888 (0.6); 2.3858 (0.9); 2.3827 (0.6); 1.7449 (12.5); 1.7333 (12.6); 0.0053 (0.4); -0.0001 (14.2); -0.0057 (0.4). | 475.1 [M+H]⁺ |
| I-048 | 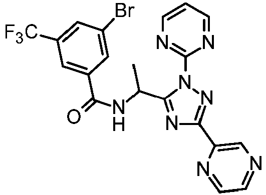 (reference compound) | ¹H-NMR(600.4 MHz, d₆-DMSO): | 521.1 [M+H]⁺ |
| | | δ= 9.5179 (2.7); 9.5062 (2.7); 9.3057 (6.4); 9.3032 (6.3); 9.0415 (15.8); 9.0334 (16.0); 8.7887 (3.8); 8.7861 (4.2); 8.7846 (5.1); 8.7820 (4.8); 8.7592 (7.5); 8.7551 (5.7); 8.2704 (4.6); 8.1623 (4.1); 8.1093 (4.4); 7.6903 (4.2); 7.6822 (8.0); 7.6741 (4.1); 6.0979 (0.4); 6.0864 (2.1); 6.0748 (3.3); 6.0631 (2.1); 6.0515 (0.4); 5.7524 (0.6); 3.3061 (350.3); 3.2827 (0.5); 2.6186 (1.0); 2.6156 (2.2); 2.6126 (2.9); 2.6095 (2.0); 2.6064 (0.9); 2.5369 (0.5); 2.5216 (8.1); 2.5185 (10.6); 2.5154 (12.4); 2.5067 (174.2); 2.5037 (352.2); 2.5006 (476.3); 2.4975 (341.8); 2.4945 (156.8); 2.3907 (1.1); 2.3876 (2.2); 2.3845 (3.0); 2.3815 (2.0); 2.3784 (0.9); 1.7416 (11.1); 1.7300 (11.1); 0.0053 (1.0); -0.0001 (27.0); -0.0057 (0.8) | |
| I-049 | 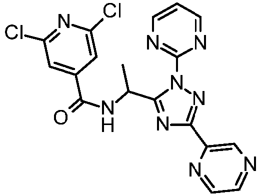 (reference compound) | ¹H-NMR(600.4 MHz, d₆-DMSO): | 442.1 [M]⁺ |
| | | δ= 9.6129 (1.3); 9.6013 (1.3); 9.3124 (3.3); 9.3099 (3.2); 9.0461 (7.9); 9.0380 (8.0); 8.7959 (2.0); 8.7933 (2.1); 8.7918 (2.6); 8.7892 (2.4); 8.7658 (3.8); 8.7617 (2.9); 7.8404 (16.0); 7.7009 (2.2); 7.6928 (4.1); 7.6847 (2.1); 6.0789 (1.0); 6.0673 (1.6); 6.0557 (1.0); 3.3066 (89.0); 2.6159 (0.6); 2.6128 (0.8); 2.6098 (0.6); 2.5219 (2.4); 2.5188 (3.1); 2.5156 (3.6); 2.5070 (50.8); 2.5039 (102.6); 2.5008 (138.9); 2.4978 (99.9); 2.4947 (46.2); 2.3910 (0.3); 2.3879 (0.6); 2.3848 (0.9); 2.3818 (0.6); 1.7286 (5.9); 1.7170 (5.9); 0.0053 (0.4); -0.0001 (10.1) | |
| I-050 | 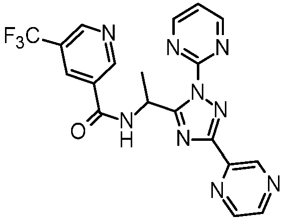 (reference compound) | ¹H-NMR(600.4 MHz, d₆-DMSO): | 442.2 [M+H]⁺ |
| | | δ= 9.6025 (2.8); 9.5908 (2.8); 9.3115 (6.7); 9.3091 (6.5); 9.1850 (4.4); 9.1819 (4.3); 9.1185 (3.8); 9.1164 (3.7); 9.0492 (15.8); 9.0411 (16.0); 8.7899 (3.9); 8.7873 (4.3); 8.7857 (5.2); 8.7832 (4.8); 8.7606 (7.8); 8.7565 (5.8); 8.5188 (4.2); 8.5155 (2.4); 7.6945 (4.3); 7.6864 (8.2); 7.6783 (4.2); 6.1340 (0.5); 6.1225 (2.3); 6.1109 (3.6); 6.0992 (2.3); 6.0876 (0.5); 3.3071 (172.8); 2.6192 (0.6); 2.6162 (1.2); 2.6131 (1.6); 2.6101 (1.1); 2.6071 (0.5); 2.5222 (4.3); 2.5191 (5.7); 2.5159 (6.7); 2.5072 (91.7); 2.5042 (184.2); 2.5011 (248.2); 2.4981 (178.7); 2.4950 (83.2); 2.3912 (0.5); 2.3881 (1.1); 2.3851 (1.5); 2.3820 (1.0); 2.3790 (0.5); 1.7576 (12.1); 1.7460 (12.1); 0.0053 (0.8); -0.0001 (21.7); -0.0056 (0.7) | |
| I-051 | 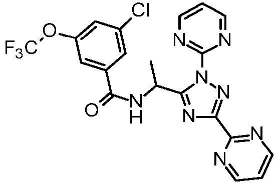 (reference compound) | ¹H-NMR(400.2 MHz, d₆-DMSO): | 491.1 [M+H]⁺ |
| | | δ= 9.4763 (2.2); 9.4586 (2.3); 9.0391 (11.0); 9.0270 (11.3); 8.9675 (10.3); 8.9553 (10.6); 7.9485 (3.1); 7.9448 (4.9); 7.9410 (3.2); 7.7752 (3.5); 7.7243 (3.5); 7.6900 (2.9); 7.6778 (5.5); 7.6657 (2.8); 7.6177 (2.8); 7.6056 (5.4); 7.5934 (2.7); 6.0943 (0.4); 6.0771 (1.6); 6.0596 (2.6); 6.0422 (1.7); 6.0246 (0.4); 5.7581 (16.0); 3.3321 (117.6); 3.1764 (0.9); 3.1633 (0.8); 2.6773 (0.7); 2.6728 (0.9); 2.6684 (0.7); 2.5262 (3.2); 2.5125 (62.4); 2.5084 (122.0); 2.5039 (156.2); 2.4994 (112.7); 2.4951 (55.3); 2.3353 (0.6); 2.3307 (0.9); 2.3262 (0.6); 1.7285 (9.0); 1.7111 (9.0); -0.0002 (2.1) | |
| I-052 | 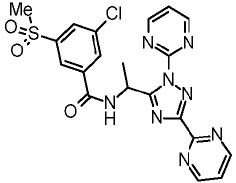 (reference compound) | ¹H-NMR(600.4 MHz, d₆-DMSO): | 485.1 [M+H]⁺ |
| | | δ= 9.5846 (3.0); 9.5727 (3.1); 9.0412 (15.6); 9.0331 (15.9); 8.9624 (15.7); 8.9543 (16.0); 8.2773 (3.9); 8.2748 (7.3); 8.2722 (4.2); 8.1829 (3.8); 8.1800 (5.9); 8.1772 (4.0); 8.1339 (4.2); 8.1310 (6.2); 8.1280 (3.5); 7.6885 (4.2); 7.6804 (8.0); 7.6723 (4.1); 7.6091 (4.2); 7.6010 (8.1); 7.5929 (4.1); 6.1167 (0.5); 6.1052 (2.2); 6.0934 (3.4); 6.0817 (2.2); 6.0701 (0.5); 3.3076 (274.6); 3.2841 (0.5); 2.6188 (0.6); 2.6158 (1.3); 2.6128 (1.8); 2.6097 (1.3); 2.6067 (0.6); 2.5218 (4.2); 2.5187 (5.1); 2.5156 (5.0); 2.5068 (99.5); 2.5038 (210.4); 2.5007 (290.4); 2.4977 (211.8); 2.4947 (99.9); 2.3908 (0.6); 2.3878 (1.3); 2.3847 (1.8); 2.3817 (1.3); 2.3786 (0.6); 1.7378 (11.5); 1.7262 (11.6); 0.0053 (0.9); -0.0001 (29.6); -0.0057 (0.9) | |
| I-053 | 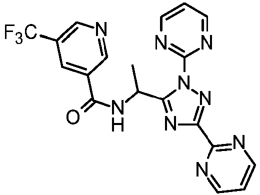 (reference compound) | ¹H-NMR(400.2 MHz, d₆-DMSO): | 442.2 [M+H]⁺ |
| | | δ= 14.7997 (0.5); 9.6442 (1.8); 9.6263 (1.9); 9.1939 (3.0); 9.1895 (3.1); 9.1230 (2.8); 9.1200 (2.8); 9.0487 (10.9); 9.0365 (11.2); 8.9987 (0.8); 8.9674 (11.0); 8.9552 (11.7); 8.9392 (1.2); 8.8744 (0.4); 8.8300 (0.6); 8.5421 (3.4); 8.5270 (1.1); 8.1565 (0.6); 7.6954 (2.8); 7.6833 (5.4); 7.6711 (2.8); 7.6179 (2.9); 7.6057 (5.6); 7.5935 (3.1); 7.1857 (0.5); 6.2713 (0.4); 6.2532 (0.4); 6.1328 (1.4); 6.1153 (2.2); 6.0977 (1.4); 5.7577 (16.0); 3.3342 (177.3); 2.8921 (0.6); 2.7332 (0.6); 2.7319 (0.6); 2.6904 (0.5); 2.6816 (0.4); 2.6775 (0.7); 2.6729 (1.0); 2.6684 (0.7); 2.5264 (3.3); 2.5216 (5.3); 2.5129 (63.3); 2.5085 (126.5); 2.5040 (163.0); 2.4994 (116.1); 2.4949 (55.2); 2.3398 (0.3); 2.3353 (0.7); 2.3307 (1.0); 2.3262 (0.7); 2.3217 (0.3); 1.9270 (1.2); 1.9096 (1.3); 1.7525 (7.6); 1.7351 (7.5); 1.2750 (0.6); 1.2593 (1.3); 1.2440 (0.9); -0.0002 (2.7) | |
| I-054 | 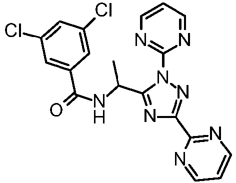 (reference compound) | ¹H-NMR(400.2 MHz, d₆-DMSO): | 441.1 [M+H]⁺ |
| | | δ= 9.4076 (2.2); 9.3898 (2.3); 9.0412 (12.4); 9.0290 (12.8); 8.9680 (11.9); 8.9558 (12.2); 7.8195 (7.6); 7.8152 (16.0); 7.8075 (6.0); 7.8035 (4.2); 7.7983 (1.8); 7.6940 (3.3); 7.6819 (6.2); 7.6697 (3.2); 7.6177 (3.2); 7.6055 (6.2); 7.5934 (3.2); 6.0832 (0.3); 6.0656 (1.6); 6.0481 (2.6); 6.0306 (1.7); 6.0132 (0.4); 5.7579 (8.0); 3.3339 (164.0); 2.6773 (0.6); 2.6728 (0.8); 2.6684 (0.6); 2.5263 (2.8); 2.5127 (54.9); 2.5084 (108.5); 2.5039 (139.6); 2.4993 (99.8); 2.4948 (47.9); 2.3352 (0.6); 2.3307 (0.8); 2.3261 (0.6); 1.7162 (9.0); 1.6988 (8.9); -0.0002 (1.9) | |
| I-055 | 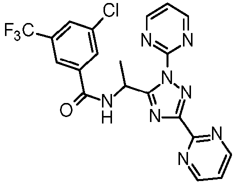 (reference compound) | ¹H-NMR(400.2 MHz, d₆-DMSO): | 475.3 [M+H]⁺ |
| | | δ= 9.5624 (2.3); 9.5446 (2.4); 9.0427 (13.4); 9.0305 (13.7); 8.9665 (13.1); 8.9543 (13.5); 8.1522 (4.3); 8.1032 (4.2); 8.0661 (3.9); 7.6921 (3.5); 7.6800 (6.6); 7.6678 (3.4); 7.6169 (3.5); 7.6047 (6.6); 7.5926 (3.4); 7.2842 (0.7); 7.2616 (0.7); 6.1178 (0.4); 6.1006 (1.7); 6.0830 (2.8); 6.0655 (1.8); 6.0485 (0.4); 5.7581 (16.0); 3.3330 (154.7); 3.1765 (0.6); 3.1634 (0.6); 2.6819 (0.3); 2.6775 (0.7); 2.6730 (0.9); 2.6684 (0.7); 2.6639 (0.3); 2.5265 (3.2); 2.5216 (5.2); 2.5130 (61.2); 2.5086 (122.8); 2.5040 (158.8); 2.4994 (113.4); 2.4950 (54.3); 2.3353 (0.6); 2.3308 (0.9); 2.3264 (0.7); 1.7382 (9.4); 1.7208 (9.4); 1.6497 (0.9); -0.0002 (2.0) | |
| I-056 | 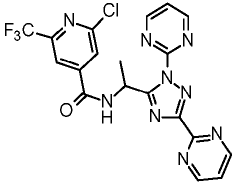 (reference compound) | ¹H-NMR(400.2 MHz, d₆-DMSO): | 476.3 [M+H]⁺ |
| | | δ= 9.8013 (2.4); 9.7837 (2.4); 9.0483 (11.7); 9.0362 (12.0); 8.9721 (10.0); 8.9599 (10.3); 8.2264 (6.0); 8.2243 (6.1); 8.1688 (6.1); 8.1406 (0.4); 7.7013 (3.1); 7.6891 (5.9); 7.6770 (3.0); 7.6231 (2.8); 7.6109 (5.3); 7.5987 (2.7); 6.1491 (0.4); 6.1320 (1.7); 6.1146 (2.7); 6.0970 (1.7); 6.0800 (0.4); 5.7573 (16.0); 3.3330 (120.1); 3.1760 (0.6); 3.1636 (0.5); 2.6774 (0.8); 2.6728 (1.1); 2.6684 (0.8); 2.5260 (3.8); 2.5124 (74.1); 2.5084 (141.2); 2.5039 (178.0); 2.4994 (129.6); 2.4954 (64.5); 2.3351 (0.8); 2.3307 (1.0); 2.3262 (0.8); 1.7472 (9.4); 1.7298 (9.4); -0.0002 (2.3) | |
| I-057 | 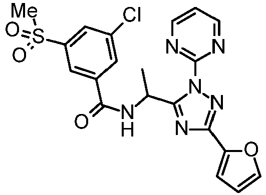 (reference compound) | ¹H-NMR(400.2 MHz, d₆-DMSO): | 473.3 [M+H]⁺ |
| | | δ= 9.5528 (2.2); 9.5353 (2.2); 9.0122 (10.2); 9.0001 (10.5); 8.2737 (3.0); 8.2701 (5.4); 8.2665 (3.3); 8.1781 (2.8); 8.1737 (4.8); 8.1698 (3.2); 8.1416 (3.4); 8.1374 (4.8); 8.1331 (2.5); 7.8677 (4.4); 7.8652 (4.3); 7.6563 (2.7); 7.6442 (5.1); 7.6320 (2.6); 7.0594 (3.8); 7.0522 (3.8); 7.0509 (3.8); 6.6757 (3.1); 6.6713 (3.2); 6.6673 (3.1); 6.6628 (2.9); 6.0904 (1.5); 6.0730 (2.4); 6.0555 (1.6); 6.0379 (0.3); 5.7570 (16.0); 4.1054 (0.4); 4.0922 (0.4); 3.3958 (1.0); 3.3308 (107.7); 3.3144 (23.8); 3.1760 (2.1); 3.1629 (2.1); 2.6767 (0.6); 2.6723 (0.8); 2.6676 (0.6); 2.5253 (2.9); 2.5116 (57.9); 2.5077 (110.0); 2.5032 (138.7); 2.4987 (99.9); 2.4946 (49.0); 2.3345 (0.6); 2.3301 (0.8); 2.3255 (0.6); 1.7087 (8.4); 1.6913 (8.4); 1.2591 (0.5); 1.2504 (0.5); 1.2427 (0.4); 1.2340 (0.5); -0.0002 (1.5) | |
| I-058 | 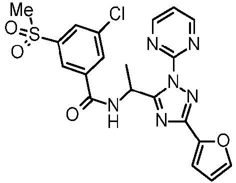 (reference compound) | ¹H-NMR(400.2 MHz, d₆-DMSO): | 463.3 [M+H]⁺ |
| | | δ= 9.5082 (2.2); 9.4905 (2.3); 9.0074 (11.5); 8.9953 (11.9); 8.1440 (4.2); 8.0926 (4.2); 8.0660 (3.8); 7.8667 (4.6); 7.8642 (4.6); 7.8626 (4.2); 7.6516 (3.0); 7.6394 (5.8); 7.6273 (3.0); 7.0558 (3.8); 7.0543 (4.1); 7.0474 (4.0); 7.0459 (4.1); 6.6753 (3.4); 6.6708 (3.5); 6.6668 (3.4); 6.6624 (3.4); 6.0945 (0.3); 6.0773 (1.7); 6.0599 (2.7); 6.0424 (1.7); 6.0251 (0.4); 5.7576 (16.0); 3.3312 (113.7); 3.1764 (0.6); 3.1633 (0.6); 2.6772 (0.7); 2.6727 (0.9); 2.6682 (0.7); 2.5260 (3.0); 2.5125 (58.3); 2.5082 (116.2); 2.5037 (150.5); 2.4992 (109.0); 2.4948 (53.4); 2.3350 (0.6); 2.3306 (0.9); 2.3260 (0.6); 1.7050 (9.3); 1.6876 (9.2); -0.0002 (1.9) | |
| I-059 | 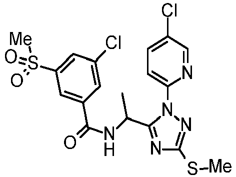 (reference compound) | ¹H-NMR (600 MHz, d₆-DMSO, ppm): δ = 9.4380 (1.2); 9.4263 (1.2); 8.6161 (2.0); 8.6154 (2.1); 8.6119 (2.0); 8.6110 (2.0); 8.2799 (1.7); 8.2773 (3.1); 8.2748 (1.8); 8.1961 (1.6); 8.1917 (1.6); 8.1815 (1.8); 8.1772 (1.9); 8.1744 (1.6); 8.1713 (2.6); 8.1686 (1.8); 8.1438 (1.8); 8.1409 (2.6); 8.1378 (1.4); 7.8733 (2.2); 7.8725 (2.3); 7.8588 (2.1); 7.8579 (2.1); 5.9879 (0.9); 5.9762 (1.5); 5.9646 (1.0); 3.3190 (13.8); 3.3097 (12.0); 2.5930 (16.0); 2.5176 (0.4); 2.5089 (6.0); 2.5059 (12.3); 2.5029 (16.8); 2.4998 (12.1); 2.4968 (5.6); 1.6275 (4.9); 1.6159 (4.9). | 486.1 [M+H]⁺ |
| I-060 | 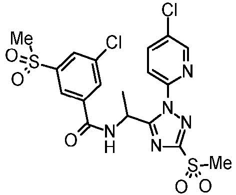 (reference compound) | ¹H-NMR (600 MHz, d₆-DMSO, ppm): δ = 9.5344 (4.3); 9.5228 (4.5); 8.6914 (7.0); 8.6872 (7.1); 8.2840 (4.8); 8.2798 (4.6); 8.2696 (5.2); 8.2653 (5.2); 8.2415 (5.1); 8.2390 (9.7); 8.2365 (5.7); 8.1543 (4.5); 8.1514 (8.5); 8.1485 (5.3); 8.1282 (5.9); 8.1254 (8.4); 8.1225 (4.6); 7.9406 (7.8); 7.9261 (7.2); 5.9662 (0.6); 5.9546 (3.0); 5.9431 (4.8); 5.9315 (3.1); 5.9198 (0.6); 3.4531 (53.6); 3.3350 (0.4); 3.3177 (56.9); 2.6173 (0.4); 2.6143 (0.6); 2.6113 (0.4); 2.5233 (1.4); 2.5202 (1.7); 2.5171 (1.7); 2.5082 (30.5); 2.5053 (64.3); 2.5022 (89.9); 2.4992 (67.4); 2.4963 (32.2); 2.3892 (0.4); 2.3862 (0.6); 2.3831 (0.4); 2.0752 (0.4); 1.6834 (15.9); 1.6718 (16.0); 0.0053 (0.4); -0.0001 (12.6); - 0.0056 (0.4). | 518.1 [M+H]⁺ |
| I-061 | 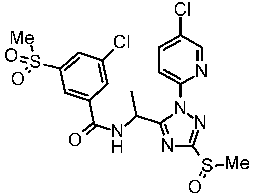 (reference compound) | ¹H-NMR(400.2 MHz, d₆-DMSO): | 502.0 [M+H]⁺ |
| | | δ= 9.5539 (2.0); 9.5367 (2.0); 8.6869 (2.5); 8.6816 (4.3); 8.6763 (2.5); 8.2731 (2.1); 8.2669 (2.1); 8.2515 (7.3); 8.1518 (5.6); 8.1470 (6.8); 8.1425 (5.1); 7.9423 (4.6); 7.9205 (4.2); 7.8722 (0.8); 7.6144 (0.4); 7.5968 (0.6); 7.4913 (0.5); 5.9862 (1.0); 5.9812 (1.1); 5.9688 (1.6); 5.9639 (1.6); 5.9513 (1.1); 5.9465 (1.0); 3.3639 (44.1); 3.3205 (32.7); 3.1450 (0.6); 3.0496 (16.0); 3.0402 (15.3); 2.9984 (1.5); 2.7152 (0.3); 2.6791 (0.7); 2.6749 (0.9); 2.6706 (0.6); 2.5452 (67.8); 2.5280 (2.9); 2.5103 (112.0); 2.5059 (141.5); 2.5015 (102.5); 2.3370 (0.6); 2.3327 (0.8); 2.3284 (0.6); 1.6903 (5.7); 1.6853 (5.7); 1.6729 (5.9); 1.6680 (5.4); 1.2364 (0.5) | |
| I-062 | 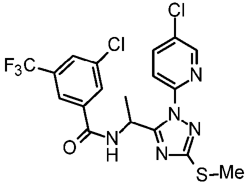 (reference compound) | ¹H-NMR(400.2 MHz, d₆-DMSO): | 476.0 [M+H]⁺ |
| | | δ= 9.4043 (1.2); 9.3869 (1.2); 8.6063 (2.3); 8.6010 (2.3); 8.5998 (2.2); 8.1961 (1.6); 8.1896 (1.5); 8.1742 (1.8); 8.1677 (1.8); 8.1387 (2.2); 8.0803 (2.4); 8.0746 (2.0); 8.0729 (2.0); 8.0696 (2.1); 7.8703 (2.4); 7.8693 (2.4); 7.8485 (2.1); 7.8474 (2.1); 5.9740 (0.9); 5.9565 (1.4); 5.9391 (0.9); 5.7574 (6.6); 3.3308 (63.4); 2.6770 (0.3); 2.6724 (0.4); 2.6680 (0.3); 2.5890 (16.0); 2.5259 (1.5); 2.5211 (2.4); 2.5124 (28.5); 2.5080 (57.0); 2.5035 (73.9); 2.4990 (53.2); 2.4945 (25.8); 2.3304 (0.4); 1.6256 (4.9); 1.6082 (4.9); - 0.0002 (0.9) | |
| I-063 | 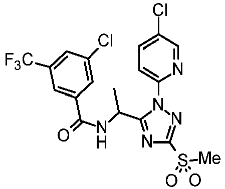 (reference compound) | ¹H-NMR(400.2 MHz, d₆-DMSO): | 508.1 [M+H]⁺ |
| | | δ= 9.4770 (3.0); 9.4598 (3.0); 8.6751 (5.7); 8.6698 (5.7); 8.2760 (4.0); 8.2696 (3.8); 8.2543 (4.5); 8.2479 (4.4); 8.0880 (5.5); 8.0844 (5.2); 8.0760 (5.2); 8.0242 (5.2); 7.9330 (5.9); 7.9123 (5.2); 7.9111 (5.3); 5.9608 (0.5); 5.9436 (2.2); 5.9263 (3.5); 5.9089 (2.2); 5.8912 (0.4); 5.7568 (16.0); 3.4507 (43.6); 3.3311 (186.8); 3.1758 (0.9); 3.1626 (0.9); 2.6769 (0.8); 2.6722 (1.2); 2.6676 (0.8); 2.5257 (4.2); 2.5122 (76.0); 2.5078 (149.6); 2.5032 (191.3); 2.4986 (135.1); 2.4941 (63.9); 2.3346 (0.8); 2.3300 (1.1); 2.3253 (0.8); 1.6830 (11.8); 1.6655 (11.8); -0.0002 (2.2) | |
| I-064 | 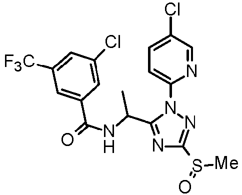 (reference compound) | ¹H-NMR(400.2 MHz, d₆-DMSO): | 492.0 [M+H]⁺ |
| | | δ= 9.4823 (2.2); 9.4652 (2.2); 8.6709 (2.5); 8.6644 (4.1); 8.6588 (2.2); 8.2610 (1.6); 8.2581 (1.8); 8.2548 (1.9); 8.2517 (1.6); 8.2393 (1.9); 8.2363 (2.0); 8.2329 (2.0); 8.2300 (1.8); 8.1018 (3.9); 8.0781 (4.0); 8.0396 (3.9); 8.0241 (0.4); 7.9294 (4.3); 7.9077 (3.9); 5.9684 (0.9); 5.9623 (1.0); 5.9510 (1.5); 5.9449 (1.6); 5.9337 (1.0); 5.9275 (1.0); 3.4522 (3.0); 3.3348 (156.4); 3.0464 (15.6); 3.0353 (16.0); 2.9971 (1.1); 2.6769 (0.6); 2.6724 (0.8); 2.6679 (0.6); 2.5429 (69.5); 2.5259 (2.5); 2.5212 (3.7); 2.5124 (48.4); 2.5080 (97.3); 2.5035 (125.8); 2.4990 (91.4); 2.4946 (45.1); 2.3349 (0.5); 2.3303 (0.7); 2.3259 (0.5); 1.6857 (5.6); 1.6809 (6.0); 1.6683 (5.8); 1.6635 (5.8); 1.2379 (0.4); -0.0002 (0.6) | |
| I-065 | 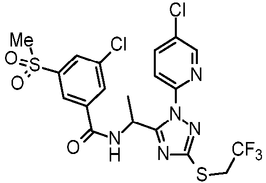 (reference compound) | ¹H-NMR(400.2 MHz, d₆-DMSO): | 554.0 [M+H]⁺ |
| | | δ= 9.4734 (2.4); 9.4563 (2.5); 8.6378 (4.4); 8.6367 (4.6); 8.6315 (4.7); 8.6302 (4.5); 8.3162 (0.4); 8.2623 (3.5); 8.2585 (6.4); 8.2547 (3.8); 8.2302 (3.3); 8.2238 (3.1); 8.2084 (3.7); 8.2019 (3.6); 8.1506 (13.0); 8.1468 (11.9); 7.8961 (4.7); 7.8950 (4.7); 7.8744 (4.2); 7.8731 (4.3); 5.9911 (0.4); 5.9735 (1.7); 5.9562 (2.7); 5.9388 (1.8); 5.9212 (0.4); 5.7566 (16.0); 4.2483 (1.2); 4.2228 (4.0); 4.1972 (4.2); 4.1716 (1.4); 3.3301 (177.2); 3.3176 (29.2); 3.1756 (0.8); 3.1625 (0.7); 2.6764 (0.9); 2.6719 (1.2); 2.6673 (0.9); 2.5253 (4.0); 2.5205 (6.4); 2.5119 (77.9); 2.5075 (154.8); 2.5029 (199.2); 2.4983 (141.9); 2.4939 (68.2); 2.3387 (0.4); 2.3342 (0.8); 2.3297 (1.2); 2.3252 (0.8); 1.6378 (9.2); 1.6204 (9.2); -0.0002 (2.5) | |
| I-066 | 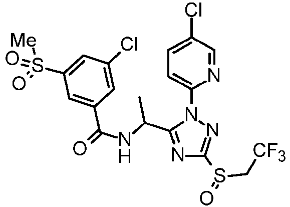 (reference compound) | ¹H-NMR(400.2 MHz, d₆-DMSO): | 570.0 [M+H]⁺ |
| | | δ= 9.5604 (2.8); 9.5434 (2.8); 8.7135 (3.0); 8.7071 (3.4); 8.6975 (2.5); 8.3156 (0.5); 8.3032 (1.8); 8.2980 (2.5); 8.2921 (2.0); 8.2815 (2.1); 8.2761 (2.8); 8.2704 (1.9); 8.2491 (6.0); 8.2459 (3.8); 8.2263 (0.6); 8.2227 (0.4); 8.1594 (3.1); 8.1550 (6.4); 8.1510 (4.6); 8.1426 (4.5); 8.1390 (5.3); 8.1118 (0.4); 8.1077 (0.5); 7.9809 (3.0); 7.9774 (3.0); 7.9591 (3.1); 7.9557 (2.8); 7.9373 (0.5); 5.9947 (1.1); 5.9872 (1.1); 5.9775 (1.7); 5.9700 (1.6); 5.9602 (1.2); 5.9529 (1.0); 5.9417 (0.4); 5.7563 (16.0); 5.2115 (0.5); 5.1875 (0.5); 4.6095 (0.6); 4.5932 (0.9); 4.5836 (2.0); 4.5808 (1.9); 4.5669 (2.1); 4.5625 (2.1); 4.5572 (2.2); 4.5537 (2.1); 4.5404 (1.9); 4.5357 (2.0); 4.5266 (0.9); 4.5138 (0.6); 4.5087 (0.6); 3.3299 (193.6); 3.3168 (31.2); 3.1752 (0.8); 3.1621 (0.8); 2.6759 (1.0); 2.6715 (1.3); 2.6670 (1.0); 2.5248 (4.5); 2.5112 (88.4); 2.5070 (171.1); 2.5026 (217.8); 2.4981 (157.4); 2.4937 (77.3); 2.3338 (0.9); 2.3294 (1.2); 2.3250 (0.9); 1.7035 (5.6); 1.6959 (6.6); 1.6861 (6.1); 1.6785 (6.3); -0.0002 (2.4) | |
| I-067 | 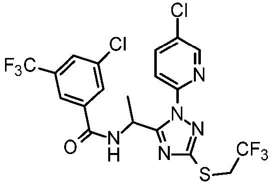 (reference compound) | ¹H-NMR(400.2 MHz, d₆-DMSO): | 544.1 [M+H]⁺ |
| | | δ= 9.4210 (3.0); 9.4038 (3.0); 8.6270 (5.7); 8.6208 (5.7); 8.2217 (3.4); 8.2153 (3.2); 8.1998 (3.8); 8.1934 (3.8); 8.1130 (5.6); 8.0716 (5.4); 8.0518 (5.6); 7.8875 (5.9); 7.8656 (5.2); 5.9721 (0.5); 5.9547 (2.2); 5.9374 (3.4); 5.9201 (2.2); 5.9032 (0.4); 5.7574 (16.0); 4.2420 (1.6); 4.2166 (5.0); 4.1910 (5.2); 4.1654 (1.8); 3.3302 (120.4); 3.1762 (0.8); 3.1631 (0.8); 2.6769 (0.9); 2.6725 (1.2); 2.6680 (0.9); 2.5256 (4.4); 2.5120 (81.7); 2.5080 (156.1); 2.5035 (197.8); 2.4990 (143.2); 2.4950 (71.0); 2.3347 (0.8); 2.3303 (1.2); 2.3259 (0.8); 1.6354 (12.1); 1.6179 (12.0); -0.0002 (2.1) | |
| I-068 | 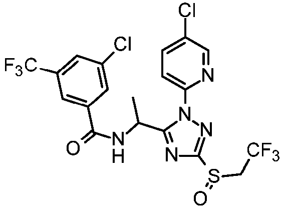 (reference compound) | ¹H-NMR(400.2 MHz, d₆-DMSO): | 560.0 [M+H]⁺ |
| | | δ= 9.5030 (1.4); 9.4973 (1.7); 9.4862 (1.6); 9.4803 (1.6); 8.7021 (2.6); 8.6957 (2.8); 8.6881 (2.1); 8.6829 (2.0); 8.6817 (2.0); 8.3024 (0.5); 8.2957 (0.7); 8.2925 (1.5); 8.2859 (2.7); 8.2795 (1.6); 8.2742 (0.9); 8.2707 (1.7); 8.2641 (3.0); 8.2576 (1.5); 8.1020 (3.8); 8.0783 (4.4); 8.0377 (3.8); 8.0044 (0.6); 7.9692 (2.7); 7.9676 (2.8); 7.9489 (2.8); 7.9477 (2.7); 7.9459 (2.5); 7.9284 (0.6); 5.9770 (0.9); 5.9707 (0.9); 5.9597 (1.4); 5.9534 (1.4); 5.9423 (1.0); 5.9361 (0.9); 5.9245 (0.5); 5.9074 (0.4); 5.7567 (16.0); 5.2062 (0.7); 5.1820 (0.7); 4.6081 (0.4); 4.6039 (0.4); 4.5820 (1.7); 4.5768 (1.4); 4.5568 (2.6); 4.5498 (1.6); 4.5305 (2.1); 4.5229 (0.6); 4.5036 (0.6); 3.3323 (207.2); 3.1758 (1.0); 3.1626 (1.0); 2.6767 (0.8); 2.6721 (1.0); 2.6676 (0.8); 2.5256 (3.6); 2.5120 (69.3); 2.5077 (136.2); 2.5032 (174.4); 2.4986 (125.8); 2.4942 (61.2); 2.3387 (0.4); 2.3345 (0.7); 2.3300 (1.0); 2.3255 (0.8); 1.7006 (4.8); 1.6941 (5.6); 1.6832 (5.1); 1.6766 (5.4); -0.0002 (1.9) | |
| I-069 | 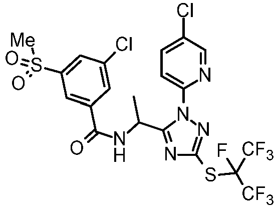 (reference compound) | ¹H-NMR(600.4 MHz, d₆-DMSO): | 640.0 [M+H]⁺ |
| | | δ= 9.5471 (3.9); 9.5360 (4.0); 8.7137 (6.9); 8.7128 (6.7); 8.7094 (7.1); 8.7085 (6.4); 8.2702 (5.5); 8.2659 (5.2); 8.2557 (5.9); 8.2514 (5.8); 8.2330 (5.6); 8.2305 (10.4); 8.2279 (5.9); 8.1553 (5.2); 8.1522 (8.5); 8.1493 (5.4); 8.1134 (6.2); 8.1107 (8.2); 8.1076 (5.0); 7.9013 (7.6); 7.9005 (7.1); 7.8868 (7.1); 7.8859 (6.7); 5.9141 (0.6); 5.9025 (2.8); 5.8911 (4.3); 5.8796 (2.8); 5.8680 (0.6); 3.3129 (51.5); 3.3063 (260.2); 3.2827 (0.5); 2.6187 (0.9); 2.6157 (1.8); 2.6126 (2.4); 2.6096 (1.7); 2.6066 (0.8); 2.5217 (6.6); 2.5186 (8.6); 2.5155 (10.2); 2.5068 (142.8); 2.5037 (289.0); 2.5007 (391.2); 2.4976 (281.8); 2.4946 (130.8); 2.3907 (0.9); 2.3877 (1.8); 2.3846 (2.4); 2.3815 (1.7); 2.3785 (0.8); 1.6686 (16.0); 1.6569 (15.9); 0.0053 (1.0); -0.0001 (25.8); -0.0057 (0.8) | |
| I-070 | 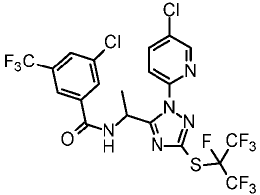 (reference compound) | ¹H-NMR(600.4 MHz, d₆-DMSO): | 630.0 [M+H]⁺ |
| | | δ= 9.4959 (3.8); 9.4848 (3.9); 8.7064 (6.6); 8.7056 (6.5); 8.7021 (6.8); 8.7013 (6.2); 8.2626 (5.1); 8.2583 (4.9); 8.2481 (5.5); 8.2438 (5.4); 8.0756 (11.5); 8.0107 (6.5); 7.8955 (7.3); 7.8810 (6.8); 7.8802 (6.5); 5.8963 (0.6); 5.8847 (2.8); 5.8733 (4.3); 5.8618 (2.8); 5.8502 (0.6); 3.3067 (340.4); 3.2825 (0.6); 2.6188 (1.0); 2.6158 (2.1); 2.6128 (2.9); 2.6097 (2.0); 2.6066 (0.9); 2.5218 (7.6); 2.5187 (9.8); 2.5156 (11.3); 2.5069 (171.1); 2.5038 (344.2); 2.5008 (463.6); 2.4977 (335.2); 2.4947 (156.6); 2.3908 (1.0); 2.3878 (2.1); 2.3847 (2.8); 2.3817 (2.0); 2.3787 (0.9); 1.6658 (16.0); 1.6541 (16.0); 0.0053 (1.2); -0.0001 (29.9); - 0.0057 (0.9) | |
| I-071 | 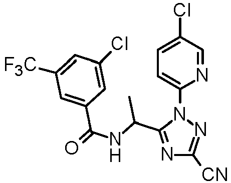 (reference compound) | ¹H-NMR (600 MHz, d₆-DMSO, ppm): δ = 9.4261 (3.4); 9.4150 (3.5); 8.6835 (6.2); 8.6827 (6.2); 8.6793 (6.6); 8.6784 (6.2); 8.2770 (5.2); 8.2727 (5.0); 8.2626 (5.6); 8.2583 (5.6); 8.0896 (6.2); 8.0872 (4.6); 8.0702 (5.6); 8.0248 (6.1); 7.9436 (7.3); 7.9427 (7.0); 7.9291 (6.7); 7.9282 (6.5); 5.9349 (0.6); 5.9234 (2.7); 5.9120 (4.2); 5.9005 (2.8); 5.8890 (0.6); 3.3106 (34.6); 2.6166 (0.3); 2.5256 (0.7); 2.5226 (0.8); 2.5195 (0.8); 2.5107 (18.0); 2.5077 (38.6); 2.5046 (53.6); 2.5016 (39.0); 2.4985 (18.3); 2.3886 (0.3); 2.0760 (0.6); 1.6647 (15.9); 1.6531 (16.0); -0.0001 (1.9). | 455.1 [M+H]⁺ |
| I-072 | 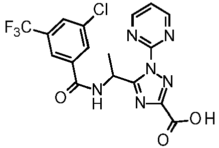 (reference compound) | ¹H-NMR(300 MHz, d₆-DMSO, ppm): δ = 9.4845 (2.0); 9.4608 (2.0); 9.0248 (15.6); 9.0085 (16.0); 8.1180 (5.2); 8.0638 (11.0); 7.6967 (3.8); 7.6804 (7.1); 7.6642 (3.6); 6.0407 (0.4); 6.0177 (1.7); 5.9942 (2.8); 5.9807 (1.0); 5.9707 (1.8); 5.9483 (0.3); 3.3143 (95.7); 2.7332 (1.0); 2.7270 (1.3); 2.7206 (0.8); 2.5130 (83.9); 2.5070 (162.9); 2.5009 (215.8); 2.4949 (146.0); 2.4890 (66.0); 2.2770 (0.9); 2.2708 (1.2); 2.2646 (0.9); 1.9088 (0.4); 1.6790 (10.6); 1.6558 (10.4); 1.2349 (1.9); 1.1662 (0.4); 1.1466 (0.4); 0.4842 (0.7); 0.0106 (1.0); -0.0003 (28.4); -0.0114 (0.9). | |
| I-073 | 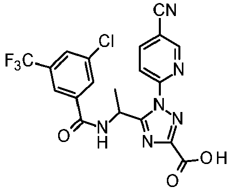 (reference compound) | ¹H-NMR (300 MHz, d₆-DMSO, ppm): δ = 9.5082 (4.2); 9.4854 (4.2); 9.0893 (8.4); 9.0842 (8.6); 8.6227 (6.5); 8.6154 (6.1); 8.5942 (7.1); 8.5868 (7.0); 8.1587 (7.8); 8.1021 (15.7); 8.0737 (15.5); 6.1150 (0.7); 6.0911 (2.6); 6.0681 (4.0); 6.0452 (2.6); 6.0221 (0.7); 3.8979 (0.4); 3.3145 (128.3); 2.7330 (1.4); 2.7272 (1.9); 2.7208 (1.4); 2.6550 (0.4); 2.5131 (120.7); 2.5071 (236.4); 2.5011 (314.8); 2.4951 (213.4); 2.4891 (96.6); 2.2770 (1.3); 2.2711 (1.9); 2.2648 (1.3); 1.9091 (0.5); 1.6742 (16.0); 1.6512 (15.8); 1.2348 (1.4); 1.1578 (0.6); 1.1471 (0.5); 1.1334 (0.4); 0.8550 (0.3); 0.8300 (0.4); 0.0107 (1.6); -0.0003 (43.6); -0.0113 (1.2). | |
| I-074 | major enantiomer (absolute configuration: S) 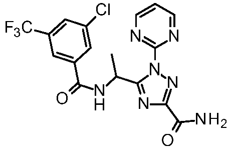 (reference compound) | 1H-NMR (300 MHz, d₆-DMSO, ppm): δ = 9.4411 (2.7); 9.4181 (2.8); 9.0179 (15.5); 9.0016 (16.0); 8.9422 (0.7); 8.9259 (0.7); 8.1082 (4.8); 8.0622 (5.1); 8.0481 (5.2); 8.0221 (0.3); 7.9255 (2.6); 7.7229 (2.6); 7.6889 (4.3); 7.6726 (7.8); 7.6564 (4.0); 6.0202 (0.4); 5.9970 (1.9); 5.9737 (3.0); 5.9507 (1.9); 5.9277 (0.4); 3.3209 (56.9); 3.2989 (25.6); 3.0842 (0.7); 3.0108 (0.5); 2.7332 (1.0); 2.7272 (1.4); 2.7210 (1.0); 2.5132 (87.2); 2.5072 (171.7); 2.5012 (228.9); 2.4952 (157.3); 2.4894 (72.3); 2.2771 (1.0); 2.2711 (1.4); 2.2650 (1.0); 2.0744 (2.7); 1.9097 (0.7); 1.6856 (10.2); 1.6625 (10.1); 1.2357 (0.4); 1.1493 (0.4); 0.0105 (1.4); -0.0004 (39.2); -0.0115 (1.3). | |
| I-075 | major enantiomer (absolute configuration: S) 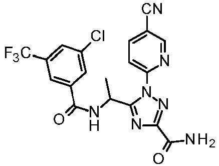 (reference compound) | ¹H-NMR (300 MHz, d₆-DMSO, ppm): δ = 9.4702 (4.3); 9.4478 (4.3); 9.0805 (7.7); 9.0781 (8.4); 9.0733 (8.5); 9.0708 (7.8); 8.6541 (6.4); 8.6468 (5.9); 8.6255 (6.8); 8.6182 (6.8); 8.1621 (8.2); 8.1511 (9.1); 8.1487 (8.6); 8.1224 (7.6); 8.1200 (7.7); 8.0950 (8.5); 8.0833 (7.0); 8.0794 (7.6); 8.0187 (4.8); 7.7975 (4.6); 6.1121 (0.6); 6.0887 (2.9); 6.0659 (4.4); 6.0525 (1.0); 6.0431 (2.8); 6.0195 (0.5); 3.3342 (492.9); 3.3106 (37.3); 2.8321 (0.4); 2.7349 (0.6); 2.7289 (0.9); 2.7229 (0.6); 2.5148 (54.4); 2.5089 (106.7); 2.5029 (141.4); 2.4969 (96.3); 2.4911 (43.4); 2.2788 (0.6); 2.2729 (0.8); 2.2667 (0.6); 1.9099 (0.3); 1.6784 (16.0); 1.6552 (15.8); 1.2357 (0.4); 0.0106 (0.4); -0.0003 (11.5). | |
| I-076 | major enantiomer (absolute configuration: S) 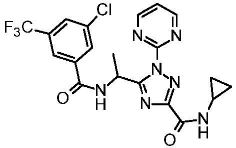 (reference compound) | ¹H-NMR (300 MHz, d₆-DMSO, ppm): δ = 9.4493 (2.7); 9.4264 (2.8); 9.0191 (15.6); 9.0029 (16.0); 8.6537 (2.9); 8.6385 (2.9); 8.1081 (4.8); 8.0655 (5.1); 8.0567 (5.1); 8.0542 (5.1); 8.0518 (5.1); 7.6931 (4.2); 7.6769 (7.8); 7.6607 (3.9); 6.0152 (0.4); 5.9917 (1.9); 5.9686 (3.0); 5.9455 (1.9); 5.9225 (0.4); 3.3244 (47.4); 3.3009 (0.4); 2.8971 (0.4); 2.8827 (0.9); 2.8698 (1.0); 2.8591 (1.9); 2.8445 (1.9); 2.8359 (1.1); 2.8215 (0.9); 2.8072 (0.4); 2.7278 (0.4); 2.5138 (22.3); 2.5079 (43.7); 2.5019 (58.0); 2.4959 (39.7); 2.4901 (18.1); 2.2715 (0.4); 2.0756 (3.4); 1.6863 (10.3); 1.6630 (10.1); 0.7195 (0.6); 0.6956 (2.9); 0.6883 (3.5); 0.6805 (3.0); 0.6640 (4.8); 0.6579 (5.3); 0.6433 (5.3); 0.6385 (4.9); 0.6200 (0.8); 0.6058 (0.5); 0.0104 (1.1); -0.0005 (25.6); -0.0114 (0.9). | |
| I-077 | major enantiomer (absolute configuration: S) 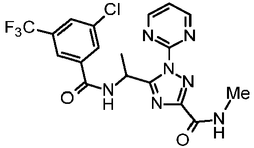 (reference compound) | ¹H-NMR (300 MHz, d₆-DMSO, ppm): δ = 9.4611 (2.9); 9.4384 (3.0); 9.0257 (15.6); 9.0095 (16.0); 8.5949 (0.8); 8.5810 (2.3); 8.5652 (2.3); 8.5507 (0.8); 8.1146 (5.0); 8.0686 (5.6); 8.0622 (7.1); 7.6973 (4.1); 7.6811 (7.7); 7.6649 (3.9); 6.0306 (0.4); 6.0078 (1.9); 5.9849 (3.1); 5.9619 (1.9); 5.9391 (0.4); 4.3681 (0.4); 4.3542 (0.4); 3.3464 (62.3); 2.7916 (13.4); 2.7758 (13.2); 2.5188 (5.3); 2.5129 (10.5); 2.5070 (13.9); 2.5012 (9.7); 1.6961 (10.5); 1.6729 (10.4); 1.0495 (2.8); 1.0291 (2.7); -0.0004 (1.8). | |
| I-078 | major enantiomer (absolute configuration: S) 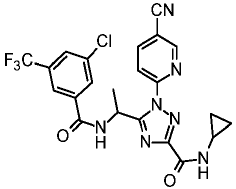 (reference compound) | ¹H-NMR (300 MHz, d₆-DMSO, ppm): δ = 9.4692 (4.5); 9.4471 (4.7); 9.0839 (7.4); 9.0816 (8.3); 9.0768 (8.4); 9.0744 (7.9); 8.6909 (5.1); 8.6762 (5.2); 8.6561 (6.3); 8.6487 (5.8); 8.6275 (6.6); 8.6201 (6.7); 8.1616 (13.2); 8.1354 (7.3); 8.1330 (7.6); 8.0961 (8.3); 8.0747 (7.6); 6.1097 (0.6); 6.0871 (3.0); 6.0642 (4.7); 6.0415 (3.0); 6.0194 (0.6); 3.3311 (131.5); 2.8894 (0.6); 2.8757 (1.5); 2.8630 (1.9); 2.8514 (2.9); 2.8378 (2.8); 2.8282 (1.7); 2.8139 (1.5); 2.8000 (0.6); 2.5176 (9.3); 2.5117 (18.0); 2.5058 (23.8); 2.4998 (16.4); 2.4940 (7.6); 2.0785 (1.8); 1.6812 (16.0); 1.6580 (15.8); 0.7451 (1.7); 0.7371 (1.4); 0.7199 (6.3); 0.7054 (4.6); 0.6944 (6.2); 0.6820 (3.6); 0.6603 (1.6); 0.6494 (3.4); 0.6360 (8.5); 0.6238 (5.7); 0.6182 (5.0); 0.5975 (1.5); 0.0105 (0.5); -0.0004 (11.2); -0.0113 (0.4). | |
| I-079 | major enantiomer (absolute configuration: S) 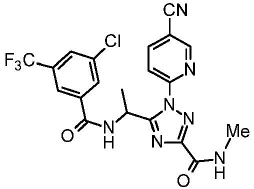 (reference compound) | ¹H-NMR (300 MHz, d₆-DMSO, ppm): δ = 9.4602 (2.0); 9.4382 (2.0); 9.0835 (3.2); 9.0813 (3.5); 9.0763 (3.6); 9.0740 (3.3); 8.6550 (2.6); 8.6476 (2.5); 8.6263 (3.1); 8.6190 (3.3); 8.6064 (1.6); 8.5904 (1.6); 8.5752 (0.5); 8.1609 (3.4); 8.1391 (3.4); 8.1369 (3.4); 8.1105 (3.4); 8.1082 (3.6); 8.0960 (3.5); 8.0722 (3.2); 6.0848 (1.3); 6.0622 (2.0); 6.0395 (1.3); 3.3279 (9.1); 2.8038 (8.8); 2.7879 (8.6); 2.5190 (4.7); 2.5131 (9.2); 2.5071 (12.1); 2.5012 (8.4); 2.4955 (3.9); 2.0799 (16.0); 1.6899 (7.0); 1.6667 (6.9); -0.0004 (1.8). | |
| I-080 | major enantiomer (absolute configuration: S) 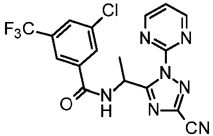 (reference compound) | ¹H-NMR (300 MHz, d₆-DMSO, ppm): δ = 9.4738 (2.6); 9.4515 (2.6); 9.0614 (15.7); 9.0451 (16.0); 8.0984 (4.7); 8.0708 (4.5); 8.0366 (4.7); 7.7633 (4.3); 7.7470 (7.9); 7.7307 (4.0); 6.0473 (0.4); 6.0242 (2.0); 6.0013 (3.1); 5.9785 (2.0); 5.9558 (0.4); 3.3150 (88.3); 2.7338 (0.4); 2.7277 (0.5); 2.7214 (0.3); 2.5136 (31.3); 2.5076 (60.7); 2.5016 (80.5); 2.4956 (54.4); 2.4897 (24.4); 2.2774 (0.3); 2.2716 (0.5); 2.2654 (0.4); 2.0734 (4.4); 1.6907 (11.2); 1.6675 (11.1); 1.2348 (0.5); 0.8591 (0.4); -0.0004 (6.3). | |
| I-081 | major enantiomer (absolute configuration: S) 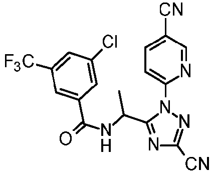 (reference compound) | ¹H-NMR(300 MHz, d₆-DMSO, ppm): δ = 9.4989 (4.0); 9.4768 (4.1); 9.1264 (6.8); 9.1240 (7.6); 9.1193 (7.7); 9.1168 (7.1); 8.6766 (5.8); 8.6693 (5.5); 8.6481 (6.4); 8.6407 (6.4); 8.1561 (7.6); 8.1537 (7.7); 8.1277 (13.1); 8.1252 (12.5); 8.0883 (7.0); 8.0633 (7.0); 6.0800 (0.6); 6.0571 (2.9); 6.0343 (4.4); 6.0116 (2.9); 5.9887 (0.6); 4.2227 (0.5); 3.3254 (93.3); 2.7350 (0.4); 2.7288 (0.6); 2.7225 (0.4); 2.5147 (36.4); 2.5087 (71.4); 2.5027 (95.0); 2.4967 (65.2); 2.4909 (29.8); 2.2788 (0.4); 2.2726 (0.6); 2.2666 (0.4); 2.0755 (1.2); 1.9102 (0.4); 1.6789 (16.0); 1.6557 (15.8); 1.2333 (0.6); 0.9368 (0.4); 0.9124 (0.9); 0.8875 (0.4); 0.0106 (0.3); -0.0004 (8.7). | |
| I-082 | 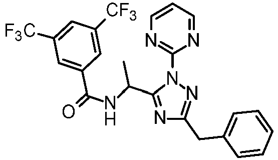 (reference compound) | ¹H-NMR (400 MHz, d₆-DMSO, ppm): δ = 9.5738 (2.8); 9.5556 (2.8); 8.9608 (15.7); 8.9486 (16.0); 8.3857 (10.2); 8.3048 (4.5); 7.5993 (4.2); 7.5872 (7.8); 7.5750 (4.1); 7.3254 (1.9); 7.3210 (2.9); 7.3042 (8.5); 7.3010 (7.4); 7.2959 (6.4); 7.2943 (6.7); 7.2888 (1.5); 7.2765 (7.6); 7.2724 (2.8); 7.2573 (2.8); 7.2252 (1.5); 7.2211 (2.4); 7.2165 (1.3); 7.2101 (1.1); 7.2037 (2.7); 7.1972 (0.8); 7.1905 (0.7); 7.1865 (1.0); 7.1823 (0.5); 6.0553 (0.4); 6.0381 (2.0); 6.0204 (3.1); 6.0027 (2.0); 5.9853 (0.4); 5.7586 (5.4); 4.1010 (0.4); 4.0611 (10.5); 4.0214 (0.4); 3.3341 (76.9); 2.6782 (0.4); 2.6738 (0.6); 2.6693 (0.4); 2.5272 (1.8); 2.5137 (34.8); 2.5093 (68.8); 2.5048 (88.3); 2.5003 (63.8); 2.4959 (31.1); 2.3361 (0.4); 2.3316 (0.5); 2.3271 (0.4); 1.6465 (11.2); 1.6291 (11.1); 0.0080 (2.1); - 0.0002 (55.9); -0.0085 (2.1). | 521.2 [M+H]⁺ |
| I-083 | 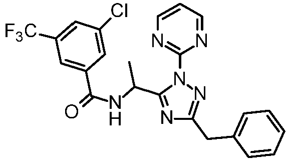 (reference compound) | ¹H-NMR(400.2 MHz, d6-DMSO): | 487.1 [M+H]⁺ |
| | | δ= 9.4144 (3.0); 9.3962 (3.0); 8.9562 (15.6); 8.9440 (16.0); 8.0879 (5.6); 8.0553 (5.4); 8.0357 (5.6); 7.5986 (4.4); 7.5865 (7.8); 7.5743 (4.2); 7.3259 (1.7); 7.3211 (2.7); 7.3048 (10.4); 7.3003 (14.5); 7.2818 (8.4); 7.2774 (2.7); 7.2624 (2.8); 7.2293 (1.6); 7.2248 (2.5); 7.2197 (1.3); 7.2144 (1.3); 7.2077 (2.8); 7.2011 (0.8); 7.1953 (0.8); 7.1910 (1.1); 7.1864 (0.6); 6.0171 (0.5); 5.9998 (2.2); 5.9820 (3.6); 5.9643 (2.3); 5.9469 (0.5); 5.7569 (12.9); 4.0974 (0.4); 4.0576 (12.8); 4.0178 (0.4); 3.3336 (240.3); 3.1762 (0.7); 3.1631 (0.7); 2.6767 (0.8); 2.6723 (1.1); 2.6678 (0.8); 2.5256 (3.5); 2.5121 (74.8); 2.5078 (148.9); 2.5033 (192.1); 2.4988 (138.1); 2.4944 (67.1); 2.3346 (0.8); 2.3301 (1.1); 2.3257 (0.8); 1.6233 (12.8); 1.6059 (12.8); - 0.0002 (2.3) | |
| I-084 | 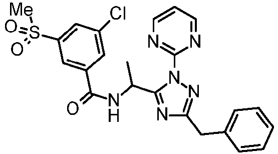 (reference compound) | ¹H-NMR(400 MHz, d₆-DMSO, ppm): δ = 9.4554 (2.8); 9.4372 (2.9); 8.9606 (15.6); 8.9484 (16.0); 8.3172 (1.2); 8.2302 (3.9); 8.2265 (7.3); 8.2227 (4.4); 8.1333 (2.8); 8.1285 (7.1); 8.1244 (5.7); 8.1198 (5.8); 8.1160 (6.4); 8.1113 (2.7); 7.6034 (4.1); 7.5912 (7.8); 7.5791 (4.0); 7.3328 (1.1); 7.3274 (2.0); 7.3219 (1.1); 7.3089 (14.8); 7.2919 (7.9); 7.2871 (2.2); 7.2770 (1.3); 7.2722 (2.3); 7.2322 (1.4); 7.2272 (2.2); 7.2213 (1.1); 7.2174 (1.3); 7.2105 (2.4); 7.1991 (0.8); 7.1942 (1.0); 7.1891 (0.6); 6.0352 (0.4); 6.0179 (2.0); 6.0002 (3.2); 5.9824 (2.1); 5.9650 (0.4); 5.7571 (0.4); 4.1077 (0.6); 4.1020 (0.5); 4.0947 (0.7); 4.0816 (0.4); 4.0625 (10.5); 4.0217 (0.4); 3.3335 (117.6); 3.3110 (32.6); 3.1761 (3.5); 3.1630 (3.4); 2.6766 (0.5); 2.6720 (0.7); 2.6676 (0.5); 2.5255 (2.3); 2.5207 (3.6); 2.5120 (44.6); 2.5076 (89.5); 2.5031 (116.1); 2.4985 (83.7); 2.4941 (40.5); 2.3344 (0.5); 2.3298 (0.7); 2.3254 (0.5); 1.6267 (11.5); 1.6093 (11.4); 0.0080 (2.3); - 0.0002 (65.1); -0.0085 (2.3) | 497.1 [M+H]⁺ |
| I-085 | 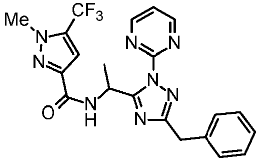 (reference compound) | ¹H-NMR(400.2 MHz, d₆-DMSO): | 457.2 [M+H]⁺ |
| | | δ= 8.9631 (10.9); 8.9509 (11.2); 8.7798 (2.0); 8.7606 (2.0); 7.6072 (2.9); 7.5950 (5.5); 7.5829 (2.8); 7.3356 (0.4); 7.3293 (1.0); 7.3145 (16.0); 7.3088 (6.6); 7.2984 (6.2); 7.2931 (1.2); 7.2838 (0.8); 7.2784 (1.3); 7.2395 (1.0); 7.2337 (1.4); 7.2258 (1.2); 7.2179 (1.6); 7.2040 (6.5); 6.0139 (0.3); 5.9967 (1.6); 5.9785 (2.1); 5.9603 (1.6); 5.9430 (0.3); 5.7559 (7.4); 4.0986 (0.4); 4.0595 (8.0); 4.0182 (0.5); 4.0028 (15.4); 3.3436 (304.1); 3.1762 (0.4); 3.1631 (0.3); 2.6769 (0.5); 2.6725 (0.7); 2.6681 (0.5); 2.5259 (2.1); 2.5211 (3.3); 2.5124 (43.4); 2.5081 (88.4); 2.5035 (115.9); 2.4990 (84.2); 2.4946 (41.4); 2.3349 (0.5); 2.3303 (0.7); 2.3259 (0.5); 1.5663 (8.9); 1.5490 (8.9); -0.0002 (0.6) | |
| I-086 | 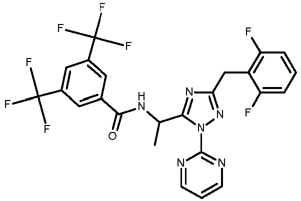 (reference compound) | ¹H-NMR(400.2 MHz, d₆-DMSO): | 557.1 [M+H]⁺ |
| | | δ= 9.5544 (2.7); 9.5362 (2.8); 8.9341 (15.6); 8.9219 (16.0); 8.3649 (9.9); 8.3163 (2.3); 8.3034 (4.3); 7.5850 (4.0); 7.5728 (7.6); 7.5607 (3.9); 7.4164 (0.6); 7.3996 (1.3); 7.3949 (1.3); 7.3783 (2.7); 7.3613 (1.4); 7.3577 (1.6); 7.3406 (0.7); 7.1268 (0.5); 7.1149 (3.9); 7.1064 (0.9); 7.0955 (5.6); 7.0755 (3.3); 7.0641 (0.4); 6.0287 (0.4); 6.0122 (2.0); 5.9945 (3.1); 5.9767 (2.1); 5.9600 (0.5); 5.7565 (4.1); 4.1007 (10.4); 3.3296 (1053.5); 2.6803 (2.6); 2.6759 (5.3); 2.6714 (7.3); 2.6668 (5.4); 2.5859 (0.7); 2.5248 (22.7); 2.5201 (36.3); 2.5114 (451.0); 2.5069 (908.2); 2.5024 (1177.9); 2.4978 (844.6); 2.4933 (405.1); 2.3382 (2.5); 2.3338 (5.1); 2.3293 (7.1); 2.3247 (5.2); 2.2976 (0.6); 1.6331 (11.1); 1.6157 (11.1); 1.2383 (0.6); 1.2105 (0.4); 1.1935 (0.3); 0.1462 (1.7); 0.0081 (16.5); -0.0001 (486.6); -0.0084 (16.8); -0.1495 (1.8) | |
| I-087 | 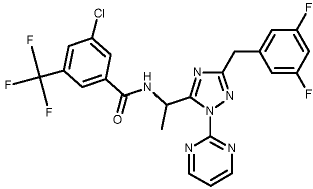 (reference compound) | ¹H-NMR(400.2 MHz, d₆-DMSO): | 522.8 [M+H]⁺ |
| | | δ= 9.4219 (3.1); 9.4042 (3.2); 8.9705 (15.6); 8.9583 (16.0); 8.0960 (5.7); 8.0586 (5.5); 8.0420 (5.8); 7.6153 (4.1); 7.6032 (7.8); 7.5910 (4.0); 7.1074 (0.7); 7.1015 (1.4); 7.0958 (1.0); 7.0839 (1.3); 7.0781 (2.8); 7.0723 (2.2); 7.0611 (1.4); 7.0485 (5.2); 7.0299 (5.0); 7.0144 (0.7); 6.0026 (0.5); 5.9853 (2.4); 5.9678 (3.7); 5.9502 (2.4); 5.9328 (0.5); 5.7584 (5.4); 4.1282 (15.6); 3.3304 (38.5); 2.6782 (0.5); 2.6736 (0.7); 2.6691 (0.5); 2.5269 (2.2); 2.5133 (42.4); 2.5091 (83.6); 2.5046 (107.9); 2.5001 (77.5); 2.4958 (37.5); 2.3359 (0.5); 2.3313 (0.6); 2.3269 (0.5); 1.6448 (13.0); 1.6274 (12.8); 1.3971 (2.3); -0.0001 (1.6) | |
| I-088 | 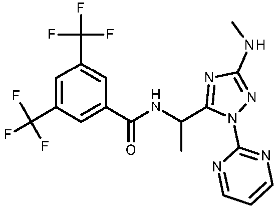 (reference compound) | ¹H-NMR(400.2 MHz, d₆-DMSO): | 459.7 [M+H]⁺ |
| | | δ= 9.5139 (2.0); 9.4960 (2.1); 8.8752 (10.0); 8.8631 (10.2); 8.5465 (0.4); 8.4725 (7.8); 8.3116 (3.4); 7.4533 (2.6); 7.4412 (4.9); 7.4291 (2.6); 6.0870 (0.3); 6.0697 (1.5); 6.0522 (2.4); 6.0346 (1.6); 6.0173 (0.3); 5.7577 (0.9); 3.6479 (1.3); 3.5691 (14.3); 3.4917 (1.0); 3.4731 (1.4); 3.4624 (1.6); 2.7451 (16.0); 2.6770 (0.5); 2.6726 (0.7); 2.6682 (0.5); 2.5260 (1.8); 2.5124 (42.9); 2.5082 (85.7); 2.5037 (110.9); 2.4992 (81.0); 2.4949 (40.2); 2.3351 (0.5); 2.3305 (0.7); 2.3261 (0.5); 1.6202 (8.6); 1.6029 (8.5); 1.5535 (0.4); 1.5368 (0.4); -0.0001 (2.4) | |
| I-089 | 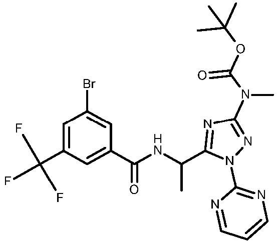 (reference compound) | ¹H-NMR(400.2 MHz, d₆-DMSO): | 570.1 [M+H]⁺ |
| | | δ= 9.4541 (0.5); 9.4364 (0.5); 8.9872 (2.8); 8.9751 (2.9); 8.2905 (1.0); 8.1719 (0.9); 8.1320 (0.9); 7.6283 (0.8); 7.6162 (1.4); 7.6040 (0.7); 6.0261 (0.4); 6.0086 (0.6); 5.9910 (0.4); 3.3291 (73.3); 3.3075 (0.5); 3.2129 (6.9); 2.6761 (0.3); 2.6716 (0.4); 2.6670 (0.3); 2.5250 (1.4); 2.5115 (28.7); 2.5072 (56.8); 2.5026 (73.1); 2.4981 (52.9); 2.4937 (25.8); 2.3294 (0.4); 2.0751 (0.6); 1.6496 (2.0); 1.6322 (2.0); 1.5033 (0.7); 1.3386 (16.0); -0.0002 (0.4) | |
| I-090 | 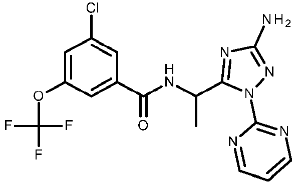 (reference compound) | ¹H-NMR(400.2 MHz, d₆-DMSO): | 428.2 [M+H]⁺ |
| | | δ= 9.2572 (2.8); 9.2395 (2.8); 8.8833 (0.3); 8.8708 (0.5); 8.8557 (15.6); 8.8436 (16.0); 8.3151 (1.1); 7.9756 (4.0); 7.9716 (6.3); 7.9677 (4.3); 7.7714 (4.4); 7.7550 (4.5); 7.7524 (4.5); 7.4429 (4.0); 7.4308 (7.6); 7.4187 (4.0); 6.0187 (0.5); 6.0011 (2.1); 5.9835 (3.3); 5.9660 (2.2); 5.9483 (0.4); 5.7555 (1.0); 5.7405 (8.6); 3.6476 (0.4); 3.5684 (0.5); 3.4962 (0.4); 3.4873 (0.4); 3.4731 (0.5); 3.4614 (0.5); 3.3392 (885.9); 2.6765 (2.7); 2.6720 (3.7); 2.6673 (2.7); 2.5254 (11.3); 2.5205 (18.0); 2.5119 (227.9); 2.5075 (459.8); 2.5030 (598.5); 2.4984 (429.9); 2.4940 (207.1); 2.3385 (1.2); 2.3343 (2.5); 2.3298 (3.5); 2.3252 (2.5); 2.3208 (1.2); 1.5849 (12.0); 1.5675 (11.9); 1.2338 (1.0); 1.1659 (0.4); -0.0001 (4.9) | |
| I-091 | 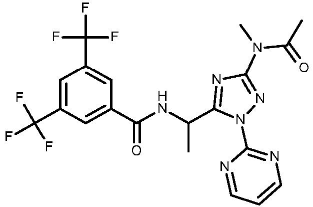 (reference compound) | ¹H-NMR(400.2 MHz, d₆-DMSO): | 501.7 [M+H]⁺ |
| | | δ= 9.5824 (1.3); 9.5649 (1.3); 8.9941 (7.3); 8.9820 (7.4); 8.4008 (4.7); 8.3164 (2.7); 7.6360 (1.9); 7.6238 (3.6); 7.6117 (1.9); 6.0600 (1.0); 6.0427 (1.6); 6.0254 (1.0); 3.6018 (0.5); 3.3250 (116.7); 3.2721 (15.9); 2.9270 (0.5); 2.9147 (0.5); 2.6758 (1.4); 2.6713 (1.9); 2.6668 (1.4); 2.5248 (5.8); 2.5200 (9.2); 2.5113 (119.5); 2.5069 (242.1); 2.5023 (314.9); 2.4978 (225.1); 2.4933 (107.2); 2.3337 (1.4); 2.3291 (1.9); 2.3246 (1.4); 2.2610 (16.0); 1.9089 (0.4); 1.7607 (0.6); 1.6950 (5.3); 1.6776 (5.3); 1.5252 (0.4); 1.5083 (0.4); 1.1819 (0.4); - 0.0001 (1.6) | |
| I-092 | 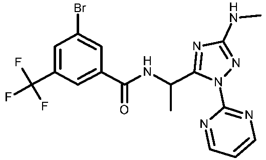 (reference compound) | ¹H-NMR(400.2 MHz, d₆-DMSO): | 471.6 [M+H]⁺ |
| | | δ= 9.3508 (3.1); 9.3331 (3.2); 8.8715 (15.6); 8.8594 (16.0); 8.3091 (6.0); 8.1655 (6.2); 8.1555 (6.4); 7.4527 (4.0); 7.4406 (7.7); 7.4285 (4.0); 6.2397 (2.4); 6.2269 (2.5); 6.2154 (0.9); 6.0473 (0.5); 6.0298 (2.4); 6.0123 (3.7); 5.9948 (2.4); 5.9771 (0.6); 5.7570 (2.8); 3.6473 (1.5); 3.5684 (3.4); 3.3285 (183.9); 2.9037 (0.4); 2.7483 (14.5); 2.7359 (14.5); 2.6761 (1.7); 2.6716 (2.3); 2.6674 (1.7); 2.5248 (7.3); 2.5112 (146.6); 2.5071 (287.0); 2.5026 (369.8); 2.4981 (267.8); 2.4939 (131.9); 2.3338 (1.6); 2.3294 (2.2); 2.3250 (1.6); 1.6166 (0.4); 1.5943 (13.2); 1.5770 (13.1); -0.0001 (1.3) | |
| I-093 | 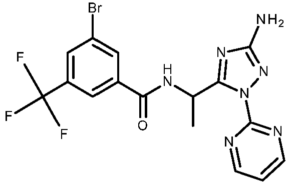 (reference compound) | ¹H-NMR(400.2 MHz, d₆-DMSO): | 457.5 [M+H]⁺ |
| | | δ= 9.3405 (3.1); 9.3226 (3.0); 8.8857 (0.9); 8.8827 (0.9); 8.8737 (1.1); 8.8705 (1.1); 8.8591 (15.5); 8.8470 (16.0); 8.3140 (5.7); 8.2930 (0.4); 8.2827 (0.4); 8.1629 (10.2); 8.1370 (0.6); 8.1178 (0.5); 7.4683 (0.5); 7.4449 (4.1); 7.4329 (7.7); 7.4208 (4.0); 6.0389 (0.5); 6.0216 (2.2); 6.0041 (3.5); 5.9864 (2.3); 5.9693 (0.5); 5.7440 (9.0); 3.7001 (0.3); 3.6471 (0.4); 3.3265 (123.5); 2.6760 (1.4); 2.6714 (1.9); 2.6669 (1.4); 2.5249 (5.8); 2.5113 (125.4); 2.5070 (247.9); 2.5025 (318.5); 2.4980 (231.3); 2.4937 (113.8); 2.3338 (1.4); 2.3293 (2.0); 2.3247 (1.4); 2.0864 (1.4); 1.5917 (12.9); 1.5744 (12.8); 1.2342 (0.8); -0.0002 (1.3) | |
| I-094 | 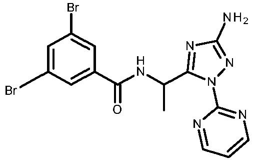 (reference compound) | ¹H-NMR(400.2 MHz, d₆-DMSO): | 467.3 [M+H]⁺ |
| | | δ= 9.1859 (2.5); 9.1680 (2.3); 8.8858 (1.3); 8.8832 (1.4); 8.8737 (1.5); 8.8711 (1.5); 8.8564 (11.8); 8.8444 (11.9); 8.3161 (0.9); 8.0246 (2.3); 8.0202 (4.9); 8.0162 (6.0); 8.0080 (16.0); 8.0039 (8.9); 7.9881 (1.5); 7.9838 (1.2); 7.9767 (1.6); 7.9724 (1.2); 7.4805 (0.4); 7.4699 (0.7); 7.4579 (0.4); 7.4453 (3.2); 7.4332 (5.9); 7.4211 (3.1); 6.6837 (0.3); 6.0031 (0.5); 5.9858 (1.8); 5.9762 (0.9); 5.9682 (2.7); 5.9587 (0.6); 5.9506 (1.8); 5.9330 (0.4); 5.7397 (6.7); 4.6238 (0.5); 3.7246 (0.4); 3.7180 (0.5); 3.7136 (0.9); 3.7097 (0.7); 3.7018 (1.0); 3.6997 (1.1); 3.6801 (1.0); 3.6781 (0.9); 3.6703 (0.6); 3.6661 (0.8); 3.6616 (0.4); 3.6551 (0.4); 3.6472 (1.8); 3.5682 (0.8); 3.5099 (0.5); 3.4964 (0.8); 3.4847 (0.6); 3.4728 (0.9); 3.4613 (0.8); 3.3261 (186.6); 2.6757 (1.8); 2.6712 (2.4); 2.6666 (1.8); 2.5246 (7.6); 2.5111 (155.0); 2.5068 (307.5); 2.5022 (402.5); 2.4977 (297.0); 2.4933 (147.5); 2.3336 (1.8); 2.3290 (2.5); 2.3245 (1.8); 1.5680 (10.4); 1.5507 (10.2); 1.3377 (0.3); 1.2347 (0.7); 0.0079 (1.9); -0.0002 (53.3); -0.0085 (2.2); - 0.0636 (1.1) | |
| I-095 | 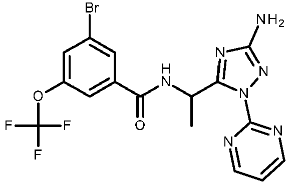 (reference compound) | ¹H-NMR(400.2 MHz, d₆-DMSO): | 473.8 [M+H]⁺ |
| | | δ= 9.3288 (0.3); 9.2572 (3.3); 9.2396 (3.1); 8.9445 (0.4); 8.9325 (0.4); 8.9282 (0.4); 8.9242 (0.4); 8.8833 (1.6); 8.8800 (1.7); 8.8713 (1.8); 8.8677 (1.8); 8.8554 (16.0); 8.8433 (16.0); 8.3161 (2.6); 8.1812 (0.4); 8.1080 (4.6); 8.1042 (7.2); 8.1006 (4.5); 8.0841 (0.7); 8.0790 (0.8); 8.0745 (0.8); 8.0557 (0.4); 8.0502 (0.4); 8.0392 (0.4); 8.0248 (0.4); 7.8755 (5.4); 7.8109 (0.5); 7.7791 (4.8); 7.7448 (0.7); 7.4971 (0.4); 7.4934 (0.3); 7.4793 (0.6); 7.4671 (0.7); 7.4555 (0.7); 7.4428 (4.2); 7.4307 (7.8); 7.4186 (4.1); 6.3516 (0.4); 5.9988 (2.6); 5.9814 (3.8); 5.9639 (2.6); 5.9465 (0.8); 5.9296 (0.3); 5.7411 (9.1); 4.6367 (0.5); 4.6243 (0.8); 4.6125 (0.4); 4.2087 (0.3); 3.8600 (0.3); 3.7294 (0.6); 3.7246 (0.7); 3.7136 (1.4); 3.7096 (1.4); 3.6997 (1.6); 3.6801 (1.7); 3.6703 (1.1); 3.6662 (1.3); 3.6615 (0.7); 3.6551 (0.8); 3.6471 (4.8); 3.6280 (0.4); 3.6218 (0.4); 3.5982 (0.5); 3.5844 (0.4); 3.5682 (0.8); 3.5570 (0.4); 3.5510 (0.3); 3.5421 (0.3); 3.5097 (0.9); 3.4962 (1.3); 3.4862 (1.1); 3.4728 (1.6); 3.4610 (1.4); 3.4498 (0.8); 3.4129 (0.6); 3.3273 (938.9); 2.8915 (0.7); 2.7311 (0.7); 2.6897 (0.8); 2.6757 (5.8); 2.6711 (8.0); 2.6668 (6.0); 2.5727 (1.0); 2.5246 (25.0); 2.5110 (522.0); 2.5067 (1017.6); 2.5022 (1314.9); 2.4976 (968.6); 2.4933 (480.5); 2.3876 (0.3); 2.3335 (5.9); 2.3291 (7.9); 2.3245 (5.8); 1.6542 (0.6); 1.6465 (0.6); 1.6422 (0.6); 1.6382 (0.6); 1.6271 (0.8); 1.5825 (14.0); 1.5652 (14.0); 1.5204 (0.8); 1.4824 (0.6); 1.4447 (0.6); 1.4389 (0.7); 1.4295 (0.7); 1.4193 (0.7); 1.3954 (1.0); 1.3751 (0.9); 1.3382 (0.8); 1.3106 (0.6); 1.2909 (0.7); 1.2589 (0.6); 1.2347 (1.5); 1.1676 (0.4); 1.1405 (0.4); 1.1270 (0.5); 1.1121 (0.4); 1.1012 (0.5); 1.0919 (0.4); 1.0821 (0.4); 1.0570 (0.3); 1.0192 (0.3); 1.0035 (0.3); 0.9734 (0.4); 0.9190 (0.3); 0.8760 (0.4); 0.8539 (0.5); 0.8352 (0.5); 0.8326 (0.5); 0.8098 (0.4); 0.1459 (0.6); 0.0079 (5.8); -0.0002 (163.7); -0.0085 (6.9); -0.1499 (0.7) | |
| I-096 | 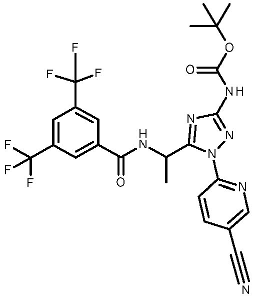 (reference compound) | ¹H-NMR(400.2 MHz, d₆-DMSO): | 570.3 [M+H]⁺ |
| | | δ= 10.0900 (1.2); 9.6042 (0.6); 9.5878 (0.6); 9.0206 (1.0); 9.0167 (1.0); 8.5486 (0.7); 8.5431 (0.6); 8.5271 (0.7); 8.5215 (0.7); 8.4790 (2.0); 8.3284 (0.9); 7.9622 (1.0); 7.9608 (0.9); 7.9406 (0.9); 7.9391 (0.9); 6.1166 (0.4); 6.0997 (0.6); 6.0825 (0.4); 3.3278 (67.0); 2.6898 (0.4); 2.6757 (0.4); 2.6714 (0.6); 2.6670 (0.4); 2.5247 (1.8); 2.5112 (35.9); 2.5069 (69.7); 2.5024 (89.7); 2.4979 (65.3); 2.4935 (31.8); 2.3337 (0.4); 2.3294 (0.5); 2.3247 (0.4); 2.0749 (0.9); 1.6582 (2.1); 1.6408 (2.0); 1.4301 (16.0); -0.0002 (5.7) | |
| I-097 | 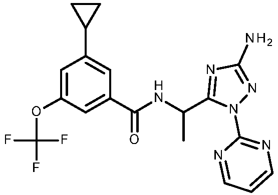 | ¹H-NMR(400.2 MHz, d₆-DMSO): | 433.9 [M+H]⁺ |
| | | δ= 9.0740 (2.9); 9.0559 (2.9); 8.8797 (0.5); 8.8754 (0.5); 8.8676 (0.6); 8.8633 (0.8); 8.8537 (15.6); 8.8417 (16.0); 8.3171 (0.4); 7.5039 (8.5); 7.5005 (8.7); 7.4751 (0.6); 7.4715 (0.6); 7.4591 (0.6); 7.4400 (4.1); 7.4279 (7.6); 7.4158 (4.0); 7.2660 (4.3); 6.0095 (0.5); 5.9925 (2.1); 5.9749 (3.3); 5.9572 (2.2); 5.9397 (0.5); 5.7238 (8.5); 3.6475 (0.8); 3.3269 (46.0); 2.6763 (0.8); 2.6717 (1.0); 2.6671 (0.8); 2.6625 (0.4); 2.5251 (3.0); 2.5117 (67.7); 2.5073 (134.5); 2.5027 (174.7); 2.4982 (127.4); 2.4937 (62.3); 2.3341 (0.8); 2.3296 (1.1); 2.3250 (0.8); 2.0630 (0.6); 2.0506 (1.3); 2.0420 (1.4); 2.0383 (1.1); 2.0296 (2.6); 2.0171 (1.6); 2.0088 (1.4); 1.9962 (0.8); 1.5840 (11.9); 1.5666 (11.8); 1.0461 (1.5); 1.0349 (4.2); 1.0294 (4.6); 1.0256 (2.5); 1.0189 (2.6); 1.0140 (4.5); 1.0085 (4.5); 0.9981 (2.0); 0.9808 (0.4); 0.7944 (1.8); 0.7837 (4.9); 0.7786 (4.9); 0.7714 (4.8); 0.7663 (5.4); 0.7548 (1.8); -0.0001 (3.0) | |
| I-098 | 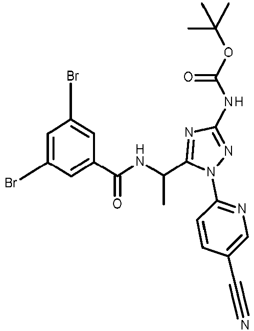 (reference compound) | ¹H-NMR(400.2 MHz, d₆-DMSO): | 592.1 [M+H]⁺ |
| | | δ= 10.0884 (1.3); 9.3024 (0.6); 9.2856 (0.6); 9.0134 (1.0); 9.0094 (1.0); 8.5470 (0.7); 8.5415 (0.6); 8.5255 (0.7); 8.5200 (0.7); 8.0371 (1.3); 8.0330 (1.1); 8.0158 (3.2); 8.0115 (2.3); 7.9548 (1.0); 7.9330 (0.9); 6.0471 (0.4); 6.0305 (0.6); 6.0131 (0.4); 3.3298 (96.8); 2.6757 (0.4); 2.6713 (0.6); 2.6669 (0.4); 2.5068 (74.4); 2.5024 (95.2); 2.4979 (71.0); 2.3335 (0.4); 2.3291 (0.6); 2.3247 (0.4); 1.6094 (2.1); 1.5921 (2.1); 1.4394 (16.0); -0.0002 (1.5) | |
| I-099 | 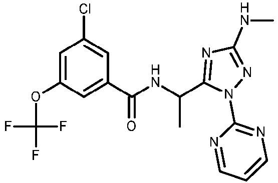 (reference compound) | ¹H-NMR(400.2 MHz, d₆-DMSO): | 441.8 [M+H]⁺ |
| | | δ= 9.2694 (1.9); 9.2516 (1.9); 8.8690 (8.8); 8.8570 (9.1); 7.9730 (2.5); 7.9689 (4.1); 7.9653 (3.0); 7.9531 (2.5); 7.7706 (3.0); 7.7503 (2.9); 7.7482 (3.0); 7.4509 (2.3); 7.4388 (4.4); 7.4267 (2.3); 6.2402 (1.3); 6.2277 (1.4); 6.0110 (1.3); 5.9936 (2.1); 5.9760 (1.4); 5.7571 (1.2); 3.3303 (54.4); 2.8915 (15.6); 2.7486 (7.4); 2.7328 (15.6); 2.7319 (16.0); 2.6765 (0.5); 2.6720 (0.6); 2.6674 (0.5); 2.5501 (0.4); 2.5075 (78.8); 2.5031 (103.7); 2.4986 (79.8); 2.3344 (0.4); 2.3299 (0.6); 2.3256 (0.5); 1.5881 (7.4); 1.5708 (7.4); -0.0001 (2.6) | |
| I-100 | 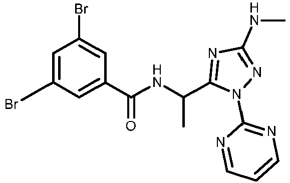 (reference compound) | ¹H-NMR(400.2 MHz, d₆-DMSO): | 481.7 [M+H]⁺ |
| | | δ= 9.1983 (2.4); 9.1802 (2.5); 8.8948 (0.3); 8.8692 (13.0); 8.8572 (13.4); 8.3167 (0.5); 8.0766 (0.4); 8.0253 (2.9); 8.0210 (6.0); 8.0168 (5.6); 8.0037 (16.0); 7.9994 (10.2); 7.9829 (0.3); 7.9780 (0.4); 7.4532 (3.5); 7.4411 (6.4); 7.4290 (3.4); 6.2531 (0.7); 6.2409 (2.3); 6.2282 (2.3); 6.2156 (0.7); 6.0117 (0.4); 5.9939 (1.8); 5.9764 (2.8); 5.9588 (1.9); 5.9420 (0.4); 5.7570 (0.6); 3.3265 (127.7); 2.9024 (0.5); 2.7469 (12.6); 2.7343 (12.6); 2.7067 (0.5); 2.6964 (0.4); 2.6756 (1.3); 2.6712 (1.7); 2.6667 (1.3); 2.5246 (5.1); 2.5111 (113.2); 2.5068 (220.3); 2.5023 (283.1); 2.4977 (207.1); 2.4933 (101.9); 2.3335 (1.3); 2.3291 (1.7); 2.3246 (1.3); 2.0751 (7.5); 1.8299 (0.5); 1.5940 (0.4); 1.5707 (10.0); 1.5534 (10.0); 1.4126 (0.3); 1.3952 (0.3); - 0.0002 (4.6) | |
| I-101 | 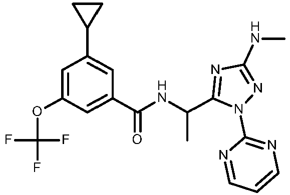 | ¹H-NMR(400.2 MHz, d₆-DMSO): | 448.3 [M+H]⁺ |
| | | δ= 9.0920 (3.0); 9.0739 (3.0); 8.8666 (15.6); 8.8545 (16.0); 7.4969 (10.0); 7.4935 (9.5); 7.4483 (4.1); 7.4363 (7.6); 7.4242 (4.0); 7.2693 (4.5); 6.2355 (0.9); 6.2236 (2.8); 6.2108 (2.8); 6.1984 (0.8); 6.0160 (0.5); 5.9990 (2.2); 5.9814 (3.4); 5.9638 (2.2); 5.9462 (0.4); 3.3328 (88.9); 2.7472 (15.2); 2.7346 (15.1); 2.6765 (0.8); 2.6719 (1.0); 2.6674 (0.8); 2.5253 (3.5); 2.5118 (66.7); 2.5075 (129.1); 2.5030 (166.8); 2.4985 (123.2); 2.4941 (60.9); 2.3342 (0.8); 2.3298 (1.0); 2.3254 (0.7); 2.0762 (1.0); 2.0623 (0.6); 2.0497 (1.3); 2.0413 (1.4); 2.0289 (2.6); 2.0164 (1.5); 2.0081 (1.4); 1.9954 (0.7); 1.5863 (12.0); 1.5689 (11.9); 1.0459 (1.5); 1.0347 (4.3); 1.0292 (4.6); 1.0187 (2.5); 1.0138 (4.4); 1.0083 (4.4); 0.9980 (1.7); 0.7929 (1.8); 0.7823 (5.0); 0.7772 (5.0); 0.7700 (4.7); 0.7649 (5.2); 0.7534 (1.4); 0.1459 (0.5); 0.0078 (5.2); - 0.0002 (119.8); -0.0085 (4.7); -0.1495 (0.5) | |
| I-102 | 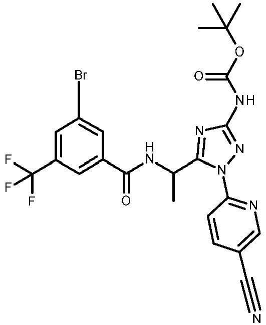 (reference compound) | ¹H-NMR(400.2 MHz, d₆-DMSO): | 581.8 [M+H]⁺ |
| | | δ= 10.0886 (1.3); 9.4491 (0.6); 9.4325 (0.6); 9.0180 (1.0); 9.0163 (1.0); 9.0126 (1.0); 9.0108 (1.0); 8.5476 (0.7); 8.5420 (0.7); 8.5260 (0.8); 8.5204 (0.8); 8.3233 (1.0); 8.1796 (1.0); 8.1632 (1.0); 7.9597 (1.0); 7.9581 (1.0); 7.9382 (0.9); 7.9364 (0.9); 6.0822 (0.4); 6.0651 (0.6); 6.0479 (0.4); 3.3257 (9.6); 2.5252 (0.7); 2.5118 (15.0); 2.5074 (29.8); 2.5029 (39.0); 2.4983 (28.5); 2.4939 (14.0); 1.6342 (2.1); 1.6168 (2.1); 1.4355 (16.0); -0.0002 (0.9) | |
| I-103 | 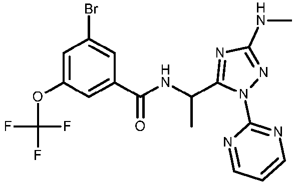 (reference compound) | ¹H-NMR(400.2 MHz, d₆-DMSO): | 487.7 [M+H]⁺ |
| | | δ= 9.2736 (3.3); 9.2557 (3.3); 8.9577 (0.4); 8.9459 (0.4); 8.8689 (15.6); 8.8569 (16.0); 8.3181 (0.8); 8.1014 (7.3); 8.0979 (4.7); 7.8788 (5.2); 7.7745 (5.2); 7.4516 (4.2); 7.4395 (7.7); 7.4274 (4.0); 6.2443 (2.2); 6.2322 (2.2); 6.0255 (0.5); 6.0077 (2.4); 5.9902 (3.7); 5.9726 (2.4); 5.9550 (0.6); 3.5683 (0.4); 3.3336 (227.8); 2.9476 (0.4); 2.9414 (0.6); 2.7471 (12.6); 2.7353 (12.6); 2.6760 (2.0); 2.6717 (2.7); 2.6673 (2.1); 2.5249 (8.7); 2.5072 (325.4); 2.5027 (422.0); 2.4983 (317.7); 2.3339 (1.9); 2.3296 (2.5); 2.3252 (1.8); 2.0757 (2.0); 1.6321 (0.4); 1.6151 (0.4); 1.5854 (13.1); 1.5681 (13.0); 1.4663 (0.4); 1.4502 (0.4); 1.4330 (2.2); 1.2584 (0.6); 1.2427 (0.7); 0.1461 (1.2); 0.0079 (11.3); -0.0001 (262.6); -0.0083 (12.1); -0.1495 (1.1) | |
| I-104 | 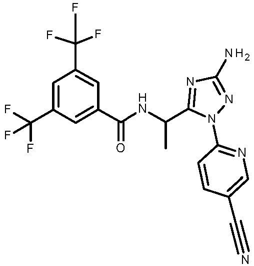 (reference compound) | ¹H-NMR(400.2 MHz, d₆-DMSO): | 469.8 [M+H]⁺ |
| | | δ= 9.5417 (4.1); 9.5242 (4.3); 9.5022 (0.3); 8.9353 (7.2); 8.9314 (7.2); 8.9298 (6.8); 8.9100 (0.3); 8.9040 (0.4); 8.5178 (14.7); 8.4898 (0.4); 8.4489 (5.7); 8.4433 (5.6); 8.4272 (5.8); 8.4216 (5.9); 8.3894 (0.4); 8.3287 (6.6); 8.3186 (2.2); 7.8283 (6.7); 7.8270 (6.7); 7.8067 (6.3); 7.8052 (6.3); 6.1428 (0.8); 6.1258 (3.2); 6.1085 (4.9); 6.0911 (3.3); 6.0741 (1.0); 6.0153 (0.7); 5.9984 (0.8); 5.9820 (0.8); 5.7582 (6.8); 3.7995 (0.4); 3.7146 (0.5); 3.7107 (0.4); 3.7009 (0.6); 3.6803 (0.6); 3.6702 (0.4); 3.6660 (0.5); 3.6616 (0.3); 3.6472 (1.1); 3.5684 (5.1); 3.5032 (1.0); 3.4906 (1.3); 3.4732 (1.6); 3.4607 (1.9); 3.3916 (58.3); 2.6897 (1.2); 2.6762 (2.8); 2.6718 (3.8); 2.6673 (2.9); 2.5253 (11.6); 2.5203 (19.3); 2.5118 (241.8); 2.5074 (484.8); 2.5028 (637.4); 2.4983 (473.0); 2.4938 (234.6); 2.3341 (2.9); 2.3297 (3.9); 2.3252 (2.9); 1.6168 (16.0); 1.5995 (15.8); 1.2757 (0.4); 1.2603 (0.6); 1.2439 (0.5); 0.1459 (1.8); 0.0080 (15.3); - 0.0001 (431.6); -0.0084 (17.5); -0.1496 (1.8) | |
| I-105 | 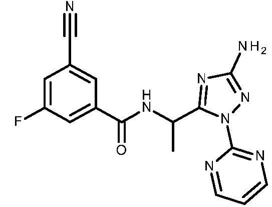 (reference compound) | ¹H-NMR(400.2 MHz, d₆-DMSO): | 352.9 [M+H]⁺ |
| | | δ= 9.2394 (2.8); 9.2217 (2.8); 8.8596 (15.7); 8.8475 (16.0); 8.3176 (2.0); 8.1623 (4.2); 8.1590 (7.3); 8.1557 (4.4); 8.0741 (1.8); 8.0709 (2.0); 8.0678 (2.1); 8.0648 (1.8); 8.0532 (1.9); 8.0500 (2.1); 8.0469 (2.1); 8.0439 (1.8); 7.9842 (1.9); 7.9805 (2.1); 7.9780 (2.0); 7.9745 (1.7); 7.9605 (2.0); 7.9568 (2.3); 7.9507 (1.7); 7.4465 (4.1); 7.4344 (7.7); 7.4223 (4.0); 6.0303 (0.5); 6.0139 (2.0); 5.9962 (3.5); 5.9787 (2.2); 5.9615 (0.5); 5.7578 (6.2); 5.7501 (8.4); 3.7146 (0.4); 3.7003 (0.4); 3.6779 (0.4); 3.6655 (0.4); 3.6470 (1.5); 3.5682 (1.0); 3.4960 (0.4); 3.4840 (0.3); 3.4726 (0.5); 3.4607 (0.5); 3.3326 (831.4); 2.6803 (1.9); 2.6759 (4.0); 2.6714 (5.3); 2.6669 (3.9); 2.5248 (17.2); 2.5114 (329.8); 2.5070 (655.1); 2.5024 (858.4); 2.4978 (630.7); 2.4934 (308.9); 2.3338 (3.7); 2.3292 (5.2); 2.3247 (3.8); 2.3205 (1.7); 2.0754 (2.0); 1.6148 (0.3); 1.5873 (11.6); 1.5700 (11.6); 1.4325 (0.9); 0.1459 (2.0); 0.0223 (0.5); 0.0080 (19.0); - 0.0001 (512.3); -0.0084 (18.6); -0.1496 (2.0) | |
| I-106 | 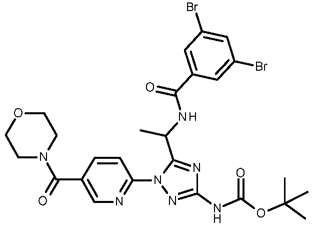 (reference compound) | | 680.1 [M+H]⁺ |
| I-107 | 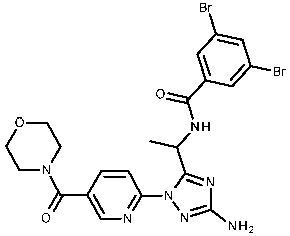 (reference compound) | ¹H-NMR(400.2 MHz, d₆-DMSO): | 579.2 [M+H]⁺ |
| | | δ= 9.1818 (1.7); 9.1640 (1.8); 8.4948 (3.0); 8.4907 (3.0); 8.0475 (1.9); 8.0418 (2.0); 8.0251 (16.0); 7.7410 (2.9); 7.7197 (2.6); 6.0556 (1.2); 6.0380 (1.8); 6.0205 (1.2); 5.8208 (4.6); 5.7564 (13.2); 3.6111 (1.9); 3.3261 (81.4); 2.6756 (0.6); 2.6712 (0.9); 2.6668 (0.7); 2.5244 (2.8); 2.5067 (106.4); 2.5023 (138.8); 2.4978 (103.3); 2.3335 (0.6); 2.3292 (0.9); 2.3248 (0.6); 2.0748 (0.3); 1.9295 (0.4); 1.7687 (0.5); 1.5773 (6.3); 1.5600 (6.2); 0.0080 (2.6); -0.0001 (67.4); -0.0084 (2.8) | |
| I-108 | 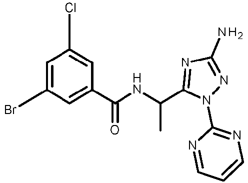 (reference compound) | ¹H-NMR(400.2 MHz, d₆-DMSO): | 423.8 [M+H]⁺ |
| | | δ= 9.1861 (3.2); 9.1679 (3.1); 8.8952 (0.4); 8.8833 (0.9); 8.8713 (0.9); 8.8566 (15.8); 8.8446 (16.0); 8.3164 (1.1); 7.9824 (4.5); 7.9785 (7.6); 7.9747 (5.2); 7.9457 (0.5); 7.9162 (4.3); 7.9116 (7.9); 7.9071 (4.6); 7.8903 (0.5); 7.8757 (5.2); 7.8718 (6.9); 7.8676 (4.2); 7.8461 (0.4); 7.4898 (0.3); 7.4689 (0.5); 7.4453 (4.2); 7.4332 (7.8); 7.4211 (4.1); 7.3043 (0.3); 7.2850 (0.7); 7.2603 (0.7); 6.3514 (0.4); 6.0042 (0.6); 5.9882 (2.4); 5.9706 (3.7); 5.9529 (2.4); 5.9351 (0.5); 5.7396 (9.3); 4.6236 (0.4); 3.8095 (0.6); 3.7247 (0.4); 3.7130 (0.6); 3.7000 (0.7); 3.6803 (0.7); 3.6700 (0.5); 3.6666 (0.6); 3.6471 (1.6); 3.5075 (0.4); 3.4958 (0.5); 3.4843 (0.4); 3.4722 (0.6); 3.4609 (0.6); 3.3264 (427.0); 2.6756 (3.0); 2.6711 (4.1); 2.6666 (3.1); 2.5244 (14.5); 2.5109 (252.2); 2.5066 (493.4); 2.5021 (640.6); 2.4976 (468.4); 2.4933 (230.4); 2.3334 (2.8); 2.3289 (3.8); 2.3245 (2.8); 1.9888 (0.3); 1.7896 (0.5); 1.6499 (0.9); 1.6135 (0.4); 1.5700 (13.4); 1.5527 (13.1); 1.4328 (1.5); 1.2981 (0.4); 1.2586 (0.7); 1.2339 (1.2); 1.1054 (0.3); 0.1460 (1.1); 0.0080 (8.9); -0.0002 (236.7); -0.0084 (9.0); -0.1495 (1.0) | |
| I-109 | 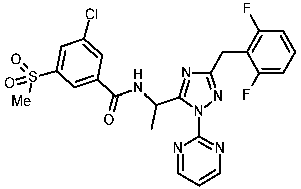 (reference compound) | ¹H-NMR(400.2 MHz, d6-DMSO): | 533.1 [M+H]⁺ |
| | | δ= 9.4435 (2.1); 9.4251 (2.2); 8.9355 (12.9); 8.9234 (13.2); 8.3950 (0.4); 8.3168 (1.1); 8.2856 (0.4); 8.2129 (3.0); 8.2090 (5.6); 8.2053 (3.4); 8.1298 (2.7); 8.1251 (5.1); 8.1209 (3.5); 8.1039 (3.7); 8.0999 (4.6); 8.0953 (2.5); 7.5908 (3.3); 7.5786 (6.2); 7.5665 (3.2); 7.4238 (0.4); 7.4073 (1.1); 7.4031 (1.0); 7.3862 (2.0); 7.3690 (1.0); 7.3654 (1.2); 7.3485 (0.6); 7.1398 (0.5); 7.1290 (3.0); 7.1196 (0.8); 7.1095 (4.4); 7.0990 (0.8); 7.0898 (2.5); 7.0781 (0.4); 6.0101 (0.3); 5.9935 (1.5); 5.9758 (2.3); 5.9580 (1.5); 5.7571 (16.0); 4.1025 (7.7); 4.0728 (0.6); 3.3732 (2.8); 3.3612 (0.8); 3.3271 (306.5); 3.3106 (25.7); 3.1312 (0.5); 3.1127 (0.4); 2.6801 (1.4); 2.6756 (2.8); 2.6711 (3.9); 2.6665 (2.9); 2.6619 (1.4); 2.5246 (11.9); 2.5198 (19.2); 2.5111 (234.8); 2.5067 (474.9); 2.5021 (617.5); 2.4975 (445.0); 2.4930 (214.2); 2.3379 (1.2); 2.3335 (2.7); 2.3289 (3.7); 2.3244 (2.7); 1.6167 (8.4); 1.5993 (8.3); 1.2508 (0.5); 1.2321 (1.0); 1.2134 (0.4); 0.1458 (1.0); 0.0080 (8.3); -0.0002 (254.0); -0.0085 (8.9); -0.1496 (0.9) | |
| I-110 | 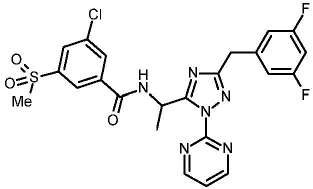 (reference compound) | ¹H-NMR(400.2 MHz, d₆-DMSO): | 532.7 [M+H]⁺ |
| | | δ= 9.4572 (3.0); 9.4394 (3.0); 8.9705 (15.6); 8.9583 (16.0); 8.2384 (4.2); 8.2347 (7.5); 8.2310 (4.6); 8.1374 (3.2); 8.1327 (7.2); 8.1286 (5.4); 8.1216 (5.6); 8.1178 (6.4); 8.1132 (3.0); 7.6164 (4.2); 7.6042 (7.8); 7.5921 (4.0); 7.1173 (0.7); 7.1115 (1.3); 7.1057 (1.0); 7.0939 (1.2); 7.0881 (2.7); 7.0822 (2.4); 7.0756 (1.1); 7.0649 (4.9); 7.0600 (5.0); 7.0438 (4.8); 7.0283 (0.7); 6.0236 (0.4); 6.0063 (2.1); 5.9887 (3.4); 5.9711 (2.2); 5.9540 (0.5); 5.7571 (2.3); 4.1335 (14.1); 3.3759 (0.6); 3.3628 (0.4); 3.3318 (112.7); 3.3103 (32.8); 2.6773 (0.5); 2.6727 (0.8); 2.6683 (0.5); 2.5261 (2.4); 2.5126 (46.7); 2.5083 (93.0); 2.5038 (120.1); 2.4992 (86.5); 2.4948 (42.0); 2.3351 (0.5); 2.3307 (0.7); 2.3260 (0.5); 2.1189 (0.4); 2.0872 (1.9); 1.6459 (11.6); 1.6285 (11.6); 1.3971 (9.8); 1.2993 (0.4); 1.2593 (0.6); 1.2342 (1.2); 1.1782 (0.3); 1.1414 (1.0); -0.0002 (1.9) | |
| I-111 | 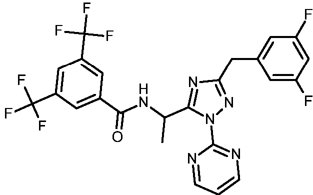 (reference compound) | ¹H-NMR(400.2 MHz, d₆-DMSO): | 557.1 [M+H]⁺ |
| | | δ= 11.3062 (3.6); 9.5815 (2.8); 9.5638 (2.9); 8.9756 (15.5); 8.9634 (16.0); 8.5882 (6.9); 8.4470 (3.0); 8.3944 (10.3); 8.3091 (4.5); 7.6171 (4.1); 7.6049 (7.8); 7.5928 (4.0); 7.0968 (0.6); 7.0909 (1.3); 7.0851 (0.9); 7.0734 (1.1); 7.0676 (2.6); 7.0618 (2.4); 7.0563 (1.0); 7.0434 (4.7); 7.0243 (4.6); 7.0090 (0.7); 6.0364 (0.4); 6.0194 (2.1); 6.0019 (3.3); 5.9843 (2.1); 5.9672 (0.4); 4.1295 (13.7); 3.3335 (81.8); 2.6793 (0.5); 2.6747 (0.7); 2.6702 (0.5); 2.5282 (2.0); 2.5235 (3.3); 2.5147 (43.2); 2.5104 (85.9); 2.5058 (110.2); 2.5013 (78.5); 2.4969 (37.5); 2.3372 (0.5); 2.3326 (0.7); 2.3281 (0.5); 1.6689 (11.4); 1.6515 (11.3); -0.0002 (2.0) | |
| I-112 | 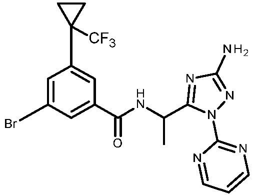 | ¹H-NMR(400.2 MHz, d₆-DMSO): | 498.0 [M+H]⁺ |
| | | δ= 9.1529 (2.4); 9.1387 (2.1); 8.8562 (7.4); 8.8475 (5.3); 8.8447 (5.2); 8.3158 (0.6); 8.0284 (4.1); 7.8748 (4.1); 7.7902 (4.3); 7.4427 (2.7); 7.4311 (3.4); 5.9950 (1.7); 5.9812 (2.1); 5.9645 (1.3); 5.7276 (6.7); 3.7913 (0.3); 3.7062 (0.4); 3.6482 (0.8); 3.3259 (321.5); 2.6734 (3.6); 2.5056 (581.4); 2.3323 (3.5); 2.0158 (0.3); 1.8007 (0.4); 1.7882 (0.5); 1.7369 (4.3); 1.6514 (0.4); 1.5846 (8.0); 1.5688 (6.6); 1.4241 (0.6); 1.3635 (6.4); 1.2984 (0.5); 1.2273 (16.0); 1.1222 (0.4); 0.8827 (0.3); 0.0112 (18.8); 0.0067 (23.1); 0.0031 (30.7); 0.0005 (34.2) | |
| I-113 | 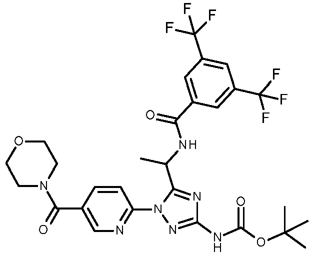 (reference compound) | ¹H-NMR(400.2 MHz, d₆-DMSO): | 658.4 [M+H]⁺ |
| | | δ= 9.9592 (1.5); 9.5816 (0.6); 9.5645 (0.7); 8.5796 (1.0); 8.5742 (1.1); 8.4747 (2.2); 8.3255 (1.0); 8.3155 (0.4); 8.1330 (0.6); 8.1273 (0.6); 8.1119 (0.7); 8.1062 (0.7); 7.8591 (1.0); 7.8378 (0.9); 6.1083 (0.4); 6.0912 (0.6); 6.0742 (0.4); 3.6879 (0.7); 3.6366 (0.6); 3.5562 (0.4); 3.3255 (200.3); 2.6754 (0.9); 2.6710 (1.2); 2.5063 (130.7); 2.5019 (168.9); 2.4976 (126.7); 2.3333 (0.8); 2.3288 (1.1); 2.3245 (0.8); 1.6635 (2.1); 1.6461 (2.1); 1.4294 (16.0); 0.1461 (0.5); 0.0077 (5.9); -0.0002 (101.2); -0.0083 (4.5); -0.1495 (0.5) | |
| I-114 | 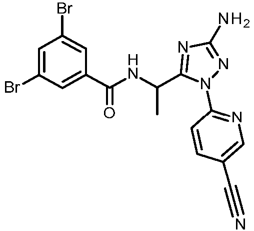 (reference compound) | ¹H-NMR(400.2 MHz, d₆-DMSO): | 491.9 [M+H]⁺ |
| | | δ= 9.2260 (2.5); 9.2083 (2.5); 8.9278 (4.4); 8.9237 (4.2); 8.9223 (4.2); 8.4439 (3.1); 8.4383 (3.0); 8.4222 (3.2); 8.4166 (3.2); 8.3160 (0.9); 8.0511 (8.5); 8.0470 (16.0); 8.0392 (6.0); 8.0352 (4.8); 8.0308 (2.2); 7.8200 (4.3); 7.8184 (4.4); 7.7983 (4.0); 7.7967 (4.1); 6.0706 (0.5); 6.0533 (1.9); 6.0359 (2.9); 6.0186 (1.9); 5.9863 (6.8); 3.3246 (222.5); 2.8908 (1.3); 2.7308 (1.2); 2.6795 (1.2); 2.6754 (2.4); 2.6709 (3.2); 2.6664 (2.4); 2.5242 (11.8); 2.5107 (199.8); 2.5064 (395.8); 2.5019 (518.0); 2.4974 (376.7); 2.4930 (184.5); 2.3333 (2.2); 2.3287 (3.1); 2.3242 (2.3); 2.0861 (4.6); 2.0745 (0.8); 1.5684 (9.7); 1.5511 (9.6); 1.3977 (0.5); 0.0079 (2.1); -0.0002 (58.1); -0.0086 (2.2) | |
| I-115 | 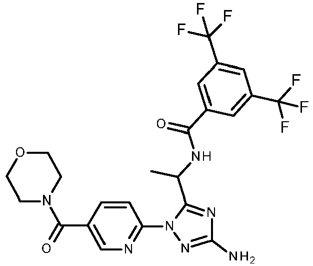 (reference compound) | ¹H-NMR(600.1 MHz, d₆-DMSO): | 558.0 [M+H]⁺ |
| | | δ= 9.4969 (3.4); 9.4855 (3.5); 8.5080 (16.0); 8.3178 (4.8); 8.0452 (3.8); 8.0414 (3.7); 8.0312 (4.3); 8.0273 (4.2); 7.7481 (5.4); 7.7342 (5.2); 6.1428 (0.6); 6.1311 (2.5); 6.1195 (3.9); 6.1078 (2.5); 6.0961 (0.6); 5.8624 (0.3); 5.8512 (0.4); 5.8490 (0.5); 5.8418 (0.6); 5.8124 (1.2); 5.7758 (0.5); 3.9265 (0.4); 3.8573 (0.5); 3.7087 (0.3); 3.6812 (0.6); 3.6055 (1.8); 3.5762 (1.6); 3.5686 (1.4); 3.5587 (1.3); 3.5328 (0.9); 3.5304 (0.9); 3.5140 (0.7); 3.5095 (0.6); 3.5038 (0.6); 3.4672 (0.5); 3.4532 (0.6); 3.4493 (0.6); 3.4422 (0.6); 3.4240 (0.7); 3.3896 (4.4); 3.3304 (159.4); 2.6168 (2.0); 2.6137 (2.7); 2.6107 (2.0); 2.5228 (5.5); 2.5197 (6.8); 2.5166 (6.6); 2.5078 (144.4); 2.5048 (319.2); 2.5018 (448.3); 2.4987 (322.7); 2.4957 (148.3); 2.4613 (0.5); 2.3917 (1.0); 2.3888 (2.0); 2.3855 (2.8); 2.3825 (1.9); 2.0729 (0.7); 1.6211 (12.8); 1.6096 (12.8); 1.2811 (0.3); 0.0054 (1.1); -0.0001 (43.8); -0.0057 (1.4) | |
| I-116 | 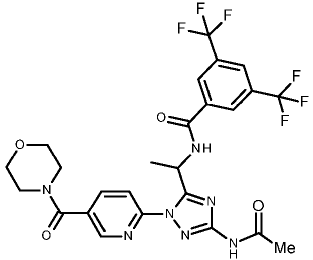 (reference compound) | ¹H-NMR(600.1 MHz, d₆-DMSO): | 600.2 [M+H]⁺ |
| | | δ= 9.5519 (4.6); 9.5405 (4.6); 8.5743 (6.8); 8.5706 (6.6); 8.4699 (13.7); 8.3221 (6.2); 8.3132 (1.8); 8.1312 (5.0); 8.1275 (4.4); 8.1172 (5.5); 8.1135 (5.2); 7.8635 (6.0); 7.8495 (5.6); 6.0950 (3.5); 6.0836 (4.8); 6.0720 (3.2); 3.6462 (2.6); 3.3180 (1400.0); 2.6167 (4.2); 2.6136 (6.0); 2.5226 (12.1); 2.5195 (15.1); 2.5164 (15.0); 2.5076 (298.5); 2.5046 (648.8); 2.5016 (900.6); 2.4985 (642.6); 2.4956 (292.3); 2.3854 (6.1); 2.0624 (2.8); 1.6712 (15.9); 1.6595 (16.0); 0.0054 (2.4); -0.0001 (93.0); -0.0057 (3.5) | |
| I-117 | 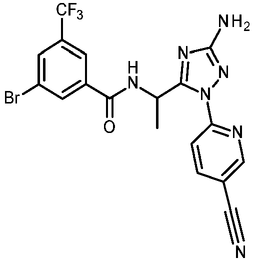 (reference compound) | ¹H-NMR(400.2 MHz, d₆-DMSO): | 482.0 [M+H]⁺ |
| | | δ= 9.3758 (4.3); 9.3583 (4.4); 8.9300 (7.6); 8.9260 (7.2); 8.9246 (7.0); 8.9087 (0.3); 8.4451 (5.1); 8.4395 (4.9); 8.4234 (5.3); 8.4178 (5.2); 8.3569 (7.8); 8.3156 (0.9); 8.1991 (7.9); 8.1793 (7.5); 8.1447 (0.3); 7.8230 (7.2); 7.8026 (6.7); 7.8012 (7.1); 6.1054 (0.8); 6.0888 (3.1); 6.0715 (4.7); 6.0541 (3.1); 6.0371 (0.8); 5.9872 (12.0); 3.5998 (0.6); 3.3255 (365.2); 2.8912 (1.6); 2.7314 (1.4); 2.6758 (2.1); 2.6713 (2.8); 2.6669 (2.1); 2.5905 (0.4); 2.5068 (321.5); 2.5023 (420.9); 2.4979 (321.7); 2.3336 (1.9); 2.3292 (2.6); 2.3247 (1.9); 2.0864 (6.1); 1.8074 (0.3); 1.7894 (0.4); 1.5925 (16.0); 1.5752 (15.8); 1.4682 (0.3); 1.4506 (1.8); 1.2347 (0.9); 0.8686 (1.3); 0.1459 (1.2); 0.0079 (13.5); -0.0002 (268.7); -0.0083 (13.1); -0.1495 (1.2) | |
| I-118 | 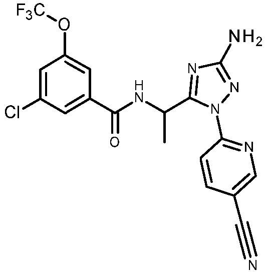 (reference compound) | ¹H-NMR(600.1 MHz, d₆-DMSO): | 452.2 [M+H]⁺ |
| | | δ= 9.2816 (3.8); 9.2700 (4.2); 9.2581 (0.5); 9.2491 (0.5); 9.2378 (0.4); 8.9223 (6.2); 8.9214 (6.1); 8.9187 (6.1); 8.9025 (0.7); 8.8988 (0.8); 8.4567 (0.4); 8.4530 (0.7); 8.4494 (0.4); 8.4385 (4.8); 8.4348 (4.3); 8.4240 (4.2); 8.4203 (4.1); 8.1051 (0.5); 8.0894 (0.7); 8.0744 (0.5); 8.0166 (4.7); 8.0140 (7.9); 8.0114 (4.5); 7.9906 (0.7); 7.9880 (0.5); 7.9703 (0.8); 7.8168 (6.1); 7.8024 (5.9); 7.7867 (13.2); 7.7678 (0.8); 7.7568 (0.9); 7.7521 (0.9); 7.7373 (0.4); 7.7197 (0.6); 7.2954 (0.3); 7.2537 (0.4); 6.3510 (0.3); 6.0723 (0.8); 6.0610 (2.8); 6.0494 (4.3); 6.0431 (1.0); 6.0378 (2.8); 6.0321 (0.8); 6.0264 (0.7); 6.0209 (0.5); 5.9824 (11.3); 5.8309 (0.4); 4.6634 (0.4); 3.7133 (0.5); 3.7104 (0.3); 3.7044 (0.4); 3.6776 (0.3); 3.6758 (0.4); 3.6671 (0.4); 3.6469 (1.8); 3.4700 (0.3); 3.4621 (0.3); 3.3168 (149.9); 2.6163 (2.3); 2.6133 (3.1); 2.6103 (2.2); 2.5543 (0.5); 2.5517 (0.5); 2.5223 (6.8); 2.5192 (8.4); 2.5161 (8.7); 2.5042 (366.1); 2.5012 (497.7); 2.4983 (370.2); 2.3881 (2.2); 2.3852 (2.9); 2.3822 (2.1); 1.5941 (1.2); 1.5827 (15.6); 1.5711 (16.0); 1.5602 (1.6); 1.2352 (0.6); 0.0053 (2.4); -0.0001 (75.1); -0.0056 (2.4); - 0.1002 (0.3) | |
| | | ¹³C-NMR(150.9 MHz, d₆-DMSO): | |
| | | δ= 163.1414 (4.1); 163.0940 (4.1); 162.2981 (0.6); 159.3685 (3.9); 152.2063 (3.2); 151.9562 (3.7); 148.8020 (2.0); 143.2362 (3.6); 137.3235 (3.2); 134.5441 (3.7); 126.8575 (5.0); 124.5384 (2.8); 120.8941 (0.9); 119.1850 (1.0); 119.1101 (3.7); 116.9726 (2.3); 114.1941 (4.0); 105.7558 (3.7); 72.1199 (0.8); 44.6020 (4.7); 40.2358 (48.0); 40.1162 (254.4); 39.9775 (757.0); 39.8386 (1484.2); 39.6995 (1737.4); 39.5604 (1471.7); 39.4213 (736.5); 39.2822 (239.0); 18.8378 (7.0); 0.2820 (11.2) | |
| I-119 | 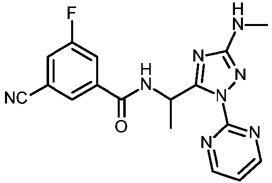 (reference compound) | ¹H-NMR(400.2 MHz, d₆-DMSO): | 367.1 [M+H]⁺ |
| | | δ= 9.2437 (3.5); 9.2259 (3.5); 8.8719 (11.2); 8.8600 (11.5); 8.1537 (7.8); 8.1000 (0.4); 8.0634 (3.0); 8.0428 (3.0); 7.9747 (3.0); 7.9522 (3.2); 7.7734 (0.4); 7.4530 (3.1); 7.4410 (5.9); 7.4291 (3.1); 6.2408 (3.1); 6.2285 (3.2); 6.0413 (0.6); 6.0240 (2.4); 6.0068 (3.7); 5.9894 (2.5); 5.9727 (0.7); 5.7553 (1.2); 3.6475 (0.5); 3.5683 (0.4); 3.3248 (60.9); 2.8912 (0.9); 2.7489 (15.7); 2.7367 (16.0); 2.6709 (1.0); 2.5029 (152.7); 2.3290 (1.0); 1.5911 (13.7); 1.5739 (13.7); -0.0002 (1.2) | |
| I-120 | 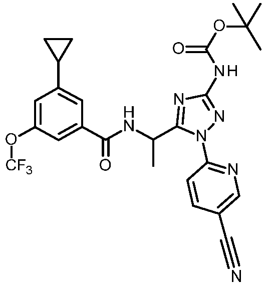 | ¹H-NMR(400.2 MHz, d₆-DMSO): | 356.2 [M+H]⁺ |
| | | δ= 10.0715 (1.4); 9.1846 (0.6); 9.1681 (0.6); 9.0050 (0.9); 9.0006 (1.0); 8.5427 (0.7); 8.5371 (0.6); 8.5212 (0.7); 8.5156 (0.7); 7.9496 (1.0); 7.9278 (0.9); 7.5113 (1.3); 7.5076 (1.3); 7.2791 (0.8); 6.0457 (0.4); 6.0284 (0.6); 6.0118 (0.4); 3.3252 (81.1); 2.6754 (0.3); 2.6711 (0.4); 2.6667 (0.3); 2.5108 (28.5); 2.5066 (55.1); 2.5021 (71.0); 2.4976 (51.7); 2.4933 (25.7); 2.3289 (0.4); 2.0352 (0.5); 1.6269 (2.1); 1.6095 (2.1); 1.4363 (16.0); 1.0386 (0.8); 1.0332 (0.9); 1.0227 (0.5); 1.0178 (0.8); 1.0122 (0.8); 0.7969 (0.3); 0.7861 (0.9); 0.7812 (0.9); 0.7739 (0.9); 0.7690 (1.0); - 0.0002 (0.3) | |
| I-121 | 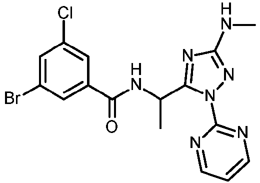 (reference compound) | ¹H-NMR(400.2 MHz, d₆-DMSO): | 438.0 [M+H]⁺ |
| | | δ= 9.1953 (3.4); 9.1773 (3.4); 8.9063 (0.6); 8.8984 (0.7); 8.8944 (0.8); 8.8863 (0.7); 8.8687 (15.4); 8.8567 (15.6); 8.3156 (0.8); 7.9775 (4.4); 7.9736 (7.6); 7.9699 (5.0); 7.9475 (0.5); 7.9145 (4.3); 7.9100 (8.0); 7.9056 (4.8); 7.8712 (5.2); 7.8671 (6.9); 7.8631 (4.1); 7.8463 (0.4); 7.4961 (0.4); 7.4839 (0.4); 7.4523 (4.2); 7.4402 (7.8); 7.4282 (4.0); 6.2490 (1.0); 6.2367 (3.2); 6.2242 (3.2); 6.2123 (1.0); 6.0136 (0.6); 5.9966 (2.4); 5.9790 (3.8); 5.9614 (2.4); 5.9442 (0.6); 5.7556 (1.5); 3.3229 (321.3); 2.9028 (1.2); 2.8836 (0.6); 2.7471 (16.0); 2.7345 (16.0); 2.6753 (2.5); 2.6707 (3.3); 2.6664 (2.4); 2.5104 (206.0); 2.5063 (392.4); 2.5018 (502.8); 2.4973 (366.3); 2.4931 (183.4); 2.3329 (2.3); 2.3287 (3.1); 2.3241 (2.3); 1.8290 (1.1); 1.6133 (0.8); 1.5961 (0.9); 1.5726 (13.3); 1.5553 (13.2); 1.4327 (0.6); 1.2350 (0.4); 0.1459 (1.6); 0.0078 (17.9); -0.0002 (355.9); -0.0084 (16.5); - 0.1497 (1.6) | |
| I-122 | 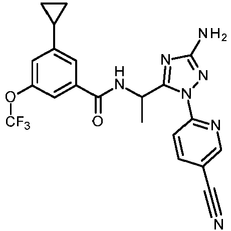 | ¹H-NMR(400.2 MHz, d6-DMSO): | 458.1 [M+H]⁺ |
| | | δ= 20.0075 (0.4); 15.9938 (0.4); 9.1085 (3.9); 9.0910 (4.1); 9.0668 (0.4); 9.0548 (0.4); 8.9206 (6.6); 8.9189 (7.2); 8.9151 (7.1); 8.9132 (6.7); 8.7024 (0.7); 8.4411 (5.8); 8.4355 (5.5); 8.4194 (6.0); 8.4138 (6.0); 8.3310 (0.4); 8.3151 (9.5); 7.9508 (0.4); 7.8183 (7.0); 7.8166 (6.9); 7.7967 (6.8); 7.7949 (6.8); 7.5534 (8.6); 7.5497 (5.8); 7.5352 (5.8); 7.5084 (0.5); 7.4758 (0.4); 7.2761 (5.6); 6.0723 (0.7); 6.0572 (3.0); 6.0398 (4.7); 6.0219 (3.1); 6.0035 (0.8); 5.9668 (10.7); 3.9783 (1.2); 3.9157 (0.4); 3.8298 (0.4); 3.8105 (0.4); 3.6991 (0.4); 3.6054 (0.4); 3.5467 (0.4); 3.4749 (0.5); 3.4583 (0.6); 3.3856 (1.1); 3.3740 (1.5); 3.3241 (1617.6); 3.3001 (4.4); 3.2667 (0.4); 3.0884 (0.4); 2.9172 (0.4); 2.8908 (3.0); 2.8689 (0.4); 2.8183 (0.4); 2.7786 (0.5); 2.7460 (0.6); 2.7307 (2.6); 2.6796 (5.5); 2.6752 (11.3); 2.6706 (15.4); 2.6661 (11.4); 2.6616 (5.5); 2.6359 (1.1); 2.5934 (1.7); 2.5241 (56.2); 2.5193 (89.4); 2.5108 (905.2); 2.5063 (1819.6); 2.5017 (2375.8); 2.4971 (1675.2); 2.4925 (783.3); 2.3376 (4.8); 2.3331 (10.4); 2.3285 (14.5); 2.3239 (10.3); 2.3193 (4.6); 2.1173 (0.5); 2.0859 (1.8); 2.0743 (0.7); 2.0624 (1.6); 2.0535 (2.0); 2.0413 (3.5); 2.0291 (2.0); 2.0203 (2.0); 2.0073 (1.0); 1.6912 (0.4); 1.6368 (0.4); 1.5855 (16.0); 1.5681 (15.9); 1.3979 (2.0); 1.3670 (0.4); 1.2478 (0.6); 1.2356 (0.9); 1.2291 (0.9); 1.2107 (0.4); 1.1399 (0.8); 1.0667 (0.4); 1.0531 (1.6); 1.0415 (5.9); 1.0362 (6.2); 1.0253 (2.9); 1.0209 (6.2); 1.0153 (6.3); 1.0045 (2.1); 0.9651 (0.5); 0.8416 (0.4); 0.8018 (2.5); 0.7921 (5.6); 0.7888 (5.7); 0.7797 (5.8); 0.7759 (5.4); 0.7639 (2.3); 0.7206 (0.4); 0.1458 (16.2); 0.1373 (0.7); 0.0897 (0.5); 0.0749 (0.5); 0.0653 (0.5); 0.0617 (0.7); 0.0566 (0.6); 0.0529 (0.8); 0.0492 (0.9); 0.0455 (1.1); 0.0434 (1.2); 0.0426 (1.2); 0.0412 (1.2); 0.0390 (0.9); 0.0382 (1.0); 0.0360 (1.7); 0.0331 (2.6); 0.0316 (2.8); 0.0309 (2.8); 0.0301 (2.8); 0.0294 (2.9); 0.0279 (3.4); 0.0272 (3.7); 0.0265 (3.9); 0.0251 (4.7); 0.0243 (4.3); 0.0220 (7.0); 0.0206 (8.3); 0.0198 (9.1); 0.0176 (12.3); 0.0169 (13.2); 0.0162 (14.1); 0.0079 (158.6); -0.0002 (4100.9); -0.0086 (145.5); -0.0238 (4.8); - 0.0398 (1.7); -0.0480 (1.0); -0.0689 (0.5); -0.0799 (0.4); -0.0959 (0.5); -0.1019 (0.4); -0.1041 (0.4); - 0.1497 (16.3); -0.1575 (0.7) | |
| I-123 | 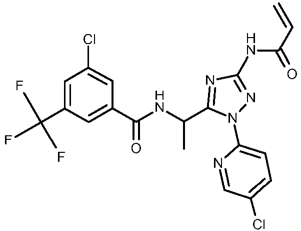 (reference compound) | ¹H-NMR(600.1 MHz, d₆-DMSO): | 499.1 [M+H]⁺ |
| | | δ= 10.9231 (3.2); 9.3798 (3.7); 9.3687 (3.8); 9.3203 (0.4); 9.3085 (0.4); 8.6025 (0.4); 8.5973 (6.1); 8.5965 (5.9); 8.5930 (6.2); 8.5922 (5.8); 8.5134 (0.6); 8.5126 (0.6); 8.5091 (0.6); 8.5083 (0.6); 8.2060 (4.6); 8.2016 (4.4); 8.1914 (5.2); 8.1871 (5.4); 8.1483 (6.0); 8.1397 (0.7); 8.1001 (0.6); 8.0915 (5.9); 8.0857 (0.9); 8.0812 (0.6); 8.0701 (5.7); 7.8237 (6.6); 7.8231 (6.3); 7.8092 (6.4); 7.8084 (6.0); 7.6987 (0.7); 7.6841 (0.6); 7.6833 (0.6); 6.5158 (0.4); 6.4913 (0.6); 6.4743 (0.5); 6.2977 (4.2); 6.2948 (4.0); 6.2693 (3.2); 6.2663 (3.1); 5.9891 (0.5); 5.9850 (0.6); 5.9735 (2.7); 5.9621 (4.1); 5.9507 (2.7); 5.9391 (0.6); 5.7950 (4.4); 5.7920 (3.7); 5.7780 (3.3); 5.7750 (3.9); 4.1052 (1.2); 4.0965 (3.7); 4.0877 (3.8); 4.0789 (1.3); 3.3363 (0.3); 3.3227 (118.7); 3.1746 (16.0); 3.1659 (15.7); 2.6182 (0.5); 2.6151 (0.7); 2.6121 (0.5); 2.5241 (1.6); 2.5211 (1.8); 2.5179 (1.8); 2.5092 (35.2); 2.5061 (75.3); 2.5031 (105.6); 2.5000 (78.0); 2.4970 (36.1); 2.3900 (0.4); 2.3870 (0.6); 2.3840 (0.4); 1.6477 (13.9); 1.6361 (14.0); 1.6248 (0.6); 1.6132 (0.4); 1.5829 (1.5); 1.5714 (1.5) | |
| | | ¹³C-NMR(150.9 MHz, d₆-DMSO): | |
| | | δ= 163.2528 (3.4); 163.1075 (0.4); 162.9138 (0.4); 162.4435 (0.5); 157.8891 (3.0); 157.7948 (0.5); 155.4866 (2.7); 148.8173 (3.4); 146.7468 (4.5); 146.3373 (0.5); 139.7420 (4.9); 139.3769 (0.5); 136.7866 (0.4); 136.5526 (3.9); 134.5459 (4.0); 131.5733 (3.1); 131.2155 (1.4); 130.9970 (1.4); 130.9185 (1.6); 130.7783 (0.5); 129.8412 (3.2); 128.3004 (2.6); 128.2539 (1.6); 125.9299 (0.6); 124.1203 (1.2); 122.9941 (1.8); 122.9692 (1.7); 122.3103 (1.2); 120.5007 (0.4); 117.7137 (4.8); 116.2207 (0.6); 48.7687 (3.4); 44.0829 (0.6); 44.0107 (4.8); 40.2360 (11.0); 40.1162 (57.8); 39.9775 (172.6); 39.8386 (338.8); 39.6995 (397.0); 39.5604 (336.4); 39.4213 (168.4); 39.2822 (54.7); 18.9961 (0.7); 18.7947 (7.0) | |
| I-124 | 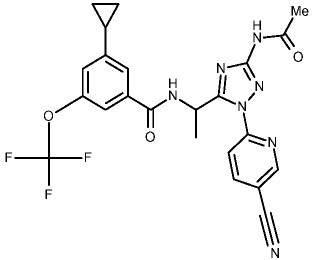 | ¹H-NMR(400.2 MHz, d₆-DMSO): | 500.1 [M+H]⁺ |
| | | δ= 10.7177 (1.9); 9.1690 (4.2); 9.1526 (4.3); 9.0193 (0.3); 9.0064 (6.7); 9.0047 (7.2); 9.0009 (7.3); 8.9992 (6.9); 8.5503 (5.7); 8.5447 (5.4); 8.5287 (6.0); 8.5231 (6.0); 8.3157 (0.6); 7.9627 (6.4); 7.9614 (6.6); 7.9413 (6.0); 7.9398 (6.2); 7.5144 (9.1); 7.5107 (8.3); 7.5054 (6.0); 7.5025 (6.2); 7.2754 (6.1); 6.0581 (0.7); 6.0411 (3.2); 6.0241 (4.6); 6.0071 (3.0); 5.9898 (0.6); 5.9690 (0.4); 5.8168 (0.4); 3.3244 (155.9); 2.6761 (1.2); 2.6715 (1.7); 2.6669 (1.2); 2.6624 (0.6); 2.5249 (5.2); 2.5201 (8.6); 2.5115 (97.6); 2.5071 (198.3); 2.5025 (260.8); 2.4980 (187.8); 2.4935 (90.7); 2.3385 (0.6); 2.3339 (1.2); 2.3294 (1.7); 2.3248 (1.2); 2.3204 (0.6); 2.0745 (6.1); 2.0680 (6.6); 2.0557 (4.9); 2.0472 (3.6); 2.0345 (4.5); 2.0258 (2.0); 2.0220 (2.6); 2.0136 (2.3); 2.0009 (1.2); 1.6395 (16.0); 1.6221 (15.9); 1.5866 (0.7); 1.5693 (0.6); 1.3469 (1.6); 1.0513 (2.2); 1.0400 (6.2); 1.0345 (6.8); 1.0307 (3.6); 1.0240 (3.5); 1.0191 (6.4); 1.0136 (6.4); 1.0032 (2.6); 0.7917 (2.8); 0.7809 (7.2); 0.7758 (7.2); 0.7686 (6.6); 0.7635 (7.6); 0.7520 (2.2); 0.1459 (1.5); 0.0080 (14.0); -0.0001 (361.3); - 0.0085 (13.8); -0.1496 (1.5) | |
| I-126 | 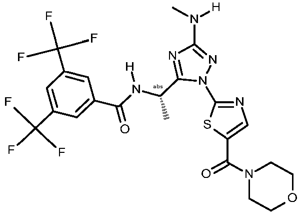 (reference compound) | ¹H-NMR(600.1 MHz, d₆-DMSO): | 578.1 [M+H]⁺ |
| | | δ= 9.5983 (3.5); 9.5871 (3.6); 8.5480 (11.2); 8.3454 (4.9); 8.0016 (15.8); 6.6601 (1.0); 6.6521 (3.2); 6.6437 (3.2); 6.6354 (1.0); 6.0209 (0.5); 6.0094 (2.4); 5.9979 (3.7); 5.9864 (2.4); 5.9748 (0.5); 3.6635 (6.1); 3.6531 (12.0); 3.6486 (11.6); 3.3193 (29.4); 2.7404 (15.9); 2.7321 (16.0); 2.6179 (0.4); 2.6151 (0.6); 2.6120 (0.4); 2.5240 (1.1); 2.5209 (1.3); 2.5178 (1.3); 2.5089 (27.2); 2.5060 (57.7); 2.5029 (80.0); 2.4999 (58.7); 2.4970 (27.6); 2.3899 (0.4); 2.3868 (0.5); 2.3838 (0.4); 2.0757 (3.1); 1.6223 (12.5); 1.6106 (12.5); 0.0053 (0.4); -0.0001 (12.5); -0.0057 (0.4) | |
| | | ¹³C-NMR(150.9 MHz, d₆-DMSO): | |
| | | δ= 164.1849 (4.3); 163.2775 (3.2); 160.2210 (3.6); 159.8269 (3.5); 158.4937 (4.1); 141.9436 (3.6); 135.9002 (3.7); 131.0035 (0.8); 130.7826 (2.6); 130.5621 (2.6); 130.3417 (0.8); 129.1643 (4.1); 128.5089 (2.7); 125.9904 (0.8); 125.3732 (1.0); 124.1812 (2.2); 122.3721 (2.2); 120.5633 (0.5); 66.1823 (6.1); 43.8331 (4.4); 40.2357 (9.7); 40.1163 (52.1); 39.9775 (155.2); 39.8386 (304.3); 39.6995 (356.3); 39.5604 (301.7); 39.4213 (151.0); 39.2822 (49.0); 29.3257 (6.4); 18.4308 (7.0); 1.3248 (0.6); 0.2743 (2.3) | |
| I-127 | 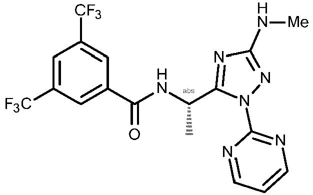 (reference compound) | ¹H-NMR(600.1 MHz, d₆-DMSO): | 460.1 [M+H]⁺ |
| | | δ= 9.5032 (3.1); 9.4913 (3.2); 8.8732 (15.1); 8.8652 (15.5); 8.4706 (10.8); 8.3118 (4.7); 7.4492 (4.0); 7.4411 (7.6); 7.4331 (4.0); 6.2383 (0.9); 6.2300 (3.0); 6.2216 (3.0); 6.2132 (0.9); 6.0749 (0.5); 6.0634 (2.3); 6.0517 (3.6); 6.0400 (2.3); 6.0284 (0.5); 3.3204 (37.7); 2.7501 (15.9); 2.7417 (16.0); 2.6184 (0.3); 2.6154 (0.4); 2.6124 (0.3); 2.5244 (1.0); 2.5213 (1.2); 2.5183 (1.2); 2.5093 (24.8); 2.5064 (52.1); 2.5034 (71.7); 2.5004 (53.6); 2.4975 (25.8); 2.3873 (0.4); 2.0762 (0.4); 1.6178 (12.6); 1.6063 (12.6); -0.0001 (1.3) | |
| I-128 | 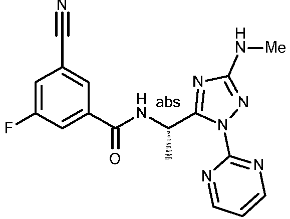 (reference compound) | ¹H-NMR(400.2 MHz, d₆-DMSO): | 367.1 [M+H]⁺ |
| | | δ= 9.2456 (3.0); 9.2278 (3.1); 8.8722 (15.6); 8.8601 (16.0); 8.3160 (0.3); 8.1539 (7.7); 8.1508 (4.9); 8.0707 (1.9); 8.0675 (2.2); 8.0644 (2.4); 8.0614 (2.1); 8.0498 (2.0); 8.0466 (2.3); 8.0436 (2.3); 8.0405 (2.0); 7.9796 (2.0); 7.9761 (2.3); 7.9736 (2.2); 7.9700 (1.8); 7.9559 (2.2); 7.9523 (2.5); 7.9499 (2.2); 7.9462 (1.8); 7.4537 (4.2); 7.4416 (7.9); 7.4295 (4.1); 6.2554 (0.9); 6.2432 (2.9); 6.2306 (2.9); 6.2183 (0.9); 6.0408 (0.5); 6.0235 (2.3); 6.0060 (3.6); 5.9885 (2.3); 5.9711 (0.5); 3.3283 (156.2); 2.7484 (15.6); 2.7358 (15.6); 2.6763 (0.8); 2.6716 (1.1); 2.6673 (0.8); 2.5250 (3.4); 2.5114 (66.6); 2.5072 (132.0); 2.5027 (170.7); 2.4982 (124.0); 2.4939 (61.7); 2.3339 (0.8); 2.3295 (1.1); 2.3251 (0.8); 2.0750 (0.9); 1.5908 (12.9); 1.5735 (12.8); -0.0002 (3.0) | |
| I-129 | 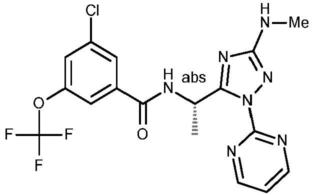 (reference compound) | ¹H-NMR(400.2 MHz, d₆-DMSO): | 442.1 [M+H]⁺ |
| | | δ= 9.2669 (2.8); 9.2491 (2.9); 8.8683 (15.6); 8.8562 (16.0); 8.3162 (0.5); 7.9719 (4.0); 7.9679 (6.2); 7.9640 (4.2); 7.7700 (4.1); 7.7496 (4.3); 7.7471 (4.3); 7.4503 (4.2); 7.4382 (7.7); 7.4261 (4.0); 6.2498 (0.8); 6.2376 (2.7); 6.2249 (2.7); 6.2126 (0.8); 6.0278 (0.4); 6.0104 (2.1); 5.9929 (3.4); 5.9753 (2.1); 5.9581 (0.4); 3.3259 (157.0); 2.7486 (14.9); 2.7360 (14.9); 2.6804 (0.6); 2.6760 (1.2); 2.6715 (1.6); 2.6669 (1.1); 2.5250 (4.9); 2.5201 (7.8); 2.5115 (93.6); 2.5071 (186.6); 2.5025 (240.4); 2.4980 (171.2); 2.4935 (82.2); 2.3339 (1.1); 2.3293 (1.5); 2.3248 (1.1); 2.3203 (0.5); 1.5877 (11.8); 1.5704 (11.8); -0.0002 (4.9) | |
| I-130 | 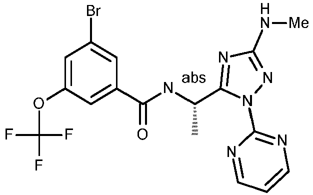 (reference compound) | ¹H-NMR(400.2 MHz, d₆-DMSO): | 486.0 [M+H]⁺ |
| | | δ= 9.2697 (2.9); 9.2518 (2.9); 8.8697 (15.6); 8.8576 (16.0); 8.1056 (4.2); 8.1019 (6.7); 8.0982 (4.2); 7.8751 (4.3); 7.7780 (4.1); 7.7754 (4.4); 7.7730 (3.8); 7.4515 (4.2); 7.4394 (7.7); 7.4273 (4.1); 6.2514 (0.8); 6.2391 (2.7); 6.2265 (2.8); 6.2141 (0.8); 6.0274 (0.4); 6.0103 (2.2); 5.9928 (3.4); 5.9752 (2.2); 5.9579 (0.4); 3.3286 (57.8); 2.7504 (15.2); 2.7378 (15.0); 2.6773 (0.4); 2.6729 (0.6); 2.6684 (0.4); 2.5263 (1.8); 2.5128 (35.3); 2.5084 (69.6); 2.5039 (88.9); 2.4993 (63.2); 2.4949 (30.2); 2.3351 (0.4); 2.3307 (0.5); 2.3260 (0.4); 1.5874 (12.0); 1.5701 (11.9); -0.0002 (1.8) | |
| I-131 | 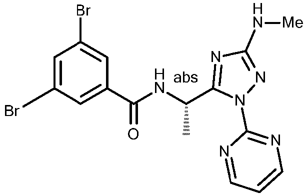 (reference compound) | ¹H-NMR(400.2 MHz, d₆-DMSO): | 481.9 [M+H]⁺ |
| | | δ = 9.1973 (2.7); 9.1795 (2.7); 8.8696 (12.7); 8.8575 (13.0); 8.0237 (2.5); 8.0197 (5.5); 8.0156 (5.4); 8.0044 (16.0); 8.0002 (10.1); 7.4531 (3.4); 7.4411 (6.3); 7.4290 (3.3); 6.2510 (0.7); 6.2393 (2.1); 6.2265 (2.1); 6.2151 (0.7); 6.0124 (0.4); 5.9953 (1.9); 5.9777 (3.0); 5.9600 (2.0); 5.9425 (0.4); 3.3280 (74.1); 2.7481 (11.5); 2.7358 (11.4); 2.6764 (0.6); 2.6719 (0.8); 2.6675 (0.6); 2.5252 (2.5); 2.5074 (100.2); 2.5029 (127.0); 2.4985 (91.7); 2.3341 (0.6); 2.3298 (0.8); 2.3253 (0.6); 1.5717 (10.9); 1.5543 (10.8); 1.0562 (0.6); -0.0002 (2.4) | |
| I-132 | 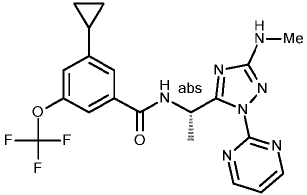 | ¹H-NMR(400.2 MHz, d₆-DMSO): | 448.1 [M+H]⁺ |
| | | δ= 9.0872 (3.3); 9.0692 (3.4); 8.8668 (14.4); 8.8548 (14.6); 7.4982 (11.1); 7.4955 (10.8); 7.4477 (4.1); 7.4357 (7.2); 7.4236 (3.7); 7.2669 (5.2); 6.2293 (1.0); 6.2169 (3.0); 6.2043 (3.0); 6.1922 (1.0); 6.0184 (0.5); 6.0014 (2.3); 5.9838 (3.6); 5.9662 (2.4); 5.9489 (0.5); 3.3300 (66.2); 3.0182 (0.5); 3.0061 (0.5); 2.7499 (16.0); 2.7373 (15.9); 2.6729 (0.6); 2.6684 (0.4); 2.5081 (69.7); 2.5038 (87.9); 2.4995 (64.5); 2.3305 (0.5); 2.3260 (0.4); 2.0626 (0.7); 2.0501 (1.4); 2.0417 (1.6); 2.0293 (2.8); 2.0169 (1.7); 2.0085 (1.5); 1.9958 (0.8); 1.5879 (13.1); 1.5706 (13.1); 1.0460 (1.6); 1.0348 (4.9); 1.0293 (5.2); 1.0185 (2.8); 1.0139 (4.9); 1.0085 (4.8); 0.9981 (1.9); 0.8575 (0.5); 0.8398 (0.6); 0.7930 (2.1); 0.7821 (5.8); 0.7774 (5.7); 0.7701 (5.3); 0.7651 (5.9); 0.7535 (1.6); -0.0002 (1.4) | |
| I-133 | 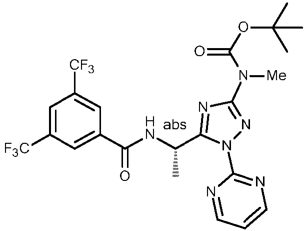 (reference compound) | ¹H-NMR(400.2 MHz, d₆-DMSO): | 560.0 [M+H]⁺ |
| | | δ= 9.6187 (0.5); 9.6013 (0.6); 8.9933 (2.8); 8.9812 (2.8); 8.4542 (1.9); 8.3265 (0.8); 7.6313 (0.7); 7.6191 (1.4); 7.6070 (0.7); 6.0630 (0.4); 6.0456 (0.6); 6.0280 (0.4); 5.7595 (0.4); 3.3327 (18.0); 3.2136 (7.1); 2.5258 (0.5); 2.5210 (0.8); 2.5124 (11.8); 2.5080 (23.8); 2.5034 (31.2); 2.4988 (22.4); 2.4943 (10.7); 1.6749 (2.1); 1.6575 (2.1); 1.3208 (16.0); -0.0002 (5.6) | |
| I-134 | 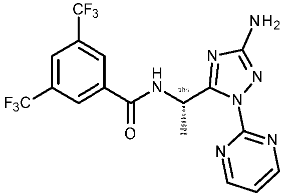 (reference compound) | ¹H-NMR(600.1 MHz, d₆-DMSO): | 446.1 [M+H]⁺ |
| | | δ= 9.4998 (0.7); 9.4880 (0.7); 8.8731 (0.3); 8.8620 (1.1); 8.8543 (1.1); 8.4770 (2.7); 8.3123 (1.2); 7.4361 (0.5); 6.3516 (0.3); 6.0516 (0.4); 6.0401 (0.5); 6.0285 (0.4); 3.6473 (0.5); 3.5683 (16.0); 2.5238 (0.7); 2.5208 (0.9); 2.5177 (0.9); 2.5087 (19.3); 2.5058 (39.8); 2.5028 (54.6); 2.4999 (40.8); 1.6155 (2.4); 1.6040 (2.3); -0.0001 (6.7) | |
| I-135 | 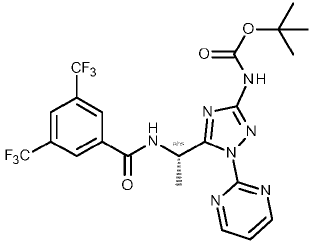 (reference compound) | ¹H-NMR(400.2 MHz, d₆-DMSO): | 546.2 [M+H]⁺ |
| | | δ= 9.9105 (1.3); 9.5857 (0.6); 9.5684 (0.6); 8.9599 (2.9); 8.9478 (3.0); 8.4393 (2.0); 8.3110 (0.9); 7.5892 (0.8); 7.5771 (1.4); 7.5650 (0.8); 6.0305 (0.4); 6.0132 (0.6); 5.9958 (0.4); 3.3263 (15.8); 2.5259 (0.4); 2.5125 (7.8); 2.5082 (15.6); 2.5036 (20.4); 2.4991 (14.7); 2.4947 (7.2); 1.6635 (2.1); 1.6461 (2.1); 1.4239 (16.0); 0.0079 (1.2); -0.0002 (29.0); -0.0086 (1.2) | |
| I-136 | 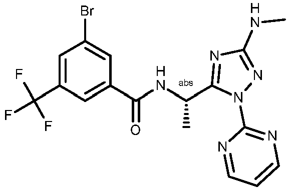 (reference compound) | ¹H-NMR(400.2 MHz, d₆-DMSO): | 472.0 [M+H]⁺ |
| | | δ= 9.3509 (3.1); 9.3330 (3.2); 8.8720 (15.5); 8.8599 (15.9); 8.3101 (5.9); 8.1598 (7.8); 7.4528 (4.2); 7.4407 (7.8); 7.4286 (4.0); 6.2514 (0.9); 6.2391 (3.0); 6.2265 (3.0); 6.2143 (0.9); 6.0486 (0.5); 6.0314 (2.3); 6.0138 (3.6); 5.9962 (2.3); 5.9792 (0.5); 3.3279 (91.3); 2.7498 (16.0); 2.7372 (15.8); 2.6766 (0.7); 2.6722 (1.0); 2.6679 (0.7); 2.5256 (2.9); 2.5120 (59.8); 2.5078 (115.9); 2.5033 (147.3); 2.4988 (106.7); 2.4947 (52.9); 2.3346 (0.7); 2.3301 (0.9); 2.3257 (0.7); 1.5954 (12.8); 1.5780 (12.8); -0.0001 (3.0) | |
| I-137 | 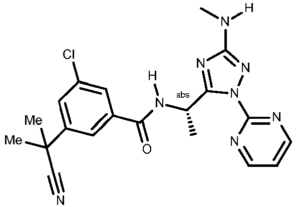 | ¹H-NMR(400.2 MHz, d₆-DMSO): | 425.1 [M+H]⁺ |
| | | δ= 9.1854 (0.9); 9.1675 (0.9); 8.8726 (3.9); 8.8605 (4.0); 7.8782 (2.9); 7.8750 (2.8); 7.7311 (1.2); 7.7267 (1.9); 7.7222 (1.0); 7.4527 (1.0); 7.4406 (2.0); 7.4286 (1.0); 6.2288 (0.8); 6.2161 (0.8); 6.0182 (0.6); 6.0006 (0.9); 5.9829 (0.6); 3.3294 (34.2); 2.7504 (4.1); 2.7379 (4.1); 2.5249 (0.7); 2.5114 (14.5); 2.5073 (27.6); 2.5029 (34.9); 2.4984 (25.0); 2.4942 (12.2); 1.7071 (16.0); 1.5967 (3.4); 1.5793 (3.3) | |
| I-138 | 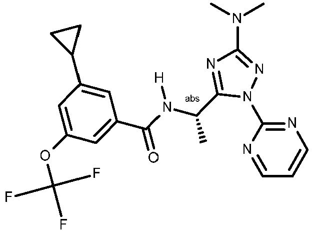 | ¹H-NMR(400.2 MHz, d₆-DMSO): | 462.2 [M+H]⁺ |
| | | δ= 9.1191 (0.7); 9.1008 (0.8); 8.8842 (3.5); 8.8721 (3.6); 7.4780 (1.1); 7.4658 (4.2); 7.4539 (1.0); 7.2665 (1.1); 5.9945 (0.5); 5.9769 (0.8); 5.9592 (0.5); 5.7561 (1.6); 3.3249 (15.9); 2.9437 (16.0); 2.5248 (0.7); 2.5115 (12.3); 2.5072 (23.8); 2.5028 (30.7); 2.4983 (22.2); 2.4940 (11.0); 2.0403 (0.4); 2.0280 (0.6); 2.0156 (0.4); 2.0072 (0.4); 1.6055 (3.0); 1.5881 (3.0); 1.0440 (0.4); 1.0335 (1.0); 1.0278 (1.1); 1.0181 (0.6); 1.0127 (1.0); 1.0070 (1.0); 0.9973 (0.4); 0.7899 (0.5); 0.7795 (1.1); 0.7763 (1.1); 0.7675 (1.1); 0.7634 (1.0); 0.7518 (0.4); 0.0076 (2.0); -0.0002 (40.6); -0.0084 (1.8) | |
| I-139 | 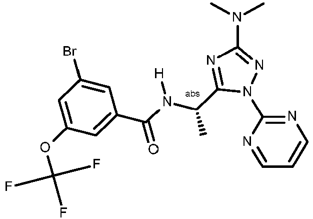 (reference compound) | ¹H-NMR(400.2 MHz, d₆-DMSO): | 502.0 [M+H]⁺ |
| | | δ= 9.3021 (0.7); 9.2841 (0.7); 8.8878 (3.8); 8.8757 (3.9); 8.0810 (1.0); 8.0772 (1.6); 8.0734 (1.0); 7.8721 (1.0); 7.7557 (0.9); 7.7531 (1.0); 7.7506 (0.9); 7.4825 (1.0); 7.4704 (1.9); 7.4583 (1.0); 6.0067 (0.5); 5.9892 (0.8); 5.9715 (0.5); 3.3276 (16.1); 2.9458 (16.0); 2.5261 (0.4); 2.5128 (8.0); 2.5084 (15.6); 2.5039 (19.9); 2.4993 (13.9); 2.4948 (6.5); 1.6076 (2.9); 1.5903 (2.9); 0.0077 (1.3); -0.0002 (27.2); -0.0087 (0.9) | |
| I-140 | 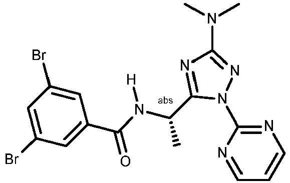 (reference compound) | ¹H-NMR(400.2 MHz, d₆-DMSO): | 496.0 [M+H]⁺ |
| | | δ= 9.2332 (0.7); 9.2153 (0.7); 8.8879 (3.5); 8.8758 (3.6); 8.0206 (0.8); 8.0163 (1.6); 8.0120 (1.2); 7.9872 (4.1); 7.9829 (3.2); 7.4846 (0.9); 7.4725 (1.8); 7.4604 (0.9); 5.9912 (0.5); 5.9736 (0.8); 5.9560 (0.6); 5.7564 (3.9); 3.3249 (11.4); 2.9448 (16.0); 2.5251 (0.6); 2.5118 (11.7); 2.5075 (22.7); 2.5030 (29.2); 2.4985 (21.1); 2.4942 (10.4); 1.5942 (3.0); 1.5768 (3.0); 0.0079 (0.8); -0.0002 (19.0); -0.0084 (0.7) | |
| I-141 | 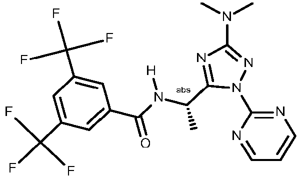 (reference compound) | ¹H-NMR(600.1 MHz, CD3CN): | 474.1 [M+H]⁺ |
| | | δ= 8.7959 (2.5); 8.7878 (2.5); 8.3201 (1.9); 8.1432 (0.8); 7.3240 (0.7); 7.3160 (1.4); 7.3079 (0.7); 6.1539 (0.5); 6.1416 (0.6); 6.1298 (0.5); 2.9874 (16.0); 2.1525 (12.4); 1.9490 (2.3); 1.9449 (4.5); 1.9408 (6.6); 1.9366 (4.5); 1.9325 (2.3); 1.6454 (3.4); 1.6339 (3.4); -0.0001 (0.4) | |
| I-142 | 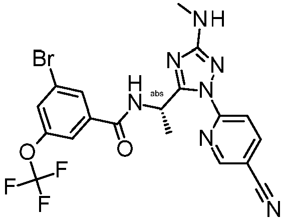 (reference compound) | ¹H-NMR(400.2 MHz, d₆-DMSO): | 510.0 [M+H]⁺ |
| | | δ= 9.3102 (3.2); 9.2928 (3.3); 8.9274 (5.4); 8.9257 (5.8); 8.9219 (5.8); 8.9201 (5.5); 8.4469 (4.6); 8.4412 (4.4); 8.4252 (4.8); 8.4195 (4.9); 8.1485 (4.6); 8.1447 (7.5); 8.1409 (4.6); 7.9123 (5.8); 7.9106 (5.8); 7.8905 (9.4); 7.8888 (10.1); 7.8108 (4.4); 7.8082 (4.9); 7.8057 (4.2); 6.4890 (0.9); 6.4769 (3.0); 6.4643 (3.0); 6.4517 (0.9); 6.0904 (0.5); 6.0734 (2.4); 6.0560 (3.8); 6.0387 (2.4); 6.0212 (0.5); 3.3280 (71.4); 2.7675 (16.0); 2.7550 (15.9); 2.6773 (0.6); 2.6727 (0.9); 2.6681 (0.7); 2.5262 (2.9); 2.5214 (4.4); 2.5128 (52.9); 2.5083 (108.5); 2.5037 (141.7); 2.4991 (99.7); 2.4946 (46.7); 2.3352 (0.6); 2.3305 (0.8); 2.3260 (0.6); 1.5883 (12.8); 1.5709 (12.7); 1.2337 (0.4); 0.1459 (0.8); 0.0080 (6.6); -0.0002 (191.3); -0.0085 (6.8); - 0.1496 (0.8) | |
| I-143 | 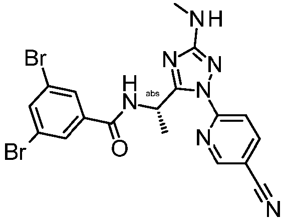 (reference compound) | ¹H-NMR(400.2 MHz, d₆-DMSO): | 506.0 [M+H]⁺ |
| | | δ= 9.2428 (2.4); 9.2254 (2.6); 8.9306 (4.3); 8.9252 (4.4); 8.4465 (2.8); 8.4409 (2.7); 8.4248 (2.9); 8.4192 (3.0); 8.0455 (7.2); 8.0419 (16.0); 8.0371 (7.0); 8.0043 (0.4); 7.9096 (4.4); 7.8879 (4.1); 6.4893 (0.7); 6.4771 (2.3); 6.4646 (2.3); 6.4524 (0.7); 6.0776 (0.4); 6.0603 (1.8); 6.0430 (2.8); 6.0256 (1.8); 6.0083 (0.4); 3.3273 (55.2); 2.9149 (0.4); 2.7658 (11.5); 2.7533 (11.5); 2.6768 (0.5); 2.6722 (0.6); 2.6677 (0.5); 2.5255 (1.9); 2.5118 (39.7); 2.5077 (80.2); 2.5033 (105.1); 2.4988 (76.3); 2.4946 (37.9); 2.3344 (0.5); 2.3300 (0.6); 2.3256 (0.5); 1.5731 (9.6); 1.5557 (9.6); 0.1457 (0.5); 0.0079 (3.6); -0.0002 (103.1); -0.0084 (4.5); -0.1497 (0.5) | |
| I-144 | 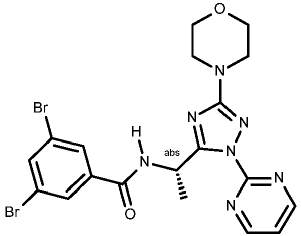 (reference compound) | ¹H-NMR(400.2 MHz, d₆-DMSO): | 538.0 [M+H]⁺ |
| | | δ= 9.2240 (2.9); 9.2061 (3.0); 8.9003 (14.1); 8.8882 (14.4); 8.0202 (3.1); 8.0160 (6.8); 8.0117 (4.6); 7.9822 (16.0); 7.9779 (12.8); 7.5104 (3.8); 7.4983 (7.2); 7.4862 (3.7); 6.0101 (0.4); 5.9930 (2.1); 5.9755 (3.3); 5.9579 (2.1); 5.9406 (0.4); 4.3628 (0.4); 4.3501 (0.7); 4.3373 (0.4); 3.6982 (6.2); 3.6869 (8.9); 3.6745 (7.0); 3.4565 (0.6); 3.4438 (0.6); 3.4390 (0.6); 3.4263 (0.6); 3.3485 (7.3); 3.3272 (77.8); 2.6768 (0.5); 2.6723 (0.7); 2.6678 (0.5); 2.5257 (2.3); 2.5123 (44.0); 2.5079 (85.7); 2.5034 (109.3); 2.4989 (77.5); 2.4945 (37.2); 2.3348 (0.5); 2.3302 (0.7); 2.3258 (0.5); 1.5959 (11.5); 1.5785 (11.4); 1.0740 (1.2); 1.0566 (2.3); 1.0391 (1.1); 0.0077 (0.5); -0.0002 (12.8); -0.0086 (0.4) | |
| I-145 | 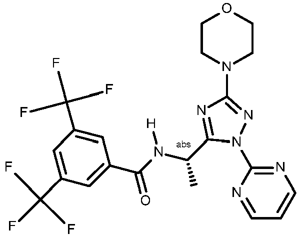 (reference compound) | ¹H-NMR(600.1 MHz, d₆-DMSO): | 516.2 [M+H]⁺ |
| | | δ= 9.5204 (3.4); 9.5085 (3.5); 8.9028 (15.6); 8.8947 (16.0); 8.4403 (11.5); 8.3084 (4.9); 7.5043 (4.2); 7.4962 (8.0); 7.4881 (4.1); 6.0724 (0.5); 6.0608 (2.4); 6.0491 (3.9); 6.0374 (2.5); 6.0258 (0.5); 3.6925 (7.7); 3.6847 (10.6); 3.6765 (8.4); 3.3499 (8.3); 3.3417 (9.8); 3.3338 (7.4); 3.3208 (17.8); 2.6163 (0.4); 2.5253 (0.8); 2.5223 (1.0); 2.5192 (1.0); 2.5102 (21.3); 2.5073 (45.2); 2.5043 (62.6); 2.5013 (46.5); 2.4984 (22.2); 2.3882 (0.4); 1.6408 (13.3); 1.6292 (13.4); 0.0053 (0.4); -0.0001 (12.6); -0.0057 (0.4) | |
| I-146 | 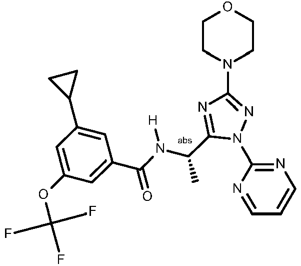 | ¹H-NMR(400.2 MHz, d₆-DMSO): | 504.2 [M+H]⁺ |
| | | δ= 9.1104 (3.3); 9.0925 (3.3); 8.8963 (15.6); 8.8842 (16.0); 7.5030 (4.2); 7.4909 (7.9); 7.4788 (4.2); 7.4639 (11.3); 7.4603 (11.4); 7.2638 (4.9); 6.0140 (0.5); 5.9968 (2.3); 5.9792 (3.7); 5.9615 (2.4); 5.9443 (0.5); 3.6970 (7.2); 3.6859 (10.6); 3.6734 (8.2); 3.3496 (8.6); 3.3295 (88.3); 2.6773 (0.4); 2.6729 (0.6); 2.6683 (0.4); 2.5261 (2.0); 2.5126 (38.5); 2.5084 (75.0); 2.5039 (95.8); 2.4994 (69.4); 2.4951 (34.6); 2.3351 (0.4); 2.3307 (0.6); 2.3263 (0.4); 2.0591 (0.6); 2.0465 (1.4); 2.0381 (1.5); 2.0257 (2.8); 2.0132 (1.7); 2.0048 (1.5); 1.9921 (0.8); 1.6084 (12.9); 1.5910 (12.9); 1.0434 (1.6); 1.0330 (4.2); 1.0274 (4.6); 1.0176 (2.7); 1.0122 (4.4); 1.0065 (4.4); 0.9968 (1.9); 0.7859 (2.0); 0.7757 (4.6); 0.7723 (4.8); 0.7634 (4.7); 0.7595 (4.5); 0.7479 (1.6); 0.0080 (0.4); -0.0001 (9.7); -0.0083 (0.4) | |
| I-147 | 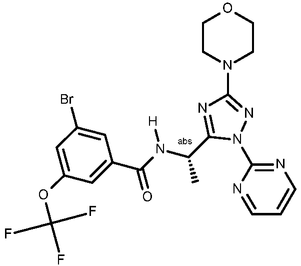 (reference compound) | ¹H-NMR(400.2 MHz, d₆-DMSO): | 544.0 [M+H]⁺ |
| | | δ= 9.2904 (3.3); 9.2727 (3.3); 8.8987 (15.7); 8.8866 (16.0); 8.0744 (4.4); 8.0708 (7.3); 8.0672 (4.6); 7.8706 (4.8); 7.7475 (4.9); 7.5068 (4.2); 7.4947 (7.9); 7.4826 (4.0); 6.0244 (0.5); 6.0074 (2.3); 5.9899 (3.7); 5.9724 (2.4); 5.9552 (0.5); 3.6976 (7.4); 3.6865 (10.5); 3.6741 (8.1); 3.3501 (8.6); 3.3371 (12.3); 3.3274 (93.5); 2.6771 (0.6); 2.6726 (0.8); 2.6682 (0.6); 2.5256 (3.2); 2.5121 (54.7); 2.5080 (104.0); 2.5036 (131.4); 2.4991 (94.0); 2.4947 (45.7); 2.3349 (0.6); 2.3304 (0.8); 2.3259 (0.6); 1.6087 (12.9); 1.5913 (12.8); 0.0078 (0.6); -0.0002 (15.4); -0.0084 (0.6) | |
| I-148 | 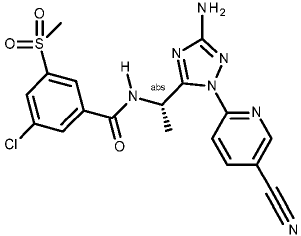 | ¹H-NMR(400.2 MHz, d₆-DMSO): | 446.1 [M+H]⁺ |
| | | δ= 9.4192 (4.0); 9.4017 (4.2); 9.0134 (0.4); 9.0013 (0.3); 8.9398 (7.0); 8.9381 (7.5); 8.9343 (7.5); 8.9325 (7.0); 8.4475 (6.1); 8.4419 (5.7); 8.4258 (6.3); 8.4201 (6.3); 8.3517 (5.8); 8.3479 (10.9); 8.3441 (6.3); 8.3153 (0.8); 8.2560 (6.0); 8.2515 (8.9); 8.2474 (6.0); 8.1540 (6.5); 8.1496 (9.8); 8.1451 (5.6); 7.8287 (7.4); 7.8271 (7.3); 7.8070 (7.1); 7.8053 (7.1); 6.3370 (0.3); 6.1225 (0.6); 6.1053 (3.0); 6.0880 (4.8); 6.0706 (3.1); 6.0529 (0.6); 5.9934 (11.2); 3.3711 (0.9); 3.3315 (62.2); 3.3256 (304.5); 2.7072 (0.7); 2.6948 (0.7); 2.6803 (0.9); 2.6758 (1.9); 2.6712 (2.6); 2.6667 (1.9); 2.6624 (0.8); 2.5248 (8.2); 2.5200 (12.3); 2.5113 (159.1); 2.5069 (326.9); 2.5023 (426.6); 2.4977 (298.3); 2.4932 (137.9); 2.3382 (0.9); 2.3337 (1.9); 2.3291 (2.6); 2.3245 (1.8); 2.3200 (0.8); 1.5994 (16.0); 1.5821 (15.9); 0.1459 (3.5); 0.0079 (30.6); - 0.0002 (837.5); -0.0086 (28.6); -0.1496 (3.5) | |
| I-149 | 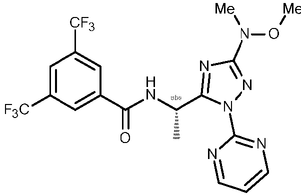 (reference compound) | ¹H-NMR(400.2 MHz, d₆-DMSO): | 490.1 [M+H]⁺ |
| | | δ= 9.8482 (0.7); 9.6000 (1.3); 9.5822 (1.4); 9.2338 (0.8); 8.9941 (1.6); 8.9459 (6.1); 8.9338 (6.2); 8.7482 (0.6); 8.7377 (0.6); 8.5275 (0.6); 8.5244 (0.7); 8.5064 (0.8); 8.4598 (0.4); 8.4308 (5.6); 8.3096 (2.2); 7.9544 (1.4); 7.5696 (1.6); 7.5575 (3.1); 7.5454 (1.6); 7.5162 (0.6); 7.5052 (0.6); 7.4950 (0.6); 7.4842 (0.5); 6.9299 (0.4); 6.9109 (0.3); 6.0546 (0.9); 6.0370 (1.4); 6.0194 (0.9); 4.0568 (0.5); 4.0390 (1.5); 4.0213 (1.5); 4.0035 (0.5); 3.7338 (15.0); 3.6526 (0.5); 3.6364 (1.2); 3.6198 (1.6); 3.6035 (1.2); 3.5868 (0.5); 3.3319 (25.3); 3.1696 (0.6); 3.1511 (1.8); 3.1328 (1.9); 3.1144 (0.8); 3.0938 (13.3); 2.9506 (7.6); 2.8929 (9.2); 2.7338 (8.1); 2.6913 (16.0); 2.6783 (0.4); 2.6735 (0.5); 2.5798 (0.5); 2.5533 (0.4); 2.5088 (63.5); 2.5045 (79.2); 2.5001 (56.8); 2.3313 (0.5); 2.3269 (0.4); 1.9900 (6.6); 1.9107 (3.9); 1.8726 (0.6); 1.6652 (5.2); 1.6478 (5.2); 1.5295 (0.7); 1.4036 (1.3); 1.3807 (11.8); 1.3423 (0.6); 1.3217 (4.2); 1.2982 (0.9); 1.2659 (15.2); 1.2497 (14.8); 1.2439 (11.1); 1.2249 (3.3); 1.2119 (0.8); 1.1941 (2.2); 1.1763 (3.5); 1.1585 (1.8); 1.0876 (0.3); 1.0816 (0.3); 0.1460 (0.3); 0.0076 (3.4); -0.0002 (75.7); -0.0083 (3.8); -0.1495 (0.4) | |
| I-150 | 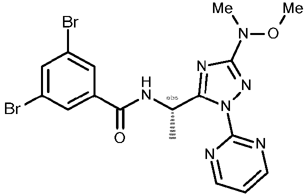 (reference compound) | ¹H-NMR(400.2 MHz, d₆-DMSO): | 511.9 [M+H]⁺ |
| | | δ= 9.2929 (1.2); 9.2750 (1.2); 8.9399 (7.0); 8.9278 (7.2); 8.0215 (1.5); 8.0171 (3.1); 8.0128 (2.0); 7.9678 (7.5); 7.9634 (6.4); 7.5709 (1.8); 7.5588 (3.5); 7.5467 (1.8); 5.9773 (0.9); 5.9598 (1.4); 5.9421 (0.9); 5.7556 (3.7); 3.7368 (16.0); 3.3259 (58.2); 3.0901 (13.6); 2.6763 (0.4); 2.6718 (0.5); 2.6672 (0.4); 2.5252 (1.7); 2.5205 (2.5); 2.5118 (30.6); 2.5074 (62.6); 2.5028 (81.9); 2.4982 (57.5); 2.4937 (26.8); 2.3341 (0.4); 2.3296 (0.5); 2.3251 (0.4); 1.6173 (5.0); 1.5998 (5.0); 0.1458 (0.4); 0.0080 (3.6); -0.0002 (99.4); -0.0086 (3.3); -0.1496 (0.4) | |
| I-151 | 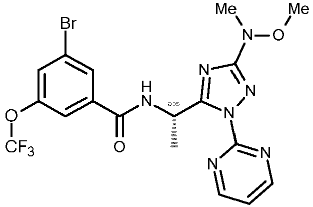 (reference compound) | ¹H-NMR(400.2 MHz, d₆-DMSO): | 516.0 [M+H]+ |
| | | δ= 9.3623 (1.1); 9.3445 (1.1); 8.9394 (6.8); 8.9272 (7.0); 8.0601 (1.6); 8.0563 (2.8); 8.0526 (1.8); 7.8731 (1.7); 7.7394 (1.5); 7.7368 (1.7); 7.7341 (1.5); 7.5680 (1.8); 7.5558 (3.3); 7.5437 (1.7); 5.9929 (0.9); 5.9753 (1.4); 5.9577 (0.9); 3.7357 (16.0); 3.3271 (34.9); 3.0913 (13.5); 2.6724 (0.3); 2.5259 (1.0); 2.5211 (1.6); 2.5125 (21.7); 2.5080 (44.7); 2.5035 (58.8); 2.4989 (41.6); 2.4943 (19.7); 2.3302 (0.4); 1.6307 (4.9); 1.6132 (4.9); 0.0079 (2.6); -0.0002 (74.0); - 0.0086 (2.6) | |
| I-152 | 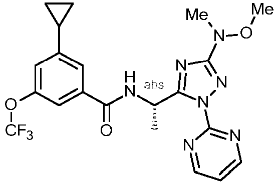 | ¹H-NMR(400.2 MHz, d₆-DMSO): | 478.1 [M+H]⁺ |
| | | δ = 9.1882 (1.4); 9.1702 (1.5); 8.9384 (6.5); 8.9263 (6.5); 7.5650 (1.8); 7.5529 (3.3); 7.5408 (1.7); 7.4530 (5.0); 7.4496 (5.0); 7.2688 (2.2); 5.9829 (1.0); 5.9653 (1.6); 5.9477 (1.0); 5.7574 (1.7); 3.7356 (16.0); 3.3304 (17.6); 3.0914 (14.1); 2.5092 (23.6); 2.5049 (28.9); 2.5005 (20.4); 2.0460 (0.6); 2.0375 (0.7); 2.0252 (1.2); 2.0127 (0.7); 2.0043 (0.6); 1.6326 (5.6); 1.6152 (5.6); 1.3970 (0.5); 1.0434 (0.7); 1.0330 (1.9); 1.0275 (2.0); 1.0176 (1.3); 1.0121 (1.9); 1.0066 (1.8); 0.9970 (0.8); 0.7864 (0.9); 0.7762 (2.1); 0.7732 (2.2); 0.7641 (2.1); 0.7605 (1.9); 0.7488 (0.7); -0.0002 (27.7); -0.0085 (1.1) | |
| I-153 | 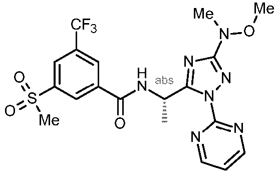 | ¹H-NMR(400.2 MHz, d₆-DMSO): | 500.1 [M+H]⁺ |
| | | δ = 9.6387 (1.2); 9.6207 (1.2); 8.9485 (6.9); 8.9363 (7.0); 8.5945 (2.3); 8.4557 (2.1); 8.3870 (2.0); 8.3162 (0.5); 7.5731 (1.8); 7.5610 (3.4); 7.5489 (1.7); 6.0650 (0.9); 6.0474 (1.4); 6.0296 (0.9); 3.7347 (16.0); 3.3687 (13.9); 3.3256 (179.6); 3.0944 (13.6); 2.6801 (0.6); 2.6756 (1.2); 2.6710 (1.7); 2.6665 (1.2); 2.6618 (0.6); 2.5245 (5.7); 2.5197 (8.6); 2.5111 (103.2); 2.5066 (211.3); 2.5021 (276.8); 2.4975 (194.4); 2.4930 (90.6); 2.3379 (0.6); 2.3334 (1.2); 2.3288 (1.7); 2.3243 (1.2); 2.3199 (0.6); 1.6677 (4.9); 1.6503 (4.9); 0.1458 (2.5); 0.0260 (0.5); 0.0217 (0.6); 0.0078 (21.5); -0.0002 (564.1); -0.0087 (19.7); -0.0205 (0.8); -0.0286 (0.4); -0.0316 (0.4); -0.1497 (2.6) | |
| I-154 | 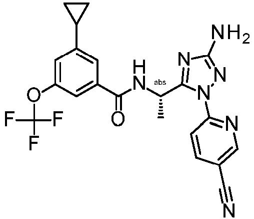 | ¹H-NMR(400.2 MHz, d₆-DMSO): | 458.3 [M+H]⁺ |
| | | δ = 9.1108 (4.0); 9.0933 (4.2); 8.9217 (7.0); 8.9199 (7.5); 8.9161 (7.5); 8.9143 (7.2); 8.4420 (6.1); 8.4364 (5.8); 8.4203 (6.4); 8.4147 (6.4); 8.3162 (0.5); 7.8197 (7.3); 7.8180 (7.4); 7.7981 (7.0); 7.7963 (7.2); 7.5552 (8.9); 7.5516 (6.1); 7.5396 (5.2); 7.5370 (5.8); 7.5345 (5.1); 7.2768 (5.7); 6.0760 (0.7); 6.0590 (3.0); 6.0415 (4.8); 6.0241 (3.1); 6.0064 (0.7); 5.9695 (9.8); 5.7558 (1.0); 3.3256 (81.7); 2.6807 (0.5); 2.6762 (1.2); 2.6716 (1.6); 2.6671 (1.2); 2.6626 (0.5); 2.5251 (5.0); 2.5204 (7.3); 2.5117 (96.6); 2.5072 (200.5); 2.5027 (263.0); 2.4981 (183.7); 2.4935 (84.9); 2.3387 (0.5); 2.3341 (1.1); 2.3295 (1.6); 2.3249 (1.1); 2.3204 (0.5); 2.0753 (0.8); 2.0627 (1.8); 2.0544 (1.9); 2.0506 (1.4); 2.0420 (3.6); 2.0333 (1.3); 2.0294 (2.0); 2.0211 (1.9); 2.0084 (1.0); 1.5869 (16.0); 1.5695 (15.9); 1.0536 (1.6); 1.0424 (6.0); 1.0368 (6.5); 1.0262 (2.6); 1.0215 (6.2); 1.0159 (6.2); 1.0056 (1.9); 0.8032 (2.6); 0.7931 (5.6); 0.7900 (5.6); 0.7872 (5.5); 0.7807 (5.8); 0.7768 (5.5); 0.7650 (2.1); 0.1459 (2.2); 0.0249 (0.4); 0.0207 (0.6); 0.0079 (19.0); -0.0001 (532.3); -0.0086 (17.8); -0.0281 (0.4); -0.1496 (2.3) | |
| I-155 | 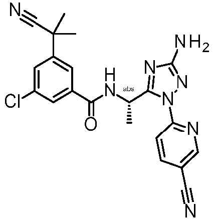 | ¹H-NMR(400.2 MHz, d₆-DMSO): | 435.2 [M+H]⁺ |
| | | δ = 9.2125 (0.9); 9.1950 (0.9); 8.9321 (1.5); 8.9305 (1.6); 8.9266 (1.6); 8.9249 (1.5); 8.4443 (1.2); 8.4387 (1.2); 8.4226 (1.3); 8.4170 (1.4); 7.9366 (0.9); 7.9328 (2.1); 7.9280 (3.0); 7.9232 (2.2); 7.9196 (0.9); 7.8245 (1.6); 7.8229 (1.6); 7.8029 (1.5); 7.8012 (1.6); 7.7468 (1.3); 7.7422 (2.3); 7.7376 (1.2); 6.0769 (0.6); 6.0595 (1.0); 6.0421 (0.7); 5.9789 (2.4); 5.7553 (6.9); 3.3254 (41.2); 2.6713 (0.4); 2.5249 (1.3); 2.5202 (1.8); 2.5115 (26.8); 2.5070 (55.7); 2.5024 (73.3); 2.4978 (51.3); 2.4933 (23.9); 2.3292 (0.4); 1.7268 (0.9); 1.7164 (16.0); 1.5957 (3.4); 1.5783 (3.4); 0.1458 (0.6); 0.0132 (0.5); 0.0078 (5.0); -0.0002 (151.0); - 0.0087 (5.3); -0.0178 (0.4); -0.1498 (0.6) | |
| I-156 | 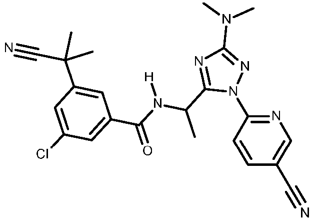 | ¹H-NMR(400.2 MHz, d6-DMSO): | 463.1 [M+H]+ |
| | | δ = 9.2645 (0.8); 9.2469 (0.8); 8.9484 (1.4); 8.9439 (1.4); 8.9429 (1.3); 8.4654 (1.0); 8.4598 (1.0); 8.4438 (1.1); 8.4381 (1.1); 7.9344 (1.4); 7.9127 (1.4); 7.9058 (0.8); 7.9020 (1.7); 7.8976 (1.6); 7.8945 (1.4); 7.8902 (1.8); 7.8866 (0.8); 7.7486 (1.1); 7.7440 (2.0); 7.7395 (1.0); 6.0987 (0.6); 6.0812 (0.9); 6.0638 (0.6); 3.3281 (14.8); 2.9666 (16.0); 2.5262 (0.5); 2.5213 (0.8); 2.5128 (9.4); 2.5084 (19.3); 2.5039 (25.2); 2.4994 (17.8); 2.4950 (8.4); 1.7148 (14.4); 1.6132 (3.1); 1.5958 (3.0); 0.0077 (1.7); -0.0002 (43.2); -0.0086 (1.5) | |
| I-157 | 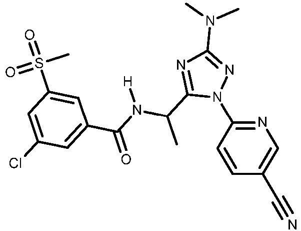 | ¹H-NMR(400.2 MHz, d6-DMSO): | 474.1 [M+H]+ |
| | | δ = 9.4694 (0.7); 9.4518 (0.8); 8.9557 (1.2); 8.9541 (1.3); 8.9502 (1.4); 8.9485 (1.3); 8.4690 (1.1); 8.4634 (1.0); 8.4473 (1.1); 8.4417 (1.1); 8.3260 (1.0); 8.3222 (2.0); 8.3184 (1.2); 8.2355 (1.1); 8.2310 (1.6); 8.2269 (1.1); 8.1547 (1.2); 8.1503 (1.8); 8.1458 (1.0); 7.9388 (1.4); 7.9372 (1.3); 7.9171 (1.3); 7.9155 (1.2); 6.1270 (0.6); 6.1095 (0.9); 6.0919 (0.6); 3.3279 (20.2); 2.9680 (16.0); 2.8923 (0.4); 2.7334 (0.4); 2.5263 (0.4); 2.5216 (0.6); 2.5128 (9.1); 2.5084 (18.9); 2.5038 (24.7); 2.4992 (17.3); 2.4947 (8.0); 1.6193 (3.0); 1.6019 (2.9); 1.2600 (0.4); 1.2446 (0.4); 0.0079 (1.7); -0.0002 (46.7); -0.0086 (1.5) | |
| I-158 | 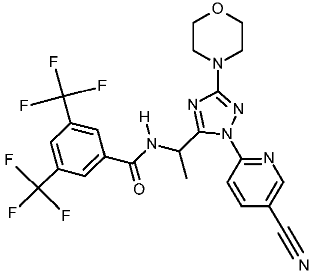 (reference compound) | ¹H-NMR (400.2 MHz, d₆-DMSO): | 540.1 [M+H]⁺ |
| | | δ = 9.5703 (3.2); 9.5528 (3.4); 8.9685 (5.7); 8.9669 (6.2); 8.9630 (6.2); 8.9613 (6.0); 8.4893 (11.4); 8.4842 (16.0); 8.4681 (5.4); 8.4625 (5.4); 8.3211 (5.1); 7.9432 (6.3); 7.9415 (6.2); 7.9216 (6.0); 7.9198 (6.0); 6.1633 (0.5); 6.1460 (2.5); 6.1286 (4.0); 6.1112 (2.6); 6.0940 (0.5); 3.6940 (6.8); 3.6829 (9.6); 3.6703 (7.7); 3.3769 (8.1); 3.3646 (9.7); 3.3530 (8.0); 3.3386 (126.8); 2.6794 (0.5); 2.6749 (0.7); 2.6703 (0.5); 2.5284 (2.0); 2.5237 (3.0); 2.5150 (40.7); 2.5105 (84.8); 2.5059 (111.0); 2.5013 (77.1); 2.4967 (35.2); 2.3373 (0.5); 2.3328 (0.6); 2.3281 (0.5); 2.0773 (0.7); 1.6407 (13.5); 1.6233 (13.4); 0.1459 (0.4); 0.0080 (3.0); -0.0002 (96.2); -0.0086 (2.9); -0.1496 (0.4) | |
| I-159 | 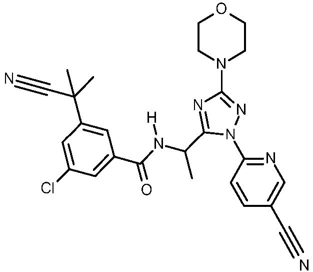 | ¹H-NMR (400.2 MHz, d₆-DMSO): | 505.1 [M+H]⁺ |
| | | δ = 9.2532 (0.8); 9.2359 (0.8); 8.9649 (1.4); 8.9611 (1.4); 8.9595 (1.4); 8.4871 (1.1); 8.4815 (1.1); 8.4655 (1.2); 8.4599 (1.2); 7.9377 (1.4); 7.9362 (1.4); 7.9162 (1.3); 7.9145 (1.4); 7.8933 (1.9); 7.8891 (3.0); 7.8851 (2.1); 7.7472 (1.1); 7.7426 (2.0); 7.7380 (1.0); 6.0947 (0.6); 6.0773 (1.0); 6.0598 (0.6); 3.6928 (1.6); 3.6814 (2.3); 3.6689 (1.8); 3.3770 (1.9); 3.3648 (2.3); 3.3535 (1.7); 3.3295 (87.6); 2.6762 (0.3); 2.6716 (0.5); 2.6672 (0.3); 2.5251 (1.4); 2.5204 (2.1); 2.5116 (28.8); 2.5072 (59.4); 2.5026 (77.9); 2.4980 (54.7); 2.4935 (25.4); 2.3340 (0.4); 2.3293 (0.5); 2.3249 (0.4); 2.0746 (16.0); 1.7112 (15.4); 1.6133 (3.1); 1.5959 (3.1); 0.0080 (2.3); -0.0002 (65.7); -0.0086 (1.9) | |
| I-160 | 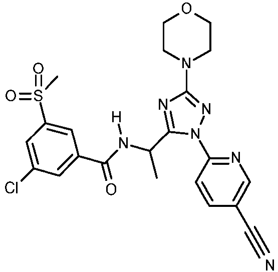 | ¹H-NMR (400.2 MHz, d₆-DMSO): | 516.1 [M+H]⁺ |
| | | δ = 9.4593 (4.2); 9.4419 (4.3); 8.9730 (7.4); 8.9712 (7.8); 8.9675 (7.9); 8.9657 (7.4); 8.4910 (6.4); 8.4854 (6.0); 8.4693 (6.7); 8.4637 (6.8); 8.3215 (6.1); 8.3177 (11.5); 8.3138 (6.7); 8.2312 (6.2); 8.2266 (9.2); 8.2226 (6.3); 8.1545 (7.0); 8.1501 (10.2); 8.1456 (5.8); 7.9508 (0.6); 7.9425 (7.6); 7.9408 (7.5); 7.9209 (7.2); 7.9191 (7.2); 6.1416 (0.6); 6.1243 (3.0); 6.1069 (4.9); 6.0895 (3.1); 6.0721 (0.6); 3.6951 (8.4); 3.6841 (11.9); 3.6715 (9.4); 3.6428 (0.3); 3.3778 (10.0); 3.3660 (11.7); 3.3548 (8.7); 3.3268 (178.1); 3.1483 (0.4); 2.8916 (3.3); 2.7328 (2.7); 2.7317 (2.7); 2.6902 (1.2); 2.6811 (0.5); 2.6764 (1.1); 2.6718 (1.5); 2.6672 (1.1); 2.6627 (0.5); 2.6106 (0.4); 2.5254 (4.5); 2.5207 (6.7); 2.5120 (91.5); 2.5075 (188.6); 2.5029 (247.2); 2.4983 (172.3); 2.4937 (79.3); 2.3388 (0.5); 2.3343 (1.1); 2.3297 (1.5); 2.3252 (1.1); 2.3209 (0.5); 1.6200 (16.0); 1.6026 (15.8); 1.2737 (1.9); 1.2590 (3.1); 1.2435 (3.1); 1.2253 (0.6); 0.1460 (1.0); 0.0080 (8.8); -0.0001 (266.6); -0.0085 (8.3); -0.1496 (1.0) | |
| I-161 | 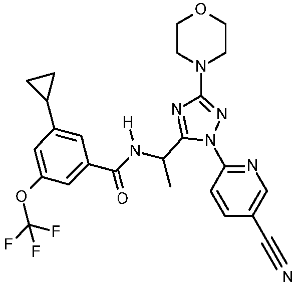 | ¹H-NMR (400.2 MHz, d₆-DMSO): | 528.1 [M+H]⁺ |
| | | δ = 9.1583 (4.1); 9.1408 (4.2); 8.9562 (7.1); 8.9546 (7.4); 8.9507 (7.5); 8.9491 (7.0); 8.4836 (5.7); 8.4780 (5.4); 8.4620 (6.1); 8.4564 (6.1); 7.9326 (7.4); 7.9311 (7.2); 7.9110 (7.0); 7.9094 (6.9); 7.5167 (8.8); 7.5131 (6.5); 7.5048 (5.3); 7.5021 (5.9); 7.2760 (5.8); 6.0914 (0.6); 6.0743 (2.9); 6.0569 (4.7); 6.0394 (3.0); 6.0220 (0.6); 3.6948 (8.3); 3.6837 (11.9); 3.6711 (9.3); 3.3734 (10.0); 3.3608 (12.9); 3.3421 (164.2); 2.6787 (0.5); 2.6741 (0.7); 2.6695 (0.5); 2.5276 (2.1); 2.5229 (3.2); 2.5142 (44.0); 2.5097 (90.8); 2.5052 (118.4); 2.5006 (82.5); 2.4960 (38.0); 2.3365 (0.5); 2.3319 (0.7); 2.3274 (0.5); 2.0762 (0.9); 2.0731 (0.8); 2.0604 (1.8); 2.0520 (1.9); 2.0483 (1.3); 2.0395 (3.6); 2.0308 (1.3); 2.0270 (2.0); 2.0187 (1.9); 2.0061 (0.9); 1.6076 (16.0); 1.5902 (15.8); 1.0531 (2.2); 1.0419 (5.9); 1.0363 (6.4); 1.0325 (3.2); 1.0258 (3.3); 1.0210 (6.1); 1.0154 (6.1); 1.0051 (2.5); 0.7982 (2.6); 0.7874 (6.8); 0.7823 (6.7); 0.7752 (6.3); 0.7700 (7.3); 0.7585 (2.1); 0.0080 (1.0); -0.0002 (34.0); -0.0085 (1.0) | |
| I-162 | 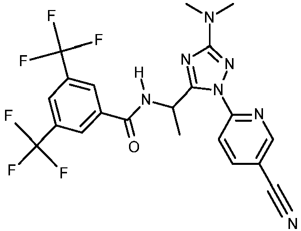 (reference compound) | ¹H-NMR (400.2 MHz, d₆-DMSO): | 498.1 [M+H]⁺ |
| | | δ = 9.5790 (0.7); 9.5615 (0.7); 8.9508 (1.3); 8.9468 (1.3); 8.9453 (1.2); 8.4876 (2.6); 8.4690 (1.0); 8.4634 (1.0); 8.4474 (1.0); 8.4417 (1.0); 8.3204 (1.2); 7.9397 (1.3); 7.9382 (1.3); 7.9181 (1.2); 7.9165 (1.2); 6.1462 (0.5); 6.1288 (0.9); 6.1112 (0.6); 3.3287 (17.1); 2.9651 (16.0); 2.5268 (0.6); 2.5133 (12.6); 2.5090 (25.2); 2.5045 (32.6); 2.5000 (23.0); 2.4955 (10.9); 1.6368 (3.0); 1.6194 (3.0); 0.0078 (2.4); -0.0002 (63.2); -0.0086 (2.4) | |
| | | ¹³C-NMR (150.9 MHz, d₆-DMSO): | |
| | | δ = 164.7515 (2.0); 163.3696 (1.6); 159.6955 (2.1); 152.2614 (1.7); 151.9645 (2.1); 143.3199 (2.0); 136.1945 (1.8); 130.9554 (0.4); 130.7351 (1.2); 130.5146 (1.3); 130.2942 (0.4); 128.4651 (1.4); 125.9801 (0.4); 125.2412 (0.6); 124.1708 (1.0); 122.3621 (0.9); 120.5526 (0.2); 116.9099 (1.2); 114.6690 (2.3); 106.0916 (1.9); 44.5403 (2.4); 40.2357 (3.8); 40.1162 (19.9); 39.9775 (59.2); 39.8386 (116.0); 39.6995 (135.8); 39.5604 (115.1); 39.4213 (57.6); 39.2822 (18.7); 37.8999 (7.0); 18.7989 (3.5); 0.2703 (0.3 | |
| I-163 | 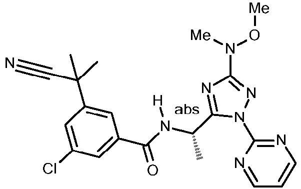 | ¹H-NMR (400.2 MHz, d₆-DMSO): | 455.1 [M+H]⁺ |
| | | δ = 9.2809 (0.8); 9.2631 (0.8); 8.9431 (4.3); 8.9310 (4.4); 7.8362 (2.2); 7.8328 (2.8); 7.8294 (2.3); 7.7314 (1.2); 7.7268 (2.0); 7.7223 (1.0); 7.5696 (1.1); 7.5575 (2.2); 7.5454 (1.1); 6.0011 (0.6); 5.9835 (1.0); 5.9658 (0.6); 5.7561 (1.9); 3.7368 (10.2); 3.3271 (25.0); 3.0928 (8.8); 2.8915 (1.8); 2.7321 (1.5); 2.5253 (0.9); 2.5119 (13.8); 2.5076 (28.0); 2.5031 (36.8); 2.4986 (26.4); 2.4941 (12.7); 1.7018 (16.0); 1.6404 (3.3); 1.6230 (3.3); 1.3973 (0.4); 0.0078 (1.5); -0.0002 (38.8); -0.0086 (1.4) | |
| I-164 | 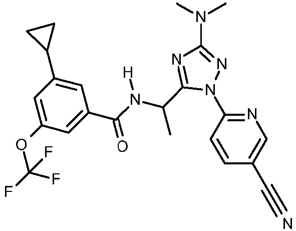 | ¹H-NMR (400.2 MHz, d₆-DMSO): | 486.1 [M+H]⁺ |
| | | δ = 9.1676 (0.7); 9.1499 (0.7); 8.9409 (1.1); 8.9394 (1.2); 8.9354 (1.2); 8.9338 (1.2); 8.4636 (1.0); 8.4579 (0.9); 8.4419 (1.0); 8.4363 (1.0); 7.9295 (1.2); 7.9279 (1.3); 7.9078 (1.2); 7.9061 (1.2); 7.5180 (1.5); 7.5143 (1.2); 7.5069 (0.9); 7.5042 (1.0); 7.2790 (1.0); 6.0744 (0.5); 6.0569 (0.8); 6.0393 (0.5); 3.3289 (21.9); 2.9622 (16.0); 2.5259 (0.7); 2.5212 (1.0); 2.5125 (12.4); 2.5080 (25.3); 2.5035 (32.9); 2.4988 (23.0); 2.4943 (10.6); 2.0421 (0.6); 2.0296 (0.3); 2.0212 (0.3); 1.6032 (2.9); 1.5858 (2.8); 1.0533 (0.4); 1.0421 (1.0); 1.0365 (1.1); 1.0327 (0.6); 1.0261 (0.6); 1.0212 (1.0); 1.0156 (1.0); 1.0053 (0.4); 0.8024 (0.4); 0.7917 (1.2); 0.7865 (1.2); 0.7794 (1.1); 0.7742 (1.2); 0.7628 (0.4); 0.0079 (2.3); -0.0002 (62.8); -0.0086 (2.0) | |
| I-165 | 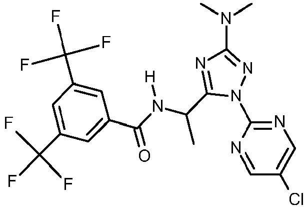 (reference compound) | ¹H-NMR (400.2 MHz, d₆-DMSO): | 508.0 [M+H]⁺ |
| | | δ = 9.5528 (0.7); 9.5350 (0.7); 9.0032 (7.3); 8.4653 (2.6); 8.3150 (1.2); 6.0304 (0.6); 6.0128 (0.9); 5.9952 (0.6); 3.3296 (15.2); 2.9489 (16.0); 2.5276 (0.4); 2.5228 (0.7); 2.5142 (8.6); 2.5098 (17.6); 2.5053 (23.1); 2.5007 (16.4); 2.4963 (7.8); 1.6340 (3.0); 1.6166 (3.0); 0.0078 (0.9); -0.0002 (24.7); -0.0086 (0.8) | |
| I-166 | 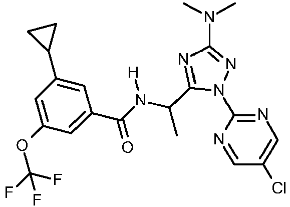 | ¹H-NMR (400.2 MHz, d₆-DMSO): | 496.0 [M+H]⁺ |
| | | δ = 9.1352 (0.7); 9.1173 (0.7); 8.9915 (8.2); 7.4836 (2.1); 7.4802 (2.1); 7.2737 (1.0); 5.9570 (0.5); 5.9394 (0.8); 5.9217 (0.5); 3.3264 (27.4); 2.9444 (16.0); 2.5253 (0.8); 2.5206 (1.2); 2.5119 (15.8); 2.5074 (33.1); 2.5029 (43.7); 2.4982 (31.0); 2.4937 (14.6); 2.0346 (0.6); 2.0220 (0.3); 2.0137 (0.3); 1.5985 (2.8); 1.5811 (2.8); 1.0485 (0.4); 1.0377 (1.0); 1.0320 (1.0); 1.0279 (0.6); 1.0218 (0.6); 1.0168 (1.0); 1.0111 (1.0); 1.0009 (0.4); 0.7959 (0.4); 0.7854 (1.1); 0.7801 (1.1); 0.7730 (1.0); 0.7680 (1.2); 0.7567 (0.4); 0.0079 (1.8); -0.0002 (53.3); -0.0086 (1.8) | |
| I-167 | 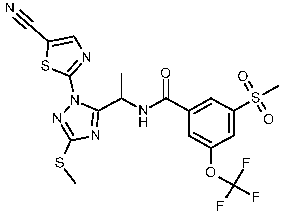 (reference compound) | ¹H-NMR (400.2 MHz, d₆-DMSO): | 533.1 [M+H]⁺ |
| | | δ = 9.6396 (1.4); 9.6234 (1.5); 8.6392 (7.7); 8.6107 (1.7); 8.4726 (2.3); 8.4690 (3.4); 8.4654 (1.9); 8.1531 (2.3); 8.1049 (2.4); 6.0480 (1.0); 6.0310 (1.4); 6.0140 (1.0); 5.9981 (0.4); 5.7560 (0.5); 4.3291 (0.8); 4.3114 (0.8); 3.3638 (17.1); 3.3545 (1.0); 3.3259 (57.9); 2.6717 (1.1); 2.6671 (0.6); 2.6012 (16.0); 2.5250 (2.2); 2.5202 (3.3); 2.5115 (46.3); 2.5071 (95.5); 2.5026 (125.5); 2.4981 (89.0); 2.4937 (42.2); 2.3338 (0.6); 2.3294 (0.8); 2.3249 (0.6); 1.6412 (5.2); 1.6237 (5.3); 1.6130 (1.4); 1.3618 (0.9); 1.3442 (1.9); 1.3265 (0.9); 1.2073 (0.4); 1.1906 (0.4); 0.1460 (0.5); 0.0079 (3.9); -0.0002 (119.0); -0.0086 (3.9); -0.1496 (0.5) | |
| I-168 | 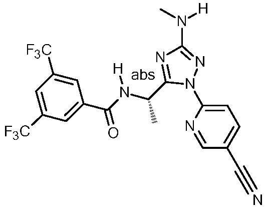 (reference compound) | ¹H-NMR (400.2 MHz, d₆-DMSO): | 484.1 [M+H]⁺ |
| | | δ = 9.5512 (3.0); 9.5338 (3.1); 8.9383 (5.2); 8.9369 (5.7); 8.9329 (5.6); 8.9312 (5.4); 8.5110 (10.8); 8.4510 (5.0); 8.4454 (5.2); 8.4294 (4.8); 8.4237 (4.8); 8.4068 (0.3); 8.3231 (4.8); 7.9201 (5.7); 7.9184 (5.7); 7.8984 (5.3); 7.8966 (5.4); 6.4879 (0.8); 6.4757 (2.8); 6.4632 (2.9); 6.4507 (0.8); 6.1528 (0.5); 6.1355 (2.4); 6.1182 (3.8); 6.1008 (2.4); 6.0836 (0.5); 3.3301 (29.6); 2.7701 (16.0); 2.7576 (15.8); 2.6786 (0.5); 2.6741 (0.6); 2.6696 (0.4); 2.5276 (2.0); 2.5229 (3.1); 2.5142 (37.8); 2.5097 (77.9); 2.5052 (101.8); 2.5006 (71.4); 2.4960 (33.1); 2.3366 (0.4); 2.3320 (0.6); 2.3274 (0.4); 1.6230 (12.6); 1.6056 (12.5); 0.1459 (0.5); 0.0079 (4.4); -0.0002 (127.9); -0.0086 (4.2); - 0.1496 (0.5) | |
| I-169 | 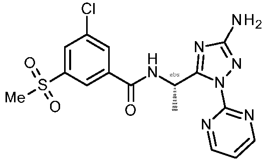 | ¹H-NMR (400.2 MHz, d₆-DMSO): | 422.1 [M+H]⁺ |
| | | δ = 9.3775 (2.7); 9.3595 (2.8); 8.8636 (15.6); 8.8515 (16.0); 8.3153 (1.2); 8.3096 (4.0); 8.3059 (7.4); 8.3021 (4.3); 8.2102 (3.9); 8.2056 (6.2); 8.2016 (4.1); 8.1376 (4.5); 8.1331 (6.8); 8.1287 (3.8); 7.4492 (4.1); 7.4371 (7.7); 7.4250 (4.1); 6.0548 (0.4); 6.0370 (2.0); 6.0196 (3.2); 6.0019 (2.0); 5.9849 (0.4); 5.7455 (8.1); 3.3709 (0.4); 3.3251 (247.6); 2.6800 (0.8); 2.6756 (1.6); 2.6710 (2.3); 2.6665 (1.7); 2.6620 (0.8); 2.5245 (7.0); 2.5198 (10.8); 2.5111 (141.8); 2.5067 (293.2); 2.5021 (385.3); 2.4975 (269.2); 2.4930 (124.6); 2.3377 (0.8); 2.3335 (1.6); 2.3289 (2.3); 2.3244 (1.6); 1.5987 (11.1); 1.5814 (11.0); 0.1458 (1.8); 0.0079 (14.7); -0.0002 (430.2); -0.0086 (13.4); -0.0202 (0.5); -0.1496 (1.8) | |
| I-170 | 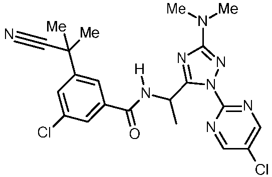 | ¹H-NMR (400.2 MHz, d₆-DMSO): | 473.1 [M+H]⁺ |
| | | δ = 9.2333 (0.7); 9.2154 (0.7); 8.9977 (8.7); 7.8679 (3.2); 7.8634 (3.5); 7.7396 (1.1); 7.7350 (1.9); 7.7304 (1.0); 5.9800 (0.6); 5.9625 (0.8); 5.9448 (0.5); 5.7556 (7.3); 3.3268 (25.8); 2.9484 (16.0); 2.5254 (0.7); 2.5207 (1.2); 2.5120 (13.4); 2.5076 (27.0); 2.5031 (35.1); 2.4985 (24.7); 2.4940 (11.6); 1.7093 (14.6); 1.6087 (2.9); 1.5913 (2.9); 0.0080 (1.5); -0.0002 (38.5); -0.0085 (1.3) | |
| I-171 | 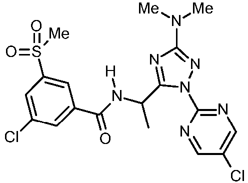 | ¹H-NMR (400.2 MHz, d₆-DMSO): | 484.0 [M+H]⁺ |
| | | δ = 9.4394 (0.8); 9.4214 (0.8); 9.0030 (7.5); 8.2954 (1.0); 8.2919 (1.9); 8.2882 (1.2); 8.2084 (1.0); 8.2042 (1.7); 8.2002 (1.1); 8.1445 (1.1); 8.1403 (1.8); 8.1360 (1.0); 6.0074 (0.6); 5.9899 (0.9); 5.9723 (0.6); 5.7556 (0.7); 3.3256 (28.6); 3.3205 (9.9); 2.9494 (16.0); 2.5249 (0.9); 2.5114 (17.7); 2.5072 (34.9); 2.5028 (45.0); 2.4984 (32.4); 1.6152 (3.1); 1.5978 (3.1); 0.0077 (2.1); -0.0002 (49.1); -0.0084 (2.0) | |
| I-172 | 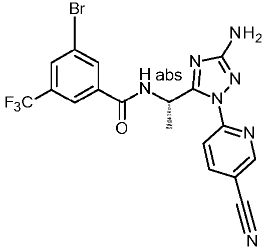 (reference compound) | ¹H-NMR (400.2 MHz, d₆-DMSO): | 480.0 [M+H]⁺ |
| | | δ = 9.3759 (4.1); 9.3585 (4.2); 8.9315 (7.0); 8.9298 (7.2); 8.9260 (7.4); 8.9242 (6.7); 8.4450 (6.0); 8.4394 (5.7); 8.4233 (6.3); 8.4177 (6.3); 8.3574 (7.3); 8.3152 (0.6); 8.1995 (7.1); 8.1787 (6.7); 7.8250 (7.4); 7.8233 (7.2); 7.8033 (7.1); 7.8016 (7.0); 6.1065 (0.6); 6.0895 (3.0); 6.0722 (4.8); 6.0548 (3.1); 6.0376 (0.7); 5.9871 (11.2); 5.7554 (2.7); 3.3262 (258.4); 2.8728 (0.6); 2.6809 (0.6); 2.6764 (1.1); 2.6718 (1.5); 2.6672 (1.1); 2.6627 (0.5); 2.5253 (4.9); 2.5205 (7.6); 2.5119 (88.8); 2.5074 (178.8); 2.5029 (234.4); 2.4982 (167.9); 2.4937 (79.4); 2.3387 (0.5); 2.3343 (1.1); 2.3297 (1.5); 2.3251 (1.0); 2.3205 (0.5); 1.5932 (16.0); 1.5759 (15.9); 0.1460 (0.6); 0.0080 (5.0); - 0.0002 (147.0); -0.0085 (4.8); -0.1495 (0.6) | |
| I-173 | 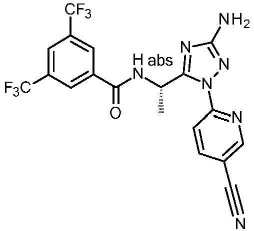 (reference compound) | ¹H-NMR (400.2 MHz, d₆-DMSO): | 470.1 [M+H]⁺ |
| | | δ = 9.5350 (4.1); 9.5176 (4.2); 8.9346 (6.9); 8.9330 (7.3); 8.9291 (7.4); 8.9273 (6.9); 8.5166 (14.0); 8.4468 (6.0); 8.4412 (5.7); 8.4251 (6.3); 8.4195 (6.3); 8.3237 (6.2); 7.8292 (7.3); 7.8276 (7.4); 7.8076 (6.9); 7.8058 (7.0); 6.1438 (0.6); 6.1270 (3.0); 6.1096 (4.8); 6.0922 (3.1); 6.0751 (0.6); 5.9900 (11.4); 3.3259 (281.4); 2.8905 (0.6); 2.6808 (0.6); 2.6763 (1.4); 2.6718 (1.9); 2.6672 (1.4); 2.6627 (0.6); 2.5253 (5.4); 2.5206 (8.0); 2.5119 (111.4); 2.5074 (228.4); 2.5028 (300.4); 2.4982 (212.8); 2.4937 (99.8); 2.3387 (0.6); 2.3342 (1.4); 2.3297 (1.9); 2.3251 (1.3); 2.3206 (0.6); 1.6177 (16.0); 1.6004 (16.0); 0.0080 (2.3); -0.0002 (74.3); -0.0085 (2.2) | |
| I-174 | 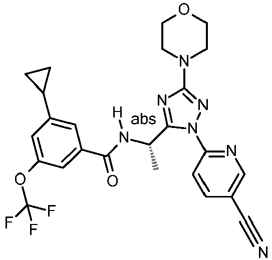 | ¹H-NMR (400.2 MHz, d₆-DMSO): | 528.3 [M+H]⁺ |
| | | δ = 9.1540 (4.1); 9.1365 (4.2); 8.9558 (6.8); 8.9541 (7.4); 8.9503 (7.3); 8.9485 (7.1); 8.4838 (6.2); 8.4782 (5.8); 8.4621 (6.5); 8.4565 (6.6); 8.3157 (0.4); 7.9324 (7.4); 7.9307 (7.4); 7.9108 (7.0); 7.9090 (7.2); 7.5159 (8.9); 7.5122 (6.4); 7.5036 (5.1); 7.5009 (5.7); 7.4983 (5.0); 7.2750 (5.5); 6.0903 (0.6); 6.0733 (3.0); 6.0558 (4.9); 6.0384 (3.1); 6.0210 (0.6); 3.6939 (8.1); 3.6827 (11.5); 3.6701 (9.1); 3.3729 (9.4); 3.3607 (11.1); 3.3491 (8.0); 3.3270 (144.4); 2.6769 (0.7); 2.6723 (0.9); 2.6677 (0.7); 2.5258 (2.5); 2.5211 (3.8); 2.5124 (51.5); 2.5079 (106.3); 2.5033 (140.8); 2.4987 (100.6); 2.4941 (47.2); 2.3348 (0.6); 2.3302 (0.9); 2.3256 (0.6); 2.0725 (0.8); 2.0599 (1.8); 2.0514 (1.8); 2.0477 (1.3); 2.0390 (3.6); 2.0304 (1.2); 2.0265 (2.0); 2.0182 (1.9); 2.0056 (0.9); 1.6063 (16.0); 1.5889 (15.9); 1.0521 (2.3); 1.0410 (5.9); 1.0354 (6.3); 1.0314 (3.3); 1.0249 (3.2); 1.0200 (6.1); 1.0144 (6.0); 1.0041 (2.5); 0.7975 (2.6); 0.7868 (6.7); 0.7816 (6.8); 0.7745 (6.2); 0.7693 (7.3); 0.7578 (2.1); 0.0080 (1.7); -0.0002 (55.3); -0.0085 (1.6) | |
| I-175 | 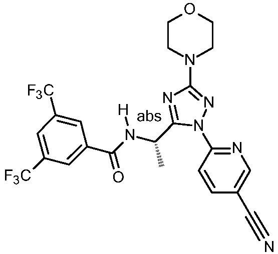 (reference compound) | ¹H-NMR (400.2 MHz, d₆-DMSO): | 540.3 [M+H]⁺ |
| | | δ = 9.5671 (1.4); 9.5497 (1.5); 8.9681 (2.5); 8.9663 (2.6); 8.9625 (2.6); 8.9607 (2.4); 8.4891 (4.5); 8.4837 (6.8); 8.4677 (2.4); 8.4620 (2.4); 8.3204 (2.1); 7.9428 (2.7); 7.9411 (2.6); 7.9212 (2.6); 7.9194 (2.5); 6.1456 (1.1); 6.1282 (1.7); 6.1108 (1.1); 3.6936 (2.8); 3.6826 (4.0); 3.6699 (3.2); 3.3768 (3.3); 3.3645 (3.9); 3.3530 (2.7); 3.3274 (34.9); 2.5272 (0.7); 2.5224 (1.1); 2.5138 (15.3); 2.5093 (31.2); 2.5047 (41.0); 2.5001 (29.1); 2.4955 (13.5); 2.0766 (16.0); 1.6401 (5.6); 1.6227 (5.6); 0.0080 (0.6); -0.0002 (17.7); -0.0086 (0.5) | |
| I-176 | 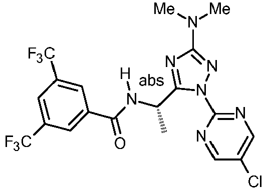 (reference compound) | ¹H-NMR (400.2 MHz, d₆-DMSO): | 508.1 [M+H]⁺ |
| | | δ = 9.5501 (0.6); 9.5323 (0.6); 9.0015 (9.0); 8.4624 (2.2); 8.3157 (1.1); 6.0276 (0.5); 6.0100 (0.8); 5.9924 (0.5); 3.3295 (62.2); 2.9473 (16.0); 2.5260 (0.6); 2.5214 (0.9); 2.5126 (14.0); 2.5081 (29.2); 2.5035 (38.8); 2.4988 (27.6); 2.4943 (12.9); 1.6319 (2.8); 1.6145 (2.8); -0.0002 (1.1) | |
| I-177 | 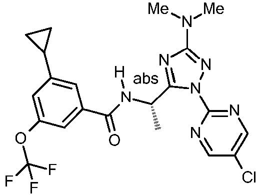 | ¹H-NMR (400.2 MHz, d₆-DMSO): | 496.2 [M+H]⁺ |
| | | δ = 9.1361 (0.7); 9.1181 (0.7); 8.9917 (9.5); 7.4850 (2.2); 7.4816 (2.1); 7.2739 (1.0); 5.9587 (0.5); 5.9410 (0.8); 5.9234 (0.5); 3.3304 (26.4); 2.9454 (16.0); 2.5219 (0.4); 2.5132 (6.1); 2.5087 (12.7); 2.5041 (16.8); 2.4994 (12.0); 2.4949 (5.6); 2.0474 (0.3); 2.0350 (0.6); 2.0224 (0.4); 2.0141 (0.3); 1.5998 (2.9); 1.5824 (2.9); 1.0490 (0.4); 1.0381 (1.0); 1.0325 (1.1); 1.0283 (0.6); 1.0222 (0.6); 1.0172 (1.0); 1.0115 (1.0); 1.0014 (0.4); 0.7965 (0.5); 0.7860 (1.1); 0.7807 (1.1); 0.7736 (1.1); 0.7685 (1.2); 0.7573 (0.4); -0.0002 (0.5) | |
| I-178 | 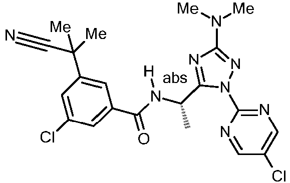 | ¹H-NMR (400.2 MHz, d₆-DMSO): | 473.2 [M+H]⁺ |
| | | δ = 9.2336 (0.7); 9.2157 (0.8); 8.9980 (9.0); 7.8686 (3.3); 7.8640 (3.6); 7.7402 (1.2); 7.7355 (2.0); 7.7309 (1.0); 5.9807 (0.5); 5.9632 (0.9); 5.9456 (0.6); 3.3281 (20.5); 2.9489 (16.0); 2.5212 (0.5); 2.5127 (6.9); 2.5083 (14.0); 2.5037 (18.2); 2.4991 (12.9); 2.4946 (6.0); 2.0758 (1.6); 1.7099 (14.5); 1.6094 (3.0); 1.5920 (2.9); -0.0002 (0.5) | |
| I-179 | 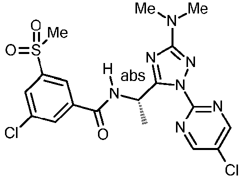 | ¹H-NMR (400.2 MHz, d₆-DMSO): | 484.1 [M+H]⁺ |
| | | δ = 9.4394 (0.7); 9.4215 (0.7); 9.0029 (9.7); 8.2963 (1.0); 8.2924 (2.0); 8.2886 (1.1); 8.2091 (1.0); 8.2045 (1.6); 8.2004 (1.1); 8.1452 (1.2); 8.1408 (1.7); 8.1362 (1.0); 6.0079 (0.5); 5.9904 (0.9); 5.9728 (0.6); 3.3296 (31.2); 3.3212 (8.8); 2.9499 (16.0); 2.5213 (0.5); 2.5127 (6.9); 2.5081 (14.2); 2.5035 (18.7); 2.4989 (13.2); 2.4944 (6.1); 1.6159 (2.9); 1.5986 (2.9); - 0.0002 (0.6) | |
| I-180 | 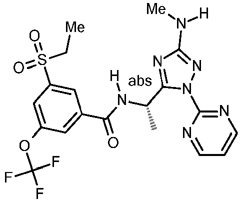 | ¹H-NMR (400.2 MHz, d₆-DMSO): | 500.1 [M+H]⁺ |
| | | δ = 9.4844 (2.4); 9.4666 (2.4); 8.8728 (12.8); 8.8607 (13.1); 8.4777 (0.4); 8.4741 (0.6); 8.4705 (0.3); 8.3836 (3.4); 8.3801 (6.1); 8.3765 (3.5); 8.2031 (0.3); 8.1322 (3.5); 8.1300 (3.2); 8.0204 (3.6); 7.9517 (0.3); 7.4523 (3.5); 7.4402 (6.6); 7.4281 (3.4); 6.2559 (0.7); 6.2437 (2.2); 6.2309 (2.2); 6.2185 (0.7); 6.0729 (0.4); 6.0559 (1.8); 6.0384 (2.8); 6.0208 (1.8); 6.0033 (0.4); 5.7584 (8.2); 3.4742 (1.8); 3.4558 (6.3); 3.4374 (6.5); 3.4237 (1.1); 3.4191 (2.0); 3.3294 (173.7); 2.8865 (10.6); 2.7516 (12.5); 2.7390 (12.4); 2.6806 (0.4); 2.6760 (0.8); 2.6715 (1.2); 2.6669 (0.8); 2.6623 (0.4); 2.5250 (3.8); 2.5203 (5.8); 2.5116 (72.9); 2.5071 (147.8); 2.5025 (191.3); 2.4979 (134.3); 2.4934 (62.7); 2.3384 (0.4); 2.3339 (0.8); 2.3293 (1.1); 2.3247 (0.8); 2.3202 (0.4); 1.6151 (9.7); 1.5978 (9.7); 1.1531 (0.8); 1.1389 (7.0); 1.1206 (16.0); 1.1021 (6.6); 0.0080 (0.6); -0.0002 (18.0); -0.0085 (0.5) | |
| I-181 | 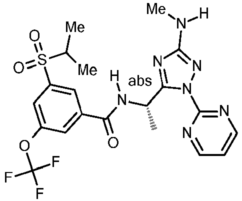 | ¹H-NMR(400.2 MHz, d6-DMSO): | 514.1 [M+H]⁺ |
| | | δ= 9.5023 (2.4); 9.4844 (2.4); 8.8699 (13.3); 8.8578 (13.6); 8.3534 (3.1); 8.3500 (5.7); 8.3465 (3.4); 8.1586 (3.4); 7.9841 (3.5); 7.4492 (3.5); 7.4371 (6.5); 7.4250 (3.4); 6.2581 (0.7); 6.2458 (2.2); 6.2331 (2.3); 6.2207 (0.6); 6.0716 (0.4); 6.0545 (1.7); 6.0370 (2.7); 6.0195 (1.7); 6.0020 (0.3); 5.7594 (1.5); 3.6369 (0.6); 3.6199 (1.7); 3.6029 (2.5); 3.5859 (1.8); 3.5690 (0.6); 3.3331 (117.8); 2.7519 (12.1); 2.7393 (12.0); 2.6763 (0.6); 2.6719 (0.8); 2.6672 (0.6); 2.5253 (2.3); 2.5205 (3.6); 2.5119 (47.7); 2.5075 (96.8); 2.5029 (127.4); 2.4983 (91.8); 2.4938 (43.6); 2.3342 (0.5); 2.3297 (0.7); 2.3251 (0.5); 1.6161 (9.4); 1.5988 (9.3); 1.1903 (12.0); 1.1754 (16.0); 1.1739 (16.0); 1.1590 (11.7); 0.0081 (0.8); -0.0002 (24.4); -0.0084 (0.7) | |
| I-182 | 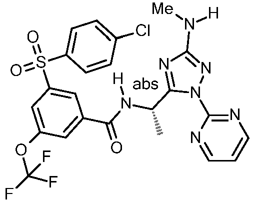 | ¹H-NMR (400.2 MHz, d₆-DMSO): | 582.0 [M+H]⁺ |
| | | δ = 9.4938 (2.6); 9.4760 (2.7); 8.8646 (15.5); 8.8525 (16.0); 8.4507 (3.7); 8.4469 (6.6); 8.4432 (3.8); 8.3161 (0.4); 8.1665 (3.7); 8.0945 (3.4); 8.0918 (3.8); 8.0890 (3.9); 8.0822 (9.6); 8.0772 (3.0); 8.0654 (3.0); 8.0603 (10.7); 8.0538 (1.2); 7.7662 (1.2); 7.7598 (10.3); 7.7546 (3.1); 7.7491 (0.4); 7.7428 (2.7); 7.7378 (9.0); 7.7315 (1.1); 7.4409 (4.2); 7.4289 (7.8); 7.4168 (4.1); 6.2435 (0.7); 6.2313 (2.6); 6.2186 (2.6); 6.2061 (0.7); 6.0510 (0.4); 6.0340 (1.9); 6.0165 (3.0); 5.9989 (1.9); 5.9815 (0.4); 3.3533 (0.9); 3.3345 (280.5); 3.3088 (0.4); 2.7495 (13.1); 2.7369 (13.0); 2.6783 (0.6); 2.6737 (0.8); 2.6690 (0.6); 2.5272 (2.3); 2.5225 (3.4); 2.5138 (46.5); 2.5093 (96.1); 2.5047 (126.4); 2.5000 (88.6); 2.4955 (40.9); 2.3361 (0.6); 2.3315 (0.8); 2.3269 (0.6); 1.6083 (10.0); 1.5910 (10.0); -0.0002 (1.5) | |
| I-183 | 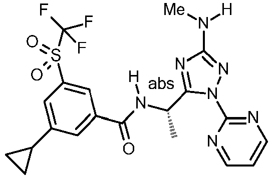 | ¹H-NMR (400.2 MHz, d₆-DMSO): | 496.0 [M+H]⁺ |
| | | δ = 9.4327 (2.8); 9.4148 (2.9); 8.8655 (15.6); 8.8534 (16.0); 8.2466 (5.4); 8.0283 (5.1); 7.9826 (3.9); 7.9789 (6.0); 7.9752 (3.2); 7.4460 (4.2); 7.4339 (7.7); 7.4218 (4.0); 6.2442 (0.8); 6.2320 (2.7); 6.2193 (2.7); 6.2069 (0.8); 6.0590 (0.4); 6.0418 (2.1); 6.0242 (3.3); 6.0067 (2.1); 5.9894 (0.4); 3.3325 (117.0); 2.8925 (0.3); 2.7539 (14.7); 2.7413 (14.5); 2.6779 (0.4); 2.6733 (0.5); 2.6687 (0.4); 2.5267 (1.8); 2.5219 (2.9); 2.5133 (32.8); 2.5089 (64.4); 2.5044 (82.5); 2.4998 (58.0); 2.4952 (26.9); 2.3357 (0.4); 2.3312 (0.5); 2.3265 (0.4); 2.2890 (0.6); 2.2763 (1.2); 2.2677 (1.3); 2.2554 (2.4); 2.2429 (1.4); 2.2347 (1.2); 2.2220 (0.6); 2.0875 (1.3); 1.6119 (11.4); 1.5946 (11.3); 1.1358 (1.5); 1.1249 (3.4); 1.1194 (3.8); 1.1090 (2.4); 1.1041 (3.5); 1.0985 (3.7); 1.0883 (1.6); 0.8920 (1.8); 0.8814 (4.2); 0.8774 (3.9); 0.8691 (3.9); 0.8648 (4.0); 0.8532 (1.3); 0.0080 (0.6); -0.0002 (16.2); -0.0085 (0.5) | |
| I-184 | 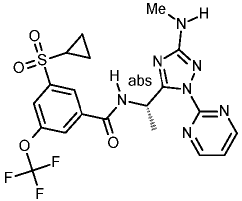 | ¹H-NMR (400.2 MHz, d₆-DMSO): | 512.2 [M+H]⁺ |
| | | δ = 9.4965 (3.1); 9.4786 (3.2); 8.8744 (15.6); 8.8623 (16.0); 8.3994 (4.2); 8.3959 (7.6); 8.3924 (4.4); 8.3154 (0.3); 8.1247 (4.4); 8.0428 (4.5); 7.4539 (4.2); 7.4419 (7.9); 7.4298 (4.1); 6.2518 (0.9); 6.2398 (3.0); 6.2271 (3.0); 6.2146 (0.9); 6.0755 (0.4); 6.0584 (2.2); 6.0409 (3.6); 6.0234 (2.3); 6.0058 (0.5); 3.3288 (201.7); 3.0567 (0.6); 3.0447 (1.4); 3.0370 (1.5); 3.0326 (1.0); 3.0253 (2.8); 3.0169 (1.1); 3.0133 (1.6); 3.0056 (1.5); 2.9936 (0.7); 2.7535 (15.5); 2.7409 (15.4); 2.6808 (0.4); 2.6764 (0.7); 2.6719 (1.0); 2.6674 (0.7); 2.5254 (3.0); 2.5206 (4.4); 2.5119 (55.8); 2.5075 (113.0); 2.5030 (148.2); 2.4984 (106.5); 2.4939 (51.3); 2.3344 (0.7); 2.3298 (0.9); 2.3252 (0.6); 2.3206 (0.3); 2.0749 (8.2); 1.6172 (12.2); 1.5999 (12.2); 1.2406 (0.5); 1.2292 (0.6); 1.2170 (2.3); 1.2044 (3.4); 1.1962 (3.9); 1.1851 (2.7); 1.1732 (1.0); 1.1607 (0.7); 1.1475 (0.5); 1.1356 (0.8); 1.1290 (1.1); 1.1140 (4.0); 1.1089 (3.5); 1.0942 (3.8); 1.0876 (3.0); 1.0722 (0.4); 0.0080 (0.9); -0.0002 (28.2); -0.0085 (0.9) | |
| I-185 | 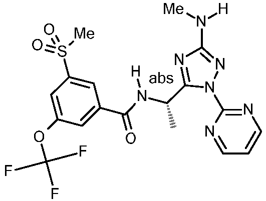 | ¹H-NMR (400.2 MHz, d₆-DMSO): | 486.2 [M+H]⁺ |
| | | δ = 9.4703 (4.2); 9.4524 (4.3); 8.8764 (8.0); 8.8645 (8.0); 8.4339 (6.3); 8.4304 (11.0); 8.4269 (6.3); 8.3159 (0.4); 8.1084 (6.6); 8.1064 (6.3); 8.0660 (6.7); 7.4556 (2.5); 7.4437 (4.4); 7.4318 (2.4); 6.2423 (2.8); 6.2299 (2.8); 6.0773 (0.5); 6.0601 (2.2); 6.0426 (3.3); 6.0252 (2.2); 6.0082 (0.5); 3.3715 (1.0); 3.3538 (52.4); 3.3314 (224.1); 2.7525 (12.8); 2.7402 (13.8); 2.6813 (0.4); 2.6769 (0.8); 2.6725 (1.0); 2.6680 (0.7); 2.6634 (0.4); 2.5259 (3.1); 2.5211 (4.8); 2.5125 (58.4); 2.5081 (116.1); 2.5035 (150.0); 2.4989 (106.5); 2.4944 (50.3); 2.3349 (0.7); 2.3303 (0.9); 2.3257 (0.7); 2.0869 (1.3); 1.6161 (16.0); 1.5988 (15.9); 0.0080 (1.0); -0.0002 (31.3); -0.0085 (1.0) | |
| I-186 | 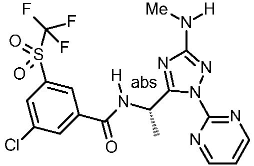 | ¹H-NMR (400.2 MHz, d₆-DMSO): | 490.1 [M+H]⁺ |
| | | δ = 9.5822 (2.9); 9.5644 (3.0); 8.8714 (15.3); 8.8593 (15.6); 8.5232 (3.8); 8.5190 (6.3); 8.5147 (4.5); 8.4654 (5.8); 8.3989 (5.2); 7.4518 (4.1); 7.4398 (7.6); 7.4277 (4.0); 6.2637 (0.8); 6.2517 (2.8); 6.2390 (2.8); 6.2265 (0.8); 6.0750 (0.4); 6.0575 (2.2); 6.0400 (3.5); 6.0225 (2.2); 6.0052 (0.4); 3.3312 (82.1); 2.7554 (14.9); 2.7428 (14.7); 2.6784 (0.3); 2.6738 (0.5); 2.6692 (0.3); 2.5270 (1.5); 2.5223 (2.4); 2.5137 (27.9); 2.5092 (55.9); 2.5047 (72.8); 2.5001 (52.1); 2.4957 (24.8); 2.3360 (0.3); 2.3316 (0.4); 2.0877 (16.0); 1.6139 (11.8); 1.5965 (11.7); 0.0080 (0.5); - 0.0002 (15.3); -0.0084 (0.5) | |
| I-187 | 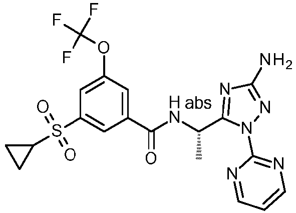 | ¹H-NMR (400.2 MHz, d₆-DMSO): | 498.1 [M+H]⁺ |
| | | δ = 9.4849 (2.4); 9.4670 (2.5); 8.8611 (15.6); 8.8490 (16.0); 8.4905 (0.3); 8.4034 (3.5); 8.3998 (6.5); 8.3962 (3.6); 8.3616 (0.7); 8.3497 (0.7); 8.3145 (2.0); 8.1298 (3.5); 8.1274 (3.2); 8.0406 (3.5); 7.4458 (4.1); 7.4337 (7.6); 7.4216 (4.0); 6.7560 (0.4); 6.0651 (0.4); 6.0482 (1.9); 6.0306 (2.9); 6.0130 (1.9); 5.9960 (0.4); 5.7460 (7.2); 3.3959 (0.4); 3.3284 (922.2); 3.2987 (0.8); 3.2899 (0.4); 3.0579 (0.6); 3.0457 (1.2); 3.0381 (1.3); 3.0332 (0.8); 3.0263 (2.5); 3.0182 (0.8); 3.0143 (1.3); 3.0065 (1.3); 2.9944 (0.6); 2.6804 (1.2); 2.6759 (2.8); 2.6713 (3.9); 2.6668 (2.7); 2.6621 (1.3); 2.5248 (11.0); 2.5202 (16.0); 2.5115 (220.5); 2.5070 (457.2); 2.5024 (601.8); 2.4977 (422.1); 2.4932 (195.1); 2.3384 (1.2); 2.3338 (2.6); 2.3292 (3.7); 2.3245 (2.6); 2.3200 (1.2); 1.6139 (9.8); 1.5965 (9.8); 1.4662 (0.5); 1.4485 (0.6); 1.2413 (0.4); 1.2287 (0.5); 1.2174 (1.9); 1.2039 (2.8); 1.1958 (3.1); 1.1848 (2.0); 1.1735 (0.8); 1.1615 (0.6); 1.1487 (0.4); 1.1357 (0.7); 1.1284 (0.9); 1.1140 (3.3); 1.1086 (2.7); 1.0942 (3.2); 1.0868 (2.3); 1.0723 (0.4); -0.0001 (8.2) | |
| I-188 | 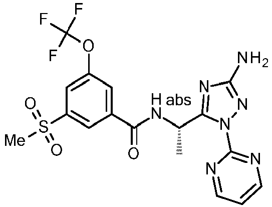 | ¹H-NMR (400.2 MHz, d₆-DMSO): | 472.1 [M+H]⁺ |
| | | δ = 9.4599 (3.3); 9.4420 (3.4); 8.8636 (15.6); 8.8516 (16.0); 8.4389 (4.7); 8.4355 (7.9); 8.4321 (4.6); 8.4182 (0.4); 8.4146 (0.5); 8.4110 (0.3); 8.1330 (0.5); 8.1142 (4.9); 8.0652 (4.9); 7.9532 (1.9); 7.4480 (4.2); 7.4359 (7.9); 7.4239 (4.1); 6.0690 (0.5); 6.0521 (2.4); 6.0345 (3.7); 6.0169 (2.4); 5.9995 (0.5); 5.7491 (9.4); 3.3833 (2.7); 3.3543 (37.6); 3.3318 (177.5); 2.8920 (14.2); 2.7326 (12.0); 2.6769 (0.8); 2.6725 (1.0); 2.6679 (0.7); 2.6634 (0.4); 2.5588 (0.3); 2.5257 (3.7); 2.5123 (65.9); 2.5080 (126.9); 2.5035 (160.2); 2.4990 (113.2); 2.4946 (54.0); 2.3348 (0.7); 2.3303 (1.0); 2.3258 (0.7); 1.6138 (13.0); 1.5964 (12.9); 0.1461 (0.4); 0.0078 (3.0); 0.0000 (77.4); -0.0082 (2.8); - 0.1494 (0.4) | |
| I-189 | 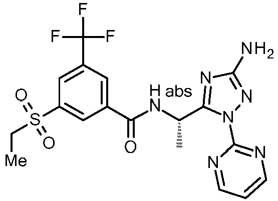 | ¹H-NMR (400.2 MHz, d₆-DMSO): | 470.1 [M+H]⁺ |
| | | δ = 9.5510 (2.4); 9.5331 (2.4); 8.8634 (12.7); 8.8513 (13.0); 8.5949 (4.6); 8.5293 (4.0); 8.3311 (4.1); 8.3150 (0.6); 7.4459 (3.5); 7.4338 (6.6); 7.4217 (3.4); 6.0888 (0.4); 6.0716 (1.7); 6.0541 (2.8); 6.0365 (1.8); 6.0190 (0.4); 5.7498 (6.8); 3.5089 (1.9); 3.4905 (6.4); 3.4721 (6.6); 3.4539 (2.1); 3.3283 (189.7); 2.6808 (0.5); 2.6763 (1.1); 2.6717 (1.5); 2.6671 (1.1); 2.6626 (0.5); 2.5252 (4.8); 2.5205 (7.1); 2.5118 (91.1); 2.5073 (185.0); 2.5028 (240.1); 2.4981 (168.5); 2.4936 (78.6); 2.3386 (0.5); 2.3341 (1.1); 2.3296 (1.5); 2.3250 (1.0); 2.3204 (0.5); 1.6224 (9.5); 1.6051 (9.5); 1.1834 (0.3); 1.1650 (0.7); 1.1469 (7.2); 1.1286 (16.0); 1.1101 (6.8); 0.0080 (1.9); -0.0001 (64.9); - 0.0085 (1.9) | |
| I-190 | 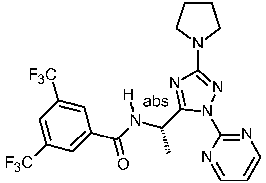 (reference compound) | ¹H-NMR (400.2 MHz, d₆-DMSO): | 500.1 [M+H]⁺ |
| | | δ = 9.5728 (2.7); 9.5550 (2.7); 8.8924 (15.6); 8.8803 (16.0); 8.4583 (9.7); 8.3145 (2.9); 8.3084 (4.4); 8.2006 (0.4); 7.4791 (4.1); 7.4670 (7.7); 7.4549 (4.0); 6.0945 (0.4); 6.0778 (2.1); 6.0602 (3.3); 6.0425 (2.1); 6.0252 (0.4); 5.7550 (1.7); 3.8100 (0.3); 3.4977 (0.3); 3.3743 (4.8); 3.3577 (12.2); 3.3285 (713.9); 2.6805 (1.3); 2.6761 (2.7); 2.6715 (3.6); 2.6670 (2.7); 2.6625 (1.3); 2.5859 (0.7); 2.5250 (11.7); 2.5203 (17.2); 2.5116 (218.2); 2.5071 (442.9); 2.5026 (576.1); 2.4980 (407.0); 2.4935 (191.2); 2.3385 (1.1); 2.3339 (2.5); 2.3294 (3.5); 2.3249 (2.5); 2.3204 (1.1); 1.9227 (0.5); 1.9079 (4.4); 1.8994 (4.7); 1.8917 (11.6); 1.8837 (4.8); 1.8752 (4.1); 1.8606 (0.6); 1.6466 (11.7); 1.6292 (11.5); 1.3427 (0.4); 1.3255 (0.4); 1.2041 (0.3); 1.0224 (0.4); 0.1458 (0.6); 0.0080 (4.9); -0.0002 (161.6); -0.0085 (4.9); -0.1497 (0.7) | |
| I-191 | 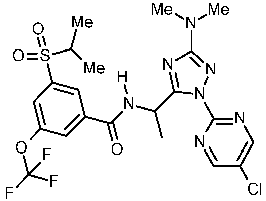 | ¹H-NMR (400.2 MHz, d₆-DMSO): | 562.0 [M+H]⁺ |
| | | δ = 9.5353 (0.7); 9.5175 (0.7); 8.9934 (8.8); 8.3398 (0.9); 8.3362 (1.7); 8.3326 (1.0); 8.1482 (1.0); 7.9856 (1.0); 6.0139 (0.5); 5.9963 (0.8); 5.9787 (0.5); 3.6137 (0.5); 3.5967 (0.7); 3.5797 (0.5); 3.3279 (34.4); 2.9513 (16.0); 2.9437 (1.2); 2.5259 (0.7); 2.5211 (1.0); 2.5125 (14.5); 2.5080 (29.8); 2.5034 (38.8); 2.4988 (27.5); 2.4942 (13.1); 1.6273 (2.8); 1.6099 (2.8); 1.1914 (3.6); 1.1753 (5.6); 1.1592 (3.5); 0.0080 (0.9); -0.0002 (28.3); -0.0086 (0.9) | |
| I-192 | 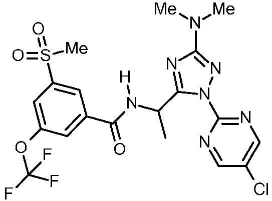 | ¹H-NMR (400.2 MHz, d₆-DMSO): | 534.0 [M+H]⁺ |
| | | δ = 9.5135 (0.8); 9.4957 (0.8); 9.0015 (8.8); 8.4198 (1.0); 8.4162 (1.9); 8.4127 (1.1); 8.1056 (1.1); 8.1034 (1.1); 8.0731 (1.2); 6.0212 (0.6); 6.0036 (0.9); 5.9860 (0.6); 3.3520 (8.9); 3.3282 (25.4); 2.9513 (16.0); 2.5261 (0.6); 2.5213 (0.8); 2.5126 (10.1); 2.5082 (20.4); 2.5037 (26.4); 2.4991 (19.0); 2.4947 (9.1); 1.6274 (3.0); 1.6100 (3.0); 0.0077 (0.8); -0.0002 (20.9); -0.0086 (0.7) | |
| I-193 | 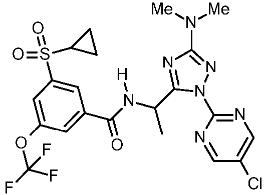 | ¹H-NMR (400.2 MHz, d₆-DMSO): | 560.0 [M+H]⁺ |
| | | δ = 9.5383 (0.7); 9.5207 (0.7); 9.0005 (8.4); 8.3860 (1.0); 8.3824 (1.8); 8.3788 (1.0); 8.1177 (1.0); 8.1154 (1.0); 8.0511 (1.1); 6.0192 (0.5); 6.0017 (0.8); 5.9841 (0.6); 3.3278 (33.7); 3.0455 (0.3); 3.0376 (0.4); 3.0260 (0.7); 3.0139 (0.4); 3.0061 (0.4); 2.9519 (16.0); 2.5257 (0.8); 2.5209 (1.1); 2.5123 (14.0); 2.5078 (27.7); 2.5033 (35.6); 2.4987 (25.0); 2.4942 (11.7); 1.6276 (2.9); 1.6102 (2.9); 1.2178 (0.6); 1.2053 (0.8); 1.1970 (0.9); 1.1858 (0.6); 1.1126 (1.0); 1.1077 (0.8); 1.0930 (0.9); 1.0863 (0.7); 0.0079 (1.0); -0.0002 (29.4); -0.0086 (1.0) | |
| I-194 | 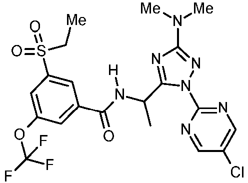 | ¹H-NMR (400.2 MHz, d₆-DMSO): | 548.0 [M+H]⁺ |
| | | δ = 9.5213 (0.7); 9.5033 (0.7); 8.9970 (8.7); 8.3700 (1.0); 8.3663 (1.8); 8.3627 (1.0); 8.1251 (1.0); 8.1227 (0.9); 8.0235 (1.0); 6.0158 (0.5); 5.9982 (0.8); 5.9806 (0.5); 3.4703 (0.5); 3.4519 (1.8); 3.4335 (1.8); 3.4152 (0.6); 3.3305 (96.3); 2.9509 (16.0); 2.6719 (0.3); 2.5255 (1.0); 2.5207 (1.4); 2.5121 (20.6); 2.5076 (42.8); 2.5030 (56.2); 2.4984 (39.8); 2.4938 (18.9); 2.3298 (0.3); 1.6265 (2.8); 1.6091 (2.8); 1.1393 (2.0); 1.1210 (4.5); 1.1026 (1.9); 0.0079 (1.3); -0.0002 (45.0); -0.0086 (1.6) | |
| I-195 | 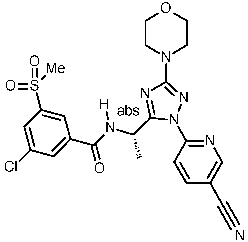 | ¹H-NMR (400.2 MHz, d₆-DMSO): | 484.0 [M+H]⁺ |
| | | δ = 9.4579 (4.1); 9.4405 (4.2); 8.9719 (7.2); 8.9702 (7.7); 8.9664 (7.7); 8.9646 (7.2); 8.4899 (6.2); 8.4843 (5.8); 8.4683 (6.5); 8.4627 (6.5); 8.3204 (5.9); 8.3166 (11.1); 8.3129 (6.7); 8.2944 (0.4); 8.2303 (6.0); 8.2258 (9.1); 8.2217 (6.1); 8.1538 (6.9); 8.1494 (10.2); 8.1449 (5.7); 7.9424 (7.3); 7.9408 (7.4); 7.9208 (7.0); 7.9191 (7.0); 6.1408 (0.6); 6.1237 (3.0); 6.1063 (4.8); 6.0889 (3.1); 6.0714 (0.6); 3.7961 (0.4); 3.6952 (8.3); 3.6842 (11.8); 3.6715 (9.3); 3.4971 (0.4); 3.3781 (10.3); 3.3660 (12.8); 3.3542 (10.4); 3.3337 (470.7); 3.3255 (60.9); 2.6811 (0.6); 2.6768 (1.2); 2.6722 (1.6); 2.6678 (1.2); 2.6632 (0.6); 2.5257 (5.3); 2.5210 (8.1); 2.5123 (99.0); 2.5078 (201.1); 2.5033 (262.4); 2.4987 (184.6); 2.4941 (86.2); 2.3392 (0.6); 2.3346 (1.2); 2.3301 (1.6); 2.3255 (1.2); 2.3210 (0.5); 1.6200 (16.0); 1.6026 (15.9); 1.2460 (0.9); 1.2300 (1.7); 1.2115 (1.1); 1.2060 (0.5); 1.1948 (0.7); 1.1892 (0.5); 0.8818 (0.3); -0.0002 (1.2) | |
| I-196 | 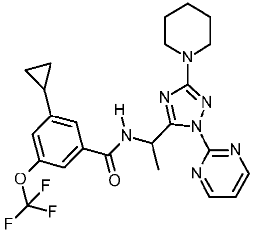 | ¹H-NMR (400.2 MHz, d₆-DMSO): | 502.1 [M+H]⁺ |
| | | δ = 9.0948 (2.7); 9.0765 (2.8); 8.8821 (15.6); 8.8700 (16.0); 8.3158 (0.7); 7.4785 (4.2); 7.4663 (9.0); 7.4610 (9.1); 7.4570 (9.4); 7.4548 (8.6); 7.2673 (3.7); 6.0095 (0.4); 5.9925 (2.0); 5.9747 (3.1); 5.9569 (2.0); 5.9392 (0.4); 3.3719 (7.3); 3.3281 (385.4); 2.6806 (0.6); 2.6761 (1.4); 2.6716 (1.9); 2.6670 (1.4); 2.6624 (0.6); 2.5251 (5.8); 2.5204 (8.6); 2.5117 (109.9); 2.5072 (225.0); 2.5027 (295.4); 2.4980 (208.3); 2.4935 (97.6); 2.3386 (0.6); 2.3340 (1.3); 2.3294 (1.8); 2.3249 (1.3); 2.3204 (0.6); 2.0610 (0.6); 2.0483 (1.2); 2.0400 (1.2); 2.0275 (2.3); 2.0150 (1.3); 2.0066 (1.2); 1.9941 (0.6); 1.5966 (11.0); 1.5792 (11.3); 1.5520 (14.1); 1.2356 (1.2); 1.0440 (1.3); 1.0339 (3.3); 1.0284 (3.7); 1.0233 (2.0); 1.0187 (2.0); 1.0130 (3.4); 1.0075 (3.5); 0.9979 (1.6); 0.7878 (1.7); 0.7778 (3.2); 0.7748 (3.7); 0.7712 (3.2); 0.7654 (3.4); 0.7626 (3.3); 0.7592 (2.6); 0.7508 (1.4); 0.0000 (1.0) | |
| I-197 | 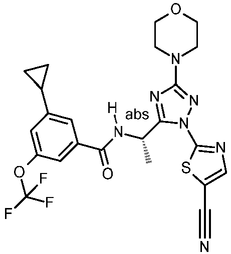 | ¹H-NMR (400.2 MHz, d₆-DMSO): | 534.4 [M+H]⁺ |
| | | δ = 9.2375 (2.8); 9.2211 (2.9); 8.5500 (16.0); 8.3106 (1.3); 7.5654 (6.0); 7.5480 (4.1); 7.2943 (4.0); 5.9711 (0.4); 5.9541 (2.0); 5.9371 (3.0); 5.9200 (2.0); 5.9027 (0.4); 3.6830 (5.7); 3.6722 (8.2); 3.6593 (6.5); 3.3602 (3117.6); 2.8920 (0.4); 2.7324 (0.3); 2.6779 (2.9); 2.6734 (3.9); 2.6689 (2.8); 2.5268 (12.0); 2.5220 (17.5); 2.5134 (235.0); 2.5090 (474.4); 2.5045 (613.6); 2.4999 (431.2); 2.4954 (201.4); 2.3357 (2.7); 2.3312 (3.7); 2.3267 (2.6); 2.0864 (0.6); 2.0734 (1.7); 2.0657 (1.4); 2.0533 (2.5); 2.0408 (1.4); 2.0323 (1.3); 2.0198 (0.7); 1.5948 (10.9); 1.5773 (10.8); 1.0602 (1.4); 1.0492 (4.2); 1.0436 (4.5); 1.0333 (2.1); 1.0282 (4.2); 1.0228 (4.2); 1.0125 (1.5); 0.8091 (1.8); 0.7985 (4.6); 0.7934 (4.4); 0.7862 (4.4); 0.7812 (4.7); 0.7698 (1.5); -0.0001 (4.1) | |
| I-198 | 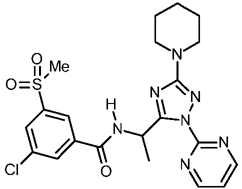 | ¹H-NMR (400.2 MHz, d₆-DMSO): | 490.2 [M+H]⁺ |
| | | δ = 9.3963 (2.7); 9.3780 (2.8); 8.8910 (12.7); 8.8789 (12.9); 8.3139 (0.4); 8.2745 (3.9); 8.2712 (6.6); 8.2676 (3.8); 8.1811 (3.6); 8.1769 (5.9); 8.1730 (3.7); 8.1360 (4.1); 8.1318 (6.0); 8.1276 (3.0); 7.4872 (3.4); 7.4751 (6.4); 7.4630 (3.2); 6.0590 (0.4); 6.0417 (2.0); 6.0241 (3.0); 6.0064 (1.9); 5.9890 (0.4); 3.3769 (8.9); 3.3378 (571.9); 3.3155 (31.0); 2.6763 (1.0); 2.6719 (1.4); 2.6675 (1.0); 2.5251 (4.5); 2.5074 (178.9); 2.5030 (224.6); 2.4985 (159.1); 2.3342 (1.0); 2.3297 (1.4); 2.3253 (1.0); 1.6150 (10.9); 1.5977 (10.9); 1.5536 (16.0); -0.0003 (1.2) | |
| I-199 | 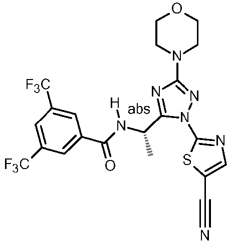 (reference compound) | ¹H-NMR (400.2 MHz, d₆-DMSO): | 546.2 [M+H]⁺ |
| | | δ = 9.6342 (2.4); 9.6178 (2.5); 8.5569 (16.0); 8.5274 (8.3); 8.3423 (3.7); 6.0400 (0.4); 6.0229 (1.8); 6.0060 (2.6); 5.9890 (1.8); 5.9714 (0.4); 3.6837 (4.8); 3.6726 (6.8); 3.6599 (5.5); 3.3771 (5.6); 3.3649 (6.8); 3.3534 (4.8); 3.3295 (110.1); 2.6778 (0.5); 2.6732 (0.6); 2.6686 (0.5); 2.5268 (1.9); 2.5221 (2.7); 2.5134 (37.5); 2.5089 (77.3); 2.5043 (101.2); 2.4997 (71.1); 2.4951 (33.3); 2.3358 (0.4); 2.3311 (0.6); 2.3265 (0.4); 1.6303 (9.5); 1.6128 (9.4); -0.0002 (0.8) | |
| I-200 | 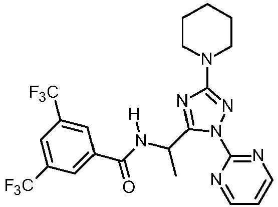 (reference compound) | ¹H-NMR (400.2 MHz, d₆-DMSO): | 514.3 [M+H]⁺ |
| | | δ = 9.5125 (2.9); 9.4944 (2.9); 8.8900 (10.5); 8.8779 (10.6); 8.4390 (10.4); 8.3068 (4.5); 7.4830 (2.9); 7.4709 (5.4); 7.4588 (2.8); 6.0807 (0.4); 6.0635 (1.8); 6.0459 (2.8); 6.0284 (1.8); 6.0110 (0.4); 3.3767 (8.5); 3.3296 (193.8); 2.6725 (1.0); 2.5076 (120.5); 2.5037 (151.9); 2.3304 (0.9); 1.6324 (10.3); 1.6151 (10.2); 1.5513 (16.0); -0.0004 (0.7) | |
| I-201 | 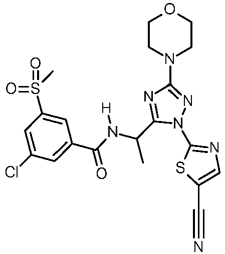 | ¹H-NMR (600.1 MHz, d₆-DMSO): | 522.0 [M+H]⁺ |
| | | δ = 9.5195 (0.2); 9.5086 (0.2); 8.5569 (0.8); 8.3507 (0.2); 8.3482 (0.4); 8.3457 (0.2); 8.2628 (0.2); 8.2598 (0.3); 8.2571 (0.2); 8.1718 (0.2); 8.1689 (0.3); 8.1659 (0.2); 5.9862 (0.1); 5.9748 (0.2); 5.9634 (0.1); 3.6809 (0.3); 3.6732 (0.4); 3.6648 (0.4); 3.3749 (0.4); 3.3667 (0.4); 3.3588 (0.3); 3.3333 (1.7); 3.3208 (16.0); 2.5225 (0.2); 2.5195 (0.2); 2.5163 (0.3); 2.5075 (4.6); 2.5045 (9.8); 2.5014 (13.8); 2.4984 (10.2); 2.4954 (4.9); 1.6084 (0.6); 1.5968 (0.6); -0.0001 (0.2) | |
| I-202 | 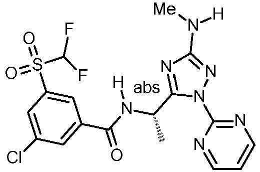 | ¹H-NMR (400.2 MHz, d₆-DMSO): | 472.0 [M+H]⁺ |
| | | δ = 9.5232 (2.6); 9.5054 (2.6); 8.8715 (15.6); 8.8594 (16.0); 8.4363 (3.6); 8.4319 (6.0); 8.4277 (4.0); 8.3475 (3.7); 8.3438 (6.5); 8.3401 (3.6); 8.1741 (3.5); 8.1697 (5.9); 8.1654 (3.2); 7.5407 (2.2); 7.4521 (4.1); 7.4400 (7.7); 7.4279 (4.1); 7.4107 (5.0); 7.2808 (2.6); 6.2589 (0.7); 6.2469 (2.4); 6.2341 (2.4); 6.2221 (0.7); 6.0661 (0.4); 6.0490 (2.1); 6.0315 (3.4); 6.0140 (2.1); 5.9966 (0.4); 3.3294 (115.6); 2.7525 (14.9); 2.7399 (14.8); 2.6768 (0.6); 2.6723 (0.8); 2.6678 (0.6); 2.5258 (2.4); 2.5211 (3.7); 2.5124 (48.8); 2.5079 (99.3); 2.5033 (129.1); 2.4987 (90.1); 2.4942 (41.7); 2.3347 (0.6); 2.3302 (0.8); 2.3255 (0.6); 1.6067 (11.4); 1.5894 (11.3); -0.0002 (0.5) | |
| I-203 | 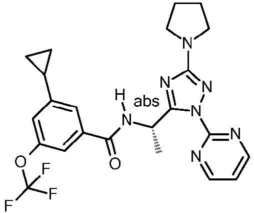 | ¹H-NMR (400.2 MHz, d₆-DMSO): | 488.2 [M+H]⁺ |
| | | δ = 9.1562 (2.6); 9.1381 (2.6); 8.8848 (15.6); 8.8728 (16.0); 8.3157 (0.5); 7.4793 (9.1); 7.4744 (11.4); 7.4621 (7.9); 7.4500 (4.0); 7.2697 (3.8); 6.0225 (0.4); 6.0052 (2.0); 5.9875 (3.2); 5.9698 (2.1); 5.9523 (0.4); 3.3717 (4.1); 3.3552 (10.8); 3.3384 (6.2); 3.3275 (203.6); 2.6806 (0.5); 2.6762 (1.0); 2.6716 (1.4); 2.6670 (1.0); 2.6626 (0.5); 2.5251 (4.3); 2.5204 (6.4); 2.5117 (82.3); 2.5072 (168.2); 2.5027 (220.1); 2.4981 (154.2); 2.4935 (71.6); 2.3386 (0.4); 2.3340 (1.0); 2.3294 (1.4); 2.3249 (1.0); 2.3204 (0.4); 2.0631 (0.6); 2.0505 (1.2); 2.0421 (1.3); 2.0383 (0.9); 2.0296 (2.5); 2.0171 (1.4); 2.0087 (1.3); 1.9961 (0.6); 1.9236 (0.5); 1.9085 (4.3); 1.8998 (4.6); 1.8921 (11.2); 1.8843 (4.6); 1.8757 (3.9); 1.8610 (0.5); 1.6131 (11.3); 1.5957 (11.2); 1.3977 (0.3); 1.2351 (0.5); 1.0455 (1.4); 1.0344 (4.0); 1.0288 (4.3); 1.0250 (2.1); 1.0184 (2.1); 1.0135 (4.1); 1.0079 (4.1); 0.9976 (1.7); 0.7945 (1.8); 0.7838 (4.6); 0.7786 (4.6); 0.7716 (4.3); 0.7663 (5.0); 0.7549 (1.4); -0.0002 (0.9) | |
| I-204 | 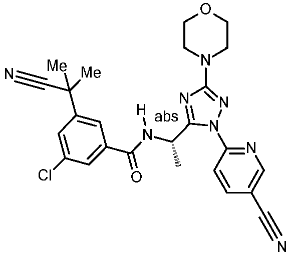 | ¹H-NMR (400.2 MHz, d₆-DMSO): | 505.4 [M+H]⁺ |
| | | δ = 9.2525 (0.8); 9.2350 (0.8); 8.9664 (1.4); 8.9647 (1.5); 8.9609 (1.5); 8.9591 (1.4); 8.4866 (1.2); 8.4810 (1.2); 8.4650 (1.3); 8.4593 (1.3); 7.9377 (1.5); 7.9360 (1.5); 7.9161 (1.5); 7.9143 (1.5); 7.8973 (0.8); 7.8936 (2.0); 7.8895 (3.0); 7.8855 (2.2); 7.8819 (0.7); 7.7472 (1.3); 7.7426 (2.2); 7.7379 (1.1); 6.0952 (0.6); 6.0778 (1.0); 6.0604 (0.6); 3.6930 (1.6); 3.6821 (2.3); 3.6693 (1.8); 3.3777 (1.9); 3.3656 (2.3); 3.3540 (1.6); 3.3292 (53.3); 2.5255 (0.8); 2.5208 (1.1); 2.5122 (15.5); 2.5077 (31.6); 2.5031 (41.4); 2.4984 (28.9); 2.4939 (13.3); 1.7117 (16.0); 1.6139 (3.2); 1.5965 (3.2); 1.3973 (0.8); -0.0002 (0.5) | |
| I-205 | 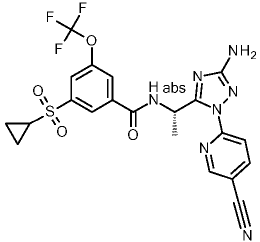 | ¹H-NMR (400.2 MHz, d₆-DMSO): | 522.2 [M+H]⁺ |
| | | δ = 9.5223 (4.2); 9.5048 (4.3); 8.9322 (6.6); 8.9305 (7.4); 8.9267 (7.2); 8.9249 (7.2); 8.4477 (7.8); 8.4464 (7.8); 8.4424 (16.0); 8.4388 (6.4); 8.4263 (6.1); 8.4207 (6.0); 8.3142 (1.0); 8.1679 (5.8); 8.1656 (5.3); 8.0559 (5.9); 7.8309 (7.0); 7.8294 (7.3); 7.8093 (6.7); 7.8077 (7.0); 6.1317 (0.6); 6.1150 (2.9); 6.0976 (4.7); 6.0803 (3.0); 6.0632 (0.6); 5.9938 (11.2); 3.3767 (1.0); 3.3328 (847.2); 3.0670 (0.8); 3.0551 (1.8); 3.0474 (2.0); 3.0356 (3.8); 3.0274 (1.4); 3.0236 (2.0); 3.0159 (2.0); 3.0040 (0.9); 2.6810 (0.8); 2.6766 (1.6); 2.6721 (2.3); 2.6675 (1.6); 2.6630 (0.8); 2.5256 (6.7); 2.5209 (10.0); 2.5122 (132.9); 2.5077 (274.4); 2.5031 (361.2); 2.4985 (254.9); 2.4940 (119.1); 2.3390 (0.7); 2.3345 (1.6); 2.3299 (2.2); 2.3253 (1.6); 2.3210 (0.8); 2.0745 (1.3); 1.6160 (15.4); 1.5986 (15.3); 1.2481 (0.6); 1.2382 (0.7); 1.2245 (2.7); 1.2102 (4.2); 1.2030 (5.5); 1.1915 (3.6); 1.1800 (1.2); 1.1681 (0.8); 1.1543 (0.6); 1.1416 (1.0); 1.1344 (1.3); 1.1191 (5.3); 1.1142 (4.6); 1.0994 (5.0); 1.0940 (4.1); 1.0768 (0.6); 0.0000 (1.5) | |
| I-206 | 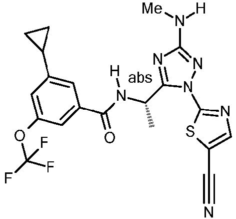 | ¹³C-NMR (150.9 MHz, d₆-DMSO): | 478.0 [M+H]⁺ |
| | | δ = 164.7995 (3.9); 164.2742 (4.6); 162.2692 (3.8); 159.7592 (4.1); 152.6531 (4.0); 148.5649 (1.8); 148.5538 (1.8); 147.5188 (3.8); 135.6386 (3.5); 123.2074 (4.4); 122.7683 (0.4); 121.5501 (2.7); 121.0678 (1.2); 119.3674 (1.1); 117.6668 (0.3); 116.9344 (3.4); 112.6459 (2.3); 101.2059 (4.2); 43.7290 (4.8); 40.3528 (0.3); 40.2358 (5.7); 40.1161 (26.7); 39.9775 (78.8); 39.8386 (154.0); 39.6995 (180.2); 39.5604 (152.5); 39.4213 (76.3); 39.2822 (24.8); 29.2656 (7.0); 18.2601 (7.0); 18.1946 (0.2); 15.1684 (6.2); 10.3230 (7.0); 10.2488 (6.9); 0.2724 (0.5) | |
| I-207 | 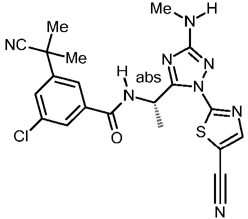 | ¹H-NMR(600.1 MHz, d₆-DMSO): | 478.0 [M+H]⁺ |
| | | δ = 9.2978 (0.1); 9.2869 (0.1); 8.5188 (0.5); 7.9606 (0.1); 7.9580 (0.2); 7.9551 (0.2); 7.9474 (0.2); 7.9446 (0.2); 7.9420 (0.1); 7.7620 (0.1); 7.7589 (0.2); 7.7558 (0.1); 5.9072 (0.1); 3.3362 (16.0); 2.7503 (0.5); 2.7420 (0.5); 2.5096 (1.3); 2.5066 (2.9); 2.5035 (4.0); 2.5005 (2.9); 2.4975 (1.3); 1.7308 (0.1); 1.7229 (1.2); 1.7218 (1.2); 1.5935 (0.4); 1.5819 (0.4); 0.0053 (0.1); -0.0001 (4.2); -0.0057 (0.1) | |
| I-208 | 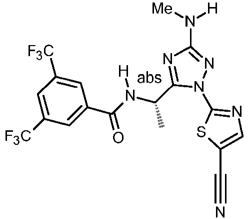 (reference compound) | ¹H-NMR(400.2 MHz, d₆-DMSO): | 490.1 [M+H]⁺ |
| | | δ = 9.6167 (2.7); 9.6004 (2.8); 8.5389 (9.7); 8.5240 (16.0); 8.3434 (4.3); 6.8834 (0.8); 6.8716 (2.5); 6.8592 (2.5); 6.8470 (0.8); 5.9915 (0.4); 5.9740 (2.0); 5.9571 (3.0); 5.9401 (2.0); 5.9225 (0.4); 3.3327 (130.4); 2.7526 (13.6); 2.7402 (13.5); 2.6780 (0.4); 2.6735 (0.6); 2.6691 (0.4); 2.5270 (1.9); 2.5222 (3.0); 2.5136 (36.3); 2.5092 (72.2); 2.5046 (92.5); 2.5000 (65.0); 2.4956 (30.5); 2.3361 (0.4); 2.3314 (0.6); 2.3268 (0.4); 2.0761 (10.4); 1.6200 (10.7); 1.6025 (10.6) | |
| I-209 | 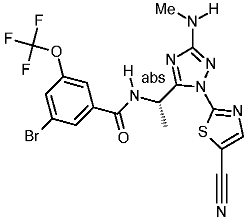 (reference compound) | ¹H-NMR (600.1 MHz, d₆-DMSO): | 518.0 [M+H]⁺ |
| | | δ = 9.3705 (0.1); 9.3596 (0.1); 8.5149 (0.6); 8.1707 (0.2); 8.1681 (0.3); 8.1655 (0.2); 7.9036 (0.1); 7.8336 (0.2); 7.8320 (0.1); 6.8621 (0.1); 6.8537 (0.1); 5.8922 (0.1); 3.3229 (16.0); 2.7484 (0.6); 2.7401 (0.6); 2.5237 (0.1); 2.5205 (0.1); 2.5174 (0.1); 2.5086 (2.3); 2.5056 (5.2); 2.5026 (7.3); 2.4995 (5.2); 2.4965 (2.4); 1.5831 (0.5); 1.5714 (0.5); 0.0054 (0.3); -0.0001 (9.0); -0.0057 (0.3) | |
| I-210 | 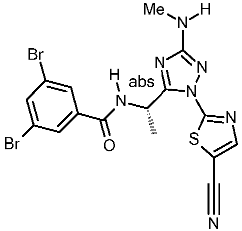 (reference compound) | ¹H-NMR (600.1 MHz, d₆-DMSO): | 511.9 [M+H]⁺ |
| | | δ = 9.3050 (0.3); 9.2941 (0.3); 8.5152 (1.2); 8.0683 (1.1); 8.0653 (1.6); 8.0505 (0.4); 8.0477 (0.5); 8.0450 (0.2); 6.8630 (0.2); 6.8546 (0.2); 5.8873 (0.2); 5.8759 (0.3); 5.8645 (0.2); 5.7526 (0.2); 3.3234 (16.0); 2.7478 (1.3); 2.7395 (1.3); 2.5239 (0.2); 2.5209 (0.2); 2.5178 (0.2); 2.5089 (3.3); 2.5060 (7.2); 2.5029 (10.0); 2.4999 (7.3); 2.4969 (3.4); 1.5703 (1.0); 1.5587 (1.0); 0.0053 (0.3); -0.0001 (8.8); -0.0057 (0.3) | |
| I-211 | 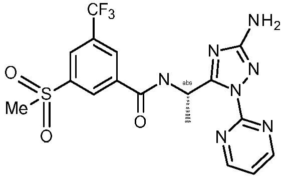 | ¹H-NMR (400.2 MHz, d₆-DMSO): | 456.1 [M+H]⁺ |
| | | δ = 9.5379 (4.1); 9.5200 (4.2); 8.9463 (0.3); 8.8675 (12.8); 8.8555 (13.0); 8.7237 (0.4); 8.6507 (2.6); 8.6414 (8.2); 8.6058 (0.3); 8.5044 (8.6); 8.4723 (2.0); 8.4033 (0.7); 8.3880 (7.3); 8.3646 (0.8); 8.3526 (0.8); 8.3491 (0.4); 8.3371 (0.4); 8.3156 (0.6); 7.4502 (4.4); 7.4382 (8.2); 7.4261 (4.2); 6.7581 (0.4); 6.0895 (0.6); 6.0725 (2.8); 6.0550 (4.4); 6.0374 (2.8); 6.0203 (0.6); 5.7507 (9.2); 5.7121 (0.5); 3.4140 (13.9); 3.3915 (2.9); 3.3776 (47.3); 3.3278 (115.4); 3.1752 (0.4); 3.1630 (0.4); 2.6764 (1.3); 2.6718 (1.8); 2.6674 (1.3); 2.6626 (0.6); 2.5252 (6.2); 2.5203 (9.9); 2.5118 (111.5); 2.5074 (222.3); 2.5029 (287.6); 2.4983 (204.4); 2.4939 (97.3); 2.3383 (0.6); 2.3342 (1.3); 2.3297 (1.8); 2.3252 (1.3); 2.3206 (0.6); 1.6221 (16.0); 1.6048 (15.9); 1.4722 (0.7); 1.4543 (0.7); 1.3961 (1.2); 1.3814 (2.2); 1.3162 (1.6); 1.2700 (0.4); 1.2519 (0.4); 1.2428 (0.4); 1.2337 (0.5); -0.0002 (0.9) | |
| I-212 | 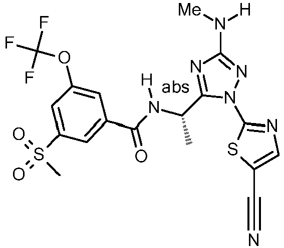 | ¹H-NMR (600.1 MHz, d₆-DMSO): | 516.0 [M+H]⁺ |
| | | δ = 9.5736 (0.2); 9.5627 (0.2); 8.5209 (1.0); 8.4946 (0.2); 8.4922 (0.5); 8.4899 (0.3); 8.1680 (0.3); 8.0981 (0.3); 6.8681 (0.2); 6.8598 (0.2); 5.9552 (0.1); 5.9439 (0.2); 5.9325 (0.2); 3.3659 (2.2); 3.3224 (16.0); 2.7516 (1.0); 2.7433 (1.0); 2.5236 (0.1); 2.5206 (0.2); 2.5175 (0.2); 2.5086 (3.1); 2.5056 (6.7); 2.5026 (9.4); 2.4995 (6.7); 2.4965 (3.1); 1.6116 (0.8); 1.6000 (0.8); 1.2346 (0.2); 0.0053 (0.2); -0.0001 (8.2); -0.0057 (0.3) | |
| I-213 | 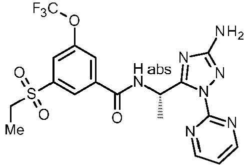 | ¹H-NMR (600.1 MHz, d₆-DMSO): | 486.1 [M+H]⁺ |
| | | δ = 9.4660 (0.5); 9.4541 (0.6); 8.8573 (2.4); 8.8493 (2.5); 8.3897 (0.6); 8.3874 (1.2); 8.3850 (0.8); 8.1377 (0.7); 8.0139 (0.7); 7.4389 (0.6); 7.4308 (1.2); 7.4228 (0.6); 6.0441 (0.4); 6.0324 (0.6); 6.0207 (0.4); 5.7411 (1.7); 3.4635 (0.4); 3.4513 (1.2); 3.4390 (1.2); 3.4268 (0.4); 3.3247 (16.0); 2.8919 (0.2); 2.6909 (1.5); 2.6180 (0.1); 2.6150 (0.2); 2.6119 (0.1); 2.5239 (0.3); 2.5209 (0.4); 2.5178 (0.4); 2.5089 (7.8); 2.5059 (17.5); 2.5029 (24.7); 2.4999 (18.6); 2.4970 (9.0); 2.3900 (0.1); 2.3867 (0.2); 2.3843 (0.1); 1.6112 (2.0); 1.5997 (2.0); 1.2431 (0.3); 1.1369 (1.4); 1.1247 (3.0); 1.1124 (1.4); 0.0053 (0.2); -0.0001 (5.0); -0.0057 (0.2) | |
| I-214 | 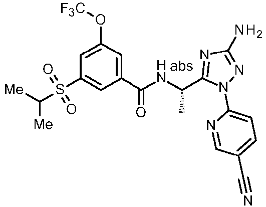 | ¹H-NMR (400.2 MHz, d₆-DMSO): | 524.0 [M+H]⁺ |
| | | δ = 9.5213 (2.6); 9.5039 (2.6); 8.9215 (4.2); 8.9199 (4.4); 8.9160 (4.5); 8.9142 (4.2); 8.4444 (3.5); 8.4388 (3.3); 8.4227 (3.7); 8.4171 (3.7); 8.3970 (3.4); 8.3935 (6.1); 8.3901 (3.5); 8.3149 (0.7); 8.1973 (3.6); 7.9926 (3.7); 7.8276 (4.5); 7.8260 (4.4); 7.8059 (4.2); 7.8043 (4.2); 6.1288 (0.4); 6.1116 (1.8); 6.0943 (2.8); 6.0769 (1.8); 6.0600 (0.4); 5.9958 (7.0); 3.6449 (0.6); 3.6280 (1.9); 3.6110 (2.6); 3.5940 (1.9); 3.5771 (0.7); 3.3602 (0.4); 3.3253 (298.9); 2.6902 (1.1); 2.6805 (0.7); 2.6759 (1.4); 2.6714 (2.0); 2.6669 (1.5); 2.6623 (0.7); 2.5249 (6.6); 2.5202 (9.8); 2.5115 (111.4); 2.5070 (223.1); 2.5024 (295.0); 2.4979 (218.5); 2.4934 (107.6); 2.3384 (0.7); 2.3339 (1.4); 2.3293 (2.0); 2.3248 (1.4); 2.3204 (0.7); 1.6155 (9.4); 1.5981 (9.4); 1.4345 (0.4); 1.1980 (12.3); 1.1836 (16.0); 1.1814 (16.0); 1.1671 (12.1); 0.1460 (1.2); 0.0081 (9.5); - 0.0001 (287.6); -0.0084 (10.2); -0.1495 (1.2) | |
| I-215 | 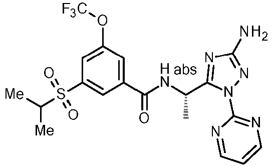 | ¹H-NMR (400.2 MHz, d₆-DMSO): | 500.0 [M+H]⁺ |
| | | δ = 9.4874 (2.5); 9.4695 (2.6); 8.8559 (15.2); 8.8438 (15.7); 8.3595 (3.4); 8.3559 (6.3); 8.3523 (3.6); 8.3150 (1.0); 8.1636 (3.5); 8.1613 (3.2); 7.9783 (3.5); 7.4394 (4.0); 7.4273 (7.5); 7.4152 (3.9); 6.0644 (0.4); 6.0472 (1.8); 6.0296 (2.9); 6.0121 (1.8); 5.9946 (0.4); 5.7477 (7.3); 3.8306 (1.3); 3.6364 (0.7); 3.6195 (1.9); 3.6025 (2.7); 3.5855 (2.0); 3.5685 (0.7); 3.3258 (420.1); 2.6804 (0.8); 2.6759 (1.9); 2.6713 (2.6); 2.6667 (1.9); 2.6621 (0.9); 2.5248 (8.6); 2.5201 (12.2); 2.5114 (139.1); 2.5069 (284.2); 2.5022 (380.2); 2.4976 (281.2); 2.4931 (137.4); 2.3383 (0.8); 2.3337 (1.8); 2.3291 (2.5); 2.3245 (1.8); 2.3200 (0.8); 1.6137 (9.7); 1.5963 (9.6); 1.1922 (12.9); 1.1778 (16.0); 1.1755 (16.0); 1.1612 (12.4); 0.1459 (1.8); 0.0219 (0.4); 0.0081 (14.0); -0.0002 (429.0); -0.0085 (14.4); -0.0251 (0.4); -0.1495 (1.8) | |
| I-216 | 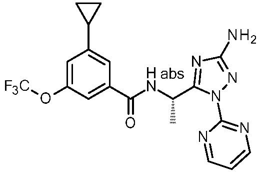 | ¹H-NMR (600.1 MHz, d₆-DMSO): | 434.2 [M+H]⁺ |
| | | δ = 8.8495 (0.2); 8.8415 (0.2); 7.4260 (0.1); 5.7100 (0.1); 3.3214 (16.0); 2.5229 (0.2); 2.5198 (0.2); 2.5167 (0.2); 2.5079 (3.3); 2.5049 (7.4); 2.5018 (10.5); 2.4987 (7.5); 2.4956 (3.3); 1.5801 (0.2); 1.5685 (0.2) | |
| I-217 | 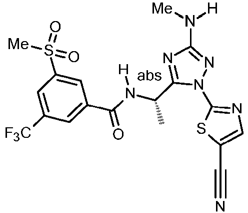 | ¹H-NMR (400.2 MHz, d₆-DMSO): | 500.0 [M+H]⁺ |
| | | δ = 9.6580 (2.6); 9.6416 (2.7); 8.6980 (4.8); 8.5662 (4.2); 8.5266 (16.0); 8.4225 (4.2); 8.4213 (4.2); 6.8886 (0.7); 6.8766 (2.4); 6.8642 (2.4); 6.8517 (0.7); 6.0006 (0.4); 5.9837 (1.8); 5.9668 (2.7); 5.9497 (1.8); 5.9325 (0.4); 3.3909 (28.3); 3.3272 (115.6); 2.7533 (12.2); 2.7409 (12.2); 2.6767 (0.5); 2.6720 (0.7); 2.6674 (0.5); 2.5256 (2.4); 2.5209 (3.5); 2.5122 (40.7); 2.5077 (80.8); 2.5031 (105.9); 2.4984 (77.5); 2.4939 (37.4); 2.3346 (0.5); 2.3299 (0.7); 2.3253 (0.5); 2.0747 (3.1); 1.6238 (9.4); 1.6064 (9.4); 1.2552 (0.6); 1.2398 (1.0); 1.2238 (0.6); 0.1459 (0.5); 0.0080 (4.0); -0.0002 (115.6); -0.0086 (3.7); -0.1495 (0.5) | |
| I-218 | 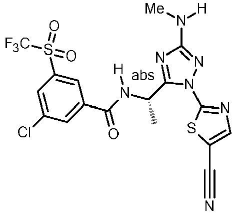 | ¹H-NMR (400.2 MHz, d₆-DMSO): | 520.1 [M+H]⁺ |
| | | δ = 9.6769 (3.1); 9.6607 (3.2); 8.5714 (3.9); 8.5674 (6.5); 8.5630 (4.6); 8.5252 (6.0); 8.5145 (16.0); 8.4330 (3.2); 8.4292 (5.2); 6.8953 (0.8); 6.8834 (2.9); 6.8709 (2.9); 6.8587 (0.8); 5.9716 (0.4); 5.9542 (2.1); 5.9373 (3.1); 5.9204 (2.1); 5.9032 (0.4); 3.3279 (167.1); 2.7544 (13.9); 2.7420 (13.9); 2.6770 (0.7); 2.6724 (0.9); 2.6678 (0.7); 2.5259 (3.0); 2.5211 (4.4); 2.5124 (51.9); 2.5080 (102.7); 2.5034 (134.8); 2.4988 (99.8); 2.4943 (49.2); 2.3347 (0.6); 2.3303 (0.9); 2.3256 (0.6); 1.6114 (11.0); 1.5939 (11.0); 0.1460 (0.6); 0.0080 (4.6); -0.0002 (132.1); -0.0084 (5.0); - 0.1495 (0.6) | |
| I-219 | 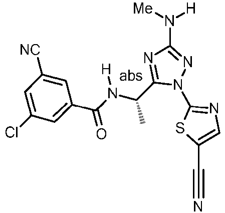 (reference compound) | ¹H-NMR (400.2 MHz, d₆-DMSO): | 413.0 [M+H]⁺ |
| | | δ = 9.3799 (3.6); 9.3636 (3.8); 8.5182 (16.0); 8.2801 (8.5); 8.2766 (6.0); 8.2628 (4.2); 8.2582 (6.8); 8.2547 (4.4); 8.2305 (5.6); 8.2264 (7.0); 8.2219 (3.9); 6.8881 (1.0); 6.8764 (3.3); 6.8639 (3.3); 6.8519 (1.0); 5.9310 (0.5); 5.9140 (2.4); 5.8970 (3.6); 5.8799 (2.4); 5.8626 (0.5); 3.9031 (1.0); 3.3275 (125.4); 2.7502 (15.9); 2.7379 (15.9); 2.6764 (0.6); 2.6720 (0.8); 2.6676 (0.6); 2.5253 (2.9); 2.5076 (94.7); 2.5031 (123.2); 2.4986 (93.2); 2.3344 (0.6); 2.3299 (0.8); 2.3254 (0.6); 2.0750 (6.9); 1.5871 (12.9); 1.5697 (12.9); 0.1459 (0.5); 0.0078 (4.0); -0.0002 (98.7); -0.0085 (4.1); -0.1495 (0.5) | |
| I-220 | 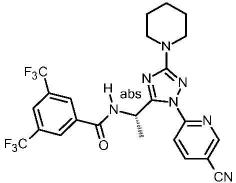 (reference compound) | ¹H-NMR (400.2 MHz, d₆-DMSO): | 538.1 [M+H]⁺ |
| | | δ = 9.5504 (3.6); 9.5327 (3.7); 8.9502 (5.8); 8.9486 (6.2); 8.9448 (6.2); 8.9431 (5.9); 8.4787 (12.1); 8.4647 (5.1); 8.4591 (4.8); 8.4430 (5.3); 8.4374 (5.3); 8.3208 (5.4); 7.9303 (6.2); 7.9288 (6.1); 7.9086 (5.9); 7.9070 (5.8); 6.1572 (0.5); 6.1402 (2.6); 6.1227 (4.1); 6.1052 (2.6); 6.0877 (0.5); 3.5689 (6.3); 3.3977 (9.3); 3.3308 (464.2); 2.6812 (0.6); 2.6769 (1.3); 2.6724 (1.9); 2.6678 (1.4); 2.6632 (0.6); 2.5258 (6.0); 2.5211 (8.6); 2.5124 (108.2); 2.5080 (218.9); 2.5034 (286.3); 2.4988 (205.9); 2.4943 (98.6); 2.3393 (0.6); 2.3348 (1.3); 2.3302 (1.8); 2.3257 (1.3); 2.3213 (0.6); 1.6293 (13.8); 1.6119 (13.8); 1.5520 (16.0); 0.1459 (1.1); 0.0079 (8.1); -0.0002 (258.9); -0.0086 (8.7); -0.1495 (1.1) | |
| I-221 | 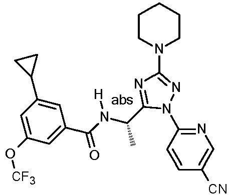 | ¹H-NMR (400.2 MHz, d₆-DMSO): | 526.1 [M+H]⁺ |
| | | δ = 9.1396 (3.3); 9.1218 (3.4); 8.9390 (5.5); 8.9376 (6.2); 8.9336 (5.9); 8.9320 (5.9); 8.4594 (4.6); 8.4538 (4.4); 8.4378 (4.9); 8.4321 (4.9); 8.3154 (0.4); 7.9201 (5.7); 7.9187 (6.0); 7.8985 (5.4); 7.8969 (5.6); 7.5105 (7.2); 7.5069 (5.7); 7.5001 (4.4); 7.4974 (4.8); 7.2783 (4.6); 6.0868 (0.5); 6.0697 (2.4); 6.0521 (3.8); 6.0345 (2.4); 6.0173 (0.5); 3.3945 (9.0); 3.3601 (0.7); 3.3295 (282.1); 2.6808 (0.5); 2.6765 (1.0); 2.6720 (1.4); 2.6674 (1.0); 2.6629 (0.5); 2.5254 (4.4); 2.5207 (6.8); 2.5120 (82.1); 2.5076 (164.0); 2.5030 (213.2); 2.4984 (153.8); 2.4939 (74.3); 2.3390 (0.4); 2.3344 (1.0); 2.3298 (1.3); 2.3253 (1.0); 2.3207 (0.4); 2.0748 (1.0); 2.0620 (1.4); 2.0536 (1.5); 2.0411 (2.8); 2.0286 (1.6); 2.0202 (1.5); 2.0077 (0.8); 1.5960 (13.3); 1.5786 (13.9); 1.5531 (16.0); 1.0531 (1.7); 1.0421 (4.6); 1.0365 (4.9); 1.0326 (2.6); 1.0262 (2.6); 1.0212 (4.7); 1.0156 (4.7); 1.0054 (1.9); 0.7998 (2.0); 0.7893 (5.2); 0.7840 (5.1); 0.7770 (4.9); 0.7718 (5.4); 0.7605 (1.6); 0.1458 (0.8); 0.0079 (6.1); -0.0002 (175.2); -0.0086 (5.9); -0.1497 (0.8) | |
| I-222 | 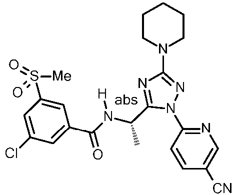 | ¹H-NMR (400.2 MHz, d₆-DMSO): | 514.2 [M+H]⁺ |
| | | δ = 9.4424 (3.3); 9.4247 (3.4); 8.9532 (5.5); 8.9517 (5.8); 8.9477 (5.8); 8.9461 (5.5); 8.4649 (4.6); 8.4593 (4.4); 8.4432 (4.9); 8.4376 (4.9); 8.3205 (4.5); 8.3167 (8.6); 8.3131 (5.4); 8.2295 (4.6); 8.2251 (7.2); 8.2209 (4.8); 8.1536 (5.0); 8.1492 (7.8); 8.1448 (4.2); 7.9300 (5.9); 7.9286 (5.8); 7.9084 (5.5); 7.9069 (5.5); 6.1395 (0.5); 6.1226 (2.4); 6.1051 (3.8); 6.0876 (2.4); 6.0701 (0.5); 3.3996 (8.9); 3.3654 (1.2); 3.3357 (467.2); 3.3255 (44.4); 2.6814 (0.4); 2.6769 (1.0); 2.6724 (1.4); 2.6678 (1.0); 2.6633 (0.5); 2.5259 (4.1); 2.5212 (6.1); 2.5124 (79.2); 2.5080 (160.6); 2.5034 (210.3); 2.4988 (151.0); 2.4943 (72.3); 2.3394 (0.5); 2.3348 (1.0); 2.3302 (1.3); 2.3257 (1.0); 2.3212 (0.5); 1.6121 (12.9); 1.5947 (13.0); 1.5549 (16.0); 0.1459 (0.8); 0.0080 (5.8); -0.0002 (182.6); -0.0085 (5.8); -0.0164 (0.4); -0.0200 (0.3); -0.1496 (0.8) | |
| I-223 | 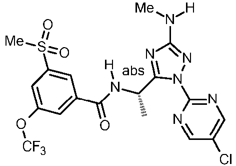 | ¹H-NMR (400.2 MHz, d₆-DMSO): | 520.0 [M+H]⁺ |
| | | δ = 9.4855 (1.4); 9.4679 (1.4); 8.9806 (16.0); 8.4468 (2.0); 8.4432 (3.4); 8.4396 (1.9); 8.3151 (0.4); 8.1264 (2.0); 8.1241 (1.8); 8.0726 (2.0); 6.3404 (0.4); 6.3290 (1.3); 6.3163 (1.3); 6.3041 (0.4); 6.0180 (1.0); 6.0006 (1.6); 5.9830 (1.0); 3.3556 (15.9); 3.3278 (174.0); 2.7481 (6.6); 2.7355 (6.6); 2.6806 (0.4); 2.6759 (0.8); 2.6715 (1.0); 2.6669 (0.7); 2.6626 (0.4); 2.5249 (3.8); 2.5202 (5.8); 2.5115 (59.4); 2.5071 (115.7); 2.5025 (150.0); 2.4979 (108.9); 2.4934 (52.3); 2.3339 (0.7); 2.3293 (0.9); 2.3248 (0.7); 1.6068 (5.2); 1.5895 (5.1); 0.1460 (0.5); 0.0080 (4.6); -0.0002 (128.0); -0.0085 (4.2); -0.1495 (0.5) | |
| I-224 | 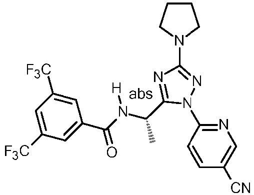 (reference compound) | ¹H-NMR (400.2 MHz, d₆-DMSO): | 524.1 [M+H]⁺ |
| | | δ = 9.6120 (4.0); 9.5944 (4.1); 8.9554 (7.1); 8.9537 (7.6); 8.9499 (7.6); 8.9480 (7.1); 8.4933 (13.8); 8.4640 (6.2); 8.4583 (5.9); 8.4423 (6.6); 8.4366 (6.6); 8.3234 (7.0); 8.1753 (0.4); 7.9317 (7.4); 7.9301 (7.3); 7.9101 (7.0); 7.9084 (6.9); 6.1772 (0.6); 6.1601 (3.0); 6.1426 (4.8); 6.1251 (3.0); 6.1079 (0.6); 3.4498 (0.3); 3.3842 (5.5); 3.3676 (14.1); 3.3512 (6.1); 3.3277 (246.2); 2.6775 (3.3); 2.6725 (2.1); 2.6679 (1.4); 2.6634 (0.7); 2.5260 (6.5); 2.5213 (9.6); 2.5126 (106.1); 2.5081 (211.9); 2.5035 (278.3); 2.4989 (202.3); 2.4943 (97.5); 2.3394 (0.6); 2.3349 (1.3); 2.3303 (1.8); 2.3258 (1.2); 2.3212 (0.6); 1.9258 (0.7); 1.9112 (6.0); 1.9025 (6.7); 1.8949 (15.6); 1.8872 (6.6); 1.8785 (5.5); 1.8635 (0.7); 1.6422 (16.0); 1.6247 (15.9); 1.2079 (1.9); 1.1915 (2.0); 1.1852 (1.6); 1.1673 (0.6); 0.1460 (0.8); 0.0080 (6.9); - 0.0001 (205.3); -0.0085 (6.5); -0.1496 (0.8) | |
| I-225 | 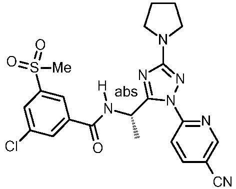 | ¹H-NMR (400.2 MHz, d₆-DMSO): | 500.1 [M+H]⁺ |
| | | δ = 9.5012 (4.1); 9.4836 (4.3); 8.9579 (7.0); 8.9563 (7.2); 8.9524 (7.6); 8.9507 (7.0); 8.4632 (5.8); 8.4575 (5.5); 8.4415 (6.2); 8.4358 (6.2); 8.3295 (5.6); 8.3257 (10.6); 8.3220 (6.3); 8.3155 (0.6); 8.2391 (5.8); 8.2346 (8.8); 8.2305 (6.0); 8.1541 (6.3); 8.1497 (9.7); 8.1452 (5.5); 7.9302 (7.3); 7.9287 (7.2); 7.9085 (6.9); 7.9070 (6.8); 6.1580 (0.6); 6.1406 (3.0); 6.1231 (4.7); 6.1056 (3.0); 6.0882 (0.6); 3.3882 (5.7); 3.3718 (15.0); 3.3556 (6.2); 3.3288 (192.6); 2.6770 (0.7); 2.6724 (1.0); 2.6679 (0.7); 2.6634 (0.3); 2.5259 (3.2); 2.5212 (4.7); 2.5125 (54.7); 2.5080 (109.7); 2.5035 (144.7); 2.4989 (106.6); 2.4944 (52.2); 2.3348 (0.6); 2.3303 (0.9); 2.3258 (0.7); 1.9284 (0.7); 1.9133 (6.1); 1.9046 (6.8); 1.8970 (16.0); 1.8896 (6.9); 1.8806 (5.6); 1.8661 (0.7); 1.6250 (15.6); 1.6076 (15.5); 0.1460 (0.4); 0.0080 (3.3); -0.0002 (96.8); -0.0085 (3.1); -0.1496 (0.4) | |
| I-226 | 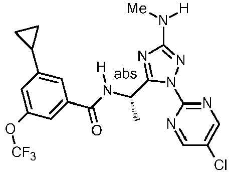 | ¹H-NMR (400.2 MHz, d₆-DMSO): | 482.0 [M+H]⁺ |
| | | δ = 9.1022 (1.2); 9.0844 (1.3); 8.9702 (16.0); 7.5144 (3.0); 7.5107 (3.0); 7.5050 (1.8); 7.5025 (1.5); 7.2725 (1.7); 6.3164 (0.3); 6.3044 (1.2); 6.2918 (1.2); 6.2793 (0.3); 5.9594 (0.9); 5.9418 (1.4); 5.9242 (0.9); 3.3303 (41.3); 2.7465 (6.2); 2.7339 (6.2); 2.5263 (0.9); 2.5216 (1.2); 2.5129 (14.0); 2.5084 (27.9); 2.5037 (36.7); 2.4991 (26.7); 2.4946 (12.8); 2.0561 (0.5); 2.0477 (0.6); 2.0438 (0.4); 2.0353 (1.1); 2.0265 (0.4); 2.0227 (0.6); 2.0144 (0.6); 1.5809 (4.9); 1.5635 (4.8); 1.3975 (8.0); 1.0499 (0.6); 1.0389 (1.8); 1.0333 (1.9); 1.0294 (0.8); 1.0228 (0.8); 1.0179 (1.8); 1.0123 (1.9); 1.0021 (0.7); 0.7980 (0.8); 0.7876 (2.0); 0.7823 (1.9); 0.7751 (1.9); 0.7701 (2.0); 0.7588 (0.6); 0.0079 (0.9); -0.0002 (27.4); -0.0086 (0.9) | |
| I-227 | 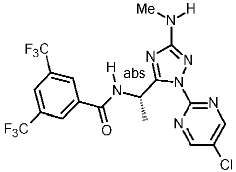 (reference compound) | ¹H-NMR (400.2 MHz, d₆-DMSO): | 494.0 [M+H]⁺ |
| | | δ = 9.5250 (1.2); 9.5073 (1.3); 8.9812 (16.0); 8.4882 (4.2); 8.3173 (1.9); 6.3376 (0.4); 6.3253 (1.2); 6.3126 (1.2); 6.2999 (0.3); 6.0284 (0.9); 6.0109 (1.4); 5.9935 (0.9); 3.3285 (47.2); 2.7484 (6.2); 2.7358 (6.2); 2.6728 (0.4); 2.5263 (1.3); 2.5217 (2.0); 2.5129 (20.2); 2.5084 (40.1); 2.5038 (52.5); 2.4992 (38.5); 2.4947 (18.8); 2.3307 (0.3); 1.6123 (4.9); 1.5949 (4.8); 1.3976 (8.8); 0.0080 (1.3); -0.0002 (37.0); - 0.0085 (1.2) | |
| I-228 | 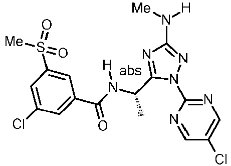 | ¹H-NMR (400.2 MHz, d₆-DMSO): | 470.0 [M+H]⁺ |
| | | δ = 9.4079 (1.4); 9.3901 (1.4); 8.9814 (16.0); 8.3188 (2.0); 8.3150 (3.7); 8.3112 (2.3); 8.2261 (2.0); 8.2216 (3.1); 8.2176 (2.2); 8.1458 (2.2); 8.1414 (3.3); 8.1370 (2.0); 6.3408 (0.4); 6.3290 (1.3); 6.3163 (1.3); 6.3039 (0.4); 6.0058 (1.0); 5.9884 (1.6); 5.9708 (1.0); 3.3744 (0.4); 3.3382 (169.9); 3.3243 (17.2); 2.7478 (6.5); 2.7353 (6.5); 2.6771 (0.3); 2.6727 (0.4); 2.6682 (0.3); 2.5260 (1.4); 2.5212 (2.2); 2.5125 (26.6); 2.5081 (52.9); 2.5036 (69.3); 2.4990 (52.4); 2.4946 (26.5); 2.3303 (0.4); 1.5944 (5.3); 1.5771 (5.3); 0.0079 (1.9); -0.0002 (52.9); -0.0085 (1.9) | |
| I-229 | 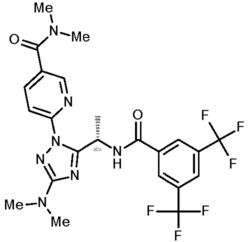 | ¹H-NMR (400.2 MHz, d₆-DMSO): | 544.2 [M+H]⁺ |
| | | δ = 9.5392 (0.7); 9.5211 (0.7); 8.5148 (1.2); 8.5132 (1.3); 8.5091 (1.3); 8.5074 (1.2); 8.4758 (2.4); 8.3151 (0.8); 8.3100 (1.1); 8.0617 (0.9); 8.0560 (0.9); 8.0406 (1.1); 8.0348 (1.1); 7.8266 (1.3); 7.8250 (1.3); 7.8055 (1.1); 7.8038 (1.2); 6.1398 (0.5); 6.1221 (0.8); 6.1046 (0.5); 3.3277 (147.4); 3.0013 (1.5); 2.9539 (16.0); 2.9207 (1.6); 2.6760 (0.6); 2.6714 (0.8); 2.6668 (0.6); 2.5249 (3.1); 2.5202 (4.7); 2.5115 (50.4); 2.5070 (100.0); 2.5024 (130.8); 2.4978 (94.3); 2.4933 (45.0); 2.3338 (0.6); 2.3293 (0.8); 2.3247 (0.6); 1.6416 (2.8); 1.6242 (2.8); 0.1459 (0.4); 0.0080 (3.6); -0.0002 (101.9); -0.0085 (3.2); -0.1496 (0.4) | |
| I-230 | 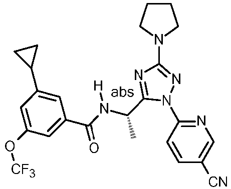 | ¹H-NMR (400.2 MHz, d₆-DMSO): | 512.1 [M+H]⁺ |
| | | δ = 9.2024 (4.0); 9.1848 (4.2); 8.9430 (6.9); 8.9415 (7.3); 8.9376 (7.4); 8.9359 (6.9); 8.4575 (5.6); 8.4519 (5.3); 8.4359 (6.0); 8.4302 (6.0); 8.3161 (0.4); 7.9218 (7.3); 7.9203 (7.1); 7.9002 (6.9); 7.8986 (6.6); 7.5235 (8.7); 7.5200 (6.2); 7.5105 (5.4); 7.5078 (5.9); 7.2809 (5.7); 6.1039 (0.6); 6.0869 (3.0); 6.0694 (4.7); 6.0519 (3.0); 6.0347 (0.6); 3.3820 (5.6); 3.3656 (14.0); 3.3492 (6.1); 3.3298 (185.0); 2.6772 (0.8); 2.6727 (1.1); 2.6682 (0.8); 2.6638 (0.4); 2.5262 (3.6); 2.5214 (5.2); 2.5127 (62.9); 2.5083 (124.1); 2.5037 (161.4); 2.4991 (117.7); 2.4947 (57.2); 2.3395 (0.4); 2.3351 (0.7); 2.3306 (1.0); 2.3260 (0.7); 2.0762 (0.8); 2.0637 (1.7); 2.0552 (1.9); 2.0428 (3.5); 2.0304 (2.0); 2.0220 (1.9); 2.0094 (0.9); 1.9271 (0.7); 1.9121 (6.2); 1.9032 (7.0); 1.8958 (16.0); 1.8883 (7.0); 1.8794 (5.7); 1.8650 (0.7); 1.6102 (15.9); 1.5927 (15.8); 1.0537 (1.7); 1.0427 (5.7); 1.0372 (6.2); 1.0267 (2.7); 1.0218 (5.9); 1.0162 (5.9); 1.0062 (2.0); 0.8049 (2.4); 0.7946 (5.9); 0.7921 (5.4); 0.7892 (5.6); 0.7823 (5.9); 0.7778 (5.8); 0.7662 (2.0); 0.1460 (0.4); 0.0081 (3.4); - 0.0001 (107.6); -0.0084 (3.9); -0.1495 (0.4) | |
| I-231 | 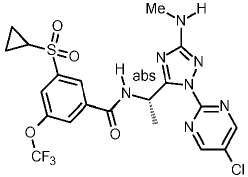 | ¹H-NMR (400.2 MHz, d₆-DMSO): | 546.0 [M+H]⁺ |
| | | δ = 9.5127 (1.2); 9.4951 (1.3); 8.9797 (16.0); 8.4157 (1.8); 8.4121 (3.2); 8.4085 (1.8); 8.1439 (1.8); 8.1416 (1.6); 8.0513 (1.8); 6.3408 (0.4); 6.3287 (1.2); 6.3160 (1.2); 6.3035 (0.3); 6.0176 (0.9); 6.0003 (1.5); 5.9827 (0.9); 3.3289 (59.4); 3.0499 (0.6); 3.0421 (0.6); 3.0374 (0.4); 3.0304 (1.2); 3.0221 (0.4); 3.0183 (0.6); 3.0106 (0.6); 2.7502 (6.3); 2.7377 (6.2); 2.6722 (0.4); 2.5256 (1.4); 2.5209 (2.2); 2.5123 (23.2); 2.5078 (45.2); 2.5032 (58.5); 2.4986 (42.3); 2.4941 (20.2); 2.3300 (0.4); 1.6095 (4.9); 1.5921 (4.8); 1.2208 (0.8); 1.2071 (1.4); 1.1992 (1.6); 1.1879 (1.0); 1.1772 (0.4); 1.1303 (0.4); 1.1158 (1.7); 1.1104 (1.4); 1.0962 (1.6); 1.0909 (1.1); 1.0887 (1.2); 0.0079 (2.0); -0.0002 (53.6); -0.0086 (1.7) | |
| I-232 | 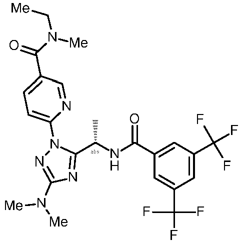 (reference compound) | ¹H-NMR (400.2 MHz, d₆-DMSO): | 558.5 [M+H]⁺ |
| | | δ = 9.5329 (0.6); 9.5148 (0.6); 8.4722 (2.5); 8.3110 (1.1); 7.8256 (1.0); 7.8045 (0.8); 6.1366 (0.5); 6.1189 (0.8); 6.1012 (0.5); 3.3279 (53.9); 2.9554 (16.0); 2.8901 (0.5); 2.6721 (0.4); 2.5256 (1.2); 2.5208 (1.9); 2.5122 (22.5); 2.5077 (45.2); 2.5031 (59.4); 2.4985 (43.2); 2.4940 (21.0); 2.3299 (0.4); 1.6413 (2.8); 1.6240 (2.8); 1.1439 (0.4); 1.0399 (0.4); 0.0080 (1.4); -0.0002 (40.2); -0.0085 (1.2) | |
| I-233 | 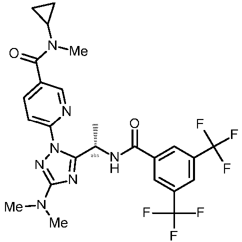 (reference compound) | ¹H-NMR (400.2 MHz, d₆-DMSO): | 570.2 [M+H]⁺ |
| | | δ = 9.5359 (0.8); 9.5180 (0.8); 8.6045 (0.8); 8.4913 (2.7); 8.3099 (1.2); 8.1432 (0.4); 8.1390 (0.4); 8.1224 (0.5); 8.1179 (0.5); 7.8081 (1.4); 7.7868 (1.3); 6.1680 (0.5); 6.1504 (0.8); 6.1327 (0.5); 3.3272 (52.8); 2.9922 (2.1); 2.9575 (16.0); 2.6719 (0.4); 2.5252 (1.5); 2.5117 (25.8); 2.5074 (50.5); 2.5028 (65.6); 2.4983 (48.1); 2.4939 (23.7); 2.3297 (0.4); 1.6395 (3.0); 1.6221 (3.0); 0.5112 (0.4); 0.4034 (0.5); 0.0079 (1.4); -0.0002 (39.6); -0.0085 (1.5) | |
| I-234 | 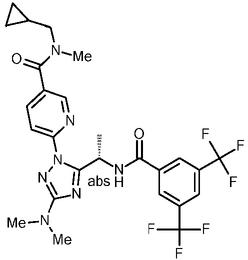 (reference compound) | ¹H-NMR (400.2 MHz, d₆-DMSO): | 584.2 [M+H]⁺ |
| | | δ = 9.5350 (0.8); 9.5173 (0.8); 8.4779 (3.1); 8.3104 (1.3); 8.0325 (0.4); 7.8288 (1.4); 7.8065 (1.2); 6.1428 (0.4); 6.1250 (0.7); 6.1076 (0.4); 3.3291 (139.5); 3.0626 (0.7); 2.9559 (16.0); 2.6762 (0.6); 2.6716 (0.8); 2.6672 (0.6); 2.5250 (3.0); 2.5114 (47.9); 2.5072 (90.9); 2.5027 (115.9); 2.4981 (85.0); 2.4938 (42.2); 2.3341 (0.5); 2.3295 (0.7); 2.3250 (0.5); 1.6413 (2.9); 1.6239 (2.8); 0.1459 (0.3); 0.0079 (3.3); -0.0002 (73.2); -0.0085 (3.1) | |
| I-235 | 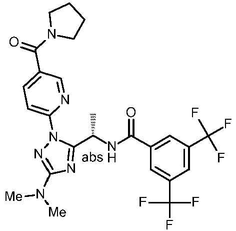 (reference compound) | ¹H-NMR (400.2 MHz, d₆-DMSO): | 570.2 [M+H]⁺ |
| | | δ = 9.5263 (0.8); 9.5082 (0.8); 8.5962 (1.3); 8.5917 (1.4); 8.4623 (2.7); 8.3148 (1.9); 8.1502 (0.9); 8.1445 (0.8); 8.1290 (1.0); 8.1232 (1.0); 7.8235 (1.3); 7.8023 (1.2); 6.1376 (0.6); 6.1202 (0.9); 6.1027 (0.5); 3.4933 (0.8); 3.4771 (1.4); 3.4595 (0.8); 3.3872 (0.6); 3.3710 (1.2); 3.3610 (1.4); 3.3260 (314.8); 2.9564 (16.0); 2.6900 (3.8); 2.6757 (1.8); 2.6711 (2.4); 2.6667 (1.8); 2.6621 (0.9); 2.5245 (9.4); 2.5111 (149.1); 2.5067 (289.2); 2.5021 (373.0); 2.4976 (272.7); 2.4931 (134.6); 2.3381 (0.8); 2.3335 (1.7); 2.3290 (2.4); 2.3243 (1.7); 1.8894 (0.6); 1.8712 (0.9); 1.8554 (0.8); 1.8396 (0.4); 1.8237 (0.3); 1.8091 (0.8); 1.7925 (0.9); 1.7763 (0.5); 1.6409 (3.0); 1.6235 (3.0); 0.1459 (1.0); 0.0079 (9.6); -0.0002 (258.2); -0.0085 (10.3); -0.1496 (1.1) | |
| I-236 | 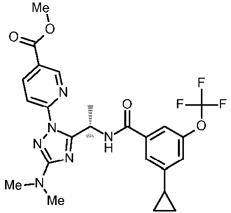 | ¹H-NMR (400.2 MHz, d₆-DMSO): | 519.2 [M+H]⁺ |
| | | δ = 9.1331 (0.7); 9.1151 (0.7); 8.9428 (1.3); 8.9413 (1.4); 8.9372 (1.3); 8.9355 (1.3); 8.4664 (1.0); 8.4606 (1.0); 8.4448 (1.1); 8.4390 (1.1); 7.9016 (1.3); 7.9002 (1.3); 7.8801 (1.2); 7.8785 (1.3); 7.4858 (2.0); 7.4822 (1.9); 7.4779 (1.2); 7.2672 (1.2); 7.1051 (0.4); 6.0893 (0.6); 6.0717 (0.9); 6.0542 (0.6); 4.0385 (0.5); 4.0207 (0.5); 3.9074 (0.3); 3.8947 (8.5); 3.3301 (48.9); 2.9641 (16.0); 2.6318 (0.6); 2.5259 (1.0); 2.5211 (1.5); 2.5125 (17.3); 2.5080 (34.3); 2.5034 (44.8); 2.4988 (32.3); 2.4942 (15.5); 2.0505 (0.3); 2.0422 (0.4); 2.0297 (0.6); 2.0172 (0.4); 2.0117 (0.3); 2.0088 (0.4); 1.9895 (2.2); 1.6146 (2.9); 1.5972 (2.9); 1.2313 (1.5); 1.2173 (0.5); 1.2097 (0.4); 1.1994 (0.4); 1.1935 (1.0); 1.1758 (1.8); 1.1581 (0.7); 1.0443 (0.4); 1.0334 (1.1); 1.0279 (1.2); 1.0238 (0.7); 1.0176 (0.6); 1.0126 (1.1); 1.0069 (1.1); 0.9969 (0.4); 0.7883 (0.5); 0.7779 (1.1); 0.7725 (1.1); 0.7655 (1.1); 0.7605 (1.1); 0.7495 (0.4); 0.0080 (1.2); -0.0002 (35.2); -0.0086 (1.2) | |
| I-237 | 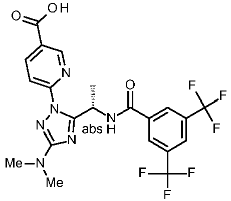 (reference compound) | ¹H-NMR (400.2 MHz, d₆-DMSO): | 517.0 [M+H]⁺ |
| | | δ = 9.5431 (0.7); 9.5253 (0.7); 8.9210 (1.2); 8.9194 (1.4); 8.9154 (1.3); 8.9136 (1.3); 8.4560 (2.5); 8.4439 (1.1); 8.4382 (1.0); 8.4224 (1.1); 8.4167 (1.1); 8.3071 (1.1); 7.8906 (1.3); 7.8891 (1.3); 7.8692 (1.2); 7.8676 (1.3); 6.1645 (0.5); 6.1470 (0.9); 6.1294 (0.5); 4.0384 (0.6); 4.0206 (0.6); 3.3305 (36.8); 2.9634 (16.0); 2.6723 (0.4); 2.6679 (0.3); 2.5259 (1.5); 2.5212 (2.3); 2.5125 (25.4); 2.5080 (50.6); 2.5034 (66.0); 2.4988 (47.4); 2.4942 (22.5); 2.3303 (0.4); 1.9895 (2.5); 1.9097 (0.8); 1.6479 (2.9); 1.6305 (2.9); 1.1935 (0.7); 1.1758 (1.4); 1.1580 (0.7); 0.0080 (1.9); 0.0059 (1.0); -0.0002 (51.5); -0.0085 (1.4) | |
| I-238 | 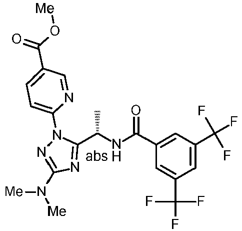 (reference compound) | ¹H-NMR (400.2 MHz, d₆-DMSO): | 531.0 [M+H]⁺ |
| | | δ = 9.5405 (0.7); 9.5224 (0.8); 8.9423 (1.3); 8.9380 (1.3); 8.4684 (1.1); 8.4625 (1.4); 8.4548 (2.7); 8.4470 (1.3); 8.4411 (1.1); 8.3155 (0.9); 8.3093 (1.2); 7.9082 (1.3); 7.8879 (1.2); 7.8866 (1.2); 6.1599 (0.6); 6.1423 (0.9); 6.1247 (0.6); 3.8906 (8.3); 3.3249 (112.0); 2.9656 (16.0); 2.6800 (0.4); 2.6757 (0.9); 2.6712 (1.2); 2.6667 (0.9); 2.5247 (4.5); 2.5199 (7.0); 2.5112 (73.5); 2.5068 (144.4); 2.5023 (187.9); 2.4977 (137.7); 2.4933 (67.9); 2.3337 (0.9); 2.3291 (1.2); 2.3245 (0.8); 1.6462 (3.0); 1.6288 (3.0); 0.1460 (0.6); 0.0080 (5.0); -0.0002 (133.0); -0.0085 (4.8); -0.1496 (0.6) | |
| I-239 | 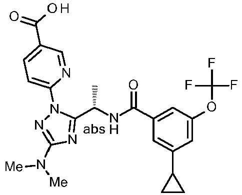 | ¹H-NMR (400.2 MHz, d₆-DMSO): | 505.1 [M+H]⁺ |
| | | δ = 9.1349 (0.7); 9.1172 (0.8); 8.9197 (1.3); 8.9183 (1.4); 8.9141 (1.3); 8.9126 (1.3); 8.4422 (1.0); 8.4365 (1.0); 8.4207 (1.1); 8.4150 (1.1); 7.8832 (1.4); 7.8617 (1.3); 7.8602 (1.2); 7.4867 (2.2); 7.4831 (2.2); 7.2680 (1.2); 6.0927 (0.6); 6.0751 (0.9); 6.0576 (0.6); 3.4233 (4.9); 3.3702 (1.4); 2.9610 (16.0); 2.6762 (0.5); 2.6716 (0.7); 2.6671 (0.5); 2.5251 (2.4); 2.5203 (4.0); 2.5117 (43.9); 2.5073 (84.6); 2.5027 (107.8); 2.4981 (76.5); 2.4935 (35.8); 2.3340 (0.5); 2.3295 (0.6); 2.3250 (0.5); 2.0516 (0.3); 2.0431 (0.4); 2.0307 (0.7); 2.0181 (0.4); 2.0100 (0.4); 1.9890 (0.5); 1.9091 (5.4); 1.6145 (3.0); 1.5971 (2.9); 1.3555 (1.9); 1.2342 (0.6); 1.2168 (0.3); 1.1930 (0.3); 1.1812 (0.5); 1.1753 (0.5); 1.1694 (0.5); 1.0436 (0.4); 1.0328 (1.1); 1.0272 (1.2); 1.0231 (0.6); 1.0170 (0.6); 1.0120 (1.1); 1.0063 (1.1); 0.9963 (0.4); 0.7911 (0.5); 0.7809 (1.2); 0.7753 (1.1); 0.7685 (1.2); 0.7638 (1.1); 0.7524 (0.4); 0.0079 (3.0); -0.0002 (76.8); -0.0086 (2.3) | |
| I-240 | 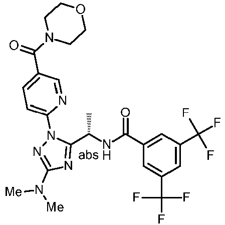 (reference compound) | ¹H-NMR (400.2 MHz, d₆-DMSO): | 586.4 [M+H]⁺ |
| | | δ = 9.5498 (0.7); 9.5320 (0.7); 8.5247 (1.2); 8.5232 (1.2); 8.5191 (1.3); 8.5175 (1.2); 8.4803 (2.4); 8.3191 (1.1); 8.3147 (1.4); 8.0687 (0.8); 8.0630 (0.8); 8.0475 (1.0); 8.0418 (1.0); 7.8444 (1.3); 7.8430 (1.3); 7.8233 (1.1); 7.8218 (1.1); 6.1401 (0.5); 6.1226 (0.8); 6.1049 (0.5); 3.6250 (0.6); 3.6070 (0.7); 3.3298 (416.1); 2.9525 (15.0); 2.6900 (16.0); 2.6804 (0.7); 2.6759 (1.3); 2.6713 (1.8); 2.6668 (1.3); 2.6625 (0.6); 2.5249 (6.4); 2.5201 (9.6); 2.5114 (103.3); 2.5070 (204.4); 2.5024 (265.1); 2.4978 (191.4); 2.4933 (92.1); 2.3383 (0.5); 2.3338 (1.1); 2.3292 (1.6); 2.3246 (1.1); 2.3204 (0.5); 1.6415 (2.7); 1.6242 (2.7); 0.1460 (0.5); 0.0080 (4.1); -0.0002 (122.4); -0.0085 (3.9); -0.1495 (0.5) | |
| I-241 | 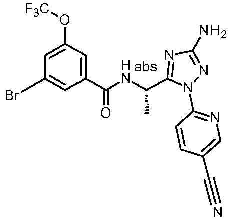 (reference compound) | ¹H-NMR (400.2 MHz, d₆-DMSO): | 497.9 [M+H]⁺ |
| | | δ = 9.2923 (4.4); 9.2748 (4.4); 8.9234 (7.0); 8.9217 (7.5); 8.9179 (7.6); 8.9161 (7.2); 8.4430 (6.0); 8.4373 (5.6); 8.4213 (6.3); 8.4156 (6.4); 8.3149 (4.7); 8.1529 (5.9); 8.1490 (9.9); 8.1452 (6.1); 7.8887 (6.0); 7.8195 (10.4); 7.8162 (6.4); 7.8135 (6.3); 7.7997 (7.0); 7.7980 (7.1); 6.0812 (0.7); 6.0652 (3.1); 6.0478 (4.8); 6.0303 (3.1); 6.0126 (0.9); 5.9867 (11.9); 5.9634 (0.4); 3.5883 (0.3); 3.5773 (0.4); 3.5680 (0.9); 3.5552 (0.4); 3.5298 (0.4); 3.4577 (0.6); 3.4496 (0.6); 3.3284 (3074.9); 3.2674 (0.6); 2.7321 (0.4); 2.7284 (0.4); 2.7076 (0.4); 2.6801 (4.4); 2.6756 (9.2); 2.6711 (13.0); 2.6665 (9.4); 2.6618 (4.6); 2.6400 (0.7); 2.6072 (1.0); 2.5245 (43.4); 2.5198 (64.4); 2.5111 (743.9); 2.5067 (1502.0); 2.5021 (1974.3); 2.4975 (1439.2); 2.4930 (699.3); 2.3900 (0.4); 2.3379 (4.1); 2.3334 (8.9); 2.3289 (12.5); 2.3243 (9.0); 2.3199 (4.2); 1.5834 (16.0); 1.5661 (16.0); 1.2362 (0.4); 0.1458 (4.8); 0.0296 (0.6); 0.0079 (37.3); -0.0003 (1172.9); -0.0086 (39.4); -0.0455 (0.4); -0.1497 (4.7) | |
| I-242 | 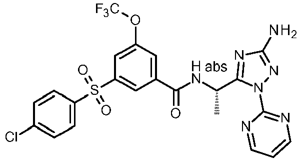 | ¹H-NMR (400.2 MHz, d₆-DMSO): | 568.1 [M+H]⁺ |
| | | δ = 9.4851 (1.4); 9.4672 (1.5); 8.8534 (8.8); 8.8413 (9.1); 8.4558 (2.0); 8.4521 (3.6); 8.4485 (2.1); 8.1701 (2.1); 8.1007 (1.9); 8.0982 (2.1); 8.0958 (1.9); 8.0925 (1.5); 8.0855 (5.1); 8.0805 (1.6); 8.0687 (1.6); 8.0636 (5.9); 8.0571 (0.7); 7.7697 (0.6); 7.7631 (5.8); 7.7581 (1.6); 7.7462 (1.5); 7.7412 (5.1); 7.7348 (0.5); 7.4349 (2.3); 7.4228 (4.3); 7.4107 (2.2); 6.0235 (1.1); 6.0061 (1.7); 5.9885 (1.1); 5.7485 (4.2); 3.3650 (0.4); 3.3385 (141.5); 2.6782 (0.4); 2.6736 (0.5); 2.6691 (0.4); 2.5438 (0.7); 2.5270 (1.5); 2.5224 (2.2); 2.5137 (29.9); 2.5092 (60.8); 2.5046 (80.0); 2.4999 (58.0); 2.4954 (28.0); 2.3361 (0.4); 2.3313 (0.5); 2.3266 (0.4); 2.0771 (16.0); 1.6059 (5.7); 1.5886 (5.6) | |
| I-243 | 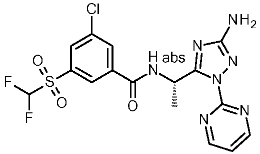 | ¹H-NMR (400.2 MHz, d₆-DMSO): | 458.0 [M+H]⁺ |
| | | δ = 9.5141 (2.2); 9.4962 (2.3); 8.8597 (15.5); 8.8476 (16.0); 8.4412 (3.3); 8.4368 (5.0); 8.4326 (3.6); 8.3515 (3.2); 8.3476 (5.6); 8.3439 (3.0); 8.1774 (3.2); 8.1730 (5.2); 8.1685 (2.8); 7.5428 (1.9); 7.4454 (4.0); 7.4333 (7.4); 7.4212 (4.1); 7.4128 (4.7); 7.2830 (2.4); 6.0375 (1.7); 6.0200 (2.7); 6.0025 (1.7); 5.9852 (0.3); 5.7557 (6.6); 3.3327 (154.9); 2.6765 (0.6); 2.6719 (0.8); 2.6673 (0.6); 2.5422 (0.7); 2.5254 (2.5); 2.5208 (3.7); 2.5120 (47.8); 2.5075 (96.6); 2.5029 (126.8); 2.4982 (91.2); 2.4937 (43.2); 2.3343 (0.6); 2.3297 (0.8); 2.3250 (0.6); 2.0758 (3.0); 1.6034 (9.1); 1.5861 (9.1); - 0.0002 (0.4) | |
| I-244 | 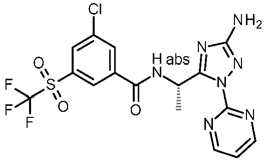 | ¹H-NMR (400.2 MHz, d₆-DMSO): | 476.0 [M+H]⁺ |
| | | δ = 9.5706 (0.6); 9.5529 (0.6); 8.8570 (4.4); 8.8449 (4.5); 8.5252 (0.9); 8.5211 (1.4); 8.5166 (1.1); 8.4656 (1.3); 8.4080 (0.8); 8.4034 (1.3); 8.3731 (0.5); 7.4433 (1.1); 7.4312 (2.1); 7.4191 (1.1); 6.0419 (0.5); 6.0243 (0.8); 6.0067 (0.5); 5.7579 (1.8); 3.3305 (390.3); 2.6800 (0.9); 2.6754 (1.9); 2.6708 (2.6); 2.6662 (1.9); 2.6617 (0.9); 2.5412 (3.7); 2.5244 (8.8); 2.5197 (12.8); 2.5110 (157.3); 2.5065 (318.9); 2.5018 (418.0); 2.4972 (299.7); 2.4926 (141.7); 2.3379 (0.8); 2.3332 (1.8); 2.3287 (2.6); 2.3241 (1.8); 2.3195 (0.8); 2.0747 (16.0); 1.6078 (2.6); 1.5905 (2.6); 1.2726 (0.8); 1.2576 (1.4); 1.2421 (1.2); -0.0002 (1.3) | |
| I-245 | 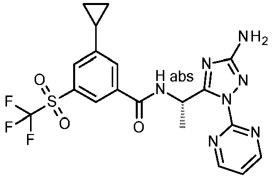 | ¹H-NMR (400.2 MHz, d₆-DMSO): | 482.0 [M+H]⁺ |
| | | δ = 9.4247 (3.2); 9.4068 (3.2); 8.8537 (15.6); 8.8416 (16.0); 8.2497 (5.9); 8.0302 (5.6); 7.9885 (3.9); 7.9849 (6.4); 7.4393 (4.1); 7.4272 (7.7); 7.4152 (4.0); 6.0474 (0.4); 6.0302 (2.2); 6.0127 (3.5); 5.9951 (2.2); 5.9777 (0.5); 5.7443 (9.4); 3.3343 (144.2); 2.6770 (0.7); 2.6725 (1.0); 2.6680 (0.7); 2.6633 (0.3); 2.5430 (1.7); 2.5259 (2.9); 2.5209 (4.2); 2.5123 (57.3); 2.5080 (116.6); 2.5035 (154.5); 2.4990 (114.3); 2.4945 (57.0); 2.3388 (0.3); 2.3348 (0.7); 2.3303 (1.0); 2.3259 (0.7); 2.3214 (0.4); 2.2899 (0.6); 2.2772 (1.4); 2.2687 (1.4); 2.2564 (2.7); 2.2440 (1.6); 2.2355 (1.4); 2.2229 (0.7); 2.0765 (3.4); 1.6082 (12.1); 1.5909 (12.1); 1.1355 (1.5); 1.1245 (3.6); 1.1192 (4.0); 1.1089 (2.5); 1.1039 (3.8); 1.0983 (3.9); 1.0882 (1.9); 0.8935 (1.8); 0.8828 (4.3); 0.8791 (4.2); 0.8709 (4.0); 0.8662 (4.3); 0.8547 (1.4); 0.0001 (0.3) | |
| I-246 | 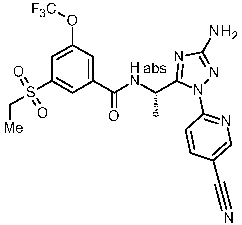 | ¹H-NMR (400.2 MHz, d₆-DMSO): | 510.1 [M+H]⁺ |
| | | δ = 9.5096 (2.5); 9.4924 (2.5); 8.9307 (4.4); 8.9290 (4.6); 8.9252 (4.6); 8.9234 (4.4); 8.4475 (3.8); 8.4419 (3.6); 8.4283 (4.0); 8.4254 (9.1); 8.4205 (6.4); 8.1733 (3.6); 8.0344 (3.6); 7.8273 (4.5); 7.8258 (4.4); 7.8057 (4.3); 7.8040 (4.3); 6.1285 (0.4); 6.1117 (1.8); 6.0944 (2.9); 6.0770 (1.8); 6.0598 (0.4); 5.9981 (6.8); 3.4840 (1.9); 3.4656 (6.5); 3.4472 (6.6); 3.4289 (2.1); 3.3319 (495.8); 3.2911 (0.4); 2.6801 (1.2); 2.6756 (2.5); 2.6711 (3.4); 2.6666 (2.5); 2.6620 (1.1); 2.5724 (0.4); 2.5415 (11.1); 2.5246 (11.2); 2.5199 (16.6); 2.5112 (207.3); 2.5067 (417.1); 2.5021 (544.6); 2.4975 (391.4); 2.4929 (185.9); 2.3380 (1.1); 2.3335 (2.4); 2.3289 (3.3); 2.3244 (2.4); 2.3198 (1.1); 2.0748 (10.9); 1.6131 (9.5); 1.5958 (9.4); 1.1465 (6.8); 1.1282 (16.0); 1.1098 (6.7); -0.0002 (1.1) | |
| I-247 | 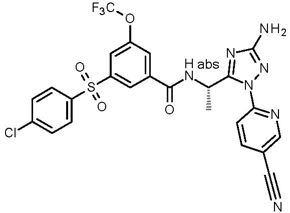 | ¹H-NMR (400.2 MHz, d₆-DMSO): | 592.0 [M+H]⁺ |
| | | δ = 9.5156 (1.6); 9.4982 (1.7); 8.9152 (2.8); 8.9111 (2.9); 8.4896 (2.1); 8.4860 (3.8); 8.4824 (2.2); 8.4439 (2.2); 8.4383 (2.1); 8.4222 (2.2); 8.4166 (2.3); 8.1823 (2.4); 8.1333 (2.3); 8.0950 (0.6); 8.0885 (5.2); 8.0837 (1.7); 8.0717 (1.8); 8.0668 (6.0); 8.0605 (0.7); 7.8222 (2.9); 7.8020 (2.7); 7.8005 (2.7); 7.7693 (0.7); 7.7625 (6.0); 7.7576 (1.8); 7.7456 (1.7); 7.7407 (5.2); 6.0869 (1.2); 6.0694 (1.8); 6.0524 (1.1); 6.0355 (0.4); 5.9893 (4.4); 3.4663 (0.4); 3.3320 (1150.1); 2.8903 (0.5); 2.7311 (0.5); 2.6800 (2.1); 2.6755 (4.3); 2.6709 (5.9); 2.6664 (4.3); 2.6620 (2.0); 2.5413 (13.9); 2.5244 (19.5); 2.5196 (30.8); 2.5110 (361.5); 2.5065 (709.4); 2.5020 (918.6); 2.4974 (665.1); 2.4929 (319.8); 2.3377 (1.9); 2.3333 (4.1); 2.3288 (5.6); 2.3242 (4.0); 2.3197 (1.8); 2.0748 (16.0); 1.6047 (5.9); 1.5873 (5.9); -0.0002 (1.6) | |
| I-248 | 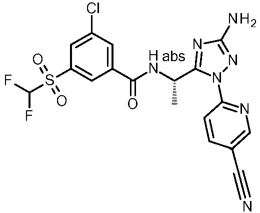 | ¹H-NMR (400.2 MHz, d₆-DMSO): | 582.0 [M+H]⁺ |
| | | δ = 9.5500 (3.3); 9.5328 (3.4); 8.9280 (5.7); 8.9264 (6.2); 8.9225 (6.2); 8.9208 (5.9); 8.4826 (4.7); 8.4783 (7.3); 8.4740 (5.0); 8.4472 (5.1); 8.4416 (4.8); 8.4255 (5.3); 8.4199 (5.4); 8.3862 (4.6); 8.3824 (8.0); 8.3787 (4.3); 8.1910 (4.5); 8.1866 (7.5); 8.1822 (4.0); 7.8270 (6.2); 7.8253 (6.1); 7.8053 (6.0); 7.8036 (5.9); 7.5490 (2.7); 7.4190 (6.4); 7.2892 (3.3); 6.1194 (0.5); 6.1022 (2.5); 6.0850 (3.9); 6.0676 (2.5); 6.0502 (0.6); 6.0003 (9.0); 3.3339 (451.2); 2.6806 (0.8); 2.6761 (1.8); 2.6715 (2.4); 2.6669 (1.7); 2.6625 (0.8); 2.5419 (12.4); 2.5250 (7.8); 2.5203 (11.5); 2.5116 (147.0); 2.5071 (297.3); 2.5025 (388.6); 2.4979 (278.8); 2.4933 (132.5); 2.3385 (0.8); 2.3339 (1.7); 2.3293 (2.4); 2.3248 (1.7); 2.3202 (0.8); 2.0752 (16.0); 1.6034 (12.9); 1.5861 (12.9); -0.0001 (0.8) | |
| I-249 | 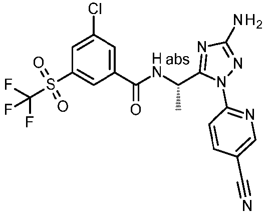 | ¹H-NMR (400.2 MHz, d₆-DMSO): | 500.0 [M+H]⁺ |
| | | δ = 9.6024 (4.1); 9.5852 (4.2); 8.9236 (7.3); 8.9218 (8.0); 8.9181 (7.8); 8.9162 (7.6); 8.5620 (6.0); 8.5576 (9.1); 8.5534 (6.9); 8.4993 (8.1); 8.4469 (6.7); 8.4413 (6.3); 8.4253 (7.6); 8.4197 (11.2); 8.4166 (7.8); 7.8262 (7.7); 7.8244 (7.9); 7.8046 (7.4); 7.8027 (7.7); 6.1237 (0.6); 6.1067 (3.1); 6.0894 (4.9); 6.0721 (3.1); 6.0545 (0.6); 6.0050 (11.0); 3.3334 (688.1); 2.6805 (1.2); 2.6760 (2.6); 2.6714 (3.6); 2.6668 (2.6); 2.6623 (1.2); 2.5418 (17.5); 2.5250 (11.9); 2.5203 (17.2); 2.5116 (215.9); 2.5071 (437.1); 2.5025 (572.7); 2.4978 (410.3); 2.4932 (193.4); 2.3384 (1.1); 2.3339 (2.5); 2.3293 (3.5); 2.3247 (2.5); 2.3202 (1.1); 2.0751 (11.8); 1.6092 (16.0); 1.5918 (15.9); -0.0002 (1.2) | |
| I-250 | 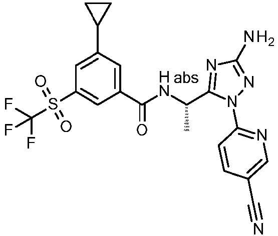 | ¹H-NMR (400.2 MHz, d₆-DMSO): | 506.0 [M+H]⁺ |
| | | δ = 9.4590 (4.2); 9.4417 (4.3); 8.9177 (7.6); 8.9158 (8.1); 8.9121 (8.2); 8.9102 (7.9); 8.4450 (6.8); 8.4394 (6.5); 8.4234 (7.2); 8.4177 (7.3); 8.2884 (7.8); 8.0404 (7.6); 8.0365 (6.2); 8.0327 (7.5); 8.0286 (8.9); 8.0247 (3.6); 7.8228 (7.8); 7.8210 (7.9); 7.8012 (7.5); 7.7993 (7.8); 6.1125 (0.6); 6.0955 (3.1); 6.0782 (4.9); 6.0608 (3.1); 6.0436 (0.7); 5.9878 (11.4); 3.3340 (474.0); 2.6808 (0.8); 2.6762 (1.9); 2.6717 (2.7); 2.6670 (1.9); 2.6625 (0.9); 2.5420 (14.3); 2.5252 (8.5); 2.5205 (12.3); 2.5118 (157.5); 2.5073 (323.6); 2.5027 (428.4); 2.4980 (310.2); 2.4934 (148.5); 2.3386 (0.9); 2.3341 (1.9); 2.3294 (2.7); 2.3249 (1.9); 2.3203 (0.9); 2.2998 (0.8); 2.2873 (1.8); 2.2789 (1.9); 2.2755 (1.4); 2.2665 (3.5); 2.2540 (2.0); 2.2456 (1.9); 2.2331 (1.0); 2.0752 (15.3); 1.6096 (16.0); 1.5922 (16.0); 1.1432 (2.0); 1.1316 (5.3); 1.1262 (5.9); 1.1153 (3.2); 1.1107 (5.5); 1.1052 (5.8); 1.0946 (2.4); 0.9030 (2.6); 0.8918 (6.5); 0.8869 (6.4); 0.8797 (5.9); 0.8747 (7.3); 0.8628 (2.0); -0.0003 (0.9) | |
| I-251 | 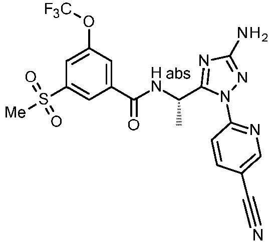 | ¹H-NMR (400.2 MHz, d₆-DMSO): | 496.1 [M+H]⁺ |
| | | δ = 9.4971 (4.0); 9.4799 (4.1); 8.9370 (7.0); 8.9354 (7.2); 8.9315 (7.3); 8.9298 (6.8); 8.4779 (5.6); 8.4743 (10.0); 8.4707 (5.6); 8.4490 (5.9); 8.4434 (5.5); 8.4273 (6.2); 8.4217 (6.1); 8.1511 (5.6); 8.0820 (5.7); 7.8284 (7.2); 7.8268 (7.0); 7.8068 (6.8); 7.8051 (6.8); 6.1315 (0.6); 6.1148 (2.8); 6.0975 (4.5); 6.0801 (2.9); 6.0626 (0.6); 5.9971 (10.7); 3.3639 (48.2); 3.3311 (864.6); 2.6801 (1.6); 2.6756 (3.4); 2.6711 (4.7); 2.6665 (3.3); 2.6620 (1.6); 2.5414 (13.3); 2.5246 (15.7); 2.5198 (24.3); 2.5112 (288.2); 2.5067 (570.1); 2.5021 (739.3); 2.4975 (530.0); 2.4929 (251.2); 2.3381 (1.5); 2.3335 (3.3); 2.3289 (4.6); 2.3244 (3.2); 2.3198 (1.5); 2.0750 (16.0); 1.6124 (14.9); 1.5951 (14.9); -0.0001 (1.5) | |
| I-252 | 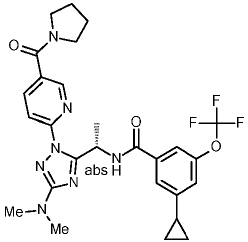 | ¹H-NMR (400.2 MHz, d₆-DMSO): | 558.2 [M+H]⁺ |
| | | δ = 9.1019 (0.7); 9.0841 (0.7); 8.5820 (1.2); 8.5805 (1.3); 8.5763 (1.3); 8.5748 (1.2); 8.3157 (0.5); 8.1415 (1.0); 8.1358 (0.9); 8.1203 (1.1); 8.1145 (1.0); 7.8084 (1.3); 7.8071 (1.3); 7.7873 (1.2); 7.7858 (1.2); 7.4727 (2.1); 7.4692 (2.1); 7.2679 (1.0); 6.0565 (0.5); 6.0388 (0.8); 6.0211 (0.5); 3.4922 (0.7); 3.4755 (1.4); 3.4583 (0.8); 3.3691 (0.5); 3.3523 (1.4); 3.3297 (173.8); 2.9551 (16.0); 2.6899 (2.3); 2.6804 (0.4); 2.6760 (0.8); 2.6714 (1.1); 2.6668 (0.8); 2.6625 (0.4); 2.5250 (3.7); 2.5202 (5.4); 2.5115 (62.5); 2.5070 (126.4); 2.5024 (167.0); 2.4978 (122.1); 2.4933 (58.9); 2.3384 (0.3); 2.3338 (0.8); 2.3292 (1.1); 2.3247 (0.8); 2.3201 (0.4); 2.0388 (0.3); 2.0259 (0.6); 2.0134 (0.4); 2.0050 (0.3); 1.8871 (0.5); 1.8707 (0.8); 1.8533 (0.7); 1.8380 (0.3); 1.8067 (0.7); 1.7911 (0.8); 1.7745 (0.4); 1.6072 (2.9); 1.5898 (2.8); 1.0319 (0.9); 1.0264 (1.0); 1.0191 (0.5); 1.0161 (0.4); 1.0111 (1.0); 1.0054 (1.0); 0.7824 (0.5); 0.7699 (1.0); 0.7651 (0.9); 0.7587 (1.0); 0.7536 (0.7); 0.7461 (0.4); 0.1460 (0.4); 0.0079 (2.8); - 0.0002 (91.1); -0.0086 (2.9); -0.1496 (0.4) | |
| I-253 | 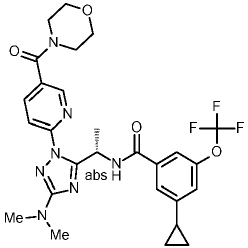 | ¹H-NMR (400.2 MHz, d₆-DMSO): | 574.2 [M+H]⁺ |
| | | δ = 9.1212 (0.7); 9.1030 (0.8); 8.5037 (1.3); 8.5024 (1.3); 8.4982 (1.3); 8.0616 (0.9); 8.0560 (0.8); 8.0405 (1.1); 8.0348 (1.1); 7.8305 (1.4); 7.8094 (1.2); 7.8079 (1.2); 7.4889 (1.1); 7.4862 (1.1); 7.4804 (1.4); 7.4767 (1.7); 7.2783 (1.1); 6.0578 (0.5); 6.0401 (0.9); 6.0225 (0.6); 3.6532 (0.4); 3.6106 (0.6); 3.6012 (0.5); 3.5694 (0.5); 3.3305 (124.4); 2.9525 (16.0); 2.6761 (0.6); 2.6714 (0.8); 2.6668 (0.6); 2.5250 (2.8); 2.5203 (4.1); 2.5115 (46.8); 2.5071 (94.2); 2.5025 (123.8); 2.4979 (90.5); 2.4933 (43.9); 2.3339 (0.6); 2.3293 (0.8); 2.3247 (0.6); 2.0748 (3.5); 2.0445 (0.3); 2.0322 (0.6); 2.0197 (0.4); 2.0113 (0.4); 1.6075 (2.9); 1.5901 (2.9); 1.0348 (0.9); 1.0294 (1.0); 1.0266 (0.8); 1.0138 (0.9); 1.0085 (1.0); 0.7847 (0.7); 0.7725 (0.9); 0.7685 (0.9); 0.7652 (0.9); 0.7537 (0.6); 0.0080 (1.7); -0.0002 (55.8); -0.0085 (1.8) | |
| I-254 | 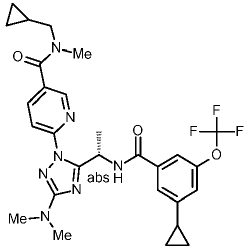 | ¹H-NMR (400.2 MHz, d₆-DMSO): | 572.3 [M+H]⁺ |
| | | δ = 9.1247 (0.6); 9.1060 (0.6); 8.4785 (0.6); 8.3161 (0.4); 8.0266 (0.3); 7.8184 (1.4); 7.7974 (1.1); 7.4979 (1.9); 7.2687 (1.1); 6.0657 (0.4); 6.0481 (0.5); 6.0301 (0.4); 3.3299 (152.3); 3.0652 (0.6); 2.9528 (16.0); 2.6805 (0.3); 2.6760 (0.7); 2.6714 (1.0); 2.6668 (0.7); 2.6624 (0.3); 2.5249 (3.6); 2.5202 (5.5); 2.5115 (60.8); 2.5070 (119.8); 2.5024 (155.2); 2.4978 (112.1); 2.4933 (53.7); 2.3384 (0.3); 2.3338 (0.7); 2.3293 (1.0); 2.3246 (0.7); 2.0438 (0.3); 2.0316 (0.6); 2.0190 (0.4); 2.0109 (0.4); 1.6073 (2.8); 1.5899 (2.8); 1.0445 (0.4); 1.0341 (1.1); 1.0284 (1.2); 1.0184 (0.6); 1.0132 (1.1); 1.0075 (1.1); 0.9977 (0.5); 0.7931 (0.4); 0.7830 (1.0); 0.7800 (1.0); 0.7708 (1.0); 0.7669 (1.0); 0.7553 (0.4); 0.0080 (2.3); -0.0002 (72.0); -0.0085 (2.4) | |
| I-255 | 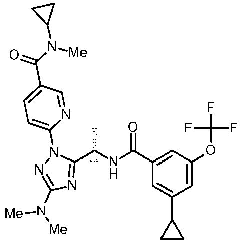 | ¹H-NMR (400.2 MHz, d₆-DMSO): | 558.2 [M+H]⁺ |
| | | δ = 9.1248 (0.8); 9.1069 (0.8); 8.6009 (0.8); 8.3163 (0.5); 8.1354 (0.4); 8.1180 (0.5); 7.7990 (1.4); 7.7775 (1.2); 7.5149 (2.5); 7.2675 (1.2); 6.0919 (0.5); 6.0744 (0.7); 6.0566 (0.5); 3.3288 (215.0); 2.9928 (2.0); 2.9539 (16.0); 2.6756 (1.3); 2.6711 (1.7); 2.6668 (1.2); 2.6017 (0.4); 2.5244 (7.3); 2.5109 (102.7); 2.5067 (194.7); 2.5022 (250.6); 2.4977 (186.4); 2.4936 (94.8); 2.3335 (1.2); 2.3290 (1.6); 2.3246 (1.2); 2.0747 (0.3); 2.0435 (0.4); 2.0311 (0.7); 2.0187 (0.4); 2.0106 (0.4); 1.6045 (3.0); 1.5871 (2.9); 1.0444 (0.4); 1.0331 (1.1); 1.0277 (1.2); 1.0170 (0.7); 1.0122 (1.2); 1.0067 (1.1); 0.9965 (0.5); 0.7940 (0.5); 0.7833 (1.3); 0.7780 (1.3); 0.7712 (1.2); 0.7660 (1.4); 0.7545 (0.4); 0.5161 (0.4); 0.4077 (0.5); 0.1458 (0.4); 0.0078 (4.0); -0.0002 (96.3); -0.0084 (4.0); -0.1495 (0.5) | |
| I-256 | 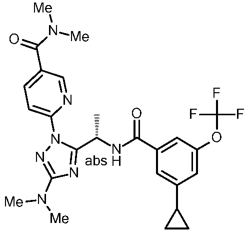 | ¹H-NMR (400.2 MHz, d₆-DMSO): | 532.2 [M+H]⁺ |
| | | δ = 9.1224 (0.7); 9.1043 (0.8); 8.5019 (1.3); 8.4977 (1.3); 8.3160 (0.7); 8.0562 (0.9); 8.0505 (0.9); 8.0350 (1.1); 8.0293 (1.0); 7.8138 (1.3); 7.7928 (1.1); 7.4942 (2.3); 7.4902 (2.5); 7.2668 (1.1); 6.0608 (0.5); 6.0431 (0.8); 6.0255 (0.6); 3.3291 (254.2); 3.0019 (1.7); 2.9516 (16.0); 2.9083 (1.8); 2.8671 (0.5); 2.6802 (0.7); 2.6758 (1.4); 2.6713 (2.0); 2.6667 (1.4); 2.6623 (0.7); 2.5971 (0.4); 2.5247 (7.1); 2.5200 (10.7); 2.5113 (114.0); 2.5068 (225.2); 2.5023 (293.4); 2.4977 (213.5); 2.4932 (103.2); 2.3382 (0.6); 2.3336 (1.4); 2.3291 (1.9); 2.3246 (1.3); 2.3202 (0.6); 2.0746 (0.4); 2.0504 (0.3); 2.0416 (0.3); 2.0294 (0.6); 2.0169 (0.4); 2.0088 (0.4); 1.6077 (2.9); 1.5903 (2.8); 1.0437 (0.4); 1.0334 (1.0); 1.0279 (1.0); 1.0180 (0.5); 1.0127 (1.0); 1.0068 (1.0); 0.9974 (0.4); 0.7903 (0.5); 0.7804 (1.0); 0.7769 (1.0); 0.7682 (1.0); 0.7642 (0.9); 0.7527 (0.4); 0.1459 (0.5); 0.0080 (4.4); -0.0002 (128.0); - 0.0085 (4.1); -0.1496 (0.5) | |
| I-257 | 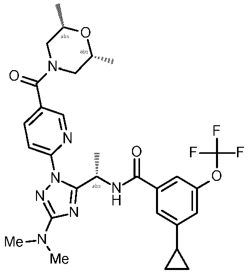 | ¹H-NMR (400.2 MHz, d₆-DMSO): | 602.3 [M+H]⁺ |
| | | δ = 9.1059 (0.7); 9.0877 (0.7); 8.4771 (1.2); 8.4756 (1.2); 8.4715 (1.3); 8.4698 (1.2); 8.3161 (1.0); 8.0459 (0.9); 8.0402 (0.8); 8.0248 (1.1); 8.0190 (1.0); 7.8318 (1.4); 7.8304 (1.4); 7.8107 (1.2); 7.8091 (1.2); 7.4676 (1.0); 7.4651 (1.0); 7.4580 (1.2); 7.4542 (1.6); 7.2709 (1.0); 6.0558 (0.5); 6.0380 (0.8); 6.0205 (0.5); 3.3312 (395.3); 2.9553 (16.0); 2.6803 (0.9); 2.6758 (2.0); 2.6712 (2.7); 2.6666 (1.9); 2.6621 (0.9); 2.6004 (0.4); 2.5248 (9.2); 2.5201 (13.2); 2.5114 (152.9); 2.5068 (309.6); 2.5022 (407.3); 2.4976 (294.7); 2.4930 (140.5); 2.3382 (0.8); 2.3336 (1.8); 2.3291 (2.6); 2.3245 (1.8); 2.3200 (0.8); 2.0252 (0.6); 2.0123 (0.3); 2.0041 (0.3); 1.6059 (2.8); 1.5886 (2.8); 1.1431 (0.5); 1.0438 (0.4); 1.0322 (1.2); 1.0270 (1.4); 1.0114 (1.4); 1.0060 (1.4); 0.9952 (0.7); 0.7783 (0.7); 0.7660 (0.9); 0.7622 (0.9); 0.7582 (0.8); 0.7470 (0.5); 0.1460 (0.8); 0.0080 (6.2); -0.0002 (206.5); -0.0085 (6.4); -0.1496 (0.8) | |
| I-258 | 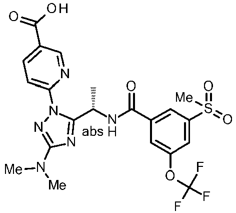 | ¹H-NMR (400.2 MHz, d₆-DMSO): | 543.0 [M+H]⁺ |
| | | δ = 9.5136 (0.8); 9.4957 (0.8); 8.9260 (1.3); 8.9245 (1.4); 8.9204 (1.4); 8.9188 (1.4); 8.4489 (1.1); 8.4432 (1.0); 8.4323 (1.2); 8.4282 (2.6); 8.4218 (1.3); 8.0985 (1.1); 8.0962 (1.1); 8.0714 (1.2); 8.0699 (1.2); 7.8955 (1.4); 7.8942 (1.4); 7.8741 (1.3); 7.8727 (1.3); 6.1630 (0.6); 6.1454 (0.9); 6.1278 (0.6); 4.0379 (0.4); 4.0201 (0.4); 3.3867 (0.7); 3.3508 (10.6); 3.3345 (37.8); 2.9666 (16.0); 2.6763 (0.5); 2.6718 (0.7); 2.6672 (0.5); 2.6108 (0.3); 2.5252 (2.5); 2.5204 (4.0); 2.5118 (39.4); 2.5074 (77.6); 2.5028 (101.2); 2.4981 (73.6); 2.4936 (35.4); 2.3342 (0.4); 2.3296 (0.6); 2.3250 (0.5); 1.9891 (1.9); 1.9091 (1.7); 1.6411 (3.0); 1.6236 (2.9); 1.3553 (0.4); 1.1932 (0.6); 1.1754 (1.1); 1.1693 (0.4); 1.1576 (0.5); 0.0080 (1.2); -0.0002 (33.5); - 0.0086 (0.9) | |
| I-259 | 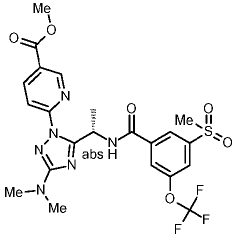 | ¹H-NMR (400.2 MHz, d₆-DMSO): | 557.0 [M+H]⁺ |
| | | δ = 9.5073 (1.0); 9.4898 (1.0); 8.9477 (1.6); 8.9422 (1.7); 8.4713 (0.9); 8.4659 (0.8); 8.4498 (1.0); 8.4459 (0.9); 8.4180 (2.0); 8.0943 (1.6); 8.0701 (1.7); 7.9114 (1.7); 7.8898 (1.6); 6.1551 (0.6); 6.1378 (1.0); 6.1201 (0.6); 3.9052 (0.5); 3.8922 (8.2); 3.3480 (9.1); 3.3253 (60.3); 2.9679 (16.0); 2.6707 (0.6); 2.6114 (0.4); 2.5010 (88.2); 2.3279 (0.6); 1.9873 (0.3); 1.6395 (3.5); 1.6221 (3.4); 0.1658 (0.4); -0.0005 (30.9); - 0.0022 (32.9) | |
| I-260 | 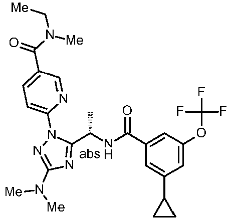 | ¹H-NMR (400.2 MHz, d₆-DMSO): | 546.2 [M+H]⁺ |
| | | δ = 9.1140 (0.6); 9.0957 (0.6); 8.3158 (0.5); 7.8123 (1.0); 7.7913 (0.9); 7.4848 (1.9); 7.2664 (1.1); 6.0572 (0.5); 6.0396 (0.8); 6.0219 (0.5); 3.3267 (92.6); 2.9528 (16.0); 2.8765 (0.6); 2.6802 (0.4); 2.6757 (0.9); 2.6712 (1.2); 2.6666 (0.9); 2.6622 (0.4); 2.5247 (4.4); 2.5199 (6.8); 2.5113 (69.8); 2.5068 (138.5); 2.5022 (181.0); 2.4976 (131.6); 2.4931 (63.4); 2.3383 (0.4); 2.3337 (0.8); 2.3290 (1.1); 2.3245 (0.8); 2.3200 (0.4); 2.0408 (0.3); 2.0284 (0.6); 2.0160 (0.4); 2.0075 (0.3); 1.6064 (2.8); 1.5890 (2.8); 1.1428 (0.4); 1.1289 (0.3); 1.0425 (0.7); 1.0326 (1.3); 1.0270 (1.3); 1.0117 (1.1); 1.0060 (1.1); 0.9966 (0.4); 0.7870 (0.4); 0.7753 (1.0); 0.7698 (1.0); 0.7637 (1.0); 0.7587 (0.6); 0.7513 (0.4); 0.0080 (1.5); -0.0002 (42.3); -0.0085 (1.3) | |
| I-261 | 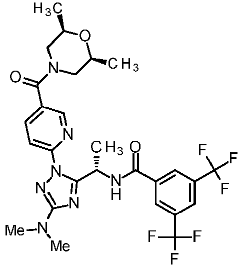 (reference compound) | ¹H-NMR (400.2 MHz, d₆-DMSO): | 614.5 [M+H]⁺ |
| | | δ = 9.5493 (0.7); 9.5315 (0.7); 8.5153 (1.4); 8.5110 (1.4); 8.4758 (2.1); 8.3161 (1.4); 8.0568 (0.9); 8.0511 (0.8); 8.0356 (1.1); 8.0299 (1.1); 7.8418 (1.4); 7.8210 (1.2); 7.8197 (1.2); 6.1488 (0.5); 6.1313 (0.9); 6.1136 (0.6); 3.5930 (0.3); 3.5870 (0.4); 3.5775 (0.5); 3.5711 (0.6); 3.5667 (0.6); 3.5603 (0.5); 3.5510 (0.5); 3.5447 (0.4); 3.3533 (0.4); 3.3304 (99.2); 3.3115 (0.3); 2.9537 (16.0); 2.6765 (0.4); 2.6719 (0.6); 2.6673 (0.4); 2.5254 (2.1); 2.5206 (3.0); 2.5120 (34.6); 2.5075 (69.0); 2.5029 (90.4); 2.4983 (65.7); 2.4938 (31.8); 2.3343 (0.4); 2.3298 (0.6); 2.3252 (0.4); 2.0750 (3.8); 1.6421 (3.0); 1.6247 (3.0); 1.1446 (0.5); 0.9877 (0.5); 0.0080 (0.6); -0.0002 (19.4); -0.0085 (0.6) | |
| I-262 | 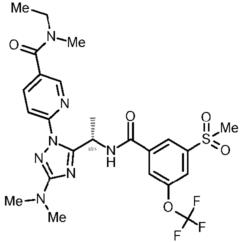 | ¹H-NMR (400.2 MHz, d₆-DMSO): | 584.2 [M+H]⁺ |
| | | δ = 9.4927 (0.6); 9.4747 (0.6); 8.4169 (1.4); 8.1062 (0.9); 8.0679 (1.0); 7.8237 (1.0); 7.8024 (0.8); 6.1225 (0.5); 6.1048 (0.8); 6.0871 (0.5); 3.3498 (8.5); 3.3313 (83.3); 2.9584 (16.0); 2.8955 (0.6); 2.6763 (0.3); 2.6717 (0.5); 2.6671 (0.3); 2.5253 (1.5); 2.5206 (2.3); 2.5119 (27.6); 2.5073 (55.2); 2.5027 (71.8); 2.4981 (51.4); 2.4935 (24.3); 2.3342 (0.3); 2.3296 (0.4); 2.0749 (0.7); 1.6351 (2.7); 1.6176 (2.6); 1.1444 (0.4); 1.0498 (0.4); 0.0080 (0.6); -0.0002 (19.7); -0.0086 (0.6) | |
| I-263 | 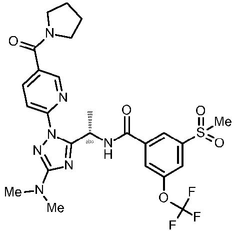 | ¹H-NMR (400.2 MHz, d₆-DMSO): | 596.2 [M+H]⁺ |
| | | δ = 9.4863 (0.8); 9.4682 (0.8); 8.5973 (1.4); 8.5931 (1.4); 8.4136 (1.1); 8.4100 (1.9); 8.4065 (1.1); 8.3157 (0.6); 8.1503 (0.9); 8.1446 (0.9); 8.1291 (1.1); 8.1233 (1.0); 8.0958 (1.2); 8.0698 (1.2); 7.8217 (1.4); 7.8006 (1.2); 6.1258 (0.6); 6.1080 (0.9); 6.0904 (0.6); 3.4945 (0.8); 3.4778 (1.5); 3.4611 (0.9); 3.4000 (0.6); 3.3927 (0.7); 3.3837 (1.1); 3.3773 (1.2); 3.3679 (0.9); 3.3605 (1.2); 3.3485 (10.0); 3.3289 (261.0); 2.9597 (16.0); 2.6897 (0.9); 2.6758 (1.3); 2.6712 (1.8); 2.6668 (1.3); 2.6622 (0.6); 2.6053 (0.5); 2.5246 (6.9); 2.5198 (11.0); 2.5112 (106.8); 2.5068 (207.0); 2.5023 (267.5); 2.4977 (195.8); 2.4932 (96.5); 2.3383 (0.6); 2.3336 (1.2); 2.3291 (1.7); 2.3245 (1.2); 2.3202 (0.6); 1.8890 (0.5); 1.8723 (0.9); 1.8559 (0.8); 1.8404 (0.5); 1.8190 (0.8); 1.8032 (0.9); 1.7869 (0.5); 1.6353 (3.0); 1.6179 (2.9); 0.0080 (1.6); -0.0002 (42.1); -0.0085 (1.5) | |
| I-264 | 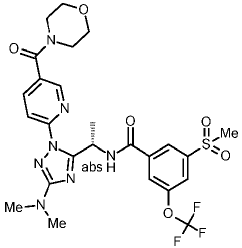 | ¹H-NMR (400.2 MHz, d₆-DMSO): | 612.2 [M+H]⁺ |
| | | δ = 9.5043 (0.9); 9.4866 (0.9); 8.5212 (1.5); 8.5158 (1.6); 8.4168 (2.0); 8.4134 (1.2); 8.3156 (0.5); 8.1103 (1.3); 8.0742 (1.4); 8.0675 (1.4); 8.0615 (0.9); 8.0460 (1.1); 8.0403 (1.1); 7.8409 (1.6); 7.8197 (1.3); 6.1239 (0.6); 6.1064 (0.9); 6.0887 (0.6); 3.6137 (0.9); 3.5237 (0.3); 3.3519 (10.4); 3.3280 (255.3); 2.9567 (16.0); 2.6755 (1.3); 2.6711 (1.8); 2.6667 (1.3); 2.6008 (0.4); 2.5243 (8.2); 2.5065 (211.4); 2.5021 (270.3); 2.4976 (200.2); 2.3332 (1.2); 2.3289 (1.7); 2.3246 (1.2); 1.6361 (3.2); 1.6187 (3.1); 0.0078 (1.5); -0.0003 (37.0); -0.0084 (1.4) | |
| I-265 | 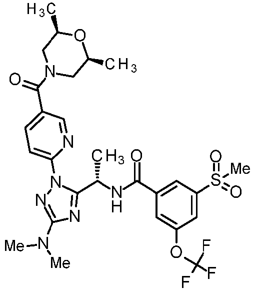 | ¹H-NMR (400.2 MHz, d₆-DMSO): | 640.3 [M+H]⁺ |
| | | δ = 9.5091 (0.9); 9.4916 (0.9); 8.5163 (1.7); 8.5115 (1.6); 8.4227 (1.6); 8.3155 (0.4); 8.1095 (1.3); 8.0733 (1.6); 8.0565 (0.9); 8.0510 (0.8); 8.0354 (1.0); 8.0299 (1.0); 7.8407 (1.6); 7.8196 (1.4); 6.1344 (0.6); 6.1169 (1.0); 6.0995 (0.6); 3.5891 (0.6); 3.5688 (0.9); 3.5528 (0.7); 3.3518 (10.1); 3.3285 (206.6); 2.9567 (16.0); 2.6713 (1.7); 2.6016 (0.4); 2.5064 (210.0); 2.5023 (250.4); 2.3290 (1.6); 2.0744 (2.2); 1.6364 (3.4); 1.6191 (3.3); 1.1472 (0.8); 1.0767 (0.3); 0.9973 (0.8); -0.0003 (29.2) | |
| I-266 | 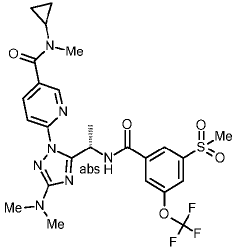 | ¹H-NMR (400.2 MHz, d₆-DMSO): | 596.2 [M+H]⁺ |
| | | δ = 9.4981 (0.7); 9.4798 (0.7); 8.6052 (0.7); 8.4382 (1.0); 8.4346 (1.8); 8.4310 (1.0); 8.1443 (0.4); 8.1403 (0.4); 8.1218 (1.4); 8.1194 (1.3); 8.0681 (1.0); 7.8088 (1.3); 7.8074 (1.3); 7.7877 (1.2); 7.7862 (1.2); 6.1523 (0.5); 6.1346 (0.7); 6.1169 (0.5); 3.3494 (13.4); 3.3369 (88.7); 2.9935 (1.8); 2.9602 (16.0); 2.6766 (0.4); 2.6720 (0.6); 2.6674 (0.4); 2.5256 (1.9); 2.5209 (2.8); 2.5122 (34.6); 2.5077 (70.8); 2.5030 (93.4); 2.4984 (67.8); 2.4938 (32.7); 2.3344 (0.4); 2.3298 (0.6); 2.3252 (0.4); 2.0746 (1.7); 1.6327 (2.8); 1.6153 (2.8); 0.5274 (0.4); 0.4110 (0.4); 0.0081 (0.4); - 0.0002 (15.1); -0.0085 (0.5) | |
| I-267 | 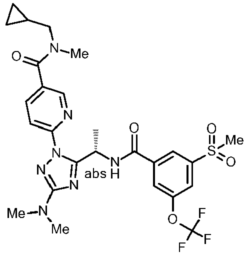 | ¹H-NMR (400.2 MHz, d₆-DMSO): | 610.3 [M+H]⁺ |
| | | δ = 9.4974 (0.8); 9.4797 (0.8); 8.4898 (0.5); 8.4244 (1.6); 8.1109 (1.2); 8.0689 (1.4); 8.0317 (0.4); 7.8279 (1.6); 7.8069 (1.3); 6.1288 (0.5); 6.1114 (0.7); 6.0938 (0.5); 3.3502 (10.7); 3.3298 (147.8); 3.0626 (0.7); 2.9588 (16.0); 2.6758 (0.7); 2.6715 (0.9); 2.6671 (0.7); 2.5244 (4.7); 2.5112 (57.7); 2.5070 (108.8); 2.5025 (139.8); 2.4980 (103.4); 2.4938 (52.2); 2.3338 (0.6); 2.3293 (0.9); 2.3249 (0.6); 2.0746 (0.5); 1.6352 (3.0); 1.6178 (2.9); 0.5060 (0.4); 0.4522 (0.3); 0.4462 (0.3); 0.2929 (0.3); 0.0080 (0.9); -0.0002 (19.3); - 0.0082 (0.8) | |
| I-268 | 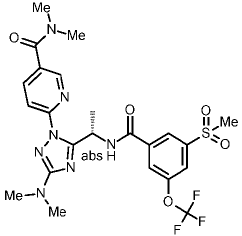 | ¹H-NMR (400.2 MHz, d₆-DMSO): | 570.2 [M+H]⁺ |
| | | δ = 9.4984 (0.7); 9.4802 (0.8); 8.5142 (1.2); 8.5126 (1.3); 8.5086 (1.3); 8.5070 (1.3); 8.4247 (1.0); 8.4211 (1.8); 8.4175 (1.0); 8.3160 (0.5); 8.1090 (1.1); 8.0673 (1.1); 8.0615 (1.5); 8.0557 (0.9); 8.0403 (1.1); 8.0346 (1.1); 7.8230 (1.4); 7.8018 (1.2); 6.1252 (0.5); 6.1077 (0.8); 6.0900 (0.5); 3.3509 (9.4); 3.3285 (226.3); 3.0017 (1.7); 2.9573 (16.0); 2.9258 (1.8); 2.6802 (0.5); 2.6757 (1.1); 2.6712 (1.5); 2.6666 (1.1); 2.6621 (0.5); 2.6029 (0.4); 2.5247 (5.1); 2.5200 (7.4); 2.5112 (86.5); 2.5068 (174.5); 2.5022 (230.4); 2.4976 (167.1); 2.4931 (80.3); 2.3381 (0.5); 2.3336 (1.0); 2.3290 (1.4); 2.3244 (1.0); 2.3199 (0.5); 1.6356 (2.9); 1.6182 (2.8); 0.0081 (1.6); -0.0001 (53.6); -0.0084 (1.6) | |
| I-269 | 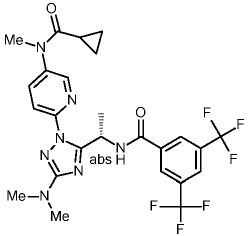 (reference compound) | ¹H-NMR (400.2 MHz, d₆-DMSO): | 570.5 [M+H]⁺ |
| | | δ = 9.5185 (0.7); 9.5005 (0.8); 8.4934 (1.0); 8.4871 (1.0); 8.4487 (2.5); 8.3164 (1.2); 8.0426 (0.4); 8.0361 (0.4); 8.0208 (0.5); 8.0143 (0.5); 7.8437 (1.0); 7.8220 (0.8); 6.0806 (0.5); 6.0630 (0.8); 6.0453 (0.5); 3.3278 (33.8); 3.2178 (0.9); 2.9504 (16.0); 2.6719 (0.4); 2.5967 (0.4); 2.5255 (1.4); 2.5208 (2.1); 2.5121 (25.3); 2.5076 (50.9); 2.5030 (67.0); 2.4983 (48.8); 2.4938 (23.5); 2.3298 (0.4); 2.0754 (1.6); 1.6440 (2.9); 1.6267 (2.8); 0.8253 (0.4); 0.8205 (0.5); 0.8133 (0.8); 0.8088 (1.0); 0.7972 (0.6); 0.6099 (0.5); 0.0080 (0.4); -0.0002 (12.0); -0.0085 (0.3) | |
| I-270 | 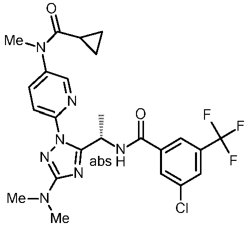 (reference compound) | ¹H-NMR (400.2 MHz, d₆-DMSO): | 536.0 [M+H]⁺ |
| | | δ = 9.3582 (0.8); 9.3402 (0.8); 8.4878 (1.0); 8.4817 (1.1); 8.1408 (1.3); 8.0995 (1.3); 8.0631 (1.3); 8.0411 (0.4); 8.0348 (0.4); 8.0193 (0.5); 8.0129 (0.5); 7.8380 (1.0); 7.8162 (0.8); 6.0416 (0.5); 6.0239 (0.8); 6.0063 (0.6); 3.3294 (54.7); 3.2250 (1.0); 2.9498 (16.0); 2.6763 (0.4); 2.6719 (0.5); 2.6673 (0.3); 2.5957 (0.4); 2.5253 (1.8); 2.5205 (2.6); 2.5119 (29.4); 2.5074 (57.8); 2.5029 (74.8); 2.4983 (54.4); 2.4938 (26.2); 2.3343 (0.3); 2.3296 (0.5); 2.3250 (0.3); 2.0751 (0.7); 1.6233 (3.0); 1.6060 (2.9); 0.8120 (1.1); 0.6202 (0.5); 0.6156 (0.5); 0.0079 (0.4); -0.0002 (12.2); -0.0086 (0.4) | |
| I-271 | 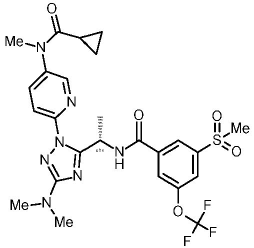 | ¹H-NMR(600.1 MHz, CD3CN 260 K): | 596.2 [M+H]⁺ |
| | | δ= 8.4535 (0.3); 8.2635 (0.6); 8.1207 (0.2); 8.1088 (0.2); 7.9868 (0.4); 7.9746 (0.5); 7.9373 (0.1); 7.9257 (0.2); 7.9228 (0.2); 7.9017 (0.3); 7.8875 (0.1); 6.1220 (0.1); 6.1102 (0.2); 6.0984 (0.1); 3.2260 (1.2); 3.1374 (3.1); 2.9836 (5.7); 2.2990 (5.0); 2.0979 (0.8); 1.9856 (0.4); 1.9775 (0.3); 1.9697 (5.8); 1.9656 (11.0); 1.9615 (16.0); 1.9574 (11.0); 1.9533 (5.6); 1.6460 (0.9); 1.6344 (1.0); 1.3587 (0.1); 1.3520 (0.1); 0.8640 (0.3); 0.8584 (0.3); 0.6121 (0.3); 0.5989 (0.3); -0.0001 (1.5) | |
| I-272 | 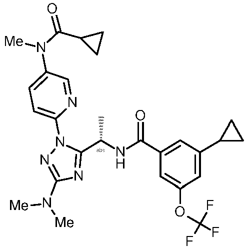 | ¹H-NMR(600.1 MHz, CD3CN 260 K): | 558.2 [M+H]⁺ |
| | | δ= 8.4133 (0.6); 8.4101 (0.6); 7.9236 (0.2); 7.9202 (0.2); 7.9092 (0.4); 7.9058 (0.4); 7.8760 (0.6); 7.8616 (0.3); 7.7802 (0.3); 7.7678 (0.3); 7.3882 (0.6); 7.3162 (0.7); 7.1663 (0.8); 6.0647 (0.3); 6.0528 (0.4); 6.0408 (0.3); 3.2034 (2.6); 2.9843 (10.0); 2.6159 (0.1); 2.3025 (7.0); 1.9944 (0.1); 1.9859 (0.6); 1.9778 (0.5); 1.9699 (6.0); 1.9658 (11.2); 1.9617 (16.0); 1.9576 (11.1); 1.9535 (5.6); 1.6214 (2.1); 1.6098 (2.1); 1.2714 (0.2); 1.2650 (0.2); 1.2586 (0.2); 1.0549 (0.8); 1.0511 (0.9); 1.0409 (0.8); 1.0371 (0.8); 0.8619 (0.2); 0.8500 (0.3); 0.8453 (0.3); 0.8269 (0.3); 0.8096 (0.1); 0.8033 (0.1); 0.7845 (0.1); 0.7779 (0.2); 0.7683 (0.3); 0.7647 (0.4); 0.7566 (0.4); 0.7475 (0.3); 0.7400 (0.3); 0.7253 (0.1); 0.5541 (0.5); 0.5413 (0.5); -0.0001 (1.5) | |
| I-273 | 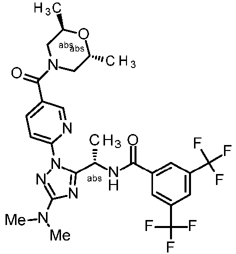 (reference compound) | ¹H-NMR (400.2 MHz, d₆-DMSO): | 614.3 [M+H]⁺ |
| | | δ = 9.5317 (0.9); 9.5141 (0.9); 8.4954 (1.5); 8.4903 (1.5); 8.4637 (3.1); 8.3154 (1.8); 8.0521 (0.9); 8.0465 (0.8); 8.0310 (1.0); 8.0254 (1.0); 7.8445 (1.5); 7.8234 (1.3); 6.1386 (0.6); 6.1212 (0.9); 6.1035 (0.6); 3.3249 (108.8); 2.9569 (16.0); 2.6899 (0.4); 2.6758 (1.0); 2.6714 (1.2); 2.6670 (0.9); 2.5243 (5.4); 2.5068 (148.9); 2.5024 (191.3); 2.4980 (143.6); 2.3336 (0.9); 2.3292 (1.2); 2.3249 (0.9); 1.6416 (3.3); 1.6243 (3.2); 1.3554 (0.4); 1.1371 (0.7); 1.0881 (0.4); 1.0708 (0.3); 1.0004 (0.6); 0.1461 (0.5); 0.0078 (5.2); -0.0001 (110.3); -0.0079 (5.0); -0.1494 (0.5) | |
| I-274 | 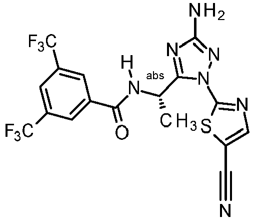 (reference compound) | ¹H-NMR(400.2 MHz, d₆-DMSO): | 476.3 [M+H]⁺ |
| | | δ= 9.6053 (2.9); 9.5889 (3.0); 8.5465 (9.9); 8.5198 (16.0); 8.3426 (4.4); 8.3153 (0.5); 6.3945 (7.7); 5.9765 (0.4); 5.9596 (2.0); 5.9426 (3.0); 5.9257 (2.0); 5.9085 (0.4); 4.3639 (0.4); 4.3514 (0.8); 4.3385 (0.4); 3.4563 (0.8); 3.4436 (0.8); 3.4388 (0.8); 3.4261 (0.8); 3.4214 (0.3); 3.3290 (166.2); 2.6907 (2.1); 2.6768 (0.9); 2.6723 (1.2); 2.6678 (0.9); 2.6632 (0.4); 2.5256 (5.2); 2.5207 (8.4); 2.5123 (76.2); 2.5079 (147.4); 2.5033 (189.7); 2.4988 (136.8); 2.4943 (66.0); 2.3346 (0.8); 2.3302 (1.2); 2.3256 (0.8); 2.3212 (0.4); 1.6180 (10.6); 1.6006 (10.4); 1.2591 (0.4); 1.2431 (0.3); 1.0740 (1.6); 1.0565 (3.1); 1.0390 (1.5); 0.1459 (0.7); 0.0079 (6.5); -0.0002 (151.5); -0.0085 (5.0); -0.1496 (0.6) | |
| I-275 | 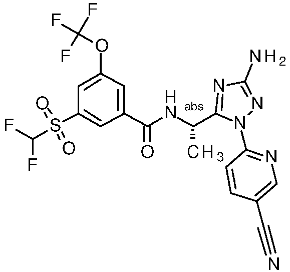 | ¹H-NMR(400.2 MHz, d₆-DMSO): | 532.0 [M+H]⁺ |
| | | δ= 9.6422 (4.3); 9.6249 (4.4); 8.9223 (7.4); 8.9206 (7.8); 8.9168 (8.0); 8.9150 (7.4); 8.5197 (5.5); 8.5161 (9.9); 8.5126 (5.6); 8.4464 (6.5); 8.4408 (6.0); 8.4247 (6.8); 8.4191 (6.8); 8.3847 (6.0); 8.3163 (2.4); 8.1235 (5.8); 7.8286 (7.6); 7.8269 (7.8); 7.8070 (7.3); 7.8052 (7.5); 7.5761 (3.3); 7.4463 (8.2); 7.3166 (4.2); 6.1313 (0.7); 6.1137 (3.1); 6.0964 (4.9); 6.0792 (3.1); 6.0619 (0.7); 6.0021 (11.6); 3.3941 (0.5); 3.3297 (943.0); 2.6946 (0.3); 2.6895 (0.4); 2.6804 (2.2); 2.6760 (4.8); 2.6714 (6.6); 2.6669 (4.8); 2.6623 (2.3); 2.5250 (22.7); 2.5203 (32.9); 2.5115 (381.8); 2.5070 (768.6); 2.5024 (1011.5); 2.4978 (736.3); 2.4933 (354.4); 2.3383 (2.1); 2.3338 (4.6); 2.3292 (6.4); 2.3247 (4.5); 2.3202 (2.1); 2.0749 (2.6); 1.6165 (16.0); 1.5992 (16.0); 0.1459 (1.8); 0.0080 (14.9); - 0.0002 (478.0); -0.0085 (14.6); -0.0231 (0.4); - 0.1496 (1.8) | |
| I-276 | 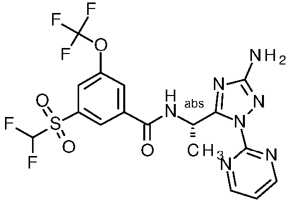 | ¹H-NMR(400.2 MHz, d₆-DMSO): | 508.0 [M+H]⁺ |
| | | δ= 9.6091 (2.4); 9.5915 (2.4); 8.8572 (15.5); 8.8451 (16.0); 8.4828 (3.2); 8.4792 (5.8); 8.4756 (3.4); 8.3497 (3.5); 8.3161 (1.2); 8.1097 (3.4); 7.5689 (2.0); 7.4420 (5.0); 7.4391 (5.1); 7.4300 (7.6); 7.4179 (4.0); 7.3093 (2.4); 6.0676 (0.4); 6.0502 (1.8); 6.0327 (2.9); 6.0152 (1.8); 5.9977 (0.4); 5.7548 (7.1); 3.3307 (425.2); 2.6807 (1.0); 2.6762 (2.0); 2.6716 (2.8); 2.6670 (2.0); 2.6625 (1.0); 2.5251 (9.4); 2.5204 (13.8); 2.5117 (159.5); 2.5072 (320.6); 2.5026 (421.0); 2.4980 (304.8); 2.4934 (145.2); 2.3386 (0.9); 2.3340 (1.9); 2.3294 (2.7); 2.3249 (1.9); 2.3204 (0.9); 1.6157 (9.7); 1.5984 (9.6); 0.1459 (0.8); 0.0080 (6.5); -0.0002 (209.8); - 0.0086 (6.3); -0.0145 (0.5); -0.1496 (0.8) | |
| I-277 | 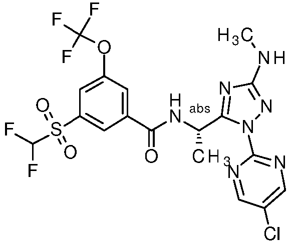 | ¹H-NMR(400.2 MHz, d₆-DMSO): | 556.3 [M+H]⁺ |
| | | δ= 9.6309 (1.4); 9.6134 (1.5); 8.9733 (16.0); 8.4874 (3.2); 8.3599 (2.0); 8.3155 (0.4); 8.1168 (2.0); 7.5708 (1.1); 7.4409 (2.6); 7.3112 (1.3); 6.3440 (0.4); 6.3323 (1.4); 6.3196 (1.4); 6.3070 (0.4); 6.0170 (1.0); 5.9996 (1.6); 5.9822 (1.0); 3.3329 (284.8); 2.8917 (0.5); 2.7493 (6.7); 2.7368 (6.7); 2.6763 (0.8); 2.6718 (1.1); 2.6673 (0.8); 2.5252 (4.0); 2.5203 (6.4); 2.5118 (69.2); 2.5074 (135.1); 2.5028 (173.9); 2.4983 (126.0); 2.4939 (61.6); 2.3342 (0.8); 2.3297 (1.1); 2.3252 (0.8); 2.3207 (0.4); 1.6100 (5.3); 1.5927 (5.3); 0.1459 (0.5); 0.0079 (4.2); -0.0003 (115.3); -0.0085 (4.0); -0.1496 (0.5) | |
| I-278 | 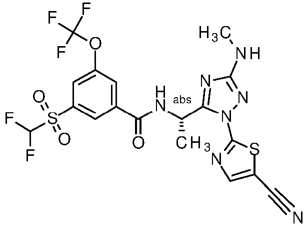 | ¹H-NMR(400.2 MHz, d₆-DMSO): | 552.2 [M+H]⁺ |
| | | δ= 9.7183 (4.0); 9.7021 (4.1); 8.5371 (8.1); 8.5159 (16.0); 8.3933 (5.7); 8.3154 (1.0); 8.1401 (5.7); 7.5811 (2.6); 7.4515 (5.8); 7.3218 (3.0); 6.8893 (1.1); 6.8777 (3.7); 6.8652 (3.7); 6.8528 (1.1); 5.9757 (0.6); 5.9593 (2.4); 5.9422 (3.6); 5.9255 (2.4); 5.9085 (0.5); 3.4338 (0.4); 3.3312 (1027.0); 2.7521 (15.6); 2.7399 (15.7); 2.6711 (4.4); 2.6180 (0.4); 2.5064 (496.7); 2.5022 (673.4); 2.4982 (546.4); 2.3290 (4.1); 1.6149 (13.1); 1.5975 (13.1); 1.2334 (0.3); 0.1454 (2.0); 0.0077 (14.0); 0.0055 (13.9); -0.0004 (413.1); -0.1504 (2.0) | |
| I-279 | 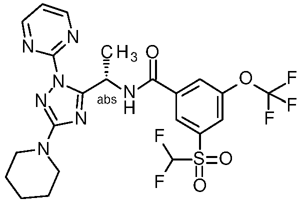 | ¹H-NMR(400.2 MHz, d₆-DMSO): | 576.4 [M+H]⁺ |
| | | δ= 9.6136 (2.7); 9.5956 (2.7); 8.8859 (15.6); 8.8738 (16.0); 8.4356 (3.6); 8.4320 (6.3); 8.4284 (3.5); 8.3155 (1.6); 8.3067 (3.8); 8.1055 (3.8); 7.5630 (2.2); 7.4804 (4.1); 7.4683 (7.7); 7.4562 (4.0); 7.4332 (5.1); 7.3035 (2.6); 6.0729 (0.4); 6.0554 (2.0); 6.0377 (3.1); 6.0201 (2.0); 6.0025 (0.4); 3.3790 (7.4); 3.3319 (702.6); 2.6809 (1.0); 2.6764 (2.1); 2.6718 (2.8); 2.6672 (2.0); 2.6627 (0.9); 2.5253 (9.8); 2.5206 (14.3); 2.5119 (165.6); 2.5074 (329.6); 2.5028 (426.5); 2.4982 (303.3); 2.4937 (142.8); 2.3388 (0.9); 2.3342 (2.0); 2.3297 (2.7); 2.3251 (1.9); 2.3207 (0.9); 1.6269 (10.7); 1.6096 (10.6); 1.5529 (13.5); 0.1460 (1.5); 0.0080 (11.8); - 0.0001 (361.9); -0.0085 (11.5); -0.1496 (1.5) | |
| I-280 | 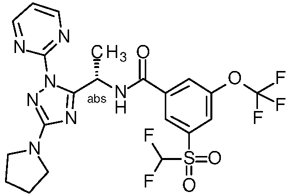 | ¹H-NMR(400.2 MHz, d₆-DMSO): | 562.3 [M+H]⁺ |
| | | δ= 9.6804 (2.5); 9.6624 (2.6); 8.8889 (15.7); 8.8768 (16.0); 8.4546 (3.4); 8.4511 (6.0); 8.4476 (3.5); 8.3198 (3.8); 8.3156 (4.7); 8.3075 (0.4); 8.1448 (0.4); 8.1062 (4.0); 8.0256 (0.3); 7.5645 (2.1); 7.4774 (4.1); 7.4653 (7.7); 7.4532 (4.5); 7.4346 (5.0); 7.3049 (2.5); 6.0883 (0.4); 6.0709 (1.9); 6.0532 (3.1); 6.0357 (2.0); 6.0183 (0.4); 5.7554 (1.5); 3.7421 (0.6); 3.5089 (0.4); 3.4923 (0.7); 3.4759 (0.5); 3.4363 (0.3); 3.3778 (4.9); 3.3615 (11.6); 3.3296 (1008.4); 2.6899 (1.5); 2.6805 (1.9); 2.6760 (3.9); 2.6714 (5.5); 2.6668 (4.0); 2.6622 (1.9); 2.5879 (1.0); 2.5249 (18.9); 2.5202 (28.1); 2.5115 (321.4); 2.5070 (644.2); 2.5024 (840.3); 2.4978 (603.8); 2.4933 (288.2); 2.3381 (1.8); 2.3338 (4.1); 2.3293 (5.5); 2.3247 (3.8); 2.3202 (1.7); 1.9106 (4.1); 1.9023 (4.5); 1.8943 (10.4); 1.8865 (4.6); 1.8777 (3.8); 1.8637 (0.7); 1.8529 (0.5); 1.8383 (0.4); 1.6428 (10.5); 1.6254 (10.4); 1.2546 (0.3); 1.2397 (0.4); 0.1459 (3.0); 0.0079 (24.8); - 0.0002 (757.6); -0.0086 (25.0); -0.0153 (2.0); - 0.0204 (1.3); -0.1496 (3.1) | |
| I-281 | 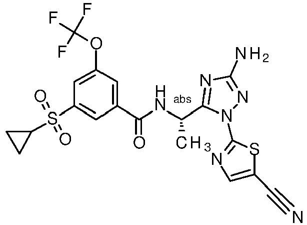 | ¹H-NMR(400.2 MHz, d₆-DMSO): | 528.3 [M+H]⁺ |
| | | δ= 9.5923 (2.3); 9.5761 (2.4); 8.5207 (16.0); 8.4716 (3.2); 8.4679 (5.9); 8.4643 (3.2); 8.3158 (1.0); 8.1935 (3.2); 8.1911 (2.9); 8.0766 (3.2); 6.3984 (5.9); 5.9441 (1.6); 5.9272 (2.3); 5.9103 (1.6); 3.3286 (455.1); 3.3119 (1.1); 3.0744 (0.5); 3.0625 (1.0); 3.0548 (1.1); 3.0503 (0.7); 3.0431 (2.2); 3.0349 (0.8); 3.0310 (1.2); 3.0233 (1.1); 3.0113 (0.6); 2.6804 (0.8); 2.6759 (1.9); 2.6713 (2.6); 2.6667 (1.9); 2.6621 (0.9); 2.5249 (8.2); 2.5202 (11.8); 2.5115 (149.2); 2.5069 (304.6); 2.5023 (401.4); 2.4977 (287.8); 2.4931 (136.0); 2.3383 (0.8); 2.3337 (1.8); 2.3292 (2.6); 2.3246 (1.8); 2.3201 (0.8); 1.6147 (8.3); 1.5973 (8.3); 1.2506 (0.4); 1.2382 (0.5); 1.2272 (1.2); 1.2242 (1.2); 1.2147 (2.4); 1.2060 (2.7); 1.1951 (1.8); 1.1845 (0.6); 1.1727 (0.5); 1.1584 (0.4); 1.1458 (0.6); 1.1376 (0.8); 1.1228 (3.3); 1.1177 (2.6); 1.1032 (3.1); 1.0980 (2.2); 1.0805 (0.4); 0.1459 (1.4); 0.0135 (0.7); 0.0128 (0.7); 0.0081 (11.2); -0.0001 (370.2); -0.0085 (11.1); -0.0165 (0.6); -0.0245 (0.4); -0.1496 (1.4) | |
| I-282 | 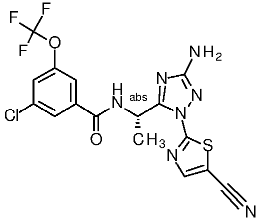 (reference compound) | ¹H-NMR(400.2 MHz, d₆-DMSO): | 458.3 [M+H]⁺ |
| | | δ= 9.3660 (2.3); 9.3495 (2.3); 8.5140 (16.0); 8.3154 (1.8); 8.0462 (3.3); 8.0421 (5.2); 8.0381 (3.4); 7.8172 (3.4); 7.8146 (3.8); 7.8112 (4.0); 7.8086 (3.6); 7.8061 (3.4); 6.3912 (5.9); 5.9123 (0.3); 5.8952 (1.6); 5.8783 (2.3); 5.8612 (1.6); 3.3969 (0.4); 3.3298 (971.5); 2.6804 (1.5); 2.6759 (3.3); 2.6713 (4.5); 2.6667 (3.2); 2.6621 (1.6); 2.5884 (0.5); 2.5248 (15.1); 2.5201 (21.6); 2.5114 (256.8); 2.5069 (524.6); 2.5023 (689.5); 2.4977 (495.0); 2.4931 (234.5); 2.3382 (1.4); 2.3338 (3.1); 2.3291 (4.4); 2.3245 (3.1); 2.3200 (1.4); 1.5862 (8.5); 1.5688 (8.5); 0.1459 (2.5); 0.0279 (0.3); 0.0147 (1.1); 0.0080 (20.4); -0.0002 (662.6); -0.0086 (20.8); -0.0231 (0.7); -0.1496 (2.5) | |
| I-283 | 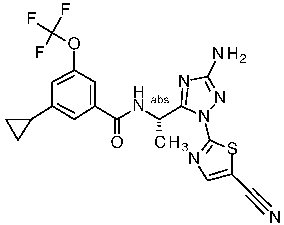 | ¹H-NMR(400.2 MHz, d₆-DMSO): | 464.4 [M+H]⁺ |
| | | δ= 9.1965 (2.7); 9.1800 (2.8); 8.5161 (16.0); 8.3155 (0.9); 7.5925 (5.6); 7.5890 (3.8); 7.5785 (3.3); 7.5760 (3.7); 7.2972 (3.6); 6.3745 (7.0); 5.9115 (0.4); 5.8943 (1.8); 5.8773 (2.8); 5.8602 (1.8); 5.8424 (0.4); 3.3324 (627.7); 2.6806 (0.9); 2.6761 (1.8); 2.6715 (2.6); 2.6670 (1.8); 2.6624 (0.9); 2.5251 (8.2); 2.5203 (12.1); 2.5116 (151.2); 2.5071 (301.2); 2.5026 (389.7); 2.4980 (278.3); 2.4934 (131.8); 2.3385 (0.8); 2.3340 (1.8); 2.3294 (2.4); 2.3248 (1.7); 2.3203 (0.8); 2.0873 (0.5); 2.0747 (1.1); 2.0662 (1.2); 2.0539 (2.2); 2.0412 (1.3); 2.0330 (1.2); 2.0204 (0.6); 1.5845 (9.8); 1.5670 (9.8); 1.0603 (0.9); 1.0490 (3.7); 1.0434 (4.0); 1.0327 (1.6); 1.0280 (3.8); 1.0225 (3.9); 1.0121 (1.1); 0.8124 (1.5); 0.8022 (3.6); 0.7987 (3.5); 0.7965 (3.5); 0.7898 (3.7); 0.7857 (3.4); 0.7740 (1.2); 0.1459 (1.0); 0.0080 (7.3); -0.0002 (236.4); - 0.0085 (7.5); -0.1496 (1.0) | |
| I-284 | 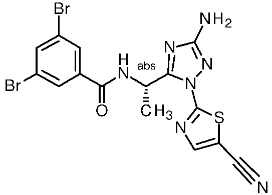 (reference compound) | ¹H-NMR(400.2 MHz, d₆-DMSO): | 498.1 [M+H]⁺ |
| | | δ= 9.3020 (2.4); 9.2855 (2.5); 8.5155 (14.4); 8.3153 (0.6); 8.0761 (10.4); 8.0717 (16.0); 8.0571 (4.9); 8.0528 (5.4); 8.0484 (2.2); 6.3900 (6.3); 5.8946 (0.3); 5.8772 (1.7); 5.8602 (2.6); 5.8432 (1.7); 5.8256 (0.3); 3.3712 (0.4); 3.3321 (393.8); 2.6808 (0.5); 2.6763 (1.2); 2.6717 (1.6); 2.6672 (1.2); 2.6625 (0.6); 2.5253 (5.1); 2.5206 (7.4); 2.5118 (94.7); 2.5074 (193.0); 2.5028 (253.4); 2.4982 (182.7); 2.4936 (87.5); 2.3386 (0.5); 2.3342 (1.2); 2.3296 (1.6); 2.3250 (1.2); 2.3204 (0.5); 1.5713 (9.0); 1.5539 (9.0); 0.1459 (0.6); 0.0080 (4.8); - 0.0002 (162.5); -0.0085 (5.1); -0.1496 (0.7) | |
| I-285 | 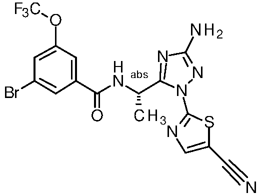 (reference compound) | ¹H-NMR(400.2 MHz, d₆-DMSO): | 504.2 [M+H]⁺ |
| | | δ= 9.3669 (3.0); 9.3507 (3.1); 8.5144 (16.0); 8.3153 (1.6); 8.1801 (3.8); 8.1763 (6.5); 8.1725 (3.9); 7.9097 (3.9); 7.8425 (4.0); 6.3909 (7.6); 5.9106 (0.4); 5.8938 (1.9); 5.8768 (3.0); 5.8597 (2.0); 5.8420 (0.4); 3.4371 (0.4); 3.3697 (1.6); 3.3331 (1546.8); 2.6806 (1.6); 2.6761 (3.3); 2.6716 (4.6); 2.6670 (3.4); 2.6624 (1.6); 2.5251 (14.7); 2.5203 (21.6); 2.5117 (273.7); 2.5072 (554.5); 2.5027 (724.7); 2.4981 (518.9); 2.4935 (247.6); 2.3385 (1.4); 2.3340 (3.2); 2.3294 (4.5); 2.3249 (3.2); 2.3204 (1.4); 2.0744 (1.0); 1.5845 (10.5); 1.5670 (10.5); 1.3695 (0.4); 0.1460 (1.6); 0.0081 (12.1); 0.0000 (387.1); -0.0084 (12.3); -0.1494 (1.7) | |
| I-286 | 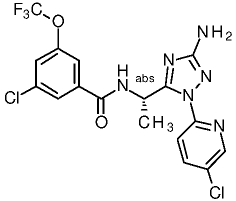 | ¹H-NMR(400.2 MHz, d₆-DMSO): | 458.3 [M+H]⁺ |
| | | δ= 9.2470 (4.0); 9.2294 (4.1); 8.5084 (7.3); 8.5073 (7.6); 8.5020 (7.9); 8.5008 (7.6); 8.3181 (0.4); 8.1044 (6.1); 8.0978 (5.8); 8.0824 (6.6); 8.0758 (6.6); 7.9924 (5.8); 7.9883 (9.2); 7.9843 (6.1); 7.7701 (8.5); 7.7677 (8.0); 7.7646 (6.1); 7.7017 (8.2); 7.7004 (8.1); 7.6797 (7.6); 7.6784 (7.5); 6.0029 (0.6); 5.9858 (3.0); 5.9684 (4.9); 5.9509 (3.1); 5.9335 (0.6); 5.7965 (1.6); 5.7571 (3.0); 3.3406 (28.2); 2.6777 (0.7); 2.6731 (1.0); 2.6686 (0.7); 2.5593 (1.9); 2.5454 (4.1); 2.5315 (2.5); 2.5267 (3.3); 2.5220 (4.7); 2.5133 (57.6); 2.5088 (117.8); 2.5042 (154.7); 2.4995 (111.2); 2.4950 (52.7); 2.3356 (0.7); 2.3310 (1.0); 2.3263 (0.7); 1.5774 (16.0); 1.5600 (15.9); 1.2812 (0.5); 1.2639 (0.8); 1.2511 (0.3); 1.2470 (0.5); 0.1459 (0.4); 0.0081 (3.1); -0.0001 (102.4); -0.0085 (3.1); -0.1495 (0.4) | |

| | | | |
|---|---|---|---|
| ¹⁾ '260 K' denotes that the measurement was conducted at 260 Kelvin. ²⁾ The stated mass corresponds to the peak from the isotope pattern of the [M+H]⁺ ion with the highest intensity. ³⁾ 'abs' denotes that the compound was obtained in an enantiomerically enriched or pure form with the major stereoisomer having the absolute configuration depicted in the drawing. | | | |

**Table 2 (Intermediates)**

| **Example** | **Structure¹⁾** | **NMR data** | **ESI Mass (m/z)²⁾** |
|---|---|---|---|
| INT-001 | 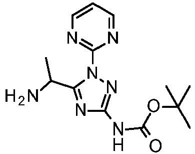 | | 306.2 [M+H]⁺ |
| INT-002 | 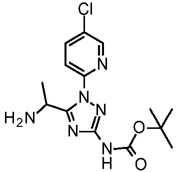 | | 339.1 [M+H]⁺ |
| INT-003 | 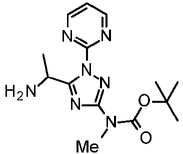 | | 320.2 [M+H]⁺ |
| INT-004 | 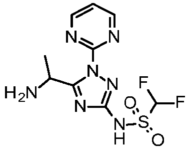 | | 320.0 [M+H]⁺ |
| INT-005 | 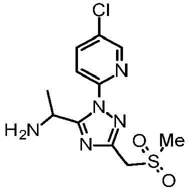 (reference compound) | | 316.1 [M+H]⁺ |
| INT-006 | 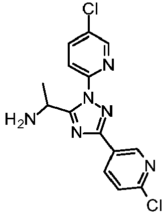 (reference compound) | | 335.1 [M+H]⁺ |
| INT-007 | 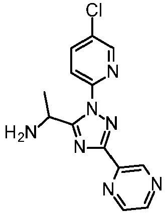 (reference compound) | | 302.1 [M+H]⁺ |
| INT-008 | 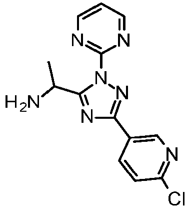 (reference compound) | | 302.1 [M+H]⁺ |
| INT-009 | 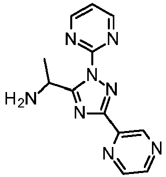 (reference compound) | | 269.1 [M+H]⁺ |
| INT-010 | 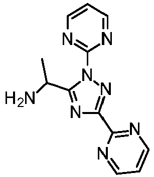 (reference compound) | | 269.2 [M+H]⁺ |
| INT-011 | 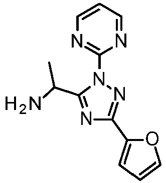 (reference compound) | | 257.2 [M+H]⁺ |
| INT-012 | 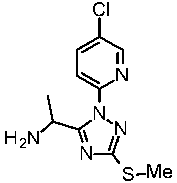 (reference compound) | | 270.1 [M+H]⁺ |
| INT-013 | 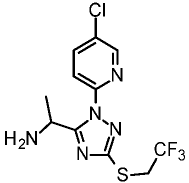 (reference compound) | | n.d. |
| INT-014 | 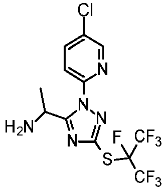 (reference compound) | | 424.1 [M+H]⁺ |
| INT-015 | 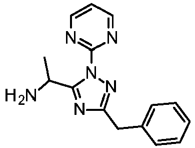 (reference compound) | | 281.1 [M+H]⁺ |
| INT-016 | 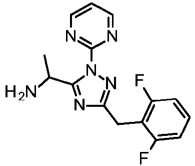 (reference compound) | | 317.1 [M+H]+ |
| INT-017 | 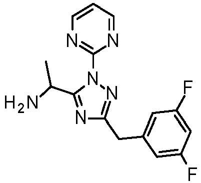 (reference compound) | | 316.9 [M+H]⁺ |
| INT-018 | 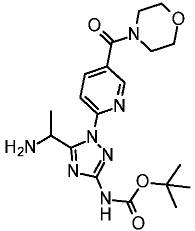 | | 418.2 [M+H]⁺ |
| INT-019 | 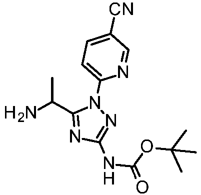 | | 330.2 [M+H]⁺ |
| INT-020 | 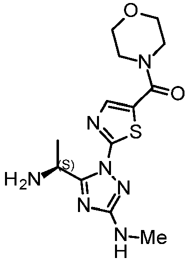 | | n.d. |
| INT-021 | 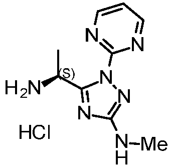 | | 220.1 [M-HCl]⁺ |
| INT-022 | 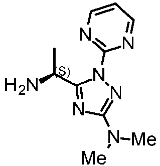 | | 234.2 [M+H]⁺ |
| INT-023 | 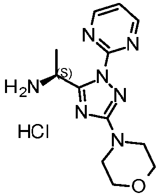 | | 276.2 [M-HCl]⁺ |
| INT-024 | 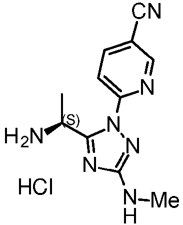 | | 244.2 [M-HCl]⁺ |
| INT-025 | 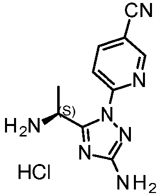 | | n.d. |
| INT-026 | 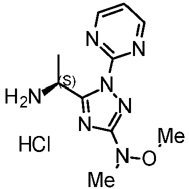 | | 250.1 [M-HCl]⁺ |
| INT-027 | 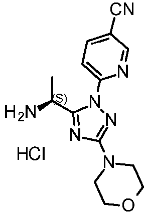 | ¹H-NMR(400.2 MHz, d₆-DMSO): | 300.2 [M-HCl]⁺ |
| | | δ= 9.0074 (4.6); 9.0057 (4.8); 9.0020 (5.0); 9.0001 (4.6); 8.6886 (4.4); 8.5344 (4.1); 8.5288 (3.9); 8.5127 (4.3); 8.5072 (4.3); 7.9635 (4.8); 7.9618 (4.8); 7.9418 (4.6); 7.9400 (4.6); 5.3594 (0.6); 5.3428 (1.8); 5.3259 (1.8); 5.3087 (0.6); 3.7366 (5.5); 3.7254 (7.9); 3.7128 (6.5); 3.5684 (16.0); 3.4722 (0.3); 3.4523 (6.4); 3.4399 (7.6); 3.4285 (5.4); 3.3367 (104.3); 2.6773 (0.3); 2.6726 (0.5); 2.6681 (0.3); 2.5262 (1.4); 2.5214 (1.9); 2.5129 (27.0); 2.5084 (56.4); 2.5038 (74.7); 2.4991 (53.2); 2.4946 (25.1); 2.3351 (0.3); 2.3305 (0.4); 2.3259 (0.3); 1.6244 (0.6); 1.6116 (11.3); 1.5947 (11.1); - 0.0002 (0.8) | |
| INT-028 | 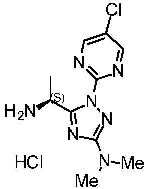 | ¹H-NMR(400.2 MHz, d₆-DMSO): | 268.1 [M-HCl]⁺ |
| | | δ = 9.0302 (8.4); 8.6536 (1.0); 5.2392 (0.5); 5.2224 (0.5); 3.5685 (0.8); 3.3430 (34.4); 3.0129 (16.0); 2.5220 (0.5); 2.5134 (6.3); 2.5089 (13.0); 2.5043 (16.9); 2.4997 (11.8); 2.4951 (5.4); 1.6075 (2.8); 1.5907 (2.7) | |
| INT-029 | 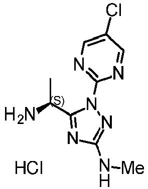 | ¹H-NMR(400.2 MHz, d₆-DMSO): | 254.1 [M-HCl]⁺ |
| | | δ= 9.0132 (16.0); 8.6489 (1.6); 8.6398 (1.6); 5.2336 (0.5); 5.2186 (0.6); 5.2030 (0.5); 3.9199 (2.4); 3.5683 (14.5); 2.7946 (13.7); 2.5266 (0.9); 2.5218 (1.4); 2.5131 (19.2); 2.5087 (39.3); 2.5041 (51.6); 2.4995 (37.4); 2.4949 (18.2); 2.3310 (0.3); 1.5948 (5.4); 1.5780 (5.4); 0.0079 (1.8); -0.0002 (52.0); - 0.0086 (1.6) | |
| INT-030 | 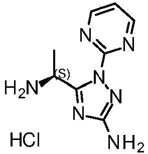 | | 206.1 [M-HCl]⁺ |
| INT-031 | 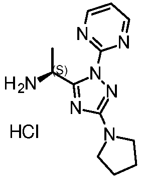 | | 260.2 [M-HCl]⁺ |
| INT-032 | 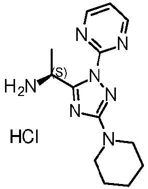 | ¹H-NMR(400.2 MHz, d₆-DMSO): | 274.2 [M-HCl]⁺ |
| | | δ= 8.9258 (6.2); 8.9137 (6.2); 8.6853 (2.8); 8.6763 (2.8); 7.5400 (2.8); 7.5279 (5.2); 7.5157 (2.7); 5.2916 (0.4); 5.2775 (0.9); 5.2626 (1.2); 5.2469 (0.9); 5.2316 (0.4); 4.2233 (16.0); 3.5686 (1.0); 3.4459 (6.2); 2.6748 (0.4); 2.5283 (1.3); 2.5236 (2.0); 2.5150 (25.9); 2.5105 (52.8); 2.5059 (68.5); 2.5013 (48.2); 2.4967 (22.3); 2.3327 (0.4); 1.7648 (8.0); 1.6182 (10.8); 1.6013 (12.9); 1.5891 (10.9); -0.0002 (0.5) | |
| INT-033 | 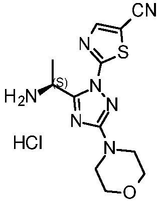 | ¹H-NMR(400.2 MHz, d₆-DMSO): | 306.2 [M-HCl]⁺ |
| | | δ= 8.8169 (5.5); 8.6075 (16.0); 5.2418 (1.6); 5.2249 (1.6); 5.2090 (0.6); 3.7261 (6.8); 3.7149 (10.1); 3.7024 (8.0); 3.5936 (0.4); 3.5684 (15.3); 3.4446 (7.9); 3.4323 (9.7); 3.4210 (6.7); 3.3438 (57.0); 2.6728 (0.4); 2.6685 (0.4); 2.6531 (0.6); 2.5430 (0.3); 2.5263 (1.5); 2.5126 (28.1); 2.5084 (56.3); 2.5039 (73.4); 2.4994 (53.4); 2.4951 (26.4); 2.3307 (0.4); 2.3263 (0.3); 1.6301 (0.5); 1.6147 (12.8); 1.5978 (12.5); -0.0002 (0.4) | |
| INT-034 | 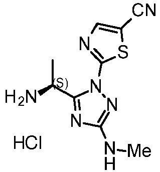 | ¹H-NMR(400.2 MHz, d₆-DMSO): | 250.1 [M-HCl]⁺ |
| | | δ= 8.7868 (4.1); 8.5721 (16.0); 7.0181 (1.0); 5.2013 (1.2); 3.5685 (10.4); 3.3878 (24.1); 2.7976 (10.7); 2.6735 (0.4); 2.5268 (1.5); 2.5135 (27.0); 2.5091 (50.8); 2.5046 (63.4); 2.5001 (44.7); 2.4956 (21.1); 2.3314 (0.4); 1.6037 (11.6); 1.5867 (11.4) | |
| INT-035 | 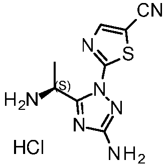 | ¹H-NMR(400.2 MHz, d₆-DMSO): | 236.1 [M-HCl]⁺ |
| | | δ= 8.6965 (4.6); 8.6747 (1.5); 8.5848 (0.6); 8.5708 (16.0); 8.3234 (0.4); 8.2507 (0.9); 7.3390 (1.6); 7.2117 (1.9); 7.0843 (1.6); 6.5522 (1.1); 5.2004 (1.1); 5.1862 (1.4); 5.1706 (1.1); 3.5681 (8.6); 3.5024 (0.4); 3.4901 (0.5); 3.4719 (0.7); 3.4617 (0.8); 3.3849 (69.6); 2.6768 (0.9); 2.6722 (1.2); 2.6678 (0.9); 2.5255 (4.2); 2.5120 (77.9); 2.5078 (152.0); 2.5033 (196.5); 2.4988 (144.5); 2.4946 (72.1); 2.3345 (0.8); 2.3301 (1.2); 2.3255 (0.8); 1.6790 (0.4); 1.6689 (0.6); 1.6520 (0.6); 1.6226 (0.8); 1.5982 (12.9); 1.5813 (12.7); 1.2346 (0.5); -0.0003 (7.2) | |
| INT-036 | 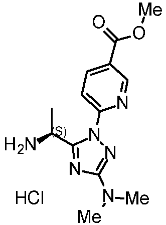 | ¹H-NMR(400.2 MHz, d₆-DMSO): | 291.5 [M-HCl]⁺ |
| | | δ= 8.9997 (1.4); 8.9987 (1.4); 8.9942 (1.4); 8.6467 (1.4); 8.5193 (1.0); 8.5136 (1.0); 8.4977 (1.1); 8.4919 (1.0); 7.9443 (1.4); 7.9227 (1.3); 5.3583 (0.9); 5.3413 (0.9); 3.9130 (8.3); 3.5687 (3.0); 3.3328 (17.5); 3.0343 (16.0); 2.6727 (0.4); 2.5262 (0.6); 2.5127 (10.1); 2.5084 (19.5); 2.5038 (25.0); 2.4993 (18.3); 2.4950 (9.0); 1.6214 (3.3); 1.6045 (3.2); 0.0078 (0.6); -0.0002 (16.9); - 0.0086 (0.6) | |
| INT-037 | 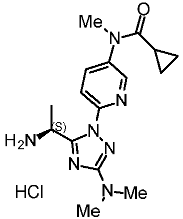 | ¹H-NMR(400.2 MHz, d₆-DMSO): | 330.4 [M-HCl]⁺ |
| | | δ= 8.5478 (1.7); 8.5421 (1.7); 8.1004 (0.4); 8.0941 (0.3); 8.0789 (0.4); 8.0728 (0.4); 7.8919 (0.9); 7.8700 (0.7); 5.3057 (0.3); 3.5681 (10.6); 3.3463 (62.6); 3.3089 (0.4); 3.2803 (0.8); 3.0193 (16.0); 2.9701 (0.8); 2.6761 (0.4); 2.6715 (0.6); 2.6669 (0.4); 2.6598 (0.5); 2.5250 (2.1); 2.5202 (3.2); 2.5116 (38.2); 2.5071 (75.5); 2.5025 (98.0); 2.4979 (70.6); 2.4934 (33.8); 2.3341 (0.4); 2.3294 (0.6); 2.3249 (0.4); 1.6129 (3.1); 1.5961 (2.9); 0.8650 (0.4); 0.8549 (0.9); 0.8474 (1.2); 0.8363 (1.0); 0.8288 (0.4); 0.7020 (0.5); 0.0079 (1.0); -0.0002 (31.1); - 0.0086 (1.0) | |
| INT-038 | 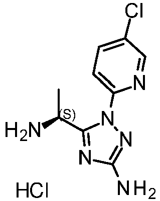 | | 239.0 [M-HCl]⁺ |
| INT-039 | 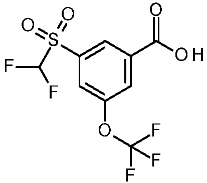 | ¹H NMR (400 MHz, d₆-DMSO ) δ 7.47 (t, 1H), 8.21 (s, 1H), 8.32 (s, 1H), 8.40 (s, 1H), 13.79 (s, 1H). | 319.0 [M+H]⁺ |

| | | | |
|---|---|---|---|
| ¹⁾ '(S)' denotes that the compound was obtained in an enantiomerically enriched or pure form with the major stereoisomer having the absolute configuration depicted in the drawing. ²⁾ The stated mass corresponds to the peak from the isotope pattern of the [M+H]⁺ ion with the highest intensity. n.d. = not determined. | | | |

### Biological examples

### Rhipicephalus (Boophilus) microplus - in-vitro contact tests larval cattle tick (Strain Parkhurst, resistant against synthetic pyrethroids)

9 mg compound is solved in 1 mL acetone and diluted with acetone to the desired concentration. 250 µL of the test solution is filled in 25 mL glass test tubes and homogeneously distributed on the inner walls by rotation and tilting on a shaking device (2 h at 30 rpm). With a compound concentration of 900 ppm, an inner surface of 44.7 cm² and a homogeneous distribution, a dose of 5 µg/cm² is achieved.

After the solvent has evaporated, each test tube is filled with 20-50 cattle tick larvae (*Rhipicephalus microplus*), closed with a perforated lid and incubated in a horizontal position at 85 % relative humidity and 27 °C in an incubator. After 48 hours efficacy is determined. The larvae are patted on the ground of the tubes and negative geotactic behaviour is recorded. Larvae that climb back to the top of the vial in a manner comparable to untreated control larvae are marked as alive, larvae not climbing back up comparable to untreated control larvae but are moving uncoordinatedly or only twitching their legs are marked as moribund, tick larvae remaining on the bottom and not moving at all are counted as dead.

A compound shows a good efficacy against *Rhipicephalus microplus*, if at a compound concentration of 5 µg/cm² an efficacy of at least 80 % is monitored. An efficacy of 100 % means all larvae are dead or moribund; 0 % means no larvae are dead or moribund.

In this test, for example, the following compounds from the preparation examples showed good activity of 100 % at an application rate of 5 µg/cm² (= 500 g/ha): I-006, I-007, I-009, I-010, I-011, I-012, I-014, I-016, I-018, I-035, I-036, I-040, I-059, I-062, I-069, I-070, I-075, I-079, I-081, I-088, I-092, I-093, I-095, I-100, I-101, I-103, I-104, I-105, I-112, I-114, I-115, I-116, I-117, I-118, I-121, I-122, I-124, I-127, I-129, I-130, I-131, I-132, I-133, I-134, I-136, I-138, I-139, I-140, I-141, I-144, I-145, I-147, I-154, I-155, I-158, I-159, I-161.

In this test, for example, the following compounds from the preparation examples showed good activity of 90 % at an application rate of 5 µg/cm² (= 500 g/ha): I-004, I-013, I-022, I-027, I-071, I-076, I-078, I-090, I-097, I-099, I-108, I-113, I-120, I-123, I-146, I-148.

In this test, for example, the following compounds from the preparation examples showed good activity of 80 % at an application rate of 5 µg/cm² (= 500 g/ha): I-053, I-058, I-089, I-106, I-137.

### Rhipicephalus (Boophilus) microplus - dip test

| | |
|---|---|
| Test animal: | cattle ticks *(Rhipicephalus microplus*) strain Parkhurst, SP-resistant |
| Solvent: | dimethyl sulfoxide |

To produce a suitable preparation of active compound, 10 mg of active compound are dissolved in 0.5 mL solvent, and the concentrate is diluted with water to the desired concentration.

This compound solution is pipetted into tubes. 8-10 engorged, adult, female cattle ticks *(Rhipicephalus microplus)* are placed in perforated tubes. These tubes are immersed in the aqueous compound solution until the ticks are completely moistened. After the liquid has drained off, the ticks are transferred to a filter paper in a plastic tray and stored in a climate chamber.

After 7 days egg deposition of fertile eggs is monitored. Eggs where fertility is not visible are stored in a climate chamber till hatching after about 42 days. An efficacy of 100 % means all eggs are infertile; 0 % means all eggs are fertile.

In this test, for example, the following compounds from the preparation examples showed good activity of 100 % at an application rate of 100 ppm: I-088.

In this test, for example, the following compounds from the preparation examples showed good activity of 80 % at an application rate of 100 ppm: I-004, I-009, I-013.

### Rhipicephalus (Boophilus) microplus - injection test

| | |
|---|---|
| Solvent: | dimethyl sulfoxide |

To produce a suitable preparation of active compound, 10 mg of active compound are dissolved in 0.5 mL solvent, and the concentrate is diluted with solvent to the desired concentration.

Five adult engorged female ticks (*Rhipicephalus microplus*) are injected with 1 µL compound solution into the abdomen. The ticks are transferred into replica plates and incubated in a climate chamber.

After 7 days egg deposition of fertile eggs is monitored. Eggs where fertility is not visible are stored in a climate chamber till hatching after about 42 days. An efficacy of 100 % means all eggs are infertile; 0 % means all eggs are fertile.

In this test, for example, the following compounds from the preparation examples showed good activity of 100 % at an application rate of 20 µg/animal: I-001, I-002, I-003, I-005, I-006, I-010, I-011, I-012, I-013, I-014, I-015, I-016, I-017, I-018, I-026, I-027, I-028, I-029, I-030, I-035, I-036, I-037, I-038, I-040, I-042, I-044, I-045, I-046, I-047, I-049, I-050, I-051, I-052, I-054, I-055, 1-056, I-057, I-058, I-059, I-060, I-062, I-063, I-065, I-066, I-067, I-068, I-069, I-070, I-071, I-076, I-077, I-078, I-079, I-121.

In this test, for example, the following compounds from the preparation examples showed good activity of 80 % at an application rate of 20 µg/animal: I-039.

In this test, for example, the following compounds from the preparation examples showed good activity of 100 % at an application rate of 4 µg/animal: I-001, I-002, I-004, I-005, I-006, I-007, I-008, I-009, I-010, I-011, I-012, I-013, I-014, I-015, I-016, I-017, I-018, I-019, I-020, I-022, I-026, I-028, I-029, I-030, I-037, I-038, I-039, I-040, I-044, I-045, I-046, I-047, I-048, I-050, I-051, I-052, I-058, I-059, I-060, I-062, I-063, I-065, I-066, I-069, I-070, I-071, I-074, I-075, I-076, I-077, I-078, I-079, I-080, I-081, I-082, I-088, I-089, I-090, I-091, I-092, I-093, I-096, I-097, I-098, I-099, I-100, I-101, I-102, I-103, I-104, I-105, I-117, I-118, I-121.

In this test, for example, the following compounds from the preparation examples showed good activity of 80 % at an application rate of 4 µg/animal: I-003, I-021, I-027, I-043, I-049, I-056, I-057, I-068.

### Ctenocephalides felis - in-vitro contact tests adult cat flea

9 mg compound is solved in 1 mL acetone and diluted with acetone to the desired concentration. 250 µL of the test solution is filled in 25 mL glass test tubes and homogeneously distributed on the inner walls by rotation and tilting on a shaking device (2 h at 30 rpm). With a compound concentration of 900 ppm, an inner surface of 44.7 cm² and a homogeneous distribution, a dose of 5 µg/cm² is achieved.

After the solvent has evaporated, each test tube is filled with 5-10 adult cat fleas (*Ctenocephalides felis*), closed with a perforated lid and incubated in a lying position at room temperature and relative humidity. After 48 hours efficacy is determined. The fleas are patted on the ground of the tubes and are incubated on a heating plate at 45-50 °C for at most 5 minutes. Immotile or uncoordinated moving fleas, which are not able to escape the heat by climbing upwards, are marked as dead or moribund.

A compound shows a good efficacy against *Ctenocephalides felis,* if at a compound concentration of 5 µg/cm² an efficacy of at least 80 % is monitored. An efficacy of 100 % means all fleas are dead or moribund; 0 % means no fleas are dead or moribund.

In this test, for example, the following compounds from the preparation examples showed good activity of 100 % at an application rate of 5 µg/cm² (= 500 g/ha): I-004, I-006, I-007, I-018, I-022, I-062, I-076, I-088, I-091, I-092, I-099, I-100, I-101, I-103, I-104, I-105, I-115, I-121, I-123, I-127, I-128, I-129, I-130, I-131, I-132, I-133, I-134, I-138, I-139, I-140, I-141, I-154, I-155.

In this test, for example, the following compounds from the preparation examples showed good activity of 90 % at an application rate of 5 µg/cm² (= 500 g/ha): I-009, I-075, 1-077, I-089, I-090, I-095, I-097, I-107, I-112, I-118, I-137, I-144.

In this test, for example, the following compounds from the preparation examples showed good activity of 80 % at an application rate of 5 µg/cm² (= 500 g/ha): I-038, I-059, I-071, I-079, I-093, I-145.

### Ctenocephalides felis - oral test

| | |
|---|---|
| Solvent: | dimethyl sulfoxide |

To produce a suitable preparation of active compound, 10 mg of active compound are dissolved in 0.5 mL solvent, and the concentrate is diluted with cattle blood to the desired concentration.

Approximately 20 adult unfed cat fleas (*Ctenocephalides felis*) are placed in flea chambers. The blood chamber, sealed with parafilm on the bottom, are filled with cattle blood supplied with compound solution and placed on the gauze covered top of the flea chamber, so that the fleas are able to suck the blood. The blood chamber is heated to 37 °C whereas the flea chamber is kept at room temperature.

After 2 days mortality in % is determined. 100 % means all the fleas have been killed; 0 % means none of the fleas have been killed.

In this test, for example, the following compounds from the preparation examples showed good activity of 100 % at an application rate of 100 ppm: I-004, I-005, I-006, I-007, I-008, I-009, I-010, I-011, I-012, I-013, I-014, I-015, I-016, I-017, I-018, I-019, I-020, I-021, I-022, I-027, I-028, I-029, I-030, I-035, I-036, I-038, I-039, I-040, I-044, I-045, I-046, I-047, I-048, I-049, I-050, I-051, I-053, I-054, I-055, I-056, I-057, I-066, I-074, I-075, I-076, I-077, I-078, I-079, I-080, I-082, I-088, I-089, I-090, I-091, I-092, I-097, I-099, I-100, I-101, I-103, I-104, I-105, I-117, I-118, I-121.

In this test, for example, the following compounds from the preparation examples showed good activity of 90 % at an application rate of 100 ppm: I-019, I-037, I-042, I-081, I-083, I-093.

In this test, for example, the following compounds from the preparation examples showed good activity of 80 % at an application rate of 100 ppm: I-058.

### Rhipicephalus sanguineus - in-vitro contact tests with adult brown dog ticks

9 mg compound is solved in 1 mL acetone and diluted with acetone to the desired concentration. 250 µL of the test solution is filled in 25 mL glass test tubes and homogeneously distributed on the inner walls by rotation and tilting on a shaking device (2 h at 30 rpm). With a compound concentration of 900 ppm, an inner surface of 44.7 cm² and a homogeneous distribution, a dose of 5 µg/cm² is achieved.

After the solvent has evaporated, each test tube is filled with 5-10 adult brown dog ticks (*Rhipicephalus sanguineus*)*,* closed with a perforated lid and incubated in a lying position at room temperature and relative humidity. After 48 hours efficacy is determined. The ticks are patted on the ground of the tubes and are incubated on a heating plate at 45-50 °C for at most 5 minutes. Immotile or uncoordinated moving ticks, which are not able to escape the heat by climbing upwards, are marked as dead or moribund.

A compound shows a good efficacy against *Rhipicephalus sanguineus,* if at a compound concentration of 5 µg/cm² an efficacy of at least 80 % is monitored. An efficacy of 100 % means all ticks are dead or moribund; 0 % means no ticks are dead or moribund.

In this test, for example, the following compounds from the preparation examples showed good activity of 100 % at an application rate of 5 µg/cm² (= 500 g/ha): I-006, I-007, I-009, I-010, I-012, I-022, I-062, I-088, I-092, I-100, I-101, I-103, I-104, I-115, I-120, I-127, I-130, I-131, I-132, I-134, I-136, I-138, I-139, I-140, I-141, I-155.

In this test, for example, the following compounds from the preparation examples showed good activity of 80 % at an application rate of 5 µg/cm² (= 500 g/ha): I-011, I-032, I-045, I-099, I-126, I-129, I-154.

In this test, for example, the following compounds from the preparation examples showed good activity of 100 % at an application rate of 1 µg/cm² (= 100 g/ha): I-007, I-010, I-012, I-022, 1-088, I-092, I-100, I-101, I-103, I-104, I-115, I-120, I-127, I-130, I-131, I-132, I-134, I-136, I-138, I-140, I-155, I-159.

In this test, for example, the following compounds from the preparation examples showed good activity of 80 % at an application rate of 1 µg/cm² (= 100 g/ha): I-006, I-009, I-093, I-129, I-139, I-141, I-154.

### Diabrotica balteata - spray test

| | |
|---|---|
| Solvent: | 78.0 parts by weight of acetone |
| | 1.5 parts by weight of dimethylformamide |
| Emulsifier: | alkylarylpolyglycol ether |

To produce a suitable preparation of active compound, 1 part by weight of active compound is mixed with the stated amount of solvent, and the concentrate is diluted with water, containing an emulsifier concentration of 1000 ppm, to the desired concentration. Further test concentrations are prepared by dilution with emulsifier containing water.

Soaked wheat seeds (*Triticum aestivum*) are placed in a multiple well plate filled with agar and some water and are incubated for 1 day to germinate (5 seeds per well). The germinated wheat seeds are sprayed with a test solution containing the desired concentration of the active ingredient. Afterwards each unit is infected with 10-20 larvae of the banded cucumber beetle (*Diabrotica balteata*)*.*

After 7 days efficacy in % is determined. 100 % means all the seedlings have grown up like in the untreated, uninfected control; 0 % means none of the seedlings have grown.

In this test, for example, the following compounds from the preparation examples showed good activity of 100 % at an application rate of 500 g/ha (= 160 µg/well): I-001, I-006, I-010, I-011, I-012, I-016, I-018, I-026, I-051, I-055, I-059, I-061, I-062, I-063, I-064.

In this test, for example, the following compounds from the preparation examples showed good activity of 100 % at an application rate of 100 g/ha (= 32 µg/well): I-004, I-006, I-007, I-009, I-010, I-011, I-012, I-016, I-018, I-023, I-024, I-059, I-062, I-063, I-064, I-075, 1-078, I-080, 1-081, I-083, I-086, I-088, I-089, I-090, I-091, I-092, I-095, I-097, I-099, I-100, I-101, I-103, I-104, I-105, I-108, I-113, I-114, I-117, I-118, I-121, I-122, I-123, I-124, I-126, I-127, I-128, I-129, I-130, I-131, I-132, I-133, I-134, I-136, I-137, I-138, I-139, I-140, I-141, I-142, I-143, I-144, I-145, I-146, I-147, I-148, I-149, I-150, I-151, I-152, I-154, I-155, I-156, I-158, I-159, I-161, I-162, I-163, I-164, I-165, I-166, I-167, I-168, I-170, I-172, I-173, I-174, I-175, I-176, I-177, I-178, I-179, I-180, I-182, I-183, I-184, I-185, I-186, I-187, I-190, I-191, I-192, I-193, I-194, I-195, I-197, I-199, I-202, I-203.

In this test, for example, the following compounds from the preparation examples showed good activity of 80 % at an application rate of 100 g/ha (= 32 µg/well): I-055, I-082, I-093, I-094, I-115, I-198.

### Meloidogyne incognita - test

| | |
|---|---|
| Solvent: | 125.0 parts by weight of acetone |

To produce a suitable preparation of active compound, 1 part by weight of active compound is mixed with the stated amount of solvent, and the concentrate is diluted with water to the desired concentration.

Vessels are filled with sand, a solution of the active ingredient, a suspension containing eggs and larvae of the southern root-knot nematode (*Meloidogyne incognita*) and salad seeds. The salad seeds germinate and the seedlings grow. Galls develop in the roots.

After 14 days the nematicidal activity is determined on the basis of the percentage of gall formation. 100 % means no galls were found and 0 % means the number of galls found on the roots of the treated plants was equal to that in untreated control plants.

In this test, for example, the following compounds from the preparation examples showed good activity of 100 % at an application rate of 20 ppm: I-051, I-128, I-145, I-146, I-147.

In this test, for example, the following compounds from the preparation examples showed good activity of 90 % at an application rate of 20 ppm: I-054, I-055, I-064, I-066, I-076, I-077, I-078, I-123, I-130, I-132, I-144.

### Myzus persicae - oral test

| | |
|---|---|
| Solvent: | 100 parts by weight acetone |

To produce a suitable preparation of active compound, 1 part by weight of active compound is mixed with the stated amount of solvent, and the concentrate is diluted with water to the desired concentration.

50 µL compound solution is filled in microtiter plates and 150 µL IPL41 insect medium (33 % + 15 % sugar) is added to obtain a total volume of 200 µL per well. Afterwards the plates are sealed with parafilm through which a mixed population of the green peach aphid (*Myzus persicae*) can suck on the compound preparation.

After 5 days mortality in % is determined. 100 % means all aphids have been killed and 0 % means none of the aphids have been killed.

In this test, for example, the following compounds from the preparation examples showed good activity of 100 % at an application rate of 4 ppm: I-004, I-006, I-007, I-009, I-011, I-012, I-017, I-022, I-023, I-024, I-027, I-051, I-057, I-058, I-059, I-060, I-061, I-063, I-064, I-074, I-076, I-077, I-079, I-088, I-089, I-091, I-092, I-093, I-094, I-095, I-097, I-099, I-100, I-101, I-103, I-104, I-105, I-108, I-117, I-118.

In this test, for example, the following compounds from the preparation examples showed good activity of 90 % at an application rate of 4 ppm: I-005, I-010, I-019, I-020, I-025, I-045, I-053, I-054, I-078, I-080, I-107, I-114, I-116.

### Myzus persicae - spray test

| | |
|---|---|
| Solvent: | 78.0 parts by weight acetone |
| | 1.5 parts by weight dimethylformamide |
| Emulsifier: | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Chinese cabbage (*Brassica pekinensis*) leaf disks infected with all instars of the green peach aphid (*Myzus persicae*)*,* are sprayed with a preparation of the active ingredient of the desired concentration.

After 5 days mortality in % is determined. 100 % means all aphids have been killed and 0 % means none of the aphids have been killed.

In this test, for example, the following compounds from the preparation examples showed good activity of 90 % at an application rate of 500 g/ha: I-012, I-061, I-095.

In this test, for example, the following compounds from the preparation examples showed good activity of 100 % at an application rate of 100 g/ha: I-088, I-097, I-130, I-134, I-155, I-163.

In this test, for example, the following compounds from the preparation examples showed good activity of 90 % at an application rate of 100 g/ha: I-007, I-076, I-077, I-090, I-092, I-101, I-103, I-127, I-129, I-132, I-138, I-140, I-148, I-152, I-153, I-165, 1-178, I-179, I-184, I-187, I-192, I-202, I-205, I-216.

### Nezara viridula - spray test

| | |
|---|---|
| Solvent: | 78.0 parts by weight of acetone |
| | 1.5 parts by weight of dimethylformamide |
| Emulsifier: | alkylarylpolyglycol ether |

To produce a suitable preparation of active compound, 1 part by weight of active compound is mixed with the stated amount of solvent, and the concentrate is diluted with water, containing an emulsifier concentration of 1000 ppm, to the desired concentration. Further test concentrations are prepared by dilution with emulsifier containing water.

Barley plants (*Hordeum vulgare*) infested with larvae of the southern green stink bug (*Nezara viridula*) are sprayed with a test solution containing the desired concentration of the active ingredient.

After 4 days mortality in % is determined. 100 % means all the stink bugs have been killed; 0 % means none of the stink bugs have been killed.

In this test, for example, the following compounds from the preparation examples showed good activity of 100 % at an application rate of 500 g/ha: I-006, I-012, I-017, I-027, I-051, I-055, I-059, I-060, I-061, I-062, I-063, I-064, I-115, I-116, I-117, I-118, I-123, I-126, I-127, I-129, I-130, I-131, I-132, I-133, I-134, I-137, I-139, I-141, I-142, I-143, I-145, I-146, I-148, I-149, I-151, I-152, I-153, I-154, I-155, I-156, I-162, I-163, I-164, I-165, I-166, I-167, I-168, I-169, I-170, I-171, I-176, I-177, I-178, I-179, I-180, I-183, I-184, I-185, I-186, I-187, I-188, I-189, I-190, I-192, I-193, I-194, I-195, I-202, I-203, I-204, I-211.

In this test, for example, the following compounds from the preparation examples showed good activity of 90 % at an application rate of 500 g/ha: I-011, I-026, I-095, I-136, I-140, I-157, I-159, I-173, I-191, I-196.

In this test, for example, the following compounds from the preparation examples showed good activity of 100 % at an application rate of 100 g/ha: I-006, I-007, I-009, I-012, I-027, I-051, I-055, I-059, I-060, I-061, I-062, I-064, I-074, I-076, I-077, I-088, I-092, I-097, I-103.

In this test, for example, the following compounds from the preparation examples showed good activity of 90 % at an application rate of 100 g/ha: I-017, I-019, I-021, I-023, I-084.

### Nilaparvata lugens - spray test

| | |
|---|---|
| Solvent: | 78.0 parts by weight of acetone |
| | 1.5 parts by weight of dimethylformamide |
| Emulsifier: | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Rice plants (*Oryza sativa*) are sprayed with a preparation of the active ingredient of the desired concentration and the plants are infested with the brown planthopper (*Nilaparvata lugens*).

After 4 days mortality in % is determined. 100 % means all planthoppers have been killed and 0 % means none of the planthoppers have been killed.

In this test, for example, the following compounds from the preparation examples showed good activity of 100 % at an application rate of 500 g/ha: I-133, I-134, I-136, I-138, I-139, I-141, I-148, I-149, I-151, I-152, I-153, I-155, I-160, I-163, I-165, I-168, I-169, I-170, I-173, I-174, I-175, I-176, I-177, I-178, I-179, I-180, I-181, I-182, I-183, I-184, I-185, I-186, I-187, I-188, I-189, I-192, I-202, I-211.

In this test, for example, the following compounds from the preparation examples showed good activity of 90 % at an application rate of 500 g/ha: I-006, I-121, I-127, I-128, I-129, I-130, I-131, I-137, I-140.

In this test, for example, the following compounds from the preparation examples showed good activity of 100 % at an application rate of 100 g/ha: I-088.

In this test, for example, the following compounds from the preparation examples showed good activity of 90 % at an application rate of 100 g/ha: I-077.

### Spodoptera frugiperda - spray test

| | |
|---|---|
| Solvent: | 78.0 parts by weight acetone |
| | 1.5 parts by weight dimethylformamide |
| Emulsifier: | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

Maize (*Zea mays*) leaf sections are sprayed with a preparation of the active ingredient of the desired concentration. Once dry, the leaf sections are infested with fall armyworm larvae (*Spodoptera frugiperda*)*.*

After 7 days mortality in % is determined. 100% means all caterpillars have been killed and 0% means none of the caterpillars have been killed.

In this test, for example, the following compounds from the preparation examples showed good activity of 100 % at an application rate of 500 g/ha: I-001, I-006, I-010, I-011, I-012, I-014, I-015, I-016, I-017, I-029, I-036, I-046, I-051, I-059, I-062, I-063, I-064.

In this test, for example, the following compounds from the preparation examples showed good activity of 83 % at an application rate of 500 g/ha: I-047, I-048.

In this test, for example, the following compounds from the preparation examples showed good activity of 100 % at an application rate of 100 g/ha: I-006, I-007, I-009, I-010, I-011, I-012, I-014, I-015, I-016, I-022, I-023, I-024, I-029, I-036, I-063, I-064, I-076, I-078, I-079, I-088, I-089, I-090, I-091, I-092, I-094, I-097, I-099, I-100, I-101, I-103, I-104, I-105, I-108, I-114, I-115, I-117, I-118, I-121, I-123, I-124, I-126, I-127, I-129, I-130, I-131, I-132, I-133, I-134, I-136, I-137, I-138, I-139, I-140, I-141, I-142, I-143, I-145, I-146, I-148, I-149, I-150, I-151, I-152, I-154, I-155, I-156, I-158, I-159, I-162, I-163, I-164, I-165, I-166, I-167, I-168, I-170, I-171, I-172, I-173, I-175, I-176, I-177, I-178, I-179, I-180, I-182, I-183, I-186, I-190, I-192, I-193, I-194, I-202, I-203, I-204, I-205, I-206, I-207, I-208, I-209, I-210, I-212, I-216.

In this test, for example, the following compounds from the preparation examples showed good activity of 83 % at an application rate of 100 g/ha: I-093.

### Tetranychus urticae - spray test OP-resistant

| | |
|---|---|
| Solvent: | 78.0 parts by weight acetone |
| | 1.5 parts by weight dimethylformamide |
| Emulsifier: | alkylarylpolyglycol ether |

To produce a suitable preparation of active compound, 1 part by weight of active compound is mixed with the stated amount of solvents and is diluted with water, containing an emulsifier concentration of 1000 ppm, to the desired concentration. Further test concentrations are prepared by dilution with emulsifier containing water.

French bean (*Phaseolus vulgaris*) leaf disks infected with all instars of the two spotted spidermite (*Tetranychus urticae*), are sprayed with a preparation of the active ingredient of the desired concentration.

After 6 days mortality in % is determined. 100 % means all spider mites have been killed and 0 % means none of the spider mites have been killed.

In this test, for example, the following compounds from the preparation examples showed good activity of 100 % at an application rate of 500 g/ha: I-064.

In this test, for example, the following compounds from the preparation examples showed good activity of 90 % at an application rate of 500 g/ha: I-003, I-012, I-016.

### Spodoptera frugiperda - spray test

| | |
|---|---|
| Solvent: | 14 parts by weight of dimethylformamide |
| Emulsifier: | alkylaryl polyglycol ether |

To produce a suitable preparation of active compound, 1 part by weight of active compound is mixed with the stated amount of solvent and is diluted with water, containing an emulsifier concentration of 1000 ppm, to the desired concentration. Further test concentrations are prepared by dilution with emulsifier containing water. Ammonium salt and/or penetration enhancer in a dosage of 1000 ppm are added to the desired concentration if necessary.

Cotton leaves (*Gossypium hirsutum*) are treated by being sprayed with the preparation of the active compound of the desired concentration and are infested with caterpillars of the fall army worm (*Spodoptera frugiperda*)*.*

After 7 days mortality in % is determined. 100 % means all the caterpillars have been killed and 0 % means none of the caterpillars have been killed.

In this test, for example, the following compounds from the preparation examples showed good activity of 100 % at an application rate of 20 ppm: I-006, I-011, I-012, I-013, I-015, I-016, I-018, I-029, I-062, I-063, I-064, I-097, I-099, I-101, I-103, I-104, I-105, I-114, I-117, I-118, I-129, I-130, I-132, I-136, I-139, I-140, I-142, I-143, I-152, I-154, I-155.

In this test, for example, the following compounds from the preparation examples showed good activity of 80 % at an application rate of 20 ppm: I-010, I-092.

### Aedes aegypti test (AEDSAE surface treatment & contact assay)

| | |
|---|---|
| Solvent: | Aceton + 2000 ppm rapeseed oil methyl ester (RME) |

In order to produce a sufficient, active ingredient containing solution it is necessary to solve the test compound in the solvent-mix (acetone at 2 mg/ml / RME 2000 ppm). This solution is pipetted onto a glazed tile and after evaporation of the acetone, adult mosquitoes of the species *Aedes aegypti* strain MONHEIM are placed onto the dried surface. The exposure time is 30 minutes. Mortality in percent (%) is determined 24 hours after contact of the insects to the treated surface. 100% mortality means that all tested insects are dead, whereas 0% means that no insect died.

The following examples showed in this test efficacy of 90-100% at a surface concentration of 20 mg/m²: I-006, I-088, I-138.

The following examples showed in this test efficacy of 90-100% at a surface concentration of 4 mg/m²: I-006, I-088, I-138.

### Anopheles funestus test (ANPHFU surface treatment & contact assay)

| | |
|---|---|
| Solvent: | Aceton + 2000 ppm rapeseed oil methyl ester (RME) |

In order to produce a sufficient, active ingredient containing solution it is necessary to solve the test compound in the solvent-mix (acetone at 2 mg/ml / RME 2000 ppm). This solution is pipetted onto a glazed tile and after evaporation of the acetone, adult mosquitoes of the species Anopheles funestus strain FUMOZ-R (Hunt et al., Med. Vet. Entomol. 2005 Sep; 19(3): 271-275) are placed onto the dried surface. The exposure time is 30 minutes.

Mortality in percent (%) is determined 24 hours after contact of the insects to the treated surface. 100% mortality means that all tested insects are dead, whereas 0% means that no insect died.

The following examples showed in this test efficacy of 90-100% at a surface concentration of 20 mg/m²: I-088, I-138.

The following examples showed in this test efficacy of 90-100% at a surface concentration of 4 mg/m²: I-088.

### Musca domestica test (MUSCDO surface treatment & contact assay)

Solvent: Aceton + 2000 ppm rapeseed oil methyl ester (RME)

In order to produce a sufficient, active ingredient containing solution it is necessary to solve the test compound in the solvent-mix (acetone at 2 mg/ml / RME 2000 ppm). This solution is pipetted onto a glazed tile and after evaporation of the acetone, adult flies of the species *Musca domestica* strain WHO-N are placed onto the dried surface. The exposure time is 30 minutes. Mortality in percent (%) is determined 24 hours after contact of the insects to the treated surface. 100% mortality means that all tested insects are dead, whereas 0% means that no insect died.

The following examples showed in this test efficacy of 90-100% at a surface concentration of 20 mg/m²: I-006, I-088, I-138.

The following examples showed in this test efficacy of 90-100% at a surface concentration of 4 mg/m²: I-006, I-088, I-138.

### Blattella germanica test (BLTTGE surface treatment & contact assay)

| | |
|---|---|
| Solvent: | Aceton + 2000 ppm rapeseed oil methyl ester (RME) |

In order to produce a sufficient, active ingredient containing solution it is necessary to solve the test compound in the solvent-mix (acetone at 2 mg/ml / RME 2000 ppm). This solution is pipetted onto a glazed tile and after evaporation of the acetone, adult animals of the species Blattella germanica strain PAULINIA are placed onto the dried surface. The exposure time is 30 minutes.

Mortality in percent (%) is determined 24 hours after contact of the insects to the treated surface. 100% mortality means that all tested insects are dead, whereas 0% means that no insect died.

The following examples showed in this test efficacy of 90-100% at a surface concentration of 20 mg/m²: I-088, I-138.

The following examples showed in this test efficacy of 90-100% at a surface concentration of 4 mg/m²: I-088, I-138.

## Claims

1. Compound of the formula (I) 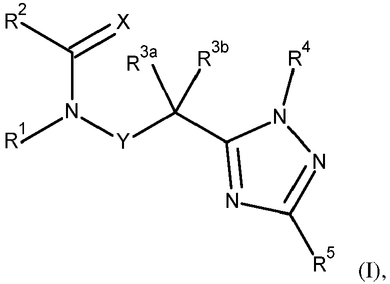 in which
X is O;
Y is a direct bond;
R¹ is hydrogen;
R² is phenyl, pyridine, pyrimidine, pyrazine or pyridazine, wherein the phenyl, pyridine, pyrimidine, pyrazine or pyridazine is substituted with a total of one to three substituents, provided the substituent(s) are not on either carbon adjacent to the carbon bonded to the C=X group and at least one and up to two substituent(s) are independently selected from group A consisting of
C₁-C₄-alkyl substituted by one to two substituents selected from the group consisting of -CN; and C₃-C₆cycloalkyl, wherein the C₃-C₆cycloalkyl is optionally substituted with halogen, -CN, C₁-C₄-alkyl or C₁-C₄-haloalkyl; C₁-C₄alkylthio, C₁-C₄alkylsulfinyl, C₁-C₄alkylsulfonyl, C₃-C₆cycloalkylsulfanyl, C₃-C₆cycloalkylsulfinyl, C₃-C₆cycloalkylsulfonyl, C₁-C₄haloalkylsulfinyl, and C₁-C₄haloalkylsulfonyl;
phenylsulfanyl wherein the phenyl is optionally substituted with one to two substituents selected from the group consisting of halogen, -CN, C₁-C₃alkyl, C₁-C₃haloalkyl and C₁-C₃haloalkoxy;
phenylsulfinyl wherein the phenyl is optionally substituted with one to two substituents selected from the group consisting of halogen, -CN, C₁-C₃alkyl, C₁-C₃haloalkyl and C₁-C₃haloalkoxy;
phenylsulfonyl wherein the phenyl is optionally substituted with one to two substituents selected from the group consisting of halogen, -CN, C₁-C₃alkyl, C₁-C₃haloalkyl and C₁-C₃haloalkoxy;
and phenyl and 5- to 6-membered heteroaryl, wherein the phenyl or 5- to 6-membered heteroaryl is optionally substituted with one to two substituents selected from the group consisting of halogen, -CN, C₁-C₃alkyl, C₁-C₃haloalkyl and C₁-C₃haloalkoxy;
the other one to two optional substituent(s) are each independently selected from group B consisting of
halogen, -CN, -NO₂, C₁-C₆alkyl, C₃-C₆cycloalkyl, C₁-C₄haloalkyl, C₁-C₄alkoxy, C₁-C₄haloalkoxy, C₁-C₄alkylthio, C₁-C₄alkylsulfinyl, C₁-C₄alkylsulfonyl, C₁-C₄haloalkylthio, C₁-C₄haloalkylsulfinyl, and C₁-C₄haloalkylsulfonyl;
or
R² is thiophene, furane, pyrazole, thiazole, isothiazole, oxazole or isoxazole each of which is optionally substituted by one to three substituents independently selected from the group consisting of
halogen, hydroxy, -CN, -NO₂; C₁-C₆alkyl optionally substituted by -CN; C₃-C₆cycloalkyl, optionally substituted by halogen, -CN, C₁-C₄-alkyl or C₁-C₄-haloalkyl; C₁-C₄haloalkyl, C₁-C₄alkoxy, C₁-C₄haloalkoxy, C₁-C₄alkylthio, C₁-C₄alkylsulfinyl, C₁-C₄alkylsulfonyl, C₃-C₆cycloalkylsulfanyl, C₃-C₆cycloalkylsulfinyl, C₃-C₆cycloalkylsulfonyl, C₁-C₄haloalkylthio, C₁-C₄haloalkylsulfinyl, C₁-C₄haloalkylsulfonyl;
and phenyl and 5- to 6-membered heteroaryl, wherein the phenyl or 5- to 6-membered heteroaryl is optionally substituted with one to two substituents selected from the group consisting of halogen, -CN, C₁-C₃alkyl and C₁-C₃haloalkyl.
R^{3a} is H;
R^{3b} is methyl;
R⁴ is pyrimidin-2-yl, 5-chloropyrimidin-2-yl, 5-chloropyridin-2-yl, 5-cyanopyridin-2-yl, 5-cyano-1,3-thiazol-2-yl, 5-carboxypyridin-2-yl, 5-(methoxycarbonyl)pyridin-2-yl, 5-(dimethylaminocarbonyl)pyridin-2-yl, 5-[[ethyl(methyl)amino]carbonyl]pyridin-2-yl, 5-[[cyclopropyl(methyl)amino]carbonyl]pyridin-2-yl, 5-[[cyclopropylmethyl(methyl)amino]carbonyl]pyridin-2-yl, 5-[(cyclopropylcarbonyl)(methyl)amino]-pyridin-2-yl, 5-(pyrrolidin-1-ylcarbonyl)pyridin-2-yl, 5-(morpholin-4-ylcarbonyl)pyridin-2-yl, 5-(morpholin-4-carbonyl)thiazol-2-yl], 5-[[rac-(2R,6R)-2,6-dimethylmorpholin-4-yl]carbonyl]pyridin-2-yl, or 5-[[(2R,6S)-2,6-dimethylmorpholin-4-yl]carbonyl]pyridin-2-yl;
R⁵ is -NHCO-tert-butyloxy, -NH₂, -N-methyl-CO-tert-butyloxy, -NHCO-methoxy, -NHCO-methyl, -NHCO-cyclopropyl, -NHCO-(1-chloro-cyclopropyl), -NHCO-(2-fluoro-cyclopropyl), -NHCO-(2,2-difluoro-cyclopropyl), -NHCO-(2,2,2-trifluoroethyl), -NHCO-(1-chloroethyl), -NHSO₂-trifluoromethyl, -N(SO₂-methyl)₂, methylamino, dimethylamino, acetyl(methyl)amino, prop-2-enoylamino, methoxy(methyl)amino, pyrrolidine-1-yl, piperidin-1-yl, or morpholin-4-yl.

2. Compound according to Claim 1, in which the structural elements X, Y, R¹, R^{3a}, R^{3b}, R⁴ and R⁵ have the meanings given in Claim 1 and
R² is phenyl or pyridine, wherein the phenyl or pyridine is substituted with a total of one to three substituents, provided the substituent(s) are not on either carbon adjacent to the carbon bonded to the C=X group and one substituent is independently selected from group A consisting of
1-cyano-1-methyl-ethyl, cyclopropyl, 1-cyanocyclopropyl, 1-(trifluoromethyl)cyclopropyl, methylthio, methylsulfinyl, methylsulfonyl, ethylthio, ethylsulfinyl, ethylsulfonyl, isopropylthio, isopropylsulfinyl, isopropylsulfonyl, cyclopropylthio, cyclopropylsulfinyl, cyclopropylsulfonyl, difluoromethylsulfinyl, difluoromethylsulfonyl, trifluoromethylsulfinyl, trifluoromethylsulfonyl;
phenylsulfonyl wherein the phenyl is optionally substituted with one two substituents selected from the group consisting of fluorine, chlorine, bromine, - CN, difluoromethyl, trifluoromethyl and trifluoromethoxy;
and phenyl and 5-membered heteroaryl wherein the phenyl or 5-membered heteroaryl is optionally substituted with one two substituents selected from the group consisting of fluorine, chlorine bromine, -CN, difluoromethyl, trifluoromethyl and trifluoromethoxy;
the other one to two optional substituent(s) are each independently selected from group B consisting of
fluorine, chlorine, bromine, iodine, -CN, -NO₂, methyl, cyclopropyl, difluoromethyl, trifluoromethyl, methoxy, difluoromethoxy, trifluoromethoxy, methylthio, methylsulfinyl, methylsulfonyl, difluoromethylthio, difluoromethylsulfinyl, difluoromethylsulfonyl, trifluoromethylthio, trifluoromethylsulfinyl, trifluoromethylsulfonyl;
or
R² is thiophene, furane, pyrazole, thiazole, oxazole or isoxazole each of which is optionally substituted by one to three substituents independently selected from the group consisting of of
fluorine, chlorine, bromine, iodine, -CN, -NO₂, methyl, cyclopropyl, difluoromethyl, trifluoromethyl, methoxy, difluoromethoxy, trifluoromethoxy, methylthio, methylsulfinyl, methylsulfonyl, difluoromethylthio, difluoromethylsulfinyl, difluoromethylsulfonyl, trifluoromethylthio, trifluoromethylsulfinyl, trifluoromethylsulfonyl.

3. Compound according to Claim 1 or 2, in which
X is O;
Y is a direct bond;
R¹ is hydrogen;
R² is 3-chloro-5-(methylsulfonyl)phenyl, 3-chloro-5-(difluoromethylsulfonyl)phenyl, 3-chloro-5-(trifluoromethylsulfonyl)phenyl, 3-methylsulfonyl-5-(trifluoromethyl)phenyl, 3-ethylsulfonyl-5-(trifluoromethyl)phenyl, 3-methylsulfonyl-5-(trifluoromethoxy)phenyl, 3-ethylsulfonyl-5-(trifluoromethoxy)phenyl, 3-isopropylsulfonyl-5-(trifluoromethoxy)phenyl, 3-cyclopropylsulfonyl-5-(trifluoromethoxy)phenyl, 3-(difluoromethylsulfonyl)-5-(trifluoromethoxy)phenyl, 3-(4-chlorophenyl)sulfonyl-5-(trifluoromethoxy)phenyl, 3-cyclopropyl-5-(trifluoromethoxy)phenyl, 3-cyclopropyl-5-(trifluoromethylsulfonyl)phenyl, 3-bromo-5-[1-(trifluoromethyl)cyclopropyl]phenyl, 3-chloro-5-(1-cyano-1-methyl-ethyl)phenyl, , or 1-methyl-5-(trifluoromethyl)-pyrazol-3-yl;
R^{3a} is H;
R^{3b} is methyl;
R⁴ is pyrimidin-2-yl, 5-chloropyrimidin-2-yl, 5-chloropyridin-2-yl, 5-cyanopyridin-2-yl, 5-cyano-1,3-thiazol-2-yl, 5-carboxypyridin-2-yl, 5-(methoxycarbonyl)pyridin-2-yl, 5-(dimethylaminocarbonyl)pyridin-2-yl, 5-[[ethyl(methyl)amino]carbonyl]pyridin-2-yl, 5-[[cyclopropyl(methyl)amino]carbonyl]pyridin-2-yl, 5-[[cyclopropylmethyl(methyl)amino]carbonyl]pyridin-2-yl, 5-[(cyclopropylcarbonyl)(methyl)amino]-pyridin-2-yl, 5-(pyrrolidin-1-ylcarbonyl)pyridin-2-yl, 5-(morpholin-4-ylcarbonyl)pyridin-2-yl, 5-(morpholin-4-carbonyl)thiazol-2-yl], 5-[[rac-(2R,6R)-2,6-dimethylmorpholin-4-yl]carbonyl]pyridin-2-yl, or 5-[[(2R,6S)-2,6-dimethylmorpholin-4-yl]carbonyl]pyridin-2-yl;
R⁵ is -NHCO-tert-butyloxy, -NH₂, -N-methyl-CO-tert-butyloxy, -NHCO-methoxy, -NHCO-methyl, -NHCO-cyclopropyl, -NHCO-(1-chloro-cyclopropyl), -NHCO-(2-fluoro-cyclopropyl), -NHCO-(2,2-difluoro-cyclopropyl), -NHCO-(2,2,2-trifluoroethyl), -NHCO-(1-chloroethyl), -NHSO₂-trifluoromethyl, -N(SO₂-methyl)₂, methylamino, dimethylamino, acetyl(methyl)amino, prop-2-enoylamino, methoxy(methyl)amino, pyrrolidine-1-yl, piperidin-1-yl, or morpholin-4-yl.

4. Compound of the formula **(a)** 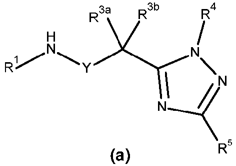 wherein R¹ is hydrogen, R^{3b} is methyl, R^{3a} is hydrogen and Y is a direct bond and wherein R⁴ and R⁵ have the meanings given in Claim 3.

5. Compound of the formula **(b-1)** 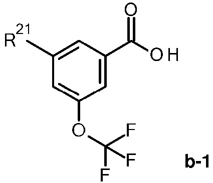 wherein
R²¹ is difluoromethylsulfanyl, difluormethylsulfinyl, or difluoromethylsulfonyl.

6. Formulation, especially agrochemical formulation, comprising at least one compound of the formula (I) according to any of Claims 1 to 3.

7. Formulation according to Claim 6, further comprising at least one extender and/or at least one surface-active substance.

8. Formulation according to Claim 6 or 7, **characterized in that** the compound of the formula (I) is in a mixture with at least one further active compound.

9. Method for controlling pests, especially animal pests, **characterized in that** a compound of the formula (I) according to any of Claims 1 to 3 or a formulation according to any of Claims 6 to 8 is allowed to act on the pests and/or their habitat, whereas methods for treatment of the human or animal body by surgery or therapy and diagnostic methods practised on the human or animal body are excluded.

10. Method according to Claim 9, **characterized in that** the pest is an animal pest and comprises an insect, an arachnid or a nematode, or **in that** the pest is an insect, an arachnid or a nematode, whereas methods for treatment of the human or animal body by surgery or therapy and diagnostic methods practised on the human or animal body are excluded.

11. Use of a compound of the formula (I) according to any of Claims 1 to 5 or of a formulation according to any of Claims 6 to 8 for controlling animal pests, whereas the use of methods for treatment of the human or animal body by surgery or therapy and diagnostic methods practised on the human or animal body is excluded.

12. Use according to Claim 11, **characterized in that** the animal pest comprises an insect, an arachnid or a nematode, or **in that** the animal pest is an insect, an arachnid or a nematode, whereas the use of methods for treatment of the human or animal body by surgery or therapy and diagnostic methods practised on the human or animal body is excluded.

13. Use according to Claim 11 or 12 in crop protection, whereas the use of methods for treatment of the human or animal body by surgery or therapy and diagnostic methods practised on the human or animal body is excluded.

14. Use according to Claim 11 or 12 in the field of animal health, whereas the use of methods for treatment of the human or animal body by surgery or therapy and diagnostic methods practised on the human or animal body is excluded.

15. Method for protecting seed or a germinating plant from pests, especially animal pests, comprising a method step in which the seed is contacted with a compound of the formula (I) according to any of Claims 1 to 3 or with a formulation according to any of Claims 6 to 8, whereas methods for treatment of the human or animal body by surgery or therapy and diagnostic methods practised on the human or animal body are excluded.

## Patentansprüche

1. Verbindung mit der Formel (I): 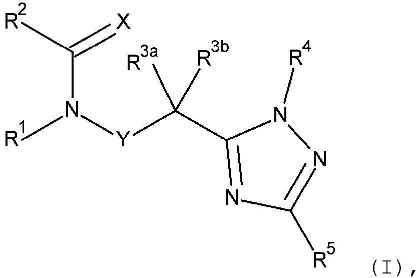 in welcher:
X O,
Y eine direkte Bindung und
R¹ Wasserstoff bedeutet,
R² Phenyl, Pyridin, Pyrimidin, Pyrazin oder Pyridazin bedeutet, wobei das Phenyl, Pyridin, Pyrimidin, Pyrazin oder Pyridazin durch insgesamt ein bis drei Substituenten substituiert ist, unter der Voraussetzung, dass der/die Substituent/en sich nicht an jedem Kohlenstoffatom befindet/befinden, das dem Kohlenstoffatom benachbart ist, an welches die C=X-Gruppe gebunden ist, und mindestens ein und bis zu zwei Substituenten unabhängig voneinander aus einer Gruppe A ausgewählt sind, die aus:
C₁-C₄-Alkyl, das durch ein bis zwei Substituenten substituiert ist, die aus der Gruppe ausgewählt sind, die aus -CN und C₃-C₆Cycloalkyl besteht, wobei das C₃-C₆Cycloalkyl wahlweise durch Halogen, -CN, C₁-C₄-Alkyl oder C₁-C₄-Halogenalkyl substituiert ist, C₁-C₄Alkylthio, C₁-C₄Alkylsulfinyl, C₁-C₄Alkylsulfonyl, C₃-C₆Cycloalkylsulfanyl, C₃-C₆Cycloalkylsulfinyl, C₃-C₆Cycloalkylsulfonyl, C₁-C₄Halogenalkylsulfinyl und C₁-C₄Halogenalkylsulfonyl,
Phenylsulfanyl, wobei das Phenyl wahlweise durch ein bis zwei Substituenten substituiert ist, die aus der Gruppe ausgewählt sind, die aus Halogen, -CN, C₁-C₃Alkyl, C₁-C₃Halogenalkyl und C₁-C₃Halogenalkoxy besteht,
Phenylsulfinyl, wobei das Phenyl wahlweise durch ein bis zwei Substituenten substituiert ist, die aus der Gruppe ausgewählt sind, die aus Halogen, -CN, C₁-C₃Alkyl, C₁-C₃Halogenalkyl und C₁-C₃Halogenalkoxy besteht,
Phenylsulfonyl, wobei das Phenyl wahlweise durch ein bis zwei Substituenten substituiert ist, die aus der Gruppe ausgewählt sind, die aus Halogen, -CN, C₁-C₃Alkyl, C₁-C₃Halogenalkyl und C₁-C₃Halogenalkoxy besteht, und
Phenyl und 5- bis 6-gliedrigem Heteroaryl, wobei das Phenyl oder 5- bis 6-gliedrige Heteroaryl wahlweise durch ein bis zwei Substituenten substituiert ist, die aus der Gruppe ausgewählt sind, die aus Halogen, -CN, C₁-C₃Alkyl, C₁-C₃Halogenalkyl und C₁-C₃Halogenalkoxy besteht, besteht, wobei
der/die andere/n eine bis zwei optionale/n Substituent/en jeweils unabhängig voneinander aus einer Gruppe B ausgewählt ist/sind, die aus:
Halogen, -CN, -NO₂, C₁-C₆Alkyl, C₃-C₆Cycloalkyl, C₁-C₄Halogenalkyl, C₁-C₄Alkoxy, C₁-C₄Halogenalkoxy, C₁-C₄Alkylthio,C₁-C₄Alkylsulfinyl, C₁-C₄Alkylsulfonyl,C₁-C₄Halogenalkylthio,C₁-C₄Halogenalkylsulfinyl und C₁-C₄Halogenalkylsulfonyl besteht,
oder
R² Thiophen, Furan, Pyrazol, Thiazol, Isothiazol, Oxazol oder Isoxazol bedeutet, wobei jedes davon wahlweise durch ein bis drei Substituenten substituiert ist, die unabhängig voneinander aus der Gruppe ausgewählt sind, die aus:
Halogen, Hydroxy, -CN, -NO₂, C₁-C₆Alkyl, das wahlweise durch -CN substituiert ist,
C₃-C₆Cycloalkyl, das wahlweise durch Halogen, -CN, C₁-C₄-Alkyl oder C₁-C₄-Halogenalkyl substituiert ist, C₁-C₄Halogenalkyl,
C₁-C₄Alkoxy, C₁-C₄Halogenalkoxy, C₁-C₄Alkylthio, C₁-C₄Alkylsulfinyl, C₁-C₄Alkylsulfonyl,
C₃-C₆Cycloalkylsulfanyl,
C₃-C₆Cycloalkylsulfinyl,
C₃-C₆Cycloalkylsulfonyl, C₁-C₄Halogenalkylthio, C₁-C₄Halogenalkylsulfinyl,
C₁-C₄Halogenalkylsulfonyl und
Phenyl und 5- bis 6-gliedrigem Heteroaryl, wobei das Phenyl oder 5- bis 6-gliedrige Heteroaryl wahlweise durch ein bis zwei Substituenten substituiert ist, die aus der Gruppe ausgewählt sind, die aus Halogen, -CN, C₁-C₃Alkyl und C₁-C₃Halogenalkyl besteht, besteht,
R^{3a} H bedeutet,
R^{3b} Methyl bedeutet,
R⁴ Pyrimidin-2-yl, 5-Chlorpyrimidin-2-yl, 5-Chlorpyridin-2-yl, 5-Cyanopyridin-2-yl, 5-Cyano-1,3-thiazol-2-yl, 5-Carboxypyridin-2-yl, 5-(Methoxycarbonyl)pyridin-2-yl, 5-(Dimethylaminocarbonyl)pyridin-2-yl, 5-[[Ethyl(methyl)amino]carbonyl]pyridin-2-yl, 5-[[Cyclopropyl(methyl)amino]carbonyl]pyridin-2-yl, 5-[[Cyclopropylmethyl(methyl)amino]carbonyl]-pyridin-2-yl,
5-[(Cyclopropylcarbonyl) (methyl)amino]-pyridin-2-yl, 5-(Pyrrolidin-1-ylcarbonyl)pyridin-2-yl, 5-(Morpholin-4-ylcarbonyl)pyridin-2-yl, 5-(Morpholin-4-carbonyl)thiazol-2-yl], 5-[[rac-(2R,6R)-2,6-Dimethylmorpholin-4-yl]carbonyl]pyridin-2-yl oder 5-[[(2R,6S)-2,6-Dimethylmorpholin-4-yl]carbonyl]pyridin-2-yl bedeutet, und
R⁵ -NHCO-*tert*.-Butyloxy, -NH₂, -N-Methyl-CO-*tert*.-Butyloxy, -NHCO-Methoxy, -NHCO-Methyl, -NHCO-Cyclopropyl, -NHCO-(1-Chlorcyclopropyl),
-NHCO-(2-Fluorcyclopropyl), -NHCO-(2,2-Difluorcyclopropyl), -NHCO-(2,2,2-Trifluorethyl), -NHCO-(1-Chlorethyl), -NHSO₂-Trifluormethyl, -N(SO₂-Methyl)₂, Methylamino, Dimethylamino, Acetyl(methyl)amino, Prop-2-enoylamino, Methoxy(methyl)amino, Pyrrolidin-1-yl, Piperidin-1-yl oder Morpholin-4-yl bedeutet,

2. Verbindung nach Anspruch 1, in welcher die Strukturelemente X, Y, R¹, R^{3a}, R^{3b}, R⁴ und R⁵ die in Anspruch 1 angegebene Bedeutung besitzen und:
R² Phenyl oder Pyridin bedeutet, wobei das Phenyl oder Pyridin durch insgesamt ein bis drei Substituenten substituiert ist, unter der Voraussetzung, dass der/die Substituent/en sich nicht an jedem Kohlenstoffatom befindet/befinden, das dem Kohlenstoffatom benachbart ist, an welches die C=X-Gruppe gebunden ist, und ein Substituent unabhängig aus Gruppe A ausgewählt ist, die aus:
1-Cyano-1-methylethyl, Cyclopropyl, 1-Cyanocyclopropyl, 1-(Trifluormethyl)cyclopropyl, Methylthio, Methylsulfinyl, Methylsulfonyl, Ethylthio, Ethylsulfinyl, Ethylsulfonyl, Isopropylthio, Isopropylsulfinyl, Isopropylsulfonyl, Cyclopropylthio, Cyclopropylsulfinyl, Cyclopropylsulfonyl, Difluormethylsulfinyl, Difluormethylsulfonyl, Trifluormethylsulfinyl, Trifluormethylsulfonyl,
Phenylsulfonyl, wobei das Phenyl wahlweise durch ein bis zwei Substituenten substituiert ist, die aus der Gruppe ausgewählt sind, die aus Fluor, Chlor, Brom, -CN, Difluormethyl, Trifluormethyl und Trifluormethoxy besteht, und
Phenyl und 5-gliedrigem Heteroaryl, wobei das Phenyl oder 5-gliedrige Heteroaryl wahlweise durch ein bis zwei Substituenten substituiert ist, die aus der Gruppe ausgewählt sind, die aus Fluor, Chlor, Brom, -CN, Difluormethyl, Trifluormethyl und Trifluormethoxy besteht, besteht, wobei
der/die andere/n optionale/n Substituent/en jeweils unabhängig voneinander aus Gruppe B ausgewählt ist/sind, die aus:
Fluor, Chlor, Brom, Iod, -CN, -NO₂, Methyl, Cyclopropyl, Difluormethyl, Trifluormethyl, Methoxy, Difluormethoxy, Trifluormethoxy, Methylthio, Methylsulfinyl, Methylsulfonyl, Difluormethylthio, Difluormethylsulfinyl, Difluormethylsulfonyl, Trifluormethylthio, Trifluormethylsulfinyl und Trifluormethylsulfonyl besteht, oder
R² Thiophen, Furan, Pyrazol, Thiazol, Oxazol oder Isoxazol bedeutet, wobei jedes davon wahlweise durch ein bis drei Substituenten substituiert ist, die unabhängig voneinander aus der Gruppe ausgewählt sind, die aus:
Fluor, Chlor, Brom, Iod, -CN, -NO₂, Methyl, Cyclopropyl, Difluormethyl, Trifluormethyl, Methoxy, Difluormethoxy, Trifluormethoxy, Methylthio, Methylsulfinyl, Methylsulfonyl, Difluormethylthio, Difluormethylsulfinyl, Difluormethylsulfonyl, Trifluormethylthio, Trifluormethylsulfinyl und Trifluormethylsulfonyl besteht.

3. Verbindung nach Anspruch 1 oder 2, in welcher:
X O bedeutet,
Y eine direkte Bindung bedeutet,
R¹ Wasserstoff bedeutet,
R² 3-Chlor-5-(methylsulfonyl)phenyl, 3-Chlor-5-(difluormethylsulfonyl)phenyl, 3-Chlor-5-(trifluormethylsulfonyl)phenyl, 3-Methylsulfonyl-5-(trifluormethyl)phenyl, 3-Ethylsulfonyl-5-(trifluormethyl)phenyl, 3-Methylsulfonyl-5-(trifluormethoxy)phenyl, 3-Ethylsulfonyl-5-(trifluormethoxy)phenyl, 3-Isopropylsulfonyl-5-(trifluormethoxy)phenyl, 3-Cyclopropylsulfonyl-5-(trifluormethoxy)phenyl, 3-(Difluormethylsulfonyl)-5-(trifluormethoxy)phenyl, 3-(4-Chlorphenyl)sulfonyl-5-(trifluormethoxy)phenyl, 3-Cyclopropyl-5-(trifluormethoxy)phenyl, 3-Cyclopropyl-5-(trifluormethylsulfonyl)phenyl, 3-Brom-5-[1-(trifluormethyl)cyclopropyl]phenyl, 3-Chlor-5-(1-cyano-1-methyl-ethyl)phenyl oder 1-Methyl-5-(trifluormethyl)-pyrazol-3-yl bedeutet,
R^{3a} H bedeutet,
R^{3b} Methyl bedeutet,
R⁴ Pyrimidin-2-yl, 5-Chlorpyrimidin-2-yl, 5-Chlorpyridin-2-yl, 5-Cyanopyridin-2-yl, 5-Cyano-1,3-thiazol-2-yl, 5-Carboxypyridin-2-yl, 5-(Methoxycarbonyl)pyridin-2-yl, 5-(Dimethylaminocarbonyl)pyridin-2-yl, 5-[[Ethyl(methyl)amino]carbonyl]pyridin-2-yl, 5-[[Cyclopropyl(methyl)amino]carbonyl]pyridin-2-yl, 5-[[Cyclopropylmethyl(methyl)amino]carbonyl]-pyridin-2-yl, 5-[(Cyclopropylcarbonyl) (methyl)amino]-pyridin-2-yl, 5-(Pyrrolidin-1-ylcarbonyl)pyridin-2-yl, 5-(Morpholin-4-ylcarbonyl)pyridin-2-yl, 5-(Morpholin-4-carbonyl)thiazol-2-yl], 5-[[rac-(2R,6R)-2,6-Dimethylmorpholin-4-yl]carbonyl]pyridin-2-yl oder 5-[[(2R,6S)-2,6-Dimethylmorpholin-4-yl]carbonyl]pyridin-2-yl bedeutet und
R⁵ -NHCO-*tert*.-Butyloxy, -NH₂, -N-Methyl-CO-*tert*.-butyloxy, - NHCO-Methoxy, -NHCO-Methyl, -NHCO-Cyclopropyl, -NHCO-(1-Chlorcyclopropyl), -NHCO-(2-Fluorcyclopropyl), -NHCO-(2,2-Difluor-cyclopropyl), -NHCO-(2,2,2-Trifluorethyl), -NHCO-(1-Chlorethyl), -NHSO₂-Trifluormethyl, -N(SO₂-Methyl)₂, Methylamino, Dimethylamino, Acetyl(methyl)amino, Prop-2-enoylamino, Methoxy(methyl)amino, Pyrrolidin-1-yl, Piperidin-1-yl oder Morpholin-4-yl bedeutet.

4. Verbindung mit der Formel **(a):** 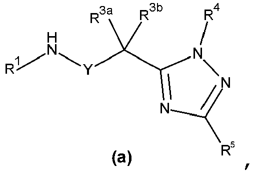 wobei R¹ Wasserstoff, R^{3b} Methyl, R^{3a} Wasserstoff und Y eine direkte Bindung bedeutet und wobei R⁴ und R⁵ die in Anspruch 3 angegebene Bedeutung besitzen.

5. Verbindung mit der Formel **(b-1):** 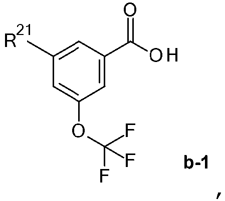 wobei:
R²¹ Difluormethylsulfanyl, Difluormethylsulfinyl oder Difluormethylsulfonyl bedeutet.

6. Formulierung, insbesondere agrochemische Formulierung, die mindestens eine Verbindung mit der Formel (I) nach einem der Ansprüche 1 bis 3 umfasst.

7. Formulierung nach Anspruch 6, die ferner mindestens ein Verdünnungsmittel und/oder mindestens eine oberflächenaktive Substanz umfasst.

8. Formulierung nach Anspruch 6 oder 7, **dadurch gekennzeichnet, dass** die Verbindung mit der Formel (I) sich in einem Gemisch mit mindestens einem weiteren Wirkstoff befindet.

9. Verfahren zur Bekämpfung von Schädlingen, insbesondere tierischen Schädlingen, **dadurch gekennzeichnet, dass** es einer Verbindung mit der Formel (I) nach einem der Ansprüche 1 bis 3 oder einer Formulierung nach einem der Ansprüche 6 bis 8 ermöglicht wird, auf die Schädlinge und/oder deren Habitat einzuwirken, wobei Verfahren zur Behandlung des menschlichen oder tierischen Körpers durch Chirurgie oder Therapie und am menschlichen oder tierischen Körper praktizierte diagnostische Verfahren ausgeschlossen sind.

10. Verfahren nach Anspruch 9, **dadurch gekennzeichnet, dass** der Schädling ein tierischer Schädling ist und ein Insekt, eine Arachnide oder einen Nematoden umfasst, oder dass der Schädling ein Insekt, eine Arachnide oder ein Nematode ist, wobei Verfahren zur Behandlung des menschlichen oder tierischen Körpers durch Chirurgie oder Therapie und am menschlichen oder tierischen Körper praktizierte diagnostische Verfahren ausgeschlossen sind.

11. Verwendung einer Verbindung mit der Formel (I) nach einem der Ansprüche 1 bis 5 oder eine Formulierung nach einem der Ansprüche 6 bis 8 zur Bekämpfung tierischer Schädlinge, wobei die Durchführung von Verfahren zur Behandlung des menschlichen oder tierischen Körpers durch Chirurgie oder Therapie und am menschlichen oder tierischen Körper praktizierten diagnostischen Verfahren ausgeschlossen ist.

12. Verwendung nach Anspruch 11, **dadurch gekennzeichnet, dass** der tierische Schädling ein Insekt, eine Arachnide oder einen Nematoden umfasst, oder dass der tierische Schädling ein Insekt, eine Arachnide oder ein Nematode ist, wobei die Durchführung von Verfahren zur Behandlung des menschlichen oder tierischen Körpers durch Chirurgie oder Therapie und am menschlichen oder tierischen Körper praktizierten diagnostischen Verfahren ausgeschlossen ist.

13. Verwendung nach Anspruch 11 oder 12 zum Pflanzenschutz, wobei die Durchführung von Verfahren zur Behandlung des menschlichen oder tierischen Körpers durch Chirurgie oder Therapie und am menschlichen oder tierischen Körper praktizierten diagnostischen Verfahren ausgeschlossen ist.

14. Verwendung nach Anspruch 11 oder 12 auf dem Gebiet der Tiergesundheit, wobei die Durchführung von Verfahren zur Behandlung des menschlichen oder tierischen Körpers durch Chirurgie oder Therapie und am menschlichen oder tierischen Körper praktizierten diagnostischen Verfahren ausgeschlossen ist.

15. Verfahren zum Schutz von Saatgut oder keimenden Pflanzen vor Schädlingen, insbesondere tierischen Schädlingen, wobei es einen Verfahrensschritt umfasst, in welchem das Saatgut mit einer Verbindung mit der Formel (I) nach einem der Ansprüche 1 bis 3 oder mit einer Formulierung nach einem der Ansprüche 6 bis 8 in Berührung gebracht wird, wobei Verfahren zur Behandlung des menschlichen oder tierischen Körpers durch Chirurgie oder Therapie und am menschlichen oder tierischen Körper praktizierte diagnostische Verfahren ausgeschlossen sind.

## Revendications

1. Composé de formule (I) 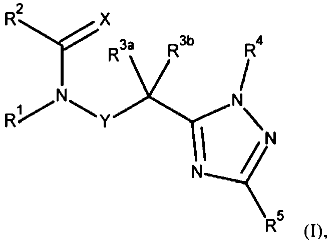 dans lequel
X représente O ;
Y représente une liaison directe ;
R¹ représente hydrogène ;
R² représente phényle, pyridine, pyrimidine, pyrazine ou pyridazine, dans lequel phényle, pyridine, pyrimidine, pyrazine ou pyridazine est substitué par un total d'un à trois substituants, à condition que le ou les substituants ne se trouvent pas sur l'un ou l'autre des carbones adjacents au carbone lié au groupe C=X et qu'au moins un et jusqu'à deux substituants soient indépendamment choisis dans le groupe A constitué de
alkyle en C₁-C₄ substitué par un à deux substituants choisis dans le groupe constitué de -CN ; et cycloalkyle en C₃-C₆, dans lequel cycloalkyle en C₃-C₆ est éventuellement substitué par halogène, -CN, alkyle en C₁-C₄ ou haloalkyle en C₁-C₄ ; alkylthio en C₁-C₄, alkylsulfinyle en C₁-C₄, alkylsulfonyle en C₁-C₄, cycloalkylsulfanyle en C₃-C₆, cycloalkylsulfinyle en C₃-C₆, cycloalkylsulfonyle en C₃-C₆, haloalkylsulfinyle en C₁-C₄ et haloalkylsulfonyle en C₁-C₄ ;
phénylsulfanyle, dans lequel phényle est éventuellement substitué par un à deux substituants choisis dans le groupe constitué d'halogène, -CN, alkyle en C₁-C₃, haloalkyle en C₁-C₃ et haloalcoxy en C₁-C₃ ;
phénylsulfinyle, dans lequel phényle est éventuellement substitué par un à deux substituants choisis dans le groupe constitué d'halogène, -CN, alkyle en C₁-C₃, haloalkyle en C₁-C₃ et haloalcoxy en C₁-C₃ ;
phénylsulfonyle, dans lequel phényle est éventuellement substitué par un à deux substituants choisis dans le groupe constitué d'halogène, -CN, alkyle en C₁-C₃, haloalkyle en C₁-C₃ et haloalcoxy en C₁-C₃ ;
et phényle et hétéroaryle à 5 à 6 chaînons, dans lequel phényle ou hétéroaryle à 5 à 6 chaînons est éventuellement substitué par un à deux substituants choisis dans le groupe constitué d'halogène, -CN, alkyle en C₁-C₃, haloalkyle en C₁-C₃ et haloalcoxy en C₁-C₃ ;
les autres un à deux substituants éventuels sont choisis chacun indépendamment dans le groupe B constitué de
halogène, -CN, -NO₂, alkyle en C₁-C₆, cycloalkyle en C₃-C₆, haloalkyle en C₁-C₄, alcoxy en C₁-C₄, haloalcoxy en C₁-C₄, alkylthio en C₁-C₄, alkylsulfinyle en C₁-C₄, alkylsulfonyle en C₁-C₄, haloalkylthio en C₁-C₄, haloalkylsulfinyle en C₁-C₄, et haloalkylsulfonyle en C₁-C₄ ;
ou
R² représente thiophène, furane, pyrazole, thiazole, isothiazole, oxazole ou isoxazole dont chacun est éventuellement substitué par un à trois substituants choisis indépendamment dans le groupe constitué de
halogène, hydroxy, -CN, -NO₂ ; alkyle en C₁-C₆ éventuellement substitué par -CN ; cycloalkyle en C₃-C₆, éventuellement substitué par halogène, -CN, alkyle en C₁-C₄ ou haloalkyle en C₁-C₄ ; haloalkyle en C₁-C₄, alcoxy en C₁-C₄, haloalcoxy en C₁-C₄, alkylthio en C₁-C₄, alkylsulfinyle en C₁-C₄, alkylsulfonyle en C₁-C₄, cycloalkylsulfanyle en C₃-C₆, cycloalkylsulfinyle en C₃-C₆, cycloalkylsulfonyle en C₃-C₆, haloalkylthio en C₁-C₄, haloalkylsulfinyle en C₁-C₄, haloalkylsulfonyle en C₁-C₄ ;
et phényle et hétéroaryle à 5 à 6 chaînons, dans lequel phényle ou hétéroaryle à 5 à 6 chaînons est éventuellement substitué par un à deux substituants choisis dans le groupe constitué d'halogène, -CN, alkyle en C₁-C₃ et haloalkyle en C₁-C₃.
R^{3a} représente H ;
R^{3b} représente méthyle ;
R⁴ représente pyrimidin-2-yle, 5-chloropyrimidin-2-yle, 5-chloropyridin-2-yle, 5-cyanopyridin-2-yle, 5-cyano-1,3-thiazol-2-yle, 5-carboxypyridin-2-yle, 5-(méthoxycarbonyl)pyridin-2-yle, 5-(diméthylaminocarbonyl)pyridin-2-yle, 5-[[éthyl(méthyl)amino]carbonyl]pyridin-2-yle, 5-[[cyclopropyl(méthyl)amino]carbonyl]pyridin-2-yle, 5-[[cyclopropylméthyl(méthyl)amino]carbonyl]pyridin-2-yle, 5-[(cyclopropylcarbonyl)(méthyl)amino]-pyridin-2-yle, 5-(pyrrolidin-1-ylcarbonyl)pyridin-2-yle, 5-(morpholin-4-ylcarbonyl)pyridin-2-yle, 5-(morpholin-4-carbonyl)thiazol-2-yle], 5-[[rac-(2R,6R)-2,6-diméthylmorpholin-4-yl]carbonyl]pyridin-2-yle, ou 5-[[(2R,6S)-2,6-diméthylmorpholin-4-yl]carbonyl]pyridin-2-yle ;
R⁵ représente -NHCO-*tert*-butyloxy, -NH₂, -N-méthyl-CO-*tert*-butyloxy, -NHCO-méthoxy, -NHCO-méthyle, -NHCO-cyclopropyle, -NHCO-(1-chloro-cyclopropyle), -NHCO-(2-fluoro-cyclopropyle), -NHCO-(2,2-difluoro-cyclopropyle), -NHCO-(2,2,2-trifluoroéthyle), -NHCO-(1-chloroéthyle), -NHSO₂-trifluorométhyle, -N(SO₂-méthyle)₂, méthylamino, diméthylamino, acétyl(méthyl)amino, prop-2-énoylamino, méthoxy(méthyl)amino, pyrrolidin-1-yle, pipéridin-1-yle, ou morpholin-4-yle.

2. Composé selon la revendication 1, dans lequel les éléments structurels X, Y, R¹, R^{3a}, R^{3b}, R⁴ et R⁵ ont les significations données dans la revendication 1 et
R² représente phényle ou pyridine, dans lequel phényle ou pyridine est substitué par un total d'un à trois substituants, à condition que le ou les substituants ne se trouvent pas sur l'un ou l'autre des carbones adjacents au carbone lié au groupe C=X et qu'un substituant soit indépendamment choisi dans le groupe A constitué de
1-cyano-1-méthyl-éthyle, cyclopropyle, 1-cyanocyclopropyle, 1-(trifluorométhyl)cyclopropyle, méthylthio, méthylsulfinyle, méthylsulfonyle, éthylthio, éthylsulfinyle, éthylsulfonyle, isopropylthio, isopropylsulfinyle, isopropylsulfonyle, cyclopropylthio, cyclopropylsulfinyle, cyclopropylsulfonyle, difluorométhylsulfinyle, difluorométhylsulfonyle, trifluorométhylsulfinyle, trifluorométhylsulfonyle ;
phénylsulfonyle, dans lequel phényle est éventuellement substitué par un deux substituants choisis dans le groupe constitué de fluor, chlore, brome, -CN, difluorométhyle, trifluorométhyle et trifluorométhoxy ;
et phényle et hétéroaryle à 5 chaînons, dans lequel phényle ou hétéroaryle à 5 chaînons est éventuellement substitué par un deux substituants choisis dans le groupe constitué de fluor, chlore, brome, -CN, difluorométhyle, trifluorométhyle et trifluorométhoxy ;
les autres un à deux substituants éventuels sont choisis chacun indépendamment dans le groupe B constitué de
fluor, chlore, brome, iode, -CN, -NO₂, méthyle, cyclopropyle, difluorométhyle, trifluorométhyle, méthoxy, difluorométhoxy, trifluorométhoxy, méthylthio, méthylsulfinyle, méthylsulfonyle, difluorométhylthio, difluorométhylsulfinyle, difluorométhylsulfonyle, trifluorométhylthio, trifluorométhylsulfinyle, trifluorométhylsulfonyle ;
ou
R² représente thiophène, furane, pyrazole, thiazole, oxazole ou isoxazole dont chacun est éventuellement substitué par un à trois substituants choisis indépendamment dans le groupe constitué de
fluor, chlore, brome, iode, -CN, -NO₂, méthyle, cyclopropyle, difluorométhyle, trifluorométhyle, méthoxy, difluorométhoxy, trifluorométhoxy, méthylthio, méthylsulfinyle, méthylsulfonyle, difluorométhylthio, difluorométhylsulfinyle, difluorométhylsulfonyle, trifluorométhylthio, trifluorométhylsulfinyle, trifluorométhylsulfonyle.

3. Composé selon la revendication 1 ou 2, dans lequel
X représente O ;
Y représente une liaison directe ;
R¹ représente hydrogène ;
R² représente 3-chloro-5-(méthylsulfonyl)phényle, 3-chloro-5-(difluorométhylsulfonyl)phényle, 3-chloro-5-(trifluorométhylsulfonyl)phényle, 3-méthylsulfonyl-5-(trifluorométhyl)phényle, 3-éthylsulfonyl-5-(trifluorométhyl)phényle, 3-méthylsulfonyl-5-(trifluorométhoxy)phényle, 3-éthylsulfonyl-5-(trifluorométhoxy)phényle, 3-isopropylsulfonyl-5-(trifluorométhoxy)phényle, 3-cyclopropylsulfonyl-5-(trifluorométhoxy)phényle, 3-(difluorométhylsulfonyl)-5-(trifluorométhoxy)phényle, 3-(4-chlorophényl)sulfonyl-5-(trifluorométhoxy)phényle, 3-cyclopropyl-5-(trifluorométhoxy)phényle, 3-cyclopropyl-5-(trifluorométhylsulfonyl)phényle, 3-bromo-5-[1-(trifluorométhyl)cyclopropyl]phényle, 3-chloro-5-(1-cyano-1-méthyl-éthyl)phényle, ou 1-méthyl-5-(trifluorométhyl)-pyrazol-3-yle ;
R^{3a} représente H ;
R^{3b} représente méthyle ;
R⁴ représente pyrimidin-2-yle, 5-chloropyrimidin-2-yle, 5-chloropyridin-2-yle, 5-cyanopyridin-2-yle, 5-cyano-1,3-thiazol-2-yle, 5-carboxypyridin-2-yle, 5-(méthoxycarbonyl)pyridin-2-yle, 5-(diméthylaminocarbonyl)pyridin-2-yle, 5-[[éthyl(méthyl)amino]carbonyl]pyridin-2-yle, 5-[[cyclopropyl(méthyl)amino]carbonyl]pyridin-2-yle, 5-[[cyclopropylméthyl(méthyl)amino]carbonyl]pyridin-2-yle, 5-[(cyclopropylcarbonyl)(méthyl)amino]-pyridin-2-yle, 5-(pyrrolidin-1-ylcarbonyl)pyridin-2-yle, 5-(morpholin-4-ylcarbonyl)pyridin-2-yle, 5-(morpholin-4-carbonyl)thiazol-2-yle], 5-[[rac-(2R,6R)-2,6-diméthylmorpholin-4-yl]carbonyl]pyridin-2-yle, ou 5-[[(2R,6S)-2,6-diméthylmorpholin-4-yl]carbonyl]pyridin-2-yle ;
R⁵ représente -NHCO-tert-butyloxy, -NH₂, -N-méthyl-CO-*tert*-butyloxy, -NHCO-méthoxy, -NHCO-méthyle, -NHCO-cyclopropyle, -NHCO-(1-chloro-cyclopropyle), -NHCO-(2-fluoro-cyclopropyle), -NHCO-(2,2-difluoro-cyclopropyle), -NHCO-(2,2,2-trifluoroéthyle), -NHCO-(1-chloroéthyle), -NHSO₂-trifluorométhyle, -N(SO₂-méthyle)₂, méthylamino, diméthylamino, acétyl(méthyl)amino, prop-2-énoylamino, méthoxy(méthyl)amino, pyrrolidin-1-yle, pipéridin-1-yle, ou morpholin-4-yle.

4. Composé de formule **(a)** 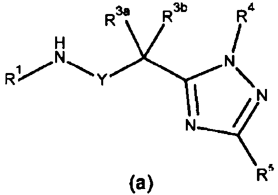 dans lequel R¹ représente hydrogène, R^{3b} représente méthyle, R^{3a} représente hydrogène et Y représente une liaison directe et dans lequel R⁴ et R⁵ ont les significations données dans la revendication 3.

5. Composé de formule **(b-1)** 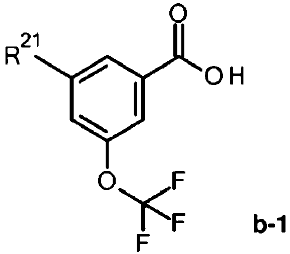 dans lequel
R²¹ représente difluorométhylsulfanyle, difluorométhylsulfinyle, ou difluorométhylsulfonyle.

6. Formulation, en particulier formulation agrochimique, comprenant au moins un composé de formule (I) selon l'une quelconque des revendications 1 à 3.

7. Formulation selon la revendication 6, comprenant en outre au moins un allongeur et/ou au moins une substance tensioactive.

8. Formulation selon la revendication 6 ou 7, **caractérisée en ce que** le composé de formule (I) est dans un mélange avec au moins un autre composé actif.

9. Procédé pour la lutte contre des organismes nuisibles, en particulier des animaux nuisibles, **caractérisé en ce qu'**un composé de formule (I) selon l'une quelconque des revendications 1 à 3 ou une formulation selon l'une quelconque des revendications 6 à 8 est autorisé à agir sur les organismes nuisibles et/ou leur habitat, tandis que des procédés pour le traitement du corps humain ou animal par chirurgie ou thérapie et des procédés de diagnostic pratiqués sur le corps humain ou animal sont exclus.

10. Procédé selon la revendication 9, **caractérisé en ce que** l'organisme nuisible est un animal nuisible et comprend un insecte, un arachnide ou un nématode, ou **en ce que** l'organisme nuisible est un insecte, un arachnide ou un nématode, tandis que des procédés pour le traitement du corps humain ou animal par chirurgie ou thérapie et des procédés de diagnostic pratiqués sur le corps humain ou animal sont exclus.

11. Utilisation d'un composé de formule (I) selon l'une quelconque des revendications 1 à 5 ou d'une formulation selon l'une quelconque des revendications 6 à 8 pour la lutte contre des animaux nuisibles, tandis que l'utilisation de procédés pour le traitement du corps humain ou animal par chirurgie ou thérapie et de procédés de diagnostic pratiqués sur le corps humain ou animal est exclue.

12. Utilisation selon la revendication 11, **caractérisée en ce que** l'animal nuisible comprend un insecte, un arachnide ou un nématode, ou **en ce que** l'animal nuisible est un insecte, un arachnide ou un nématode, tandis que l'utilisation de procédés pour le traitement du corps humain ou animal par chirurgie ou thérapie et de procédés de diagnostic pratiqués sur le corps humain ou animal est exclue.

13. Utilisation selon la revendication 11 ou 12 dans la protection des cultures, tandis que l'utilisation de procédés pour le traitement du corps humain ou animal par chirurgie ou thérapie et de procédés de diagnostic pratiqués sur le corps humain ou animal est exclue.

14. Utilisation selon la revendication 11 ou 12 dans le domaine de la santé animale, tandis que l'utilisation de procédés pour le traitement du corps humain ou animal par chirurgie ou thérapie et de procédés de diagnostic pratiqués sur le corps humain ou animal est exclue.

15. Procédé permettant de protéger une semence ou une plante germée contre des organismes nuisibles, en particulier des animaux nuisibles, comprenant une étape de procédé dans laquelle la semence est mise en contact avec un composé de formule (I) selon l'une quelconque des revendications 1 à 3 ou avec une formulation selon l'une quelconque des revendications 6 à 8, tandis que des procédés pour le traitement du corps humain ou animal par chirurgie ou thérapie et des procédés de diagnostic pratiqués sur le corps humain ou animal sont exclus.
